(19) Europäisches Patentamt / European Patent Office / Office européen des brevets

(11) **EP 2 831 240 B1**

(12) **EUROPEAN PATENT SPECIFICATION**

(45) Date of publication and mention
of the grant of the patent:
**15.11.2017 Bulletin 2017/46**

(21) Application number: **13713359.1**

(22) Date of filing: **27.03.2013**

(51) Int Cl.:
***C12N 15/67*** (2006.01)

(86) International application number:
**PCT/EP2013/000938**

(87) International publication number:
**WO 2013/143700 (03.10.2013 Gazette 2013/40)**

(54) **ARTIFICIAL NUCLEIC ACID MOLECULES COMPRISING A 5'TOP UTR**

KÜNSTLICHE NUKLEINSÄURENMOLEKÜLE ENTHALTEND EIN 5'TOP UTR

ACIDES NUCLÉIQUES ARTIFICIELLES COMPRENANT UNE 5'TOP UTR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.03.2012 PCT/EP2012/001334**
**08.06.2012 PCT/EP2012/002448**

(43) Date of publication of application:
**04.02.2015 Bulletin 2015/06**

(73) Proprietor: **CureVac AG**
**72076 Tübingen (DE)**

(72) Inventor: **THEß, Andreas**
**72127 Kusterdingen (DE)**

(74) Representative: **Graf von Stosch, Andreas et al**
**Graf von Stosch**
**Patentanwaltsgesellschaft mbH**
**Prinzregentenstraße 22**
**80538 München (DE)**

(56) References cited:
**US-A1- 2005 048 549     US-A1- 2010 120 152**

• **DATABASE EMBL [Online] 2 October 2002 (2002-10-02), "Homo sapiens RPL36AL mRNA for ribosomal protein L36a-like, complete cds.", XP002699423, retrieved from EBI accession no. EM_STD:AB063609 Database accession no. AB063609**

• **DATABASE Geneseq [Online] 11 December 2008 (2008-12-11), "Human transcriptional regulatory element SEQ ID NO 6587.", XP002699424, retrieved from EBI accession no. GSN:ATN08647 Database accession no. ATN08647**

• **S. LEVY ET AL: "Oligopyrimidine tract at the 5' end of mammalian ribosomal protein mRNAs is required for their translational control.", PROCEEDINGS OF THE NATIONAL ACADEMY OF SCIENCES, vol. 88, no. 8, 15 April 1991 (1991-04-15) , pages 3319-3323, XP055044877, ISSN: 0027-8424, DOI: 10.1073/pnas.88.8.3319**

• **ULF ANDERSSON ØROM ET AL: "MicroRNA-10a Binds the 5'UTR of Ribosomal Protein mRNAs and Enhances Their Translation", MOLECULAR CELL, vol. 30, no. 4, 1 May 2008 (2008-05-01), pages 460-471, XP055044753, ISSN: 1097-2765, DOI: 10.1016/j.molcel.2008.05.001 cited in the application**

• **AVNI: "Vertebrate mRNAs with a 5'-terminal pyrimidine tract are candidates for translational repression in quiescent cells: characterization of the translational cis-regulatory element.", MOLECULAR AND CELLULAR BIOLOGY, vol. 14, no. 6, 1 January 1994 (1994-01-01), page 3822, XP055044702, ISSN: 0270-7306, DOI: 10.1128/MCB.14.6.3822 cited in the application**

• **D. AVNI ET AL: "The 5' Terminal Oligopyrimidine Tract Confers Translational Control on Top Mrnas in a Cell Type-and Sequence Context-Dependent Manner", NUCLEIC ACIDS RESEARCH, vol. 25, no. 5, 1 March 1997 (1997-03-01), pages 995-1001, XP055044700, ISSN: 0305-1048, DOI: 10.1093/nar/25.5.995**

EP 2 831 240 B1

- LEDDA M ET AL: "Effect of 3'UTR length on the translational regulation of 5'-terminal oligopyrimidine mRNAs", GENE, ELSEVIER, AMSTERDAM, NL, vol. 344, 3 January 2005 (2005-01-03), pages 213-220, XP004712180, ISSN: 0378-1119, DOI: 10.1016/J.GENE.2004.09.023
- C. K. DAMGAARD ET AL: "Translational coregulation of 5'TOP mRNAs by TIA-1 and TIAR", GENES & DEVELOPMENT, vol. 25, no. 19, 1 October 2011 (2011-10-01), pages 2057-2068, XP055044892, ISSN: 0890-9369, DOI: 10.1101/gad.17355911
- R. YAMASHITA ET AL: "Comprehensive detection of human terminal oligo-pyrimidine (TOP) genes and analysis of their characteristics", NUCLEIC ACIDS RESEARCH, vol. 36, no. 11, 1 January 2008 (2008-01-01), pages 3707-3715, XP055044886, ISSN: 0305-1048, DOI: 10.1093/nar/gkn248 cited in the application -& Riu Yamshita ET AL: "Comprehensive detection of human terminal oligo-pyrimidine (TOP) genes and analysis of their characteristics - Supplementary data 7", Nucleic Acids Research, 14 May 2008 (2008-05-14), pages 1-2, XP055067985, Retrieved from the Internet: URL:http://nar.oxfordjournals.org/content/36/11/3707/suppl/DC1 [retrieved on 2013-06-24] -& RIU YAMASHITA ET AL: "Comprehensive detection of human terminal oligo-pyrimidine (TOP) genes and analysis of their characteristics - Supplementary Table 2", INTERNET CITATION, vol. 36, no. 11, 1 June 2008 (2008-06-01), pages 1-4, XP002687762, ISSN:
- DAVULURI RAMANA V ET AL: "CART classification of human 5' UTR sequences", GENOME RESEARCH, COLD SPRING HARBOR LABORATORY PRESS, WOODBURY, NY, US, vol. 10, no. 11, 1 November 2000 (2000-11-01), pages 1807-1816, XP002605204, ISSN: 1088-9051, DOI: 10.1101/GR.146000
- ODED MEYUHAS: "Synthesis of the translational apparatus is regulated at the translational level", EUROPEAN JOURNAL OF BIOCHEMISTRY, vol. 267, no. 21, 1 November 2000 (2000-11-01), pages 6321-6330, XP055044924, ISSN: 0014-2956, DOI: 10.1046/j.1432-1327.2000.01719.x cited in the application
- V. IADEVAIA ET AL: "All translation elongation factors and the e, f, and h subunits of translation initiation factor 3 are encoded by 5'-terminal oligopyrimidine (TOP) mRNAs", RNA, vol. 14, no. 9, 1 January 2008 (2008-01-01), pages 1730-1736, XP055044697, ISSN: 1355-8382, DOI: 10.1261/rna.1037108 cited in the application
- S. CALDAROLA: "Translational Regulation of Terminal Oligopyrimidine mRNAs Induced by Serum and Amino Acids Involves Distinct Signaling Events", JOURNAL OF BIOLOGICAL CHEMISTRY, vol. 279, no. 14, 1 January 2004 (2004-01-01), pages 13522-13531, XP055045294, ISSN: 0021-9258, DOI: 10.1074/jbc.M310574200
- CHRISTINE I. WOODDELL ET AL: "Sustained liver-specific transgene expression from the albumin promoter in mice following hydrodynamic plasmid DNA delivery", THE JOURNAL OF GENE MEDICINE, vol. 10, no. 5, 1 May 2008 (2008-05-01), pages 551-563, XP055041510, ISSN: 1099-498X, DOI: 10.1002/jgm.1179
- A. KNAPINSKA ET AL: "Molecular Mechanisms Regulating mRNA Stability: Physiological and Pathological Significance", CURRENT GENOMICS, vol. 6, no. 6, 1 October 2005 (2005-10-01), pages 471-486, XP055076794, ISSN: 1389-2029, DOI: 10.2174/138920205774482954

**Description**

The invention relates to artificial nucleic acid molecules comprising a 5'UTR element derived from the 5'UTR of a TOP gene, an open reading frame, and a 3'UTR element, and, optionally, a poly(A) sequence and/or a polyadenylation signal. The invention relates further to a vector comprising a 5'UTR element derived from the 5'UTR of a TOP gene, to a pharmaceutical composition comprising the artificial nucleic acid molecule or the vector; the artificial nucleic acid molecule, the vector or the pharmaceutical composition, preferably for use in the field of gene therapy and/or genetic vaccination.

Gene therapy and genetic vaccination belong to the most promising and quickly developing methods of modern medicine. They may provide highly specific and individual options for therapy of a large variety of diseases. Particularly, inherited genetic diseases but also autoimmune diseases, cancerous or tumour-related diseases as well as inflammatory diseases may be the subject of such treatment approaches. Also, it is envisaged to prevent (early) onset of such diseases by these approaches.

The main conceptual rational behind gene therapy is appropriate modulation of impaired gene expression associated with pathological conditions of specific diseases. Pathologically altered gene expression may result in lack or overproduction of essential gene products, for example, signalling factors such as hormones, housekeeping factors, metabolic enzymes, structural proteins or the like. Altered gene expression may not only be due to misregulation of transcription and/or translation, but also due to mutations within the ORF coding for a particular protein. Pathological mutations may be caused by e.g. chromosomal aberration, or by more specific mutations, such as point or frame-shift-mutations, all of them resulting in limited functionality and, potentially, total loss of function of the gene product.

However, misregulation of transcription or translation may also occur, if mutations affect genes encoding proteins which are involved in the transcriptional or translational machinery of the cell. Such mutations may lead to pathological up- or down-regulation of genes which are - as such - functional. Genes encoding gene products which exert such regulating functions, may be, e.g., transcription factors, signal receptors, messenger proteins or the like. However, loss of function of such genes encoding regulatory proteins may, under certain circumstances, be reversed by artificial introduction of other factors acting further downstream of the impaired gene product. Such gene defects may also be compensated by gene therapy via substitution of the affected gene itself.

Genetic vaccination allows evoking a desired immune response to selected antigens, such as characteristic components of bacterial surfaces, viral particles, tumour antigens or the like. Generally, vaccination is one of the pivotal achievements of modern medicine. However, effective vaccines are currently available only for a smaller number of diseases. Accordingly, infections that are not preventable by vaccination still affect millions of people every year.

Commonly, vaccines may be subdivided into "first", "second" and "third" generation vaccines. "First generation" vaccines are, typically, whole-organism vaccines. They are based on either live and attenuated or killed pathogens, e.g. viruses, bacteria or the like. The major drawback of live and attenuated vaccines is the risk for a reversion to life-threatening variants. Thus, although attenuated, such pathogens may still intrinsically bear unpredictable risks. Killed pathogens may not be as effective as desired for generating a specific immune response. In order to minimize these risks, "second generation" vaccines were developed. These are, typically, subunit vaccines, consisting of defined antigens or recombinant protein components which are derived from pathogens.

Genetic vaccines, i.e. vaccines for genetic vaccination, are usually understood as "third generation" vaccines. They are typically composed of genetically engineered nucleic acid molecules which allow expression of peptide or protein (antigen) fragments characteristic for a pathogen or a tumor antigen *in vivo.* Genetic vaccines are expressed upon administration to a patient and uptake by competent cells. Expression of the administered nucleic acids results in production of the encoded proteins. In the event these proteins are recognized as foreign by the patient's immune system, an immune response is triggered.

As can be seen from the above, both methods, gene therapy and genetic vaccination, are essentially based on the administration of nucleic acid molecules to a patient and subsequent transcription and/or translation of the encoded genetic information. Alternatively, genetic vaccination or gene therapy may also comprise methods which include isolation of specific body cells from a patient to be treated, subsequent *in vitro* transfection of such cells, and re-administration of the treated cells to the patient.

DNA as well as RNA may be used as nucleic acid molecules for administration in the context of gene therapy or genetic vaccination. DNA is known to be relatively stable and easy to handle. However, the use of DNA bears the risk of undesired insertion of the administered DNA-fragments into the patient's genome potentially resulting in loss of function of the impaired genes. As a further risk, the undesired generation of anti-DNA antibodies has emerged. Another drawback is the limited expression level of the encoded peptide or protein that is achievable upon DNA administration and its transcription/translation. Among other reasons, the expression level of the administered DNA will be dependent on the presence of specific transcription factors which regulate DNA transcription. In the absence of such factors, DNA transcription will not yield satisfying amounts of RNA. As a result, the level of translated peptide or protein obtained is limited.

By using RNA instead of DNA for gene therapy or genetic vaccination, the risk of undesired genomic integration and generation of anti-DNA antibodies is minimized or avoided. However, RNA is considered to be a rather unstable molecular species which may readily be degraded by ubiquitous RNAses.

*In vivo,* RNA-degradation contributes to the regulation of the RNA half-life time. That effect was considered and proven to fine tune the regulation of eukaryotic gene expression (Friedel et al., Conserved principles of mammalian transcriptional regulation revealed by RNA half-life, Nucleic Acid Research, 2009, 1-12). Accordingly, each naturally occurring mRNA has its individual half-life depending on the gene from which the mRNA is derived. It contributes to the regulation of the expression level of this gene. Unstable RNAs are important to realize transient gene expression at distinct points in time. However, long-lived RNAs may be associated with accumulation of distinct proteins or continuous expression of genes. *In vivo,* the half life of mRNAs may also be dependent on environmental factors, such as hormonal treatment, as has been shown, e.g., for insulin-like growth factor I, actin, and albumin mRNA (Johnson et al., Newly synthesized RNA: Simultaneous measurement in intact cells of transcription rates and RNA stability of insulin-like growth factor I, actin, and albumin in growth hormone-stimulated hepatocytes, Proc. Natl. Acad. Sci., Vol. 88, pp. 5287-5291, 1991).

For gene therapy and genetic vaccination, usually stable RNA is desired. This is, on the one hand, due to the fact that the product encoded by the RNA-sequence shall accumulate *in vivo.* On the other hand, the RNA has to maintain its structural and functional integrity when prepared for a suitable dosage form, in the course of its storage, and when administered. Thus, considerable attention was dedicated to provide stable RNA molecules for gene therapy or genetic vaccination in order to prevent them from being subject to early degradation or decay.

It has been reported that the G/C-content of nucleic acid molecules may influence their stability. Thus, nucleic acids comprising an increased amount of guanine (G) and/or cytosine (C) residues may be functionally more stable than nucleic acids containing a large amount of adenine (A) and thymine (T) or uracil (U) nucleotides. In this context, WO02/098443 provides a pharmaceutical composition containing an mRNA that is stabilised by sequence modifications in the translated region. Such a sequence modification takes advantage of the degeneracy of the genetic code. Accordingly, codons which contain a less favourable combination of nucleotides (less favourable in terms of RNA stability) may be substituted by alternative codons without altering the encoded amino acid sequence. This method of RNA stabilization is limited by the provisions of the specific nucleotide sequence of each single RNA molecule which is not allowed to leave the space of the desired amino acid sequence. Also, that approach is restricted to coding regions of the RNA.

As an alternative option for mRNA stabilisation, it has been found that naturally occurring eukaryotic mRNA molecules contain characteristic stabilising elements. For example, they may comprise so-called untranslated regions (UTR) at their 5'-end (5'UTR) and/or at their 3'-end (3'UTR) as well as other structural features, such as a 5'-cap structure or a 3'-poly(A) tail. Both, 5'UTR and 3'UTR are typically transcribed from the genomic DNA and are, thus, an element of the premature mRNA. Characteristic structural features of mature mRNA, such as the 5'-cap and the 3'-poly(A) tail (also called poly(A) tail or poly(A) sequence) are usually added to the transcribed (premature) mRNA during mRNA processing.

A 3'-poly(A) tail is typically a monotonous sequence stretch of adenine nucleotides added to the 3'-end of the transcribed mRNA. It may comprise up to about 400 adenine nucleotides. It was found that the length of such a 3'-poly(A) tail is a potentially critical element for the stability of the individual mRNA.

Also, it was shown that the 3'UTR of α-globin mRNA may be an important factor for the well-known stability of α-globin mRNA (Rodgers et al., Regulated α-globin mRNA decay is a cytoplasmic event proceeding through 3'-to-5' exosome-dependent decapping, RNA, 8, pp. 1526-1537, 2002). The 3'UTR of α-globin mRNA is obviously involved in the formation of a specific ribonucleoprotein-complex, the α-complex, whose presence correlates with mRNA stability *in vitro* (Wang et al., An mRNA stability complex functions with poly(A)-binding protein to stabilize mRNA in vitro, Molecular and Cellular biology, Vol 19, No. 7, July 1999, p. 4552-4560).

Irrespective of factors influencing mRNA stability, effective translation of the administered nucleic acid molecules by the target cells or tissue is crucial for any approach using nucleic acid molecules for gene therapy or genetic vaccination. Along with the regulation of stability, also translation of the majority of mRNAs is regulated by structural features like UTRs, 5'-cap and 3'-poly(A) tail. In this context, it has been reported that the length of the poly(A) tail may play an important role for translational efficiency as well. Stabilizing 3'-elements, however, may also have an attenuating effect on translation.

Further regulative elements, which may have an influence on expression levels, may be found in the 5'UTR. For example, it has been reported that synthesis of particular proteins, e.g. proteins belonging to the translational apparatus, may be regulated not only at the transcriptional but also at the translational level. For example, translation of proteins encoded by so called 'TOP-genes' may be down-regulated by translational repression. Therein, the term 'TOP-gene' relates to a gene corresponding to an mRNA that is characterized by the presence of a TOP sequence at the 5'end and in most cases by a growth-associated translation regulation (Iadevaia et al., All translation elongation factors and the e, f, and h subunits of translation initiation factor 3 are encoded by 5'-terminal oligopyrimidine (TOP) mRNAs; RNA, 2008, 14:1730-1736). In this context, a TOP sequence - also called the '5'-terminal oligopyrimidine tract' - typically consists of a C residue at the cap site, followed by an uninterrupted sequence of up to 13 or even more pyrimidines (Avni et al., Vertebrate mRNAs with a 5'-terminal pyrimidine tract are Candidates for translational repression in quiescent cells: characterization of the translational cis-regulatory element, Molecular and Cellular Biology, 1994, p. 3822-3833). These TOP sequences are reported to be present in many mRNAs encoding components of the translational machinery and to be responsible for selective repression of the translation of these TOP containing mRNAs due to growth arrest (Meyuhas, et al., Translational Control of Ribosomal Protein mRNAs in Eukaryotes, Translational Control. Cold Spring Harbor Monograph Archive. Cold Spring Harbor Laboratory Press, 1996, p. 363-388). These TOP sequences are thought to serve as a cis-regulatory element which inhibits the binding of translational regulatory proteins or the translational machinery itself. As a result, the translation of these genes is inhibited at the growth arrest of cells. More specifically, when a cell is faced with starvation or treated by some chemicals such as 12-Tetradecanoyl-1-phorbol-13-acetate (TPA), mRNAs of TOP genes, which are normally associated with polysomes, change their status into the translationally inactive 'sub-polysome' while most non-TOP mRNAs stay in the'polysome' state (Yamashita et al., Comprehensive detection of human terminal oligo-pyrimidine (TOP) genes and analysis of their characteristics. Nucleic Acids Res. 2008 Jun; 36(11):3707-15. doi: 10.1093/nar/gkn248. Epub 2008 May 14). In this context, it was shown that the oligopyrimidine tract at the 5'end of the 5'UTR (TOP motif) was required for translational repression of TOP genes. The oligopyrimidine tract at the 5' end of mammalian ribosomal protein mRNAs is required for their translational control (Levy et al., Proc Natl Acad Sci USA. 1991 Apr 15; 88(8):3319-23). Furthermore, it was shown that miRNA miR-10a positively controls the translation of ribosomal proteins by binding downstream of the TOP motif present in the 5'UTRs of TOP genes. Such an enhancement of translation was dependent on the presence of the TOP motif in the 5'UTR. Furthermore this translational regulation of ribosomal TOP genes was dependent on the presence of miR-10a or its human homolog miR-10b which is highly overexpressed in several tumor types and is reportedly involved in the progression of cancer (Ørom et al., MicroRNA-10a binds the 5'UTR of ribosomal protein mRNAs and enhances their translation. Mol Cell. 2008 May 23; 30(4):460-71).

Damgaard et al. (Genes & Development, Vol. 25, no. 19, 2011, p. 2057 - 2068) discloses artificial nucleic acid moleculs comprising a 5'UTR of a TOP gene, an open readily frame and a 3'UTR of human β-globin.

It is the object of the invention to provide nucleic acid molecules which may be suitable for application in gene therapy and/or genetic vaccination. Particularly, it is the object of the invention to provide artificial nucleic acid molecules, such as an mRNA species, which provide for increased protein production from said artificial nucleic acid molecules, preferably which exhibit increased translational efficiency. Another object of the present invention is to provide nucleic acid molecules coding for such a superior mRNA species which may be amenable for use in gene therapy and/or genetic vaccination. It is a further object of the present invention to provide a pharmaceutical composition for use in gene therapy and/or genetic vaccination. In summary, it is the object of the present invention to provide improved nucleic acid species which overcome the above discussed disadvantages of the prior art by a cost-effective and straight-forward approach.

The object underlying the present invention is solved by the claimed subject-matter.

For the sake of clarity and readability the following definitions are provided. Any technical feature mentioned for these definitions may be read on each and every embodiment of the invention. Additional definitions and explanations may be specifically provided in the context of these embodiments.

Adaptive immune response: The adaptive immune response is typically understood to be an antigen-specific response of the immune system. Antigen specificity allows for the generation of responses that are tailored to specific pathogens or pathogen-infected cells. The ability to mount these tailored responses is usually maintained in the body by "memory cells". Should a pathogen infect the body more than once, these specific memory cells are used to quickly eliminate it. In this context, the first step of an adaptive immune response is the activation of naïve antigen-specific T cells or different immune cells able to induce an antigen-specific immune response by antigen-presenting cells. This occurs in the lymphoid tissues and organs through which naïve T cells are constantly passing. The three cell types that may serve as antigen-presenting cells are dendritic cells, macrophages, and B cells. Each of these cells has a distinct function in eliciting immune responses. Dendritic cells may take up antigens by phagocytosis and macropinocytosis and may become stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. MHC-molecules are, typically, responsible for presentation of an antigen to T-cells. Therein, presenting the antigen on MHC molecules leads to activation of T cells which induces their proliferation and differentiation into armed effector T cells. The most important function of effector T cells is the killing of infected cells by CD8+ cytotoxic T cells and the activation of macrophages by Th1 cells which together make up cell-mediated immunity, and the activation of B cells by both Th2 and Th1 cells to produce different classes of antibody, thus driving the humoral immune response. T cells recognize an antigen by their T cell receptors which do not recognize and bind the antigen directly, but instead recognize short peptide fragments e.g. of pathogen-derived protein antigens, e.g. so-called epitopes, which are bound to MHC molecules on the surfaces of other cells.

Adaptive immune system: The adaptive immune system is essentially dedicated to eliminate or prevent pathogenic growth. It typically regulates the adaptive immune response by providing the vertebrate immune system with the ability to recognize and remember specific pathogens (to generate immunity), and to mount stronger attacks each time the pathogen is encountered. The system is highly adaptable because of somatic hypermutation (a process of accelerated somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of such a cell will then inherit genes encoding the same receptor specificity, including the Memory B cells and Memory T cells that are the keys to long-lived specific immunity.

Adjuvant/adjuvant component: An adjuvant or an adjuvant component in the broadest sense is typically a pharmacological and/or immunological agent that may modify, e.g. enhance, the effect of other agents, such as a drug or vaccine. It is to be interpreted in a broad sense and refers to a broad spectrum of substances. Typically, these substances are able to increase the immunogenicity of antigens. For example, adjuvants may be recognized by the innate immune systems and, e.g., may elicit an innate immune response. "Adjuvants" typically do not elicit an adaptive immune response. Insofar, "adjuvants" do not qualify as antigens. Their mode of action is distinct from the effects triggered by antigens resulting in an adaptive immune response.

Antigen: In the context of the present invention "antigen" refers typically to a substance which may be recognized by the immune system, preferably by the adaptive immune system, and is capable of triggering an antigen-specific immune response, e.g. by formation of antibodies and/or antigen-specific T cells as part of an adaptive immune response. Typically, an antigen may be or may comprise a peptide or protein which may be presented by the MHC to T-cells.

Artificial nucleic acid molecule: An artificial nucleic acid molecule may typically be understood to be a nucleic acid molecule, e.g. a DNA or an RNA, that does not occur naturally. In other words, an artificial nucleic acid molecule may be understood as a non-natural nucleic acid molecule. Such nucleic acid molecule may be non-natural due to its individual sequence (which does not occur naturally) and/or due to other modifications, e.g. structural modifications of nucleotides which do not occur naturally. An artificial nucleic acid molecule may be a DNA molecule, an RNA molecule or a hybrid-molecule comprising DNA and RNA portions. Typically, artificial nucleic acid molecules may be designed and/or generated by genetic engineering methods to correspond to a desired artificial sequence of nucleotides (heterologous sequence). In this context an artificial sequence is usually a sequence that may not occur naturally, i.e. it differs from the wild type sequence by at least one nucleotide. The term 'wild type' may be understood as a sequence occurring in nature. Further, the term 'artificial nucleic acid molecule' is not restricted to mean 'one single molecule' but is, typically, understood to comprise an ensemble of identical molecules. Accordingly, it may relate to a plurality of identical molecules contained in an aliquot.

Bicistronic RNA, multicistronic RNA: A bicistronic or multicistronic RNA is typically an RNA, preferably an mRNA, that typically may have two (bicistronic) or more (multicistronic) open reading frames (ORF). An open reading frame in this context is a sequence of codons that is translatable into a peptide or protein.

Carrier / polymeric carrier: A carrier in the context of the invention may typically be a compound that facilitates transport and/or complexation of another compound (cargo). A polymeric carrier is typically a carrier that is formed of a polymer. A carrier may be associated to its cargo by covalent or non-covalent interaction. A carrier may transport nucleic acids, e.g. RNA or DNA, to the target cells. The carrier may - for some embodiments - be a cationic component.

Cationic component: The term "cationic component" typically refers to a charged molecule, which is positively charged (cation) at a pH value typically from 1 to 9, preferably at a pH value of or below 9 (e.g. from 5 to 9), of or below 8 (e.g. from 5 to 8), of or below 7 (e.g. from 5 to 7), most preferably at a physiological pH, e.g. from 7.3 to 7.4. Accordingly, a cationic component may be any positively charged compound or polymer, preferably a cationic peptide or protein which is positively charged under physiological conditions, particularly under physiological conditions *in vivo*. A 'cationic peptide or protein' may contain at least one positively charged amino acid, or more than one positively charged amino acid, e.g. selected from Arg, His, Lys or Orn. Accordingly, 'polycationic' components are also within the scope exhibiting more than one positive charge under the conditions given.

5'-cap: A 5'-cap is an entity, typically a modified nucleotide entity, which generally 'caps' the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an RNA. Further examples of 5'cap structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety.

Cellular immunity/cellular immune response: Cellular immunity relates typically to the activation of macrophages, natural killer cells (NK), antigen-specific cytotoxic T-lymphocytes, and the release of various cytokines in response to an antigen. In more general terms, cellular immunity is not based on antibodies, but on the activation of cells of the immune system. Typically, a cellular immune response may be characterized e.g. by activating antigen-specific cytotoxic T-lymphocytes that are able to induce apoptosis in cells, e.g. specific immune cells like dendritic cells or other cells, displaying epitopes of foreign antigens on their surface. Such cells may be virus-infected or infected with intracellular bacteria, or cancer cells displaying tumor antigens. Further characteristics may be activation of macrophages and natural killer cells, enabling them to destroy pathogens and stimulation of cells to secrete a variety of cytokines that influence the function of other cells involved in adaptive immune responses and innate immune responses.

DNA: DNA is the usual abbreviation for deoxy-ribonucleic-acid. It is a nucleic acid molecule, i.e. a polymer consisting of nucleotides. These nucleotides are usually deoxy-adenosine-monophosphate, deoxy-thymidine-monophosphate, deoxy-guanosine-monophosphate and deoxy-cytidine-monophosphate monomers which are - by themselves - composed of a sugar moiety (deoxyribose), a base moiety and a phosphate moiety, and polymerize by a characteristic backbone structure. The backbone structure is, typically, formed by phosphodiester bonds between the sugar moiety of the nucleotide, i.e. deoxyribose, of a first and a phosphate moiety of a second, adjacent monomer. The specific order of the monomers, i.e. the order of the bases linked to the sugar/phosphate-backbone, is called the DNA-sequence. DNA may be single stranded or double stranded. In the double stranded form, the nucleotides of the first strand typically hybridize with the nucleotides of the second strand, e.g. by A/T-base-pairing and G/C-base-pairing.

Epitope: Epitopes (also called 'antigen determinant') can be distinguished in T cell epitopes and B cell epitopes. T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form. B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Such epitopes of proteins or peptides may furthermore be selected from any of the herein mentioned variants of such proteins or peptides. In this context antigenic determinants can be conformational or discontinuous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

Fragment of a sequence: A fragment of a sequence may typically be a shorter portion of a full-length sequence of e.g. a nucleic acid molecule or an amino acid sequence. Accordingly, a fragment, typically, consists of a sequence that is identical to the corresponding stretch within the full-length sequence. A preferred fragment of a sequence in the context of the present invention, consists of a continuous stretch of entities, such as nucleotides or amino acids corresponding to a continuous stretch of entities in the molecule the fragment is derived from, which represents at least 20%, preferably at least 30%, more preferably at least 40%, more preferably at least 50%, even more preferably at least 60%, even more preferably at least 70%, and most preferably at least 80% of the total (i.e. full-length) molecule from which the fragment is derived.

G/C modified: A G/C-modified nucleic acid may typically be a nucleic acid, preferably an artificial nucleic acid molecule as defined herein, based on a modified wild-type sequence comprising a preferably increased number of guanosine and/or cytosine nucleotides as compared to the wild-type sequence. Such an increased number may be generated by substitution of codons containing adenosine or thymidine nucleotides by codons containing guanosine or cytosine nucleotides. If the enriched G/C content occurs in a coding region of DNA or RNA, it makes use of the degeneracy of the genetic code. Accordingly, the codon substitutions preferably do not alter the encoded amino acid residues, but exclusively increase the G/C content of the nucleic acid molecule.

Gene therapy: Gene therapy may typically be understood to mean a treatment of a patient's body or isolated elements of a patient's body, for example isolated tissues/cells, by nucleic acids encoding a peptide or protein. It typically may comprise at least one of the steps of a) administration of a nucleic acid, preferably an artificial nucleic acid molecule as defined herein, directly to the patient - by whatever administration route - or *in vitro* to isolated cells/tissues of the patient, which results in transfection of the patient's cells either *in vivo*/*ex vivo* or *in vitro*; b) transcription and/or translation of the introduced nucleic acid molecule; and optionally c) re-administration of isolated, transfected cells to the patient, if the nucleic acid has not been administered directly to the patient.

Genetic vaccination: Genetic vaccination may typically be understood to be vaccination by administration of a nucleic acid molecule encoding an antigen or an immunogen or fragments thereof. The nucleic acid molecule may be administered to a subject's body or to isolated cells of a subject. Upon transfection of certain cells of the body or upon transfection of the isolated cells, the antigen or immunogen may be expressed by those cells and subsequently presented to the immune system, eliciting an adaptive, i.e. antigen-specific immune response. Accordingly, genetic vaccination typically comprises at least one of the steps of a) administration of a nucleic acid, preferably an artificial nucleic acid molecule as defined herein, to a subject, preferably a patient, or to isolated cells of a subject, preferably a patient, which usually results in transfection of the subject's cells either *in vivo* or *in vitro*; b) transcription and/or translation of the introduced nucleic acid molecule; and optionally c) re-administration of isolated, transfected cells to the subject, preferably the patient, if the nucleic acid has not been administered directly to the patient.

Heterologous sequence: Two sequences are typically understood to be 'heterologous' if they are not derivable from the same gene. I.e., although heterologous sequences may be derivable from the same organism, they naturally (in nature) do not occur in the same nucleic acid molecule, such as in the same mRNA.

Humoral immunity/humoral immune response: Humoral immunity refers typically to antibody production and optionally to accessory processes accompanying antibody production. A humoral immune response may be typically characterized, e.g., by Th2 activation and cytokine production, germinal center formation and isotype switching, affinity maturation and memory cell generation. Humoral immunity also typically may refer to the effector functions of antibodies, which include pathogen and toxin neutralization, classical complement activation, and opsonin promotion of phagocytosis and pathogen elimination.

Immunogen: In the context of the present invention an immunogen may be typically understood to be a compound that is able to stimulate an immune response. Preferably, an immunogen is a peptide, polypeptide, or protein. In a particularly preferred embodiment, an immunogen in the sense of the present invention is the product of translation of a provided nucleic acid molecule, preferably an artificial nucleic acid molecule as defined herein. Typically, an immunogen elicits at least an adaptive immune response.

Immunostimulatory composition: In the context of the invention, an immunostimulatory composition may be typically understood to be a composition containing at least one component which is able to induce an immune response or from which a component which is able to induce an immune response is derivable. Such immune response may be preferably an innate immune response or a combination of an adaptive and an innate immune response. Preferably, an immunostimulatory composition in the context of the invention contains at least one artificial nucleic acid molecule, more preferably an RNA, for example an mRNA molecule. The immunostimulatory component, such as the mRNA may be complexed with a suitable carrier. Thus, the immunostimulatory composition may comprise an mRNA/carrier-complex. Furthermore, the immunostimulatory composition may comprise an adjuvant and/or a suitable vehicle for the immunostimulatory component, such as the mRNA.

Immune response: An immune response may typically be a specific reaction of the adaptive immune system to a particular antigen (so called specific or adaptive immune response) or an unspecific reaction of the innate immune system (so called unspecific or innate immune response), or a combination thereof.

Immune system: The immune system may protect organisms from infection. If a pathogen succeeds in passing a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts typically contains so called humoral and cellular components.

Immunostimulatory RNA: An immunostimulatory RNA (isRNA) in the context of the invention may typically be an RNA that is able to induce an innate immune response. It usually does not have an open reading frame and thus does not provide a peptide-antigen or immunogen but elicits an immune response e.g. by binding to a specific kind of Toll-like-receptor (TLR) or other suitable receptors. However, of course also mRNAs having an open reading frame and coding for a peptide/protein may induce an innate immune response and, thus, may be immunostimulatory RNAs.

Innate immune system: The innate immune system, also known as non-specific (or unspecific) immune system, typically comprises the cells and mechanisms that defend the host from infection by other organisms in a non-specific manner. This means that the cells of the innate system may recognize and respond to pathogens in a generic way, but unlike the adaptive immune system, it does not confer long-lasting or protective immunity to the host. The innate immune system may be, e.g., activated by ligands of Toll-like receptors (TLRs) or other auxiliary substances such as lipopolysaccharides, TNF-alpha, CD40 ligand, or cytokines, monokines, lymphokines, interleukins or chemokines, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like receptor, an immunostimulatory nucleic acid, an immunostimulatory RNA (isRNA), a CpG-DNA, an antibacterial agent, or an anti-viral agent. The pharmaceutical composition according to the present invention may comprise one or more such substances. Typically, a response of the innate immune system includes recruiting immune cells to sites of infection, through the production of chemical factors, including specialized chemical mediators, called cytokines; activation of the complement cascade; identification and removal of foreign substances present in organs, tissues, the blood and lymph, by specialized white blood cells; activation of the adaptive immune system; and/or acting as a physical and chemical barrier to infectious agents.

Cloning site: A cloning site is typically understood to be a segment of a nucleic acid molecule, which is suitable for insertion of a nucleic acid sequence, e.g., a nucleic acid sequence comprising an open reading frame. Insertion may be performed by any molecular biological method known to the one skilled in the art, e.g. by restriction and ligation. A cloning site typically comprises one or more restriction enzyme recognition sites (restriction sites). These one or more restrictions sites may be recognized by restriction enzymes which cleave the DNA at these sites. A cloning site which comprises more than one restriction site may also be termed a multiple cloning site (MCS) or a polylinker.

Nucleic acid molecule: A nucleic acid molecule is a molecule comprising, preferably consisting of nucleic acid components. The term nucleic acid molecule preferably refers to DNA or RNA molecules. It is preferably used synonymous with the term "polynucleotide". Preferably, a nucleic acid molecule is a polymer comprising or consisting of nucleotide monomers which are covalently linked to each other by phosphodiester-bonds of a sugar/phosphate-backbone. The term "nucleic acid molecule" also encompasses modified nucleic acid molecules, such as base-modified, sugar-modified or backbone-modified etc. DNA or RNA molecules.

Open reading frame: An open reading frame (ORF) in the context of the invention may typically be a sequence of several nucleotide triplets which may be translated into a peptide or protein. An open reading frame preferably contains a start codon, i.e. a combination of three subsequent nucleotides coding usually for the amino acid methionine (ATG or AUG), at its 5'-end and a subsequent region which usually exhibits a length which is a multiple of 3 nucleotides. An ORF is preferably terminated by a stop-codon (e.g., TAA, TAG, TGA). Typically, this is the only stop-codon of the open reading frame. Thus, an open reading frame in the context of the present invention is preferably a nucleotide sequence, consisting of a number of nucleotides that may be divided by three, which starts with a start codon (e.g. ATG or AUG) and which preferably terminates with a stop codon (e.g., TAA, TGA, or TAG or UAA, UAG, UGA, respectively). The open reading frame may be isolated or it may be incorporated in a longer nucleic acid sequence, for example in a vector or an mRNA. An open reading frame may also be termed 'protein coding region'.

Peptide: A peptide or polypeptide is typically a polymer of amino acid monomers, linked by peptide bonds. It typically contains less than 50 monomer units. Nevertheless, the term peptide is not a disclaimer for molecules having more than 50 monomer units. Long peptides are also called polypeptides, typically having between 50 and 600 monomeric units.

Pharmaceutically effective amount: A pharmaceutically effective amount in the context of the invention is typically understood to be an amount that is sufficient to induce a pharmaceutical effect, such as an immune response, altering a pathological level of an expressed peptide or protein, or substituting a lacking gene product, e.g., in case of a pathological situation.

Protein A protein typically comprises one or more peptides or polypeptides. A protein is typically folded into 3-dimensional form, which may be required for to protein to exert its biological function.

Poly(A) sequence: A poly(A) sequence, also called poly(A) tail or 3'-poly(A) tail, is typically understood to be a sequence of adenine nucleotides, e.g., of up to about 400 adenine nucleotides, e.g. from about 20 to about 400, preferably from about 50 to about 400, more preferably from about 50 to about 300, even more preferably from about 50 to about 250, most preferably from about 60 to about 250 adenine nucleotides. A poly(A) sequence is typically located at the 3'end of an mRNA. In the context of the present invention, a poly(A) sequence may be located within an mRNA or any other nucleic acid molecule, such as, e.g., in a vector, for example, in a vector serving as template for the generation of an RNA, preferably an mRNA, e.g., by transcription of the vector.

Polyadenylation: Polyadenylation is typically understood to be the addition of a poly(A) sequence to a nucleic acid molecule, such as an RNA molecule, e.g. to a premature mRNA. Polyadenylation may be induced by a so called polyadenylation signal. This signal is preferably located within a stretch of nucleotides at the 3'-end of a nucleic acid molecule, such as an RNA molecule, to be polyadenylated. A polyadenylation signal typically comprises a hexamer consisting of adenine and uracil/thymine nucleotides, preferably the hexamer sequence AAUAAA. Other sequences, preferably hexamer sequences, are also conceivable. Polyadenylation typically occurs during processing of a pre-mRNA (also called premature-mRNA). Typically, RNA maturation (from pre-mRNA to mature mRNA) comprises the step of polyadenylation.

Restriction site: A restriction site, also termed 'restriction enzyme recognition site', is a nucleotide sequence recognized by a restriction enzyme. A restriction site is typically a short, preferably palindromic nucleotide sequence, e.g. a sequence comprising 4 to 8 nucleotides. A restriction site is preferably specifically recognized by a restriction enzyme. The restriction enzyme typically cleaves a nucleotide sequence comprising a restriction site at this site. In a double-stranded nucleotide sequence, such as a double-stranded DNA sequence, the restriction enzyme typically cuts both strands of the nucleotide sequence.

RNA, mRNA: RNA is the usual abbreviation for ribonucleic-acid. It is a nucleic acid molecule, i.e. a polymer consisting of nucleotides. These nucleotides are usually adenosine-monophosphate, uridine-monophosphate, guanosine-monophosphate and cytidine-monophosphate monomers which are connected to each other along a so-called backbone. The backbone is formed by phosphodiester bonds between the sugar, i.e. ribose, of a first and a phosphate moiety of a second, adjacent monomer. The specific succession of the monomers is called the RNA-sequence. Usually RNA may be obtainable by transcription of a DNA-sequence, e.g., inside a cell. In eukaryotic cells, transcription is typically performed inside the nucleus or the mitochondria. *In vivo,* transcription of DNA usually results in the so-called premature RNA which has to be processed into so-called messenger-RNA, usually abbreviated as mRNA. Processing of the premature RNA, e.g. in eukaryotic organisms, comprises a variety of different posttranscriptional-modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino acid sequence of a particular peptide or protein. Typically, a mature mRNA comprises a 5'-cap, a 5'UTR, an open reading frame, a 3'UTR and a poly(A) sequence. Aside from messenger RNA, several non-coding types of RNA exist which may be involved in regulation of transcription and/or translation.

Sequence of a nucleic acid molecule: The sequence of a nucleic acid molecule is typically understood to be the particular and individual order, i.e. the succession of its nucleotides. The sequence of a protein or peptide is typically understood to be the order, i.e. the succession of its amino acids.

Sequence identity: Two or more sequences are identical if they exhibit the same length and order of nucleotides or amino acids. The percentage of identity typically describes the extent to which two sequences are identical, i.e. it typically describes the percentage of nucleotides that correspond in their sequence position with identical nucleotides of a reference-sequence. For determination of the degree of identity, the sequences to be compared are considered to exhibit the same length, i.e. the length of the longest sequence of the sequences to be compared. This means that a first sequence consisting of 8 nucleotides is 80% identical to a second sequence consisting of 10 nucleotides comprising the first sequence. In other words, in the context of the present invention, identity of sequences preferably relates to the percentage of nucleotides of a sequence which have the same position in two or more sequences having the same length. Gaps are usually regarded as non-identical positions, irrespective of their actual position in an alignment.

Stabilized nucleic acid molecule: A stabilized nucleic acid molecule is a nucleic acid molecule, preferably a DNA or RNA molecule that is modified such, that it is more stable to disintegration or degradation, e.g., by environmental factors or enzymatic digest, such as by an exo- or endonuclease degradation, than the nucleic acid molecule without the modification. Preferably, a stabilized nucleic acid molecule in the context of the present invention is stabilized in a cell, such as a prokaryotic or eukaryotic cell, preferably in a mammalian cell, such as a human cell. The stabilization effect may also be exerted outside of cells, e.g. in a buffer solution etc., for example, in a manufacturing process for a pharmaceutical composition comprising the stabilized nucleic acid molecule.

Transfection: The term 'transfection' refers to the introduction of nucleic acid molecules, such as DNA or RNA (e.g. mRNA) molecules, into cells, preferably into eukaryotic cells. In the context of the present invention, the term 'transfection' encompasses any method known to the skilled person for introducing nucleic acid molecules into cells, preferably into eukaryotic cells, such as into mammalian cells. Such methods encompass, for example, electroporation, lipofection, e.g. based on cationic lipids and/or liposomes, calcium phosphate precipitation, nanoparticle based transfection, virus based transfection, or transfection based on cationic polymers, such as DEAE-dextran or polyethylenimine etc. Preferably, the introduction is non-viral.

Vaccine: A vaccine is typically understood to be a prophylactic or therapeutic material providing at least one antigen, preferably an immunogen. The antigen or immunogen may be derived from any material that is suitable for vaccination. For example, the antigen or immunogen may be derived from a pathogen, such as from bacteria or virus particles etc., or from a tumor or cancerous tissue. The antigen or immunogen stimulates the body's adaptive immune system to provide an adaptive immune response.

Vector: The term 'vector' refers to a nucleic acid molecule, preferably to an artificial nucleic acid molecule. A vector in the context of the present invention is suitable for incorporating or harboring a desired nucleic acid sequence, such as a nucleic acid sequence comprising an open reading frame. Such vectors may be storage vectors, expression vectors, cloning vectors, transfer vectors etc. A storage vector is a vector which allows the convenient storage of a nucleic acid molecule, for example, of an mRNA molecule. Thus, the vector may comprise a sequence corresponding, e.g., to a desired mRNA sequence or a part thereof, such as a sequence corresponding to the open reading frame and the 3'UTR of an mRNA. An expression vector may be used for production of expression products such as RNA, e.g. mRNA, or peptides, polypeptides or proteins. For example, an expression vector may comprise sequences needed for transcription of a sequence stretch of the vector, such as a promoter sequence, e.g. an RNA promoter sequence. A cloning vector is typically a vector that contains a cloning site, which may be used to incorporate nucleic acid sequences into the vector. A cloning vector may be, e.g., a plasmid vector or a bacteriophage vector. A transfer vector may be a vector which is suitable for transferring nucleic acid molecules into cells or organisms, for example, viral vectors. A vector in the context of the present invention may be, e.g., an RNA vector or a DNA vector. Preferably, a vector is a DNA molecule. Preferably, a vector in the sense of the present application comprises a cloning site, a selection marker, such as an antibiotic resistance factor, and a sequence suitable for multiplication of the vector, such as an origin of replication. Preferably, a vector in the context of the present application is a plasmid vector.

Vehicle: A vehicle is typically understood to be a material that is suitable for storing, transporting, and/or administering a compound, such as a pharmaceutically active compound. For example, it may be a physiologically acceptable liquid which is suitable for storing, transporting, and/or administering a pharmaceutically active compound.

3'-untranslated region (3'UTR): A 3'UTR is typically the part of an mRNA which is located between the protein coding region (i.e. the open reading frame) and the poly(A) sequence of the mRNA. A 3'UTR of the mRNA is not translated into an amino acid sequence. The 3'UTR sequence is generally encoded by the gene which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'capping, splicing the pre-mature mRNA to excise optional introns and modifications of the 3'-end, such as polyadenylation of the 3'-end of the pre-mature mRNA and optional endo- or exonuclease cleavages etc. In the context of the present invention, a 3'UTR corresponds to the sequence of a mature mRNA which is located 3' to the stop codon of the protein coding region, preferably immediately 3' to the stop codon of the protein coding region, and which extends to the 5'-side of the poly(A) sequence, preferably to the nucleotide immediately 5' to the poly(A) sequence. The term "corresponds to" means that the 3'UTR sequence may be an RNA sequence, such as in the mRNA sequence used for defining the 3'UTR sequence, or a DNA sequence which corresponds to such RNA sequence. In the context of the present invention, the term "a 3'UTR of a gene", such as "a 3'UTR of an albumin gene", is the sequence which corresponds to the 3'UTR of the mature mRNA derived from this gene, i.e. the mRNA obtained by transcription of the gene and maturation of the pre-mature mRNA. The term "3'UTR of a gene" encompasses the DNA sequence and the RNA sequence of the 3'UTR.

5'-untranslated region (5'UTR): A 5'UTR is typically understood to be a particular section of messenger RNA (mRNA). It is located 5' of the open reading frame of the mRNA. Typically, the 5'UTR starts with the transcriptional start site and ends one nucleotide before the start codon of the open reading frame. The 5'UTR may comprise elements for controlling gene expression, also called regulatory elements. Such regulatory elements may be, for example, ribosomal binding sites or a 5'-Terminal Oligopyrimidine Tract. The 5'UTR may be posttranscriptionally modified, for example by addition of a 5'-cap. In the context of the present invention, a 5'UTR corresponds to the sequence of a mature mRNA which is located between the 5'cap and the start codon. Preferably, the 5'UTR corresponds to the sequence which extends from a nucleotide located 3' to the 5'-cap, preferably from the nucleotide located immediately 3' to the 5'-cap, to a nucleotide located 5' to the start codon of the protein coding region, preferably to the nucleotide located immediately 5' to the start codon of the protein coding region. The nucleotide located immediately 3' to the 5'cap of a mature mRNA typically corresponds to the transcriptional start site. The term "corresponds to" means that the 5'UTR sequence may be an RNA sequence, such as in the mRNA sequence used for defining the 5'UTR sequence, or a DNA sequence which corresponds to such RNA sequence. In the context of the present invention, the term "a 5'UTR of a gene", such as "a 5'UTR of a TOP gene", is the sequence which corresponds to the 5'UTR of the mature mRNA derived from this gene, i.e. the mRNA obtained by transcription of the gene and maturation of the pre-mature mRNA. The term "5'UTR of a gene" encompasses the DNA sequence and the RNA sequence of the 5'UTR.

5'Terminal Oligopyrimidine Tract (TOP): The 5'terminal oligopyrimidine tract (TOP) is typically a stretch of pyrimidine nucleotides located at the 5' terminal region of a nucleic acid molecule, such as the 5' terminal region of certain mRNA molecules or the 5' terminal region of a functional entity, e.g. the transcribed region, of certain genes. The sequence starts with a cytidine, which usually corresponds to the transcriptional start site, and is followed by a stretch of usually about 3 to 30 pyrimidine nucleotides, more often 3 to 15 pyrimidine nucleotides. For example, the TOP may comprise 3, 4, 5, 6, 7, 8, 9, 10, 11, 12, 13, 14, 15, 16, 17, 18, 19, 20, 21, 22, 23, 24, 25, 26, 27, 28, 29, 30 or even more nucleotides. The pyrimidine stretch and thus the 5' TOP ends one nucleotide 5' to the first purine nucleotide located downstream of the TOP. Messenger RNA that contains a 5'-terminal oligopyrimidine tract is often referred to as TOP mRNA. Accordingly, genes that provide such messenger RNAs are referred to as TOP genes. TOP sequences have, for example, been found in genes and mRNAs encoding peptide elongation factors and ribosomal proteins.

TOP motif: In the context of the present invention, a TOP motif is a nucleic acid sequence which corresponds to a 5'TOP as defined above. Thus, a TOP motif in the context of the present invention is preferably a stretch of pyrimidine nucleotides having a length of 3-30 nucleotides. Preferably, the TOP-motif consists of at least 3 pyrimidine nucleotides, preferably at least 4 pyrimidine nucleotides, preferably at least 5 pyrimidine nucleotides, more preferably at least 6 nucleotides, more preferably at least 7 nucleotides, most preferably at least 8 pyrimidine nucleotides, wherein the stretch of pyrimidine nucleotides preferably starts at its 5'end with a cytosine nucleotide. In TOP genes and TOP mRNAs, the TOP-motif preferably starts at its 5'end with the transcriptional start site and ends one nucleotide 5' to the first purin residue in said gene or mRNA. A TOP motif in the sense of the present invention is preferably located at the 5'end of a sequence which represents a 5'UTR or at the 5'end of a sequence which codes for a 5'UTR. Thus, preferably, a stretch of 3 or more pyrimidine nucleotides is called "TOP motif" in the sense of the present invention if this stretch is located at the 5'end of a respective sequence, such as the artificial nucleic acid molecule according to the present invention, the 5'UTR element of the artificial nucleic acid molecule according to the present invention, or the nucleic acid sequence which is derived from the 5'UTR of a TOP gene as described herein. In other words, a stretch of 3 or more pyrimidine nucleotides which is not located at the 5'-end of a 5'UTR or a 5'UTR element but anywhere within a 5'UTR or a 5'UTR element is preferably not referred to as "TOP motif".

TOP gene: TOP genes are typically characterised by the presence of a 5' terminal oligopyrimidine tract. Furthermore, most TOP genes are characterized by a growth-associated translational regulation. However, also TOP genes with a tissue specific translational regulation are known. As defined above, the 5'UTR of a TOP gene corresponds to the sequence of a 5'UTR of a mature mRNA derived from a TOP gene, which preferably extends from the nucleotide located 3' to the 5'cap to the nucleotide located 5' to the start codon. A 5'UTR of a TOP gene typically does not comprise any start codons, preferably no upstream AUGs (uAUGs) or upstream open reading frames (uORFs). Therein, upstream AUGs and upstream open reading frames are typically understood to be AUGs and open reading frames that occur 5' of the start codon (AUG) of the open reading frame that should be translated. The 5'UTRs of TOP genes are generally rather short. The lengths of 5'UTRs of TOP genes may vary between 20 nucleotides up to 500 nucleotides, and are typically less than about 200 nucleotides, preferably less than about 150 nucleotides, more preferably less than about 100 nucleotides. Exemplary 5'UTRs of TOP genes in the sense of the present invention are the nucleic acid sequences extending from the nucleotide at position 5 to the nucleotide located immediately 5' to the start codon (e.g. the ATG) in the sequences according to SEQ ID NOs. 1-1363, SEQ ID NO. 1395, SEQ ID NO. 1421 and SEQ ID NO. 1422.

In a first aspect, the present invention relates to an artificial nucleic acid molecule comprising An artificial nucleic acid molecule comprising:

   a. at least one 5'-untranslated region element (5'UTR element) which comprises or consists of

      a nucleic acid sequence, which consists of a continuous stretch of nucleotides corresponding to a continuous stretch of nucleotides in the full-length 5'UTR of a TOP gene, which represents at least 80% of the full-length 5'UTR of a TOP gene,
      or
      a nucleic acid sequence, which consists of a continuous stretch of nucleotides corresponding to a continuous stretch of nucleotides in a variant of the 5'UTR of a TOP gene, which represents at least 80% of the variant of the 5'UTR of a TOP gene, wherein the variant of the 5'UTR of a TOP gene is at least 80% identical to the naturally occuring 5'UTR, from which the variant is derived,

   wherein the at least one 5'UTR element does not comprise a functional 5'TOP motif;

   b. at least one open reading frame (ORF); and

   c. at least one 3'UTR element, which comprises or consists of

      a nucleic acid sequence which consists of a continuous stretch of nucleotides corresponding to a continuous stretch of nucleotides in the full-length 3'UTR of an albumin gene, which represents at least 80% of the full-length 3'UTR of an albumin gene,
      or
      a nucleic acid sequence which consists of a continuous stretch of nucleotides corresponding to a continuous stretch of nucleotides in a variant of a 3'UTR of an albumin gene, which represents at least 80% of the variant of the 3'UTR of an albumin gene, wherein the variant of the 3'UTR of an albumin gene is at least 80% identical to the naturally occuring 3'UTR the variant is derived from.

Such an artificial nucleic acid molecule may be DNA or RNA. In case the artificial nucleic acid molecule is DNA it may be used for providing RNA, preferably an mRNA with a corresponding sequence as is described further below. The inventive artificial nucleic acid molecule is particularly useful in gene therapy and genetic vaccination because it may provide increased and/or prolonged protein production of the protein encoded by the open reading frame. It is preferred, if the components (a) and (b) are heterologous, such that the inventive nucleic acid molecule does not occur naturally, but is an artificial chimeric recombinant nucleic acid molecule.

In this context, the term '5'UTR element' preferably refers to a nucleic acid sequence which represents a 5'UTR of an artificial nucleic acid sequence, such as an artificial mRNA, or which codes for a 5'UTR of an artificial nucleic acid molecule. Thus, preferably, a 5'UTR element may be the 5'UTR of an mRNA, preferably of an artificial mRNA, or it may be the transcription template for a 5'UTR of an mRNA. Thus, a 5'UTR element preferably is a nucleic acid sequence which corresponds to the 5'UTR of an mRNA, preferably to the 5'UTR of an artificial mRNA, such as an mRNA obtained by transcription of a genetically engineered vector construct. Preferably, a 5'UTR element in the sense of the present invention functions as a 5'UTR or codes for a nucleotide sequence that fulfils the function of a 5'UTR. The term '5'UTR element' may also refer to a fragment or part of a 5'UTR of an artificial nucleic acid sequence, such as an artificial mRNA, or which codes for a part or fragment of a 5'UTR of an artificial nucleic acid molecule. This means that the 5'UTR element in the sense of the present invention may be comprised in the 5'UTR of an artificial nucleic acid sequence, such as an artificial mRNA, or which codes for a 5'UTR of an artificial nucleic acid molecule.

According to the invention, the 5'UTR element comprises or consists of a nucleic acid sequence that is derived from the 5'UTR of a TOP gene or from a variant of the 5'UTR of a TOP gene as defined by claim 1.

The term 'a nucleic acid sequence which is derived from the 5'UTR of a TOP gene' preferably refers to a nucleic acid sequence which is based on the 5'UTR sequence of a TOP gene or on a fragment thereof. This term includes sequences corresponding to the entire 5'UTR sequence, i.e. the full length 5'UTR sequence of a TOP gene, and sequences corresponding to a fragment of the 5'UTR sequence of a TOP gene. Preferably, a fragment of a 5'UTR of a TOP gene consists of a continuous stretch of nucleotides corresponding to a continuous stretch of nucleotides in the full-length 5'UTR of a TOP gene, which represents at least 20%, preferably at least 30%, more preferably at least 40%, more preferably at least 50%, even more preferably at least 60%, even more preferably at least 70%, even more preferably at least 80%, and most preferably at least 90% of the full-length 5'UTR of a TOP gene. Such a fragment, in the sense of the present invention, is preferably a functional fragment as described herein. A particularly preferred fragment of a 5'UTR of a TOP gene is a 5'UTR of a TOP gene lacking the 5'TOP motif, which typically corresponds to a pyrimidine stretch of 3 to 30 pyrimidine nucleotides at the 5' terminus of the 5'UTR of a TOP gene. For the above preferred embodiment of the invention employing a 5'UTR of a TOP gene, the 5'UTR (comprised by the inventive nucleic acid molecule) starts with the first nucleotide following the most 3'-terminal nucleotide of the 5'TOP motif. In case the 5'TOP motif does not correspond to the 5'terminal part of the 5'UTR of the TOP gene, the 5' UTR (of the TOP gene) employed in the inventive nucleic acid may consist of the nucleotide sequence located upstream of the 5'terminus of the 5'TOP motif and/or of the nucleotide sequence located downstream of the 3'terminus of the 5'TOP motif. In an alternative embodiment, the 5' motif of a 5'UTR of a TOP gene may be rendered dysfunctional by e.g. introducing one or more purine nucleotides, which interupt the monotonic pyrimidine nucleotide stretch of the 5'TOP motif such that the modified (interrupted) 5'TOP motif sequence cannot exert its regulatory function any longer, in particular cannot exert its function as an element for translational control. Another way of rendering the 5' TOP motif dysfunctional is the deletion of one or more pyrimidine nucleotides of the 5'TOP motif sequence (either at the termini and/or within the 5'TOP motif).

In one embodiment, the 5'UTR of a TOP gene will not be derived from the 5'UTR of ribosomal proteins (rp) mRNA (in particular not from mammalian 5'UTR of rp mRNA, more specifically not from rpP2 (e.g. rat rpP2), rpL32, rpL30, rpL13a (e.g. mouse transplantation antigen P198), rpS20, rpS6, rpL12 or rpS16 mRNA or not from an rpS19 mRNA (e.g. from Xenopus). In anotther embodiment, the 5'UTR of a TOP gene is not derived from the 5'UTR of a EF1alpha or (hamster) EF2 mRNA. The 5'UTRs of these afore-mentioned rp mRNAs are specifically not used, if they are linked to reporter genes in the ORF of the inventive nucleic acid. If e.g. the 5'UTR of rpS16 mRNA is used for the inventive nucleic acid, that 5'UTR will either not contain the 5'TOP motif sequence (composed ot the oligonucleotide (CCTTTTCC or CCUUUUCC) or will contain a dysfunctional variant therof by e.g. interruption of the oligopyrimidine sequence by purine nucleotides or by deltion of one or more pyrimidine nucleotides of that 5'TOP motif. Accordingly, the dysfunctional mutants may e.g. contain one or more purine nucleotides within the 5'TOP motif sequence thereby lacking the translational control function exerted by the 5'TOP motif, e.g. by abolishing its interaction with other regulatory compounds, e.g. miRNA or interaction with granule-associated proteins TIA-1 and TIAR.

The term '5'UTR of a TOP gene' preferably refers to the 5'UTR of a naturally occurring TOP gene.

The terms 'variant of the 5'UTR of a TOP gene' and 'variant thereof' in the context of a 5'UTR of a TOP gene refers to a variant of the 5'UTR of a naturally occurring TOP gene, preferably to a variant of the 5'UTR of a vertebrate TOP gene, preferably to a variant of the 5'UTR of a mammalian TOP gene, more preferably to a variant of the 5'UTR of a human TOP gene. Such variant may be a modified 5'UTR of a TOP gene. For example, a variant 5'UTR may exhibit one or more nucleotide deletions, insertions, additions and/or substitutions compared to the naturally occurring 5'UTR from which the variant is derived. Preferably, a variant of a 5'UTR of a TOP gene is at least 40%, preferably at least 50%, more preferably at least 60%, more preferably at least 70%, even more preferably at least 80%, even more preferably at least 90%, most preferably at least 95% identical to the naturally occurring 5'UTR the variant is derived from. Preferably, the variant is a functional variant as described herein.

The term "a nucleic acid sequence that is derived from a variant of the 5'UTR of a TOP gene" preferably refers to a nucleic acid sequence which is based on a variant of a 5'UTR sequence of a TOP gene or on a fragment thereof. This term includes sequences corresponding to the entire variant 5'UTR sequence, i.e. the full length variant 5'UTR sequence of a TOP gene, and sequences corresponding to a fragment of the variant 5'UTR sequence of a TOP gene. Preferably, a fragment of a variant of the 5'UTR of a TOP gene consists of a continuous stretch of nucleotides corresponding to a continuous stretch of nucleotides in the full-length variant 5'UTR of a TOP gene, which represents at least 20%, preferably at least 30%, more preferably at least 40%, more preferably at least 50%, even more preferably at least 60%, even more preferably at least 70%, even more preferably at least 80%, and most preferably at least 90% of the full-length variant 5'UTR of a TOP gene. Such a fragment of a variant, in the sense of the present invention, is preferably a functional fragment as described herein.

Thus, the 5'UTR element of the artificial nucleic acid molecule may comprise or consist of a fragment of the 5'UTR of a TOP gene or of a fragment of a variant of the 5'UTR of a TOP gene or may comprise or consist of the entire 5'UTR of a TOP gene or may comprise or consist of a variant of the 5'UTR of a TOP gene as defined by claim 1.

The 5'UTR element is preferably suitable for increasing protein production from the artificial nucleic acid molecule.

Preferably, the at least one 5'UTR element is functionally linked to the ORF. This means preferably that the 5'UTR element is associated with the ORF such that it may exert a function, such as a protein production increasing function for the protein encoded by the ORF or a stabilizing function on the artificial nucleic acid molecule. Preferably, the 5'UTR element and the ORF are associated in 5'→3' direction. Thus, preferably, the artificial nucleic acid molecule comprises the structure 5'-5'UTR element-(optional)linker-ORF-3', wherein the linker may be present or absent. For example, the linker may be one or more nucleotides, such as a stretch of 1-50 or 1-20 nucleotides, e.g., comprising or consisting of one or more restriction enzyme recognition sites (restriction sites).

Preferably, the 5'UTR element and the at least one open reading frame are heterologous. The term 'heterologous' in this context means that the open reading frame and the 5'UTR element are not occurring naturally (in nature) in this combination. Preferably, the 5'UTR element is derived from a different gene than the open reading frame. For example, the ORF may be derived from a different gene than the 5'UTR element, e.g. encoding a different protein or the same protein but of a different species etc. For example, the ORF does not encode the protein which is encoded by the gene from which the 5'UTR element is derived.

In a preferred embodiment, the 5'UTR element, preferably the artificial nucleic acid molecule, does not comprise a complete TOP-motif or 5'TOP sequence. Thus, preferably, the 5'UTR element, preferably the artificial nucleic acid molecule, does not comprise the complete TOP-motif of the TOP gene from which the nucleic acid sequence of the 5'UTR element is derived. For example, the 5'UTR element or the artificial nucleic acid molecule according to the present invention may comprise 1, 2, 3, 4, 5, 6, 7, 8, 9, 10 or more pyrimidine residues of the TOP-motif or 5'TOP, preferably 1, 2, 3, 4, 5, 6, 7, 8, 9, 10 or more pyrimidine residues of the TOP-motif located at the 3'side of the TOP-motif or 5'TOP. For example, the 5'UTR element may comprise or consist of a nucleic acid sequence which starts at its 5'end with a pyrimidine residue that corresponds to residue 2, 3, 4, 5, 6, 7, 8, 9, 10 etc. of the TOP-motif or 5'TOP of the TOP gene from which the nucleic acid sequence of the 5'UTR element is derived.

It is particularly preferred that the 5'UTR element, preferably the artificial nucleic acid molecule according to the present invention, does not comprise a TOP-motif or a 5'TOP sequence. For example, the nucleic acid sequence of the 5'UTR element, which is derived from a 5'UTR of a TOP gene starts at its 5'-end with a nucleotide located at position 1, 2, 3, 4, 5, 6, 7, 8, 9 or 10 downstream of the 5'terminal oligopyrimidine tract (TOP) of the 5'UTR of a TOP gene. Position 1 downstream of the 5'terminal oligopyrimidine tract (TOP) is the first purine based nucleotide 3' of the TOP-motif or the 5'TOP. Accordingly, position 1 downstream of the 5'terminal oligopyrimidine tract is the first nucleotide following the 3'-end of the 5'terminal oligopyrimidine tract in 5'-3'-direction. Likewise, position 2 downstream of the 5'TOP is the second nucleotide following the end of the 5'terminal oligopyrimidine tract, position 3 the third nucleotide and so on.

Therefore, the 5'UTR element preferably starts 5, 10, 15, 20, 25, 30, 40 or 50 nucleotides downstream of the transcriptional start site of the 5'UTR of a TOP gene.

In some embodiments, the nucleic acid sequence of the 5'UTR element, which is derived from a 5'UTR of a TOP gene terminates at its 3'-end with a nucleotide located at position 1, 2, 3, 4, 5, 6, 7, 8, 9 or 10 upstream of the start codon (e.g. A(U/T)G) of the gene or mRNA it is derived from. Thus, the 5'UTR element does not comprise any part of the protein coding region. Thus, preferably, the only protein coding part of the inventive artificial nucleic acid molecule is provided by the open reading frame. However, the open reading frame is preferably derived - as said above - from a gene that is different to the gene the 5'UTR element is derived from.

It is particularly preferred that the 5'UTR element does not comprise a start codon, such as the nucleotide sequence A(U/T)G. Thus, preferably, the artificial nucleic acid molecule will not comprise any upstream AUGs (or upstream ATGs in case it is a DNA molecule). In other words, in some embodiments, it may be preferred that the AUG or ATG, respectively, of the open reading frame is the only start codon of the artificial nucleic acid molecule.

Additionally, it is preferred that the 5'UTR element does not comprise an open reading frame. Thus, preferably, the artificial nucleic acid molecule will not comprise any upstream open reading frames.

The nucleic acid sequence which is derived from the 5'UTR of a TOP gene is derived from a eukaryotic TOP gene, preferably a plant or animal TOP gene, more preferably a chordate TOP gene, even more preferably a vertebrate TOP gene, most preferably a mammalian TOP gene, such as a human or mouse TOP gene.

Preferably, the artificial nucleic acid molecule according to the present invention comprises a 5'UTR element,

which comprises or consists of a nucleic acid sequence, which is derived from the 5'UTR of a TOP gene or which is derived from a variant of the 5'UTR of a TOP gene, wherein the TOP gene is a plant or animal TOP gene, more preferably a chordate TOP gene, even more preferably a vertebrate TOP gene, most preferably a mammalian TOP gene, such as a human or mouse TOP gene and which optionally does not comprise the nucleotide sequence A(U/T)G and optionally does not comprise an open reading frame; at least one open reading frame (ORF); wherein optionally the 5'UTR element does not comprise a TOP motif and wherein optionally the 5'UTR element starts at its 5'-end with a nucleotide located at position 1, 2, 3, 4, 5, 6, 7, 8, 9 or 10 downstream of the 5'terminal oligopyrimidine tract (TOP) of the 5'UTR of a TOP gene and wherein further optionally the 5'UTR element which is derived from a 5'UTR of a TOP gene terminates at its 3'-end with a nucleotide located at position 1, 2, 3, 4, 5, 6, 7, 8, 9 or 10 upstream of the start codon (A(U/T)G) of the gene or mRNA it is derived from.

For example, the 5'UTR element comprises or consists of a nucleic acid sequence as defined by the claims, which is derived from a nucleic acid sequence selected from the group consisting of SEQ ID NOs. 1-1363, SEQ ID NO. 1395, SEQ ID NO. 1421 and SEQ ID NO. 1422, from the homologs of SEQ ID NOs. 1-1363, SEQ ID NO. 1395, SEQ ID NO. 1421 and SEQ ID NO. 1422, from a variant thereof, or a corresponding RNA sequence. The term "homologs of SEQ ID NOs. 1-1363, SEQ ID NO. 1395, SEQ ID NO. 1421 and SEQ ID NO. 1422" refers to sequences of other species, e.g. other species than Homo sapiens (human) or Mus musculus (mouse), which are homologous to the sequences according to SEQ ID NOs. 1-1363, SEQ ID NO. 1395, SEQ ID NO. 1421 or SEQ ID NO. 1422. For example, SEQ ID NO. 1 relates to a sequence comprising the 5'UTR of Homo sapiens alpha 2 macroglobulin (A2M). A homolog of SEQ ID NO. 1 in the context of the present invention is any such sequence derived from an alpha 2 macroglobulin (A2M) gene or mRNA of another species than Homo sapiens, such as any vertebrate, preferably any mammalian alpha 2 macroglobulin (A2M) gene other than the human alpha 2 macroglobulin (A2M) gene, such as a mouse, rat, rabbit, monkey etc. alpha 2 macroglobulin (A2M) gene.

In a preferred embodiment, the 5'UTR element comprises or consists of a nucleic acid sequence as defined by the claims, which is derived from a nucleic acid sequence extending from nucleotide position 5 (i.e. the nucleotide that is located at position 5 in the sequence) to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1395, SEQ ID NO. 1421 or SEQ ID NO. 1422, from the homologs of SEQ ID NOs. 1-1363, SEQ ID NO. 1395, SEQ ID NO. 1421 or SEQ ID NO. 1422, from a variant thereof, or a corresponding RNA sequence. It is particularly preferred that the 5' UTR element is derived from a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1395, SEQ ID NO. 1421 or SEQ ID NO. 1422, from the homologs of SEQ ID NOs. 1-1363, SEQ ID NO. 1395, SEQ ID NO. 1421 or SEQ ID NO. 1422, from a variant thereof, or a corresponding RNA sequence as defined by the claims.

In a preferred embodiment, the 5'UTR element comprises or consists of a nucleic acid sequence as defined by the claims, which has an identity of at least about 80%, more preferably of at least about 90%, even more preferably of at least about 95%, even more preferably of at least about 99% to a nucleic acid sequence extending from nucleotide position 5 to the nucleotide position immediately 5' to the start codon (located at the 3' end of the sequences), e.g. the nucleotide position immediately 5' to the ATG sequence of a nucleic acid sequence selected from SEQ ID NOs. 1-1363, SEQ ID NO. 1395, SEQ ID NO. 1421 or SEQ ID NO. 1422,or a corresponding RNA sequence, or wherein the at least one 5'UTR element comprises or consists of a fragment of a nucleic acid sequence which has an identity of at least about 80%, more preferably of at least about 90%, even more preferably of at least about 95%, even more preferably of at least about 99% to a nucleic acid sequence extending from nucleotide position 5 to the nucleotide position immediately 5' to the start codon (located at the 3' end of the sequences), e.g. the nucleotide position immediately 5' to the ATG sequence of a nucleic acid sequence, selected from SEQ ID NOs. 1-1363, SEQ ID NO. 1395, SEQ ID NO. 1421 or SEQ ID NO. 1422, or a corresponding RNA sequence, wherein, preferably, the fragment is as described above, i.e. being a continuous stretch of nucleotides representing at least 80%of the full-length 5'UTR, the fragment is derived from.

Preferably, the 5'UTR element comprises or consists of a nucleic acid sequence as defined by the claims, which has an identity of at least about 70%, more preferably of at least about 80%, more preferably of at least about 90%, even more preferably of at least about 95%, even more preferably of at least about 99% to a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1395, SEQ ID NO. 1421 or SEQ ID NO. 1422, or a corresponding RNA sequence, or wherein the at least one 5'UTR element comprises or consists of a fragment of a nucleic acid sequence which has an identity of at least about 80%, more preferably of at least about 90%, even more preferably of at least about 95%, even more preferably of at least about 99% to a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1395, SEQ ID NO. 1421 or SEQ ID NO. 1422, or a corresponding RNA sequence, wherein, preferably, the fragment is as described above, i.e. being a continuous stretch of nucleotides representing at least 80% of the full-length 5'UTR, the fragment is derived from.

Preferably, the above defined fragments and variants (e.g. exhibiting at least 80% identity) of the sequences according to SEQ ID NOs. 1-1363, SEQ ID NO. 1395, SEQ ID NO. 1421 or SEQ ID NO. 1422, are functional fragments and variants as described herein.

Furthermore, the artificial nucleic acid molecule according to the present invention may comprise more than one 5'UTR elements as described above. For example, the artificial nucleic acid molecule according to the present invention may comprise one, two, three, four or more 5'UTR elements, wherein the individual 5'UTR elements may be the same or they may be different. For example, the artificial nucleic acid molecule according to the present invention may comprise two essentially identical 5'UTR elements as described above, e.g. two 5'UTR elements comprising or consisting of a nucleic acid sequence, which is derived from a nucleic acid sequence selected from the group consisting of SEQ ID NOs. 1-1363, SEQ ID NO. 1395, SEQ ID NO. 1421 and SEQ ID NO. 1422, from the homologs of SEQ ID NOs. 1-1363, SEQ ID NO. 1395, SEQ ID NO. 1421 and SEQ ID NO. 1422, from a variant thereof, or a corresponding RNA sequence or from functional variants thereof, functional fragments thereof, or functional variant fragments thereof as described above.

In a particularly preferred embodiment, the 5'UTR element comprises or consists of a nucleic acid sequence as defined by the claims, which is derived from a 5'UTR of a TOP gene encoding a ribosomal protein or from a variant of a 5'UTR of a TOP gene encoding a ribosomal protein. Particularly preferred 5'UTR elements comprise or consist of a nucleic acid sequence which are derived from a 5' UTR of a TOP gene coding for a ribosomal protein selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, UBA52. Particularly preferred are nucleic acid sequences which are derived from a 5' UTR of TOP genes vertebrate coding for ribosomal proteins, such as mammalian ribosomal proteins e.g. human or mouse ribosomal proteins.

For example, the 5'UTR element comprises or consists of a nucleic acid sequence as defined by the claims, which is derived from a 5'UTR of a nucleic acid sequence according to any of SEQ ID NOs: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360; a corresponding RNA sequence, a homolog thereof, or a variant thereof as described herein, preferably lacking the 5'TOP motif. As described above, the sequence extending from position 5 to the nucleotide immediately 5' to the ATG (which is located at the 3'end of the sequences) corresponds to the 5'UTR of said sequences.

Preferably, the 5'UTR element comprises or consists of a nucleic acid sequence as defined by the claims, which has an identity of at least about 80%, more preferably of at least about 90%, even more preferably of at least about 95%, even more preferably of at least about 99% to the 5'UTR of a nucleic acid sequence according to any of SEQ ID NOs.: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360; or a corresponding RNA sequence, preferably lacking the 5'TOP motif, or wherein the at least one 5'UTR element comprises or consists of a fragment of a nucleic acid sequence, which has an identity of at least about 80%, more preferably of at least about 90%, even more preferably of at least about 95%, even more preferably of at least about 99% to the 5'UTR of a nucleic acid sequence according to SEQ ID NOs: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360; or a corresponding RNA sequence, wherein, preferably, the fragment is as described above, i.e. being a continuous stretch of nucleotides representing at least 80% etc. of the full-length 5'UTR, preferably lacking the 5'TOP motif. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

Preferably, the 5'UTR element comprises or consists of a nucleic acid sequence as defined by the claims, which is derived from a 5'UTR of a TOP gene encoding a ribosomal Large protein (RPL) or from a variant of a 5'UTR of a TOP gene encoding a ribosomal Large protein (RPL). For example, the 5'UTR element comprises or consists of a nucleic acid sequence, which is derived from a 5'UTR of a nucleic acid sequence according to any of SEQ ID NOs: 67, 259, 1284-1318, 1344, 1346, 1348-1354, 1357, 1358, 1421 and 1422, a corresponding RNA sequence, a homolog thereof, or a variant thereof as described herein, preferably lacking the 5'TOP motif.

Preferably, the 5'UTR element comprises or consists of a nucleic acid sequence as defined by the claims, which has an identity of at least about 80%, more preferably of at least about 90%, even more preferably of at least about 95%, even more preferably of at least about 99% to the 5'UTR of a nucleic acid sequence according to any of SEQ ID NOs. 67, 259, 1284-1318, 1344, 1346, 1348-1354, 1357, 1358, 1421 and 1422 or a corresponding RNA sequence, preferably lacking the 5'TOP motif, or wherein the at least one 5'UTR element comprises or consists of a fragment of a nucleic acid sequence which has an identity of at least about 80%, more preferably of at least about 90%, even more preferably of at least about 95%, even more preferably of at least about 99% to the 5'UTR of a nucleic acid sequence according to SEQ ID NOs: 67, 259, 1284-1318, 1344, 1346, 1348-1354, 1357, 1358, 1421 and 1422 or a corresponding RNA sequence, wherein, preferably, the fragment is as described above, i.e. being a continuous stretch of nucleotides representing at least 80% etc. of the full-length 5'UTR, preferably lacking the 5'TOP motif. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

In a particularly preferred embodiment, the 5'UTR element comprises or consists of a nucleic acid sequence as defined by the claims, which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene (HSD17B4), an androgen-induced 1 gene (AIG1), cytochrome c oxidase subunit VIc gene (COX6C), or a N-acylsphingosine amidohydrolase (acid ceramidase) 1 gene (ASAH1) or from a variant thereof, preferably from a vertebrate ribosomal protein Large 32 gene (RPL32), a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit 1, cardiac muscle (ATP5A1) gene, a vertebrate hydroxysteroid (17-beta) dehydrogenase 4 gene (HSD17B4), a vertebrate androgen-induced 1 gene (AIG1), a vertebrate cytochrome c oxidase subunit VIc gene (COX6C), or a vertebrate N-acylsphingosine amidohydrolase (acid ceramidase) 1 gene (ASAH1) or from a variant thereof, more preferably from a mammalian ribosomal protein Large 32 gene (RPL32), a ribosomal protein Large 35 gene (RPL35), a ribosomal protein Large 21 gene (RPL21), a mammalian ATP synthase, H+ transporting, mitochondrial F1 complex, alpha subunit 1, cardiac muscle (ATP5A1) gene, a mammalian hydroxysteroid (17-beta) dehydrogenase 4 gene (HSD17B4), a mammalian androgen-induced 1 gene (AIG1), a mammalian cytochrome c oxidase subunit VIc gene (COX6C), or a mammalian N-acylsphingosine amidohydrolase (acid ceramidase) 1 gene (ASAH1) or from a variant thereof, most preferably from a human ribosomal protein Large 32 gene (RPL32), a human ribosomal protein Large 35 gene (RPL35), a human ribosomal protein Large 21 gene (RPL21), a human ATP synthase, H+ transporting, mitochondrial F1 complex, alpha subunit 1, cardiac muscle (ATP5A1) gene, a human hydroxysteroid (17-beta) dehydrogenase 4 gene (HSD17B4), a human androgen-induced 1 gene (AIG1), a human cytochrome c oxidase subunit VIc gene (COX6C), or a human N-acylsphingosine amidohydrolase (acid ceramidase) 1 gene (ASAH1) or from a variant thereof, wherein preferably the 5'UTR element does not comprise the 5'TOP of said gene.

Accordingly, in a particularly preferred embodiment, the 5'UTR element comprises or consists of a nucleic acid sequence as defined by the claims, which has an identity of at least about 80%, more preferably of at least about 90%, even more preferably of at least about 95%, even more preferably of at least about 99% to the nucleic acid sequence according to SEQ ID No. 1368, or SEQ ID NOs 1412-1420, or a corresponding RNA sequence, or wherein the at least one 5'UTR element comprises or consists of a fragment of a nucleic acid sequence, which has an identity of at least about 40%, preferably of at least about 80%, more preferably of at least about 90%, even more preferably of at least about 95%, even more preferably of at least about 99% to the nucleic acid sequence according to SEQ ID No. 1368, or SEQ ID NOs 1412-1420, wherein, preferably, the fragment is as described above, i.e. being a continuous stretch of nucleotides representing at least 80% etc. of the full-length 5'UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

Preferably, the at least one 5'UTR element exhibits a length of at least about 20 nucleotides or more, preferably of at least about 30 nucleotides or more, more preferably of at least about 40 nucleotides or more. However, it may be preferred if the 5'UTR element of the artificial nucleic acid molecule is rather short. Accordingly, it may have a length of less than about 200, preferably less than 150, more preferably less than 100 nucleotides. For example, the 5'UTR may have a length of less than about 25, 30, 35, 40, 45, 50, 55, 60, 65, 70, 80, 85, 90, 95, 100, 105, 110, 115, 120, 125, 130, 135, 140, 145, 150, 155, 160, 165, 170, 175, 180, 185, 190, 195, 200 nucleotides. Preferably, the 5'UTR element may have a length of about 20-25, 26-30, 31-35, 36-40, 41-45, 46-50, 51-55, 56-60, 61-65, 66-70, 71-80, 81-85, 86-90, 91-95, 96-100, 101-105, 106-110, 111-115, 116-120, 121-125, 126-130, 131-135, 136-140, 141-145, 146-150, 151-155, 156-160, 161-165, 166-170, 171-175, 176-180, 181-185, 186-190, 191-195, 196-200 or more nucleotides. For example, the 5'UTR element may have a length of about 20, 26, 31, 36, 41, 46, 51, 56, 61, 66, 71, 81, 86, 91, 96, 101, 106, 111, 116, 121, 126, 131, 136, 141, 146, 151, 156, 161, 166, 171, 176, 181, 186, 191 or 196 nucleotides. Preferably, the 5'UTR element may have a length from about 20, 30, 40 or more to less than about 200 nucleotides, more preferably from about 20, 30, 40 or more to less than about 150 nucleotides, most preferably from about 20, 30, 40 or more to less than about 100 nucleotides.

Preferred 5'UTR elements are derived from a 5' UTR of a TOP gene selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB or from a variant thereof.

In some embodiments, the artificial nucleic acid molecule comprises a 5'UTR element which comprises or consists of a nucleic acid sequence as defined by the claims, which is derived from the 5'UTR of a vertebrate TOP gene, such as a mammalian, e.g. a human TOP gene, selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB, or from a variant thereof, wherein preferably the 5'UTR element does not comprise a TOP-motif or the 5'TOP of said genes, and wherein optionally the 5'UTR element starts at its 5'-end with a nucleotide located at position 1, 2, 3, 4, 5, 6, 7, 8, 9 or 10 downstream of the 5'terminal oligopyrimidine tract (TOP) and wherein further optionally the 5'UTR element which is derived from a 5'UTR of a TOP gene terminates at its 3'-end with a nucleotide located at position 1, 2, 3, 4, 5, 6, 7, 8, 9 or 10 upstream of the start codon (A(U/T)G) of the gene it is derived from.

The artificial nucleic acid molecule according to the present invention further comprises at least one 3'UTR element, which comprises or consists of a nucleic acid sequence derived from the 3'UTR of an albumin gene, preferably a vertebrate gene, more preferably a mammalian gene, most preferably a human gene, or from a variant of the 3'UTR of an albumin gene, preferably a vertebrate gene, more preferably a mammalian gene, most preferably a human gene as defined by the claims.

The term '3'UTR element' refers to a nucleic acid sequence, which comprises or consists of a nucleic acid sequence that is derived from a 3'UTR or from a variant of a 3'UTR as claimed. A 3'UTR element in the sense of the present invention may represent the 3'UTR of an mRNA, e.g., in the event that the artificial nucleic acid molecule is an mRNA, or it may represent a sequence in a nucleic acid construct, such as a vector construct, that when transcribed represents the 3'UTR of the transcription product, such as the mRNA. Thus, in the sense of the present invention, preferably, a 3'UTR element may be the 3'UTR of an mRNA, preferably of an artificial mRNA, or it may be the transcription template for a 3'UTR of an mRNA. Thus, a 3'UTR element preferably is a nucleic acid sequence, which corresponds to the 3'UTR of an mRNA, preferably to the 3'UTR of an artificial mRNA, such as an mRNA obtained by transcription of a genetically engineered vector construct. Preferably, the 3'UTR element fulfils the function of a 3'UTR or encodes a sequence which fulfils the function of a 3'UTR. The term '3UTR element' furthermore refers to a fragment or part of a 3'UTR of an artificial nucleic acid sequence, such as an artificial mRNA, or which codes for a part or fragment of a 3'UTR of an artificial nucleic acid molecule. This means that the 3'UTR element in the sense of the present invention may be comprised in the 3'UTR of an artificial nucleic acid sequence, such as an artificial mRNA, or which codes for a 3'UTR of an artificial nucleic acid molecule.

Preferably, the 3'UTR element and the at least one open reading frame are heterologous. For example, the artificial nucleic acid molecule may consist of at least two sequence parts that are derivable from two different genes, the 5'UTR element, which is derivable from a TOP gene and the open reading frame and the 3'UTR, which may be derivable from the gene encoding the desired protein product. More preferably, the artificial nucleic molecule consist of three sequence parts that are derivable from three different genes: the 5'UTR element, which is derivable from a TOP gene, the open reading frame, which is derivable from the gene encoding the desired gene product, and the 3'UTR element, which is derivable from an albumin gene that relates to an mRNA with an enhanced half-life, i.e. a 3'UTR element as claimed.

Preferably, the at least one 3'UTR element is functionally linked to the ORF. This means preferably that the 3'UTR element is associated with the ORF such that it may exert a function, such as a stabilizing function on the expression of the ORF or a stabilizing function on the artificial nucleic acid molecule. Preferably, the ORF and the 3'UTR element are associated in 5'→3' direction. Thus, preferably, the artificial nucleic acid molecule comprises the structure 5'-ORF-(optional)linker-3'UTR element-3', wherein the linker may be present or absent. For example, the linker may be one or more nucleotides, such as a stretch of 1-50 or 1-20 nucleotides, e.g., comprising or consisting of one or more restriction enzyme recognition sites (restriction sites).

Preferably, the at least one 5'UTR element and the at least one 3'UTR element are functionally linked to the ORF. This means preferably that the 5'UTR element and the 3'UTR element are associated with the ORF such that they may exert a function, preferably in an additive, more preferably in a synergistic manner, such as a stabilizing function on the expression of the ORF, a protein production increasing function for the protein encoded by the ORF, or a stabilizing function on the artificial nucleic acid molecule. Preferably, the 5'UTR element, the ORF, and the 3'UTR element are associated in 5'→3' direction. Thus, preferably, the artificial nucleic acid molecule comprises the structure 5'-5'UTR element-(optional)linker-ORF-(optional)linker-3'UTR element-3', wherein the linker may be present or absent. For example, the linker may be one or more nucleotides, such as a stretch of 1-50 or 1-20 nucleotides, e.g., comprising or consisting of one or more restriction enzyme recognition sites (restriction sites).

In a particularly preferred embodiment, the 5'UTR element and the 3'UTR element are heterologous, e.g. preferably the 5'UTR and the 3'UTR are derived from different genes of the same or of different species.The 3'UTR is not derived from the TOP gene the 5'UTR is derived from.

The 3'UTR element is advantageously chosen such that it exerts at least an additive, preferably a synergistic function with the 5'UTR element on the protein production from the ORF of the artificial nucleic acid molecule. Preferably, the protein production is increased in at least an additive, preferably a synergistic way by the 3'UTR element and the 5'UTR element. Thus, the protein amount of the protein encoded by the ORF, such as a reporter protein, e.g. luciferase, at a certain time point after initiation of expression of the ORF, e.g. after transfection of a test cell or cell line, is preferably at least the same, preferably higher than what would be expected if the protein production increasing effects of the 3'UTR element and the 5'UTR element were purely additive. The additive, preferably the synergistic effect may, for example, be determined by the following assay. Four artificial nucleic acid molecules, e.g. mRNAs, comprising an ORF encoding, e.g. a reporter protein such as luciferase, are generated, i.e. (i) lacking UTR elements (E0), (ii) containing a 5'UTR element derived from a 5'UTR of a TOP gene or of a variant thereof (E1), (iii) containing a test 3'UTR element (E2), and (iv) containing both the 5'UTR element and the test 3'UTR element (E1E2). Expression of the ORF contained in the artificial nucleic acid molecules is initiated, for example, by transfecting a test cell line, such as a mammalian cell line, e.g. HELA cells, or primary cells, e.g. HDF cells. Samples are taken at specific time points after initiation of expression, for example, after 6 hours, 24 hours, 48 hours, and 72 hours and the amount of protein produced by expression of the ORF contained in the artificial nucleic acid molecules is measured, for example, by an ELISA assay or a luciferase test, depending on the type of protein encoded by the ORF. The predicted amount of protein at a certain time point after initiation of expression obtained by construct E1E2 if the effects of the 3'UTR element and the 5'UTR element were purely additive (PPA) may be calculated as follows:

$$PPA_x = (E1_x - E0_x) + (E2_x - E0_x) + E0_x,$$

E0 is the amount of protein obtained for the construct E0 (lacking UTRs), E1 is the amount of protein obtained for the construct E1, E2 is the protein amount obtained for the construct E2, and x is the time point after initiation of expression. The effect on increasing protein production is additive if $E1E2_x = PPA_x$ and synergistic in the sense of the present invention if $E1E2_x > PPA_x$, wherein $E1E2_x$ is the amount of protein obtained from construct E1E2 at time point x. Preferably, E1E2 is at least 1.0, preferably at least 1.1, more preferably at least 1.3, more preferably at least 1.5, even more preferably at least 1.75 times PPA at a given time point post initiation of expression, such as 24 hours, 48 hours or 72 hours post initiation of expression.

Thus, in a preferred embodiment, the present invention provides an artificial nucleic acid molecule as claimed, wherein the 3'UTR element and the 5'UTR element act at least additively, preferably synergistically to increase protein production from the ORF, preferably wherein E1E2 ≥ PPA, preferably E1E2 is at least 1.0 times PPA, preferably E1E2 is at least 1.1 times PPA, more preferably E1E2 is at least 1.3 times PPA, even more preferably E1E2 is at least 1.5 times PPA at a given time point post initiation of expression of the ORF, for example 24 hours, preferably 48 hours post initiation of expression, such as post transfection, wherein E1E2 and PPA are as described above.

Furthermore, it is preferred that the 3'UTR element and the 5'UTR element have at least an additive, preferably a synergistic effect on the total protein production from the artificial nucleic acid molecule in a certain time span, such as within 24 hours, 48 hours, or 72 hours post initiation of expression. The additive or the synergistic effect may be determined as described above, with the difference that the area under the curve (AUC) for the amount of protein over time predicted for E1E2 if the effects were purely additive is compared to the actual AUC measured for E1E2.

As disclosed herein, the 3'UTR element comprises or consists of a nucleic acid sequence, which is derived from the 3'UTR of a stable mRNA or from a variant of the 3'UTR of a stable mRNA. Thus, the 3'UTR element advantageously comprises or consists of a sequence, which is derived from a gene providing a stable mRNA or from a variant of a 3'UTR of a gene providing a stable mRNA. The term "stable mRNA", typically refers to mRNAs, which exhibit a longer half-life in mammalian cells than the average half-life of mRNA molecules in mammalian cells. Preferably, a stable mRNA in the sense of the present application refers to an mRNA which exhibits a half-life of more than 5 hours, preferably more than 8 hours, in a mammalian cell, such as in a mammalian cell line, e.g. in HELA cells, or in primary cells, e.g. in HDF cells, preferably determined by using a transcription inhibitor such as actinomycin D.

For example, the half-life of an mRNA in mammalian cells, such as HELA or HDF cells, may be determined by culturing the cells in presence of a transcription inhibitor, e.g. actinomycin D, 5,6-dichloro-1-$\beta$-D-ribofuranosylbenzimi-dazole (DRB), or $\alpha$-amanitin, harvesting the cells at different time points after inhibition of transcription, and determining the amount of the mRNA present in the cell samples by methods well known to the person skilled in the art, e.g. by quantitative RT-PCR. The half-life of a particular mRNA may be calculated based on the amounts of the particular mRNA measured at the different time points post inhibition of transcription. Alternatively, pulse-chase methods, e.g. using radioactively labelled nucleotides, or constructs comprising inducible promoters may be used for determining the half-life of an mRNA in mammalian cells.

It is also disclosed herein that the enhanced stability of a stable mRNA in the sense of the present invention is affected by its 3'UTR. Thus, the 3'UTR element advantageously comprises or consists of a nucleic acid sequence, which is derived from the 3'UTR of a stable mRNA, which exhibits a half-life of more than 5 hours, preferably more than 8 hours, in a mammalian cell, such as in a mammalian cell line, e.g. in HeLa cells, or in mammalian primary cells, e.g. in HDF cells, preferably determined by using a transcription inhibitor such as actinomycin D, wherein the enhanced stability of said stable mRNA is effected by its 3'UTR. The ability of a 3'UTR for enhancing stability may be tested as described herein, e.g. by using a reporter open reading frame such as a luciferase encoding open reading frame. Alternatively, an artificial construct encoding the test stable mRNA may be generated, wherein the 3'UTR of the stable mRNA is replaced with a reference 3'UTR, such as a 3'UTR of a short lived mRNA, e.g. a Myc 3'UTR. The stability of the wild type stable mRNA and the 3'UTR modified mRNA may be determined as described above. In the event the 3'UTR modified mRNA exhibits a shorter half-life than the wild type stable mRNA, it may be concluded that a stability enhancing effect is exerted by the 3'UTR of the stable mRNA.

It is also disclosed herein that the 3'UTR element comprises or consists of a nucleic acid sequence, which is derived from a 3'UTR of a gene selected from the group consisting of, an $\alpha$-globin gene, a $\beta$-globin gene, a tyrosine hydroxylase gene, a lipoxygenase gene, and a collagen alpha gene, such as a collagen alpha 1(I) gene, or from a variant of a 3'UTR of a gene selected from the group consisting of, an $\alpha$-globin gene, a $\beta$-globin gene, a tyrosine hydroxylase gene, a lipoxygenase gene, and a collagen alpha gene, such as a collagen alpha 1(I) gene. In a particularly preferred embodiment, the 3'UTR element comprises or consists of a nucleic acid sequence, which is derived from a 3'UTR of a vertebrate albumin gene, more preferably a mammalian albumin gene, most preferably a human albumin gene. It is also disclosed that the 3'UTR element comprises or consists of a nucleic acid sequence, which is derived from a 3'UTR of an $\alpha$-globin gene, or a vertebrate $\alpha$-globin gene or a mammalian $\alpha$-globin gene, or a human $\alpha$-globin gene.

It is also disclosed that the at least one 3'UTR element comprises or consists of a nucleic acid sequence, which is derived from the 3'UTR of a vertebrate $\alpha$-globin gene, a vertebrate $\beta$-globin gene, a vertebrate tyrosine hydroxylase gene, a vertebrate lipoxygenase gene, and a vertebrate collagen alpha gene, such as a vertebrate collagen alpha 1(I) gene, or from a variant thereof, or from the 3'UTR of a mammalian $\alpha$-globin gene, a mammalian $\beta$-globin gene, a mammalian tyrosine hydroxylase gene, a mammalian lipoxygenase gene, and a mammalian collagen alpha gene, such as a mammalian collagen alpha 1(I) gene, or from a variant thereof, or from the 3'UTR of a human $\alpha$-globin gene, a human $\beta$-globin gene, a human tyrosine hydroxylase gene, a human lipoxygenase gene, and a human collagen alpha gene, such as a human collagen alpha 1(I) gene, or from a variant thereof. Preferably, it is derived from the 3'UTR of the human albumin gene according to GenBank Accession number NM_000477.5 or from a variant thereof. In a preferred embodiment, the 3'UTR element is not derived from the 3'UTR of a *Xenopus* albumin gene. Preferably, the 3'UTR element does not comprise a poly(A) limiting element B (PLEB) of a 3'UTR from a *Xenopus* albumin gene. Preferably, the 3'UTR element does not consist of a PLEB of a 3'UTR from a *Xenopus* albumin gene.

Preferably, the 3'UTR element and the at least one open reading frame are heterologous, e.g. preferably the 3'UTR element and the ORF are derived from different genes of the same or of different species. It is disclosed that the ORF does not encode an $\alpha$-globin protein, if the 3'UTR element is derived from an $\alpha$-globin gene. It is disclosed that the ORF does not encode a $\beta$-globin protein, if the 3'UTR element is derived from a $\beta$-globin gene. It is also disclosed that the ORF does not encode an albumin protein, if the 3'UTR element is derived from an albumin gene. It is also disclosed that the ORF does not encode a tyrosine hydroxylase protein, if the 3'UTR element is derived from a tyrosine hydroxylase gene. Preferably, the ORF does not encode a lipoxygenase protein if the 3'UTR element is derived from a lipoxygenase gene. Preferably, the ORF does not encode a collagen alpha protein if the 3'UTR element is derived from a collagene alpha gene. Preferably, the ORF does not code for a protein selected from the group consisting of albumin proteins, growth hormones, e.g. human growth hormone (hGH), α-globin proteins, β-globin proteins, tyrosine hydroxylase proteins, lipoxygenase proteins, and collagen alpha proteins. Furthermore, it is preferred that the open reading frame does not code for a reporter protein, e.g., selected from the group consisting of globin proteins, in particular beta-globin" luciferase protein, GFP proteins, e.g. EGFP, or variants thereof, for example, variants exhibiting at least 70% sequence identity to a globin protein, a luciferase protein, or a GFP protein.

The term 'a nucleic acid sequence which is derived from the 3'UTR of a [...] gene' refers to a nucleic acid sequence, which is based on the 3'UTR sequence of a [...] gene or on a part thereof, such as on the 3'UTR of an albumin gene, an α-globin gene, a β-globin gene, a tyrosine hydroxylase gene, a lipoxygenase gene, or a collagen alpha gene, such as a collagen alpha 1(I) gene. For the present invention, it is derived from the 3'UTR of an albumin gene or on a part thereof. This term includes sequences corresponding to the entire 3'UTR sequence, i.e. the full length 3'UTR sequence of a gene, and sequences corresponding to a fragment of the 3'UTR sequence of a gene, such as an albumin gene, α-globin gene, β-globin gene, tyrosine hydroxylase gene, lipoxygenase gene, or collagen alpha gene, such as a collagen alpha 1(I) gene, or of an albumin gene for the present invention. A fragment in this context preferably consists of a continuous stretch of nucleotides corresponding to a continuous stretch of nucleotides in the full-length 3'UTR, which represents at least 80%, and most preferably at least 90% of the full-length 3'UTR. Such a fragment, in the sense of the present invention, is preferably a functional fragment as described herein. The term '3'UTR of a [...] gene' preferably refers to the 3'UTR of a naturally occurring gene, such as of a naturally occurring albumin gene. It is also disclosed that it may refer to a naturally occurring α-globin gene, β-globin gene, tyrosine hydroxylase gene, lipoxygenase gene, or collagen alpha gene.

The terms 'variant of the 3'UTR of a [...] gene' and 'variant thereof' in the context of a 3'UTR refers to a variant of the 3'UTR of a naturally occurring gene, such as a naturally occurring albumin gene, a naturally occurring α-globin gene, a naturally occurring β-globin gene, a naturally occurring tyrosine hydroxylase gene, a naturally occurring lipoxygenase gene, or a naturally occurring collagen alpha gene, such as a naturally occurring collagen alpha 1(I) gene, or to a variant of the 3'UTR of a vertebrate albumin gene, a vertebrate α-globin gene, a vertebrate β-globin gene, a vertebrate tyrosine hydroxylase gene, a vertebrate lipoxygenase gene, and a vertebrate collagen alpha gene, such as a vertebrate collagen alpha 1(I) gene; preferably to a variant of the 3'UTR of a mammalian albumin gene for the present invention: it is also disclosed that it refers to a variant of the 3'UTR of a mammalian α-globin gene, a mammalian β-globin gene, a mammalian tyrosine hydroxylase gene, a mammalian lipoxygenase gene, and a mammalian collagen alpha gene, such as a mammalian collagen alpha 1 (I) gene, more preferably to a variant of the 3'UTR of a human albumin gene, for the present invention, it is also disclosed that it refers to a variant of a 3'UTR of a human α-globin gene, a human β-globin gene, a human tyrosine hydroxylase gene, a human lipoxygenase gene, and a human collagen alpha gene, such as a human collagen alpha 1(I) gene. Such variant may be a modified 3'UTR of a gene. For example, a variant 3'UTR may exhibit one or more nucleotide deletions, insertions, additions and/or substitutions compared to the naturally occurring 3'UTR from which the variant is derived. Preferably, a variant of a 3'UTR is at least 80%, even more preferably at least 90%, most preferably at least 95% identical to the naturally occurring 3'UTR the variant is derived from. Preferably, the variant is a functional variant as described herein.

The term 'a nucleic acid sequence which is derived from a variant of the 3'UTR of a [...] gene' refers to a nucleic acid sequence, which is based on a variant of the 3'UTR sequence of a gene, such as on a variant of the 3'UTR of an albumin gene, an α-globin gene, a β-globin gene, a tyrosine hydroxylase gene, a lipoxygenase gene, or a collagen alpha gene, such as a collagen alpha 1(I) gene, or on a part thereof as described above. This term includes sequences corresponding to the entire sequence of the variant of the 3'UTR of a gene, i.e. the full length variant 3'UTR sequence of a gene, and sequences corresponding to a fragment of the variant 3'UTR sequence of a gene. A fragment in this context preferably consists of a continuous stretch of nucleotides corresponding to a continuous stretch of nucleotides in the full-length variant 3'UTR, which represents at least 80%, and most preferably at least 90% of the full-length variant 3'UTR. Such a fragment of a variant, in the sense of the present invention, is preferably a functional fragment of a variant as described herein.

The terms 'functional variant', 'functional fragment', and 'functional fragment of a variant' (also termed 'functional variant fragment') in the context of the present invention, mean that the fragment of the 5'UTR or the 3'UTR, the variant of the 5'UTR or the 3'UTR, or the fragment of a variant of the 5'UTR or the 3'UTR of a gene fulfils at least one, preferably more than one, function of the naturally occurring 5'UTR or 3'UTR of the gene of which the variant, the fragment, or the fragment of a variant is derived. Such function may be, for example, stabilizing mRNA and/or stabilizing and/or prolonging protein production from an mRNA and/or increasing protein production from an mRNA, preferably in a mammalian cell, such as in a human cell. It is particularly preferred that the variant, the fragment, and the variant fragment in the context of the present invention fulfil the function of stabilizing an mRNA, preferably in a mammalian cell, such as a human cell, compared to an mRNA comprising a reference 5'UTR and/or a reference 3'UTR or lacking a 5'UTR and/or a 3'UTR, and/or the function of stabilizing and/or prolonging protein production from an mRNA, preferably in a mammalian cell, such as in a human cell, compared to an mRNA comprising a reference 5'UTR and/or a reference 3'UTR or lacking a 5'UTR and/or a 3'UTR, and/or the function of increasing protein production from an mRNA, preferably in a mammalian cell, such as in a human cell, compared to an mRNA comprising a reference 5'UTR and/or a reference 3'UTR or lacking a 5'UTR and/or a 3'UTR. A reference 3'UTR may be, for example, a 3'UTR naturally occurring in combination with the ORF.

Furthermore, a functional variant, a functional fragment, or a functional variant fragment of a 5'UTR or of a 3'UTR of a gene preferably does not have a substantially diminishing effect on the efficiency of translation of the mRNA, which comprises such variant of a 5'UTR and/or such variant of a 3'UTR compared to the wild type 5'UTR and/or 3'UTR, from which the variant is derived. A particularly preferred function of a "functional fragment", a "functional variant" or a "functional fragment of a variant" of the 3'UTR of an albumin gene (or $\alpha$-globin gene, $\beta$-globin gene, tyrosine hydroxylase gene, lipoxygenase gene, or collagen alpha gene, such as a collagen alpha 1(I) gene, which are not claimed) in the context of the present invention is the stabilization and/or prolongation of protein production by expression of an mRNA carrying the functional fragment, functional variant or functional fragment of a variant as described above. A particularly preferred function of a "functional fragment", a "functional variant" or a "functional fragment of a variant" of the 5'UTR in the context of the present invention is the protein production increasing function.

Preferably, the efficiency of the one or more functions exerted by the functional variant, the functional fragment, or the functional variant fragment, such as mRNA and/or protein production stabilizing efficiency and/or the protein production increasing efficiency, is at least 40%, more preferably at least 50%, more preferably at least 60%, even more preferably at least 70%, even more preferably at least 80%, most preferably at least 90% of the mRNA and/or protein production stabilizing efficiency and/or the protein production increasing efficiency exhibited by the naturally occurring 5'UTR and/or 3'UTR, from which the variant, the fragment or the variant fragment is derived.

In the context of the present invention, a fragment or part of the 3'UTR of an albumin gene (or $\alpha$-globin gene, $\beta$-globin gene, tyrosine hydroxylase gene, lipoxygenase gene, or collagen alpha gene, such as a collagen alpha 1(I) gene, all of which are not claimed) or of a variant thereof preferably exhibits a length of at least about 40 nucleotides, preferably of at least about 50 nucleotides, preferably of at least about 75 nucleotides, more preferably of at least about 100 nucleotides, even more preferably of at least about 125 nucleotides, most preferably of at least about 150 nucleotides. Preferably, such fragment of the 3'UTR of a gene or of a variant of the 3'UTR of a gene is a functional fragment as described above.

In the context of the present invention, a fragment or part of the 5'UTR of a TOP gene or of a variant thereof preferably exhibits a length of at least about 20 nucleotides, preferably of at least about 30 nucleotides, more preferably of at least about 50 nucleotides. Preferably, such fragment of the 5'UTR of a TOP gene or of a variant of the 5'UTR of a TOP gene is a functional fragment as described above.

In some embodiments, the at least one 3'UTR element of the artificial nucleic acid molecule according to the present invention comprises or consists of a "functional fragment", a "functional variant" or a "functional fragment of a variant" of the 3'UTR of an albumin gene (or an $\alpha$-globin gene, $\beta$-globin gene, tyrosine hydroxylase gene, lipoxygenase gene, or collagen alpha gene, such as a collagen alpha 1(I) gene, all of which are not claimed) or of a variant thereof.

In some embodiments, the at least one 5'UTR element of the artificial nucleic acid molecule according to the present invention comprises or consists of a "functional fragment", a "functional variant" or a "functional fragment of a variant" of the 5'UTR of a TOP gene.

Preferably, the at least one 3'UTR element of the artificial nucleic acid molecule according to the present invention increases the stability of the artificial nucleic acid molecule, e.g. increases the stability of an mRNA according to the present invention, compared to a respective mRNA (reference mRNA) lacking a 3'UTR element or comprising a reference 3'UTR element, such as a 3'UTR naturally occurring in combination with the ORF. Preferably, the at least one 3'UTR element of the artificial nucleic acid molecule according to the present invention increases the stability of protein production from the artificial nucleic acid molecule according to the present invention, e.g. from an mRNA according to the present invention, compared to a respective mRNA lacking a 3'UTR element or comprising a reference 3'UTR element, such as a 3'UTR naturally occurring in combination with the ORF. Preferably, the at least one 3'UTR element of the artificial nucleic acid molecule according to the present invention prolongs protein production from the artificial nucleic acid molecule according to the present invention, e.g. from an mRNA according to the present invention, compared to a respective mRNA lacking a 3'UTR element or comprising a reference 3'UTR element, such as a 3'UTR naturally occurring in combination with the ORF. Preferably, the at least one 3'UTR element of the artificial nucleic acid molecule according to the present invention increases the protein production from the artificial nucleic acid molecule according to the present invention, e.g. from an mRNA according to the present invention, compared to a respective mRNA lacking a 3'UTR element or comprising a reference 3'UTR element, such as a 3'UTR naturally occurring in combination with the ORF. Preferably, the at least one 3'UTR element of the artificial nucleic acid molecule according to the present invention does not negatively influence translational efficiency of an mRNA compared to the translational efficiency of a respective mRNA lacking a 3'UTR element or comprising a reference 3'UTR element, such as a 3'UTR naturally occurring in combination with the ORF. The term 'respective mRNA' in this context means that - apart from the different 3'UTR - the reference mRNA is comparable, preferably identical, to the mRNA comprising the 3'UTR element.

Preferably, the at least one 5'UTR element of the artificial nucleic acid molecule according to the present invention increases the stability of the artificial nucleic acid molecule, e.g. increases the stability of an mRNA according to the present invention, compared to a respective mRNA (reference mRNA) lacking a 5'UTR element or comprising a reference 5'UTR element, such as a 5'UTR naturally occurring in combination with the ORF. Preferably, the at least one 5'UTR element of the artificial nucleic acid molecule according to the present invention increases protein production from the artificial nucleic acid molecule according to the present invention, e.g. from an mRNA according to the present invention, compared to a respective mRNA lacking a 5'UTR element or comprising a reference 5'UTR element, such as a 5'UTR naturally occurring in combination with the ORF. The term 'respective mRNA' in this context means that - apart from the different 5'UTR - the reference mRNA is comparable, preferably identical, to the mRNA comprising the inventive 5'UTR element.

Preferably, the at least one 5'UTR element and the at least one 3'UTR element act synergistically to increase protein production from the artificial nucleic acid molecule according to the present invention, e.g. from an mRNA according to the present invention, as described above.

The term 'stabilizing and/or prolonging protein production from an mRNA' preferably means that the protein production from the mRNA is stabilized and/or prolonged compared to the protein production from a reference mRNA, e.g. comprising a reference 3'UTR element or lacking a 3'UTR element.

'Stabilized protein expression' in this context preferably means that there is more uniform protein production from the artificial nucleic acid molecule according to the present invention over a predetermined period of time, such as over 24 hours, more preferably over 48 hours, even more preferably over 72 hours, when compared to a reference nucleic acid molecule, for example, an mRNA comprising a reference 3'UTR element or lacking a 3'UTR element. Thus, the level of protein production, e.g. in a mammalian system, from the artificial nucleic acid molecule comprising a 3'UTR element according to the present invention, e.g. from an mRNA according to the present invention, preferably does not drop to the extent observed for a reference nucleic acid molecule, such as a reference mRNA as described above. For example, the amount of a protein (encoded by the ORF) observed 6 hours after initiation of expression, e.g. 6 hours post transfection of the artificial nucleic acid molecule according to the present invention into a cell, such as a mammalian cell, may be comparable to the amount of protein observed 48 hours after initiation of expression, e.g. 48 hours post transfection. Thus, the ratio of the amount of protein encoded by the ORF, such as of a reporter protein, e.g., luciferase, observed at 48 hours post initiation of expression, e.g. 48 hours post transfection, to the amount of protein observed 6 hours after initiation of expression, e.g. 6 hours post transfection, is preferably above 0.4, preferably above 0.5, more preferably above 0.6, even more preferably above 0.7, e.g. between about 0.4 and about 4, preferably between about 0.65 and about 3, more preferably between about 0.7 and about 2 for a nucleic acid molecule according to the present invention. For a respective reference nucleic acid molecule, e.g. an mRNA comprising a reference 3'UTR element or lacking a 3'UTR element, said ratio may be, e.g. between about 0.05 and about 0.3. Thus, the present invention provides an artificial nucleic acid molecule comprising an ORF and a 3'UTR element as described above, wherein the ratio of the (reporter) protein amount observed 48 hours after initiation of expression to the (reporter) protein amount observed 6 hours after initiation of expression, preferably in a mammalian expression system, such as in mammalian cells, is preferably above 0.4, preferably above 0.5, more preferably above 0.6, even more preferably above 0.7, e.g. between about 0.4 and about 4, preferably between about 0.65 and about 3, more preferably between about 0.7 and about 2.

'Increased protein expression' in the context of the present invention may refer to increased protein expression at one time point after initiation of expression compared to a reference molecule or to an increased total protein production within a certain time period after initiation of expression. Thus, the protein level observed at a certain time point after initiation of expression, e.g. after transfection, of the artificial nucleic acid molecule according to the present invention, e.g. after transfection of an mRNA according to the present invention, for example, 24, 48, or 72 hours post transfection, or the total protein produced in a time span of, e.g. 24, 48 or 72 hours, is preferably higher than the protein level observed at the same time point after initiation of expression, e.g. after transfection, or the total protein produced within the same time span, for a reference nucleic acid molecule, such as a reference mRNA comprising a reference 5' and/or a reference 3'UTR or lacking a 5'UTR element and/or 3'UTR element. As set forth above, it is a particularly preferred function of the 5'UTR element to affect the increase in protein production from the artificial nucleic acid molecule. Preferably, the increase in protein production effected by the 5'UTR element compared to a reference nucleic acid molecule lacking such 5'UTR element at a given time point post initiation of expression is at least 1.5-fold, more preferably at least 2-fold, more preferably at least 3-fold, even more preferably at least 4-fold, most preferably at least 5-fold of the protein production observed for a reference nucleic acid molecule lacking the 5'UTR element. The same holds preferably for the total protein production in a given time period, for example in a time period of 24, 48 or 72 hours post initiation of expression.

Said increase in stability of the artificial nucleic acid molecule, said increase in stability of protein production, said prolongation of protein production and/or said increase in protein production is preferably determined by comparison with a respective reference nucleic acid molecule lacking a 5'UTR element and/or a 3'UTR element, e.g. an mRNA lacking a 5'UTR element and/or a 3'UTR element, or a reference nucleic acid molecule comprising a reference 5'UTR element and/or a reference 3'UTR element, such as a 3'UTR and/or a 5'UTR naturally occurring with the ORF or a 5'UTR and/or a 3'UTR of a reference gene.

The mRNA and/or protein production stabilizing effect and efficiency and/or the protein production increasing effect and efficiency of the variants, fragments and/or variant fragments of the 3'UTR of an albumin gene as well as the mRNA and/or protein production stabilizing effect and efficiency and/or the protein production increasing effect and efficiency of the at least one 3'UTR element, the at least one 5'UTR element, or the at least one 3'UTR element and the at least one 5'UTR element of the artificial nucleic acid molecule according to the present invention may be determined by any method suitable for this purpose known to the skilled person. For example, artificial mRNA molecules may be generated comprising a coding sequence for a reporter protein, such as luciferase, and no 3'UTR and/or no 5'UTR, a 5'UTR element derived from a TOP gene and/or a 3'UTR elem,ent derived from a gene as described above, a 5'UTR element derived from a reference gene and/or a 3'UTR derived derived from a reference gene (i.e., a reference 3'UTR element or a reference 5'UTR element, such as a 5'UTR or a 3'UTR naturally occurring with the ORF), as 3'UTR a variant of a 3'UTR of a gene as described above, as 3'UTR a fragment of a 3'UTR of a gene as described above, or as 3'UTR a fragment of a variant of a 3'UTR of a gene as described above, as 5'UTR a variant of a 5'UTR of a TOP gene, as 5'UTR a fragment of a 5'UTR of a TOP gene, or as 5'UTR a fragment of a variant of a 5'UTR of a TOP gene. Such mRNAs may be generated, for example, by *in vitro* transcription of respective vectors such as plasmid vectors, e.g. comprising a T7 promoter and a sequence encoding the respective mRNA sequences. The generated mRNA molecules may be transfected into cells by any transfection method suitable for transfecting mRNA, for example they may be electroporated into mammalian cells, such as HELA or HDF cells, and samples may be analyzed certain time points after transfection, for example, 6 hours, 24 hours, 48 hours, and 72 hours post transfection. Said samples may be analyzed for mRNA quantities and/or protein quantities by methods well known to the skilled person. For example, the quantities of reporter mRNA present in the cells at the sample time points may be determined by quantitative PCR methods. The quantities of reporter protein encoded by the respective mRNAs may be determined, e.g., by ELISA assays or reporter assays such as luciferase assays depending on the reporter protein used. The effect of stabilizing protein expression and/or prolonging protein expression may be, for example, analyzed by determining the ratio of the protein level observed 48 hours post transfection and the protein level observed 6 hours post transfection. The closer said value is to 1, the more stable the protein expression is within this time period. Said value may also be above 1 if the protein level is higher at the later time point. Such measurements may of course also be performed at 72 or more hours and the ratio of the protein level observed 72 hours post transfection and the protein level observed 6 hours post transfection may be determined to determine stability of protein expression.

Preferably, the at least one 3'UTR element of the artificial nucleic acid molecule according to the present invention comprises or consists of a nucleic acid sequence, which has an identity of at least about 80%, more preferably of at least about 90%, even more preferably of at least about 95%, even more preferably of at least about 99%, most preferably of 100% to a nucleic acid sequence selected from SEQ ID No. 1369-1377, 1391, 1392, and 1393 and wherein the variants of the sequences according to SEQ ID No. 1369-1377, 1391, 1392 and 1393 are preferably functional variants as described above.

The at least one 3'UTR element of the artificial nucleic acid molecule according to the present invention may also comprise or consist of a fragment of a nucleic acid sequence, which has an identity of at least about 80%, more preferably of at least about 90%, even more preferably of at least about 95%, even more preferably of at least about 99%, most preferably of 100% to the nucleic acid sequence according to SEQ ID No. 1369-1377, 1391, 1392, or 1393 wherein the fragment is preferably a functional fragment or a functional variant fragment as described above. Preferably, the fragment is as described above, i.e. being a continuous stretch of nucleotides representing at least 80% etc. of the full-length 3'UTR the fragment is derived from. Such fragment preferably exhibits a length of at least about 40 nucleotides, preferably of at least about 50 nucleotides, preferably of at least about 75 nucleotides, more preferably of at least about 100 nucleotides, even more preferably of at least about 125 nucleotides, most preferably of at least about 150 nucleotides.

For example, such fragment may exhibit a nucleic acid sequence according to SEQ ID Nos. 1378-1390, such as

```
AAAAGCATCT CAGCCTACCA TGAGAATAAG AGAAAGAAAA TGAAGATCAA AAGCTTATTC
ATCTGTTTTT CTTTTTCGTT GGTGTAAAGC CAACACCCTG TCTAAAAAAC ATAAATTTCT
TTAATCATTT TGCCTCTTTT CTCTGTGCTT CAATT (SEQ ID No. 1378)
```

```
CATCACATTT AAAAGCATCT CAGCCTACCA TGAGAATAAG AGAAAGAAAA TGAAGATCAA
AAGCTTATTC ATCTGTTTTT CTTTTTCGTT GGTGTAAAGC CAACACCCTG (SEQ ID No.
1379)
```

AAAAGCATCT CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC (SEQ ID No. 1380)

CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT (SEQ ID No. 1381)

TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT (SEQ ID No. 1382)

AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT (SEQ ID No. 1383)

TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT (SEQ ID No. 1384)

AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA (SEQ ID No. 1385)

ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA AAAATGGAAA (SEQ ID No. 1386)

CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No. 1387)

TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No. 1388)

CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No. 1389)

AAGCTTATTC ATCTGTTTTT CTTTTTCGTT GGTGTAAAGC CAACACCCTG TCTAAAAAAC (SEQ ID No. 1390) or the corresponding RNA sequence, or a nucleic acid sequence, which is at least about 80%, more preferably at least about 90%, even more preferably at least about 95%, even more preferably at least about 99% identical to said nucleic acid sequences or the corresponding RNA sequence. Thus, the at least one 3'UTR element of the artificial nucleic acid molecule according to the present invention may comprise or consist of a nucleic acid fragment as described above. Obviously, the thymidine nucleotides comprised in the fragments according to SEQ ID Nos. 1378-1390 may be replaced by uridine nucleotides.

Preferably, said variants, fragments or variant fragments are functional variants, functional fragments, or functional variant fragments as described above, exhibiting at least one function of the nucleic acid sequence according to SEQ ID Nos. 1369-1377, 1391, 1392, or 1393, such as stabilization of the artificial nucleic acid molecule according to the invention, stabilizing and/or prolonging protein expression from the artificial nucleic acid molecule according to the invention, and/or increasing protein production, preferably with an efficiency of at least 40%, more preferably of at least 50%, more preferably of at least 60%, even more preferably of at least 70%, even more preferably of at least 80%, most preferably of at least 90% of the stabilizing efficiency and/or protein production increasing efficiency exhibited by the nucleic acid sequence according to SEQ ID Nos. 1369-1377, 1391, 1392, or 1393. Preferably, variants, fragments or variant fragments are functional variants, functional fragments, or functional variant fragments exhibit the function of acting synergistically with the 5'UTR element to increase protein production from the artificial nucleic acid molecule.

Preferably, the at least one 3'UTR element of the artificial nucleic acid molecule according to the present invention exhibits a length of at least about 40 nucleotides, preferably of at least about 50 nucleotides, preferably of at least about 75 nucleotides, more preferably of at least about 100 nucleotides, even more preferably of at least about 125 nucleotides, most preferably of at least about 150 nucleotides. For example, the 3'UTR may exhibit a length of about 50 to about 300 nucleotides, preferably of about 100 to about 250 nucleotides, more preferably of about 150 to about 200 nucleotides.

Furthermore, the artificial nucleic acid molecule according to the present invention may comprise more than one 3'UTR elements as described above. For example, the artificial nucleic acid molecule according to the present invention may comprise one, two, three, four or more 3'UTR elements, wherein the individual 3'UTR elements may be the same or they may be different. For example, the artificial nucleic acid molecule according to the present invention may comprise two essentially identical 3'UTR elements as described above, e.g. two 3'UTR elements comprising or consisting of a nucleic acid sequence which is derived from the 3'UTR of an albumin gene or from a variant of the 3'UTR of an albumin gene, such as a nucleic acid sequence according to SEQ ID No. 1369 or 1376, functional variants thereof, functional fragments thereof, or functional variant fragments thereof as described above.

Surprisingly, the inventors found that an artificial nucleic acid molecule comprising a 5'UTR element comprising or consisting of a nucleic acid sequence derived from a TOP gene as described above may represent or may provide an mRNA molecule exhibiting strongly enhanced protein production from said artificial nucleic acid molecule.

The artificial nucleic acid molecule according to the present invention may be RNA, such as mRNA, DNA, such as a DNA vector, or may be a modified RNA or DNA molecule. It may be provided as a double-stranded molecule having a sense strand and an anti-sense strand, for example, as a DNA molecule having a sense strand and an anti-sense strand.

The artificial nucleic acid molecule according to the present invention may further comprise a 5'-cap. The optional 5'-cap is preferably attached to the 5'-side of the 5'UTR element.

In a preferred embodiment, the artificial nucleic acid sequence comprises a 5'UTR element, which comprises or consists of a nucleic acid sequence, which is derived from the 5'UTR of a TOP gene encoding a ribosomal protein as described above, for example, encoding a ribosomal Large protein, or from a variant thereof, and a 3'UTR element which comprises or consists of a nucleic acid sequence, which is derived from the 3'UTR of an albumin gene or a variant thereof as described above.

In a particularly preferred embodiment, the artificial nucleic acid sequence comprises a 5'UTR element, which comprises or consists of a nucleic acid sequence, which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene (HSD17B4), an androgen-induced 1 gene (AIG1), cytochrome c oxidase subunit VIc gene (COX6C), or a N-acylsphingosine amidohydrolase (acid ceramidase) 1 gene (ASAH1) or from a variant thereof, preferably from a vertebrate ribosomal protein Large 32 gene (RPL32), a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit 1, cardiac muscle (ATP5A1) gene, a vertebrate hydroxysteroid (17-beta) dehydrogenase 4 gene (HSD17B4), a vertebrate androgen-induced 1 gene (AIG1), a vertebrate cytochrome c oxidase subunit VIc gene (COX6C), or a vertebrate N-acylsphingosine amidohydrolase (acid ceramidase) 1 gene (ASAH1) or from a variant thereof, more preferably from a mammalian ribosomal protein Large 32 gene (RPL32), a ribosomal protein Large 35 gene (RPL35), a ribosomal protein Large 21 gene (RPL21), a mammalian ATP synthase, H+ transporting, mitochondrial F1 complex, alpha subunit 1, cardiac muscle (ATP5A1) gene, a mammalian hydroxysteroid (17-beta) dehydrogenase 4 gene (HSD17B4), a mammalian androgen-induced 1 gene (AIG1), a mammalian cytochrome c oxidase subunit VIc gene (COX6C), or a mammalian N-acylsphingosine amidohydrolase (acid ceramidase) 1 gene (ASAH1) or from a variant thereof, most preferably from a human ribosomal protein Large 32 gene (RPL32), a human ribosomal protein Large 35 gene (RPL35), a human ribosomal protein Large 21 gene (RPL21), a human ATP synthase, H+ transporting, mitochondrial F1 complex, alpha subunit 1, cardiac muscle (ATP5A1) gene, a human hydroxysteroid (17-beta) dehydrogenase 4 gene (HSD17B4), a human androgen-induced 1 gene (AIG1), a human cytochrome c oxidase subunit VIc gene (COX6C), or a human N-acylsphingosine amidohydrolase (acid ceramidase) 1 gene (ASAH1) or from a variant thereof, wherein preferably the 5'UTR element does not comprise the 5'TOP of said gene, and a 3'UTR element, which comprises or consists of a nucleic acid sequence, which is derived from an albumin gene as described above.

In a particularly preferred embodiment, the artificial nucleic acid molecule according to the present invention comprises a 5'UTR element, which comprises or consists of a nucleic acid sequence, which has an identity of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 1368 or SEQ ID NOs 1412-1420, or a corresponding RNA sequence, and a 3'UTR element, which comprises or consist of a nucleic acid sequence, which has an identity of at least about 80%, more preferably of at least about 90%, even more preferably of at least about 95%, even more preferably of at least about 99%, most preferably of 100% to the nucleic acid sequence according to SEQ ID No. 1369, 1376, 1377, 1391, or 1392, e.g., a 5'UTR element, which comprises or consists of a nucleic acid sequence, which has an identity of at least about 90% to the nucleic acid sequence according to SEQ ID No. 1368 or a corresponding RNA sequence and a 3'UTR element, which comprises or consist of a nucleic acid sequence, which has an identity of at least about 90% to the nucleic acid sequence according to SEQ ID No. 1369, 1376, 1377, 1391, or 1392.

Preferably, the artificial nucleic acid molecule according to the present invention further comprises a poly(A) sequence and/or a polyadenylation signal. Preferably, the optional poly(A) sequence is located 3' to the ORF or the at least one 3'UTR element, preferably is connected to the 3'-end of the ORF or the 3'UTR element. The connection may be direct or indirect, for example, via a stretch of 2, 4, 6, 8, 10, 20 etc. nucleotides, such as via a linker of 1-50, preferably of 1-20 nucleotides, e.g. comprising or consisting of one or more restriction sites.

In one embodiment, the optional polyadenylation signal is located within the 3'UTR element. Preferably, the polyadenylation signal comprises the consensus sequence NN(U/T)ANA, with N = A or U, preferably AA(U/T)AAA or A(U/T)(U/T)AAA. Such consensus sequence may be recognised by most animal and bacterial cell-systems, for example by the polyadenylation-factors, such as cleavage/polyadenylation specificity factor (CPSF) cooperating with CstF, PAP, PAB2, CFI and/or CFII. Preferably, the polyadenylation signal, preferably the consensus sequence NNUANA, is located less than about 50 nucleotides, more preferably less than about 30 nucleotides, most preferably less than about 25 nucleotides, for example 21 nucleotides, upstream of the 3'-end of the 3'UTR element.

Using an appropriate transcription system will then lead to attachment of a poly(A) sequence to the premature-RNA. For example, the inventive artificial nucleic acid molecule may be a DNA molecule comprising a 3'UTR element as described above and a polyadenylation signal, which may result in polyadenylation of an RNA upon transcription of this DNA molecule. Accordingly, a resulting RNA may comprise a combination of the 3'UTR element followed by a poly(A) sequence.

Potential transcription systems are *in vitro* transcription systems or cellular transcription systems etc. Accordingly, transcription of an artificial nucleic acid molecule according to the invention, e.g. transcription of an artificial nucleic acid molecule comprising a 5'UTR element, an open reading frame, a 3'UTR element and a polyadenylation-signal, may result in an mRNA molecule comprising a 5'UTR element, an open reading frame, a 3'UTR element and a poly(A) sequence.

The invention also provides an artificial nucleic acid molecule, which is an mRNA molecule comprising a 5'UTR element, an open reading frame, an 3'UTR element as described above and a poly(A) sequence.

In one embodiment, the invention provides an artificial nucleic acid molecule, which is an artificial DNA molecule comprising a 5'UTR element as described above, an open reading frame and preferably a nucleic acid sequence according to any one of SEQ ID Nos. 1369-1377, 1391, and 1392 or a sequence having an identity of at least 80% or more to a nucleic acid sequence according to any one of SEQ ID Nos. 1369-1377, 1391, and 1392 or a fragment thereof. Furthermore, the invention provides an artificial nucleic acid molecule, which is an artificial RNA molecule comprising a 5'UTR element as described above, an open reading frame and preferably an RNA sequence corresponding to a sequence according to any one of SEQ ID Nos. 1369-1377, 1391, and 1392 or a sequence having an identity of at least about 80 % or more to any one of SEQ ID Nos. 1369-1377, 1391, and 1392, or a fragment thereof.

Accordingly, the invention provides an artificial nucleic acid molecule, which may be a template for an RNA molecule, preferably for an mRNA molecule, which is stabilised and optimized with respect to translation efficiency. In other words, the artificial nucleic acid molecule may be a DNA or RNA, which may be used for production of an mRNA. The obtainable mRNA, may, in turn, be translated for production of a desired peptide or protein encoded by the open reading frame. If the artificial nucleic acid molecule is a DNA, it may, for example, be used as a double-stranded storage form for continued and repetitive *in vitro* or *in vivo* production of mRNA.

In one embodiment, the artificial nucleic acid molecule according to the present invention further comprises a poly(A) sequence. The length of the poly(A) sequence may vary. For example, the poly(A) sequence may have a length of about 20 adenine nucleotides up to about 300 adenine nucleotides, preferably of about 40 to about 200 adenine nucleotides, more preferably from about 50 to about 100 adenine nucleotides, such as about 60, 70, 80, 90 or 100 adenine nucleotides.

For example, the artificial nucleic acid molecule according to the present invention may comprise a nucleic acid sequence corresponding to the DNA sequence

```
CATCACATTT AAAAGCATCT CAGCCTACCA TGAGAATAAG AGAAAGAAAA TGAAGATCAA

AAGCTTATTC ATCTGTTTTT CTTTTTCGTT GGTGTAAAGC CAACACCCTG TCTAAAAAAC

ATAAATTTCT TTAATCATTT TGCCTCTTTT CTCTGTGCTT CAATTAATAA AAAATGGAAA

GAATCTAGAT CTAAAAAAAA AAAAAAAAAA AAAAAAAAAA AAAAAAAAAA AAAAAAAAAA

AAAAAAAAAA AAAAAA (SEQ ID No. 1377).
```

Transcription of such a sequence may result in an artificial nucleic acid molecule comprising a corresponding RNA sequence.

Such artificial RNA molecule may also be obtainable *in vitro* by common methods of chemical synthesis without being necessarily transcribed from a DNA progenitor.

In a particularly preferred embodiment, the artificial nucleic acid molecule according to the present invention is an RNA molecule, preferably an mRNA molecule comprising in 5'-to-3'-direction a 5'UTR element as described above, an open reading frame, a 3'UTR element as described above and a poly(A) sequence.

In a preferred embodiment, the open reading frame does not code for human albumin, provided that the 3'UTR element is identical to the 3'UTR of human albumin. In some further embodiments, it is preferred that the open reading frame does not code for human albumin according to GenBank Accession number NM_000477.5 provided that the 3'UTR element is identical to the 3'UTR of human albumin. In some further embodiments, it is preferred that the open reading frame does not code for human albumin or variants thereof provided that the 3'UTR element is a sequence, which is identical to SEQ ID No. 1369. Furthermore, in some embodiments, it is preferred that the open reading frame does not code for a reporter protein, e.g., selected from the group consisting of globin proteins, luciferase proteins, GFP proteins or variants thereof, for example, variants exhibiting at least 70% sequence identity to a globin protein, a luciferase protein, or a GFP protein.

It is also disclosed that the 3'UTR element does not consist of a histone stem-loop or does not comprise a histone stem-loop. In one embodiment, the artificial nucleic acid molecule according to the present invention does not comprise a histone stem-loop. However, in some embodiments, the 3'UTR element of the artificial nucleic acid molecule or the artificial nucleic acid molecule according to the present invention may comprise a histone stem-loop in addition to the nucleic acid sequence derived form the 3'UTR of an albumin gene. Such artificial nucleic acid molecule according to the present invention, for example, may comprise in 5'-to-3'-direction a 5'UTR element as defined above, an ORF, a 3'UTR element as defined above, preferably comprising a polyadenylation signal, an optional histone stem-loop and an optional poly(A) sequence. It may also comprise in 5'-to-3'-direction a 5'UTR element as described above, an ORF, a 3'UTR element as defined above, e.g. comprising a polyadenylation signal, a poly(A) sequence and an optional histone stem-loop.

In the context of the present invention, such a histone stem-loop is typically derived from a histone gene and comprises an intramolecular base pairing of two neighbored entirely or partially reverse complementary sequences, thereby forming a stem-loop. A stem-loop can occur in single-stranded DNA or, more commonly, in RNA. The structure is also known as a hairpin or hairpin loop and usually consists of a stem and a (terminal) loop within a consecutive sequence, wherein the stem is formed by two neighbored entirely or partially reverse complementary sequences separated by a short sequence as sort of spacer, which builds the loop of the stem-loop structure. The two neighbored entirely or partially reverse complementary sequences may be defined as e.g. stem-loop elements stem1 and stem2. The stem loop is formed when these two neighbored entirely or partially reverse complementary sequences, e.g. stem-loop elements stem1 and stem2, form base-pairs with each other, leading to a double stranded nucleic acid sequence comprising an unpaired loop at its terminal ending formed by the short sequence located between stem-loop elements stem1 and stem2 on the consecutive sequence. The unpaired loop thereby typically represents a region of the nucleic acid which is not capable of base pairing with either of these stem-loop elements. The resulting lollipop-shaped structure is a key building block of many RNA secondary structures. The formation of a stem-loop structure is thus dependent on the stability of the resulting stem and loop regions, wherein the first prerequisite is typically the presence of a sequence that can fold back on itself to form a paired double strand. The stability of paired stem-loop elements is determined by the length, the number of mismatches or bulges it contains (a small number of mismatches is typically tolerable, especially in a long double strand), and the base composition of the paired region. In the context of the present invention, optimal loop length is 3-10 bases, more preferably 3 to 8, 3 to 7, 3 to 6 or even more preferably 4 to 5 bases, and most preferably 4 bases.

An example for a histone stem-loop sequence is the sequence according to SEQ ID NO: 1394 (CAAAGGCTCTTTTCAGAGCCACCA) or the corresponding RNA sequence.

Thus, in some embodiments, the artificial nucleic acid molecule according to the present invention comprises at least one histone-stem loop which may, for example, comprise or consist of a sequence having a sequence identity of at least about 75%, preferably of at least about 80%, preferably at least about 85%, more preferably at least about 90%, even more preferably at least about 95% to the sequence according to SEQ ID NO. 1394 or the corresponding RNA sequence, wherein preferably positions 6, 13 and 20 of the sequence having a sequence identity of at least about 75%, preferably of at least about 80%, preferably at least about 85%, more preferably at least about 90%, even more preferably at least about 95% to the sequence according to SEQ ID NO. 1394 or the corresponding RNA sequence are conserved, i.e. are identical to the nucleotides at positions 6, 13 and 20 of SEQ ID NO. 1394.

In some embodiments, the artificial nucleic acid molecule comprises further elements such as a 5'-cap, a poly(C) sequence and/or an IRES-motif. A 5'-cap may be added post-transcriptionally to the 5'end of an RNA. Further, the inventive artificial nucleic acid molecule, particularly if the nucleic acid is in the form of an mRNA or codes for an mRNA, may be modified by a sequence of at least 10 cytidines, preferably at least 20 cytidines, more preferably at least 30 cytidines (so-called "poly(C) sequence"). Particularly, the inventive nucleic acid molecule may contain, especially if the nucleic acid is in the form of an (m)RNA or codes for an mRNA, a poly(C) sequence of typically about 10 to 200 cytidine nucleotides, preferably about 10 to 100 cytidine nucleotides, more preferably about 10 to 70 cytidine nucleotides or even more preferably about 20 to 50 or even 20 to 30 cytidine nucleotides.

An internal ribosome entry side (IRES) sequence or IRES-motif may separate several open reading frames, for example if the artificial nucleic acid molecule encodes for two or more peptides or proteins. An IRES-sequence may be particularly helpful if the mRNA is a bi- or multicistronic RNA.

Furthermore, the artificial nucleic acid molecule may comprise additional 5'-elements such as a promoter containing-sequence. The promoter may drive and or regulate transcription of the artificial nucleic acid molecule according to the present invention, for example of an artificial DNA-molecule according to the present invention.

In preferred embodiments, the invention provides artificial nucleic acid molecules, preferably mRNA molecules, comprising in 5'-to-3'-direction at least one of the following structures (with the 5'UTR and 3'UTR elements as defined above)

5'-cap - 5'UTR element - ORF - 3'UTR element - histone stem-loop - poly(A) sequence

5'-cap - 5'UTR element - ORF - 3'UTR element - poly(A) sequence - histone stem-loop

5'-cap - 5'UTR element - ORF - IRES - ORF - 3'UTR element - histone stem-loop - poly(A) sequence

5'-cap - 5'UTR element - ORF - IRES - ORF - 3'UTR element - poly(A) sequence - histone stem-loop

5'-cap - 5'UTR element - ORF - 3'UTR element - poly(A) sequence - poly(C) sequence

5'-cap - 5'UTR element - ORF - 3'UTR element - poly(A) sequence - poly(C) sequence - histone stem-loop

5'-cap - 5'UTR element - ORF - IRES - ORF - 3'UTR element - histone stem-loop - poly(A) sequence - poly(C) sequence

Preferably, the artificial nucleic acid molecule, preferably the open reading frame, is at least partially G/C modified. Thus, the inventive artificial nucleic acid molecule may be thermodynamically stabilized by modifying the G (guanosine)/C (cytidine) content of the molecule. The G/C content of the open reading frame of an artificial nucleic acid molecule according to the present invention may be increased compared to the G/C content of the open reading frame of a corresponding wild type sequence, preferably by using the degeneration of the genetic code. Thus, the encoded amino acid sequence of the nucleic acid molecule is preferably not modified by the G/C modification compared to the coded amino acid sequence of the particular wild type sequence. The codons of a coding sequence or a whole nucleic acid molecule, e.g. an mRNA, may therefore be varied compared to the wild type coding sequence, such that they include an increased amount of G/C nucleotides, while the translated amino acid sequence is maintained. In respect to the fact that several codons code for one and the same amino acid (so-called degeneration of the genetic code), the most favourable codons for the stability can be determined (so-called alternative codon usage).

Depending on the amino acid to be encoded by the coding region of the inventive nucleic acid molecule as defined herein, there are various possibilities for modification of the nucleic acid sequence, e.g. the open reading frame, compared to its wild type coding region. In the case of amino acids which are encoded by codons, which contain exclusively G or C nucleotides, no modification of the codon is necessary. Thus, the codons for Pro (CCC or CCG), Arg (CGC or CGG), Ala (GCC or GCG) and Gly (GGC or GGG) require no modification, since no A or U/T is present.

In contrast, codons which contain A and/or U/T nucleotides may be modified by substitution of other codons, which code for the same amino acids but contain no A and/or U/T. For example

the codons for Pro can be modified from CC(U/-F) or CCA to CCC or CCG;

the codons for Arg can be modified from CG(U/T) or CGA or AGA or AGG to CGC or CGG;

the codons for Ala can be modified from GC(U/T) or GCA to GCC or GCG;

the codons for Gly can be modified from GG(U/T) or GGA to GGC or GGG.

In other cases, although A or (U/T) nucleotides cannot be eliminated from the codons, it is, however, possible to decrease the A and (U/T) content by using codons, which contain a lower content of A and/or (U/T) nucleotides. Examples of these are:

The codons for Phe can be modified from (U/T)(U/T)(U/T) to (U/T) (U/T)C;

the codons for Leu can be modified from (U/T) (U/T)A, (U/T) (U/T)G, C(U/T) (U/T) or C(U/T)A to C(U/T)C or C(U/T)G;

the codons for Ser can be modified from (U/T)C(U/T) or (U/T)CA or AG(U/T) to (U/T)CC, (U/T)CG or AGC;

the codon for Tyr can be modified from (U/T)A(U/T) to (U/T)AC;

the codon for Cys can be modified from (U/T)G(U/T) to (U/T)GC;

the codon for His can be modified from CA(U/T) to CAC;

the codon for Gln can be modified from CAA to CAG;

the codons for Ile can be modified from A(U/T)(U/T) or A(U/T)A to A(U/T)C;

the codons for Thr can be modified from AC(U/T) or ACA to ACC or ACG;

the codon for Asn can be modified from AA(U/T) to AAC;

the codon for Lys can be modified from AAA to AAG;

the codons for Val can be modified from G(U/T)(UT) or G(U/T)A to G(U/T)C or G(U/T)G;

the codon for Asp can be modified from GA(U/T) to GAC;

the codon for Glu can be modified from GAA to GAG;

the stop codon (U/T)AA can be modified to (U/T)AG or (U/T)GA.

In the case of the codons for Met (A(U/T)G) and Trp ((U/T)GG), on the other hand, there is no possibility of sequence modification without altering the encoded amino acid sequence.

The substitutions listed above can be used either individually or in all possible combinations to increase the G/C content of the open reading frame of the inventive nucleic acid sequence as defined herein, compared to its particular wild type open reading frame (i.e. the original sequence). Thus, for example, all codons for Thr occurring in the wild type sequence can be modified to ACC (or ACG).

Preferably, the G/C content of the open reading frame of the inventive artificial nucleic acid molecule as defined herein is increased by at least 7%, more preferably by at least 15%, particularly preferably by at least 20%, compared to the G/C content of the wild type coding region. According to a specific embodiment at least 5%, 10%, 20%, 30%, 40%, 50%, 60%, more preferably at least 70 %, even more preferably at least 80% and most preferably at least 90%, 95% or even 100% of the substitutable codons in the open reading frame of the inventive artificial nucleic acid molecule or a fragment, variant or derivative thereof are substituted, thereby increasing the G/C content of said open reading frame.

In this context, it is particularly preferable to increase the G/C content of the open reading frame of the inventive nucleic acid sequence as defined herein, to the maximum (i.e. 100% of the substitutable codons), compared to the wild type open reading frame.

Furthermore, the open reading frame is preferably at least partially codon-optimized. Codon-optimization is based on the finding that the translation efficiency may be determined by a different frequency in the occurrence of transfer RNAs (tRNAs) in cells. Thus, if so-called "rare codons" are present in the coding region of the inventive artificial nucleic acid molecule as defined herein, to an increased extent, the translation of the corresponding modified nucleic acid sequence is less efficient than in the case where codons coding for relatively "frequent" tRNAs are present.

Thus, the open reading frame of the inventive nucleic acid sequence is preferably modified compared to the corresponding wild type coding region such that at least one codon of the wild type sequence, which codes for a tRNA, which is relatively rare in the cell, is exchanged for a codon, which codes for a tRNA which is comparably frequent in the cell and carries the same amino acid as the relatively rare tRNA. By this modification, the open reading frame of the inventive artificial nucleic acid molecule as defined herein, is modified such that codons, for which frequently occurring tRNAs are available may replace codons, which correspond to rare tRNAs. In other words, according to the invention, by such a modification all codons of the wild type open reading frame which code for a rare tRNA may be exchanged for a codon which codes for a tRNA, which is more frequent in the cell and which carries the same amino acid as the rare tRNA. Which tRNAs occur relatively frequently in the cell and which, in contrast, occur relatively rarely is known to a person skilled in the art; cf. e.g. Akashi, Curr. Opin. Genet. Dev. 2001, 11(6): 660-666. Accordingly, preferably, the open reading frame is codon-optimized, preferably with respect to the system, in which the nucleic acid molecule according to the present invention is to be expressed, preferably with respect to the system, in which the nucleic acid molecule according to the present invention is to be translated. Preferably, the codon usage of the open reading frame is codon-optimized according to mammalian codon usage, more preferably according to human codon usage. Preferably, the open reading frame is codon-optimized and G/C-content modified.

For further improving degradation resistance, e.g. resistance to *in vivo* degradation by an exo- or endonuclease, and/or for further improving protein production from the artificial nucleic acid molecule according to the present invention, the artificial nucleic acid molecule may further comprise modifications, such as backbone modifications, sugar modifications and/or base modifications, e.g., lipid-modifications or the like. Preferably, the transcription and/or the translation of the artificial nucleic acid molecule according to the present invention is not significantly impaired by said modifications.

Nucleotide analogues/modifications that may be used in the context of the present invention may be selected, for example, from 2-amino-6-chloropurineriboside-5'-triphosphate, 2-aminoadenosine-5'-triphosphate, 2-thiocytidine-5'-triphosphate, 2-thiouridine-5'-triphosphate, 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-iodocytidine-5'-triphosphate, 5-iodouridine-5'-triphosphate, 5-methylcytidine-5'-triphosphate, 5-methyluridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

Further, lipid-modified artificial nucleic acid molecules may typically comprise at least one linker which is covalently linked with the artificial nucleic acid molecule, and at least one lipid which is covalently linked with this linker. Alternatively, a lipid-modified artificial nucleic acid molecule may comprise at least one artificial nucleic acid molecule as defined herein and at least one, preferably bifunctional lipid which is covalently linked, preferably without a linker, with that artificial nucleic acid molecule. According to a third alternative, a lipid-modified artificial nucleic acid molecule may comprise an artificial nucleic acid molecule as defined herein, at least one linker which is covalently linked with that artificial nucleic acid molecule, at least one lipid which is covalently linked with this linker, and additionally at least one, preferably bifunctional lipid which is covalently linked, preferably without a linker, with the artificial nucleic acid molecule.

In a further aspect, the present invention provides a vector comprising

a. at least one 5'-untranslated region element (5'UTR element) which comprises or consists of

a nucleic acid sequence which consists of a continuous stretch of nucleotides corresponding to a continuous stretch of nucleotides in the full-length 5'UTR of a TOP gene, which represents at least 80% of the full-length 5'UTR of a TOP gene, or
a nucleic acid sequence which consists of a continuous stretch of nucleotides corresponding to a continuous stretch of nucleotides in a variant of the 5'UTR of a TOP gene, which represents at least 80% of the variant of the 5'UTR of a TOP gene, wherein the variant of the 5'UTR of a TOP gene is at least 80% identical to the naturally occuring 5'UTR the variant is derived from,
wherein the at least one 5'UTR element does not comprise a functional 5'TOP motif;

b. at least one open reading frame (ORF) and/or at least one cloning site; and c. at least one 3'UTR element, which comprises or consists of

a nucleic acid sequence which consists of a continuous stretch of nucleotides corresponding to a continuous stretch of nucleotides in the full-length 3'UTR of an albumin gene, which represents at least 80% of the full-length 3'UTR of an albumin gene, or
a nucleic acid sequence which consists of a continuous stretch of nucleotides corresponding to a continuous stretch of nucleotides in a variant of a 3'UTR of an albumin gene, which represents at least 80% of the variant of the 3'UTR of an albumin gene, wherein the variant of the 3'UTR of an albumin gene is at least 80% identical to the naturally occuring 3'UTR the variant is derived from.

The at least one 5'UTR element, the optional at least one 3'UTR element and the at least one ORF are as described herein for the artificial nucleic acid molecule according to the present invention. The cloning site may be any sequence that is suitable for introducing an open reading frame or a sequence comprising an open reading frame, such as one or more restriction sites. The vector comprising a cloning site is preferably suitable for inserting an open reading frame into the vector 3' to the 5'UTR element, preferably directly 3' to the 5'UTR element. Thus, the vector comprising a cloning site is preferably suitable for inserting an open reading frame into the vector, preferably for inserting an open reading frame between the 5'UTR element and the optional 3'UTR element, preferably 5' to the optional 3'UTR element and 3' to the 5'UTR element. Preferably, the cloning site or the ORF is located 5' to the 3'UTR element, preferably in close proximity to the 5'-end of the 3'UTR element. For example, the cloning site or the ORF may be directly connected to the 5'-end of the 3'UTR element or they may be connected via a stretch of nucleotides, such as by a stretch of 2, 4, 6, 8, 10, 20 etc. nucleotides as described above for the artificial nucleic acid molecule according to the present invention. Preferably the cloning site or the ORF is located 3' to the 5'UTR element, preferably in close proximity to the 3'-end of the 5'UTR element. For example, the cloning site or the ORF may be directly connected to the 3'-end of the 5'UTR element or they may be connected via a stretch of nucleotides, such as by a stretch of 2, 4, 6, 8, 10, 20 etc. nucleotides as described above for the artificial nucleic acid molecule according to the present invention.

Preferably, the vector according to the present invention is suitable for producing the artificial nucleic acid molecule according to the present invention, preferably for producing an artificial mRNA according to the present invention, for example, by optionally inserting an open reading frame or a sequence comprising an open reading frame into the vector and transcribing the vector. Thus, preferably, the vector comprises elements needed for transcription, such as a promoter, e.g. an RNA polymerase promoter. Preferably, the vector is suitable for transcription using eukaryotic, prokaryotic, viral or phage transcription systems, such as eukaryotic cells, prokaryotic cells, or eukaryotic, prokaryotic, viral or phage *in vitro* transcription systems. Thus, for example, the vector may comprise a promoter sequence, which is recognized by a polymerase, such as by an RNA polymerase, e.g. by a eukaryotic, prokaryotic, viral, or phage RNA polymerase. In a preferred embodiment, the vector comprises a phage RNA polymerase promoter such as an SP6 or T7, preferably a T7 promoter. Preferably, the vector is suitable for *in vitro* transcription using a phage based *in vitro* transcription system, such as a T7 RNA polymerase based *in vitro* transcription system.

The vector may further comprise a poly(A) sequence and/or a polyadenylation signal as described above for the artificial nucleic acid molecule according to the present invention.

The vector may be an RNA vector or a DNA vector. Preferably, the vector is a DNA vector. The vector may be any vector known to the skilled person, such as a viral vector or a plasmid vector. Preferably, the vector is a plasmid vector, preferably a DNA plasmid vector. In a preferred embodiment, the vector according to the present invention comprises the artificial nucleic acid molecule according to the present invention.

Preferably, a vector according to the present invention comprises a sequence according to SEQ ID NOs. 1-1363, 1395, 1421, 1422, 1368, or 1412-1420, or a sequence having an identity of at least about 80%, more preferably of at least about 90%, even more preferably of at least about 95%; even more preferably of at least about 99%; even more preferably of 100% sequence identity to a sequence according to any one of SEQ ID NOs. 1-1363, 1395, 1421, 1422, 1368, or 1412-1420, or a fragment thereof, preferably a functional fragment thereof, or a corresponding RNA sequence.

Preferably, a vector, such as a DNA vector, according to the present invention comprises a sequence according to SEQ ID NOs. 1368-1392 or 1412-1420, or a sequence having an identity of at least about 80%, more preferably of at least about 90%, even more preferably of at least about 95%; even more preferably of at least about 99%; even more preferably of 100% sequence identity to a sequence according to any one of SEQ ID NOs. 1368-1392 or 1412-1420 or a fragment thereof, preferably a functional fragment thereof, or a corresponding RNA sequence.

Preferably, the vector is a circular molecule. Preferably, the vector is a double-stranded molecule, such as a double stranded DNA molecule. Such circular, preferably double stranded DNA molecule may be used conveniently as a storage form for the inventive artificial nucleic acid molecule. Furthermore, it may be used for transfection of cells, for example, cultured cells. Also it may be used for *in vitro* transcription for obtaining an artificial RNA molecule according to the invention.

Preferably, the vector, preferably the circular vector, is linearizable, for example, by restriction enzyme digestion. In a preferred embodiment, the vector comprises a cleavage site, such as a restriction site, preferably a unique cleavage site, located immediately 3' to the ORF, or - if present - immediately 3' to the 3'UTR element, or - if present - immediately 3' to the poly(A) sequence or polyadenylation signal, or - if present - located 3' to the poly(C) sequence, or - if present - located 3' to the histone stem-loop". Thus, preferably, the product obtained by linearizing the vector terminates at the 3'end with the stop codon, or - if present - the 3'-end of the 3'UTR element, or - if present - with the 3'-end of the poly(A) sequence or with the 3'-end of the polyadenylation signal, or - if present - with the 3'-end of the poly(C) sequence, or - if present - with the 3'-end of the histone stem-loop, plus optionally some nucleotides remaining from the restriction site after cleavage.

In a further aspect, the present invention relates to a cell comprising the artificial nucleic acid molecule according to the present invention or the vector according to present invention. The cell may be any cell, such as a bacterial cell, insect cell, plant cell, vertebrate cell, e.g. a mammalian cell. Such cell may be, e.g., used for replication of the vector of the present invention, for example, in a bacterial cell. Furthermore, the cell may be used for transcribing the artificial nucleic acid molecule or the vector according to the present invention and/or translating the open reading frame of the artificial nucleic acid molecule or the vector according to the present invention. For example, the cell may be used for recombinant protein production.

The cells according to the present invention are, for example, obtainable by standard nucleic acid transfer methods, such as standard transfection methods. For example, the artificial nucleic acid molecule or the vector according to the present invention may be transferred into the cell by electroporation, lipofection, e.g. based on cationic lipids and/or liposomes, calcium phosphate precipitation, nanoparticle based transfection, virus based transfection, or based on cationic polymers, such as DEAE-dextran or polyethylenimine etc.

Preferably, the cell is a mammalian cell, such as a cell of human subject, a domestic animal, a laboratory animal, such as a mouse or rat cell. Preferably the cell is a human cell. The cell may be a cell of an established cell line, such as a CHO, BHK, 293T, COS-7, HELA, HEK etc. cell, or the cell may be a primary cell, e.g. a HDF cell, preferably a cell isolated from an organism. In a preferred embodiment, the cell is an isolated cell of a mammalian subject, preferably of a human subject. For example, the cell may be an immune cell, such as a dendritic cell, a cancer or tumor cell, or any somatic cell etc., preferably of a mammalian subject, preferably of a human subject.

In a further aspect, the present invention provides a pharmaceutical composition comprising the artificial nucleic acid molecule according to the present invention, the vector according the present invention, or the cell according to the present invention. The pharmaceutical composition according to the invention may be used, e.g., as a vaccine, for example, for genetic vaccination. Thus, the ORF may, e.g., encode an antigen to be administered to a patient for vaccination. Thus, in a preferred embodiment, the pharmaceutical composition according to the present invention is a vaccine. Furthermore, the pharmaceutical composition according to the present invention may be used, e.g., for gene therapy.

Preferably, the pharmaceutical composition further comprises one or more pharmaceutically acceptable excipients, vehicles, fillers and/or diluents. In the context of the present invention, a pharmaceutically acceptable vehicle typically includes a liquid or non-liquid basis for the inventive pharmaceutical composition. In one embodiment, the pharmaceutical composition is provided in liquid form. In this context, preferably, the vehicle is based on water, such as pyrogen-free water, isotonic saline or buffered (aqueous) solutions, e.g. phosphate, citrate etc. buffered solutions. The buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of mammalian cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

One or more compatible solid or liquid fillers or diluents or encapsulating compounds suitable for administration to a patient may be used as well for the inventive pharmaceutical composition. The term "compatible" as used herein preferably means that these components of the inventive pharmaceutical composition are capable of being mixed with the inventive nucleic acid, vector or cells as defined herein in such a manner that no interaction occurs which would substantially reduce the pharmaceutical effectiveness of the inventive pharmaceutical composition under typical use conditions.

The pharmaceutical composition according to the present invention may optionally further comprise one or more additional pharmaceutically active components. A pharmaceutically active component in this context is a compound that exhibits a therapeutic effect to heal, ameliorate or prevent a particular indication or disease. Such compounds include, without implying any limitation, peptides or proteins, nucleic acids, (therapeutically active) low molecular weight organic or inorganic compounds (molecular weight less than 5000, preferably less than 1000), sugars, antigens or antibodies, therapeutic agents already known in the prior art, antigenic cells, antigenic cellular fragments, cellular fractions, cell wall components (e.g. polysaccharides), modified, attenuated or de-activated (e.g. chemically or by irradiation) pathogens (virus, bacteria etc.).

Furthermore, the inventive pharmaceutical composition may comprise a carrier for the artificial nucleic acid molecule or the vector. Such a carrier may be suitable for mediating dissolution in physiological acceptable liquids, transport and cellular uptake of the pharmaceutical active artificial nucleic acid molecule or the vector. Accordingly, such a carrier may be a component, which may be suitable for depot and delivery of an artificial nucleic acid molecule or vector according to the invention. Such components may be, for example, cationic or polycationic carriers or compounds which may serve as transfection or complexation agent.

Particularly preferred transfection or complexation agents in this context are cationic or polycationic compounds, including protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, prolin-rich peptides, arginine-rich peptides, lysine-rich peptides, MPG-peptide(s), Pep-1, L-oligomers, Calcitonin peptide(s), Antennapedia-derived peptides (particularly from *Drosophila antennapedia*), pAntp, pIsl, FGF, Lactoferrin, Transportan, Buforin-2, Bac715-24, SynB, SynB(1), pVEC, hCT-derived peptides, SAP, or histones.

Furthermore, such cationic or polycationic compounds or carriers may be cationic or polycationic peptides or proteins, which preferably comprise or are additionally modified to comprise at least one -SH moiety. Preferably, a cationic or polycationic carrier is selected from cationic peptides having the following sum formula (I):

$$\{(Arg)_l;(Lys)_m;(His)_n;(Orn)_o;(Xaa)_x\}; \qquad \text{formula (I)}$$

wherein $l + m + n + o + x = 3\text{-}100$, and $l$, $m$, $n$ or $o$ independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100 provided that the overall content of Arg (Arginine), Lys (Lysine), His (Histidine) and Orn (Ornithine) represents at least 10% of all amino acids of the oligopeptide; and Xaa is any amino acid selected from native (= naturally occurring) or non-native amino acids except of Arg, Lys, His or Orn; and x is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the oligopeptide. Any of amino acids Arg, Lys, His, Orn and Xaa may be positioned at any place of the peptide. In this context cationic peptides or proteins in the range of 7-30 amino acids are particular preferred.

Further, the cationic or polycationic peptide or protein, when defined according to formula $\{(Arg)_l;(Lys)_m;(His)_n;(Orn)_o;(Xaa)_x\}$ (formula (I)) as shown above and which comprise or are additionally modified to comprise at least one -SH moiety, may be, without being restricted thereto, selected from subformula (Ia);

$$\{(Arg)_l;(Lys)_m;(His)_n;(Orn)_o;(Xaa')_x(Cys)_y\} \qquad \text{subformula (Ia)}$$

wherein $(Arg)_l;(Lys)_m;(His)_n;(Orn)_o$; and x are as defined herein, Xaa' is any amino acid selected from native (= naturally occurring) or non-native amino acids except of Arg, Lys, His, Orn or Cys and y is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 and 81-90, provided that the overall content of Arg (Arginine), Lys (Lysine), His (Histidine) and Orn (Ornithine) represents at least 10% of all amino acids of the oligopeptide. Further, the cationic or polycationic peptide may be selected from subformula (Ib):

$$Cys_1 \{(Arg)_l;(Lys)_m;(His)_n;(Orn)_o;(Xaa)_x\} Cys_2 \qquad \text{subformula (Ib)}$$

wherein empirical formula $\{(Arg)_l;(Lys)_m;(His)_n;(Orn)_o;(Xaa)_x\}$ (formula (III)) is as defined herein and forms a core of an amino acid sequence according to (semiempirical) formula (III) and wherein $Cys_1$ and $Cys_2$ are Cysteines proximal to, or terminal to $(Arg)_l;(Lys)_m;(His)_n;(Orn)_o;(Xaa)_x$.

Further preferred cationic or polycationic compounds, which can be used as transfection or complexation agent may include cationic polysaccharides, for example chitosan, polybrene, cationic polymers, e.g. polyethyleneimine (PEI), cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-($\alpha$-trimethylammonio-acetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as $\beta$-aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, chitosan, etc., silan backbone based polymers, such as PMOXA-PDMS copolymers, etc., blockpolymers consisting of a combination of one or more cationic blocks (e.g. selected from a cationic polymer as mentioned above) and of one or more hydrophilic or hydrophobic blocks (e.g polyethyleneglycole); etc.

In this context, it is particularly preferred that the inventive artificial nucleic acid molecule or the inventive vector is complexed at least partially with a cationic or polycationic compound, preferably cationic proteins or peptides. Partially means that only a part of the inventive artificial nucleic acid molecule or the inventive vector is complexed with a cationic or polycationic compound and that the rest of the inventive artificial nucleic acid molecule or the inventive vector is in uncomplexed form ("free"). Preferably the ratio of complexed nucleic acid to: free nucleic acid is selected from a range. of about 5:1 (w/w) to about 1:10 (w/w), more preferably from a range of about 4:1 (w/w) to about 1:8 (w/w), even more preferably from a range of about 3:1 (w/w) to about 1:5 (w/w) or 1:3 (w/w), and most preferably the ratio of complexed nucleic acid to free nucleic acid is selected from a ratio of about 1:1 (w/w).

The pharmaceutical composition according to the present invention may optionally further comprise one or more adjuvants, for example, adjuvants for stimulating the innate immune system or for enhancing cellular uptake of the artificial nucleic acid molecule or vector. In this context, an adjuvant may be understood as any compound, which is suitable to initiate or increase an immune response of the innate immune system, i.e. a non-specific immune response.

In other words, when administered, the inventive pharmaceutical composition preferably elicits an innate immune response due to the adjuvant, optionally contained therein. Preferably, such an adjuvant may be an adjuvant supporting the induction of an innate immune response in a mammal. Such an adjuvant may be, for example, an immunostimulatory nucleic acid, i.e. a nucleic acid that may bind to a Toll-like-receptor or the like, preferably an immunostimulatory RNA.

Such adjuvants, preferably such immunostimulatory nucleic acids, may induce an innate, i.e. unspecific, immune response which may support a specific, i.e. adaptive, immune response to the peptide or protein, i.e. the antigen, encoded by the artificial nucleic acid molecule of the pharmaceutical composition, preferably the vaccine.

The inventive pharmaceutical composition may also additionally comprise any further compound, which is known to be immunostimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

Further additives which may be included in the inventive pharmaceutical composition are, e.g., emulsifiers, such as, for example, Tween®; wetting agents, such as, for example, sodium lauryl sulfate; colouring agents; taste-imparting agents, pharmaceutical carriers; tablet-forming agents; stabilizers; antioxidants; preservatives etc.

The pharmaceutical composition according to the present invention preferably comprises a "safe and effective amount" of the components of the pharmaceutical composition, particularly of the inventive nucleic acid sequence, the vector and/or the cells as defined herein. As used herein, a "safe and effective amount" means an amount sufficient to significantly induce a positive modification of a disease or disorder as defined herein. At the same time, however, a "safe and effective amount" preferably avoids serious side-effects and permits a sensible relationship between advantage and risk. The determination of these limits typically lies within the scope of sensible medical judgment.

In a further aspect, the present invention provides the artificial nucleic acid molecule according to the present invention, the vector according to the present invention, the cell according to the present invention, or the pharmaceutical composition according to the present invention for use as a medicament, for example, as vaccine (in genetic vaccination) or in gene therapy.

The artificial nucleic acid molecule according to the present invention, the vector according to the present invention, the cell according to the present invention, or the pharmaceutical composition according to the present invention are particularly suitable for any medical application which makes use of the therapeutic action or effect of peptides, polypeptides or proteins, or where supplementation of a particular peptide or protein is needed. Thus, the present invention provides the artificial nucleic acid molecule according to the present invention, the vector according to the present invention, the cell according to the present invention, or the pharmaceutical composition according to the present invention for use in the treatment or prevention of diseases or disorders amenable to treatment by the therapeutic action or effect of peptides, polypeptides or proteins or amenable to treatment by supplementation of a particular peptide, polypeptide or protein. For example, the artificial nucleic acid molecule according to the present invention, the vector according to the present invention, the cell according to the present invention, or the pharmaceutical composition according to the present invention may be used for the treatment or prevention of genetic diseases, autoimmune diseases, cancerous or tumour-related diseases, infectious diseases, chronic diseases or the like, e.g., by genetic vaccination or gene therapy.

In particular, such therapeutic treatments, which benefit from a stable, prolonged and/or increased presence of therapeutic peptides, polypeptides or proteins in a subject to be treated are especially suitable as medical application in the context of the present invention, since the 5'UTR element optionally in combination with the 3'UTR element provides for increased protein expression from the ORF and the 3'UTR element provides for a stable and prolonged expression of the ORF of the inventive nucleic acid molecule. Thus, a particularly suitable medical application for the artificial nucleic acid molecule according to the present invention, the vector according to the present invention, the cell according to the present invention, or the pharmaceutical composition according to the present invention is vaccination, for example against infections or tumours. Thus, the present invention provides the artificial nucleic acid molecule according to the present invention, the vector according to the present invention, the cell according to the present invention, or the pharmaceutical composition according to the present invention for vaccination of a subject, preferably a mammalian subject, more preferably a human subject. Preferred vaccination treatments are vaccination against infectious diseases, such as bacterial, protozoal or viral infections, and anti-tumour-vaccination. Such vaccination treatments may be prophylactic or therapeutic.

Depending on the disease to be treated or prevented, the ORF may be selected. For example, the open reading frame may code for a protein that has to be supplied to a patient suffering from total lack or at least partial loss of function of a protein, such as a patient suffering from a genetic disease. Additionally, the open reading frame may be chosen from an ORF coding for a peptide or protein which beneficially influences a disease or the condition of a subject. Furthermore, the open reading frame may code for a peptide or protein which effects down-regulation of a pathological overproduction of a natural peptide or protein or elimination of cells expressing pathologically a protein or peptide. Such lack, loss of function or overproduction may, e.g., occur in the context of tumour and neoplasia, autoimmune diseases, allergies, infections, chronic diseases or the like. Furthermore, the open reading frame may code for an antigen or immunogen, e.g. for an epitope of a pathogen or for a tumour antigen. Thus, in preferred embodiments, the artificial nucleic acid molecule or the vector according to the present invention comprises an ORF encoding an amino acid sequence comprising or consisting of an antigen or immunogen, e.g. an epitope of a pathogen or a tumour-associated antigen, a 5'UTR element as described above, and optional further components, such as a 3'UTR element and/or a poly(A) sequence etc. as described above.

In the context of medical application, in particular, in the context of vaccination, it is preferred that the artificial nucleic acid molecule according to the present invention is RNA, preferably mRNA, since DNA harbours the risk of eliciting an anti-DNA immune response and tends to insert into genomic DNA. However, in some embodiments, for example, if a viral delivery vehicle, such as an adenoviral delivery vehicle is used for delivery of the artificial nucleic acid molecule or the vector according to the present invention, e.g., in the context of gene therapeutic treatments, it may be desirable that the artificial nucleic acid molecule or the vector is a DNA molecule.

The artificial nucleic acid molecule according to the present invention, the vector according to the present invention, the cell according to the present invention, or the pharmaceutical composition according to the present invention may be administered orally, parenterally, by inhalation spray, topically, rectally, nasally, buccally, vaginally or via an implanted reservoir. The term parenteral as used herein includes subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or infusion techniques.

Preferably, the artificial nucleic acid molecule according to the present invention, the vector according to the present invention, the cell according to the present invention, or the pharmaceutical composition according to the present invention is administered parenterally, e.g. by parenteral injection, more preferably by subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, sublingual injection or via infusion techniques. Particularly preferred is intradermal and intramuscular injection. Sterile injectable forms of the inventive pharmaceutical composition may be aqueous or oleaginous suspension. These suspensions may be formulated according to techniques known in the art using suitable dispersing or wetting agents and suspending agents.

The artificial nucleic acid molecule according to the present invention, the vector according to the present invention, the cell according to the present invention, or the pharmaceutical composition according to the present invention may also be administered orally in any orally acceptable dosage form including, but not limited to, capsules, tablets, aqueous suspensions or solutions.

The artificial nucleic acid molecule according to the present invention, the vector according to the present invention, the cell according to the present invention, or the pharmaceutical composition according to the present invention may also be administered topically, especially when the target of treatment includes areas or organs readily accessible by topical application, e.g. including diseases of the skin or of any other accessible epithelial tissue. Suitable topical formulations are readily prepared for each of these areas or organs. For topical applications, the artificial nucleic acid molecule according to the present invention, the vector according to the present invention, the cell according to the present invention, or the pharmaceutical composition according to the present invention may be formulated in a suitable ointment suspended or dissolved in one or more carriers.

In one embodiment, the use as a medicament comprises the step of transfection of mammalian cells, preferably *in vitro* transfection of mammalian cells, more preferably *in vitro* transfection of isolated cells of a subject to be treated by the medicament. If the use comprises the *in vitro* transfection of isolated cells, the use as a medicament may further comprise the (re)administration of the transfected cells to the patient. The use of the inventive artificial nucleic acid molecules or the vector as a medicament may further comprise the step of selection of successfully transfected isolated cells. Thus, it may be beneficial if the vector further comprises a selection marker. Also, the use as a medicament may comprise *in vitro* transfection of isolated cells and purification of an expression-product, i.e. the encoded peptide or protein from these cells. This purified peptide or protein may subsequently be administered to a subject in need thereof.

The present invention also provides a method for treating or preventing a disease or disorder as described above comprising administering the artificial nucleic acid molecule according to the present invention, the vector according to the present invention, the cell according to the present invention, or the pharmaceutical composition according to the present invention to a subject in need thereof.

Furthermore, the present invention provides a method for treating or preventing a disease or disorder comprising transfection of a cell with an artificial nucleic acid molecule according to the present invention or with the vector according to the present invention. Said transfection may be performed *in vitro* or *in vivo.* In a preferred embodiment, transfection of a cell is performed *in vitro* and the transfected cell is administered to a subject in need thereof, preferably to a human patient. Preferably, the cell which is to be transfected *in vitro* is an isolated cell of the subject, preferably of the human patient. Thus, the present invention provides a method of treatment comprising the steps of isolating a cell from a subject, preferably from a human patient, transfecting the isolated cell with the artificial nucleic acid molecule according to the present invention or the vector according to the present invention, and administering the transfected cell to the subject, preferably the human patient.

The method of treating or preventing a disorder according to the present invention is preferably a vaccination method and/or a gene therapy method as described above.

As described above, the 5'UTR element and the 3'UTR element are capable of increasing protein production from an artificial nucleic acid molecule, such as an mRNA or vector, comprising the 5'UTR element an ORF and the 3'UTR. Also disclosed is a method for increasing protein production from an artificial nucleic acid molecule comprising the step of associating the artificial nucleic acid molecule, preferably the ORF contained within the artificial nucleic acid molecule, with (i) at least one 5'-untranslated region element (5'UTR element), which comprises or consists of a nucleic acid sequence which is derived from the 5'UTR of a TOP gene or which is derived from a variant of the 5'UTR of a TOP gene as described above and (ii) optionally at least one 3'UTR element, which comprises or consists of a nucleic acid sequence derived from the 3'UTR of a chordate gene or a vertebrate gene or a mammalian gene or a human gene, or from a variant of the 3'UTR of a chordate gene or a vertebrate gene or a mammalian gene, or a human gene as described above.

The term "associating the artificial nucleic acid molecule or the vector with a 5'UTR element and an (optional) 3'UTR element" typically means functionally associating or functionally combining the artificial nucleic acid molecule, such as the mRNA or the vector, with the 5'UTR element and the 3'UTR element. This means that the artificial nucleic acid molecule, preferably the ORF contained within the artificial nucleic acid molecule, the 5'UTR element and the 3'UTR element as described above are associated or coupled such that the function of the 5'UTR element and the 3'UTR element, e.g., protein production increasing function, is exerted. Typically, this means that the 5'UTR element and the 3'UTR element are integrated into the artificial nucleic acid molecule, preferably into the mRNA molecule or the vector, such that the open reading frame is positioned 3' to the 5'UTR element, preferably between the 5'UTR element and the optional 3'UTR element.

Also disclosed is the use of at least one 5'-untranslated region element (5'UTR element), which comprises or consists of a nucleic acid sequence, which is derived from the 5'UTR of a TOP gene or which is derived from a variant of the 5'UTR of a TOP gene as described above and optionally at least one 3'UTR element, which comprises or consists of a nucleic acid sequence derived from the 3'UTR of a chordate gene, or a vertebrate gene, or a mammalian gene, or a human gene, or from a variant of the 3'UTR of a chordate gene or a vertebrate gene, or a mammalian gene, or a human gene as described above for increasing protein production from an artificial nucleic acid molecule, such as an mRNA or a vector.

The method for increasing protein production from an artificial nucleic acid molecule and the above use may also comprise associating the artificial nucleic acid molecules with one or more further elements, such as a polyadenylation signal, a poly(A) sequence, a poly(C) sequence and/or a histone stem loop as described above.

The compounds and ingredients of the inventive pharmaceutical composition may also be manufactured and traded separately of each other. Thus, a kit or kit of parts comprising an artificial nucleic acid molecule according to the invention, a vector according the invention, a cell according to the invention, and/or a pharmaceutical composition according to the invention is also disclosed. Such a kit or kit of parts may, additionally, comprise instructions for use, cells for transfection, an adjuvant, a means for administration of the pharmaceutical composition, a pharmaceutically acceptable carrier and/or an pharmaceutically acceptable solution for dissolution or dilution of the artificial nucleic acid molecule, the vector, the cells or the pharmaceutical composition.

The following Figures, Sequences and Examples are intended to illustrate the invention further. They are not intended to limit the subject-matter of the invention thereto.

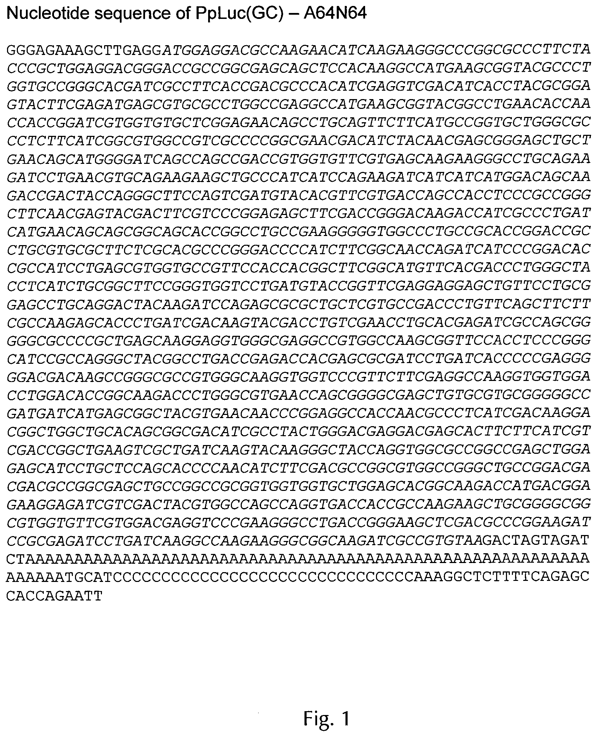

Fig. 1      shows the nucleotide sequence of a *Photinus pyralis* luciferase encoding nucleic acid molecule PpLuc(GC) - A64N64. This artificial construct does not comprise a 5'UTR element or a 3'UTR element in the sense of the present invention. The coding region for PpLuc(GC) is depicted in italics.

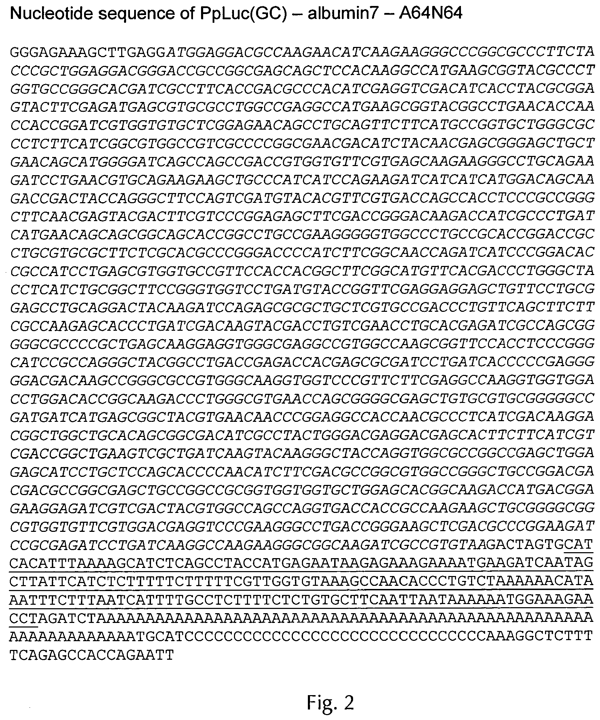

Fig. 2      shows the nucleotide sequence of PpLuc(GC) - albumin7 - A64N64 (not according to the invention). The 3'UTR of human albumin, with a T7 termination signal as well as a HindIII and XbaI restriction site removed by three single point mutations, was inserted between the ORF and poly(A) of the construct shown in Figure 1. The coding region for PpLuc(GC) is depicted in italics. The albumin 3'UTR is underlined.

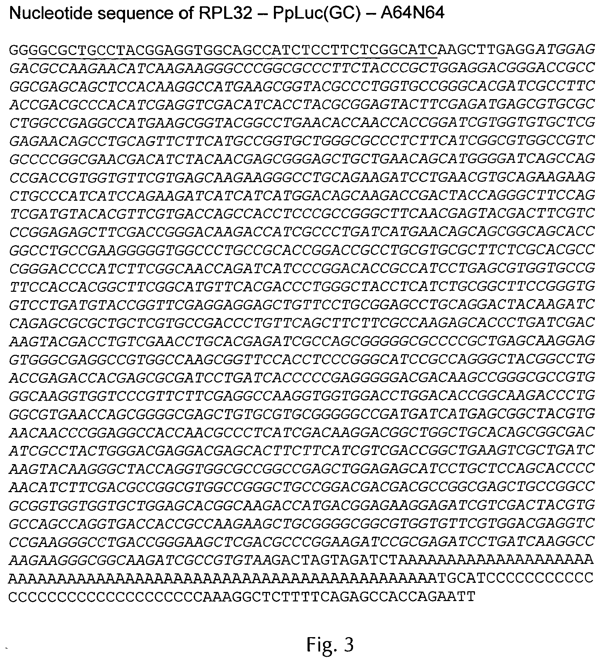

Fig. 3      shows the nucleotide sequence of RPL32 - PpLuc(GC) - A64N64 (not according to the invention). The 5'UTR of human ribosomal protein Large 32 gene lacking the 5' terminal oligopyrimidine tract (RPL32) according to SEQ ID NO. 1368 was inserted 5' of the ORF in the construct shown in Figure 1. The coding region for PpLuc(GC) is depicted in italics. The RPL32 5'UTR is underlined.

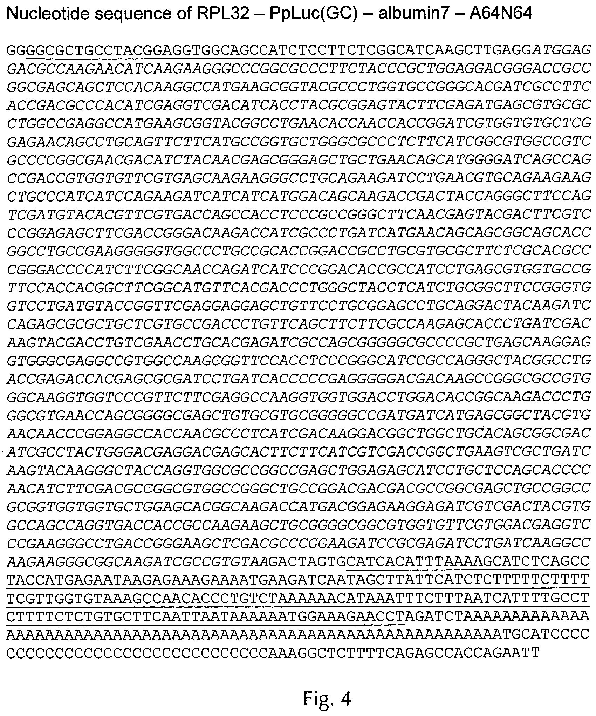

Fig. 4      shows the nucleotide sequence of RPL32 - PpLuc(GC) - albumin7 - A64N64. The 5'UTR of human ribosomal protein Large 32 gene lacking the 5' terminal oligopyrimidine tract (RPL32) according to SEQ ID NO. 1368 and the albumin7 3'UTR element according to SEQ ID NO. 1376 were inserted 5' and 3' of the ORF in the construct shown in Figure 1, respectively.

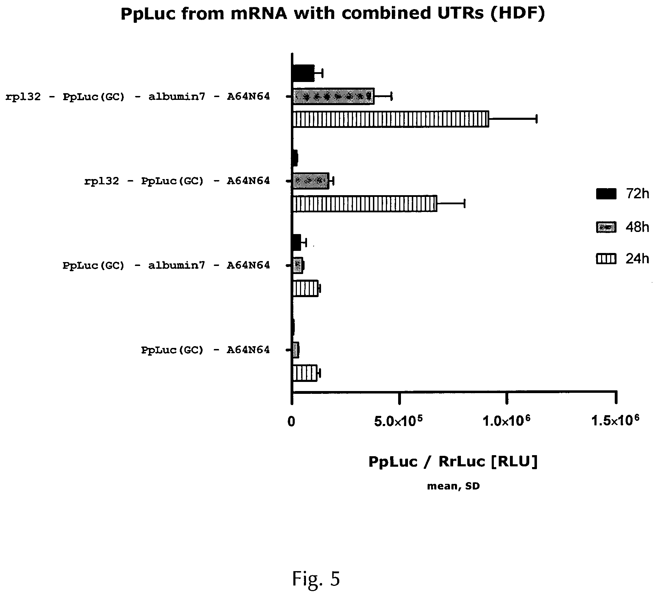

Fig. 5     is a graphical representation of the effect of the TOP 5'UTR element which is derived from the 5'UTR of the TOP gene RPL23 according to SEQ ID NO. 1368, the albumin 3' UTR element according to SEQ ID NO. 1376 and the combination of the TOP 5'UTR element and the albumin 3'UTR element on luciferase expression from mRNA. A variety of mRNAs were transfected into human dermal fibroblasts (HDF) by lipofection. Luciferase levels were measured at 24, 48, and 72 hours after transfection. The albumin 3'UTR element extends luciferase expression, while the TOP 5'UTR element increases luciferase levels compared to mRNA lacking 5'- and 3'UTR elements. Strikingly, the combination of TOP 5'UTR element and albumin 3'UTR element further strongly increases the luciferase level, much above the level observed with either of the individual elements, thus acting synergistically. Data are graphed as mean RLU $\pm$ SD (relative light units $\pm$ standard deviation) for triplicate transfections. RLU are summarized in Example 5.1.

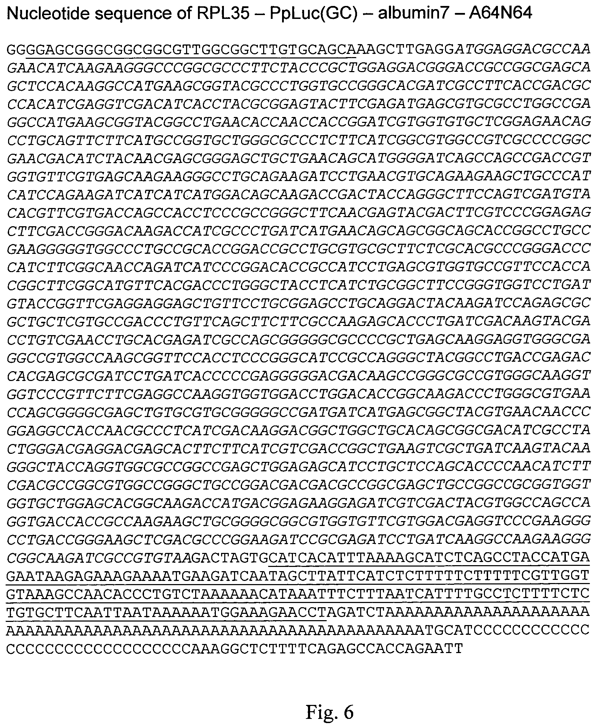

Fig. 6     shows the nucleotide sequence of RPL35 - PpLuc(GC) - albumin7 - A64N64. The 5'UTR of human ribosomal protein Large 35 gene lacking the 5' terminal oligopyrimidine tract (RPL35) according to SEQ ID NO. 1412 and the albumin7 3'UTR element according to SEQ ID NO. 1376 were inserted 5' and 3' of the ORF in the construct shown in Figure 1, respectively.

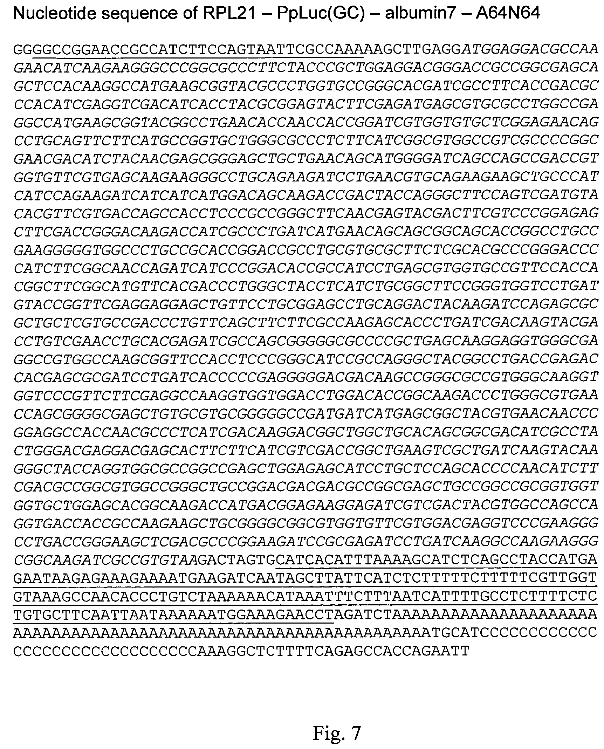

Fig. 7     shows the nucleotide sequence of RPL21 - PpLuc(GC) - albumin7 - A64N64. The 5'UTR of human ribosomal protein Large 21 gene lacking the 5' terminal oligopyrimidine tract (RPL21) according to SEQ ID NO. 1413 and the albumin7 3'UTR element according to SEQ ID NO. 1376 were inserted 5' and 3' of the ORF in the construct shown in Figure 1, respectively.

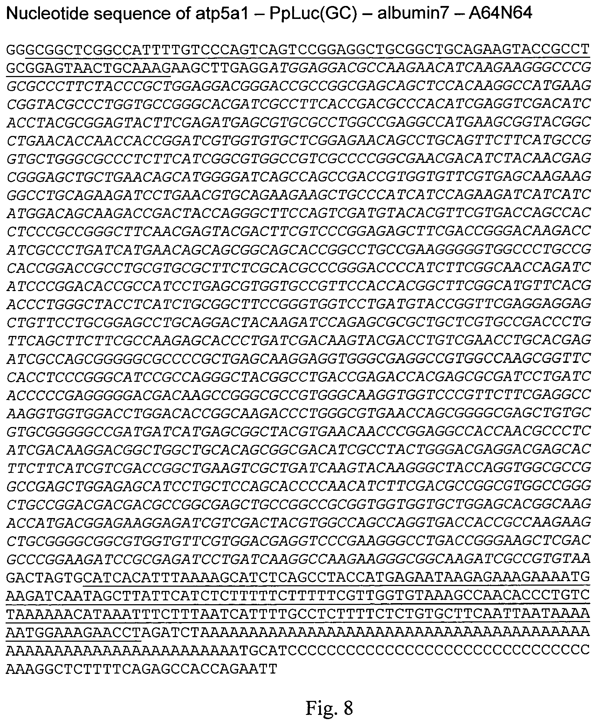

Fig. 8     shows the nucleotide sequence of atp5a1 - PpLuc(GC) - albumin7 - A64N64. The 5'UTR of human ATP synthase, H+ transporting, mitochondrial F1 complex, alpha subunit 1 gene lacking the 5' terminal oligopyrimidine tract (atp5a1) according to SEQ ID NO. 1414 and the albumin7 3'UTR element according to SEQ ID NO. 1376 were inserted 5' and 3' of the ORF in the construct shown in Figure 1, respectively.

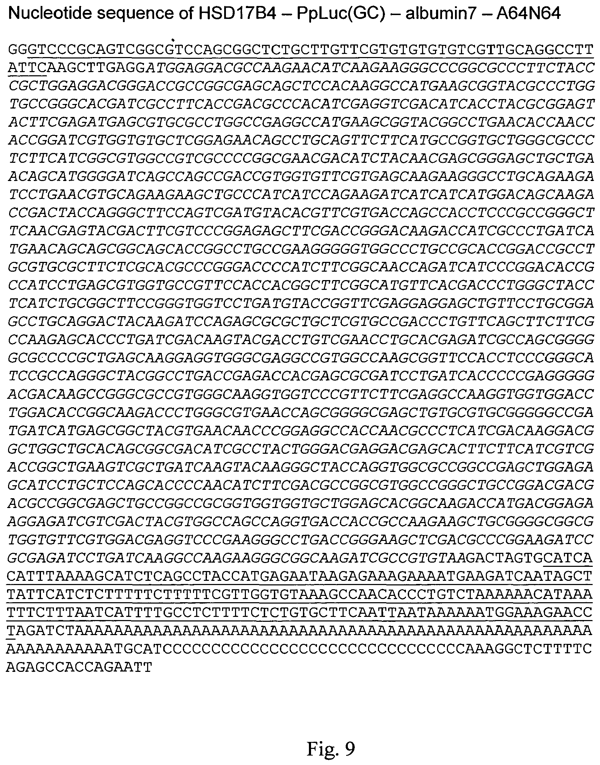

Fig. 9     shows the nucleotide sequence of HSD17B4 - PpLuc(GC) - albumin7 - A64N64. The 5'UTR of human hydroxysteroid (17-beta) dehydrogenase 4 gene lacking the 5' terminal oligopyrimidine tract (HSD17B4) according to SEQ ID NO. 1415 and the albumin7 3'UTR element according to SEQ ID NO. 1376 were inserted 5' and 3' of the ORF in the construct shown in Figure 1, respectively.

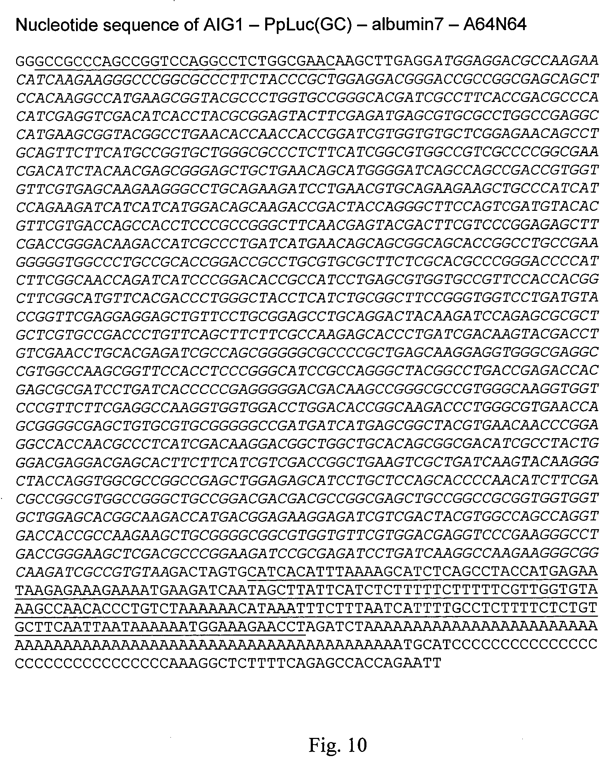

Fig. 10     shows the nucleotide sequence of AIG1 - PpLuc(GC) - albumin7 - A64N64. The 5'UTR of human androgen-induced 1 gene lacking the 5' terminal oligopyrimidine tract (AIG1) according to SEQ ID NO. 1416 and the albumin7 3'UTR element according to SEQ ID NO. 1376 were inserted 5' and 3' of the ORF in the construct shown in Figure 1, respectively.

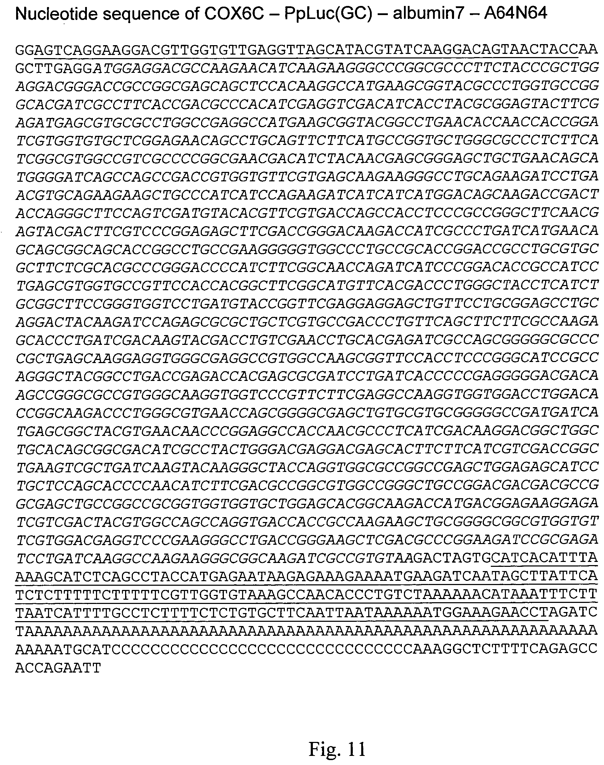

Fig. 11     shows the nucleotide sequence of COX6C - PpLuc(GC) - albumin7 - A64N64. The 5'UTR of human cytochrome c oxidase subunit VIc gene lacking the 5' terminal oligopyrimidine tract (COX6C) according to SEQ ID NO. 1417 and the albumin7 3'UTR element according to SEQ ID NO. 1376 were inserted 5' and 3' of the ORF in the construct shown in Figure 1, respectively.

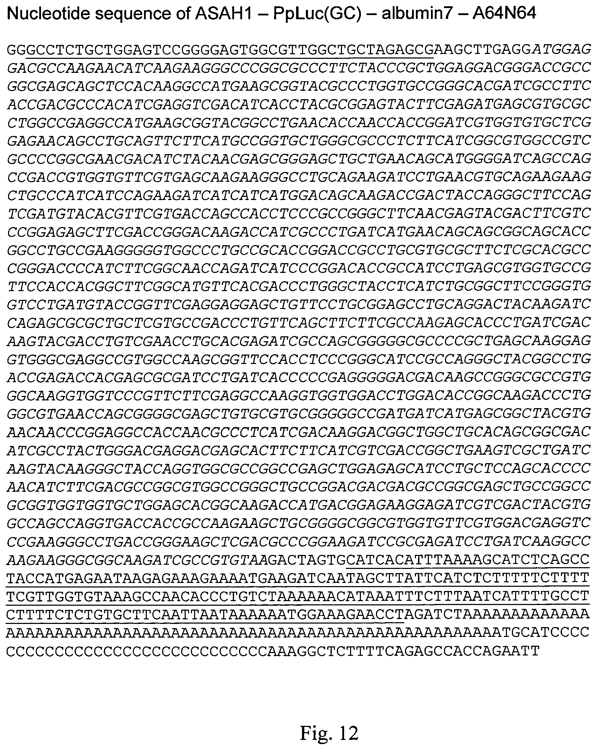

Fig. 12     shows the nucleotide sequence of ASAH1 - PpLuc(GC) - albumin7 - A64N64. The 5'UTR of human N-acylsphingosine amidohydrolase (acid ceramidase) 1 lacking the 5' terminal oligopyrimidine tract (ASAH1) according to SEQ ID NO. 1418 and the albumin7 3'UTR according to SEQ ID NO. 1376 were inserted 5' and 3' of the ORF in the construct shown in Figure 1, respectively.

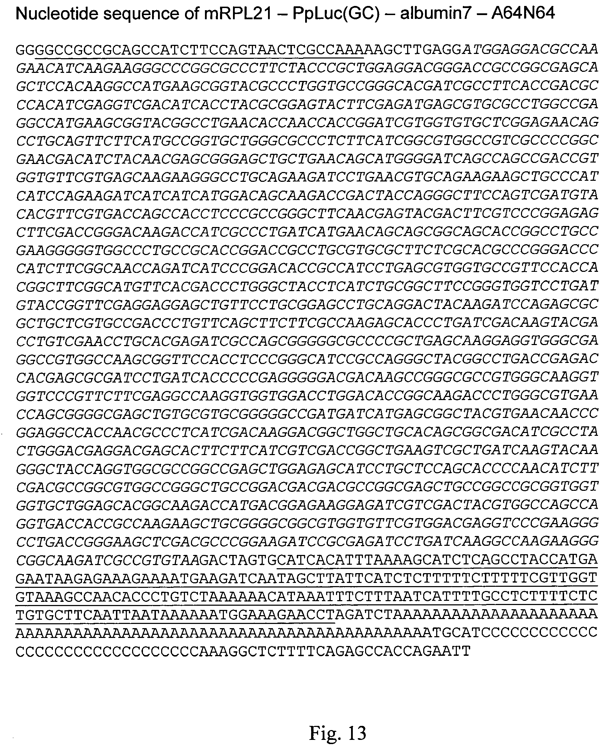

Fig. 13     shows the nucleotide sequence of mRPL21 - PpLuc(GC) - albumin7 - A64N64. The 5'UTR of murine ribosomal protein Large 21 gene lacking the 5' terminal oligopyrimidine tract (mRPL21) according to SEQ ID NO. 1419 and the albumin7 3'UTR element according to SEQ ID NO. 1376 were inserted 5' and 3' of the ORF in the construct shown in Figure 1, respectively.

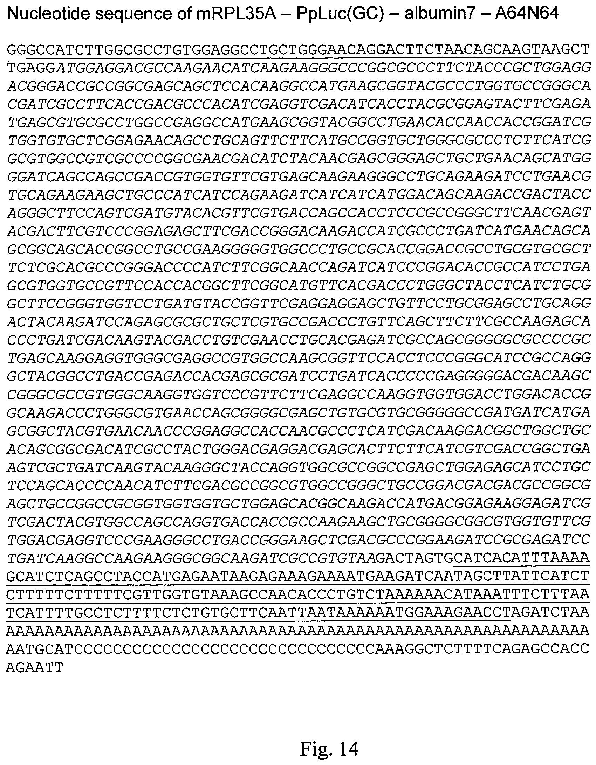

Fig. 14     shows the nucleotide sequence of mRPL35A - PpLuc(GC) - albumin7 - A64N64. The 5'UTR of murine ribosomal protein Large 35a gene lacking the 5' terminal oligopyrimidine tract (mRPL35A) according to SEQ ID NO. 1420 and the albumin7 3'UTR element according to SEQ ID NO. 1376 were inserted 5' and 3' of the ORF in the construct shown in Figure 1, respectively.

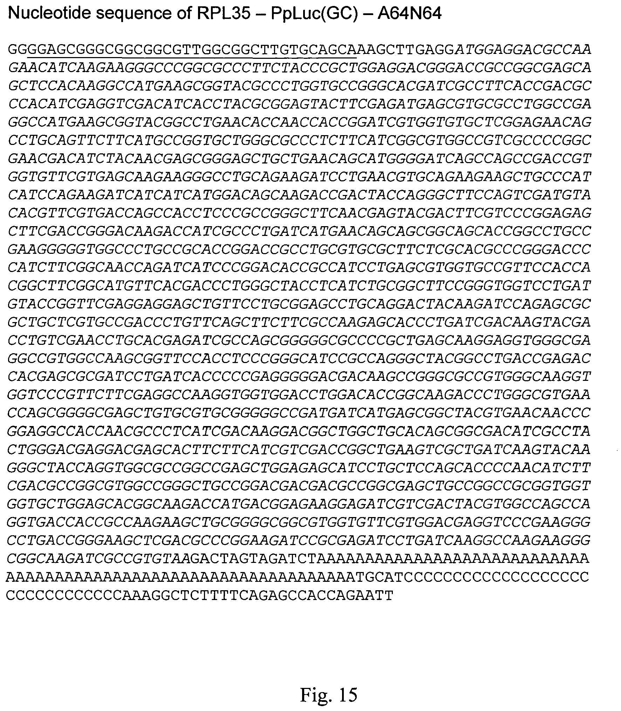

Fig. 15    shows the nucleotide sequence of RPL35 - PpLuc(GC) - A64N64 (not according to the invention). The 5'UTR of human ribosomal protein Large 35 gene lacking the 5' terminal oligopyrimidine tract (RPL35) according to SEQ ID NO. 1412 was inserted 5' of the ORF in the construct shown in Figure 1.

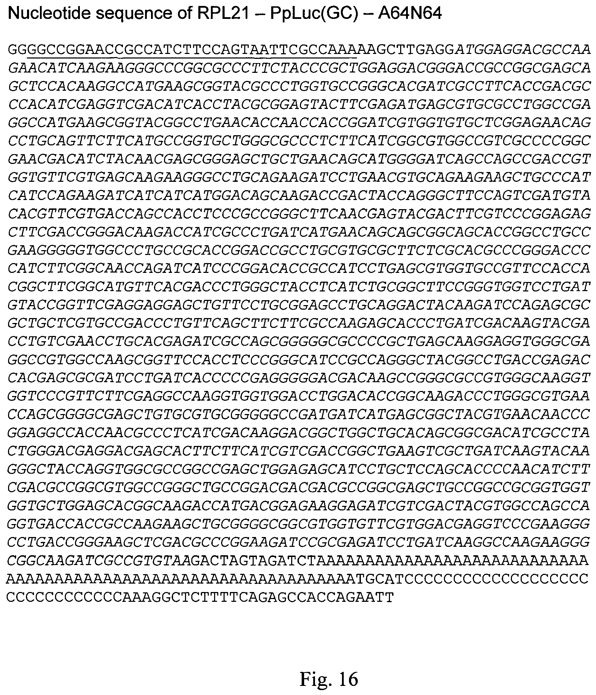

Fig. 16    shows the nucleotide sequence of RPL21 - PpLuc(GC) - A64N64 (not according to the invention). The 5'UTR of human ribosomal protein Large 21 gene lacking the 5' terminal oligopyrimidine tract (RPL21) according to SEQ ID NO. 1413 was inserted 5' of the ORF in the construct shown in Figure 1.

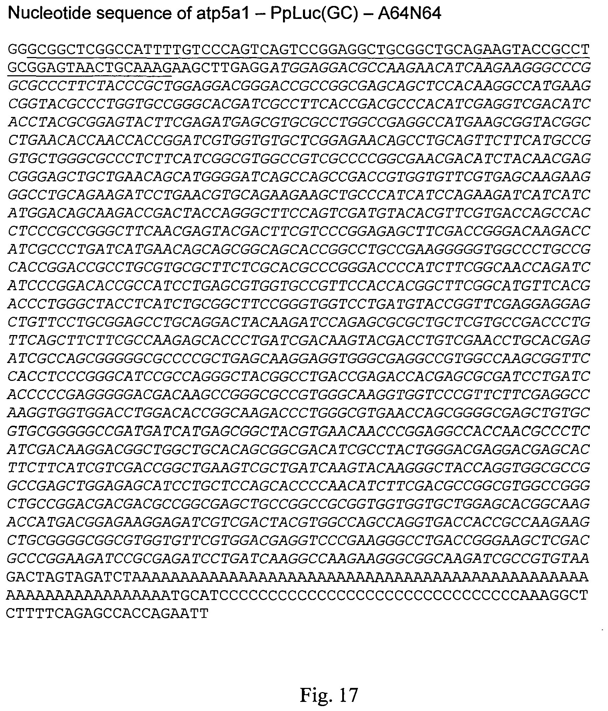

Fig. 17    shows the nucleotide sequence of atp5a1 - PpLuc(GC) - A64N64 (not according to the invention). The 5'UTR of human ATP synthase, H+ transporting, mitochondrial F1 complex, alpha subunit 1 gene lacking the 5' terminal oligopyrimidine tract (atp5a1) according to SEQ ID NO. 1414 was inserted 5' of the ORF in the construct shown in Figure 1.

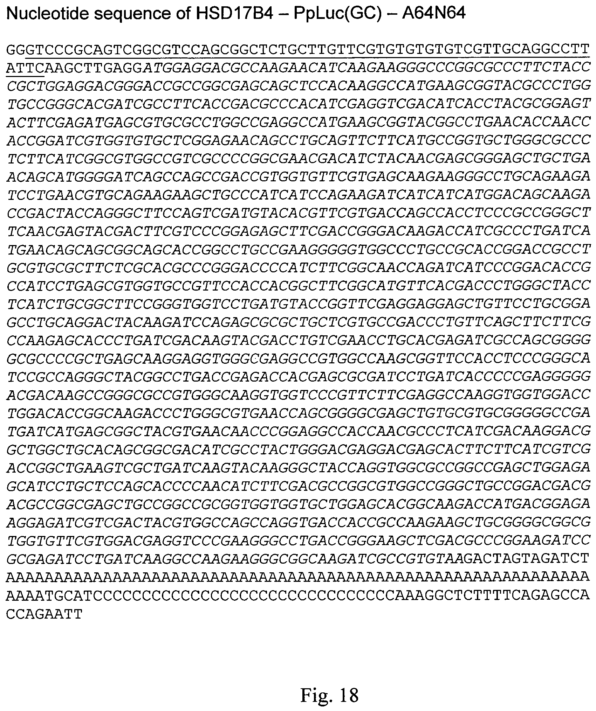

Fig. 18    shows the nucleotide sequence of HSD1 7B4 - PpLuc(GC) - A64N64 (not according to the invention). The 5'UTR of human hydroxysteroid (17-beta) dehydrogenase 4 gene lacking the 5' terminal oligopyrimidine tract (HSD17B4) according to SEQ ID NO. 1415 was inserted 5' of the ORF in the construct shown in Figure 1.

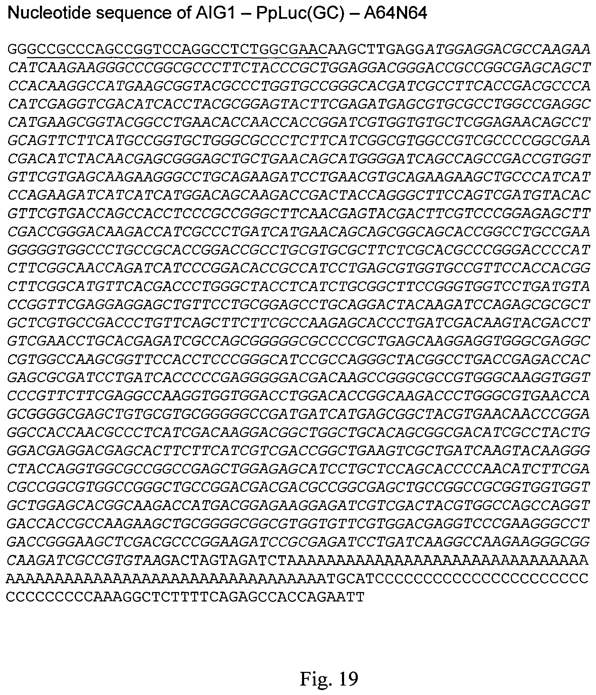

Fig. 19    shows the nucleotide sequence of AIG1 - PpLuc(GC) - A64N64 (not according to the invention). The 5'UTR of human androgen-induced 1 gene lacking the 5' terminal oligopyrimidine tract (AIG1) according to SEQ ID NO. 1416 was inserted 5' of the ORF in the construct shown in Figure 1.

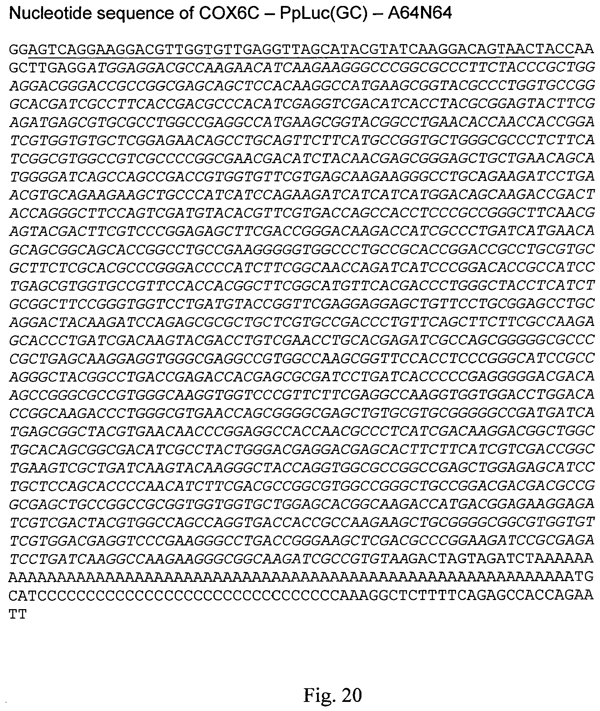

Fig. 20    shows the nucleotide sequence of COX6C - PpLuc(GC) - A64N64 (not according to the invention). The 5'UTR of human cytochrome c oxidase subunit VIc gene lacking the 5' terminal oligopyrimidine tract (COX6C) according to SEQ ID NO. 1417 was inserted 5' of the ORF in the construct shown in Figure 1.

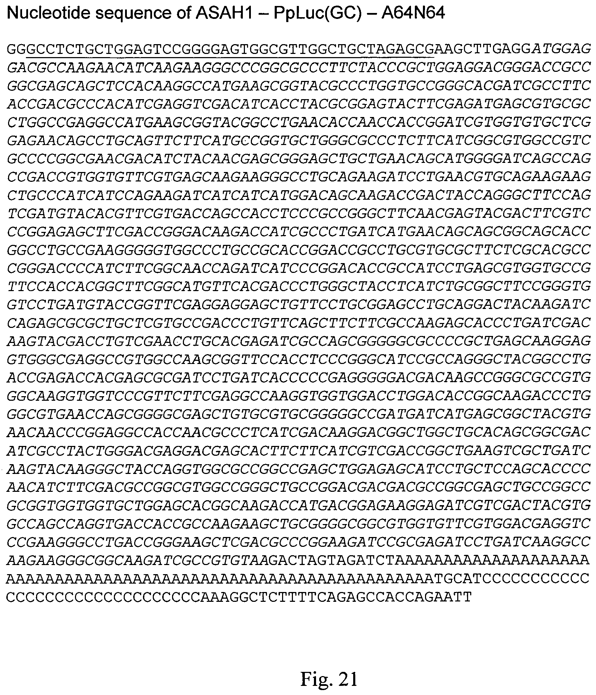

Fig. 21    shows the nucleotide sequence of ASAH1 - PpLuc(GC) - A64N64 (not according to the invention). The 5'UTR of human N-acylsphingosine amidohydrolase (acid ceramidase) 1 gene lacking the 5' terminal oligopyrimidine tract (ASAH1) according to SEQ ID NO. 1418 was inserted 5' of the ORF in the construct shown in Figure 1.

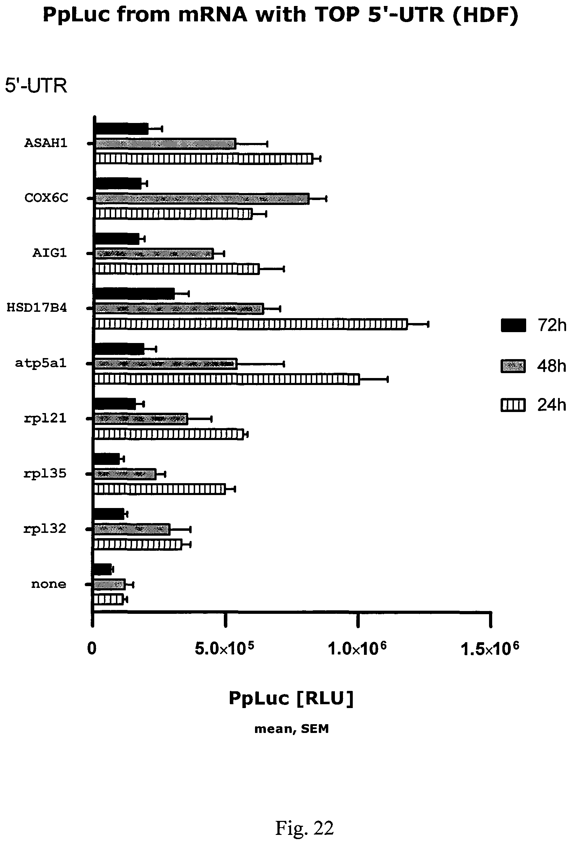

Fig. 22    is a graphical representation of the effect of different TOP 5'UTR elements on luciferase expression from mRNA. A variety of mRNAs were transfected into human dermal fibroblasts (HDF) by lipofection. Luciferase levels were measured at 24, 48, and 72 hours after transfection. TOP 5'UTR elements strongly increase luciferase levels compared to mRNA lacking a 5'UTR element. mRNAs comprising 5'UTR elements derived from the 5'UTRs of the TOP genes ASAH1, COX6C, AIG1, HSD17B4, atp5a1, RPL21, RPL35 and RPL32 were transfected into human dermal fibroblasts (HDF) by lipofection. Luciferase levels were measured at 24, 48, and 72 hours after transfection. The TOP 5'UTR elements increases luciferase levels compared to mRNA lacking a 5'UTR element. Data are graphed as mean RLU $\pm$ SEM (relative light units $\pm$ standard error) for triplicate transfections. RLU are summarized in Example 5.2.

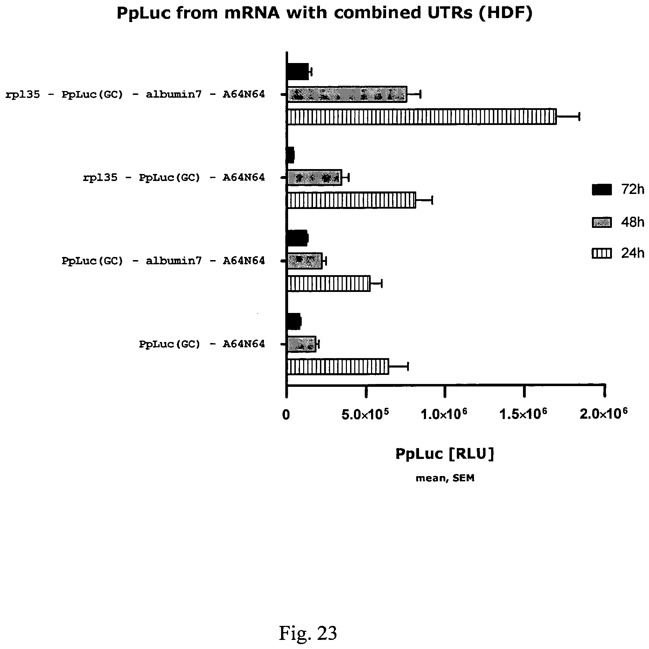

Fig. 23    is a graphical representation of the effect of the RPL35 TOP 5'UTR element, the albumin 3'UTR element and the combination of RPL35 TOP 5'UTR element and albumin 3'UTR element on luciferase expression from mRNA. A variety of mRNAs were transfected into human dermal fibroblasts (HDF) by lipofection. Luciferase levels were measured at 24, 48, and 72 hours after transfection. The albumin 3'UTR element extends luciferase expression, while the RPL35 TOP 5'UTR element increases luciferase levels compared to mRNA lacking 5'- and 3'UTR elements. Strikingly, the combination of RPL35 TOP 5'UTR element and albumin 3'UTR element further strongly increases the luciferase level, much above the level observed with either of the individual elements, thus acting synergistically. Data are graphed as mean RLU $\pm$ SEM (relative light units $\pm$ standard error) for triplicate transfections. Synergy is summarized in Example 5.3.

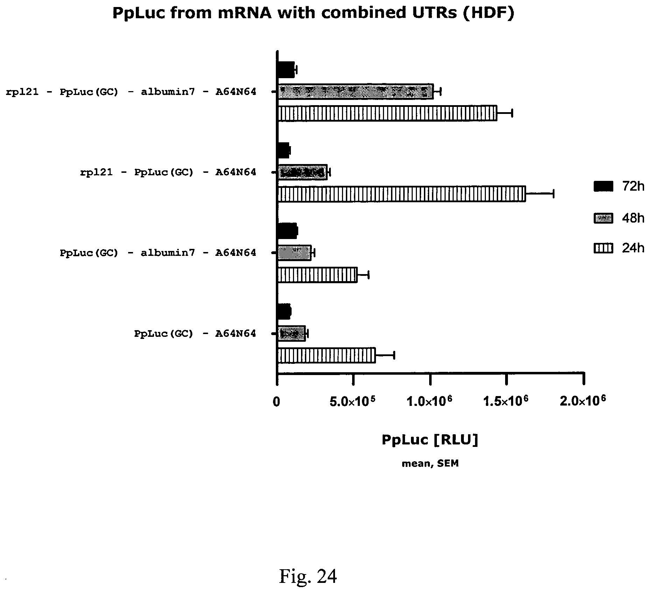

Fig. 24    is a graphical representation of the effect of the RPL21 TOP 5'UTR element, the albumin 3'UTR element and the combination of RPL21 TOP 5'UTR element and albumin 3'UTR element on luciferase expression from mRNA. A variety of mRNAs were transfected into human dermal fibroblasts (HDF) by lipofection. Luciferase levels were measured at 24, 48, and 72 hours after transfection. The albumin 3'UTR element extends luciferase expression, while the RPL21 TOP 5'UTR element increases luciferase levels compared to mRNA lacking 5'- and 3'UTR elements. Strikingly, the combination of RPL21 TOP 5'UTR element and albumin 3'UTR element further strongly increases the luciferase level, much above the level observed with either of the individual elements, thus acting synergistically. Data are graphed as mean RLU $\pm$ SEM (relative light units $\pm$ standard error) for triplicate transfections. Synergy is summarized in Example 5.3.

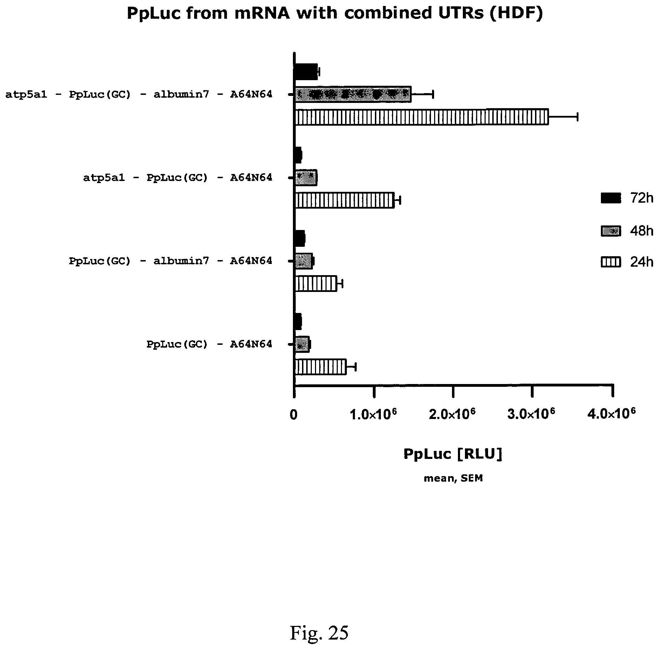

Fig. 25    is a graphical representation of the effect of the atp5a1 TOP 5'UTR element, the albumin 3'UTR element and the combination of atp5a1 TOP 5'UTR element and albumin 3'UTR element on luciferase expression from mRNA. A variety of mRNAs were transfected into human dermal fibroblasts (HDF) by lipofection. Luciferase levels were measured at 24, 48, and 72 hours after transfection. The albumin 3'UTR element extends luciferase expression, while the atp5a1 TOP 5'UTR element increases luciferase levels compared to mRNA lacking 5'- and 3'UTR elements. Strikingly, the combination of atp5a1 TOP 5'UTR element and albumin 3'UTR element further strongly increases the luciferase level, much above the level observed with either of the individual elements, thus acting synergistically. Data are graphed as mean RLU $\pm$ SEM (relative light units $\pm$ standard error) for triplicate transfections. Synergy is summarized in Example 5.3.

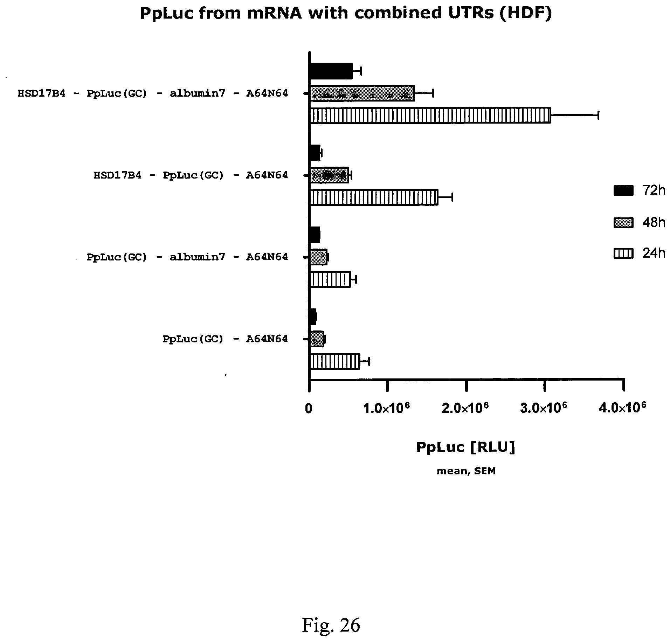

Fig. 26    is a graphical representation of the effect of the HSD17B4 TOP 5'UTR element, the albumin 3'UTR element and the combination of HSD17B4 TOP 5'UTR element and albumin 3'UTR element on luciferase expression from mRNA. A variety of mRNAs were transfected into human dermal fibroblasts (HDF) by lipofection. Luciferase levels were measured at 24, 48, and 72 hours after transfection. The albumin 3'UTR element extends luciferase expression, while the HSD17B4 TOP 5'UTR element increases luciferase levels compared to mRNA lacking 5'- and 3'UTR elements. Strikingly, the combination of HSD1 7B4 TOP 5'UTR element and albumin 3'UTR element further strongly increases the luciferase level, much above the level observed with either of the individual elements, thus acting synergistically. Data are graphed as mean RLU $\pm$ SEM (relative light units $\pm$ standard error) for triplicate transfections. Synergy is summarized in Example 5.3.

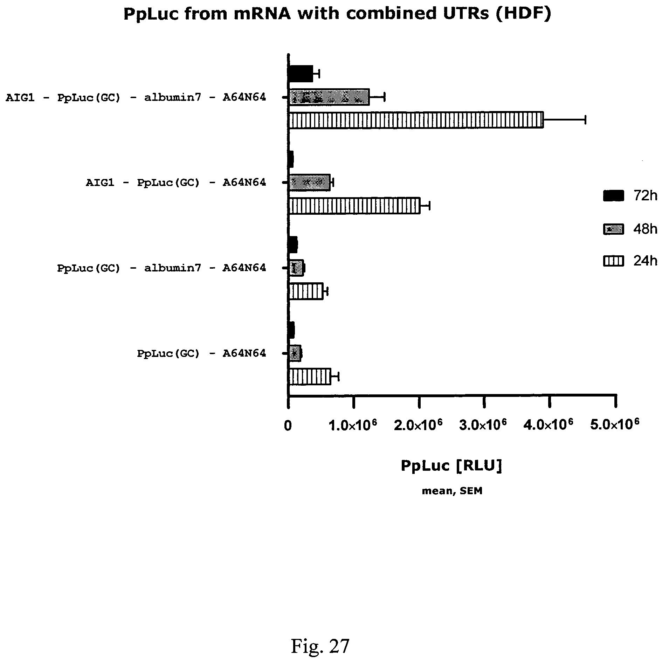

Fig. 27    is a graphical representation of the effect of the AIG1 TOP 5'UTR element, the albumin 3'UTR element and the combination of AIG1 TOP 5'UTR element and albumin 3'UTR element on luciferase expression from mRNA. A variety of mRNAs were transfected into human dermal fibroblasts (HDF) by lipofection. Luciferase levels were measured at 24, 48, and 72 hours after transfection. The albumin 3'UTR element extends luciferase expression, while the AIG1 TOP 5'UTR element increases luciferase levels compared to mRNA lacking 5'- and 3'UTR elements. Strikingly, the combination of AIG1 TOP 5'UTR element and albumin 3'UTR element further strongly increases the luciferase level, much above the level observed with either of the individual elements, thus acting synergistically. Data are graphed as mean RLU $\pm$ SEM (relative light units $\pm$ standard error) for triplicate transfections. Synergy is summarized in Example 5.3.

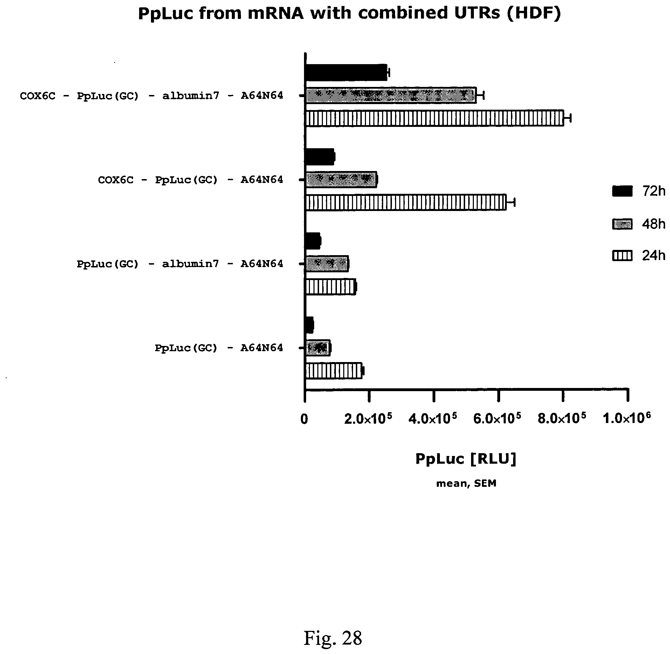

Fig. 28    is a graphical representation of the effect of the COX6C TOP 5'UTR element, the albumin 3'UTR element and the combination of COX6C TOP 5'UTR element and albumin 3'UTR element on luciferase expression from mRNA. A variety of mRNAs were transfected into human dermal fibroblasts (HDF) by lipofection. Luciferase levels were measured at 24, 48, and 72 hours after transfection. The albumin 3'UTR element extends luciferase expression, while the COX6C TOP 5'UTR element increases luciferase levels compared to mRNA lacking 5'- and 3'UTR elements. Strikingly, the combination of COX6C TOP 5'UTR element and albumin 3'UTR element further strongly increases the luciferase level, much above the level observed with either of the individual elements, thus acting synergistically. Data are graphed as mean RLU $\pm$ SEM (relative light units $\pm$ standard error) for triplicate transfections. Synergy is summarized in Example 5.3.

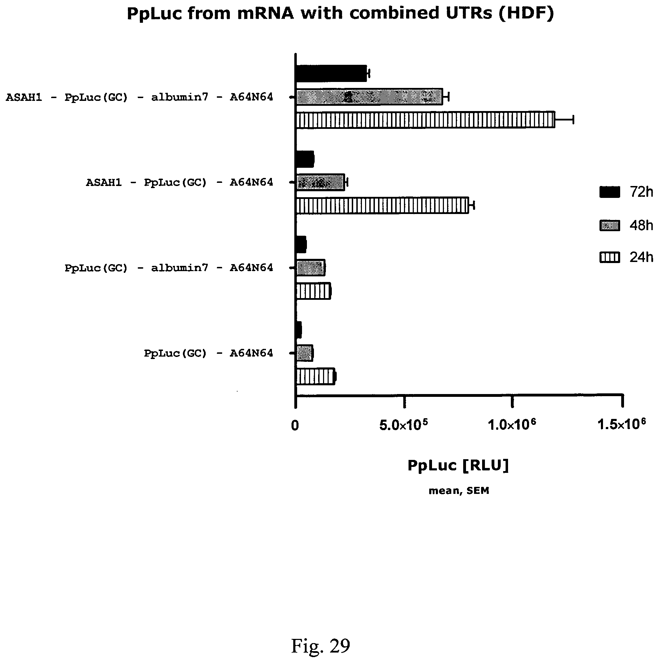

Fig. 29    is a graphical representation of the effect of the ASAH1 TOP 5'UTR element, the albumin 3'UTR element and the combination of ASAH1 TOP 5'UTR element and albumin 3'UTR element on luciferase expression from mRNA. A variety of mRNAs were transfected into human dermal fibroblasts (HDF) by lipofection. Luciferase levels were measured at 24, 48, and 72 hours after transfection. The albumin 3'UTR element extends luciferase expression, while the ASAH1 TOP 5'UTR element increases luciferase levels compared to mRNA lacking 5'- and 3'UTR elements. Strikingly, the combination of ASAH1 TOP 5'UTR element and albumin 3'UTR element further strongly increases the luciferase level, much above the level observed with either of the individual elements, thus acting synergistically. Data are graphed as mean RLU $\pm$ SEM (relative light units $\pm$ standard error) for triplicate transfections. Synergy is summarized in Example 5.3.

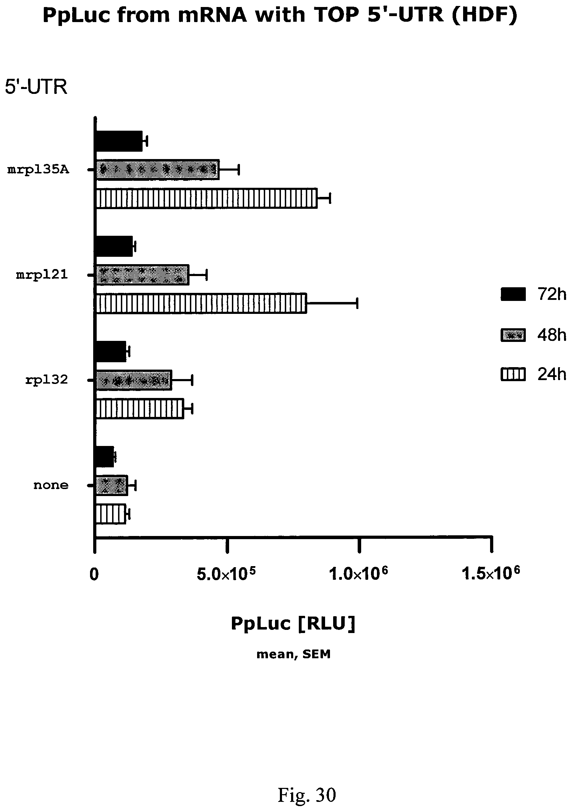

Fig. 30    is a graphical representation of the effect of the TOP 5'UTR element from mouse genes on luciferase expression from mRNA. mRNAs containing either a mouse or a human TOP 5'UTR element were transfected into human dermal fibroblasts (HDF) by lipofection. Luciferase levels were measured at 24, 48, and 72 hours after transfection. Mouse TOP 5'UTR elements strongly increase luciferase levels compared to mRNA lacking a 5'-element, similarly as the human TOP 5'UTR element. Data are graphed as mean RLU $\pm$ SEM (relative light units $\pm$ standard error) for triplicate transfections. RLU are summarized in Example 5.4.

| | |
|---|---|
| SEQ ID No. 1-1363, 1395, 1421, and 1422 | Sequences comprising 5'UTRs of TOP genes |
| SEQ ID No. 1364 | PpLuc(GC) - A64N64 |
| SEQ ID No. 1365 | PpLuc(GC) - albumin7 - A64N64 |
| SEQ ID No. 1366 | RPL32 - PpLuc(GC) - A64N64 |
| SEQ ID No. 1367 | RPL32 - PpLuc(GC) - albumin7 - A64N64 |
| SEQ ID No. 1368 | 5'UTR of human ribosomal protein Large 32 lacking the 5' terminal oligopyrimidine tract |
| SEQ ID No. 1369 | Human albumin 3'UTR |
| SEQ ID No. 1370 | 3'UTR of Homo sapiens hemoglobin, alpha 1 (HBA1) |
| SEQ ID No. 1371 | 3'UTR of Homo sapiens hemoglobin, alpha 2 (HBA2) |
| SEQ ID No. 1372 | 3'UTR of Homo sapiens hemoglobin, beta (HBB) |
| SEQ ID No. 1373 | 3'UTR of Homo sapiens tyrosine hydroxylase (TH) |
| SEQ ID No. 1374 | 3'UTR of Homo sapiens arachidonate 15-lipoxygenase (ALOX15) |
| SEQ ID No. 1375 | 3'UTR of Homo sapiens col lagen, type I, alpha 1 (COL1A1) |
| SEQ ID No. 1376 | albumin7 3'UTR |
| SEQ ID No. 1377 | Human albumin 3'UTR + poly(A) sequence |
| SEQ ID No. 1378 | Human albumin 3'UTR fragment 1 |
| SEQ ID No. 1379 | Human albumin 3'UTR fragment 2 |
| SEQ ID No. 1380 | Human albumin 3'UTR fragment 3 |
| SEQ ID No. 1381 | Human albumin 3'UTR fragment 4 |
| SEQ ID No. 1382 | Human albumin 3'UTR fragment 5 |
| SEQ ID No. 1383 | Human albumin 3'UTR fragment 6 |
| SEQ ID No. 1384 | Human albumin 3'UTR fragment 7 |
| SEQ ID No. 1385 | Human albumin 3'UTR fragment 8 |
| SEQ ID No. 1386 | Human albumin 3'UTR fragment 9 |
| SEQ ID No. 1387 | Human albumin 3'UTR fragment 10 |
| SEQ ID No. 1388 | Human albumin 3'UTR fragment 11 |
| SEQ ID No. 1389 | Human albumin 3'UTR fragment 12 |
| SEQ ID No. 1390 | Human albumin 3'UTR fragment 13 |
| SEQ ID No. 1391 | Albumin7 3'UTR - poly(A) sequence - poly(C) sequence - HL |
| SEQ ID No. 1392 | Albumin7 3'UTR - poly(A) sequence - poly(C) sequence |
| SEQ ID No. 1393 | Center, $\alpha$-complex-binding portion of the 3'UTR of an $\alpha$-globin gene |
| SEQ ID No. 1394 | Histone stem-loop |
| SEQ ID NO. 1396 | RPL35 - PpLuc(GC) - albumin7 - A64N64 |
| SEQ ID NO. 1397 | RPL21 - PpLuc(GC) - albumin7 - A64N64 |
| SEQ ID NO. 1398 | ATP5A1 - PpLuc(GC) - albumin7 - A64N64 |
| SEQ ID NO. 1399 | HSD17B4 - PpLuc(GC) - albumin7 - A64N64 |
| SEQ ID NO. 1400 | AIG1 - PpLuc(GC) - albumin7 - A64N64 |
| SEQ ID NO. 1401 | COX6C - PpLuc(GC) - albumin7 - A64N64 |
| SEQ ID NO. 1402 | ASAH1 - PpLuc(GC) - albumin7 - A64N64 |
| SEQ ID NO. 1403 | mRPL21 - PpLuc(GC) - albumin7 - A64N64 |
| SEQ ID NO. 1404 | mRPL35A - PpLuc(GC) - albumin7 - A64N64 |
| SEQ ID NO. 1405 | RPL35 - PpLuc(GC) - A64N64 |
| SEQ ID NO. 1406 | RPL21 - PpLuc(GC) - A64N64 |
| SEQ ID NO. 1407 | ATP5A1 - PpLuc(GC) - A64N64 |
| SEQ ID NO. 1408 | HSD17B4 - PpLuc(GC) - A64N64 |
| SEQ ID NO. 1409 | AIG1 - PpLuc(GC) - A64N64 |
| SEQ ID NO. 1410 | COX6C - PpLuc(GC) - A64N64 |
| SEQ ID NO. 1411 | ASAH1 - PpLuc(GC) - A64N64 |
| SEQ ID NO. 1412 | 5'UTR of human ribosomal protein Large 35 (RPL35) lacking the 5' terminal oligopyrimidine tract |

(continued)

| | |
|---|---|
| SEQ ID NO. 1413 | 5'UTR of human ribosomal protein Large 21 (RPL21) lacking the 5' terminal oligopyrimidine tract |
| SEQ ID NO. 1414 | 5'UTR of human ATP synthase, H+ transporting, mitochondrial F1 complex, alpha subunit 1, cardiac muscle (ATP5A1) lacking the 5' terminal oligopyrimidine tract |
| SEQ ID NO. 1415 | 5'UTR of human hydroxysteroid (1 7-beta) dehydrogenase 4 (HSD17B4) lacking the 5' terminal oligopyrimidine tract |
| SEQ ID NO. 1416 | 5'UTR of human androgen-induced 1 (AIG1) lacking the 5' terminal oligopyrimidine tract |
| SEQ ID NO. 1417 | 5'UTR of human cytochrome c oxidase subunit VIc (COX6C) lacking the 5' terminal oligopyrimidine tract |
| SEQ ID NO. 1418 | 5'UTR of human N-acylsphingosine amidohydrolase (acid ceramidase) 1 (ASAH1) lacking the 5' terminal oligopyrimidine tract |
| SEQ ID NO. 1419 | 5'UTR of mouse ribosomal protein Large 21 (mRPL21) lacking the 5' terminal oligopyrimidine tract |
| SEQ ID NO. 1420 | 5'UTR of mouse ribosomal protein large 35A (mRPL35A) lacking the 5' terminal oligopyrimidine tract |

## Examples

### 1. Preparation of DNA-templates

A vector for *in vitro* transcription was constructed containing a T7 promoter followed by a GC-enriched sequence coding for *Photinus pyralis* luciferase (PpLuc(GC)) and an A64 poly(A) sequence. The poly(A) sequence was followed by a restriction site used for linearization of the vector before *in vitro* transcription. mRNA obtained from this vector accordingly by *in vitro* transcription is designated as "PpLuc(GC) - A64N64".

This vector was modified to include untranslated sequences 5' or 3' of the open reading frame (5'UTR or 3'UTR, respectively). In summary, vectors comprising the following mRNA encoding sequences have been generated (the mRNA coding sequences are depicted in Figures 1 to 4 and 6 to 21):

SEQ ID No. 1364 (Fig. 1): PpLuc(GC) - A64N64
SEQ ID No. 1365 (Fig. 2): PpLuc(GC) - albumin7 - A64N64
SEQ ID No. 1366 (Fig. 3): RPL32 - PpLuc(GC) - A64N64
SEQ ID No. 1367 (Fig. 4): RPL32 - PpLuc(GC) - albumin7 - A64N64
SEQ ID NO. 1396 (Fig. 6): RPL35 - PpLuc(GC) - albumin7 - A64N64
SEQ ID NO. 1397 (Fig. 7): RPL21 - PpLuc(GC) - albumin7 - A64N64
SEQ ID NO. 1398 (Fig. 8): ATP5A1 - PpLuc(GC) - albumin7 - A64N64
SEQ ID NO. 1399 (Fig. 9): HSD17B4 - PpLuc(GC) - albumin7 - A64N64
SEQ ID NO. 1400 (Fig. 10): AIG1 - PpLuc(GC) - albumin7 - A64N64
SEQ ID NO. 1401 (Fig. 11): COX6C - PpLuc(GC) - albumin7 - A64N64
SEQ ID NO. 1402 (Fig. 12): ASAH1 - PpLuc(GC) - albumin7 - A64N64
SEQ ID NO. 1403 (Fig. 13): mRPL21 - PpLuc(GC) - albumin7 - A64N64
SEQ ID NO. 1404 (Fig. 14): mRPL35A - PpLuc(GC) - albumin7 - A64N64
SEQ ID NO. 1405 (Fig. 15): RPL35 - PpLuc(GC) - A64N64
SEQ ID NO. 1406 (Fig. 16): RPL21 - PpLuc(GC) - A64N64
SEQ ID NO. 1407 (Fig. 17): ATP5A1 - PpLuc(GC) - A64N64
SEQ ID NO. 1408 (Fig. 18): HSD17B4 - PpLuc(GC) - A64N64
SEQ ID NO. 1409 (Fig. 19): AIG1 - PpLuc(GC) - A64N64
SEQ ID NO. 1410 (Fig. 20): COX6C - PpLuc(GC) - A64N64
SEQ ID NO. 1411 (Fig. 21): ASAH1 - PpLuc(GC) - A64N64

### 2. *In vitro* transcription

The DNA-template according to Example 1 was linearized and transcribed *in vitro* using T7-Polymerase. The DNA-template was then digested by DNase-treatment. mRNA transcripts contained a 5'-CAP structure obtained by adding an excess of N7-Methyl-Guanosine-5'-Triphosphate-5'-Guanosine to the transcription reaction. mRNA thus obtained was purified and resuspended in water.

**3. Luciferase expression by mRNA lipofection**

Human dermal fibroblasts (HDF) were seeded in 24 well plates at a density of 5x10$^4$ cells per well. The following day, cells were washed in opti-MEM and then transfected with 50 ng per well of Lipofectamine2000-complexed PpLuc-encoding mRNA in opti-MEM. As a control, mRNA not coding for PpLuc was lipofected separately. mRNA coding for *Renilla reniformis* luciferase (RrLuc) was transfected together with PpLuc mRNA to control for transfection efficiency (20 ng of RrLuc mRNA per well). 90 minutes after start of transfection, opti-MEM was exchanged for medium. 24, 48, 72 hours after transfection, medium was aspirated and cells were lysed in 200 µl of lysis buffer (25 mM Tris, pH 7.5 (HCl), 2 mM EDTA, 10% glycerol, 1% Triton X-100, 2 mM DTT, 1 mM PMSF). Lysates were stored at -20°C until luciferase activity was measured.

Alternatively, HDF were seeded in 96 well plates three days before transfection at a density of 10$^4$ cells per well. Immediately before lipofection, cells were washed in opti-MEM. Cells were lipofected with 25 ng of PpLuc-encoding mRNA per well complexed with Lipofectamine2000. mRNA coding for Renilla reniformis luciferase (RrLuc) was transfected together with PpLuc mRNA to control for transfection efficiency (2.5 ng of RrLuc mRNA per well). 90 minutes after start of transfection, opti-MEM was exchanged for medium. 24, 48, 72 hours after transfection, medium was aspirated and cells were lysed in 100 µl of lysis buffer (Passive Lysis Buffer, Promega). Lysates were stored at -80°C until luciferase activity was measured.

**4. Luciferase measurement**

Luciferase activity was measured as relative light units (RLU) in a BioTek SynergyHT plate reader. PpLuc activity was measured at 15 seconds measuring time using 50 µl of lysate and 200 µl of luciferin buffer (75 µM luciferin, 25 mM Glycylglycin, pH 7.8 (NaOH), 15 mM MgSO4, 2 mM ATP). RrLuc activity was measured at 15 seconds measuring time using 50 µl of lysate and 200 µl of coelenterazin buffer (40 µM coelenterazin in phosphate buffered saline adjusted to 500 mM NaCl).

Alternatively, luciferase activity was measured as relative light units (RLU) in a Hidex Chameleon plate reader. PpLuc activity was measured at 2 seconds measuring time using 20 µl of lysate and 50 µl of luciferin buffer (Beetle-Juice, PJK GmbH). RrLuc activity was measured at 2 seconds measuring time using 20 µl of lysate and 50 µl of coelenterazin buffer (Renilla-Juice, PJK GmbH).

**Results**

**5.1 The combination of TOP 5'UTR element and albumin 3'UTR element increases protein expression from mRNA in a synergistic manner.**

To investigate the effect of the combination of a TOP 5'UTR element and an albumin 3'UTR element on protein expression from mRNA, mRNAs with different UTRs were synthesized: mRNAs either lacked both TOP 5'UTR element and albumin 3'UTR element, or contained either a TOP 5'UTR element (RPL32) or an albumin 3'UTR element (albumin7), or both TOP 5'UTR element and albumin 3'UTR element (according to the invention). Luciferase encoding mRNAs or control mRNA were transfected into human dermal fibroblasts (HDF). Luciferase levels were measured at 24, 48, and 72 hours after transfection. The PpLuc signal was corrected for transfection efficiency by the signal of cotransfected RrLuc (see following Table 1 and Figure 5).

Table 1:

| mRNA | RLU at 24 hours | RLU at 48 hours | RLU at 72 hours |
|---|---|---|---|
| PpLuc(GC)-A64N64 | 115147 | 28973 | 8371 |
| PpLuc(GC)-albumin7-A64N64 | 120234 | 48546 | 38138 |
| RPL32-PpLuc(GC)-A64N64 | 671815 | 168741 | 21709 |
| RPL32-PpLuc(GC)-albumin7-A64N64 | 913310 | 381288 | 100890 |

Luciferase was clearly expressed from mRNA having neither TOP 5'UTR nor albumin 3'UTR (PpLuc(GC)-A64N64). The albumin 3'UTR element extended luciferase expression, while the TOP 5'UTR element increased luciferase levels compared to mRNA lacking 5'- and 3'UTR elements. Strikingly, however, the combination of TOP 5'UTR element and albumin 3'UTR element further strongly increased the luciferase level, much above the level observed with either of the individual elements. The magnitude of the rise in luciferase level due to combining TOP 5'UTR element and albumin 3'UTR element in the same mRNA demonstrates that they are acting synergistically.

The synergy between TOP 5'UTR element and albumin 3'UTR element was quantified by dividing the signal from mRNA combining both elements by the sum of the signal from mRNA lacking both elements plus the rise in signal effected by the TOP 5'UTR element plus the rise in signal effected by the albumin 3'UTR element. This calculation was performed for the three time points individually and for total protein expressed from 0 to 72 hours calculated from the area under the curve (AUC) (see following Table 2).

Table 2:

| 24 h | | | | | |
|---|---|---|---|---|---|
| RPL32 | albumin | RLU | Δ RLU | RLU predicted (additive) | synergy |
| - | - | 115147 | | | |
| - | + | 120234 | 5088 | | |
| + | - | 671815 | 556668 | | |
| + | + | 913310 | | 676903 | **1,35** |
| 48 h | | | | | |
| RPL32 | albumin | RLU | Δ RLU | RLU predicted (additive) | synergy |
| - | - | 28973 | | | |
| - | + | 48546 | 19573 | | |
| + | - | 168741 | 139768 | | |
| + | + | 381288 | | 188313 | **2,02** |
| 72 h | | | | | |
| RPL32 | albumin | RLU | Δ RLU | RLU predicted (additive) | synergy |
| - | - | 8371 | | | |
| - | + | 38138 | 29767 | | |
| + | - | 21709 | 13338 | | |
| + | + | 100890 | | 51476 | **1,96** |
| AUC 0 - 72 hours | | | | | |
| RPL32 | albumin | RLU | Δ RLU | RLU predicted (additive) | synergy |
| - | - | 3559000 | | | |
| - | + | 4508000 | 949000 | | |
| + | - | 20430000 | 16871000 | | |
| + | + | 32280000 | | 21379000 | **1,51** |

The synergy thus calculated specifies how much higher the luciferase level from mRNA combining TOP 5'UTR element and albumin 3'UTR element is than would be expected, if the effects of TOP 5'UTR element and albumin 3'UTR element were purely additive. The luciferase level from mRNA combining TOP 5'UTR element and albumin 3'UTR element was up to two times higher than if their effects were purely additive. This result confirms that the combination of TOP 5'UTR element and albumin 3'UTR element effects a markedly synergistic increase in protein expression.

**5.2 TOP 5'UTR elements increase protein expression from mRNA.**

To investigate the effect of TOP 5'UTR elements on protein expression from mRNA, mRNAs comprising different TOP 5'UTR elements were synthesized. In addition, mRNAs contained the albumin7 3'UTR element. Luciferase encoding mRNAs were transfected into human dermal fibroblasts (HDF). Luciferase levels were measured at 24, 48, and 72 hours after transfection (see following Table 3 and Figure 22).

Table 3:

| 5'UTR | RLU at 24 hours | RLU at 48 hours | RLU at 72 hours |
|---|---|---|---|
| none | 114277 | 121852 | 68235 |
| RPL32 | 332236 | 286792 | 114148 |
| RPL35 | 495917 | 234070 | 96993 |
| RPL21 | 563314 | 352241 | 156605 |
| atp5a1 | 1000253 | 538287 | 187159 |
| HSD17B4 | 1179847 | 636877 | 299337 |
| AIG1 | 620315 | 446621 | 167846 |
| COX6C | 592190 | 806065 | 173743 |
| ASAH1 | 820413 | 529901 | 198429 |

Luciferase was clearly expressed from mRNA lacking a 5'UTR element. Strikingly however, all TOP 5'UTR elements strongly increased the luciferase level.

**5.3 The combination of TOP 5'UTR elements and albumin 3'UTR element increases protein expression from mRNA in a synergistic manner.**

To investigate the effect of the combination of TOP 5'UTR elements and an albumin 3'UTR element on protein expression from mRNA, mRNAs comprising different UTR elements were synthesized: mRNAs either lacked both TOP 5'UTR element and albumin 3'UTR element, or contained an albumin 3'UTR element, or contained one of different TOP 5'UTR elements, or contained both one of different TOP 5'UTR elements and an albumin 3'UTR element. Luciferase encoding mRNAs were transfected into human dermal fibroblasts (HDF). Luciferase levels were measured at 24, 48, and 72 hours after transfection (see Figures 23 to 30). Luciferase was clearly expressed from mRNA having neither a TOP 5'UTR element nor an albumin 3'UTR element. The albumin 3'UTR element extended luciferase expression, while TOP 5'UTR elements increased luciferase levels compared to mRNA lacking 5' and 3'UTRs. Strikingly however, the combinations of TOP 5'UTR elements and albumin 3'UTR element further strongly increased the luciferase level, much above the level observed with either of the individual elements. The magnitude of the rise in luciferase level due to combining TOP 5'UTR element and albumin 3'UTR element in the same mRNA demonstrates that they are acting synergistically.

The synergy between TOP 5'UTR element and albumin 3'UTR element was quantified by dividing the signal from mRNA combining both elements by the sum of the signal from mRNA lacking both elements plus the rise in signal effected by the TOP 5'UTR element plus the rise in signal effected by the albumin 3'UTR element. This calculation was performed for total protein expressed from 0 to 72 hours calculated from the area under the curve (AUC) (see following Table 4).

Table 4:

| TOP 5'UTR | Synergy with albumin 3'UTR |
|---|---|
| RPL35 | 2,25 |
| RPL21 | 1,30 |
| atp5a1 | 3,19 |
| HSD17B4 | 2,18 |
| AIG1 | 2,03 |
| COX6C | 1,56 |
| ASAH1 | 1,84 |

The synergy thus calculated specifies how much higher the luciferase level from mRNA combining TOP 5'UTR elements and albumin 3'UTR element is than would be expected if the effects of TOP 5'UTR element and albumin 3'UTR element were purely additive. The luciferase level from mRNA combining TOP 5'UTR element and albumin 3'UTR element was up to three times higher than if their effects were purely additive. This result confirms that the combination of TOP 5'UTR element and albumin 3'UTR element effects a markedly synergistic increase in protein expression.

## 5.4 TOP 5'UTR elements from mouse genes increase protein expression from mRNA.

To investigate the effect of TOP 5'UTR elements from mouse genes on protein expression from mRNA, mRNAs with two different mouse TOP 5'UTR elements were synthesized. In addition, mRNAs contained the albumin7 3'UTR element. Luciferase encoding mRNAs were transfected into human dermal fibroblasts (HDF). For comparison, mRNA containing the human RPL32 TOP 5'UTR element was transfected. Luciferase levels were measured at 24, 48, and 72 hours after transfection (see following Table 5 and Figure 30).

Table 5:

| 5'UTR | RLU at 24 hours | RLU at 48 hours | RLU at 72 hours |
|---|---|---|---|
| none | 114277 | 121852 | 68235 |
| 32L | 332236 | 286792 | 114148 |
| m21L | 798233 | 351894 | 139249 |
| m35AL | 838609 | 466236 | 174949 |

Luciferase was clearly expressed from mRNA lacking a 5'UTR element. Both mouse TOP 5'UTR elements strongly increased the luciferase level, similarly as the human TOP 5'UTR element.

SEQUENCES:

Homo sapiens alpha-2-macroglobulin (A2M): gctccttctttctgcaacatg (Seq ID No: 1)

Homo sapiens acyl-CoA dehydrogenase, C-4 to C-12 straight chain (ACADM): ggctctcttttccgcgctgcggtcagcctcg-gcgtcccacagagagggccagaggtg-gaaacgcagaaaaccaaaccaggactatcagagattgcccggagaggggatg (Seq ID No: 2)

Homo sapiens arylsulfatase E (chondrodysplasia punctata 1) (ARSE):

```
cttcctcttcttgatcggggattcaggaaggagcccaggagcagaggaagtagagagagaga-
caacatg (Seq ID No: 3)
```

Homo sapiens Bruton agammaglobulinemia tyrosine kinase (BTK):

```
tgtccttcctctctggactgtaagaatatgtctccagggccagtgtctgctgcgatcgag-
tcccaccttccaagtcctggcatctcaatgcatctgggaagctacctgcattaagtcag-
gactgagcacacaggtgaactccagaaagaagaagctatg (Seq ID No: 4)
```

Homo sapiens complement component 2 (C2): tgac-cttttccctcccgcggctctctacctctcgccgcccctagggaggacaccatg (Seq ID No: 5)

Homo sapiens cyclin-dependent kinase 4 (CDK4): gggcctctctagctt-gcggcctgtgtctatggtcgggccctctgcgtccagctgctccg-gaccgagctcgggtg-tatgggccgtaggaaccggctccggggccccgataacgggccgcccccacag-caccccgggctggcgtgagggtctccctt-gatctgagaatg (Seq ID No: 6)

Homo sapiens cytochrome P450, family 17, subfamily A, polypeptide 1 (CYP17A1): agctcttctactccactgctgtctatcttgcctgccggcacccagccaccatg (Seq ID No: 7)

Homo sapiens endoglin (ENG): cttcctctacccggttgg-caggcggcctggcccagcccccttctctaaggaagcg-catttcctgcctccct-

gggccggccgggctggatg (Seq ID No: 8)

Homo sapiens excision repair cross-complementing rodent repair defic iency, complementation group 3 (ERCC3): tcttctctctgctgctg-tagctgccatg (Seq ID No: 9)

Homo sapiens excision repair cross-complementing rodent repair defic iency, complementation group 5 (ERCC5): ctgtctttcttccgg-gaggcggtgacagctgctgagacgtgttgcagccagagtctctccgctttaatgcgctcccat-tagtgccgtcccccactggaaaaccgtggcttctgtattatttgccatctttgttgtgtag-gagcaggggagggcttcctcccggggtcctaggcggcggtgcagtc-cgtcgtagaagaatt-agagtagaagttgtcggggtccgctcttaggacgcagccgcctcatg (Seq ID No: 10)

Homo sapiens ferritin, light polypeptide (FTL): cgtcccctcgcag-ttcggcggtcccgcgggtctgtctcttgcttcaacagtgtttggacg-gaacagatccggg-gactctcttccagcctccgaccgccctccgatttcctctccgcttgcaacctccgggac-catcttctcggccatctcctgcttctgggacct-gccagcaccgttttt-gtggttagctccttcttgccaaccaaccatg (Seq ID No: 11)

Homo sapiens galactosylceramidase (GALC): ccgcctccctgggcgccggag-tcatgtgacccacacaatg (Seq ID No: 12)

Homo sapiens gap junction protein, alpha 1, 43kDa (GJA1):

```
ttttctttcattaggggggaaggcgtgaggaaagtaccaaacagcagcggag-
ttttaaactttaaatagacaggtctgagtgcctgaactt-
gccttttcattttacttcatcctccaaggagttcaatcacttggcgtgacttcac-
tacttttaagcaaaagagtggtgcccaggcaacatg (Seq ID No: 13)
```

Homo sapiens gap junction protein, beta 1, 32kDa (GJB1):

```
cattctctgggaaagggcagcagcagccaggtgtggcag-
tgacagggaggtgtgaatgaggcaggatg (Seq ID No: 14)
```

Homo sapiens glucose-6-phosphate isomerase (GPI): cgctccttcctcctcggctcgcgtctcactcagtgtaccttctagtcccgccatg (Seq ID No: 15)

Homo sapiens hydroxyacyl-CoA dehydrogenase/3-ketoacyl-CoA thiolase/e noyl-CoA hydratase (trifunctional protein), alpha subunit (HADHA): ctgtcctcttcagctcaagatg (Seq ID No: 16)

Homo sapiens hydroxyacyl-CoA dehydrogenase/3-ketoacyl-CoA thiolase/e noyl-CoA hydratase (trifunctional protein), beta subunit (HADHB):

```
gggccctttctgggcaggacccgccccttggtcccgcagagccttggtacttggacctgaac-
cttgctccgagagggagtcctcgcggacgtcagccaagattccagaatg (Seq ID No: 17)
```

Homo sapiens complement factor H (CFH):

```
cttccttttgcagcaagttctttcctgcactaatcacaattcttggaagaggagaactggacgttg
tgaacagagttagctggtaaatgtcctcttaaaagatccaaaaaatg (Seq ID No: 18)
```

Homo sapiens sarcoglycan, gamma (35kDa dystrophin-associated glycoprotein) (SGCG):

```
agccctttctccagggacagttgctgaagcttcatcctttgctctcattctgtaagtcatagaaaa
gtttgaaacattctgtctgtggtagagctcgggccagctgtagttcattcgccagtgtgcttttct
taatatctaagatg (Seq ID No: 19)
```

Homo sapiens lipase A, lysosomal acid, cholesterol esterase (LIPA):

ggtcccctatccgcaccccggcccctgagagctggcactgcgactcgagacagcggcccggcagga
cagctccagaatg (Seq ID No: 20)

Homo sapiens lipoprotein lipase (LPL):

cccctcttcctcctcctcaagggaaagctgcccacttctagctgccctgccatcccctttaaagg
gcgacttgctcagcgccaaaccgcggctccagcctctccagcctccggctcagccggctcatcag
tcggtccgcgccttgcagctcctccagagggacgcgccccgagatg (Seq ID No: 21)

Homo sapiens mutL homolog 1, colon cancer, nonpolyposis type 2 (E. coli) (MLH1): ggctcttctggcgccaaaatg (Seq ID No: 22)

Homo sapiens Niemann-Pick disease, type C1 (NPC1):

cttccttcctgaccggcgcgcgcagcctgctgccgcggtcagcgcctgctcctgctcctccgctcc
tcctgcgcggggtgctgaaacagcccgggggaagtagagccgcctccggggagcccaaccagccgaa
cgccgccggcgtcagcagccttgcgcggccacagcatg (Seq ID No: 23)

Homo sapiens peroxisomal biogenesis factor 12 (PEX12):

gcgcctctcttccgccaggcatcccagaggtcctggtggtttcatttccgggtgcggcttctgtca
taaagcggagacctcccttcaaacgtggcgtcgtgggttgtttgcgcctcgcctggggtcagcgag
caaggacgggcgcgggcggggatactcaaagccaacagctggagtcagcccttgtgtcccgggctc
acagtggcacgactgaatcctcagagtcggctggcttttgagctctcacgattggggaggaggggg
cgtttctggttcgcagctccagaggattgcgttccttcccccatacctgtcccccacagtcacgct
ctgccctgacgtgcagcatttgacaagttacccccctcgccacatactacttccacccacgtccgag
ttaactttgttcttaaccttcttgagactaccctcggcctccaggtctttttttcccagttcattt
ttgcccataagattgagtttcgagtttcagatatcatgcagaaagtttacctttaagactgagcac
ccatctgatactcttcctcccgaaaaagttcatgctcacgagagagtttgtgggaaaagtgaaagc
cagtacacgcaggaaactatg (Seq ID No: 24)

Homo sapiens peroxisomal biogenesis factor 6 (PEX6): cgctccttcaccctcctcgttggtgtcctgtcaccatg (Seq ID No: 25)

Homo sapiens phosphofructokinase, muscle (PFKM):

gagccttcttgtcagcatctgttagtggaggttgggaagcctctcctccttcccctccctctttg
cctccacctggctcctcccatgttcgtccatcacccctccccctttcccaaggacaatctgcaa
gaaagcagcggcggaggagagctaagactaaaagagtggatcatg (Seq ID No: 26)

Homo sapiens serpin peptidase inhibitor, clade A (alpha-1 antiproteinase, antitrypsin), member 1 (SERPINA1): ctgtctcctcagcttcaggcaccaccactgacctgggacagtgaatcgacaatg (Seq ID No: 27)

Homo sapiens phosphatase and tensin homolog (PTEN):

```
agttctctcctctcggaagctgcagccatgatggaagtttgagagttgagccgctgtgaggcgagg
ccgggctcaggcgagggagatgagagacggcggcggccgcggcccggagcccctctcagcgcctgt
gagcagccgcgggggcagcgccctcggggagccggccggcctgcggcggcggcagcggcggcgttt
ctcgcctcctcttcgtctttctaaccgtgcagcctcttcctcggcttctcctgaaagggaaggtg
gaagccgtgggctcgggcgggagccggctgaggcgcggcggcggcggcggcacctcccgctcctgg
agcgggggggagaagcggcggcggcggcggccgcggcggctgcagctccagggagggggtctgagt
cgcctgtcaccatttccagggctgggaacgccggagagttggtctctcccttctactgcctccaa
cacggcggcggcggcggcggcacatccagggacccgggccggttttaaacctcccgtccgccgccg
ccgcacccccgtggcccgggctccggaggccgccggcggaggcagccgttcggaggattattcgt
cttctccccattccgctgccgccgctgccaggcctctggctgctgaggagaagcaggcccagtcgc
tgcaaccatccagcagccgccgcagcagccattacccggctgcggtccagagccaagcggcggcag
agcgaggggcatcagctaccgccaagtccagagccatttccatcctgcagaagaagccccgccacc
agcagcttctgccatctctctcctccttttcttcagccacaggctcccagacatg
```
(Seq ID No: 28)

Homo sapiens solute carrier family 3 (cystine, dibasic and neutral amino acid transporters, activator o f cystine, dibasic and neutral amino acid transport), member 1 (SLC3A1): cctcccttactgcaggaaggcactccgaagacataagtcggtgagacatg (Seq ID No: 29)

Homo sapiens aldehyde dehydrogenase 3 family, member A2 (ALDH3A2): ccgcctcccactccccagcgcccccggaccgtgcagttctctgcaggaccaggccatg (Seq ID No: 30)

Homo sapiens bleomycin hydrolase (BLMH):

```
gtttctcccagcctcagcctccccgccgccgccgccgccgccgccgagccggtttcctttttc
cggcgctccgggtgcgagagacaggtcgggccccctaggcagcgagccgcagcgcaatcccggcgc
tcgcccaaggaccctggaagctaccgttaccccgccgggcagcgtgggcgccatg
```
(Seq ID No: 31)

Homo sapiens cathepsin K (CTSK):

```
cctcctcctcttacccaaattttccagccgatcactggagctgacttccgcaatcccgatggaata
aatctagcacccctgatggtgtgcccacactttgctgccgaaacgaagccagacaacagatttcca
tcagcaggatg (Seq ID No: 32)
```

Homo sapiens GM2 ganglioside activator (GM2A):

```
gcttctttgcgtaaccaatactggaaggcatttaaaggcacctctgccgccacagaccttgcagtt
aactccgccctgacccacccttcccgatg (Seq ID No: 33)
```

Homo sapiens hydroxysteroid (17-beta) dehydrogenase 4 (HSD17B4):

```
ccgcctcctcctgtcccgcagtcggcgtccagcggctctgcttgttcgtgtgtgtgtcgttgcagg
ccttattcatg (Seq ID No: 34)
```

Homo sapiens neutrophil cytosolic factor 2 (NCF2):

```
ctctctctgcttctttcctttctctctcatggtagggttatgagtcagttgccaaaaggtgggga
catttcctgatgcatttgcaacactgagaagttatcttaagggaggctgggcccccattctactcat
ctggcccagaaagtgaacaccttgggggccactaaggcagccctgctaggggagacgctccaacct
gtcttctctctgtctcctggcagctctcttggcctcctagtttctacctaatcatg
```
(Seq ID No: 35)

Homo sapiens 3-oxoacid CoA transferase 1 (OXCT1): cagcctcctcctgcctcaccgcccgaagatg (Seq ID No: 36)

Homo sapiens sulfite oxidase (SUOX):

```
ccgccccttctcgagaactcgcagagctgggctggtaaaattgcagtgctgaagacactggacccg
caaaaggctgtccctcccaaacctgggattctgggctcactgagttcacctgcgagtcagccctac
ctgcactgctctggtctagtacaaacaggctgctggcattgagggacggagtctccaactcctggc
ctctagcagtcctcctgtgtaggtctcccaaagtgctagtgtgtccggaattggtgggttcttggt
ctcactgacttcaagaatgaagccgcggaccctcgcagtctgctacaatg (Seq ID No: 37)
```

Homo sapiens albumin (ALB): ttttctcttctgtcaaccccacacgcctttggca-caatg (Seq ID No: 38)

Homo sapiens arylsulfatase A (ARSA):

```
ctccctctagcgccttcccccggcccgactccgctggtcagcgccaagtgacttacgcccccgac
cctgagcccggaccgctaggcgaggaggatcagatctccgctcgagaatctgaaggtgccctggtc
ctggaggagttccgtcccagcccgcggtctcccggtactgtcgggccccggccctctggagcttca
ggaggcggccgtcagggtcggggagtatttgggtccggggtctcagggaagggcggcgcctggtc
tgcggtatcggaaagagcctgctggagccaagtagccctccctctcttgggacagacccctcggtc
ccatg (Seq ID No: 39)
```

Homo sapiens elastin (ELN):

```
ctccctccctctttccctcacagccgacgaggcaacaattaggctttggggataaaacgaggtgcg
gagagcgggctggggcatttctccccgagatg (Seq ID No: 40)
```

Homo sapiens hemoglobin, alpha 2 (HBA2): cactcttctggtccccaca-gactcagagagaacccaccatg (Seq ID No: 41)

Homo sapiens hexosaminidase B (beta polypeptide) (HEXB):

```
cttcctctgatccgggccgggcgggaagtcgggtcccgaggctccggctcggcagaccgggcggaa
agcagccgagcggccatg (Seq ID No: 42)
```

Homo sapiens mannosidase, alpha, class 2B, member 1 (MAN2B1): cggcctttccagggccgggggaaccccaggaggaagct-gctgagccatg (Seq ID No: 43)

Homo sapiens recombination activating gene 2 (RAG2):

```
cactctctttacagtcagccttctgcttgccacagtcatagtgggcagtcagtgaatcttccccaa
gtgctgacaattaatacctggtttagcggcaaagattcagagaggcgtgagcagcccctctggcct
tcagacaaaaatctacgtaccatcagaaactatg (Seq ID No: 44)
```

Homo sapiens CD53 molecule (CD53):

```
tctccttttacacaaatagccccggatatctgtgttaccagccttgtctcggccacctcaaggata
atcactaaattctgccgaaaggactgaggaacggtgcctggaaaagggcaagaatatcacggcatg
(Seq ID No: 45)
```

Homo sapiens Fc fragment of IgG, low affinity IIIa, receptor (CD16a) (FCGR3A): tggtccctttagggctccggatatctttggtgacttgtccactccag-tgtggcatcatg (Seq ID No: 46)

Homo sapiens interleukin 1, beta (IL1B):

aaacctcttcgaggcacaaggcacaacaggctgctctgggattctcttcagccaatcttcattgct caagtgtctgaagcagccatg (Seq ID No: 47)

Homo sapiens CD4 molecule (CD4):

ctgtctctcttcatttaagcacgactctgcagaaggaacaaagcaccctccccactgggctcctgg ttgcagagctccaagtcctcacacagatacgcctgtttgagaagcagcgggcaagaaagacgcaag

cccagaggccctgccatttctgtgggctcaggtccctactggctcaggcccctgcctccctcggca aggccacaatg (Seq ID No: 48)

Homo sapiens serpin peptidase inhibitor, clade A (alpha-1 antiproteinase, antitrypsin), member 5 (SERPINA5):

agccctctgccctttctgagcccgagggactgccacctccactgtgtgcacactcagctacgggac acatttcaggtatccaaggcagcagaggtgagtgggtcccccgagctctgtgaccttatgctccac actaactctggcagagcctccgtttcctcatagaacaaagaacagccaccatg (Seq ID No: 49)

Homo sapiens vitronectin (VTN):

tgccctccttccctgtctctgcctctccctcccttcctcaggcatcagagcggagacttcagggag accagagcccagcttgccaggcactgagctagaagccctgccatg (Seq ID No: 50)

Homo sapiens aldehyde dehydrogenase 9 family, member A1 (ALDH9A1):

ccgcccctcccgcggccccgcccctcccgcggcccgtcagcctctgccgcggagctgcgtccgcca ctcatg (Seq ID No: 51)

Homo sapiens annexin A1 (ANXA1):

cttcctttaaaatcctataaaatcagaagcccaagtctccactgccagtgtgaaatcttcagagaa gaatttctctttagttctttgcaagaaggtagagataaagacacttttttcaaaaatg (Seq ID No: 52)

Homo sapiens ATPase, Na+/K+ transporting, alpha 1 polypeptide (ATP1A1): ttttctctctgattctccagcgacaggacccggcgccgggcactgagcaccgc-caccatg (Seq ID No: 53)

Homo sapiens ATPase, Na+/K+ transporting, alpha 2 polypeptide (ATP1A2):

ctttctctgtctgccagggtctccgactgtcccagacgggctggtgtgggcttgggatcctcctgg tgacctctcccgctaaggtccctcagccactctgccccaagatg (Seq ID No: 54)

Homo sapiens calcium channel, voltage-dependent, beta 3 subunit (CACNB3):

ccctccttcgcgctctctcgctccctgccgccgcccgcagggctgcggggctcggtggcatctccc gggcgcggcccgcagtccttgcccctgcctccgggccgctcccgcccccggcgccgctcgctcccc cgacccggactcccccatg (Seq ID No: 55)

Homo sapiens cholinergic receptor, nicotinic, alpha 7 (neuronal) (CHRNA7):

gtgcctctgtggccgcaggcgcaggcccgggcgacagccgagacgtggagcgcgccggctcgctgc
agctccgggactcaacatg (Seq ID No: 56)

Homo sapiens cytochrome P450, family 51, subfamily A, polypeptide 1 (CYP51A1):

gcttctctcgttccgtcgattgggaggagcggtggcgacctcggccttcagtgtttccgacggagt
gaatg (Seq ID No: 57)

Homo sapiens glutamate decarboxylase 1 (brain, 67kDa) (GAD1):

atctctctcttctcctggcgctcgcgtgcgagagggaactagcgagaacgaggaagcagctggagg
tgacgccgggcagattacgcctgtcagggccgagccgagcggatcgctgggcgctgtgcagaggaa
aggcgggagtgcccggctcgctgtcgcagagccgagcctgtttctgcgccggaccagtcgaggact
ctggacagtagaggccccgggacgaccgagctgatg (Seq ID No: 58)

Homo sapiens gamma-glutamyl carboxylase (GGCX):

aattctcctggcggcctccgttcagacgcggcagctgtgacccacctgcctcctccgcagagcaat
g (Seq ID No: 59)

Homo sapiens glutamate receptor, metabotropic 3 (GRM3):

tcccctctttccccaacctcctccctctcttctactccacccctccgttttcccactccccactga
ctcggatgcctggatgttctgccaccgggcagtggtccagcgtgcagccgggagggggcaggggca
gggggcactgtgacaggaagctgcgcgcacaagttggccatttcgagggcaaaataagttctccct
tggatttggaaaggacaaagccagtaagctacctctttgtgtcggatgaggaggaccaaccatga
gccagagcccgggtgcaggctcaccgccgccgctgccaccgcggtcagctccagttcctgccagga
gttgtcggtgcgaggaatttttgtgacaggctctgttagtctgttcctcccttatttgaaggacagg
ccaaagatccagtttggaaatgagagaggactagcatgacacattggctccaccattgatatctcc
cagaggtacagaaacaggattcatgaagatg (Seq ID No: 60)

Homo sapiens guanylate cyclase 1, soluble, alpha 3 (GUCY1A3):

ggttcctttgggggtgatcaaagagggagacacagacacagagagacaaaggcaaggaggactgtct
gggagccacgcgggcgatacagtttccgaggcacgccgcgtcccgcctagcctgttgaacaggtag
acatgagcgacccaagctgcggatttgcgaggcgcgccctggagctgctagagatccggaagcaca
gccccgaggtgtgcgaagccaccaagtcaagttcctaacgagtcttcagaggaggcagcaggaagc
tcagagagctgcaaagcaaccgtgcccatctgtcaagacattcctgagaagaacatacaagaaagt
cttcctcaaagaaaaaccagtcggagccgagtctatcttcacactttggcagagagtatttgcaaa
ctgattttcccagagtttgaacggctgaatgttgcacttcagagaacattggcaaagcacaaaata
aaagaaagcaggaaatctttggaaagagaagactttgaaaaaacaattgcagagcaagcagttgca
gcaggagttccagtggaggttatcaaagaatctcttggtgaagaggttttttaaaatatgttacgag
gaagatgaaaacatccttggggtggttggaggcacccttaaagattttttaaacagcttcagtacc
cttctgaaacagagcagccattgccaagaagcaggaaaaaggggcaggcttgaggacgcctccatt
ctatgcctggataaggaggatgattttctacatgtttactacttcttccctaagagaaccacctcc
ctgattcttcccggcatcataaaggcagctgctcacgtattatatgaaacggaagtggaagtgtcg
ttaatg (Seq ID No: 61)

Homo sapiens 3-hydroxy-3-methylglutaryl-CoA reductase (HMGCR):

```
ggctccttccgctccgcgactgcgttaactggagccaggctgagcgtcggcgccggggttcggtgg
cctctagtgagatctggaggatccaaggattctgtagctacaatg (Seq ID No: 62)
```

Homo sapiens IMP (inosine 5'-monophosphate) dehydrogenase 2 (IMPDH2): aggtctctgcggcgcggtcctcggagacacgcggcggtgtcctgtgttggccatg (Seq ID No: 63)

Homo sapiens leukotriene A4 hydrolase (LTA4H):

```
acttccttttcccggcgtgcaccgcgaatccctcctcctcttctttacctctctccctcctcctcag
gttctctatcgacgagtctggtagctgagcgttgggctgtaggtcgctgtgctgtgtgatcccccca
gagccatg (Seq ID No: 64)
```

Homo sapiens neuropeptide Y receptor Y1 (NPY1R):

```
ccttctttaataagcaggagcgaaaaagacaaattccaaagaggattgttcagttcaagggaatga
agaattcagaataattttggtaaatggattccaatatggggaataagaataagctgaacagttgac
ctgctttgaagaaacatactgtccatttgtctaaataatctataacaaccaaaccaatcaaaatg
(Seq ID No: 65)
```

Homo sapiens pyruvate dehydrogenase (lipoamide) beta (PDHB): cggccctctgttgtcgtttggcagcggatagaggacacgac-caagatg (Seq ID No: 66)

Homo sapiens ribosomal protein L36a-like (RPL36AL):

```
cttcccttttcctgttaggcgagagctgcgaaaggcgagagctgcgaagggccaggtgtcgggcgct
gtttctcgttttcatcatatagacaaaacagccctgctgcaaagatg (Seq ID No: 67)
```

Homo sapiens ATPase, Ca++ transporting, type 2C, member 1 (ATP2C1):

```
gcttcttctcacgccgggagcaggctcccgcctcgcaccgctgccccgcgagcagctcctcttctc
ccgaggcgcgcggggcgcccccgcgagccccgcggctgagaccccgcagcctggaggagggctgtc
cggggctttggatgctgctgctaggggtggtgggagcagccgtgggacgcgtggccgggagcgggg
gtgacagcctgggattccggggggcttctcttccttgtcctcctcctctcctctctattcccagtgt
ggccgtggctgacactaaagactttgtagccatcaacccgagtgcagtttcgatggaaaatg
(Seq ID No: 68)
```

Homo sapiens UDP-glucose pyrophosphorylase 2 (UGP2):

```
ccgcctctttcattgaagaaatttaagttcgtgtggttttacctttttccgggagtctccagctggc
cctcatttgtgtccggagctcaggagttcccaaaccgactcagtcgcaccaagtttccgtcttttg
gaattggggaaggagtttctttctttcttttctttttcttgagccagttttaatcgctttgaata
aatactcccttaagtagttaaatataggaggagaaagaatacatcggttgttaaagcaggagagga
agagagacctgccctgtagcgtgactcctctagaaaaaaaaaaaaaaagccggagtattttactaa
gcccctaaaatg (Seq ID No: 69)
```

Homo sapiens ATPase, Na+/K+ transporting, beta 1 polypeptide (ATP1B1):

```
cctcctcctgctcctgccttggctcctccgccgcgcgtctcgcactccgagagccgcagcggcagc
ggcgcgtcctgcctgcagagagccaggccggagaagccgagcggcgcagaggacgccagggcgcgc
gccgcagccacccaccctccggaccgcggcagctgctgacccgccatcgccatg
(Seq ID No: 70)
```

Homo sapiens glycoprotein M6B (GPM6B):

```
ctgtctttatggaccagtaggcagagcgaaattgacgctgacaagacttttgcatcttggaaggga
ctgtaatctactgtagtgaagaacagagcctctcaatcagacgggtgtaaataagagacggagggg
agtccaaaagaaaaggaagaggaggaaaaacaagtgtgtgttggggggaacaggggaaaaagcatt
tttggtggatggtatg (Seq ID No: 71)
```

Homo sapiens wntless homolog (Drosophila) (WLS):

```
gctcctttaagcgtccacaggcggcggagcggccacaatcacagctccgggcattgggggaacccg
agccggctgcgccggggggaatccgtgcgggcgccttccgtcccggtcccatcctcgccgcgctcca
gcacctctgaagttttgcagcgcccagaaaggaggcgaggaaggagggagtgtgtgagaggaggga
gcaaaaagctcaccctaaaacatttatttcaaggagaaaagaaaaaggggggggcgcaaaaatg
(Seq ID No: 72)
```

Homo sapiens flavin containing monooxygenase 3 (FMO3):

```
ttttctctttcaaactgcccagacggttggacaggacgtagacacacagaagaaaagaagacaaag
aacgggtaggaaaattaaaaaggttaccatg (Seq ID No: 73)
```

Homo sapiens multiple C2 domains, transmembrane 1 (MCTP1):

```
cagcctcttttgccggtattcagtgaagaaagcaagtctaaatatgcagttctctcactggagtga
aagatgttttgttcatttctaatcaactatg (Seq ID No: 74)
```

Homo sapiens structural maintenance of chromosomes 4 (SMC4):

```
ccgcctctcggcgagcccgccctcttctgaagaggcgtttctggaccactgagccccgcctcccac
tgtgagcggaaccctaccgttttttaaaaaaatcttttttcaaaacttgccaggttgtctttccaaat
atttttaataatagtgctgctgctgtagaccacagagaaaagaatccctcgctcttccttttcact
tagtagaaacttctaccgcgtaggtcccgccaggagttcgcgcatgcgcaggagcgacaataagat
```

```
ggcggtgataatcgccgcacttttttttcaaattagtggatcccagaaatcattgcgcgcatttgta
acgaatttccgttcgagtttgtattttaggcgccattttcgagtgaaggacccggagccgaaacac
cggtaggagcggggaggtgggtactacacaaccgtctccagccttggtctgagtggactgtcctgc
agcgaccatg (Seq ID No: 75)
```

Homo sapiens GLE1 RNA export mediator homolog (yeast) (GLE1): tggccttcccggcggctgattcgagggcttgtttggtcagaag-ggggggcgtcagagaagctgcccc ttagccaaccatg (Seq ID No: 76)

Homo sapiens tripartite motif containing 6 (TRIM6):

```
gagtctttcggcctgggtggaggacgcggctgcttcaagtccttggctctgatccaggccacagat
tccaggattctacaggcaggaaacatcttagaaatcagggttgggcaggcaggagccaggagagta
gctacaatg (Seq ID No: 77)
```

Homo sapiens ecotropic viral integration site 2A (EVI2A):

tatccttttttactgcagatttactttaaggctcatattctccaagtctattctgctttaaaaaga
agacaagaaaagaagtggtttatcaaaatcacgttataatcagattttgaccaagcattttgtaag
tatacaaatgtcagccaatgacatataacaaccatttcttataaaaccttgatgttcaaaagcctg
actagcagtggcatccatg (Seq ID No: 78)

Homo sapiens heterogeneous nuclear ribonucleoprotein L (HNRNPL):

tgctcttttcgatccgggacggccggtcaggctcgccgccgagctggagaactacgatgacccgca
caaaaccccctgcctccccagttgtccacatcaggggcctgattgacggtgtggtggaagcagacct
tgtggaggccttgcaggagtttggacccatcagctatgtggtggtaatg (Seq ID No: 79)

Homo sapiens mitochondrial translational initiation factor 2 (MTIF2):

cattcttccgggtccagaaggtgatctccgcccgtgctcagaatccagggggcccgggggctgtagat
tccttgacaaggatatcctagcggcgaaacaacaccgtactgggagtcagaacgtctgggttctag
tcttgactgccattaactagcggtatgacattggagaagctttttttgacccttctggatttccgtt
tcctttctgtaaaatgaggagcttggaagatccggaaaatgaggcccataggaaacaagtgactt
gctgagtccagataacactgactgtcagagagaaacatg (Seq ID No: 80)

Homo sapiens nuclear factor of kappa light polypeptide gene enhanc er in B-cells inhibitor, zeta (NFKBIZ):

tggcctcctcttgccacgaggtcagacggcgagttcttagagaaaaaggctgcttagctgctgctt
atcatgtaacctcaaaaggaaactgatcgtctttctcatgctgtcacgtacttgggttattatcgc
tgattacagctggaaacaattgatttgctcttacgtatttgtgtgacttgactcttcaaacacaaa
ggttaacaggaagatctcgagggccctggctgaacttcacctttttggctttcttggcctgatgctg
aactctcgaggttgagccccatatg (Seq ID No: 81)

Homo sapiens v-erb-b2 erythroblastic leukemia viral oncogene homol og 3 (avian) (ERBB3):

atccctcccggactccggctccggctccgattgcaatttgcaacctccgctgccgtcgccgcagc
agccaccaattcgccagcggttcaggtggctcttgcctcgatgtcctagcctaggggcccccgggc
cggacttggctgggctcccttcaccctctgcggagtcatg (Seq ID No: 82)

Homo sapiens podoplanin (PDPN): ccgcctcctcgggagagataaatg (Seq ID No: 83)

Homo sapiens ribonucleotide reductase M1 (RRM1):

gcgcccctttgtgcgtcacgggtggcgggcgcgggaaggggatttggattgttgcgcctctgctct
gaagaaagtgctgtctggctccaactccagttctttcccctgagcagcgcctggaacctaaccctt


cccactctgtcaccttctcgatcccgccggcgctttagagccgcagtccagtcttggatccttcag
agcctcagccactagctgcgatg (Seq ID No: 84)

Homo sapiens solute carrier family 2 (facilitated glucose transporter), member 4 (SLC2A4):

gcgtctttcccccagccccgctccaccagatccgcgggagccccactgctctccgggtccttggc
ttgtggctgtgggtcccatcgggcccgccctcgcacgtcactccgggaccccgcggcctccgcag
gttctgcgctccaggccggagtcagagactccaggatcggttctttcatcttcgccgccctgcgc
gtccagctcttctaagacgagatg (Seq ID No: 85)

Homo sapiens steroid-5-alpha-reductase, alpha polypeptide 1 (3-oxo-5 alpha-steroid delta 4-dehydrogenase alpha 1) (SRD5A1):

```
aaccctttctgcagagtcccggcagtgcgggactccggtagccgcccctccggtagccgcccctcc
tgcccccgcgccgccgccctatatgttgcccgccgcggcctctggggcatggagcacgctgcccag
ccctggcgatg (Seq ID No: 86)
```

Homo sapiens thromboxane A synthase 1 (platelet) (TBXAS1):

```
gttcccttttctacctgcagagcacggttcccataagggcggcgagatcagcctcctgtctcatct
ggaagaccaccactctggggtctcagaggaatg (Seq ID No: 87)
```

Homo sapiens transketolase (TKT): ctatctctgtgtgtccgcgtgtgcgcccggtccccgcctgccgcaccatg (Seq ID No: 88)

Homo sapiens tumor necrosis factor receptor superfamily, member 1A (TNFRSF1A):

```
cctcctcctccagctcttcctgtcccgctgttgcaacactgcctcactcttcccctcccaccttct
ctcccctcctctctgctttaattttctcagaattctctggactgaggctccagttctggcctttgg
ggttcaagatcactgggaccaggccgtgatctctatgcccgagtctcaaccctcaactgtcacccc
aaggcacttgggacgtcctggacagaccgagtcccgggaagccccagcactgccgctgccacactg
ccctgagcccaaatggggggagtgagaggccatagctgtctggcatg (Seq ID No: 89)
```

Homo sapiens tubulin, beta 2A class IIa (TUBB2A):

```
aggtctctgcgcagcccagcccgccggtccacgccgcgcaccgctccgagggccagcgccacccgc
tccgcagccggcaccatg (Seq ID No: 90)
```

Homo sapiens actin, beta (ACTB): tcgcctttgccgatccgccgcccgtcca-cacccgccgccagctcaccatg (Seq ID No: 91)

Homo sapiens adenylosuccinate synthase (ADSS):

```
ggctccttcttcctctgcatgtggctggcggccgcagagcagttcagttcgctcactcctcgccgg
ccgcctctccttcgggctctcctcgcgtcactggagccatg (Seq ID No: 92)
```

Homo sapiens alanyl (membrane) aminopeptidase (ANPEP): cgttctctgcctggcctgaggctccctgagccgcctccccaccatcaccatg (Seq ID No: 93)

Homo sapiens beaded filament structural protein 1, filensin (BFSP1):

```
gcctcctttctttctcagcccagacctggccctctggagagggttttggagtcctgggtaggcagg
gtacctcaggcagcaggcagcacaccttggatgtgagctgaatggattttcaaatttcacagaagg
agcctccatgctggagaaagtatgtatg (Seq ID No: 94)
```

Homo sapiens basic transcription factor 3 (BTF3):

```
cggcctccctttagctgccatcttgcgtccccgcgtgtgtgcgcctaatctcaggtggtccacccg
```

```
agaccccttgagcaccaaccctagtcccccgcgcggccccttattcgctccgacaagatg
(Seq ID No: 95)
```

Homo sapiens complement component 1, q subcomponent binding protei n (C1QBP): ttgtcctttgcatctgcacgtgttcg-cagtcgtttccgcgcgatg (Seq ID No: 96)

Homo sapiens calsequestrin 1 (fast-twitch, skeletal muscle) (CASQ1):

```
tttcctttcttaatatggcgatgagctcttaggccagtgtggggaccggggctgaggtgccctgga
cactggaggaggggggagggaaggagcccctgggagcctggggtagaagtgtaggaggtgggaggat
tccggcccgcatggagctgtcctggcctcagaaggttatccgtctctcctgccaaccatggagaca
tatttagacaggaccaggtggggactgaggggtgccaatttcaggggggcagctccggttccctccc
cgcccctgctcctattcctccacctgacccttttcccttggctctgtcggcagtttctccagga
cccagcagtgccctctgtccactgctctgggccattccccaatcccccctcccacttgagcccta
actcagaatctgggacccaggggcccctccctaccccagctaacctcttctggaccaggagagcca
acccagatcccactacctccatg (Seq ID No: 97)
```

Homo sapiens caveolin 3 (CAV3):

```
gtctctctgcccctctctgccccaagtattttcagccccagccggccacacagctcggatctcctc
ctgtggatcccccagctctgcgatg (Seq ID No: 98)
```

Homo sapiens serpin peptidase inhibitor, clade H (heat shock protein 47), member 1, (collagen binding protein 1) (SERPINH1):

```
aggtctttggctttttttggcggagctggggcgccctccggaagcgtttccaacttttccagaagtt
tctcgggacgggcaggaggggggtggggactgccatatatagatcccgggagcaggggagcgggcta
agagtagaatcgtgtcgcggctcgagagcgagagtcacgtcccggcgctagcccagcccgacccag
gcccaccgtggtgcacgcaaaccacttcctggccatg (Seq ID No: 99)
```

Homo sapiens CD68 molecule (CD68):

```
tttcctcctttccaagagagggctgagggagcagggttgagcaactggtgcagacagcctagctgg
actttgggtgaggcggttcagccatg (Seq ID No: 100)
```

Homo sapiens cell division cycle 20 homolog (S. cerevisiae) (CDC20):

```
gggtccctttctgtccctgagcaccgtcgcctcctttcctccagggctccgtaggcaccaactgc
aaggacccctcccctgcgggcgctcccatg (Seq ID No: 101)
```

Homo sapiens cadherin 13, H-cadherin (heart) (CDH13):

```
gagcctctcctcaaagcctggctcccacggaaaatatgctcagtgcagccgcgtgcatgaatgaaa
acgccgccgggcgcttctagtcggacaaaatg (Seq ID No: 102)
```

Homo sapiens regulator of chromosome condensation (RCC1) and BTB (POZ) domain containing protein 2 (RCBTB2):

cgctcccttcgtttccgtctcggccgggcacccgagcgcatcccgccgaggccgggccgttttcagg
gggaggcgccaactcatcgcggcgccgggcccctgaccgtgcagtaaccgctaccaggaggcgga
gcggacaaggctccggcctgcgaggagtcacattaactttgctctagaagacaactttacaaggat
ctaaaaggaacaggattaaagatgactgaatactgggttccagaaatttaaaacaatcagcttagc
aaatcatatattcttctgtggagctgagaattgatgtccgctcttccccgtgatttggaactttcc
aatcccagagaaaagttgacaaagggactgcccaggactgagtccatatg
(Seq ID No: 103)

Homo sapiens cold inducible RNA binding protein (CIRBP):

ccccccctcactcgcgcgttaggaggctcgggtcgttgtggtgcgctgtcttcccgcttgcgtcag
ggacctgcccgactcagtggccgccatg (Seq ID No: 104)

Homo sapiens LIM domain binding 2 (LDB2):

cctcctctcctctccctctcctctcctgctatagagggctccgacagcagttcccagccagcgtgt
tcagcctgcctgcctgcctcgtgtgtgtgtgagcgtgtgtgcgtgcgtctactttgtactg
ggaagaacacagcccatgtgctctgcatggacgttactgatactctgtttagcttgattttcgaaa
agcaggcaagatg (Seq ID No: 105)

Homo sapiens chloride channel, nucleotide-sensitive, 1A (CLNS1A):

ctgcctcttccagggcgggcggtgtggtgcacgcattgctgtgctccaactccctcagggcctgtg
ttgccgcactctgctgctatg (Seq ID No: 106)

Homo sapiens collapsin response mediator protein 1 (CRMP1):

cctcctccttctcccgccctcctcgccgatccgggcggtgctggcagccggagcggcggcgggcgg
gccgagcagccggggcagccgcgcgtgggcatccacgggcgccgagcctccgtccgtgtctctatc
cctcccgggcctttgtcagcgcgcccgctgggagcggggccgagagcgccggttccagtcagacag
ccccgcaggtcagcggccgggccgagggcgccagaggggggccatg (Seq ID No: 107)

Homo sapiens catenin (cadherin-associated protein), delta 1 (CTNND1):

ttgcctttggctgggtgcaacttccattttaggtgttggatctgagggggaaaaaaaagagagagg
gagagagagagaaagaagagcaggaaagatcccgaaaggaggaagaggtggcgaaaatcaactgc
cctgctggatttgtctttctcagcaccttggcgaagccttgggtttctttcttaaaggactgattt
ttagaactccacatttgaggtgtgtggcttttgaagaaatgtatgtactgacgggaaaggagga
taagcaagtcgaatttttgtcttacgctctctccttcctgcttcctccttgctgtggtggctggga
tgcttcttccatgattttttgaatctagactgggctgttctctgtgttaaaccaatcagttgcgac
cttctcttaacagtgtgaagtgaggggtctctctccctccttctccttcctctgtgattcacctt
ccttttaccctgccctgcggcggctccgccccttaccttcatg (Seq ID No: 108)

Homo sapiens diacylglycerol kinase, alpha 80kDa (DGKA):

ccgtcccctccagcccagctcgggctccagctccagcgccggcgcttcagctgcgaccgcgagccc
tctcaagcaagatataacttccccaagtcacacagtggtatcagagctaagaatgggacccagata
tgactgatctagttctgttccaaaaccgtgctgtattatattaacgcctaccctctgaagaggtcc
aagcaacggaagtactactacgaagctgcctttctggccatccttgagaaaaatagacagatgagt
tcctgccagtgagtccctaggcctccatctctctcccttgctgtaccaccttcaccaccatccatg
cgaccccaagagccttaatgactctagaagagactccaggcaggggaagctgaaaggacctttcac
tccctacttttggccagggccttctgtgccacctgccaagaccagcaggcctaccctctgaagagg
tccaagcaacggaagtactactacgaagctgcctttctggccatccttgagaaaaatagacagatg
(Seq ID No: 109)

Homo sapiens aspartyl-tRNA synthetase (DARS):

cgatctttctggagccgcacctccacgcggagtccgagcgcgtgtgctgagaccccagggtcgggga
gggcggagactgggagggagggagaagcccctttggcctgccttacggaagcctgcgagggagggt
ggtgtccactgcccagttccgtgtcccgatg (Seq ID No: 110)

Homo sapiens dynein, cytoplasmic 1, intermediate chain 2 (DYNC1I2):

agttcttctcgatcgtgtcagtttgtaaggcgagggcggaagttggattcctggcctgagaatatt
aggcgtagttttccagttttttggcaaagcggaaatacttaaggcccctgggttgactgggttcttt
gttttatctaccggcttctgctttacgacaggtcacaaacatg (Seq ID No: 111)

Homo sapiens dedicator of cytokinesis 1 (DOCK1):

tttcctccccatcctgtcgcggctcgaaaggaatggaaaatggcggcctagacgcggagtttcctg
cccgacccgcggcggctccggcggcgccatg (Seq ID No: 112)

Homo sapiens dihydropyrimidinase-like 2 (DPYSL2):

ctctctctttttttttccgccctagctggggctgtgttggaggagaggaagaaagagagacagagga
ttgcattcatccgttacgttcttgaaatttcctaatagcaagaccagcgaagcggttgcacccttt
tcaatcttgcaaaggaaaaaaacaaaacaaaacaaaaaaaacccaagtccccttcccggcagtttt
tgccttaaagctgccctcttgaaattaattttttcccaggagagagatg (Seq ID No: 113)

Homo sapiens developmentally regulated GTP binding protein 2 (DRG2):

tgttctctttggcttccgggcgcacgctactctgtcgccgccgtcagaccggaattgccggtgccg
ccgccaccgctgtctgtgcgcccacctctgctgctaccatg (Seq ID No: 114)

Homo sapiens eukaryotic translation elongation factor 1 alpha 1 (EEF1A1):

cgttctttttcgcaacgggtttgccgccagaacacaggtgtcgtgaaaactacccctaaaagccaa
aatg (Seq ID No: 115)

Homo sapiens eukaryotic translation elongation factor 1 gamma (EEF1G):

tctcctctttccccctcccttctctcccgggcggcttactttgcggcagcgccgagaaccccaccc
cctttctttgcggaatcaccatg (Seq ID No: 116)

Homo sapiens eukaryotic translation initiation factor 2, subunit 3 gamma, 52kDa (EIF2S3): atttccttcctcttttggcaacatggcgggc (Seq ID No: 117)

Homo sapiens eukaryotic translation initiation factor 4B (EIF4B): gggtcttttgcgttctctttccctctcccaacatg (Seq ID No: 118)

Homo sapiens eukaryotic translation initiation factor 4 gamma, 2 (EIF4G2): tattcttttgaagattcttcgttgtcaagccgccaaagtg (Seq ID No: 119)

Homo sapiens epithelial membrane protein 1 (EMP1):

```
cttcccctcagtgcggtcacatacttccagaagagcggaccagggctgctgccagcacctgccact
cagagcgcctctgtcgctgggacccttcagaactctctttgctcacaagttaccaaaaaaaaaaga
gccaacatg (Seq ID No: 120)
```

Homo sapiens fibrillarin (FBL):

```
cgctcttttccacgtgcgaaagccccggactcgtggagttgtgaacgccgcggactccggagccgc
acaaaccagggctcgccatg (Seq ID No: 121)
```

Homo sapiens exostoses (multiple)-like 2 (EXTL2):

```
ctgtcccttgctccaggcgctcactttgcgggcggcactttttccaggttgttaatccagctaatg
gagaaggatagatgcacgctacttggtttagaaaaaaaaacaaaaatgagcaaacgagacgcccct
tccgttttatgataactaagctgcagggaaataaatcggctggccctactgcaatctactgcactc
gagaaacatcacagaaaattctttgatttatcttaatagtgacaagtgagcctgcttctgtcaatt
actgaagctataaggagattttttaaaaattaaacttcaacacaatg (Seq ID No: 122)
```

Homo sapiens solute carrier family 37 (glucose-6-phosphate transporter), member 4 (SLC37A4):

```
ccgcctctgttcaggacactgggtccccttggagcctccccaggcttaatgattgtccagaaggcg
gctataaagggagcctgggaggctgggtggaggagggagcagaaaaaacccaactcagcagatctg
ggaactgtgagagcggcaagcaggaactgtggtcagaggctgtgcgtcttggctggtagggcctgc
tcttttctaccatg (Seq ID No: 123)
```

Homo sapiens GDP dissociation inhibitor 2 (GDI2):

```
agccctcccctcctcgctccctcccctcctctccccgcccagttcttctcttcccgtctgaggtgg
cggtcggtctcgccttgtcgccagctccattttcctctctttctcttccccttttccttcgcgccca
agagcgcctcccagcctcgtagggtggtcacggagcccctgcgcctttccttgctcgggtcctgc
gtccgcgcctgccccgccatg (Seq ID No: 124)
```

Homo sapiens UDP-Gal:betaGlcNAc beta 1,4- galactosyltransferase, p olypeptide 1 (B4GALT1): caccttcttaaagcg-gcggcgggaagatg (Seq ID No: 125)

Homo sapiens GDP-mannose 4,6-dehydratase (GMDS):

```
ggccctccctgcacggcctcccgtgcgcccctgtcagactgtggcggccggtcgcgcggtgcgctc
tccctccctgcccgcagcctggagaggcgcttcgtgctgcacacccccgcgttcctgccggcaccg
cgcctgccctctgccgcgctccgccctgccgccgaccgcacgcccgccgcgggacatg
(Seq ID No: 126)
```

Homo sapiens histone deacetylase 2 (HDAC2):

```
ggccccctcctcgcgagttggtgccgctgccacctccgattccgagctttcggcacctctgccggg
tggtaccgagccttcccggcgccccctcctctcctcccaccggcctgcccttccccgcgggactat
cgcccccacgtttccctcagccctttctctcccggccgagccgcggcggcagcagcagcagcagc
agcagcaggaggaggagcccggtggcggcggtggccggggagcccatg (Seq ID No: 127)
```

Homo sapiens protein arginine methyltransferase 2 (PRMT2):

```
gggccttcccggctgacggcctgcgtgcactgcgcttgcgcgggttgagggcggtggctcaggctc
ctggaaaggaccgtccacccctccgcgctggcggtgtggacgcggaactcagcggagaaacgcgat
tgagagcagtgtgtggattacactatcactggaaaaatacgaattgagaagaaggaaaagactgga
agatgcagaccttggttcctgttagtggaaacactgtaaggtcccagaaatggaaaagaaaatgaa
ataaatcagcagttatgaggcagagcctaagagaactatg (Seq ID No: 128)
```

Homo sapiens immunoglobulin (CD79A) binding protein 1 (IGBP1): gttcctctctccccaagatg (Seq ID No: 129)

Homo sapiens eukaryotic translation initiation factor 3, subunit E (EIF3E): actcccttttctttggcaagatg (Seq ID No: 130)

Homo sapiens activated leukocyte cell adhesion molecule (ALCAM):

```
gtccctctactcagagcagcccggagaccgctgccgccgctgccgctgctaccaccgctgccacct
gaggagacccgccgcccccccgtcgccgcctcctgcgagtccttcttagcacctggcgtttcatgc
acattgccactgccattattattatcattccaatacaaggaaaataaaagaagataccagcgaaaa
gaaccgcttacacctttccgaattactcaagtgtctcctggaaacagagggtcgttgtccccggag
gagcagccgaagggcccgtgggctggtgttgaccgggagggaggaggagttgggggcattgcgtgg
tggaaagttgcgtgcggcagagaaccgaaggtgcagcgccacagcccaggggacggtgtgtctggg
agaagacgctgcccctgcgtcgggacccgccagcgcgcgggcaccgcggggcccgggacgacgccc
cctcctgcggcgtggactccgtcagtggcccaccaagaaggaggaggaatatg
(Seq ID No: 131)
```

Homo sapiens acyloxyacyl hydrolase (neutrophil) (AOAH):

```
ttttctttatcctgcagtctttacctcagcagaaccgcacaccacagactccctccagctctttgt
gtgtggctctctcagggtccaacaagagcaagctgtgggtctgtgagtgtttatgtgtgcttttat
```

```
tcacttcacacttattgaaaagtgtgtatgtgagagggtggggtgtgtgtgtcaaagagagtgagg
aagagaaggagagagagatcaattgattctgcagcctcagctccagcatccctcagttgggagctt
ccaaagccgggtgatcacttggggtgcatagctcggagatg (Seq ID No: 132)
```

Homo sapiens ADP-ribosylation factor 1 (ARF1):

```
ccgccccttacccggcgtgccccgcgcccggaggcgctgacgtggccgccgtcagagccgccatct
tgtggggagcaaaaccaacgcctggctcggagcagcagcctctgaggtgtccctggccagtgtcctt
ccacctgtccacaagcatg (Seq ID No: 133)
```

Homo sapiens ADP-ribosylation factor 6 (ARF6):

gcgccttttccggcagcggcggcggcagaactgggaggaggagttggaggccggagggagcccgcg
ctcggggcggcggctggaggcagcgcaccgagttcccgcgaggatccatgacctgacggggcccccg
gagccgcgctgcctctcgggtgtcctgggtcggtggggagcccagtgctcgcaggccggcggggcgg
gccggagggctgcagtctccctcgcggtgagaggaaggcggaggagcgggaaccgcggcggcgctc
gcgcggcgcctgcggggggaagggcagttccgggccgggccgcgcctcagcagggcggcggctccc
agcgcagtctcagggcccgggtggcggcggcgactggagaaatcaagttgtgcggtcggtgatgcc
cgagtgagcggggggcctgggcctctgcccttaggaggcaactcccacgcaggccgcaaaggcgct
ctcgcggccgagaggcttcgtttcggtttcgcggcggcggcggcgttgttggctgaggggacccgg
gacacctgaatgcccccggccccggctcctccgacgcgatg (Seq ID No: 134)

Homo sapiens ras homolog family member A (RHOA):

cgccctcccgccgccgcccgccctcgctctctcgcgctaccctcccgccgcccgcggtcctccgtc
ggttctctcgttagtccacggtctggtcttcagctacccgccttcgtctccgagtttgcgactcgc
ggaccggcgtccccggcgcgaagaggctggactcggattcgttgcctgagcaatg
(Seq ID No: 135)

Homo sapiens ras homolog family member G (RHOG):

cggcctcccgctctcacttccttctcgagcccggagccgctgccgccgcccccagctcccccgcct
cggggagggcaccaggtcactgcagccagaggggtccagaagagagaggaggcactgcctccacta
cagcaactgcacccacgatg (Seq ID No: 136)

Homo sapiens ATP synthase, H+ transporting, mitochondrial F1 compl ex, O subunit (ATP5O): ctctcttcccactcgggtttgacctacagccgcccgggagaa-gatg (Seq ID No: 137)

Homo sapiens B lymphoid tyrosine kinase (BLK):

ccacctctgtctgctgccggcagaaagccacaagccatgaaaactgattgagatgagaagaattca
tctgggactggcttttgctttaggatggtgttggaagttgctcgttgtcgctaggagcctgctcca
ctgtaagggtgtcaggatctgaagagctatggtgaaacaccactgaagcattgccaaggatg
(Seq ID No: 138)

Homo sapiens B-cell translocation gene 1, anti-proliferative (BTG1):

gcatctcttcgcctctcggagctggaaatgcagctattgagatcttcgaatgctgcggagctggag
gcggaggcagctgggggaggtccgagcgatgtgaccaggccgccatcgctcgtctcttcctctctcc
tgccgcctcctgtctcgaaaataactttttttagtctaaagaaagaaagacaaaagtagtcgtccgc
ccctcacgccctctcttcctctcagccttccgcccggtgaggaagcccggggtggctgctccgccg
tcggggccgcgccgccgagccccagccgccccgggccgcccccgcacgccgccccatg
(Seq ID No: 139)

Homo sapiens calcium modulating ligand (CAMLG):

cggcctctagtcatcgccctcgcagcggcggccaacatcaccgccactgccacccctcccagactg
tggacgggaggatg (Seq ID No: 140)

Homo sapiens calnexin (CANX):

aggcctcttggttctgcggcacgtgacggtcgggccgcctccgcctctctctttactgcggcgcgg
ggcaaggtgtgcgggcgggaaggggcacgggcacccccgcggtccccgggaggctagagatcatg
(Seq ID No: 141)

Homo sapiens calpain 2, (m/II) large subunit (CAPN2): cgacctttctctgcgcagtacggccgccgggaccgcagcatg (Seq ID No: 142)

Homo sapiens caveolin 1, caveolae protein, 22kDa (CAV1):

gcgcctttttttccccccatacaatacaagatcttccttcctcagttcccttaaagcacagcccag
ggaaacctcctcacagttttcatccagccacgggccagcatg (Seq ID No: 143)

Homo sapiens CD1d molecule (CD1D):

cgacctctttgcagctcgcacagctaagggcgagggcgcccttcggcagaagcagcaaaccgccgg
caagcccagcgaggagggctgccgggggtctgggcttgggaattggctggcacccagcggaaaggga
cgtgagctgagcggcggggggagaagagtgcgcaggtcagagggcggcgcgcagcggcgctccgcga
ggtccccacgccgggcgatatg (Seq ID No: 144)

Homo sapiens CD22 molecule (CD22):

tctccttttgctctcagatgctgccagggtccctgaagagggaagacacgcggaaacaggcttgca
cccagacacgacaccatg (Seq ID No: 145)

Homo sapiens CD37 molecule (CD37):

cttcctcttttggggttcttcctttctctctcagctctccgtctctctttctctctcagcctcttt
ctttctccctgtctcccccactgtcagcacctcttctgtgtggtgagtggaccgcttacccacta
ggtgaagatg (Seq ID No: 146)

Homo sapiens CD38 molecule (CD38):

gcctctctcttgctgcctagcctcctgccggcctcatcttcgcccagccaaccccgcctggagccc
tatg (Seq ID No: 147)

Homo sapiens CD48 molecule (CD48): cggccttttctagccaggctctcaactgtctcctgcgttgctgggaagttctggaaggaagcatg (Seq ID No: 148)

Homo sapiens chromogranin B (secretogranin 1) (CHGB): cttcctttccgcacaggggccgccgagcggggccatg (Seq ID No: 149)

Homo sapiens chloride channel, voltage-sensitive 3 (CLCN3):

ttcccccttccgtgggtcagggccggtccggtccggaacctgcagcccctttcccagtgttctagtt
cgcccgtgacccggaataatgagcaaggagggtgtggtgggttgaaagccatcctactttactccc
gagttagagcatggattcagtttatgtcttaagggggaagtgagattggagattttttattttaat
tttgggcagaagcaggttgactctagggatctccagagcgagaggatttaacttcatgttgctccc
gtgtttgaaggaggacaataaaagtcccaccgggcaaaattttcgtaacctctgcggtagaaaacg
tcaggtatcttttaaatcgcgatagttttcgctgtgtcaggctttcttcggtggagctccgagggt
agctaggttctaggtttgaaacagatgcagaatccaaaggcagcgcaaaaaacagccaccgatttt
gctatgtctctgagctgcgagataatcagacagctaaatg (Seq ID No: 150)

Homo sapiens colipase, pancreatic (CLPS):

```
ttccccttccgtgggtcagggccggtccggtccggaacctgcagcccctttcccagtgttctagtt
cgcccgtgacccggaataatgagcaaggagggtgtggtgggttgaaagccatcctactttactccc
gagttagagcatggattcagtttagtcttaaggggggaagtgagattggagattttttatttttaat
tttgggcagaagcaggttgactctagggatctccagagcgagaggatttaacttcatgttgctccc
gtgtttgaaggaggacaataaaagtcccaccgggcaaaattttcgtaacctctgcggtagaaacg

tcaggtatcttttaaatcgcgatagttttcgctgtgtcaggctttcttcggtggagctccgagggt
agctaggttctaggtttgaaacagatgcagaatccaaaggcagcgcaaaaaacagccaccgatttt
gctatgtctctgagctgcgagataatcagacagctaaatg (Seq ID No: 151)
```

Homo sapiens cytochrome c oxidase subunit IV isoform 1 (COX4I1):

```
ctaccctttttccgctccacggtgacctccgtgcggccgggtgcgggcggagtcttcctcgatcccg
tggtgctccgcggcgcggccttgctctcttccggtcgcgggacaccgggtgtagagggcggtcgcg
gcgggcagtggcggcagaatg (Seq ID No: 152)
```

Homo sapiens cytochrome c oxidase subunit VIIc (COX7C):

```
ctttcttttcagtccttgcgcaccgggggaacaaggtcgtgaaaaaaaaggtcttggtgaggtgccg
ccatttcatctgtcctcattctctgcgcctttcgcagagcttccagcagcggtatg
(Seq ID No: 153)
```

Homo sapiens activating transcription factor 2 (ATF2):

```
cagccttttcctccagggggtgctttgtaaacacggctgtgctcagggctcgcgggtgaccgaaagg
atcatgaactagtgacctggaaagggtactagatggaaacttgagaaaggactgcttattgataac
agctaaggtattcctggaagcagagtaaataaagctcatggcccaccagctagaaagtattcttgc
catgagaaaaagaatgtgataagttattcaacttatg (Seq ID No: 154)
```

Homo sapiens casein kinase 1, alpha 1 (CSNK1A1):

```
agatccctttcccagagtgctctgcgccgtgaagaagcggctcccgggggactggggggcattttgtg
ttggctggagctggagtaacaagatggcgtcgtccgcggagtgacaggggtccctctgggccggag
ccggcggcagtggtggcagcggtatcgccgccctagctcaccgcgcccctttttccagcccgcgacg
tcgccgcgcaagcgaggcagcggcggccgccgagaaacaagtggcccagcctggtaaccgccgaga
agcccttcacaaactgcggcctggcaaaaagaaacctgactgagcggcggtgatcaggttcccctc
tgctgattctgggccccgaaccccggtaaaggcctccgtgttccgtttcctgccgccctcctccgt
agccttgcctagtgtaggagccccgaggcctccgtcctcttcccagaggtgtcggggcttggcccc
agcctccatcttcgtctctcaggatg (Seq ID No: 155)
```

Homo sapiens catenin (cadherin-associated protein), beta 1, 88kDa (CTNNB1):

```
aagcctctcggtctgtggcagcagcgttggcccggcccccgggagcggagagcgaggggaggcggag
acggaggaaggtctgaggagcagcttcagtccccgccgagccgccaccgcaggtcgaggacggtcg
gactcccgcggcgggaggagcctgttcccctgagggtatttgaagtataccatacaactgtttttga
aaatccagcgtggacaatg (Seq ID No: 156)
```

Homo sapiens dCMP deaminase (DCTD):

ccgcctcctcccccgacttccttccctgagcacggcggcggcggggacgagcaccggcctgcgcgc
ggagccggcaccggatgacccaacatg (Seq ID No: 157)

Homo sapiens damage-specific DNA binding protein 1, 127kDa (DDB1):

ctgtcttttcgcttgtgtccctctttctagtgtcgcgctcgagtcccgacgggccgctccaagcct
cgacatg (Seq ID No: 158)

Homo sapiens desmin (DES):

ctgtctccctcgccgcatccactctccggccggccgcctgcccgccgcctcctccgtgcgcccgc
cagcctcgcccgcgccgtcaccatg (Seq ID No: 159)

Homo sapiens deoxyhypusine synthase (DHPS):

cgttccctacttcctgtgctcttgcggagacgcgcgcgtcggggtttaacgcgtttctgggccgcc
gtaagcccggcctaggggcagctttgactcgagagccggctataggcgcatg
(Seq ID No: 160)

Homo sapiens dihydrolipoamide S-acetyltransferase (DLAT):

caccctttcggatgcctcccctagaaccctaccactttccaccccttccgtctgttatttctccc
aaacttgcgcccgcacaggcccctctggaacactcctgccccgtagtgcccctcgtccccgctccg
tagagaaagagcgtgcgtgccgcgcatttctggcctggggagcgggtggagtaaacctgcgggaac
cattttacgacaacgtgcggctgtgcggtgtggctgacggcaacgccgctgctcttggagaggtca
ctccggagacggcgttggttttggggtgtggggggttggtggcactatg (Seq ID No: 161)

Homo sapiens down-regulator of transcription 1, TBP-binding (negative cofactor 2) (DR1):

ccttccctggcatctggagggaccaccgttgccgcgtcttcggcttccacgatctgcgttcgggct
acgcggccacggcggcagccactgcgactcccactgtgcctggctctgtccatattagttcccagg
cggccgtcgccgttccagcagcggcagcggcagcggcagcggcggacatgttgtgaggcggcggcg
cgggtgtctgaaggatggtttggccgaggcggcggcaacggctgctggcggcggcggcagcggcag
cggggcctcgggctctatagagccgagcccgctgggtacccgcccggtaccgcggcgaggccagtg
cccctggatcttgcctctgctccgacgccgttggggaccagttaggcgacagcgcccgcccctctg
aggagacacgaaggtggttccccagccgctcaaatttccggaccaccgcgctttcccctcctcagc
ctgggctgtgctctctctagaatcctcgggcccccactttcttcccaaactcatcctaaatctctc
acacacgcgagtgttcccagccctcaagccagctgctcctccgttcattttctgcaccctcttcgc
aaagcacccccccgggatcactctccgagggcgactttttgagaaatctcggtggagtagtggacca
gagctggggagttttttaaaagccggggcgcgagaaacaggaaggtactatg
(Seq ID No: 162)

Homo sapiens endothelin receptor type A (EDNRA):

ttttcttttcgtgcgagccctcgcgcgcgcgtacagtcatcccgctggtctgacgattgtggaga
ggcggtggagaggcttcatccatcccacccggtcgtcgccggggattggggtcccagcgagacctc
cccgggagaagcagtgcccaggaggtttctgaagccggggaagctgtgcagccgaagccgccgcc
gcgccggagcccgggacaccggccaccctccgcgccacccaccctcgccggctccggcttcctctg
gcccaggcgccgcgcggacccggcagctgtctgcgcacgccgagctccacggtgaaaaaaaagtga
aggtgtaaaagcagcacaagtgcaataagagatatttcctcaaatttgcctcaagatg
(Seq ID No: 163)

Homo sapiens eukaryotic translation elongation factor 1 alpha 2 (EEF1A2):

```
cagtccctctggctgagacctcggctccggaatcactgcagccccctcgccctgagccagagcac
cccgggtcccgccagcccctcacactcccagcaaaatg (Seq ID No: 164)
```

Homo sapiens eukaryotic translation elongation factor 2 (EEF2):

```
cgttctcttccgccgtcgtcgccgccatcctcggcgcgactcgcttctttcggttctacctgggag
aatccaccgccatccgccaccatg (Seq ID No: 165)
```

Homo sapiens eukaryotic translation initiation factor 4A2 (EIF4A2): ctgtcttttcagtcgggcgctgagtggtttttcggatcatg (Seq ID No: 166)

Homo sapiens egf-like module containing, mucin-like, hormone recep tor-like 1 (EMR1): gtttcttttctttgaatgacagaactacagcataatg (Seq ID No: 167)

Homo sapiens enolase 2 (gamma, neuronal) (ENO2):

```
gcgcctcctccgcccgccgcccgggagccgcagccgccgccgccactgccactcccgctctctcag
cgccgccgtcgccaccgccaccgccaccgccactaccaccgtctgagtctgcagtcccgagatccc
agccatcatg (Seq ID No: 168)
```

Homo sapiens esterase D (ESD):

```
ccgcctttacttcggcccgcttcttctggtcactccgccaccgtagaatcgcctaccatttggtg
caagcaaaaagcaatcagcaattggacaggaaaagaatg (Seq ID No: 169)
```

Homo sapiens Finkel-Biskis-Reilly murine sarcoma virus (FBR-MuSV) ubiquitously expressed (FAU): cttcctctttctcgactccatcttcgcggtagctgggaccgccgttcagtcgccaatatg (Seq ID No: 170)

Homo sapiens Friend leukemia virus integration 1 (FLI1):

```
ctgtctctttcgctccgctacaacaacaaacgtgcacaggggagtgagggcagggcgctcgcaggg
ggcacgcagggagggcccagggcgccagggaggccgcgccgggctaatccgaaggggctgcgaggt
caggctgtaaccgggtcaatgtgtggaatattggggggctcggctgcagacttggccaaatg
(Seq ID No: 171)
```

Homo sapiens fibromodulin (FMOD):

```
gcccctttttcacaatatttgattaggaatttggggcgggaccctggtctggcacaggcacgcacac
tctcagtagactctttcactcctctctctcttcctctctcacacgttctccaacccaaggaggcca
gacagagggacgtggtcactctctgaaaagttcaacttgagagacaaaatg
(Seq ID No: 172)
```

Homo sapiens ferritin, heavy polypeptide 1 (FTH1):

```
cgttcttcgccgagagtcgtcggggtttcctgcttcaacagtgcttggacggaacccggcgctcgt
tccccacccggccggccgcccatagccagccctccgtcacctcttcaccgcaccctcggactgcc
ccaaggccccgccgccgctccagcgccgcgcagccaccgccgccgccgcctctccttagtcg
ccgccatg (Seq ID No: 173)
```

Homo sapiens glyceraldehyde-3-phosphate dehydrogenase (GAPDH):

```
cgctctctgctcctcctgttcgacagtcagccgcatcttcttttgcgtcgccagccgagccacatc
gctcagacaccatg (Seq ID No: 174)
```

Homo sapiens glycyl-tRNA synthetase (GARS): caccctctctggacagcccagggccgcaggctcatg (Seq ID No: 175)

Homo sapiens glutamic-oxaloacetic transaminase 2, mitochondrial (aspartate aminotransferase 2) (GOT2):

```
ctgtccttaccttcagcaggagccggttccctgtgtgtgtgtccgctcgccctctgctccgtcctg
cggctgcccactgccctcctacggtccaccatg (Seq ID No: 176)
```

Homo sapiens general transcription factor IIF, polypeptide 1, 74kD a (GTF2F1):

```
gcgcctcttccggttaccttttcccagcgccagaggcgcctagggttggggtcctcgctcaggcac
agagacccgacaccgagcggcggcttccccgggatcgagggacgcgcacgccagaggagacgaaag
gaacccgggtcggaccagatcggaaccactgaccattgcccatg (Seq ID No: 177)
```

Homo sapiens glycogen synthase 1 (muscle) (GYS1):

```
cggcctccttctgcctaggtcccaacgcttcggggcaggggtgcggtcttgcaataggaagccgag
cgtcttgcaagcttcccgtcgggcaccagctactcggccccgcaccctacctggtgcattccctag
acacctccggggtccctacctggagatccccggagcccccccttcctgcgccagccatg
(Seq ID No: 178)
```

Homo sapiens major histocompatibility complex, class I, C (HLA-C): cattctccccagaggccgagatg (Seq ID No: 179)

Homo sapiens major histocompatibility complex, class II, DP beta 1 (HLA-DPB1):

```
gctccctttagcgagtccttcttttcctgactgcagctcttttcattttgccatccttttccagct
ccatg (Seq ID No: 180)
```

Homo sapiens 3-hydroxy-3-methylglutaryl-CoA synthase 1 (soluble) (HMGCS1): ctgtcctttcgtggctcactccctttcctctgctgccgctcggtcacgcttgctctttcaccatg (Seq ID No: 181)

Homo sapiens hippocalcin (HPCA): ccgccttccctgcgcagtcggtgtctccgcgtcgctgggtgggacttggctcggcggccatg (Seq ID No: 182)

Homo sapiens hydroxysteroid (17-beta) dehydrogenase 2 (HSD17B2):

```
ctcccttcttgactctctgttcacagaactcaggctgcctccagccagcctttgcccgctagactc
actggccctgagcacttgaaggtgcagcaagtcactgagaatg (Seq ID No: 183)
```

Homo sapiens heat shock 60kDa protein 1 (chaperonin) (HSPD1):

```
ctgtccctcactcgccgccgacgacctgtctcgccgagcgcacgccttgccgccgccccgcagaaa
tg (Seq ID No: 184)
```

Homo sapiens intercellular adhesion molecule 3 (ICAM3): ccgccttttcccctgcctgcccttcgggcacctcaggaaggcaccttcctctgtcagaatg (Seq ID No: 185)

Homo sapiens inositol polyphosphate-1-phosphatase (INPP1):

```
cgtcctctggccgcgcctgcggccgcacgcccagcgcccctcgcctaacctcgcgcccgggccgcg
cctcctcctcctgctccccgccgcttccgtttctcgagggaaaggctgctgcctcctgctctg
tcctcatccccggcttagctgacggcccagagggtgggtgccaattccaccagcagctgcaactga
aaagcaaggttcagaaatg (Seq ID No: 186)
```

Homo sapiens interferon regulatory factor 2 (IRF2):

```
gtttcctctccttgttttgctttcgatctggactgttctcaggcaagccggggagtaacttttagt
tttgctcctgcgattattcaactgacgggctttcatttccatttcacataccctagcaacacttat
accttgcggaattgtattggtagcgtgaaaaaagcacactgagagggcaccatg
(Seq ID No: 187)
```

Homo sapiens inter-alpha-trypsin inhibitor heavy chain 2 (ITIH2):

```
ttttcttcttttttcttcttttcttaaagcgaactgtactcctctgctgttcctttgaacttggttc
agtaggaagaagtgatatcctccccagaccatctgctttggggagcttggcaaaactgtccagcaa
aatg (Seq ID No: 188)
```

Homo sapiens karyopherin (importin) beta 1 (KPNB1):

```
ccgccttcctccctccctcgctccctccctgcgcgccgcctctcactcacagcctcccttccttct
ttctccctccgcctcccgagcaccagcgcgctctgagctgcccccagggtccctcccccgccgcca
gcagcccatttggagggaggaagtaagggaagaggagaggaaggggagccggaccgactacccaga
cagagccggtgaatgggtttgtggtgaccccgcccccccaccccacccccttcccacccgaccc
ccaaccccatccccagttcgagccgccgcccgaaaggccgggccgtcgtcttaggaggagtcgcc
gccgccgccacctccgccatg (Seq ID No: 189)
```

Homo sapiens karyopherin alpha 3 (importin alpha 4) (KPNA3):

```
ctctcccctcctcccctcccgctccaagattcgccgccgccgccgccgcagccgcaggagtagc
cgccgccggagccgcgcgcagccatg (Seq ID No: 190)
```

Homo sapiens keratin 19 (KRT19): gctcctcccgcgaatcgcagcttctgagaccagggttgctccgtccgtgctccgcctcgccatg (Seq ID No: 191)

Homo sapiens laminin, beta 1 (LAMB1):

```
attcccttctttgggctcggggggctcccggagcagggcgagagctcgcgtcgccggaaaggaagac
gggaagaaagggcaggcggctcggcgggcgtcttctccactcctctgccgcgtccccgtggctgca
gggagccggcatg (Seq ID No: 192)
```

Homo sapiens ribosomal protein SA (RPSA):

```
ctgtcttttccgtgctacctgcagaggggtccatacggcgttgttctggattcccgtcgtaactta
aagggaaattttcacaatg (Seq ID No: 193)
```

Homo sapiens lymphocyte cytosolic protein 1 (L-plastin) (LCP1):

ttttctttcctggctgatgatttgtcattctagtcacttcctgccttgtgaccacacacccaggct
tgacaaagctgttctgcagatcagaaagaaggggttcctggtcatacaccagtactaccaaggaca
gcttttttcctgcaagatctgttacctaaagcaataaaaaatg (Seq ID No: 194)

Homo sapiens lectin, galactoside-binding, soluble, 1 (LGALS1):

ccatctctctcgggtggagtcttctgacagctggtgcgcctgcccgggaacatcctcctggactca
atcatg (Seq ID No: 195)

Homo sapiens SH2 domain containing 1A (SH2D1A):

ttctctcttttttgcacatctggctgaactgggagtcaggtggttgacttgtgcctggctgcagta
gcagcggcatctcccttgcacagttctcctcctcggcctgcccaagagtccaccaggccatg
(Seq ID No: 196)

Homo sapiens mannosidase, alpha, class 2A, member 1 (MAN2A1):

tgttcctttcccctccgcttctctgacctagctgcgcggccccggcccgggagctgccgaacccgc
gcctcccctgggtgaggaggacacgcctgccctcgtcgagaaaacttttcctgccgactcagttgg
ggcggcggtggcaggaagtgcgggcagcgacctctcctccgcctgccccgcgcgccctgccggagg
tcggcgctgagcttgcgatcaagtttgtggggcccccccttcccagttgccggcgagtctcgcctc
gagaggggcgcccgaccccggggagggcggcaggccagggcgaaggccaagggcgtgtggtggcgc
cggagactaggtgcggagcaaggcggggactcgcacccgcatccgagagcgcggaggtcgcgcagc
ccgggagaagggagcctccggcggctgcttcctagagtccacagtgcgctgtctcctttggctgag
gagagtgtcctggccccgagtctatcgaggaaaatg (Seq ID No: 197)

Homo sapiens myelin basic protein (MBP):

ccgcctcttttcccgagatgccccgggggagggaggacaacaccttcaaagacaggccctctgagtc
cgacgagctccagaccatccaagaagacagtgcagccacctccgagagcctggatgtgatg
(Seq ID No: 198)

Homo sapiens melanocortin 1 receptor (al-

pha melanocyte stimulating hormone receptor) (MC1R):
cattcttcccaggacctcagcgcagccctggcccaggaaggcaggagacagaggccaggacggtcc
agaggtgtcgaaatgtcctggggacctgagcagcagccaccagggaagaggcagggaggggagctga
ggaccaggcttggttgtgagaatccctgagcccaggcggtagatgccaggaggtgtctggactggc
tgggccatgcctgggctgacctgtccagccagggagagggtgtgagggcagatctggggtgtgccca
gatggaaggaggcaggcatgggggggacacccaaggcccctggcagcaccatgaactaagcaggaca
cctggaggggaagaactgtggggacctggaggcctccaacgactccttcctgcttcctggacagga
ctatg (Seq ID No: 199)

Homo sapiens malic enzyme 1, NADP(+)-dependent, cytosolic (ME1):

gggcctttcccagtgcggccgccgccgccacagctgcagtcagcaccgtcaccccagcagcatccg

ccgcctgcaccgcgcgtgcggcccgccccggcctgaccccgccgccgaacccggcgccagccatg
(Seq ID No: 200)

Homo sapiens myocyte enhancer factor 2C (MEF2C):

```
agctctctgctcgctctgctcgcagtcacagacacttgagcacacgcgtacacccagacatcttcg
ggctgctattggattgactttgaaggttctgtgtgggtcgccgtggctgcatgtttgaatcaggtg
gagaagcacttcaacgctggacgaagtaaagattattgttgttattttttttttttctctctctct
ctcttaagaaaggaaaatatcccaaggactaatctgatcgggtcttccttcatcaggaacgaatgc
aggaatttgggaactgagctgtgcaagtgctgaagaaggagatttgtttggaggaaacaggaaaga
gaaagaaaaggaaggaaaaaatacataatttcagggacgagagagagaagaaaaacggggactatg
(Seq ID No: 201)
```

Homo sapiens mannosyl (alpha-1,3-)-glycoprotein beta-1,2-N-acetylglucosaminyltransferase (MGAT1):

```
agcccttcttggggaagtcagctacccagcagcctgtagtcctcggctacccaccctcaccgcctg
gggtcccatggtgagacagctgggtgggcatcaggcttctgcagagggccaggccggagggagctg
ggcgagggagtggggctggctcctggcttgcaccggcctcgtggaatccaggcctcagacctgatc
gctggcgaaactggctctgtgcgctggagcccctggtcttctgcgtctgtcctcctcccggccaga
ctttactcctggctcagcgacaggtatttgctatggaagagctgtccctccctcccctcggtgggc
ctgggtccacctccacctcctcttcaggtccgcaccttcctcccctttaaaacaccagccgggcgc
agacccgttctaggcttttccatggtgcttccgccaaagcttgtgaccgagtccttcccgcctagg
gctggtgggcctcccctgctggtaggtctctcttcgctttctttactcagaactgaagctctcatt
ccccacccaccaaggaaaaacaaaagggaagaagccacagctggccccggcttgctttggcacagg
tgtttccccccggcccccgtcgggcaccctggttcctgttctgtccctgccccacgcgaccctgg
ggctcccacccgggctcctcagcctccctgggttggggtggggggactggctcccagcccttggc
ctagggtttggtgaacgcctttcctggactgcgggcccacttcaggcgcggctccaggctgggcag
ctgcgctggagggccgagggcaggggtggggtcgggcgtccaccctcagggttgcgccagggagcc
ggaaagccgactcccgaagttggggtcctgggaaaacttgggtcctgggttgactgagaagcggcg
gggaaaggaggcgggccaggaggaggggggcctggcggacgccggccggggggcggggcgcggcggg
gctgtcggtcacgcccctcagtccgccccgccccgccccgcctgccggggaagggccacgttgccc
gcccggccgtccggccccggcgcgccgcagaaagggctggcgagtcgaaaggcgaggcggccgcgg
cagcgcttgggacgcgcctgggcaccgggctcgctccctgcgccccggagcaggccaagttcgggg
ccaggacgtcgggaggacctggtgcatggctgcctcctaatcccatagtccagaggaggcatcct
aggactgcgggcaagggagccgggcaagcccagggcagccttgaaccgtccctggcctgccctcc
ccggtgggggccaggatg (Seq ID No: 202)
```

Homo sapiens mitogen-activated protein kinase kinase kinase 11 (MAP3K11):

```
ctgcctcccgcccccggggccaaagtacaaagggaggaggaagaagggagcggggtcggagccgtc
ggggccaaaggagacggggccaggaacaggcagtctcggcccaactgcggacgctccctccacccc
ctgcgcaaaaagacccaaccggagttgaggcgctgcccctgaaggccccaccttacacttggcggg
ggccggagccaggctccaggactgctccagaaccgagggaagctcgggtccctccaagctagcca
tggtgaggcgccggaggccccggggccccacccccccggcctgaccacactgccctgggtgccctc
ctccagaagcccgagatgcggggggccgggagacaacactcctggctccccagagaggcgtgggtc
tggggctgagggccagggcccggatgcccaggttccgggactagggccttggcagccagcggggggt
ggggaccacgggcacccagagaaggtcctccacatcccagcgccggctcccggccatg
(Seq ID No: 203)
```

Homo sapiens membrane protein, palmitoylated 1, 55kDa (MPP1):

```
ccgccttctccgcagccccgcaggccccgggccctgtcattcccagcgctgccctgtcttgcgttc
cagtgttccagcttctgcgagatg (Seq ID No: 204)
```

Homo sapiens v-myc myelocytomatosis viral oncogene homolog (avian) (MYC):

```
ggccctttataatgcgagggtctggacggctgaggacccccgagctgtgctgctcgcggccgccac
cgccgggccccggccgtccctggctcccctcctgcctcgagaagggcagggcttctcagaggcttg
gcgggaaaaagaacggagggagggatcgcgctgagtataaaagccggttttcggggctttatctaa
ctcgctgtagtaattccagcgagaggcagagggagcgagcgggcggccggctaggtggaagagcc
gggcgagcagagctgcgctgcgggcgtcctgggaagggagatccggagcgaataggggggcttcgcc
tctggcccagccctcccgctgatcccccagccagcggtccgcaaccttgccgcatccacgaaact
ttgcccatagcagcgggcgggcactttgcactggaacttacaacacccgagcaaggacgcgactct
cccgacgcggggaggctattctgcccatttggggacacttccccgccgctgccaggacccgcttct
ctgaaaggctctccttgcagctgcttagacgctg (Seq ID No: 205)
```

Homo sapiens nuclear cap binding protein subunit 1, 80kDa (NCBP1): tggcctctcggttccgcggcgcaccggagggcagcatg (Seq ID No: 206)

Homo sapiens necdin homolog (mouse) (NDN):

```
cttcctctccaggaatccgcggagggagcgcaggctcgaagagctcctggacgcagaggccctgcc
cttgccagacggcgcagacatg (Seq ID No: 207)
```

Homo sapiens NADH dehydrogenase (ubiquinone) 1 beta subcomplex, 5, 16kDa (NDUFB5): ccttcttcctcctgcccgtagtagccatg (Seq ID No: 208)

Homo sapiens NADH dehydrogenase (ubiquinone) Fe-S protein 4, 18kDa (NADH-coenzyme Q reductase) (NDUFS4): ccgtcctttcatcctggcgtttgcctgcagcaagatg (Seq ID No: 209)

Homo sapiens nuclear factor of kappa light polypeptide gene enhanc er in B-cells 2 (p49/p100) (NFKB2):

```
tgccccttccccggccaagcccaactccggatctcgctctccaccggatctcacccgccacacccg
gacaggcggctggaggaggcgggcgtctaaaattctgggaagcagaacctggccggagccactaga
cagagccgggcctagcccagagacatg (Seq ID No: 210)
```

Homo sapiens non-metastatic cells 2, protein (NM23B) expressed in (NME2):

```
gcccctcctccgccgccggctcccgggtgtggtggtcgcaccagctctctgctctcccagcgcagc
gccgccgcccggcccctccagcttcccggaccatg (Seq ID No: 211)
```

Homo sapiens nucleophosmin (nucleolar phosphoprotein B23, numatrin) (NPM1):

```
gcgtcctttccctggtgtgattccgtcctgcgcggttgttctctggagcagcgttctttttatctcc
gtccgccttctctcctacctaagtgcgtgccgccacccgatg (Seq ID No: 212)
```

Homo sapiens 5'-nucleotidase, ecto (CD73) (NT5E):

```
cattccttttgtagaaaaacccgtgcctcgaatgaggcgagactcagagaggacccaggcgcgggg
cggacccctccaattccttcctcgcgcccccgaaagagcggcgcaccagcagccgaactgccggcg
cccaggctccctggtccggccgggatgcggccggtacccgctccccgccgggaacaacctctccac
tcttcctgcagggagctggtgccagccgacagccgcgccagggccgctccgggtaccagggtcgga
tcgggtgacgtcgcgaacttgcgcctggccgccaagccggcctccaggctgaagaaggacccgccc
cggccttgacccgggccccgcccctccagccggggcaccgagcccggccctagctgctcgcccct
actcgccggcactcgcccggctcgcccgctttcgcacccagttcacgcgccacagctatg
(Seq ID No: 213)
```

Homo sapiens phosphatidylethanolamine binding protein 1 (PEBP1):

gcgtcttcccgagccagtgtgctgagctctccgcgtcgcctctgtcgcccgcgcctggcctaccgc
ggcactcccggctgcacgctctgcttggcctcgccatg (Seq ID No: 214)

Homo sapiens poly(A) binding protein, cytoplasmic 1 (PABPC1):

gcttccccttctccccggcggttagtgctgagagtgcggagtgtgtgctccgggctcggaacacac
atttattattaaaaaatccaaaaaaaatctaaaaaaatcttttaaaaaaccccaaaaaaatttaca
aaaaatccgcgtctcccccgccggagactttattttttttcttcctcttttataaaataacccgg
tgaagcagccgagaccgacccgcccgcccgcggccccgcagcagctccaagaaggaaccaagagac
cgaggccttcccgctgcccggacccgacaccgccaccctcgctccccgccggcagccggcagccag
cggcagtggatcgacccccgttctgcggccgttgagtagttttcaattccggttgattttgtccct
ctgcgcttgctccccgctcccctcccccggctccggcccccagccccggcactcgctctcctcct
ctcacggaaaggtcgcggcctgtggccctgcgggcagccgtgccgagatg
(Seq ID No: 215)

Homo sapiens proprotein convertase subtilisin/kexin type 2 (PCSK2):

cgctctttctctccggtacacacagctccccacattcgcacccctgcccgcgcgccgggccgcctg
actgcacggcttcccctccagccagatgctggagaacacacactgattcgctgctttccaagaccc
tgttcagtctctttctctatacaaagattttttttaaaaactatatataagaattctttatttgcac
cctccctccgagtcccctgctccgccagcctgcgcgcctcctagcaccacttttcactcccaaaga
aggatg (Seq ID No: 216)

Homo sapiens phosphogluconate dehydrogenase (PGD): gggtctttccctcactcgtcctccgcgcgtcgccgctcttcggttctgctctgtccgccgccatg (Seq ID No: 217)

Homo sapiens phosphoglucomutase 1 (PGM1):

cgctcccccttcccctcccgccggacctgccaggaggtgggctggcgcggagggagggccctgtcc
cctgtcccctttaaggaggagggccaaacgccggcctagagtgcggcgtagcccccacccgccgtgc
cctcaccccagagcagctgcagcctcagccggccgcccctccgccagccaagtccgccgctctgac
ccccggcagcaagtcgccaccatg (Seq ID No: 218)

Homo sapiens solute carrier family 25 (mitochondrial carrier; phosphate carrier), member 3 (SLC25A3):

cggcctctgtgagccgcaacctttccaagggagtggttgtgtgatcgccatcttagggagtgagtg
tggccggggccttctcctgtggcgggtgtggggagcggagcccagagctcctgtggggccgctgctt
tggcggtgggcccagccgggagcagcctctttcgaaggccgccgtgacctcttcaagggcgtggag
acgggaaggaaaaggccccggttggggttccagggcgccggtaacgttaaccggcgccttgcctgt
cctctaaccgtcgctccctcctcccctagaaagatg (Seq ID No: 219)

Homo sapiens pim-1 oncogene (PIM1):

cctccccttttactcctggctgcggggcgagccgggcgtctgctgcagcggccgcggtggctgagga
ggcccgagaggagtcggtggcagcggcggcggcgggaccggcagcagcagcagcagcagcagcagc
agcaaccactagcctcctgccccgcggcgctgccgcacgagccccacgagccgctcaccccgccgt
tctcagcgctgcccgaccccgctggcgcgccctcccgcgccagtcccggcagcgcctcagttgt
cctccgactcgccctcggccttccgcgccagccgcagccacagccgcaacgccacccgcagccaca
gccacagccacagccccaggcatagccttcggcacagccccggctccggctcctgcggcagctcct
ctgggcaccgtccctgcgccgacatcctggaggttgggatg (Seq ID No: 220)

Homo sapiens pyruvate kinase, muscle (PKM2):

```
ggatctcttcgtctttgcagcgtagcccgagtcggtcagcgccggaggtgagcggtgcaggaggct
acgccatcagtccccaccaagggccagtcgcccggctagtgcggaatcccggcgcgccggccggcc
ccgggcacgcaggcagggcggcgcaggatccagggcgtctgggatgcagtggagctcagagagagg


agaacggctcctcacgcctggggcctgctcttcagaagtccccagcgccgttccttccagatcagg
acctcagcagccatg (Seq ID No: 221)
```

Homo sapiens pleiomorphic adenoma gene-like 1 (PLAGL1):

```
cggcctcctcggcgcagccatcctcttggctgccgcgggcggcaaagcccacggcatctgccattt
gtcattcagcccgtcggtaccgccccgagccttgatttagacacggctggggcgtgctctggcctc
actctccgggcgggtgctggacggacggacggacggggcagccgtgctcacagctcagcagcgcgg
ggccttggcgcgcggggcgcttccccgggtcgccgtcatggccgcggaggtggcacgcccgagcgg
cctcgcctgagctccgggggtcgtcgccccgcagggattgctgtcacgtctaatgtggctgctgcc
tcgtgtcacatctgaaactcatctgtacctcacttagaaagtggttctgattagacaagacttttc
gttgcagtcgacagaaacctaatgggaccattgaagaattccaaacaggtatttgcataggaatca
gaggagttaatcttgtctcttctcacaggtttgaatcttcagacaaacttctgggaggactcggtc
cctgcctcgcagcagatgttccctgtcactcagtaggcatatg (Seq ID No: 222)
```

Homo sapiens phospholipase D2 (PLD2): tgctctcttggctccggaacccccgcgggcgctggctccgtctgccagggatg (Seq ID No: 223)

Homo sapiens proteolipid protein 2 (colonic epithelium-enriched) (PLP2):

```
cccccttcccggccagacggcgggcaagacagctgggtgtacagcgtcctcgaaaccacgagcaag
tgagcagatcctccgaggcaccagggactccagcccatgccatg (Seq ID No: 224)
```

Homo sapiens pinin, desmosome associated protein (PNN):

```
cagtcctttcgcgcctcggcggcgcggcatagcccggctcggcctgtaaagcagtctcaagcctgc
cgcagggagaagatg (Seq ID No: 225)
```

Homo sapiens phosphoribosyl pyrophosphate amidotransferase (PPAT): ggtccttccacgtgctttcggcggcgacatg (Seq ID No: 226)

Homo sapiens protein phosphatase 1, catalytic subunit, gamma isozy me (PPP1CC):

```
tgttcttctcgtggttccagtggggagagaaggaggaagtagggagcggggtggcaggggggggac
ccgccgcggctgctgccaccgccgccaccaccgcctctgctcgtggcgtgggaaaggaggtgtgag
tcccgggcgcgagccggcggcggcgccgctgcgggagggtcggcggtgggaaggcgatg
(Seq ID No: 227)
```

Homo sapiens protein phosphatase 1, regulatory subunit 8 (PPP1R8): cggtcttccagtttcccggcgtgcttagggcgcgccaaat-gggaggggggagacgcaagatg (Seq ID No: 228)

Homo sapiens protein phosphatase 6, catalytic subunit (PPP6C):

```
cggcctccgccgctgccgccgccgctgctacagccgccgccgccgctgttgccgcggcttgttatt
cttaaaatg (Seq ID No: 229)
```

Homo sapiens protein kinase C substrate 80K-H (PRKCSH):

ctttctttctgcagcaggaaccgcggctgctggacaagagggtgcggtggatactgacctttgct
ccggcctcgtcgtgaagacacagcgcatctccccgctgtaggcttcctcccacagaacccgtttcg
ggcctcagagcgtctggtgagatg (Seq ID No: 230)

Homo sapiens mitogen-activated protein kinase 6 (MAPK6):

cgccccctcttcctcgccctctctcgcgggtcggggttacatggcggcgactgcggcaaagcgaga
gcctcggagacgccgctgccgccagcacagccggagacctgagccgacactgggggcagtccgcga
gccccgcactctctcgatgagtcggagaagtcccgttgtatcagagtaagatggacggtagctttg

attgtgattgtggtgagctggagccacctgatcactaacaaaagacatcttctgttaaccaacagc
cgccagggcttcctgttgaaataaatatatagcaacaaaggaaaaaaagaagcaaaacggaaatag
tgcttaccagcaccttagaatgatgctgctcaggaccagtccaacactgaatgtatctgcactgtg
aggagaatgttcatagaagcctgttgtgtgcatatttattcacattttttgttaaatgttaaatcgt
ttagcacggtaatctgagtgcacagtatgtcatttcattccgtttgagtttcttgttttcgttaaa
tgtctgcagagttgctgccccttcttgaactatgagtactgcaatcttttaattctcaatatga
atagagcttttgagctttaaatctaaggggaactcgacaggcctgtttggcatatgcaatgaaca
tcaagaaaccatcttgctgtggaagcataattattttttcttctcccttttgaaagatctttcctt
ttgatgccagttttcttccttgtttacacaagttcaatttgaaaggaaaaggcaatagtaagggtt
tcaaaatg (Seq ID No: 231)

Homo sapiens phosphoribosyl pyrophosphate synthetase 2 (PRPS2):

cctcccccttccctacatctagccgccgcgctttcccgctcccgcagcagcagcctcccgcgtcgct
gtcgctgttgcctccgccacctcctccgccgccgcgcgcccctcggagttccgcgccccaccatg
(Seq ID No: 232)

Homo sapiens phosphoribosyl pyrophosphate synthetase-associated pr otein 1 (PRPSAP1):

ttgcctctggctctgaggcggcggcgccgggcgctgcgaaggctcggccgctgtagtcagtggtgt
ggggtgcgcaagggcacggacctcggagctctccccgcttgcgccgagtttctcagcgccttcccc
acccaaaccggggtctcgcagtcggaagcactcagagtgcagccccgcgcggggccggtcgtaacc
gcgccgcgggccggacgatg (Seq ID No: 233)

Homo sapiens proteasome (prosome, macropain) subunit, beta type, 5 (PSMB5): agttctttctgcccacactagacatg (Seq ID No: 234)

Homo sapiens proteasome (prosome, macropain) 26S subunit, non-ATPase, 13 (PSMD13): tgttcttctgtgccgggggtcttcctgctgtcatg (Seq ID No: 235)

Homo sapiens protein tyrosine phosphatase, receptor type, N (PTPRN):

cagcccctctggcaggctcccgccagcgtcgctgcggctccggcccgggagcgagcgcccggagct
cggaaagatg (Seq ID No: 236)

Homo sapiens RAB3A, member RAS oncogene family (RAB3A):

ctccctttgcaggacgtcacggaggactgcaggggcctgagccgctgctgccgccgccgcgca
gccccacatcaacgcaccggggtcctgtcaccgccaccgccaaaaaagtcaccgccgctagggtcg
ccgttgcatcggtgcagggcaagatg (Seq ID No: 237)

Homo sapiens RNA binding motif, single stranded interacting protei n 2 (RBMS2): ctctctctctctctctctcgctcgttccctaacattaaagagaaaatg (Seq ID No: 238)

Homo sapiens reticulocalbin 1, EF-hand calcium binding domain (RCN1):

```
gcgccctctgctccggctcggggcgggcactggcggagggactggccagtcccctcctccgcgcc
ggccccaaccctgtcgctgccgccgcgctccgagtccccattcccgagctgccgctgttgtcgctc
gctcagcgtctccctctcggccgccctctcctcgggacgatg (Seq ID No: 239)
```

Homo sapiens radixin (RDX):

```
ccgccttttcccgcggaggcgccgagcggccatattgcggagctgtctgcggtggcggcggcgcct
ctcgtctcccgcggcccagcgctcgcaccaccgcttctccctccctgtcgcagccgcgccgccgcg
```

```
cagcgccccagccacacgccggcgggcagaagccgcccgctctccggaaagtgataacagaattca
ttgaagtggagaatttttaaagaaggtaacaaaaagagaaagaaaatg (Seq ID No: 240)
```

Homo sapiens replication factor C (activator 1) 1, 145kDa (RFC1):

```
tcgccttcttgcacttcgcgggagaagttgttggcgcgaatggatcctgagcctcgataacagatt
cctcaaccggcccacccgccagccagccagcgccttcatcctggggctgcgatg
(Seq ID No: 241)
```

Homo sapiens ring finger protein 4 (RNF4):

```
gcatctttctcgaggagctctcctgggcggctgaagaaggagcttcttctccggagtgcgccggcg
gtggcgcctgcggacctaactagctccaggttaggccgagctttgcgggaaagcagcggacttgaa
aatactggaaatctgtccggatccaaattattttgcaagccagatgagtaaccagagggcatgaaa
ggttgagaacatttgacttccctgcaaaccttggtatagatcacttccttttctgtaggaaaggaa
aggcaccaaagagcacaatg (Seq ID No: 242)
```

Homo sapiens ribophorin I (RPN1): tgctcttcccggtcatg (Seq ID No: 243)

Homo sapiens ribosomal protein S27a (RPS27A): cgttcttcctttcgatccgccatctgcggtggagccgccaccaaaatg (Seq ID No: 244)

Homo sapiens secreted and transmembrane 1 (SECTM1):

```
cttcctttagcgtgaaccgcgggtgcggtgcctcccgtgaaaataataaattcaccgtcacgcttg
ttgtgaacgcgggtggttcccgaaacttggaggcttcccgtaaacccagctccttcctcatctggg
aggtgggtcccgcgcgggtccgccgcctcctccctggcccctccctctcgtgtctttcattttcct
ggggctccggggcgcggagaagctgcatcccagaggagcgcgtccaggagcggacccgggagtgtt
tcaagagccagtgacaaggaccaggggcccaagtcccaccagccatg (Seq ID No: 245)
```

Homo sapiens small glutamine-rich tetratricopeptide repeat (TPR)-containing, alpha (SGTA):

```
ctttcttttgcgcaggcgtcgcgccctggggccggggccgggcggcaccgcggtgcgcaagcgcaa
ccgtcggtgggtcggggatcggtcgcctgagaggtatcacctcttctgggctcaagatg
(Seq ID No: 246)
```

Homo sapiens SH3 domain binding glutamic acid-rich protein like (SH3BGRL):

```
agttctccttccaccttcccccacccttctctgccaaccgctgtttcagcccctagctggattcca
gccattgctgcagctgctccacagccctttttcaggacccaaacaaccgcagccgctgttcccagga
tg (Seq ID No: 247)
```

Homo sapiens solute carrier family 1 (glutamate/neutral amino acid transporter), member 4 (SLC1A4):

```
cgccctcctacttccccgtctgcgtccgcgttcgcggctcccgtttgcatcatccccgtctgcgtc
cgcgttcgcggctcccgtttgcatcatctccagccggcggctgctccagggaggctgggcgcgatc
ctctccgcccgcggctccaacccgcactctgcgcctctcctcgcctttctcgcacctgctcctgcg
ccaggcccggagacccccggggcggcttcccagaacctgcggagcacaactggccgaccgacccat
tcattgggaaccccgtcttttgccagagcccacgtcccctgccacctctagctcggagcggcgtgt
agcgccatg (Seq ID No: 248)
```

Homo sapiens small nuclear RNA activating complex, polypeptide 2, 45kDa (SNAPC2): ctgcctctttctgagcggcatg (Seq ID No: 249)

Homo sapiens sorting nexin 1 (SNX1): ctatctctcga-taaagttgttgttgcggcttccgccgcgggtggaagaagatg (Seq ID No: 250)

Homo sapiens signal recognition particle 54kDa (SRP54):

```
ctatctctcatctttccgctcttagctgggagtgctccgcctagtcacttttcttaaggtggctcg
tcgaggcctgacttcttccccgaaatcacgtccctagacagcctcctattttaccactaactttac
tcctgcagttattcagcggtaggaaactgaaaccaaaaaccagtgtaagcaagtaaacatctaaac
tgtttcaggagccgcgtagaaggaacgcggcggtgtgcccccggaagcggaagtagattctcctata
gaaaggctggactacgcggagtggtgacgtttcctcattgggcggaaggttcgctggcactccgtt
ggtcttccagctggtgggagttgacgacgtggtgctgggcgttgggaccctactttatctagttcg
ggaagttgggttgtggggtcatacctgtctgtctgctcccagctttcttgggtttcttccgacggc
gtggggcctcgctaaggaattcccggcccctcagggccacggctttagcggtgtctttgcgagtt
cttcgtaagtacatcttaaagctgtcaagatg (Seq ID No: 251)
```

Homo sapiens signal sequence receptor, beta (translocon-associated protein beta) (SSR2):

```
cggtctttcggatgctgacgctctcttcctgtctttgtggctccggaaaggcgtttgggatgccaa
cgatg (Seq ID No: 252)
```

Homo sapiens signal transducer and activator of transcription 6, i nterleukin-4 induced (STAT6):

```
ttttctttttggtggtggtggtggaaggggggaggtgctagcagggccagccttgaactcgctgga
cagagctacagacctatggggcctggaagtgcccgctgagaaagggagaagacagcagaggggttg
ccgaggcaacctccaagtcccagatcatg (Seq ID No: 253)
```

Homo sapiens suppressor of Ty 4 homolog 1 (S. cerevisiae) (SUPT4H1): tgttcttcccatcggcgaagatg (Seq ID No: 254)

Homo sapiens transcription factor 7 (T-cell specific, HMG-box) (TCF7):

```
ggtccttcccctaaaacttggcactgccgatactcccagcccgttccttcccaagtcaggaacttg
caggggacccccttggcaattcttttttctctcaagagcagacagccttcagtcccagccgctgccag
ggctggtgtgtctgacccagctgtggttttttccaggcctgaaggcccccggagtgcaccagcggcat
g (Seq ID No: 255)
```

Homo sapiens TEA domain family member 4 (TEAD4):

```
cagtctcctccccgaggtgccggtggccccgccgccactccctccggctccctccctcccgccgcg
gcgcgcatctcattccagccctcattccgcgcattccagcgtcctcctcgcacactcgaggccagg
gggcgggagggccgcagctccggcgccgccgcgtcccgccaggtgagaggcgcccgcgcccgccgc
accgccggcgccctcacgggccgcgcgccccacgccgccgcagccgaccgctcgcgccgcgtgct
cggctgctcttttctttccgccgcccgcgttcccgccttggacctctgcgctccgacgcgctccgt
cccgacctctggcttccctccgcgctccggcgctgctcgctgccctctcccgcttccctcctgtc
cgccccgcgctcccctcctcgctcccggttgactcactcctccaggaatagggatccccgtgtttt
ccgtcagtccattctgggaaaactcctccctccgcgcgctccgctccgctccgctgggcgcacc
ggggccggtcggcgcggggtgggcttggccccgcggccccgccttcactgcgccgcccgtcggccc
cggccggagcccggctctgcgcgctgacgccctgtcgtccccgcagaacgatcgccgcggccggaa
gagttggcgctcggggcggactccttggaactggcttagcgcacccatccaccttcccgcaccct
gggaccggtcggaacgagctgattgcccgctacatcaagctccggacagggaagacccgcaccagg
aagcaggtctccagccacatccaggtgctggctcgtcgcaaagctcgcgagatccaggccaagcta
aaggaccaggcagctaaggacaaggccctgcagagcatg (Seq ID No: 256)
```

Homo sapiens G protein-coupled receptor 137B (GPR137B):

```
ttttctttcctccagtctcggggctgcaggctgagcgcgatgcgcggagacccccgcggggggcggc
ggcggccgtgagccccgatg (Seq ID No: 257)
```

Homo sapiens tumor protein, translationally-controlled 1 (TPT1):

```
cggccttttccgcccgctccccccctcccccccgagcgccgctccggctgcaccgcgctcgctccgag


    tttcaggctcgtgctaagctagcgccgtcgtcgtctcccttcagtcgccatcatg
    (Seq ID No: 258)
```

Homo sapiens ubiquitin A-52 residue ribosomal protein fusion produ ct 1 (UBA52): ctatcttcttttttcttcagcgaggcg-gccgagctgacgcaaacatg (Seq ID No: 259)

Homo sapiens ubiquinol-cytochrome c reductase core protein II (UQCRC2):

```
cggcctccgccaccatcttgctttcctttaatccggcagtgaccgtgtgtcagaacaatcttgaat
catg (Seq ID No: 260)
```

Homo sapiens ubiquitin specific peptidase 1 (USP1):

```
ctgcctttcgtgtctctgcagcgtggagactggaaccggcaatttcaaaggacgccacgttcaatc
gcagcgctggcgcggggcggaggctaaaacacgggggtcctgagactgaggaaaacgcgccaagttc
ccctcggtggcggagtgctaaagaccctagcggttcaggcgttcggcgagcggggccgctgcttgt
tgcgctcctggctctcccggggcgggcgcagatgggcgccgctcccgggatgtagttggtgttggt
gcaagacgggagcgagcggcggtcggggttcccgctcttgggagcggatggtcactcccccgcggg
gagggcgagccgaccagattttcctggggccggggacccggcgggctcggggcagggactcacctg
tcgcacccacactcattcgggttggacttgccggcgtcaccgccgcggacttcgctttgggccatg
accagatataattggtgattacaactttcctctataaattaactcttgacactccttgggatttga
agaaaaaaatg (Seq ID No: 261)
```

Homo sapiens voltage-dependent anion channel 2 (VDAC2):

gtgtctccttcacttcgccctccagctgctggagctgcagcccgaccgcgagcgtgccaagcggct
tcagcagctagcggagcggtggcggcggcccccctcaggacaccaccagattcccctcttcccgcg
gcctcgccatg (Seq ID No: 262)

Homo sapiens vimentin (VIM):

gcctcttctccgggagccagtccgcgccaccgccgccgcccaggccatcgccaccctccgcagcca
tg (Seq ID No: 263)

Homo sapiens very low density lipoprotein receptor (VLDLR):

ccccctccccgctgctcacccogctctccggccgccgccggtgcgggtgctccgctaccggctcct
ctccgttctgtgctctcttctgctctcggctccccacccctctcccttccctcctctccccttgc
ctcccctcctctgcagcgcctgcattattttctgcccgcaggctcggcttgcactgctgctgcagc
ccggggaggtggctgggtgggtggggaggagactgtgcaagttgtaggggagggggtgccctcttc
ttccccgctcccttcccccgccaactccttcccctccttctcccccctttcccctccccgcccccac
cttcttcctcctttcggaaggactggtaacttgtcgtgcggagcgaacggcggcggcggcggcggc
ggcggcaccatccaggcgggcaccatg (Seq ID No: 264)

Homo sapiens wingless-type MMTV integration site family, member 10 B (WNT10B):

agtcctttgctcgccggcttgctagctctctcgatcactccctcccttcctccctcccttcctccc
ggcggccgcggcggcgctggggaagcggtgaagaggagtggcccggccctggaagaatgcggctct
gacaaggggacagaacccagcgcagtctccccacggtttaagcagcactagtgaagcccaggcaac
ccaaccgtgcctgtctcggaccccgcacccaaaccactggaggtcctgatcgatctgcccaccgga
gcctccgggcttcgacatg (Seq ID No: 265)

Homo sapiens CCHC-type zinc finger, nucleic acid binding protein (CNBP):

cagcctctaccttgcgagccgtcttccccaggcctgcgtccgagtctccgccgctgcgggcccgct
ccgacgcggaagatctgactgcagccatg (Seq ID No: 266)

Homo sapiens zinc finger protein 43 (ZNF43):

gggcctttgtctctggctgcagttggagctctgcgtctcgtcttcgttcttctgtgtcctctgctg
ctagaggtccagcctctgtggctctgtgacctgcgggtattgggggatccacagctaagacgccag
gaccccccggaagcctagaaatg (Seq ID No: 267)

Homo sapiens zinc finger protein 74 (ZNF74):

cagtccttttgtggggagtccggtctgtccacttgccggtccctcagaccgtcggcggtctctgtcc
gcttcgggacctgtccgctggtcgctccgcgtccgatggctcctggccgcggaaccttaggcctgg
ccctggtctccgagcgcgggttcgccgggaggagcgtgtggcgggggtgtgccggggcgtgagtgc
gccgagcatggggctgagcctggtgtggggagtgggtatctgcggagccggcctgaaccccacctc
agccgggcgcggggagggggctccgtgcgtgtgatcgtgcagctgtgagcgcgtggccgccccgcg
gggctccgctgcaggcccctcagccccaggagcagtactcgctcttcagggcctgccctggatcct
ggaggctacacagctgcccactcctcctggggaggctgccgtggaggccatg
(Seq ID No: 268)

Homo sapiens zinc finger protein 85 (ZNF85):

```
gggcctttgtctctcgctgcagcctgagctctaggtcttgttttccctgctttgtgttttctgctc
gtggacgcccagcctctgtggccctgtggcctgcaggtattgggagatccacagctaagacgccgg
gaccccctggaagcctagaaatg (Seq ID No: 269)
```

Homo sapiens zinc finger protein 91 (ZNF91):

```
gggcctttgtctctcgctgccgccggagtttccaggtctcgacttcactgctctgtgtcctctgct
ccaggaggcccagcctgtgtggccctgtgacctgcaggtattggagagccacagctaagatg
(Seq ID No: 270)
```

Homo sapiens zinc finger protein 141 (ZNF141):

```
gggtctttgcgtctggctactaccagaccgcgggttaggggcttcatctctctgcgttctcagttg
tgggaggccttggtgattcggccacagcctcagcctccgtcgctctgtgacctgcgggtattggat
gattggtagctaagactcccgaatacttcagaagtggggaaatg (Seq ID No: 271)
```

Homo sapiens zinc finger protein 205 (ZNF205): tgttctttctagctctgaaatagaaaatg (Seq ID No: 272)

Homo sapiens transmembrane protein 187 (TMEM187):

```
ctccctttttcggagatttgaatttcccccagcgaggcgagtgaggcgaaatacccgtatggtgata
gctggccttttcgcgccaatactgaaaaaggcagaacgttcctccgctggcgccagccaatcagca
ggactcctgccttccttcggggcaaggtcgcagcatctgcctcggaaatcacgaaatcacggggct
tctttctgctggctcagccgggaggcccagagtgttctgcagaggctgcgtattgaaggctgctct
ctgaagctccctgccccaggtcacgccgccggttccagatg (Seq ID No: 273)
```

Homo sapiens histone cluster 2, H2be (HIST2H2BE): acttcttttcttggctaagccgcgtttgtactgtgtcttaccatg (Seq ID No: 274)

Homo sapiens solute carrier family 25 (mitochondrial carrier; oxoglutarate carrier), member 11 (SLC25A11):

```
ccgcctttgcgctgcgcgcctgcgcccgcgccggcttccagcgggtgtcggacctgagagctggag
gggcgtgcgcgcgccctcgctctgttgcgcgcgcggtgtcaccttgggcgcgagcggggccgcgcg
cgcacgggacccggagccgagggccattgagtggcgatg (Seq ID No: 275)
```

Homo sapiens tyrosylprotein sulfotransferase 2 (TPST2):

```
cctcccccttccccggctggggcggctggagagccgggagtcgctgggtgcgtggggctgcctcgc
cgcgtctcgccacgggctctgccagcagacagccttggcacacaggcacaagggctggagcccaga
gatgagagtgcccaagggagatgtgagcctggcgggctgcccgctaacctgtcgctgaagccccag
aagcgggccctcaggccaggcctaccctgcctccggcccagcatg (Seq ID No: 276)
```

Homo sapiens sorbin and SH3 domain containing 2 (SORBS2):

```
aagcctcttttatacatctcttcagggaagagagaagcaatgggcatgttagtatacaatgatcac
agccacgcaggcctgcaagctgccttttggacaggctgttgactgccgttccaattagctgattgg
agaatgtggaatgcagagtgataatgctgcatatctgctatcaggcagcagcaaaggttttttgtct
tgggaaggcaagctttccctgcaatattatctcagcagctccctagctgcttaccctgaaaacgag
ggatccaaacggagggtgttgcactctgctaacgctggtcctgtgcgtggctgtggcatatgagcg
gcaggtctgaaaaagcaggtgtgtgctgggacgggcactggactggaacgcaggcggacgctctcg
ggtttacctgcttcctgttaacagattgtgggctcccagggcatatgtctgcacgctgaggccgag
gcggagaaggggcttcctgagcgtcccagtacactgacagagacacttggattggacttaatctta
aacctctggagttcaagacctttaaaaagggctaaataaacaatctctacatgtaaaaggccact
gactcctacttcctctgtatagagcaactgttgaactcagctgcctgtaggaaaactgaagacttt
aataacaaactctccaaggtgaaaatg (Seq ID No: 277)
```

Homo sapiens G protein-coupled receptor 65 (GPR65):

```
gtttctcttcttgacttgatgcaggcacagatttatcaagctcctcagtcaacaaacacatcaccg
gaagaaatatggaaggaaaggaattttaaaaggaaataccaatctctgtgcaaacaaagccttgta
tattcatgtttgcaccaatctactgtgagatttatgaagaaaaacaaattgcggacaactctctat
gtacacttacaaatgcctcagttgatgcttgtgggctgtttgtcagcgttctgtgataatgaacac
atggacttctgtttattaaattcagttgacccctttagccaattgccaggagcctggattttttact
tccaactgctgatatctgtgtaaaaattgatctacatccacccctttaaaagcattgatgaattaat
tagaactttagacaacaaagaaaaattgaaaaagaattctcagtaaaagcgaattcgatgttcaaa
acaaactacaaagagacaagacttctctgtttactttctaagaactaatataattgctaccttaaa
aaggaaaaaatg (Seq ID No: 278)
```

Homo sapiens nipsnap homolog 1 (C. elegans) (NIPSNAP1): gggccttcctgcaacctttgcggctccaacatg (Seq ID No: 279)

Homo sapiens inhibitor of kappa light polypeptide gene enhancer in B-cells, kinase complex-associated protein (IKBKAP):

```
gcttctttgcagcgcttcagcgttttcccctggagggcgcctccatccttggaggcctagtgccgt
cggagagagagcgggagccgcggacagagacgcgtgcgcaattcggagccgactctgggtgcggac
tgtgggagctgactctgggtagccggctgcgcgtggctggggaggcgaggccggacgcacctctgt
ttggggggtcctcagagattaatgattcatcaagggatagttgtacttgtctcgtgggaatcacttc
atcatg (Seq ID No: 280)
```

Homo sapiens COP9 constitutive photomorphogenic homolog subunit 3 (Arabidopsis) (COPS3): ctgccttcgccgctcgggccgcccgggggaaaacatg (Seq ID No: 281)

Homo sapiens pirin (iron-binding nuclear protein) (PIR):

```
ccgcctcctctaggccgccggccgcgaagcgctgagtcacggtgaggctactggacccacactctc
ttaacctgccctccctgcactcgctcccggcggctcttcgcgtcaccccgccgctaaggctccag
gtgccgctaccgcagcgtgagtacctggggctcctgcaggggtccactagccctccatcctctaca
gctcagcatcagaacactctcttttttagactccgatatg (Seq ID No: 282)
```

Homo sapiens THO complex 5 (THOC5):

```
ccttccttacttccggttctctatggtgcgcgggcaagctttgctccgcctccggcagtggcttac
tcccggtgccaggttcttggagctgtgaggaggaacaaccatg (Seq ID No: 283)
```

Homo sapiens RuvB-like 1 (E. coli) (RUVBL1):

gggcctttgcaaaattgccctagtaacggccgcatggtaactcaggcgccgggcgcactgtcctag
ctgctggttttccacgctggttttagctcccggcgtctgcaaaatg (Seq ID No: 284)

Homo sapiens Kruppel-like factor 7 (ubiquitous) (KLF7):

tttccttttagttgactgaaacaaaacaaaacaaaagggccactggatgtctgccttcttggggg
gtgagccagacagactgacaaacaaacagccccaactgtgttcgggggagggtttcgcctcccgtt
ttgcccggcagcagcagcatg (Seq ID No: 285)

Homo sapiens USO1 vesicle docking protein homolog (yeast) (USO1):

gctccccttttgccttcaaccttcgagccgccacgtaatgccacgtccccgcgcatgcgcatcttg
gccgctgctggcggctgtttccgggcttagagggctggagtggccgccgagttggaggcggtggtg
gcagcagtaggagtgtgtagagtgcgggattggggggccaggccctgcggagggcggggggaagttgt
cttctttttttttccggagggggccggtaaacctggtggctgaacggcaagatg
(Seq ID No: 286)

Homo sapiens unc-5 homolog C (C. elegans) (UNC5C):

cccccttttggcccctgcctttggagaaagtggagtgtggcgcttggttgtcgttatttcttcgga
ctgcttcgcggtgcacggattcagcttctgcccagtggggctttcagctgtttgcgcgtctctctg
tcccctcccctcccccggcacacctctgtctacgatg (Seq ID No: 287)

Homo sapiens RNA terminal phosphate cyclase domain 1 (RTCD1):

gcttcttccgctttctcgtcaggctcctgcgccccaggcatgaaccaaggtttctgaactactggg
cgggagccaacgtctcttctttctcccgctctggcggaggctttgtcgctgcgggctgggccccag
ggtgtcccccatg (Seq ID No: 288)

Homo sapiens eukaryotic translation initiation factor 3, subunit A (EIF3A):

ggctccttccttttccgtctctggccggctgggcgcgggcgactgctggcgaggcgcgtgggacctt
acgctggttcccccttcgtctcctctcccggcccgggccactagagagttcgctgacgccgggtgag
ctgagcctgccgccaagatg (Seq ID No: 289)

Homo sapiens eukaryotic translation initiation factor 3, subunit D (EIF3D):

gtttcctcttttcctggtttctcaagagtgctgctgctaacgcggtccccggcacgcaccatctgt
tgccatcccggccggccgaggccattgcagattttggaagatg (Seq ID No: 290)

Homo sapiens eukaryotic translation initiation factor 3, subunit F (EIF3F): ccgcctccttctttctcgacaagatg (Seq ID No: 291)

Homo sapiens eukaryotic translation initiation factor 3, subunit G (EIF3G): cgctctctggccgggcttgggctgcgtggagaatactttttgcgatg (Seq ID No: 292)

Homo sapiens eukaryotic translation initiation factor 3, subunit H (EIF3H): gtttctctttcttcctgtctgcttggaaagatg (Seq ID No: 293)

Homo sapiens eukaryotic translation initiation factor 3, subunit I (EIF3I): aaacctttttccggtcttactcacgttgcggccttcctcgcgt-cacagccgggatg (Seq ID No: 294)

Homo sapiens eukaryotic translation initiation factor 3, subunit J (EIF3J): ctccctctcacacacgctcacacccggctcgagatg (Seq ID No: 295)

Homo sapiens poly(A) binding protein, cytoplasmic 4 (inducible form) (PABPC4):

```
ccgcctctctccgccccgggtcgctgccgcctccgccgctttcgggcttcgcagcctgaggaaaaa
aagagaaaaagataaaaaaaatctgaaaacgcttcaaaatcctgaaaaaaaaaaggaaaagaaaa
```

```
aacgaatcctcggagaacccgcggggaagtcactttcgtacgcttccggcctgccccgcgcccgcc
gccgcagcgcttggcgtccgtcggtctccgtccgtcggtccggggggtgagccgcccgcccggcccg
ccgtgccctcccccgctcgggccccgagccccgcgccccgcgcctgccccggcgcaccacgtgtc
cgtgctgcccttcgccgcccgcccggggctcgccgagtcggcgcccacaaagatttggtttccctc
tgccccggcggttgtaatcttaaaccgccggagcccgaggcctatatttatagagaaacgcgtgtc
cccgaggccgccgtgggcagcgtccggtcgcctcttaaaggattttacccttcggaagggggattc
cccgtttaattttttcctactttgattttttgaaatttggagcttcgcaccaggaccgcggagaa
gtgcaaagtcgcggggagggccgtattgtgcggagagcctttgtctgcggtgctgcggccgtggg
agccggccccgcctcccgtttccgtcccgtctccaagcccgccgactccagctcgtcctcgccgc
gccggtgccacctgtgagccgcggcgcgggcccgggctccgaaggcgcccctttgtcctgcggcgg
gcccgataagaagtcctcctggcggggctcggggtggtggggggcggggagatg
(Seq ID No: 296)
```

Homo sapiens receptor-interacting serine-threonine kinase 2 (RIPK2):

```
agctctttcgcggcgctacggcgttggcaccagtctctagaaaagaagtcagctctggttcggaga
agcagcggctggcgtgggccatccggggaatgggcgccctcgtgacctagtgttgcggggcaaaaa
gggtcttgccggcctcgctcgtgcaggggcgtatctgggcgcctgagcgcggcgtgggagccttgg
gagccgccgcagcagggggcacacccggaaccggcctgagcgcccgggaccatg
(Seq ID No: 297)
```

Homo sapiens neuropilin 1 (NRP1):

```
ctttcttttctccaagacgggctgaggattgtacagctctaggcggagttggggctcttcggatcg
cttagattctcctctttgctgcatttccccccacgtcctcgttctcccgcgtctgcctgcggaccc
ggagaagggagaatg (Seq ID No: 298)
```

Homo sapiens guanine monphosphate synthetase (GMPS):

```
tggtcttctctcccgcggcgctggggcccgcgctccgctgctgttgctccattcggcgcttttctg
gcggctggctcctctccgctgccggctgctcctcgaccaggcctccttctcaacctcagcccgcgg
cgccgaccttccggcaccctcccgccccgtctcgtactgtcgccgtcaccgccgcggctccggcc
ctggccccgatg (Seq ID No: 299)
```

Homo sapiens far upstream element (FUSE) binding protein 1 (FUBP1):

```
ttttctttctttcttagctgttagctgagaggaagtctctgaacaggcggcagcggctcttatagt
gcaaccatg (Seq ID No: 300)
```

Homo sapiens eukaryotic translation initiation factor 2B, subunit 5 epsilon, 82kDa (EIF2B5):

```
gatccttttgtcccctactgcgtgcggtggcagcttccttgcggaagtggtgaccgtgagagaag
aagatg (Seq ID No: 301)
```

Homo sapiens eukaryotic translation initiation factor 2, subunit 2 beta, 38kDa (EIF2S2):

```
gtttcctttcgctgatgcaagagcctagtgcggtggtgggagaggtatcggcaggggcagcgctgc
cgccggggcctggggctgacccgtctgacttcccgtccgtgccgagcccactcgagccgcagccat
g (Seq ID No: 302)
```

Homo sapiens adaptor-related protein complex 1, sigma 2 subunit (AP1S2):

```
cctcccctctccgcctaagcctgccctatgccagccgggtgtcctccccacagcaccacggcttct
cttcctcagcacggcgacaggggcttccccttcgccgccgccgccgccggccaagctccgccg
cgcccgcggcccgcggccgccatg (Seq ID No: 303)
```

Homo sapiens suppression of tumorigenicity 13 (colon carcinoma) (Hsp70 interacting protein) (ST13):

```
cgccccttctgcgcggtcacgccgagccagcgcctgggcctggaaccgggccgtagcccccccag
tttcgcccaccacctccctaccatg (Seq ID No: 304)
```

Homo sapiens solute carrier family 7 (cationic amino acid transporter, y+ system), member 7 (SLC7A7):

```
ctccctttcttaaatgcttggggtgagagagaagagaggctagggtggggcatggaggacacagag
agagagagtgctgtgtattccttccccgctactgtcctgtcctcagctaacttgctctgggacagc
ttccccagggctacagatactgcactcagctgactgtcctttcttctgggcccctggtcccagagc
agagctgacaaaggagattcctgagagagcaccttcttatcacagaaagtgctgagccaagagctc
ctagctgcccctttttgcagatgtgaagggccagtgaaccttggacccagatggttgcttaatactc
cttccccctccctcactccttcctttgcgggctgcctcacctcctcacccttcttgcttaaatc
cataggcatttgtctggccttcccttttactgctggctgggaaggaggagcatcagaccacagatc
ctggaaggcacttctctccctgactgctgctcacactgccgtgagaacctgcttatatccaggacc
aaggaggcaatgccaggaagctggtgaagggtttcctctcctccaccatg
(Seq ID No: 305)
```

Homo sapiens paired box 2 (PAX2):

```
ctccctttttctcctcaagtcctgaagttgagtttgagaggcgacacggcggcggcggccgcgctgc
tcccgctcctctgcctccccatg (Seq ID No: 306)
```

Homo sapiens 5-aminoimidazole-4-carboxamide ribonucleotide formylt ransferase/IMP cyclohydrolase (ATIC):

```
agccctcctacctgcgcacgtggtgccgccgctgctgcctcccgctcgccctgaacccagtgcctg
cagccatg (Seq ID No: 307)
```

Homo sapiens ATP synthase, H+ transporting, mitochondrial F1 compl ex, alpha subunit 1, cardiac muscle (ATP5A1):

```
ccttctttgcggctcggccattttgtcccagtcagtccggaggctgcggctgcagaagtaccgcct
gcggagtaactgcaaagatg (Seq ID No: 308)
```

Homo sapiens cyclin G1 (CCNG1):

cggcccccttcggctccgagctgaccctgatcagggccgagttgtctcggcggcgctgccgaggcct
ccacccaggacagtccccctcccgggcctctctcctcttgcctacgagtcccctctcctcgtagg
cctctcggatctgatatcgtggggtgaggtgagcaggcccgggggagggtggttaccgctgaggagc
tgcagtctctgtcaagatg (Seq ID No: 309)

Homo sapiens cadherin 16, KSP-cadherin (CDH16):

agctctcttcttgcttggcagctggaccaagggagccagtcttgggcgctggagggcctgtcctga
ccatg (Seq ID No: 310)

Homo sapiens cyclin-dependent kinase inhibitor 1B (p27, Kipl) (CDKN1B):

ttttcttcttcgtcagcctcccttccaccgccatattgggccactaaaaaaaggggggctcgtcttt
tcggggtgtttttctccccctcccctgtccccgcttgctcacggctctgcgactccgacgccggca
aggtttggagagcggctgggttcgcgggacccgcgggcttgcacccgcccagactcggacgggctt
tgccaccctctccgcttgcctggtcccctctcctctccgccctcccgctcgccagtccatttgatc
agcggagactcggcggccgggccggggcttccccgcagcccctgcgcgctcctagagctcgggccg
tggctcgtcggggtctgtgtcttttggctccgagggcagtcgctgggcttccgagaggggttcggg
ctgcgtaggggcgctttgttttgttcggttttgttttttttgagagtgcgagagaggcggtcgtgca
gacccgggagaaagatg (Seq ID No: 311)

Homo sapiens chimerin (chimaerin) 2 (CHN2):

tctcctcttcttcctttgtgtgtgcgcgagcggagttggggcggagggagaagggggaggtcgctc
tgtctgtccgtctcccgccgcctctgcccggtctactcgaagtgcggcgggagaggcgggagccca
ggagagggtgcgggagctggcggggcggctcggagctgccaggacgccctggtcccagccgcgcac
aggggagcgtggacggcagagggggctcggcgggagccgagatccgcccgtcccggctgcccctcgg
cctccctctgctcccacctaccccctgacacccatagaaaagcgtgcaaaggcgcgggagcgggacg
gaaaccacaaataaatagcggcggcggcagcgcgtcatctggtggagcaggaagtgcaggcagagt
ccggaggctggtgctttctgcgcgtccccaggactttgccatgggctggggggccgcggaggctgcg
agcggccgggcgagggcagcggcggcggcgtccgcaccggggctgagcgagcagcgacgcgaggggg
cgcgcggagatg (Seq ID No: 312)

Homo sapiens citrate synthase (CS):

gggcctccttgaggaccccgggctgggcgccgccgccggttcgtctactctttccttcagccgcct
cctttcaaccttgtcaacccgtcggcgcggcctctggtgcagcggcggcggctcctgttcctgccg
cagctctctccctttcttacctccccaccagatcccggagatcgcccgccatggctttacttactg
cggccgcccggctcttgggaaccaaggcacccagtggcaagtactagctgagcatttgggagatgc
ttgtcttacttggctgttgcttctcctgctgctggggaaaaggaatgcatcttgtcttgttcttgc
agcccggcatgccagtgcttcctccacgaatttgaaagacatattggctgacctgatacctaagga
gcaggccagaattaagactttcaggcagcaacatggcaagacggtggtgggccaaatcactgtgga
catg (Seq ID No: 313)

Homo sapiens cathepsin S (CTSS):

atttctttttcaagtcaattgaactgaaatctccttgttgctttgaaatcttagaagagagcccact
aattcaaggactcttactgtgggagcaactgctggttctatcacaatg (Seq ID No: 314)

Homo sapiens deoxynucleotidyltransferase, terminal (DNTT):

cagtctccctcccttctggagacaccaccagatgggccagccagaggcagcagcagcctcttcccatg (Seq ID No: 315)

Homo sapiens dual specificity phosphatase 3 (DUSP3): cgctctccgcctcgcttgctcctgccgggcgtgcagggccccgccgccgccatg (Seq ID No: 316)

Homo sapiens coagulation factor II (thrombin) receptor-like 2 (F2RL2):

catcctttccctgcggaggaccagggcaagtttcctgcctgcacggcacaggagagcaaacttctacagacagaccaaggcttccatttgctgctgacacatggaactgaggtgaaattgtgctccatgattttacagatttcataacgtttaagagacgggactcaggtcatcaaaatg (Seq ID No: 317)

Homo sapiens Fc fragment of IgG, receptor, transporter, alpha (FCGRT): cgtcctctcagcatg (Seq ID No: 318)

Homo sapiens guanylate binding protein 2, interferon-inducible (GBP2):

ttacctcttttcttgtctctcgtcaggtctctgacattgacagagcctggacgttggaggaagccccaggacgttggaggggtaaagtaaaagtccacagttaccgtgagagaaaaaagagggagaaagcagtgcagccaaactcggaagaaaagagaggaggaaaaggactcgactttcacattggaacaaccttctttccagtgctaaaggatctctgatctggggaacaacaccctggacatg (Seq ID No: 319)

Homo sapiens G protein pathway suppressor 1 (GPS1):

cgctctttctcccttcagcagccagccagctctgtgtcagggtcggggggtgcagaaagtcaggacagaatg (Seq ID No: 320)

Homo sapiens general transcription factor IIF, polypeptide 2, 30kD a (GTF2F2):

gttcctcttttcctcggttcccagtgttctggcaggtaaggaacgccggctcttcgcctctcagcgcggcttgtcctttgttccggacgcccgctcctcagccctgcggctcctggggtcgctgctgcatcccgcacgcctccaccggctgcagacccatg (Seq ID No: 321)

Homo sapiens glycogenin 1 (GYG1):

cgctccctcccggtgccggcttctctgagtcaccaacctgaggctgccccggccgcctgcgcacccggcagcaccatg (Seq ID No: 322)

Homo sapiens heat shock 70kDa protein 9 (mortalin) (HSPA9): agctctttgccgtcggagcgcttgtttgctgcctcgtactcctccatttatccgccatg (Seq ID No: 323)

Homo sapiens iron-responsive element binding protein 2 (IREB2):

cttccttcttcctcccttgccagtccgcctgtcttcctccccgtcttccctgcccggcctcccccttcttcccccgctggcccccctccccggagggataatatggtctccggcgatg (Seq ID No: 324)

Homo sapiens origin recognition complex, subunit 1 (ORC1):

ccaccttcttttcatttctagtgagacacacgctttggtcctggctttcggcccgtagttgtagaa
ggagccctgctggtgcaggttagaggtgccgcatcccccggagctctcgaagtggaggcggtagga
aacggagggcttgcggctagccggaggaagctttggagccggaagccatg
(Seq ID No: 325)

Homo sapiens RAB1A, member RAS oncogene family (RAB1A):

cattcctttctttcgattacccgtggcgcggagagtcagggcggcggctgcggcagcaagggcggc
ggtggcggcggcggcagctgcagtgacatg (Seq ID No: 326)

Homo sapiens cytohesin 2 (CYTH2):

gagtcttttcagcgctgaggactggcgctgaggaggcggcggtggctcccggggcgtttgagcggg
ctcacccgagcccgcgggccaacgcggatccaggcccgactggcgggaccgccccggattccccgc
gggccttcctagccgccatg (Seq ID No: 327)

Homo sapiens COP9 constitutive photomorphogenic homolog subunit 2 (Arabidopsis) (COPS2): atttctcctccccctcccggccaagatg (Seq ID No: 328)

Homo sapiens solute carrier family 9 (sodium/hydrogen exchanger), member 3 regulator 1 (SLC9A3R1):

ggtcctctctcggctcctcgcggctcgcggcggccgacggttcctgggacacctgcttgcttggcc
cgtccggcggctcagggcttctctgctgcgctcccggttcgctggacgggaagaagggctgggccg
tcccgtccgtccccatcggaaccccaagtcgcgccgctgacccgtcgcagggcgagatg
(Seq ID No: 329)

Homo sapiens peptidase (mitochondrial processing) beta (PMPCB): ctaccttccttctagcagaaatg (Seq ID No: 330)

Homo sapiens RAB3D, member RAS oncogene family (RAB3D):

cggcccttcctccgccttctgggcggagcccgcgcgggatccgggtggctgcaggctgctggcttc
tgcggctgcggggtcggggtcgcggccagggccaagccgcagcgagttcacaggcggaaccccctgc
aggcggcgccccctacgcgaggtcacccctgggaaggagcgcagcccacccggcccctccgcatcc
gagcaggacgcccgtctcctctccctgaggatttcaggtctccctgtcccaggaggcttgtgccaa
gatg (Seq ID No: 331)

Homo sapiens ATP-binding cassette, sub-family B (MDR/TAP): tcttctctcggttcctctttcctcgctcaagatg (Seq ID No: 332)

Homo sapiens N-acylsphingosine amidohydrolase (acid ceramidase) 1 (ASAH1): ggctcttctttgcctctgctggagtccggggagtggcgttggctgctagagcgatg (Seq ID No: 333)

Homo sapiens cytochrome c oxidase subunit VIc (COX6C):

ttttcctttagtcaggaaggacgttggtgttgaggttagcatacgtatcaaggacagtaactacca
tg (Seq ID No: 334)

Homo sapiens COX15 homolog, cytochrome c oxidase assembly protein (yeast) (COX15):

gcttctcttttccttggcggaggagggagaccacagagccctgggttgtggaagaggtggctgttc
cctgtcatcagtatg (Seq ID No: 335)

Homo sapiens c-src tyrosine kinase (CSK):

```
ccccttccccgcctttcttccctccgcgacccgggccgtgcgtccgtcccctgcctctgcctg
gcggtccctcctccctctccttgcacccatacctctttgtaccgcacccctggggacccctgcg
cccctccctcccccctgaccgcatggaccgtcccgcaggccgctgatgccgcccgcggcgaggtg
gcccggaccgcagtgccccaagagagctctaatggtaccaagtgacaggttggctttactgtgact
cggggacgccagagctcctgagaagatg (Seq ID No: 336)
```

Homo sapiens versican (VCAN):

```
gagcctttctggggaagaactccaggcgtgcggacgcaacagccgagaacattaggtgttgtggac
aggagctgggaccaagatcttcggccagccccgcatcctcccgcatcttccagcaccgtcccgcac
cctccgcatccttccccgggccaccacgcttcctatgtgacccgcctgggcaacgccgaacccagt
cgcgcagcgctgcagtgaattttccccccaaactgcaataagccgccttccaaggccaagatg
(Seq ID No: 337)
```

Homo sapiens dystroglycan 1 (dystrophin-associated glycoprotein 1) (DAG1):

```
gcgcctcttaggcttggcggtggcggcggcggcagcttcgcgccgaatccccgggggagcggcggtg
gcggcgtcctggggccaggaggagcgaacacctgccgcggtcctcccgccggcgctgggctctgtg
tgctccgggatggagcaggtgtgcagagggtgagaacccagctctgggaccaagtcacttgcttcc
ttacttagcaagactatcgacttgagcaaacttggacctgggatg (Seq ID No: 338)
```

Homo sapiens DEAD (Asp-Glu-Ala-Asp) box helicase 5 (DDX5):

```
ccccctcttttggttacagacgtgagggctctttggagacgtaaacatctccgagtggcgagggtg
ggcggggctgggcttgggaaagggcggggtggcttgcttgaggtgtggaaagaccagaagaaggtg
aggtcaagagagtgcagaatgaggcattccaatggtgggtgggccctgacctgagagagtggcgcg
gggaggggtgaaagcgcggcgatcctggaacgccagcgggcgttgcggcctatgcgcgaggggcgg
ggcgattaggtcatagagcggctcccagcgttccctgcggcgtaggaggcggtccagactataaaa
gcggctgccggaaagcggccggcacctcattcatttctaccggtctctagtagtgcagcttcggct
ggtgtcatcggtgtccttcctccgctgccgccccgcaaggcttcgccgtcatcgaggccatttcc
agcgacttgtcgcacgcttttctatatacttcgttccccgccaaccgcaaccattgacgccatg
(Seq ID No: 339)
```

Homo sapiens desmoplakin (DSP):

```
gctcctctgcgcccttgccgccctccgagccacagctttcctcccgctcctgcccccggcccgtcg
ccgtctccgcgctcgcagcggcctcgggagggcccaggtagcgagcagcgacctcgcgagccttcc
gcactcccgcccggttccccggccgtccgcctatccttggccccctccgctttctccgcgccggcc
cgcctcgcttatgcctcggcgctgagccgctctcccgattgcccgccgacatg
(Seq ID No: 340)
```

Homo sapiens glutamyl-prolyl-tRNA synthetase (EPRS):

```
cttcctttcgcggggtcctccgtagttctggcacgagccaggcgtactgacaggtggaccagcgga
ctggtggagatg (Seq ID No: 341)
```

Homo sapiens acyl-CoA synthetase long-chain family member 4 (ACSL4):

```
gctcctcctcgtcccagcgctagcgggcacgcggttccttttttgcgagctttccgagtgccaggcg
ccggccggctgcgaagacgcggtgggccgcccctccgattgaaatcacagaagatattcgtgttct
tcttaagagaaaaagaggacattttagctttctcagttgaaggcgtactttattgtcggcttccaa
agattactaacttttatctgtatcactaagattgaactgccttggctgtactgctattcttactgc
tgcttctattattgccttcttcagcacaataaggctttcaaaagccaagaataacaagaaataag
caccattttagaagcctttccactatg (Seq ID No: 342)
```

Homo sapiens fibroblast activation protein, alpha (FAP):

```
tggtccttttcaacggttttcacagatccagtgacccacgctctgaagacagaattagctaacttt
caaaaacatctggaaaaatg (Seq ID No: 343)
```

Homo sapiens UDP-N-acetyl-alpha-D-galactosamine:polypeptide N-acet ylgalactosaminyltransferase 3 (GalNAc-T3) (GALNT3):

```
ctgcctctccaggcaacgcgggaggcccagcgggaaggcaggaggcggcggcggaggaggagctct
actgagccgcaactgtggcgacagcaaccggagtcgcagccgccgccacctgcacctggcgcctag
cccacgtccagcgcctgcccggccgccgcttcccgccaccctgccctgcccacccgccaggtacta
ccattaaagataccttcttctcagcaaatctatgataaaaaatataagtaacagaagaagaaataa
ctgttatttgtcaagtgacaagcttttaatgtcagaatg (Seq ID No: 344)
```

Homo sapiens glypican 3 (GPC3):

```
acgtctcttgctcctcagggccactgccaggcttgccgagtcctgggactgctctcgctccggctg
ccactctcccgcgctctcctagctccctgcgaagcaggatg (Seq ID No: 345)
```

Homo sapiens interleukin enhancer binding factor 2, 45kDa (ILF2):

```
acgcctcttcagttgtctgctactcagaggaaggggcggttggtgcggcctccattgttcgtgttt
taaggcgccatg (Seq ID No: 346)
```

Homo sapiens nucleosome assembly protein 1-like 1 (NAP1L1):

```
gggtctttttttagcgccatctgctcgcggcgccgcctcctgctcctcccgctgctgctgccgctgc
cgccctgagtcactgcctgcgcagctccggccgcctggctccccatactagtcgccgatatttgga
gttcttacaacatg (Seq ID No: 347)
```

Homo sapiens asparaginyl-tRNA synthetase (NARS):

```
cgctctctgatgcaacgccggaatcgcggaaaccgccggtgcacgttggagtcataagacggcgtc
ggtgttgcagtctgtgtccttggaggtgaccagggccactgcaggcatg (Seq ID No: 348)
```

Homo sapiens NADH dehydrogenase (ubiquinone) 1 alpha subcomplex, 10, 42kDa (NDUFA10): cgtccccttgggtccttgatcctgagctgaccgggtagccatg (Seq ID No: 349)

Homo sapiens NADH dehydrogenase (ubiquinone) Fe-S protein 2, 49kDa (NADH-coenzyme Q reductase) (NDUFS2): ttctccttcccgcagtctgcagccggagtaagatg (Seq ID No: 350)

Homo sapiens NADH dehydrogenase (ubiquinone) Fe-S protein 5, 15kDa (NADH-coenzyme Q reductase) (NDUFS5):

catcctttacggcaggcgtccgcgtcgctagctagtcgttctgaagcggcggccagagaagagtca
agggcacgagcatcgggtagccatg (Seq ID No: 351)

Homo sapiens phosphoenolpyruvate carboxykinase 2 (mitochondrial) (PCK2):

ccctcctttttaagcgcctcccgccagcctctgctgtggctcgcttcgccgcgctccctccttccc
cgccttccatacctccccggctccgctcggttcctggccaccccgcagcccctgcccaggtgccat
g (Seq ID No: 352)

Homo sapiens serpin peptidase inhibitor, clade B (ovalbumin), member 6 (SERPINB6):

ctccctcgcgctccggacgggcgacggtagctcgagacccgggactccgcccgcctccccgcgag
tatttgaggtccgggggcggctccggcgcctctgcccgccgttctgctcgctcgctccccgctctgg
agtctgccatcatg (Seq ID No: 353)

Homo sapiens Rab geranylgeranyltransferase, alpha subunit (RABGGTA):

ttctctcctcagacttcaagggctaccactggacccttcccctgtcttgaaccctgagccggcacc
atg (Seq ID No: 354)

Homo sapiens Rab geranylgeranyltransferase, beta subunit (RABGGTB): ctctctcctttccctgttagacatg (Seq ID No: 355)

Homo sapiens small nuclear ribonucleoprotein polypeptide A (SNRPA):

agttctctccgcacgcgggctggagaagcgggtcctacgcacgctttgttgtcgcgctttgcctcc
gtccttgcccctactcccgccttacctgacttccttttcggaggaagatccttgagcagccgacgt
tgggacaaaggatttggagaaacccagggctaaagtcacgttttttcctcctttaagacttacctca
acacttcactccatg (Seq ID No: 356)

Homo sapiens sterol regulatory element binding transcription facto r 2 (SREBF2):

cgccctttctgtgcggcgcccgggcgcaacgcaaacatggcggcgggtggcacccgtcggtgaggc
ggtgccgggcggggggttgtcgggtgtcatgggcggtggcgacggcaccgcccccgcgtctccctga
gcgggacggcaggggggggcttctgcgctgagccgggcgatg (Seq ID No: 357)

Homo sapiens translin (TSN):

ctgccctttggacgcgcgcctcggttccgaacgcagcggacggcgcctcaggcagcgcggcggaca
gcccgtcctccggcgcgccgcgagcctcggaggacccctagcgacggtcgtggcgtaagaccgggggg
gacgcggcggtagcggcggccgttgcgattgattgcgctggttgcctgcggcgtccacttccttgg
ccgcccttgctacactggctgattgttgtgcagccggcgccatg (Seq ID No: 358)

Homo sapiens Fanconi anemia, complementation group G (FANCG):

ccaccctttctcgaggctgtggcctccgcgagagccgagcgggccgcaccgccggccgtgcgactg
ccccagtcagacacgaccccggcttctagcccgcctaagcctgtttggggttgctgactcgtttcc
tccccgagtttcccgcgggaactaactcttcaagaggaccaaccgcagcccagagcttcgcagacc
cggccaaccagaggcgaggttgagagcccggcgggccgcggggagagagcgtcccatctgtcctgg
aaagcctgggcggggtggattgggaccccgagagaagcaggggagctcggcggggtgcagaagtgcc
caggcccctcccgctggggttgggagcttgggcaggccagcttcacccttcctaagtccgcttct
ggtctccgggcccagcctcggccaccatg (Seq ID No: 359)

Homo sapiens DEAD (Asp-Glu-Ala-Asp) box polypeptide 39B (DDX39B):

```
ttccctccttcgtcgctgttgctgccgccatacgcgctctccctgtttagctcttctgttagaaat
agtatctttgttttcctttgctgttcctcaatcccctactcttcaccccttgttttcacctatttt
gcgagaacccatccagatcccccttcccttcttcccctgccggcccagttatg
(Seq ID No: 360)
```

Homo sapiens RAB11A, member RAS oncogene family (RAB11A): ccgccctttcgctcctcggccgcgcaatg (Seq ID No: 361)

Homo sapiens SPARC-like 1 (hevin) (SPARCL1):

```
agctctttcccttttggtttgcaagcactgcctgtaaagccctcgcatgagaggccagcctgctag
ggaaatccaggaatctgcaacaaaaacgatgacagtctgaaatactctctggtgccaacctccaaa
ttctcgtctgtcacttcagaccccccactagttgacagagcagcagaatttcaactccagtagactt
gaatatgcctctgggcaaagaagcagagctaacgaggaaagggatttaaagagttttttcttgggtg
tttgtcaaacttttattccctgtctgtgtgcagaggggattcaacttcaattttttctgcagtggct
ctgggtccagcccccttacttaaagatctggaaagcatg (Seq ID No: 362)
```

Homo sapiens cyclin B2 (CCNB2): ctcccttttcagtccgcgtccctccctgggccgggctggcactcttgccttccccgtccctcatg (Seq ID No: 363)

Homo sapiens cytochrome c oxidase subunit VIIa polypeptide 2 like (COX7A2L): ggtccttctctggggcggtcgcgttggcagcggatgcgggaagccggactctg-ggcgtcatg (Seq ID No: 364)

Homo sapiens lysophosphatidic acid receptor 2 (LPAR2):

```
cgccctctcagcaacccgcacagggcgcacccggacgctctaccgctcccgccgcagtcgccgggc
catgggcctcgagcccgcccccgaaccccgcgagcccgccttgtctgcggcgtgactggaggccca
gatg (Seq ID No: 365)
```

Homo sapiens adaptor-related protein complex 4, mu 1 subunit (AP4M1):

```
cgttcttttgttccggggccgcagggcggggcaggcccgactttcgccgtcttcttgtctactctc
cagaacggccatg (Seq ID No: 366)
```

Homo sapiens budding uninhibited by benzimidazoles 3 homolog (yeast) (BUB3):

```
cttcctctccgcctccttcgcctagcctgcgagtgttctgagggaagcaaggaggcggcggcggcc
gcagcgagtggcgagtagtggaaacgttgcttctgaggggagcccaagatg
(Seq ID No: 367)
```

Homo sapiens DEAD (Asp-Glu-Ala-Asp) box helicase 21 (DDX21): ctacctcttcctctccacgcggttgagaagaccggtcggcctgggcaacctgcgctgaagatg (Seq ID No: 368)

Homo sapiens solute carrier family 33 (acetyl-CoA transporter), member 1 (SLC33A1):

```
tgctctctgccgcattgatagcagcgagagctggaggtgttgggtcgggagaccagccgttcgatc
ccgccgcaggtaggagctggtttccatcctggcaccacggcacacacctccagcctcgagcccggc
gctgctgcccgggggtctccttcaggctctttgacgccgttccaggggggcacctatccaggcatcc
tctgggcctctagcagaggactggctcccggcttcagcactccgggctgcagtaagaagtgccct
tatcgctctgagccctgccaccatcccgtgaaccaccgaaaccctggtccagcgcgacagccttgg
acctgggactggacggatccaaaacgctcagcctcggccccccacagacgggggctctgcatcgtct
ctgatatg (Seq ID No: 369)
```

Homo sapiens G protein-coupled receptor 37 like 1 (GPR37L1): tgctcttcctgggctggctgtctcctgctcatccagccatg (Seq ID No: 370)

Homo sapiens neuronal regeneration related protein homolog (rat) (NREP):

```
ctgtctttctagcatgttgccctttttcaaccacatttgtgtttcaggtgtagagaggagagagag
tgaacaggggagcggggcttttgtctgttggtctccctggactgaagagagggagaatagaagccca
agactaagattctcaaaatg (Seq ID No: 371)
```

Homo sapiens vesicle-associated membrane protein 3 (cellubrevin) (VAMP3): gcttctctgctgaccctctctcgtcgccgctgccgccgccgcagctgccaaaatg (Seq ID No: 372)

Homo sapiens synaptosomal-associated protein, 29kDa (SNAP29): cctccttctgtttcccagaccgagagccgcgccggcaccatg (Seq ID No: 373)

Homo sapiens lon peptidase 1, mitochondrial (LONP1):

```
ccccctcttctccgcgtaggcccagctccctgaagcggctgtttcgagccacgcgcccatcgggta
ccgaggcacgcgccgggcgtcacgtgcgtttcgcggcgagcggaaatgacgcgagttgtgtgagcc
gccagtatggccgggctatg (Seq ID No: 374)
```

Homo sapiens kinesin family member 3B (KIF3B):

```
ctgtctctccccatccggggcagcggggaatggctgagccaggggttcgccgcccccgccgccgcc
gccgccgccgccgccgccgcccgctttcggctcgggcctcaggaccgtagcatcctgaga
cattttgaattgacacttctcaagatttgactggatcagagttcatcatg
(Seq ID No: 375)
```

Homo sapiens transmembrane 9 superfamily member 2 (TM9SF2):

```
cttcctttatctctggcggccttgtagtcgtctccgagactcccaccccctccttccctcttgacc
ccctaggtttgattgccctttcccccgaaacaactatcatg (Seq ID No: 376)
```

Homo sapiens cytosolic iron-sulfur protein assembly 1 (CIAO1):

```
gagcctctgtcggccgcggaagcctggagtgggcggtacgcagacgcgcgcggtgagacccgctgt
ctgctcagcggactctgcccgccccacctccccctgcgtcgggccgacatg
(Seq ID No: 377)
```

Homo sapiens GRB2-related adaptor protein 2 (GRAP2):

caccctctttcagagtggtacatggaagacagcacaaagtggatccatactctgaaatgcagtaac
tctgatgcttgaatttgtctcccttcttgccagaaaggattctaataactcggtgtcaaagccaag
acataaactcaaccccttctcttccaaaagcttcacgttacagcatg (Seq ID No: 378)

Homo sapiens leupaxin (LPXN):

gtacctttctcggggtgtctgcgtaactgcccagacttgccttggtttggtcagatgacacctcct
ctgggactggctagccagcgttcatg (Seq ID No: 379)

Homo sapiens SH3-domain binding protein 5 (BTK-associated) (SH3BP5):

tttcctctgctccgccgcggccggaggtatccgcatcggcgagctgcgtctcccgggtgtcggccc
cggcggctccccgaccgtgcccggctgtggcgaggcggctccagcccagcctgtggcagccgcgac
ccccgggggcgctccggagcccactgcgcggcgcgcgtgccggctgcctgcatg
(Seq ID No: 380)

Homo sapiens phosphatidylinositol glycan anchor biosynthesis, clas s B (PIGB): ctttcttccgccttaggaaggtggcggccagggatg (Seq ID No: 381)

Homo sapiens lipopolysaccharide-induced TNF factor (LITAF):

cggccctttttctcggggcgcccgagaggccagctcagacctcccggctcgacaggcggcgcgggcg
gcggtgagtgcggcgcggggacgccggggcgcggggaccagcgggagacagcgggggggccggtggc
gccagcacctgctggggggccccgggcactgagcccttggctggggcctcctgggatgccagggggc

gcgggtcgggtcgcgggcatcgaggcgcggcggagggcgtgggggcccggccggggcggggtccgg
cctcccagcgctggtcccggccgcgtctccggttgggttcagctcctgcgtcccagagtggcccga
tcgcgcgtggcgggggtcgtccggcccccacccgaacgagcgcccttcgcggcccgccgcgtccccc
tccccggagaggacggcccctgggcttttagaaaaaggcgcgattctctctagtgactcaggttg
agatttccagaaatatcccccggggttcagaaacaaaaccaaacaaacaaaaaaaccccaacga
attcccaaatgctatttgccaaacatttgacttctaggggcgcgggtacccgcgtttctctccctg
cccccgcgacttcgcgcaagatccgggaaggacacccgaggcccctgggagaccctggggaggtga
aaatcagagagcgaagcgggccgtggcccctaggcctgacccctccccgcgggtaaggcgggcac
cccgcgagcgcagggtcctcttactgctgatggcacccagctctgggcccagacgccgctcaccg
tccaccgccggtgctgggtaaaatg (Seq ID No: 382)

Homo sapiens etoposide induced 2.4 mRNA (EI24):

ccaccccttcggctctgggccccgcctcgtggtgccggctggttcttcgcgctcgcccgacttccc
agcggccccgtgcggcccgggcatgcccagtgcgggcgcagcggccccggccctggaagcgccccg
gcggagctggcctgcggtgggctaggggcagggccggagccgcggcggcggagctgtggatccttc
atgatgagagatttggggacacttctctctcctgtgtgtagttgatagtttggtggtgaagagatg
(Seq ID No: 383)

Homo sapiens chromosome 14 open reading frame 2 (C14orf2): tgacctttccgagttggctgcagatttgtggtgcgttctgagccgtctgtcctgcgccaagatg (Seq ID No: 384)

Homo sapiens peroxiredoxin 6 (PRDX6):

attcctccgcgcgctgggacaggctgcttcttcgccagaaccaaccggttgcttgctgtcccagcg
gcgcccctcatcaccgtcgccatg (Seq ID No: 385)

Homo sapiens solute carrier family 29 (nucleoside transporters), member 1 (SLC29A1):

```
ctctcttccgcccggcggcccacaccggtcaggcccggcgcgggctgcgctctccagctgtggcta
tggccccagccccgagatgaggagggagagaactaggggcccgcaggcctgggaatttccgtcccc
caccaagtccggatgctcactccaaagtctcagcaggcccctgagggagggagctgtcagccaggg
aaaaccgagaacaccatcaccatg (Seq ID No: 386)
```

Homo sapiens heterogeneous nuclear ribonucleoprotein F (HNRNPF):

```
cgaccttcctgccgggccgggcggtccgaggctgctggagtgccgtgagcaggccgcgggaacgtc
gccgtcaccttgtctcggggcctcggcgctgcttcccgccaaaacacgtttaccgcgcgcccgggc
ctccaccttgcggaagggaccccaccaccacttggatttctgttgcaggttgagaacaaaaacat
gcacctggagtttccccggagccctctgcgtggttgagcttcggtggaatttcggggctcttggct
gccagccgcgcttgcctggtagcaacagaaaccagtcctgctcgcctccgtggacatttcattacc
atccagaagtgtctcccactgaaggcatccgtggttgtttttaagccacaaaaaagccacacccaa
gatcacctgacacccaccctgacaagtgtccatg (Seq ID No: 387)
```

Homo sapiens islet cell autoantigen 1, 69kDa (ICA1):

```
ccgcccctttccctcgccttcggctgacgctgacgtcggatgagtgatccggagggacgctccgac
cgcggccgggaggctcctgggggccggggctccgaggttataatataacttatcctctcatgcttt
tttcctgccccttctccccaaatcatcaacaatagaagaagaagaaacatg
(Seq ID No: 388)
```

Homo sapiens PWP2 periodic tryptophan protein homolog (yeast) (PWP2): gtgtctctgtgggcggccgccgggttgagctgcggcacacgtgcgacggccgtgatg (Seq ID No: 389)

Homo sapiens glutaminyl-tRNA synthetase (QARS): gtttcttttagtttccggtgtctctgcaatg (Seq ID No: 390)

```
Homo sapiens stearoyl-CoA desaturase (delta-9-desaturase) (SCD):
cggcctctgtctcctcccctcccgcccttacctccacgcgggaccgcccgcgccagtcaactcct
cgcactttgcccctgcttggcagcggataaaaggggctgaggaaataccggacacggtcacccgt
tgccagctctagcctttaaattcccggctcggggacctccacgcaccgcggctagcgccgacaacc
agctagcgtgcaaggcgccgcggctcagcgcgtaccggcgggcttcgaaaccgcagtcctccggcg
accccgaactccgctccggagcctcagcccccctggaaagtgatcccggcatccgagagccaagatg
(Seq ID No: 391)
```

Homo sapiens fragile X mental retardation, autosomal homolog 1 (FXR1): cggcctttgcggttccaacatg (Seq ID No: 392)

Homo sapiens musculin (MSC):

```
tagccttttcaaaaggcgcagcttaccgcggtgcgcgcggattctggacttgggcgccaactcgta
gtccacgctccccggggtcagcagaggggcgctcacgctctcgccacccacctcgctttctcaccc
cgcgcttcccggcctgggttttttagtcttccttggagcgctctctggcctccgcctccgccaggga
gcggaaggcggagacagcgagactggccaggggggaggaaagaggacgcgtgtgggcaaggggggac
aacgggatg (Seq ID No: 393)
```

Homo sapiens RNA binding motif protein 8A (RBM8A):

```
cgacctttccctctgcgacagtttcccgaggtacctagtgtctgagcggcacagacgagatctcg
atcgaaggcgagatg (Seq ID No: 394)
```

Homo sapiens heparan sulfate (glucosamine) 3-O-sulfotransferase 1 (HS3ST1):

```
ggtcctctgcgccctggcagccaggagtcgccgccacgaccgccgggtctcagtgggtgcctgcgc
cttctccccgcccgcctgccccgggccatccagaaacttgctctacccgccgcgggtgctcggcag
tgctgcccatggcccagcccaggagcctatttagggcgccggacgggctggacagaggcgcggctc
agtaattgaaggcctgaaacgcccatgtgccactgactaggaggcttccctgctgcggcacttcat
gacccagcggcgcgcggcccagtgaagccaccgtggtgtccagcatg (Seq ID No: 395)
```

Homo sapiens solute carrier family 12 (potassium/chloride transporters), member 6 (SLC12A6):

```
ctgtctcttgtaggcagggatcacagtctgaaacgacagcaaggaagaggtaggcagggaaaacta
actggaaggaagtttaaatacagaaagagcaaagtattatctaactataacaatg
(Seq ID No: 396)
```

Homo sapiens apelin receptor (APLNR):

```
cttcctccagggtctggagaacccagaggcagctcctcctgagtgctgggaaggactctgggcatc
ttcagcccttcttactctctgaggctcaagccagaaattcaggctgcttgcagagtgggtgacaga
gccacggagctggtgtccctgggaccctctgcccgtcttctctccactccccagcatg
(Seq ID No: 397)
```

Homo sapiens calpain 1, (mu/l) large subunit (CAPN1):

```
cgctcttcctggttgggccctgccctgagctgccaccgggaagccagcctcagggactgcagcgac
ccccaaacacccctcccccaggatg (Seq ID No: 398)
```

Homo sapiens cyclin C (CCNC):

```
cttcctttcgccgtcgccgccgcggagcggagtcgagccgagctgatttgatcgaggagcgcggtt
accggacgggctgggtctatggtcgctccgcgggccgctccgccggctggtgctttttttatcaggg
caagctgtgttccatg (Seq ID No: 399)
```

Homo sapiens glutamate dehydrogenase 1 (GLUD1):

```
cttcctccctagtcgcggggagtctgagaaagcgcgcctgtttcgcgaccatcacgcacctcccct
ccgcttgtggccatg (Seq ID No: 400)
```

Homo sapiens guanine nucleotide binding protein-like 1 (GNL1):
```
cctccttcctcgccgccggggcgccctctcggtgccactggctctcacgtgccagtagcccacccc
gcatcatcctctcgcctcgctcctggagggaagtgactatatctcccccgtccgccttccatcgcc
gccgcggcggtaattctgtcgggcccgcccgctgacgtcacctgctagccccgcctcctctagggt
cccgggcccctgcggcggggggctgccccggggggcagtcagttgaggcggcgggagctcggcggag
ggcgggccaggtgactggtccgggccatg (Seq ID No: 401)
```

Homo sapiens lysophosphatidic acid receptor 4 (LPAR4):

aggccttttgtgtcctgtttgctaaaggcatgcgggctacagcattcaagagagggagtcgttaa
caaagggaaagagataaatgtaaataagctcacatttacagaatgagcggtttgcagtaaaaagct
gcggcagcccagagtctgctactttaggctgggctaacctttccctgtaaaaaaaaaaaaaaaaaa
aaaaaaaaaaaatggataaaaatatgcacttccaaagggcgagttgcccatttacatgttttatta
gctaattatctacaggcatcagcacattctctcatctagcacactctttcttggggaggaaaatat
ttcctaccggtccatagtgtcagagtggtgaacccctgcagccagcaggcctcctgaaaaaaagt
ccatg (Seq ID No: 402)

Homo sapiens G protein-coupled receptor kinase 5 (GRK5):

gctcctctttgcagagggggaaactcttgggctgagagcaggaataatgcggtaggcaaggcgggc
tgctggctcccccggctccggcagcagcggcggcagcccgagcagcggcagcagcagcggcagcac
cccaggcgctgacagccccgccggccggctccgttgctgaccgccgactgtcaatg
(Seq ID No: 403)

Homo sapiens glutamic-pyruvate transaminase (alanine aminotransferase) (GPT):

agcccttctgtccctcccagtgaggccagctgcggtgaagagggtgctctcttgcctggagttcc
ctctgctacggctgccccctcccagccctggcccactaagccagacccagctgtcgccattcccac
ttctggtcctgccacctcctgagctgccttccgcctggtctgggtagagtcatg
(Seq ID No: 404)

Homo sapiens hydroxyacyl-CoA dehydrogenase (HADH): gggtctcctcgctgtcgccgccgctgccacaccatg (Seq ID No: 405)

Homo sapiens high density lipoprotein binding protein (HDLBP):

tcttctcctttaccaagatggcggcttgtccctgtttcgccacagttcctaccttatgagctcggt
tttcttatgcttataagagtggaacagcaaaagctggcaggctgacagaggcggcctcaggacgga
ccttctggctactgaccgttttgctgtggttttcccggattgtgtgtaggtgtgagatcaaccatg
(Seq ID No: 406)

Homo sapiens histidine triad nucleotide binding protein 1 (HINT1): gttcctcccttcttccgagcctctcctctggccgccgcgcgggagagaggccgagatg (Seq ID No: 407)

Homo sapiens heat shock 70kDa protein 1A (HSPA1A):

ctaccttttttcgagagtgactcccgttgtcccaaggcttcccagagcgaacctgtgcggctgcagg
caccggcgcgtcgagtttccggcgtccggaaggaccgagctcttctcgcggatccagtgttccgtt
tccagcccccaatctcagagcggagccgacagagagcagggaaccggcatg
(Seq ID No: 408)

Homo sapiens nucleolin (NCL):

cagtctttcgcctcagtctcgagctctcgctggccttcgggtgtacgtgctccgggatcttcagca
cccgcggccgccatcgccgtcgcttggcttcttctggactcatctgcgccacttgtccgcttcaca
ctccgccgccatcatg (Seq ID No: 409)

Homo sapiens nuclear factor, interleukin 3 regulated (NFIL3):

```
ccgccccttttctttctcctcgccggcccgagagcaggaacacgataacgaaggaggcccaacttca
ttcaataaggagcctgacggatttatcccagacggtagaacaaaaggaagaatattgatggattttt
aaaccagagttttttaaagagcttgagaatacgggggaaattaatttgttctcctacacacatagata
gggtaaggttgtttctgatg (Seq ID No: 410)
```

Homo sapiens protein phosphatase 1, regulatory subunit 3C (PPP1R3C):

```
cagtctctcccagcgaccgccgcgggggcaaggcctggagctgtggttcgaatttgtgcaggcagc
gggtgctggcttttagggtccgccgcctctctgcctaatg (Seq ID No: 411)
```

Homo sapiens protein tyrosine phosphatase, non-receptor type 14 (PTPN14):

```
agttcttttccaacttttttctcggcggagtgagcgcagcgggcgcagactcggggggcaggttgctgt
gcttctccgggctcagccgcctgctctcctggctcaggtcctcggggagccctagacagacatcaa
gtggccactggcgctccttcccctcccagctgagccatcctccccggcctcctcgggcgggacagc
cccgtgcttaggttttttctccttttctcccccggtgcgcctctgctcggactctcgcgccgggatc
gcggcggaaacctccctccccttttcgcctcctgcggctccttccttcgcccctcctccgccagtc
actggaatcaattccgtggggaatcggctccgccgccgcgaaggacagcctttccgcgcgggactc
cggggcgccacgggggggccatgtaagcagctatcttccagagggccacactgggcatggacacccctt
ttccctgcctggaggagcacaggtgatagtgtaattttccagtcacgaaactgctaaggccatctc
aggggcgtgtgcgccaggataggcgggcggcgtccgaggaccacatagccatg
(Seq ID No: 412)
```

Homo sapiens selenoprotein P, plasma, 1 (SEPP1):

```
ctttcttttaagttgataacaatcagctcaggggtttgctctgcttgcaaggtcactgcaagaatg
aacattgaactttggactatacctgaggggtgaggtaaacaacaggactataaatatcagagtgtg
ctgctgtggctttgtggagctgccagagtaaagcaaagagaaaggaagcaggcccgttggaagtgg
ttgtgacaaccccagcaatg (Seq ID No: 413)
```

Homo sapiens serine hydroxymethyltransferase 2 (mitochondrial) (SHMT2):

```
agctcttctcgcgcatgcgttctccgaacggtcttcttccgacagcttgctgccctagaccagagt
tggtggctggacctcctgcgacttccgagttgcgatg (Seq ID No: 414)
```

Homo sapiens tyrosine kinase with immunoglobulin-like and EGF-like domains 1 (TIE1):

```
tttcctcttcctccccagcaccgacccacactgaccaacacaggctgagcagtcaggcccacagca
tctgaccccaggcccagctcgtcctggctggcctgggtcggcctctggagtatg
(Seq ID No: 415)
```

Homo sapiens coiled-coil domain containing 6 (CCDC6): cctcctttcccccagcccgccgcggccatg (Seq ID No: 416)

Homo sapiens nuclear receptor coactivator 4 (NCOA4):

```
ggacctttcgcactcgggtcaggggtaaagcagcctgtcgcttgccgggcagctggtgagtcggtg
acctggcctgtgaggagcagtgaggagaatg (Seq ID No: 417)
```

Homo sapiens chromatin assembly factor 1, subunit B (p60) (CHAF1B):

gtgcctctgactgtccgggtccctccagcattttgcagctttctcctgtcttgaagaagtagaacg
gtgcccgagaaacgttttttccccttcgagactcaggaggatgaaagtcatcacttgtgaaatagcc
tggcacaacaaggagcccgtgtacagcctggacttccagcatg (Seq ID No: 418)

Homo sapiens 3'-phosphoadenosine 5'-phosphosulfate synthase 1 (PAPSS1):

agccccgccccgctcgctggcctgccctcctcttgctaccctcccggcgcagagaacccccggctgc
tcagcgcgctccgcggtcatg (Seq ID No: 419)

Homo sapiens Fas apoptotic inhibitory molecule 3 (FAIM3): tgccctcctcttgctaccctcccggcgcagagaacccccggctgct-cagcgcgctccgcggtcatg (Seq ID No: 420)

Homo sapiens N-acetylated alpha-linked acidic dipeptidase 2 (NAALAD2):

cagcctcctgccagcgcgctctctgtttctctgcagccccgaagctcgcgaatgtagcaggcgccc
caagctcggtcctcaagaagccatggcggaatccaggggccgtctgtacctttggatgtgcttggc
tgctgcgctggcatctttcctgatgggatttatggtgggtaagt (Seq ID No: 421)

Homo sapiens abl-interactor 1 (ABI1): ctgtctctttaacgcgagaggaag-cgatgcagaggggtggaaatg (Seq ID No: 422)

Homo sapiens potassium voltage-gated channel, Isk-related family, member 3 (KCNE3):

cttccttttctgccttctctcctgctttctagctctgggctttcccagctccgaagtcaatactga
gatcccagatgtgtccagagacatcctgaagaggctcggggggtggaggagccttagtgtgtccaca
aagggactcctgaaactgactgagagccagt (Seq ID No: 423)

Homo sapiens target of mybl (chicken)-like 1 (TOM1L1): ggccctctggcgctaccatg (Seq ID No: 424)

Homo sapiens ubiquitin-like modifier activating enzyme 2 (UBA2):

cgcccttcccccacccgcttccggccgcgggctcggttctcccgcctccgcctccgccgcggctcgt
ggttgtcccgccatg (Seq ID No: 425)

Homo sapiens scavenger receptor class B, member 2 (SCARB2):

ctccctccttgcagttggatccctggcgggtgcggcccggcccggcccgtgagcggcgcacagaat
g (Seq ID No: 426)

Homo sapiens insulin induced gene 1 (INSIG1):

actcctcctttcccccgccccgcctccgttcggagagccggcgggcgggcgcctctcggccaggaa
gcgcctcttggacgcgtgtgaccgatg (Seq ID No: 427)

Homo sapiens kinesin family member C3 (KIFC3):

aggcctcttctgaggctctaggtgccccagtagcagggccttctgcagcaaggccgggaactgctg
caccattggtgtgtgttttaccttaagggactccaggcagcttccttgctgggaagatattcatttgc
tggggtggggctgggggtgcagaggtaggaagtgctgtggctagaaggcggcctggccagcgagta
ggtggtggagcgagtgagagcgtgtgcgctgtaaacagtgtgagtgcatg
(Seq ID No: 428)

Homo sapiens LIM domain kinase 2 (LIMK2):

```
aggcctcttctgaggctctaggtgccccagtagcagggccttctgcagcaaggccgggaactgctg
caccattggtgtgttttaccttaagggactccaggcagcttccttgctgggaagatattcatttgc
tggggtggggctgggggtgcagaggtaggaagtgctgtggctagaaggcggcctggccagcgagta
ggtggtggagcgagtgagagcgtgtgcgctgtaaacagtgtgagtgcatgtgcgccagcgcgtgca
aggacacggtaagggatgtacatgtattgtctcgtgagtaagagcttgtgtgtgtgttgggatggg
aagacacgtactggtatgagagcccgcgtgagaagtgtatgtgtgagtactcgcgtggaagttttg
cactcgggtttgaggctgtgcaaaagtacgcatggctcaccaggtgtggggctgtgtgggctgcct
```

```
cgtgtgtgccagcccgtgtgcaggcctgttttgtgagagccttcagggaacgcatgagcacgtgtg
ccagtgcgagtgcgggacgcggggaggcgggagagaccgagtgggaggccccgcgaaggagtggga
gtgggagtgggagtgccggcgggagacctgcggggggcgcgcccgggctgacgcgtgcgcgccagtg
cgcgtgagtgcgggcgcgcgccgccgccccccgccggggtcggagccggttgccatgggaacgcgc
cgcggccccgagttaatcatttcctgtggaaagtgtgcgggaggggcgcgagcgggctggccgagga
ggaggcggcggcgtggagctgcctcctgccggcgggccgggccgggccgagccccgggcgctgcgg
cgacgcctggatcctgcctccgccaggccggctgcctggtgccccgaggaggctgctgagccccag
gccatg (Seq ID No: 429)
```

Homo sapiens lectin, mannose-binding, 1 (LMAN1): cctcctccgcgttcca-gaatccaagatg (Seq ID No: 430)

Homo sapiens MRE11 meiotic recombination 11 homolog A (S. cerevisiae) (MRE11A):

```
cgttctctcccgcggaattcaggtttacggccctgcgggttctcagaggcaagttcagaccgtgtt
gttttcttttcacggatcctgccctttcttcccgaaaagaagacagccttgggtcgcgattgtggg
gcttcgaagagtccagcagtgggaatttctagaatttggaatcgagtgcattttctgacatttgag
tacagtacccaggggttcttggagaagaacctggtcccagaggagcttgactgaccataaaaatg
(Seq ID No: 431)
```

Homo sapiens nascent polypeptide-associated complex alpha subunit (NACA):

```
cttccttctgcaacaggcgtgggtcacgctctcgctcggtctttctgccgccatcttggttccgcg
ttccctgcacagtaagtactttctgtgccgctactgtctatccgcagccatccgcctttctttcgg
gctaagccgccccggggactgagagttaaggagagttggaggctttactgggccacagggttccta
ctcgcccctgggcctccggacaaaatggggtctgcggttggtgtcctggcaaaagcagggtagaag
ggctgcggggcgggcccagaatccgagcctgcagagatgggagcagttgcagtgttgagggcggaa
gaggagtgcgtcttgttttgggaactgcttcacaggatccagaaaaggaaatg
(Seq ID No: 432)
```

Homo sapiens claudin 11 (CLDN11):

```
cgcccttcgccgctgagctcgcagcctccggcgcccacctccacctccagtgtcccgcctcgggcc
gtcgccctccagcggctcgcgagcgtgggagacgtacctgggcaggcactgtccagcccaggccca
ggcacagccgtgaggggcgaggcacggggacatcctggcggccaccatg (Seq ID No: 433)
```

Homo sapiens retinoblastoma binding protein 4 (RBBP4): ccgcccctcccgcaacgctcgaccccaggattcccccggctcgcctgcccgccatg (Seq ID No: 434)

Homo sapiens acyl-CoA synthetase medium-chain family member 3 (ACSM3):

```
ccctcttctttagactgccacgaggaaaaagcagatgtgagaactcaaggttcagggctgctcttc
taagaaacaagtctgccataatctccatctgtgttggaatctgttaactaatgaactggtctctgt
gcaaatcctgagtgctaaagcttccaacaagactgatg (Seq ID No: 435)
```

Homo sapiens syndecan binding protein (syntenin) (SDCBP):

```
cgctctcttacactcgggcctcagaagtccgtgccagtgaccggaggcggcggcggcgagcggttc
cttgtgggctagaagaatcctgcaaaaatg (Seq ID No: 436)
```

Homo sapiens serum/glucocorticoid regulated kinase 1 (SGK1):

```
agtccttctcattccttgcccccgcccaaggctctcttcaccttccccgcgggggtcctctcgttt
tctgtctcccaaatgctggcttccgcctttcctcccccgcttatttacttaattaaggccctggg
gctgcaccccaccggcagctccttcggggggtgtggccgaagagctccgagggcggggctgaccgag
ccatattcgggcgtggccggtggtgattggtgagggcggggcctgccgcaggggggcggggcctgca
```

```
ggtttggccccgcagggagcgcagctggcgccgctgggagctggtggcgcggcgcaggtcccggc
cgagtgtggcgcagcagtggcggcgcttcccattcgccatgcgccgggggtgggtgcccgaaggtt
gcatgatggaatttgaacattacttcaagaggttttgtattttggattagttaattgggtttgtcc
tctgctgactgtttcttcggatgcattttttggtgtgctcttgagggattaaatg
(Seq ID No: 437)
```

Homo sapiens Wolf-Hirschhorn syndrome candidate 2 (WHSC2):

```
cgtccttccggctctcggctttgccacaaagcttcccgaagacgcggccgctacccggagacgcgg
tcgccacccagaagcgctctcccgggaagccccgctcgtgggaccgcgccacctgcgccgcctctg
cggcccgcagcccgacgggcgccgccatgttggggtcctagcgagggacgcgtaggtgtcttcata
agatg (Seq ID No: 438)
```

Homo sapiens nuclear receptor subfamily 1, group H, member 3 (NR1H3):

```
cagtccttttgcaagagctgctaagagcgctgggtaaggagaggaaggggagagacatggaacttg
gctggtctgcagggaaatgccactgttttggccgggagtaggggcgggagtggcgggagaggggg
tggccggctggggaggagccagcctggtggagaagctgccctgtgggcggggggtgaggaggggagg
gctgtggtcaccaggcaggaaggaggggtggcctgacccctcggcagtccctcccctcagcctttc
cccaaattgctacttctctggggctccaggtcctgcttgtgctcagctccagctcactggctggcc
accgagacttctggacaggaaactgcaccatcctcttctcccagcaaggggggctccagagactgcc
cacccaggaagtctggtggcctggggatttggtgggtctgctccttag (Seq ID No: 439)
```

Homo sapiens glypican 6 (GPC6):

```
cctcctttctccttccctcttgcctccagtgactgtctccaggatttctctcttcctatttcagga
ggactctcacaggctcccacagcctgtgttaagctgaggtttcccctagatctcgtatatccccaa
cacatacctccacgcacacacatccccaagaacctcgagctcacaccaacagacacgcgcgcat
acacactcgctctcgcttgtccatctccctcccggggggagccggcgcgcgctcccacctttgccgc
acactccggcgagccgagcccgcagcgctccaggattctgcggctcggaactcggattgcagctct
gaaccccatggtggtttttttaaacacttcttttccttctcttcctcgttttgattgcaccgtttc
catctggggggctagaggagcaaggcagcagccttcccagccagcccttgttggcttgccatcgtcc
atctggcttataaaagtttgctgagcgcagtccagagggctgcgctgctcgtcccctcggctggca
gaagggggtgacgctgggcagcggcgaggagcgcgccgctgcctctggcgggctttcggcttgagg
ggcaaggtgaagagcgcaccggccgtggggtttaccgagctggatttgtatgttgcaccatg
(Seq ID No: 440)
```

Homo sapiens peptidylprolyl isomerase F (PPIF):

```
cggccttctgggcgcgcgcgacgtcagtttgagttctgtgttctccccgcccgtgtcccgcccgac
ccgcgcccgcgatg (Seq ID No: 441)
```

Homo sapiens ARP1 actin-related protein 1 homolog A, centractin al pha (yeast) (ACTR1A): agttccttccccagaaggagagattcctctgccatg (Seq ID No: 442)

Homo sapiens tripartite motif containing 28 (TRIM28):

```
ggctctttctgcgagcggggcgcgcgggcgagcggttgtgcttgtgcttgtggcgcgtggtgcgggt
ttcggcggcggctgaggaagaagcgcgggcggcgccttcgggaggcgagcaggcagcagttggccg
tgccgtagcagcgtcccgcgcgcggcgggcagcggcccaggaggcgcgtggcggcgctcggcctcg
cggcggcggcggcggcagcggcccagcagttggcggcgagcgcgtctgcgcctgcgcggcgggccc
cgcgcccctcctcccccctgggcgccccggcggcgtgtgaatg (Seq ID No: 443)
```

Homo sapiens aminoadipate-semialdehyde synthase (AASS):

```
cggccttccatcccagtttcttctaggaattcggagcctcccctgcagcgactcggaagattcgag
gcggcgggggacaagtcggcgccccagagcggacgagtcaccaggtgtcaagatg
(Seq ID No: 444)
```

Homo sapiens cornichon homolog (Drosophila) (CNIH): ccgcctttctccgctggcaacggcgccgctccccgctcctcctccccagccatg (Seq ID No: 445)

Homo sapiens M-phase phosphoprotein 10 (U3 small nucleolar ribonucleoprotein) (MPHOSPH10): ctcccttcccttgcatgctgcattgtgtcgggagttgctgacagccatg (Seq ID No: 446)

Homo sapiens ubiquitin specific peptidase like 1 (USPL1):

```
ccgccttcctagtggagacgcgagtgggggaggagcagtccgaggggaacgtgggttgaacgttgc
aactagggtggagatcaagctggaacaggagttccgatcgacccggtaccaagaaggggagtgccc
gcggcagggttcattgaaaaaatccttagtgatattgacatgtctcaagtgacataaattagccaa
tgactcggaatg (Seq ID No: 447)
```

Homo sapiens solute carrier family 23 (nucleo-base transporters), member 1 (SLC23A1): tggcctttgtcaagtcatcccctcttctcctcaggaactgctcaaacctgtgccccaaagatg (Seq ID No: 448)

Homo sapiens splicing factor 3b, subunit 4, 49kDa (SF3B4): ggatctctttcgccatg (Seq ID No: 449)

Homo sapiens DnaJ (Hsp40) homolog, subfamily A, member 2 (DNAJA2):

ctgtctccctcggcctgtgccgccgccgacgccgcttgtgggcccgactccgctctgtctgcttcg
ccaccttctccccgagcactgcccggccggccgccatg (Seq ID No: 450)

Homo sapiens calicin (CCIN):

catcctctcttccaccctctcttctccctggtcaaccgctctgcaaacaaccatcaatctgatccc
acaggcctgagaaagtctgctctccagtacctgctgctgatctgtttcagccgacaagaggcacca
tg (Seq ID No: 451)

Homo sapiens mannosidase, beta A, lysosomal (MANBA): ctgcctttcgatctctccacatctcggtggcgcgggatctcaagatg (Seq ID No: 452)

Homo sapiens microtubule-associated protein 1B (MAP1B):

aatcctttctcctgccgcagtggagaggagcggccggagcgagacacttcgccgaggcacagcagc
cggcaggatg (Seq ID No: 453)

Homo sapiens malate dehydrogenase 1, NAD (soluble) (MDH1):

gagccttttctcgctaacaccgctcgccctctccgagtcagttccgcggtagaggtgacctgactc
tctgaggctcattttgcagttgttgaaattgtccccgcagttttcaatcatg
(Seq ID No: 454)

Homo sapiens microfibrillar-associated protein 1 (MFAP1):

gtttctctatcagtcgcgcagctgtgttcgcggactcaggtggaaggaatttcttctcttcgttga
cgttgctggtgttcactgtttggaattagtcaagtttcgggaatcaccgtcgctgccatcaacatg
(Seq ID No: 455)

Homo sapiens chaperonin containing TCP1, subunit 3 (gamma) (CCT3):

ggttctctctctccagaaggttctgccggttcccccagctctgggtacccggctctgcatcgcgtc
gccatg (Seq ID No: 456)

Homo sapiens tubulin, alpha 1a (TUBA1A): caacctctcctcttcgtctccgccatcagctcggcagtcgcgaagcagcaaccatg (Seq ID No: 457)

Homo sapiens CD164 molecule, sialomucin (CD164): ctttctcccgaacgccagcgctgaggacacgatg (Seq ID No: 458)

Homo sapiens cysteine-rich secretory protein 3 (CRISP3): ctctctctgcaccttccttctgtcaatagatg (Seq ID No: 459)

Homo sapiens SMYD family member 5 (SMYD5):

cggcctccatgtgcgacgtgttctccttctgcgtgggcgtggcgggccgcgcgcgcgggtctccgtgg
aagtccgtttcgtgagcagcgccaaggtgaggtcggggcgggtcctgccgggagcctctcccccagt
ccggccatg (Seq ID No: 460)

Homo sapiens kelch repeat and BTB (POZ) domain containing 10 (KBTBD10):

ctgcctttttacagctagacctgtgtgctgcaaggagctaaggccttcagtgtccccttccttacc
caggtttctcacagaatg (Seq ID No: 461)

Homo sapiens aldo-keto reductase family 1, member A1 (aldehyde reductase) (AKR1A1):

ccgcccttgcaccgcccacgtggccagcgccacctgcctcattgtgcccaggagttctccaaacc
cgcgctgcggagtgagtgaccaagttccggccagttcgacctcgaggatccagaggtggagacggt
actacctccagctctgttttccatccccttcaggtccttcctcgggaggcggcgaaggcggtcca
ccctgcgcgtgatcctttatgcccggcccctgccctccctccgggtggaacttccccctcaccgc
cagacttaagctgaggatcgttggatctctggcggggtgcagaactgagcccaggccacagtaccc
tattcacgctctgtgcttgtgccaaggtttcaagtgatcctcccgcctcagcctgcccaggtgctg
agattacatgtatgagccactgcacctggaaaggagccagaaatgtgaagtgctagctgaaggatg
agcagcagctagccaggcaaaggggggcaatg (Seq ID No: 462)

Homo sapiens TRK-fused gene (TFG):

tgttcttcccccacctgccacgtacagagcccaagttctcgctaggcttgttgggtcagcgcgatt
ggccgggggcccgcgcgagcctgcgagcgaggtgcggcggtcgcgaagggcaaccgagggggccgtg
accaccgcctccccgcgacgccccagtccagtggcctcgcgtccgcccattcagcggagacctgcg
gagaggcggcggccgcggcctccgcaagccgtctttctctagagttgtatatatagaacatcctgg
agtccaccatg (Seq ID No: 463)

Homo sapiens 3'(2'), 5'-bisphosphate nucleotidase 1 (BPNT1):

catccttctcaaaagacttattgacagtgccaaagctcggtactggacacaacgagggacctgggt
ctacgataacgcgcttttgctcctcctgaagtgtctttggtccaacgttgttccagagtgtaccat
g (Seq ID No: 464)

Homo sapiens guanine nucleotide binding protein (G protein):

ttttctctctctctttcactgcaaggcggcggcaggagaggttgtggtgctagtttctctaagcca
tccagtgccatcctcgtcgctgcagcgacacacgctctcgccgccgccatg
(Seq ID No: 465)

Homo sapiens major histocompatibility complex, class II, DM alpha (HLA-DMA):

caccctctcggggagggagttggggaagctgggttggctgggttggtagctcctacctactgtgtg
gcaagaaggtatg (Seq ID No: 466)

Homo sapiens transmembrane protein 50B (TMEM50B):

tctccttcctgcgcgcgcgcctgaagtcggcgtgggcgtttgaggaagctgggatacagcatttaa
tgaaaaatttatgcttaagaagtaaaaatg (Seq ID No: 467)

Homo sapiens lactoperoxidase (LPO):

cagtctttcctgctaagcctcagcgtctcctccaagccacatcaaaatctttccttctgggccttt
cccagaagtgaattcttgctggaaggtataaaagaccagctcctccaagcagagcaactccctggc
tgccgtgaaaagacaaggcactgggcagtgatg (Seq ID No: 468)

Homo sapiens NEL-like 2 (chicken) (NELL2): ctgcctttacaacagaggga-gacgatggactgagctgatccgcaccatg (Seq ID No: 469)

Homo sapiens nucleobindin 1 (NUCB1): cgccctctgcggtgaaggagagacca-cactgccatg (Seq ID No: 470)

Homo sapiens paired box 9 (PAX9):

```
aagcctcttttcatcggggcacagacttccttttacttcttccttttgccctctcgcctcctcctcc
tgggaagaagcggaggcgccggcggtcggccgggatagcaacaggccgggccactgaggcggtgcg
gaaagtttctgtctgggagtgcggaactggggccgggttggtgtactgctcggagcaatg
(Seq ID No: 471)
```

Homo sapiens cyclin-dependent kinase 16 (CDK16):

```
cgccctttattcttgctcggcctcgccacagagagcaaatcagattggctgggcgacaacctcaaa
gggcggggctgcacacgttcactacgggaatgaggtagcggtggaggggcagttgggcggggata
ggccgtcctagctaaggtggtaaaggccaataactcttcaggctgcctctcctcgaaaagtcatct
tctcgcgaacctttaaaatgccttcctccccaagcacctcaagggactagaactgagtgcttcatt
tgtcttttttcctccttgcaaaagtcccgtttgccaccatggggatgtaccaagtgagaccgagta
gggggaacgagtggtgattgacgcgccaggttactggccactgctcacctaggcgctagcaaactt
ctgccaagatcggaactgagtactaaacagcctccacagttctccctggtgccgtctccggcttgg
cgccgcatcctcctctgggctcgcgatggccgcgtcccctcccgctgcggacgggtcctttggtac
atg (Seq ID No: 472)
```

Homo sapiens serpin peptidase inhibitor, clade E (nexin, plasminogen activator inhibitor type 1), member 2 (SER-PINE2):

```
ctgcctcttttccggctgtgaccctcctcgccgccgccgcttggctgcgtcctccgactccccgcgc
cgccgagaccaggctcccgctccggttgcggccgcaccgccctccgcggccgcccccctggggatcc
agcgagcgcggtcgtccttggtggaaggaaccatg (Seq ID No: 473)
```

Homo sapiens pancreatic lipase-related protein 1 (PNLIPRP1):

```
aactcctttccccctgctgtgacgtacaggtgaggtaaacagtactgaagtccaggtcgtcggtgc
tcactgctctggcaatgcccggtgagactgaattatgtttaaatttattgtagatg
(Seq ID No: 474)
```

Homo sapiens peripherin (PRPH): ggctccttcccagcccccggcctagctctgcgaacggtgactgcccatccttggccgcaatg (Seq ID No: 475)

Homo sapiens RAD21 homolog (S. pombe) (RAD21):

```
gacccttttcccctccccgggccacccagcccgcccaactcccagcggagagcaaggttttcttct
gttttcatagccagccagaacaatg (Seq ID No: 476)
```

Homo sapiens signal sequence receptor, delta (SSR4): ttttcttttcctctaggcagagaagaggcgatg (Seq ID No: 477)

Homo sapiens tissue factor pathway inhibitor (lipoprotein-associated coagulation inhibitor) (TFPI): ctccctctttgctctaacagacagcagcgactttaggctggataatagtcaaattcttacctcgct

ctttcactgctagtaagatcagattgcgtttctttcagttactcttcaatcgccagtttcttgatc
tgcttctaaaagaagaagtagagaagataaatcctgtcttcaatacctggaaggaaaaacaaaata
acctcaactccgtttttgaaaaaaacattccaagaactttcatcagagatttttacttagatg
(Seq ID No: 478)

Homo sapiens ubiquinol-cytochrome c reductase binding protein (UQCRB): gcttctctttctggtcaaaatg (Seq ID No: 479)

Homo sapiens mitogen-activated protein kinase kinase kinase 12 (MAP3K12):

ccgccttttgtgctgcggccgcggagcccccgagggcccagtgttcaccatcataccaggggccag
aggcgatg (Seq ID No: 480)

Homo sapiens sushi-repeat containing protein, X-linked (SRPX):

tggtctcttcggtctcctgccgcccccgggaagcgcgctgcgctgccgaggcgagctaagcgcccg
ctcgccatg (Seq ID No: 481)

Homo sapiens aminopeptidase puromycin sensitive (NPEPPS):

 cccctctccctccctccttgcgggccctcctcccttccctccctccgccccttccccgtagg
cagcccgcccgccagtccgcccgcaccgcctccttcccagcccctagcgctccggctgggtctctc
ccccgcccccaggctcccccggtcgctctcctccggcggtcgcccgcgctcggtggatg
(Seq ID No: 482)

Homo sapiens fibulin 5 (FBLN5):

tcgccttctgcccgggcgctcgcagccgagcgcggccgggggaagggctctcctcccagcgccgagc
actgggccctggcagacgccccaagattgttgtgaggagtctagccagttggtgagcgctgtaatc
tgaaccagctgtgtccagactgaggcccatttgcattgtttaacatacttagaaaatgaagtgtt
cattttaacattcctcctccaattggtttaatgctgaattactgaagagggctaagcaaaaccag
gtgcttgcgctgagggctctgcagtggctgggaggacccggcgctctccccgtgtcctctccacg
actcgctcggcccctctggaataaaacaccgcgagccccgagggcccagaggaggccgacgtgcc
cgagctcctcggggggtcccgcccgcgagctttcttctcgccttcgcatctcctcctcgcgcgtct
tggacatg (Seq ID No: 483)

Homo sapiens lysophospholipase I (LYPLA1): cgctcttccttccgcttgcgctgtgagctgaggcggtgtatg (Seq ID No: 484)

Homo sapiens high mobility group nucleosomal binding domain 4 (HMGN4):

tcgtcttctctgtcttagggctggtgctggccctgcccacgcctagggctccggcgcgtcacgggc
ctcagctgggattcccgcgcccctcggacggccacgagactcggacatctttccaggaacagcgtg
aggaggacagaagcacccaacaggactgctcaagccacctgcgaacactgctgctaccatg
(Seq ID No: 485)

Homo sapiens eukaryotic translation initiation factor 3, subunit M (EIF3M): agttcccttttccggtcggcgtggtcttgcgagtggagtgtccgctgtgcccgggcctgcaccatg (Seq ID No: 486)

Homo sapiens Sec23 homolog A (S. cerevisiae) (SEC23A): cctcctcttgacgtggcagaggcggcgccagccatg (Seq ID No: 487)

Homo sapiens cartilage associated protein (CRTAP):

```
cgtcctctttcctttccttctccctcccctttttcccttccttcgtcccttccttccttcctttcgc
cgggcgcgatg (Seq ID No: 488)
```

Homo sapiens vesicle amine transport protein 1 homolog (T. californica) (VAT1):

```
ccgcccctcccgctggatcccgcagccgcggctcttcccgacgcgttccgccttccccagctgtgc
actctccatccagctgtgcgctctcgtcgggagtcccagccatg (Seq ID No: 489)
```

Homo sapiens importin 7 (IPO7):

```
gcttctctttcctttcgcgccggttgccgctgcggagcgcggcgggtccatgtgcgcagtgagtgg
cgctattcctggcccagtagcacccgagccccgggtttgaccgagtccgcgctgcgatg
(Seq ID No: 490)
```

Homo sapiens ATG7 autophagy related 7 homolog (S. cerevisiae) (ATG7):

```
gctcctttgcgcacgcgcgccgcttcccagtggcaagcgcgggcaggaccgcgttgcgtcatcggg
gcgcgcgcctcagagagagctgtggttgccggaagttgagcggcggtaagtgagccgcggcgggcg
agggtgtagtggggtcttgctgggccggttttggaggcctggagtcaaggggcgagctcgccaggg
agggcgagggtcacagcaagtctcaggatcctcctctgccagtttctgggtggtccttcctcctcc
aggactcactgattccggctggcgccttcgtctgtagccgcgtcccctcagactggttcagtcc
ggggtcttctgacttggaagctcgtgctgatttcctaagtcagcccctcctgtcctcttggtaggc
agtgctcagaatcttcagtgttggaacacgggagatgggacatttggattcccagcctggctgtgt
ctggatttgctgtctctggcacgttccttccccatctaagctgcttttccatctgcaaaatgggaa
tgataatccgccatttgtttaagtgaggaggttaaataagtttactttctgagaaagaagattctc
gattccttggttacagggttagaaactaatg (Seq ID No: 491)
```

Homo sapiens dynactin 2 (p50) (DCTN2):

```
cgctccctttgccgccgccttagcccgggacccgaacccagcctctcccctacccgaacaccggcc
ccggctccaccgaggcccgggtcccccagcccgtctcgccgccgccatg (Seq ID No: 492)
```

Homo sapiens acidic (leucine-rich) nuclear phosphoprotein 32 family, member B (ANP32B):

```
agcccccttttccctccatggtttctctccgctcccgtgagtaacttggctccggggggctccgctc
gcctgcccgcacgccgcccgccacccaggaccgcgccgccggcctccgccgctagcaaacccttcc
gacggccctcgctgcgcaagccgggacgcctctcccccctccgcccccgccgcggaaagttaagtt
tgaagagggggggaagaggggaacatg (Seq ID No: 493)
```

Homo sapiens protein C receptor, endothelial (PROCR):

```
acttctcttttccctagactgcagccagcggagcccgcagccggcccgagccaggaacccaggtcc
ggagcctcaacttcaggatg (Seq ID No: 494)
```

Homo sapiens actin related protein 2/3 complex, subunit 1A, 41kDa (ARPC1A):

```
cgctccctctgggcttccgtcctccgcccgcgcccgacggagcctgttcgcgtcgactgcccagag
tccgcgaatcctccgctccgagcccgtccggactcccccgatcccagctttctctcctttgaaaac
actaagaataatg (Seq ID No: 495)
```

Homo sapiens chaperonin containing TCP1, subunit 4 (delta) (CCT4):

```
aggccccccttctccgcctccgcctcctcccgacgccggcgccgctttctggaaggttcgtgaaggc
agtgagggcttaccgttattacactgcggccggccagaatccgggtccatccgtccttcccgagcc
aacccagacacagcggagtttgccatg (Seq ID No: 496)
```

Homo sapiens Niemann-Pick disease, type C2 (NPC2): gcttctttcccgagcttggaacttcgttatccgcgatg (Seq ID No: 497)

Homo sapiens phosphoribosylaminoimidazole carboxylase, phosphoribo sylaminoimidazole succinocarboxamide synthetase (PAICS):

```
acccctcttttctagagttctgcctcgcttcccggcgcggtcgcagccctcagcccacttaggata
atg (Seq ID No: 498)
```

Homo sapiens ST6 (alpha-N-acetyl-neuraminyl-2,3-beta-galactosyl-1,3)-N-acetylgalactosa minide alpha-2,6-sialyltransferase 2 (ST6GALNAC2): ctcccttctgcctgggacgtcagcggacggggcgctcgcgggccggggctgtatg (Seq ID No: 499)

Homo sapiens polymerase (RNA) III (DNA directed) polypeptide C (62kD) (POLR3C) :

```
aagccctttccgaggatggcaaaggatctgggaatgcttctccaaagatatgtggatggacgaaat
aggtctctggtgatactgaggcggggtggggacggggaggcaaagacttggcttcttaggaattgg
aagaaataagtaaacaatgtttggtagcaatttgtaataaggaagtaatcataaaattaactacgt
ccgtttctgattgtgtcaactttgtcaaggagtagaagtttaagaattgaatactgtcctgcaaac
aacgtaacctcatctcctgtttgacacaccctgttgagaagcagtcctttacctcctaaatttctt
tttcgaaattatcatttcctttatggactgagaataacactgcctgttcactcccaccgagctgtg
aacagtgaccttaattcttccaagcagggaagtgtagaaactaaggtctgtgacagaccgcaaat
catctcccaatctttaaggaaaatcagaatcacgcataatcccatagagataaatttgatgcatag
tcttttcctatgcatacatttttcctttttttttacaataattgaattttttatattttttcagctt
gcttctgtcacttaatatattatgagtaatttttttttggttttttttgttttggagacagaatctc
gcactgtcgcccgggttggagtgcagtggcgcgatctcggctcactgcaacctctgcctcccggct
tcaagcgattctcctgtctcagcctccctagtagctgggattacaggcacccgccaccacgcccag
ctaattttttttgtgtgttttttagtagagaaggggtttcactatattggccaggctggtctcaaact
cctgacctcatgatacgcccacctcggtctcccaaagtgctaggattacaggcctgagccaccgcg
ccagcctattatgaataattttctacatgaatacgcatcgtactaaataactttaaatgttggtgt
agtatgccattgtatgggtatggcatcatttattgttagacgttagattgtttccactaagtcggt
attataaagagaactaatgacttcattattattagcttttctttctttggacacaatatccaaaa
agaaattgttgtttcaaagatatgcaagattttttaaggcttttttgatatgtattgtcaaattgccc
tccagaaagaatacatgaatttacactcagcagctctgcttccagcgtgaaagactttctattgta
ccattttggtgttttttccctagctctcagactccccagtacaatg (Seq ID No: 500)
```

Homo sapiens influenza virus NS1A binding protein (IVNS1ABP):

```
gtgtctcccggtcgcgcgtggaggtcggtcgctcagagctgctgggcgcagtttctccgcctgctg
cttcggcgcggctgtatcggcgagcgagcgagttcccgcgagttctcggtggcgctccccttcct
ttcagtctccacggactggcccctcgtccttctacttgaccgctcccgtcttcgccgccttctgg
cgctttccgttgggccgattcccgcccgcttcctcctgcttcccatcgaagctctagaaatgaatg
tttccatctcttcagagatgaaccagattatgatgcatcattatcacagaagaaattcgtgtctat
agcttttaaggacttgattacatcattttcaagcctgatagttttggaatcaccattagagcttaa
gacacacctgccttcatttcaaccacctgtcttcataccctgacgaagtgcacctttttaacactcc
tttgtccttggattacttaagagttcccagaaatacatttgccaccaacagagtagccaaatttat
aaggaaaaatg (Seq ID No: 501)
```

Homo sapiens thioredoxin interacting protein (TXNIP):

```
acccctctttttctccaaaggagtgcttgtggagatcggatctttctccagcaattggggggaaag
aaggcttttctctgaattcgcttagtgtaaccagcggcgtatatttttaggcgccttttcgaaa
acctagtagttaatattcatttgtttaaatcttattttattttaagctcaaactgcttaagaata
ccttaattccttaaagtgaaataattttttgcaaaggggtttcctcgatttggagctttttttttc
ttccaccgtcatttctaactcttaaaccaactcagttccatcatg (Seq ID No: 502)
```

Homo sapiens ecotropic viral integration site 2B (EVI2B):

```
ttttccttttcttagccaaatcaccaaaatgtccagttagaacaagaatttagcattctgcaaaaga
agttaacagctgagataacgaggaaatattctgaaatg (Seq ID No: 503)
```

Homo sapiens guanine nucleotide binding protein (G protein), alpha inhibiting activity polypeptide 3 (GNAI3):

```
ggttcttctgggcgctaagggagctgacggagagggccaccgcccagcaatagacggtgcctcagc
ctgccgagccgcagtttccgtggtgtgagtgagtccgggcccgtgtccctctcccgccgccgcca
tg (Seq ID No: 504)
```

Homo sapiens polymerase (DNA directed), eta (POLH):

```
cggcccttcgcagcgggcgcgctgtcagacctcagtctggcggctgcattgctgggcgcgccgctc
tcgtctgatccctgctggggacggttgcccgggcaggatcctttacgatcccttctcggtttctcc
gtcgtcacaggggaataaatctcgctcgaaactcactggaccgctcctagaaaggcgaaaagatatt
caggagcccttccattttccttccagtaggcaccgaacccagcattttcggcaaccgctgctggca
gttttgccaggtgtttgttaccttgaaaaatg (Seq ID No: 505)
```

Homo sapiens solute carrier family 2 (facilitated glucose transporter), member 1 (SLC2A1):

```
cgctctctggcaagaggcaagaggtagcaacagcgagcgtgccggtcgctagtcgcgggtccccga
gtgagcacgccagggagcaggagaccaaacgacgggggtcggagtcagagtcgcagtgggagtccc
cggaccggagcacgagcctgagcgggagagcgccgctcgcacgcccgtcgccaccgcgtacccgg
cgcagccagagccaccagcgcagcgctgccatg (Seq ID No: 506)
```

Homo sapiens zinc finger protein 138 (ZNF138):

```
gggtctttgtctcgctgcagcgggtgctgcaggtctggccttcacttttctgcgtcctcttactcc
tagaggcccagcctctgtggcgctgtgatctggttattgggagattcacagctaagacgccaggat
cccccggaagcctagaaatg (Seq ID No: 507)
```

Homo sapiens ubiquitin specific peptidase 3 (USP3):

```
ctttctttgacgcaagggctcgagacgcagccgccgtcggccgagcgcccggctagaagcgacacc
agacggagcctccggagttcctccgcccccacctcgccgggtcctggagccgcagtcctcccagct
gccctcctcgtggccatg (Seq ID No: 508)
```

Homo sapiens calcium channel, voltage-dependent, gamma subunit 3 (CACNG3):

```
ctgtcttttctccagtttgagcggggggtgtcgggagcaggcggagagctttcctgcgaggctgtgg
aagcagtgaacactcttctcagcggctcgcctcccagcagtgctattttttgccatccgccctcac
ccccagcacacgcgctcgcacacacacgcacgcacgcacacacacacacacactcacacaga
gacctctctgggtttctttgccttgagtctcccggggctgtgagaagccaggcgcatctcaaaccg
agctggcagctccaggctccggagccatgccctgcacggaccctcgtctttaccacgctcctgagg
aatgaaaggaacccagggaccctcagaaggcagcagtgatgcggaccaacccccggagcctgcac
ccttccgagggccataggcgacccagggaactggagagagctccagaaaggaaatcccagcttttcc
caaagtccctgtggatgctgacaaaaggagacctgaattttttggaagagcctgtactaggttaccc
ggctgcagagtgattttcccctccggcactgactctcccccctccaaccccagccgtccagagtac
catgaagaattatg (Seq ID No: 509)
```

Homo sapiens guanine nucleotide binding protein (G protein), beta 5 (GNB5): ttccctctccgctgcgtccccgcgcgaagatg (Seq ID No: 510)

Homo sapiens chaperonin containing TCP1, subunit 8 (theta) (CCT8): cttcctccgcggtcttccgagcggtcgcgtgaactgcttcctgcaggctggccatg (Seq ID No: 511)

Homo sapiens prostaglandin E synthase 3 (cytosolic) (PTGES3):

```
cgctctttccgcgcggtgcattctggggcccgaggtcgagcccgccgctgccgccgtcgcctgagg
gaagcgagaagaggccgcgaccggagagaaaaagcggagtcgccaccggagagaagtcgactccct
```

```
agcagcagccgccgccagagaggcccgcccaccagttcgcccgtccccctgccccgttcacaatg
(Seq ID No: 512)
```

Homo sapiens zinc finger protein 266 (ZNF266):

```
ttttcttcctggtggcgtttgggcttaatacagctttggcgaggtcggatgacgggtgggagccag
cggtggaaggggtggcgaaagtaccggtttgccccaggccgccgagggggcctccttagagagacct
tgcctgctccgctcgcgtccgccggggccgcgcgggtcctcctggcgccgccaggttcaaaaagcc
actcgagttgtcactgcgacggccctgggccaggagccgtttcgggatctgtcaaacaacgagttt
tcgtcgttcgaatcaggttgactggtccttcatccccccaatctcccgtacctggcgagtccagct
cgtcgcggcaatgctaagaaaagagtgatatgcaagctgagaccaaaaatatggtatgatttagcc
atactgaaggggaaggaaataagagctgggcaaagcattctgtgaattggctgactccacttctat
ggtgagagagaggagtgcatcaaagattactcccagtagagatggtttcagcatgttggccagtct
ggtctcagactcctgacctcaagtgatccacccacctcggcctcccaaaatgctgggattacaggt
ataagccactgtgcctggccaaagataccgttaaccctggataaagagaatggaggttacctctgt
ccgtgtagattcctaagctgtcctggagtgatccttggagtaaaggaaaggtgctttgaagcacat
tcagccatcagccctgtgggatggcagccactgatttgtcctatggtctttacagggacccagtct
gccttcaagaaaagacagaagtagaaagggtggtggctgactgtctgacaaattgttatcaggtat
gcaggaagtatatccttctccaaaatatcatacttgcatcaccaggtagacacatttccttctaca
cagaattatcttcagagcttcttaaagcaaataaagcctgcttcaaggactgagtccctagtcgaa
ttcccggaaggagtggagcctgtcatattgtgtttatctagcatctgctcaagagtgtgctgcagt
ggagggaaatcagatgacctcccagtctggttgtgttacatacaatcatgtgtaagaagtgccatt
caagccgtgtcactggaggggactgacagtgagattcagtgacttttgatgatctggctgtggact
tcaccccagaagaatggactttactggacccaactcagagaaacctctacagagatgtgatg
(Seq ID No: 513)
```

Homo sapiens methylenetetrahydrofolate dehydrogenase (NADP+ dependent) 2, methenyltetrahydrofolate cyclohydrolase (MTHFD2): gcttccctcccggcgcagtcaccggcgcggtctatg (Seq ID No: 514)

Homo sapiens chemokine (C-C motif) receptor 9 (CCR9):

cttcctttctcgtgttgttatcgggtagctgcctgctcagaacccacaaagcctgcccctcatccc
aggcagagagcaacccagctctttccccagacactgagagctggtggtgcctgctgtcccagggag
agttgcatcgccctccacagagcaggcttgcatctgactgacccaccatg
(Seq ID No: 515)

Homo sapiens heat shock 105kDa/110kDa protein 1 (HSPH1):

cctccccttttgggtcggtagttcagcgccggcgccggtgtgcgagccgcggcagagtgaggcagg
caacccgaggtgcggagcgacctgcggaggctgagccccgctttctcccagggtttcttatcagcc
agccgccgctgtccccggggagtaggaggctcctgacaggccgcggctgtctgtgtgtccttctg
agtgtcagaggaacggccagaccccgcgggccggagcagaacgcggccagggcagaaagcggcggc
aggagaagcaggcaggggccggaggacgcagaccgagacccgaggcggaggcggaccgcgagccg
gccatg (Seq ID No: 516)

Homo sapiens StAR-related lipid transfer (START) domain containing 10 (STARD10):

tggtcctttcttttatgattcacaaggaatgaccctcttcatcgcctctcctaattcagtcctcac
aacagtccttttacaaatgggacaacaggttagaggaagtcaggcagatttccagcatcatagaga
gtaaaggaccagggaaggatcaggattcaaggactgcacccaggctctgcttccagcttgctgtgt
gactttgggtaattttgttcccttagggaactgagctttctcatttgtaaatgcaaacaggctgtt
gggaggatcaaatgagatccaggggtgaaaacagcttagtttactttcaggaatttacccacgcgg
tatataaaggcaaaatattattatagtcaggtgattgtagattgaggaacccatttcctcattctg
caaattgcaaacctgagggcccaaagagggacaggggcttgccccaggtctcagcaggctgtgagc
aagagctaaagcctaatcctcctgcctttgggcctggagcccttccttgtaccccagggggtcagtg

tctttgttggatacaggcttagattgactgactgtaccctgagaacctaggggagtccctgttccc
aattcttctcctaccccaccttggcctgatggaggaagaccctgctgtgttgagatgagcaccag
agccaagaagctgaggaggatctggagaattctggaggaagaggagagtgttgctggagctgtaca
gaccctgcttctcaggtcccaggaaggtggcgtcagcatctgcagccgcgtcgacgttgtcggagc
ctccgcggaggacccaggagagccggactaggaccagggccctgggcctccccacactccccatg
(Seq ID No: 517)

Homo sapiens UTP14, U3 small nucleolar ribonucleoprotein, homolog A (yeast) (UTP14A): ctttccttcggcttccgttcttggtccatgtgagagaagctggct-gctgaaatg (Seq ID No: 518)

Homo sapiens SUB1 homolog (S. cerevisiae) (SUB1):

ggttctctgtcagtcgcgagcgaacgaccaagagggtgttcgactgctagagccgagcgaagcgat
g (Seq ID No: 519)

Homo sapiens minichromosome maintenance complex component 5 (MCM5) :

ccgcctcttgttttttcccgcgaaactcggcggctgagcgtggaggttcttgtctcccctggtttgt
gaagtgcggaaaaccagaggcgcagtcatg (Seq ID No: 520)

Homo sapiens RNA binding motif (RNP1, RRM) protein 3 (RBM3):

tactctttatcaatcgtcttccggcgcagccccgtccctgttttttgtgctcctccgagctcgctg
ttcgtccgggttttttacgttttaatttccaggacttgaactgccatg (Seq ID No: 521)

Homo sapiens KDEL (Lys-Asp-Glu-Leu) endoplasmic reticulum protein retention receptor 1 (KDELR1):

```
ctcccccctctcgctctcctccctcttcccggctccagctccgccgccagctccagcctttgctccc
cctcccaaagtcccctcccggagcggagcgcacctagggtccctcttccgtcccccagcccagc
tacccgttcagaccagcagcctcggggggcacccccccgccagcctgcctccctcccgctcagccc
tgccagggttccccagccatg (Seq ID No: 522)
```

Homo sapiens StAR-related lipid transfer (START) domain containing 3 (STARD3):

```
agatcttcttccgctctgaggcgctactgaggccgcggagccggactgcggttggggcgggaagag
ccggggccgtggctgacatggagcagccctgctgctgaggccgcgccctccccgccctgaggtggg
ggcccaccaggatg (Seq ID No: 523)
```

Homo sapiens heterogeneous nuclear ribonucleoprotein A0 (HNRNPA0):

```
cggcctctttgtgtggtgcccagatagggg agcggaggtggcggcggcggcggtagcggtggcctt
ggttgtcttccagtctcctcggctcgccctttagccggcaccgctccccttccctcccccttcctc
tcttccttccttccctcccccttccttttttccttccccgtcggtgagcggcggggggtggctccag
caacggctgggcccaagctgtgtagaggccttaaccaacgataacggcggcgacggcgaaacctcg
gagctcgcagggcgggggcaaggcccgggccttggagatg (Seq ID No: 524)
```

Homo sapiens chromobox homolog 1 (CBX1):

```
ggctcttttgttcggctgaggggagggccgttggccggggcctgcggtacgccgcttcagtgaggg
acgccactgcggccacccggcttgctgccttcctgggcgccactcccccaggcgacccgacgcgac
gcgccagcagcgcagcaccgattcctctcgggctcttgggcgctgctctgaggtgaggagcccgct
ggaggcgggagagctggggggaggggggcgcggcggcggcggcggcgggagccctgcgtgagggaacg
cgctttcgaggcggaggttaggagcggggagcgcgcccgggtccagcgtcctgcttctccgcttcc
cgcgctgagctcttcgcctgtcgctgaggcgtcggtgccagctgcgtgaaggatggagagggcggg
gcgcgaatcctgagccagagactgagtgcttggggtgggccgagcacttgggggccgctcttcgg
ggcccgggtggtctggaacaatgttgcttggctgggcggctgcgggatagggcggaaggggacagg
```

```
cttgaggcttggataggcgtgaggaggcgcatacgaccgcacaacccgaggtttgtaactgtattc
ggaagacgccgggtccggctgggactgccagaggaacctggctttgcaggactacggaggagtaac
gtcgagtgaattggaagagggcccagggccgcacaagcagcgtcaccctttacaccagaaagctgg
cgggcactatg (Seq ID No: 525)
```

Homo sapiens myeloid/lymphoid or mixed-lineage leukemia (trithorax homolog, Drosophila); translocated to, 11 (MLLT11):

```
cgcccttcttaggaggggctgcattgcaggggg agagtgaactgacagactcagtcactgaagagg
gaaaaggagtgagaagacaaagccgtcaaagccccaacagctttgtatttctccagcccggcgcag
accccggagctcccgaggcactccctccatctttggaacacgccagtaattgattgataacaggaa
gctatg (Seq ID No: 526)
```

Homo sapiens interferon-induced protein 44-like (IFI44L):

```
ttttctttctttcctagagtctctgaagccacagatctcttaagaactttctgtctccaaaccgtg
gctgctcgataaatcagacagaacagttaatcctcaatttaagcctgatctaacccctagaaacag
atatagaacaatg (Seq ID No: 527)
```

Homo sapiens cyclin I (CCNI):

acttcttcctcccttcccctctcttcccctccctccccagccttccccgcgagcggacgcggcagc
gcctctgtctcgcttttttcttattttttccccctttcccctttctttttttttttttctttttcttt
tctccctccccccctttcaccatttcccctcggaggcgctttccccgggcaggggcagagccggt
ctcaccccccgcctctccccggccccgccgccctatggcgagagggagcccctcccaacccggg
ctcgagcggcggcggcctcaggccgggggtcatcatggaactaattcgctgaccgacccagcggcc
gcagccgtgcgtcccgctcgagcgccagcgcccgcgcccgcgccccccgatccgcttcccctttct
ccctcctcagttggccgagtcgtcccgcgcgcaccgcctccgcgcgcctatgagaatgaggtggta
acgggcccccggatgaccccgcgtcaccactgtgaggcctacagctctgccggggaggaggaggag
gaggaagaggaggagaaggtagctacagcaagctgggtagcaggcagatccaaaggatatcatg
(Seq ID No: 528)

Homo sapiens methionyl aminopeptidase 2 (METAP2): cattccctcgcgctctctcgggcaacatg (Seq ID No: 529)

Homo sapiens leukocyte immunoglobulin-like receptor, subfamily B (with TM and ITIM domains), member 4 (LILRB4):

gtctctttgtcctgccggcactgaggactcatccatctgcacagctggggcccctgggaggagacg
ccatg (Seq ID No: 530)

Homo sapiens destrin (actin depolymerizing factor) (DSTN):

gggtctctcggtcccgcagccgtgaggaggacggtctgcatactcgctgcccgccggctccctccc
ccgcgtccctgcgaccgccgcggcgaagatg (Seq ID No: 531)

Homo sapiens eukaryotic translation initiation factor 2D (EIF2D):

gggcccttttcgcggccgggcccccagcatggctgcccccacggctgagggcctggcagctgctgcg
ccctcgctttcttgacattccctggcttctgtgctctcttccccaggccaccccagcagacatg
(Seq ID No: 532)

Homo sapiens histamine N-methyltransferase (HNMT): ctgtctttctca-gaaaaccaaatatg (Seq ID No: 533)

Homo sapiens ras-related C3 botulinum toxin substrate 1 (rho family, small GTP binding protein Rac1) (RAC1):

gtttctctgcagttttcctcagctttgggtggtggccgctgccgggcatcggcttccagtccgcgg
agggcgaggcggcgtggacagcggccccggcacccagcgccccgccgcccgcaagccgcgcgccccg
tccgccgcgccccgagcccgccgcttcctatctcagcgccctgccgccgccgccgcggcccagcga

gcggccctgatgcaggccatcaagtgtgtggtggtgggagacggaaacaagaatctcagtgtaacc
cgagcaaaatcgcgcgtctcagcgttgcttgtatagagctgtaggtaaaacttgcctactgatcag
ttacacaaccaatgcatttcctggagaatatatccctactgtctttgacaattattctgccaatgt
tatg (Seq ID No: 534)

Homo sapiens signal recognition particle 72kDa (SRP72): tcgtctcctccaagatg (Seq ID No: 535)

Homo sapiens zinc finger protein 33B (ZNF33B):

ccgcctttcctttttgtttgtctcacgtttttgcgtgggaggcggtcccgggatttcaggggtctacc
ggctctcttatggcgaatgcaacccgaagagagagtgagctgtatcttcagagttgtctccgtctt
tccaagaacagaacaaaatg (Seq ID No: 536)

Homo sapiens zinc finger protein 16 (ZNF16): gcctcctttccaagcgcgacccgttgaggtccttgtcatg (Seq ID No: 537)

Homo sapiens zinc finger protein 33A (ZNF33A):

```
ccgcctttccttttgttttttctcaggttttgcgtgggaggcggtcccgggatttcaagggtctacg
cgcttttctatggcgaatgcaacccgacgagggagtgggctgtatcttcagagttgtctccgtctt
tccaagaacagaacaaaatg (Seq ID No: 538)
```

Homo sapiens butyrophilin, subfamily 3, member A3 (BTN3A3):

```
ctttctttttcctttcttcggaatgagagactcaaccataatagaaagaatggagaactattaacc
accattcttcagtgggctgtgattttcagaggggaatactaagaaatggttttccatactggaacc
caaaggtaaagacactcaaggacagacattttggcagagctgctcactccttgctcagctcagtt
ttctgtgcttggaccctctgggcccatcctggccatg (Seq ID No: 539)
```

Homo sapiens butyrophilin, subfamily 2, member A2 (BTN2A2):

```
ctctttgggatgctttgttgtctggtggtgactgtgcccatgggtgagttgtatcggaaaatcgtc
atgtgaggatcagaggggaaaagaaaacagaggcctctggtctctgcctgccctgggtgctcatg
(Seq ID No: 540)
```

Homo sapiens nudix (nucleoside diphosphate linked moiety X)-type motif 21 (NUDT21):

```
acgcctcctcttgcgctgtcctgttaatggcgggcagtagccgctgaggggattgcagataaccgc
ttcccgcacggggaaagtctaccctgcctgccactttctgctcgccgtcagcgccggagctcgcca
gcatg (Seq ID No: 541)
```

Homo sapiens stathmin-like 2 (STMN2):

```
tgctctttctctagcacggtcccactctgcagactcagtgccttattcagtcttctctctcgctct
ctccgctgctgtagccggaccctttgccttcgccactgctcagcgtctgcacatccctacaatg
(Seq ID No: 542)
```

Homo sapiens katanin p60 (ATPase containing) subunit A 1 (KATNA1):

```
caccctcttccgccgctcccgcccagcgacctcgctcccggggcgacgccccgcgtgcgccagagt
cgccgaggtcgtccccggcaccggaagtgaccctggcgggtttgtcttcaaattctcggcgagcag
gagccgcgccggcaggtggtgttgacgattgaactgggcagtactggggccgtgagcggagagcaa
agtgggctggactgggtcaggccctccttcctcgctgccgggatctccactccgccaatcccctgt
gcctggcgttgggcggtttcccgaggagcttgggccgccgcagcttacagttgaacatg
(Seq ID No: 543)
```

Homo sapiens butyrophilin, subfamily 3, member A2 (BTN3A2):

```
ctttctctttttcctttcttccggatgagaggctaagccataatagaaagaatggagaattattga
ttgaccgtctttattctgtgggctctgattctccaatgggaataccaagggatggttttccatact


      ggaacccaaaggtaaagacactcaaggacagacattttggcagagcatagatg
      (Seq ID No: 544)
```

Homo sapiens CLK4-associating serine/arginine rich protein (CLASRP):

cggccttttcatttccgcttccggtgcgggccgcgcgcgagcgcagcggtgggaggcggcgaccagc
cggttgaggccccaggcttggcctcaccacaatg (Seq ID No: 545)

Homo sapiens clathrin, light chain A (CLTA):

ctccctcctggcgcttgtcctcctctcccagtcggcaccacagcggtggctgccgggcgtggtgtc
ggtgggtcggttggttttttgtctcaccgttggtgtccgtgccgttcagttgcccgccatg
(Seq ID No: 546)

Homo sapiens NADH dehydrogenase (ubiquinone) flavoprotein 1, 51kDa (NDUFV1):

gcgtctctatcgcgccagttcctcagcctcagtgctatgaaggtgacagcgtgaggtgacccatct
ggcccgccgcgatg (Seq ID No: 547)

Homo sapiens signal sequence receptor, gamma (translocon-associated protein gamma) (SSR3):
gggcctttgcccgccttggcggccggctctacgttccctgttctcgcctgcagctccgccatg (Seq ID No: 548)

Homo sapiens valosin containing protein (VCP):

gcttcccttccgatgattcggctcttctcggctcagtctcagcgaagcgtctgcgaccgtcgtttg
agtcgtcgctgccgctgccgctgccactgccactgccacctcgcggatcaggagccagcgttgttc
gcccgacgcctcgctgccggtgggaggaagcgagagggaagccgcttgcgggtttgtcgccgctgc
tcgcccaccgcctggaagagccgagccccggcccagtcggtcgcttgccaccgctcgtagccgtta
cccgcgggccgccacagccgccggccgggagaggcgcgcgccatg (Seq ID No: 549)

Homo sapiens zinc finger protein 195 (ZNF195):

gggcctttgtcccgacagagctccacttcctgtccccgcggctctgtgtcccctgctagccgtagg
tcgtgtgacccgcaggcaccgggagatccagaagtgaaacgccaggctctctggaggccaggagat
g (Seq ID No: 550)

Homo sapiens testis-specific kinase 2 (TESK2):

cagtctttcgcggcccgggagctcagcagagctaccagctgccctgttggcttcgctggtcggatc
gtcctcctggccccgccaaacaggcgggggggagcggccccgactgtggggccatggcagtagtctc
ctcgttcgccgccgccgctagcctagctgagtcgccggcttctgcgctaggggctcccaccgcctc
cgcaggctaaggagccgctgccaccaacgagctgtgagggttactatgctccctcttttgccgccgt
ctcctcctcttgcccgcgcaggcacccctctggctgctcagtcctgcctcagtgtcaaaccagaag
agaagtaaaattcaacaaaaatttatgtgtggagttccttcttaaaagaagaaaaagtgattatt
tagactatg (Seq ID No: 551)

Homo sapiens family with sequence similarity 107, member A (FAM107A):

agccctccttgctagtctgggacttcccggtggagtgaggaacccagcaacacgctcctgacttcc
cttcccaaggactcgacctgagaaggacacagcagtctctgaatttcatgctctcctctttgatgt
gaagaaaatgaaaagctgaacagttgtggaactgtggatagagttagacaataaggccgccatg
(Seq ID No: 552)

Homo sapiens serine/threonine kinase receptor associated protein (STRAP):

```
ccctccctccctttccctccctcgtcgactgttgcttgctggtcgcagactccctgacccctccct
```

```
cacccctccctaacctcggtgccaccggattgcccttcttttcctgttgcccagcccagccctagt
gtcagggcggggggcctggagcagcccgaggcactgcagcagaagagagaaaagacaacgacgaccc
tcagctcgccagtccggtcgctggcttcgccgccgccatg (Seq ID No: 553)
```

Homo sapiens mitochondrial ribosomal protein L3 (MRPL3):

```
ctttctttccgtcgcagagagcatcggccggcgaccgttccggcggccattgcgaaaacttcccca
cggctactgcgtccacgtggcggtggcgtggggactccctgaaagcagagcggcagggcgcccgga
agtcgtgagtcgagtcttcccgggctaatccatg (Seq ID No: 554)
```

Homo sapiens zinc fingers and homeoboxes 1 (ZHX1):

```
ctcccttcccccctccgcccccggacggccgctggggcgcgcgcctctcctcgcacccccaccctga
gtccccacactccgcggggccaccgagctgctgaggcccctttgcgggcccgccgagcggttccgg
gtttagggttcacaggtcagagttgactccctgaaaagtgcagccggtttgaaatgcaagatggcg
gcggcgtggcgctgagaggcgcggcggcccctgcaggagaagacagactgctgctttggacctgtt
ggtaatgatggcctgagctaaacatctaactagaagggatacccttccatttcaaagaacagaatg
ctaaggaagctgtggcaagtgattggagttgtgcttcaaaaatttcagaaattcagcagtatttta
tctgccaacaataagctctttacttgattgcaccatgagaaagctgctaatgagacttgttgagca
caaaaatggacttgaagaaccaaaagccattgttttcaaatgaagaacactgaacagttttaagcc
tcgatgcttttaatcaccactgagcttttcctcataacatcagaatg (Seq ID No: 555)
```

Homo sapiens calcium binding protein P22 (CHP):

```
ccttccttccctccctccttccctcctgtcgccgtctcttctggcgccgctgctcccggaggagct
cccggcacggcgatg (Seq ID No: 556)
```

Homo sapiens ecdysoneless homolog (Drosophila) (ECD):

```
ctttctctcaggatttccgctggcttcaggttccggtcaggcgtcgggacagagcctgatccaggc
ttcggcggccggtggcagctctcgatcagctctcgcagtcggagaggcggctaaggaaaggtgcca
cagcagagacgcgaaggagaggccctagaaccttttcaaagaagaatg (Seq ID No: 557)
```

Homo sapiens V-set and immunoglobulin domain containing 4 (VSIG4): gagcctctttggtagcaggaggctggaagaaaggacagaagtagctctggctgtgatg (Seq ID No: 558)

Homo sapiens prohibitin 2 (PHB2):

```
tgccctttctttcgccagccttacgggcccgaaccctcgtgtgaagggtgcagtacctaagccgga
gcggggtagaggcgggccggcacccccttctgacctccagtgccgccggcctcaagatcagacatg
(Seq ID No: 559)
```

Homo sapiens signal transducer and activator of transcription 1, 9 1kDa (STAT1):

```
ctgccttttctcctgccgggtagtttcgctttcctgcgcagagtctgcggaggggctcggctgcac
cggggggatcgcgcctggcagaccccagaccgagcagaggcgacccagcgcgctcgggagaggctg
caccgccgcgcccccgcctagcccttccggatcctgcgcgcagaaaagtttcatttgctgtatgcc
atcctcgagagctgtctaggttaacgttcgcactctgtgtatataacctcgacagtcttggcacct
aacgtgctgtgcgtagctgctcctttggttgaatccccaggcccttgttggggcacaaggtggcag
gatg (Seq ID No: 560)
```

Homo sapiens heat shock protein 90kDa alpha (cytosolic), class B member 1 (HSP90AB1):

```
agctctctcgagtcactccggcgcagtgttgggactgtctgggtatcggaaagcaagcctacgttg
ctcactattacgtataatccttttcttttcaagatg (Seq ID No: 561)
```

Homo sapiens cancer susceptibility candidate 3 (CASC3): cgttctccgtaagatg (Seq ID No: 562)

Homo sapiens nuclear cap binding protein subunit 2, 20kDa (NCBP2): gcttctctgcactatg (Seq ID No: 563)

Homo sapiens non-POU domain containing, octamer-binding (NONO):

```
cgctcttttctcgggacgggagaggccgtgtagcgtcgccgttactccgaggagataccagtcggt
agaggagaagtcgaggttagagggaactgggaggcactttgctgtctgcaatcgaagttgagggtg
caaaaatg (Seq ID No: 564)
```

Homo sapiens lectin, galactoside-binding, soluble, 9 (LGALS9):

```
atttctttgttaagtcgttccctctacaaaggacttcctagtgggtgtgaaaggcagcggtggcca
cagaggcggcggagagatg (Seq ID No: 565)
```

Homo sapiens chaperonin containing TCP1, subunit 5 (epsilon) (CCT5): cggtctccgccggttggggggaagtaattccggttgttgcaccatg (Seq ID No: 566)

Homo sapiens haloacid dehalogenase-like hydrolase domain containin g 1 (HDHD1): cttcctcctcgcccccacccagacccagaaggcgccaccatg (Seq ID No: 567)

Homo sapiens glutamate dehydrogenase 2 (GLUD2):

```
cttccttcctagtcgcggggagtctgagaaagcgcacctgttccgcgaccgtcacgcacccctcct
ccgcctgccgcgatg (Seq ID No: 568)
```

Homo sapiens general transcription factor IIIC, polypeptide 3, 102 kDa (GTF3C3): ggttctctgtcccggttcctggggttgcacagacagaccctgtaaacatg (Seq ID No: 569)

Homo sapiens general transcription factor IIIC, polypeptide 5, 63k Da (GTF3C5):

```
gggtccctcgctggctagtaggagagactggtgcttgccccgcccggtggactaactcgcttaatt
ttaaataaaaagtcgaggacacggcggtcgttttcccgaagacatgggccctcccatgggccattt
gctccctggaggccctcgcgtcttgctgagcccggggagttaggatgacgcgagcggtgagggagc
ccggaacgattccttcgcggaacaattgaggcgaggcctttgggagtactttgtgggacggaccct
ggcgggccctgccagacgcacagggatg (Seq ID No: 570)
```

Homo sapiens ancient ubiquitous protein 1 (AUP1):

```
ccgccttcccaagagcccctgcggccgggcgcgaaaatggcggcggcggcgacggccgggcgctcc
tgaagcagcagttatg (Seq ID No: 571)
```

Homo sapiens coatomer protein complex, subunit gamma 2 (COPG2):

```
cggccttcctgcagcctcttccgctcgccggctgcggcgcctgggacggttgcggtgggtctgggc
gctgggaagtcgtccaagatg (Seq ID No: 572)
```

Homo sapiens apoptosis antagonizing transcription factor (AATF):

```
cggtctctggcggagtcggggaatcggatcaaggcgagaggatccggcagggaaggagcttcggggg
ccggggggttgggccgcacatttacgtgcgcgaagcggagtggaccgggagctggtgacgatg
(Seq ID No: 573)
```

Homo sapiens integrator complex subunit 6 (INTS6): tctcctctttctccaccacctcgggccccggtgtccccggccagcactatg (Seq ID No: 574)

Homo sapiens F-box and leucine-rich repeat protein 4 (FBXL4):

```
tcttccttccgggtcgcgctaggccgggcttgcggcggttgtgccgcatctagagagtcggggagc
```

```
cgcccccgcacccaggccttctcgcgctgcctggtcgctggtgaagcccgcggcgcgcgcctctcc
cggaccctgcagggtaaaagaatgtcacatgtcagcatttgtacctgaagtcagcatgcaaagttc
agggtacctggatgaatgccaacttttgcatttcccatgtgtatcctgtgaccattctatctggga
acatccttcaaagagttcatgcatcttactgaggacacctgacctttgaagcttcataattcaca
tctagatg (Seq ID No: 575)
```

Homo sapiens guanine nucleotide binding protein (G protein), gamma 3 (GNG3):

```
gctccttctagcatccttcatccttcaggtaccagccatccagacagtgcttgagctgcagaaact
gagaccagacctctggcctggccctccccaggggcctcctttcgtatagtcactgcttctgcatca
gatactttcagctgcaactccctactgggtggggcacccatttcaggcagaaggttttggtaccct
ccactgaccctacacccagggctgctactgccgcttgtggcttcaggatg
(Seq ID No: 576)
```

Homo sapiens histidyl-tRNA synthetase 2, mitochondrial (putative) (HARS2): aggccttttgttcctgtcccggaaagccggcgtcctgccgcgcgatg (Seq ID No: 577)

Homo sapiens interleukin enhancer binding factor 3, 90kDa (ILF3):

```
cctcctcctcctcttctcgccattgcagttggacccagcagcccggcgcgcaccgcgtggcttttg
ggggcagaccccggcgggctgtggcaggagggcggcggcggcggctgcggtcgaagaaggggacgc
cgacaagagttgaagtattgataacaccaaggaactctatcacaatttgaaaagataagcaaaagt
ttgatttccagacactacagaagaagtaaaaatg (Seq ID No: 578)
```

Homo sapiens polymerase I and transcript release factor (PTRF):

```
gtttcctctgctctccgctctcgcccgctagctctcctcccttccgctcctgcttctctccgggtc
tcccgctccagctccagccccacccggccggtcccgcacggctccgggtagccatg
(Seq ID No: 579)
```

Homo sapiens 5'-3' exoribonuclease 2 (XRN2):

```
tgccctctgccgctgctcccgtctctttggttacgctcgtcagccggtcggccgccgcctccagcc
gtgtgccgctatg (Seq ID No: 580)
```

Homo sapiens 2-hydroxyacyl-CoA lyase 1 (HACL1):

```
ccgcctcttccttcccgttgtttaaggcagttggttgccctcctgtccgtcagaggtgcagtacca
gaggtggcgtgctgccgatttcgcgtttgccttgctggatgattccgcttgtttgccggctgcgtg
agtgcttagagcttttcggtggaagatg (Seq ID No: 581)
```

Homo sapiens zinc finger protein 346 (ZNF346): ggctctctaccggtgagggtttgcggggaagatg (Seq ID No: 582)

Homo sapiens microtubule-associated protein, RP/EB family, member 3 (MAPRE3):

```
cagtctctgtgcgttgaagccggagaccgcggcggcctcagcgaggaccctccgccccggagccgc
cggccggagccgcagcctctgccgcagcgcccccgccacctgtcccctcccccctccgcctccgccg
gagccgcctcgtgcactctggggtatg (Seq ID No: 583)
```

Homo sapiens splicing factor 3b, subunit 3, 130kDa (SF3B3):

```
gtgcctttttccgccgcgcgccaccagaatgtccctgtcttgaggtctaatggcggacgccagtat
gttggagttggtggtggcttaagtttttgaagggaggtagcatccgttggatatccacaccatcctt
ctcgctgcaggctttcttggactccgtactgttggtgtaaccaaggcctggaggtctgggtggctc
aggtttcctgcagccatg (Seq ID No: 584)
```

Homo sapiens spondin 2, extracellular matrix protein (SPON2):

```
ctgcctctcgctggaggccaggccgtgcagcatcgaagacaggaggaactggagcctcattggccg
gcccgggggcgccggcctcgggcttaaataggagctccgggctctggctgggacccgaccgctgccg
gccgcgctcccgctgctcctgccgggtgatg (Seq ID No: 585)
```

Homo sapiens solute carrier family 13 (sodium/sulfate symporters), member 4 (SLC13A4):

```
ttttcttttctgctttgcaggcccaggctcaaggcaaattataagtagggaaccaatttgagggaa
agacatgtgaacagagttaaggtaccacgtcctgggagcgaccagcagccccacctgaagtccgca
tgcaactctgacaagctcaggtgcttgtttttaaggaaaggggctactagagtcttaccaacagcga
gcccaggtgggagatgaaacaggtactccccaaaataggtcatccgagggaggaaaactgatggag
agcacaatgtgctctgagcgttttttaatgttttttaagcttttaaatgatttcttcaaggccgagca
gcagcagcaaaggtgtggcttaaaggattaaggggggtttctgctgacacctagaatgaagttactc
tattactaatcaagccgagaggaggcccactatgccccgtttatcatcctttcccagttccttttt
tgctggtcacaaaacgatgctcatcaatcccacctaaagcaggaggccaggagcccagcctcttgt
agaaacagcgagggtataactgccctcccgttctgcccccaagacgaaggaggactctcggaagcc
aagaaaggtttaagaagtctttctggatagagagcagtgcccaggcaggaagcctttcgccggcag
agcggggtccaaggacgagctggagaggacagaggcgcgatg (Seq ID No: 586)
```

Homo sapiens PRP6 pre-mRNA processing factor 6 homolog (S. cerevisiae) (PRPF6): attcctttccttcctagccttggtcgtcgccgccaccatg (Seq ID No: 587)

Homo sapiens eukaryotic translation initiation factor 3, subunit K (EIF3K):

ccacctcttcctgttcccgtccttgaggacgccgtgccgggtcagtgttagcctccagccctggtt
gtggaaggcgacagaagtcatg (Seq ID No: 588)

Homo sapiens ataxin 10 (ATXN10):

cccctccccgcggcgccgtctcctcctcccgcctgaggcgagtctgggctcagcctagagctct
ccggcggcggcgcagcttcagggcagcgcgggctgcagcggcggcggcggttagggctgtgtaggg
cgaggcctcccccttcctcctcgccatcctactcctccctcctcgtcatcctcccccttcgtcctc
ctcgccttcctcctcctcgtcaggctcgacccagctgtgagcggcaagatg
(Seq ID No: 589)

Homo sapiens secretogranin III (SCG3):

cttccttcctcacttcctctgcaggagggagcgagagtaaagctacgccctggcgcgcagtctccg
cgtcacaggaacttcagcacccacagggcggacagcgctcccctctacctggagacttgactcccg
cgcgccccaaccctgcttatcccttgaccgtcgagtgtcagagatcctgcagccgcccagtcccgg
cccctctcccgccccacacccaccctcctggctcttcctgttttttactcctccttttcattcataa
caaaagctacagctccaggagcccagcgccgggctgtgacccaagccgagcgtggaagaatg
(Seq ID No: 590)

Homo sapiens polymerase (DNA directed), mu (POLM):

cttccttccgtctcgctcggagtttccctctgcgttcgctccgcgctgctggaggctgtcgtccca
atg (Seq ID No: 591)

Homo sapiens epsin 1 (EPN1):

cctccttctgttgcttcccgtctcctcggcggctcccctcccccgcccggctctccgcgcccccttc
tgggcggcggggcggcggagccgtcggcgtgcggccctccttgcgttcgtgcgtgcgcccgtggcc
cggcgcacgtcccgcgacaccgaggccgagcggggcagggggctgaccgccatgaccccccagagc
ccggcgtgaggggggccgagatgcggtgacctgccagcacctgccgcagccttcgtccgggagtcgc
cccatctctccacgcatcggggccctgtgccccttgctgctgcagccgggcaccatg
(Seq ID No: 592)

Homo sapiens Sec61 alpha 1 subunit (S. cerevisiae) (SEC61A1):

gtgtctctcggcggagctgctgtgcagtggaacgcgctgggccgcgggcagcgtcgcctcacgcgg
agcagagctgagctgaagcgggacccggagcccgagcagccgccgccatg
(Seq ID No: 593)

Homo sapiens Obg-like ATPase 1 (OLA1):

cgttctctcctccttcctcccccgcctccagctgccggcaggacctttctctcgctgccgctgggac
cccgtgtcatcgcccaggccgagcacgatg (Seq ID No: 594)

Homo sapiens sorting nexin 12 (SNX12): aggcctctgtcccccaccccctttcccccggtcccaggctctccttcggaaagatg (Seq ID No: 595)

Homo sapiens LAG1 longevity assurance homolog 2 (S. cerevisiae) (LASS2):

```
cggcctttttttcccggctgggctcgggctcagctcgactgggctcggcgggcggcggcggcggcg
ccggcggctggcggaggaggggagggcgaggcgggcgcgggccggcgggcgggcggaagagggagg
agaggcgcggggagccaggcctcggggcctcggagcaaccacccgagcagacggagtacacggagc
agcggccccggccccgccaacgctgccgccggctactccctcttgatgccctcccctttgcccctc
actcaggatg (Seq ID No: 596)
```

Homo sapiens cytohesin 4 (CYTH4): tcatcttttccccagaggcgtcggaatg (Seq ID No: 597)

Homo sapiens transportin 2 (TNPO2):

```
aattctctctctttggctccctccttccgcgcgagtctctggagaagccgcagcgcgagttgccgc
cgctgctgcccggggccgggtaagtgggcctcactcagagcccgaccctcttggccccggcttgcg
tcgaccccgccgggcaccgagcctgcgccgcgcgcggcccgggcgtcggggccgcgcccgaccgg
gaaaggccgggaagccggttgggcccgatcctcctggcagctagaacgggccgggcgggggagggg
ggaaccgagcagagcttaggggtgggcctcggagccaggccatgtcggggctcctcaagaagag
ggccagtgggactgctggggtcgggctggagggggatctgattgggggaagcgtctggggactgctt
ggggcctgattggggacgtcgcgaggatcggcttgccttgcgccatg (Seq ID No: 598)
```

Homo sapiens makorin ring finger protein 1 (MKRN1): gggcctttgctgtgtgggataaacagtaatg (Seq ID No: 599)

Homo sapiens vinculin (VCL):

```
ctgtctcttcgccggttcccggccccgtggatcctacttctctgtcgcccgcggttcgccgccccg
ctcgccgccgcgatg (Seq ID No: 600)
```

Homo sapiens DEAH (Asp-Glu-Ala-His) box polypeptide 38 (DHX38):

```
cctccttttcctgcccccagactagaggcgggatgtagtctcttaggctaagagtgattggtcaca
aggagactcggaagtgtctgatcagagccccagaggaggccttgagagcctgttggcgtaccgttc
cacacttggatccaggaatcgggcgtgttccaggctgctctctatggtagctttgggcggatagag
ggggcgcgcaaagtattaagggacaataatggccgctttcaaggtgtggattttggctccttgagc
ctgtctgagcgaggggtggcagcgccggcgccccagaatccgggacagaagggtcccaagagtcgc
gcttggtgagagaaatcccagatcctgtgatg (Seq ID No: 601)
```

Homo sapiens osteoglycin (OGN):

```
catcctctaagctttaaatattgcttcgatggtctgaatttttatttccagggaaaaagagagtt
ttgtcccacagtcagcaggccactagtttattaacttccagtcaccttgattttgctaaaatg
(Seq ID No: 602)
```

Homo sapiens NIN1/RPN12 binding protein 1 homolog (S. cerevisiae) (NOB1): gctcccctctcacgcagccaacatg (Seq ID No: 603)

Homo sapiens nudix (nucleoside diphosphate linked moiety X)-type motif 5 (NUDT5):

```
catccttttagcaccgcgagaggcgccggtgtttcgagccgtggcaccggcatcggctgacactgc
tgcctccagctagttatttcgtcctcttccgttcttcacccctacaccttggaggtgaacttctca
cctgagggctgtaaagactcgtttgaaaatg (Seq ID No: 604)
```

Homo sapiens WD repeat domain 91 (WDR91): cgtccctcaccgcac-cacccctaaagacgctagcgctgcgatg (Seq ID No: 605)

Homo sapiens nuclear transcription factor Y, gamma (NFYC):

gggcctctgcattgcccgactccgtaggagcgcggggggcggctcctgctcttcctggactcctgag
cagagttgtcgagatg (Seq ID No: 606)

Homo sapiens protein phosphatase 2, regulatory subunit A, alpha (PPP2R1A):

ccgcccttccttcttctcccagcattgccccccccacgtttcagcacagcgctggccgcagtctga
caggaaagggacggagccaagatg (Seq ID No: 607)

Homo sapiens vesicle-associated membrane protein 2 (synapto-brevin 2) (VAMP2):

ccatctttccgtcccgggcagccagcgccagtcggagccagcgcgagccgccgccgccatcactgc
cgctgccaagtcctccacccgctgccccgccatg (Seq ID No: 608)

Homo sapiens transmembrane protein 5 (TMEM5): gattctctttccgcccgctccatggcggtggatgcctgactggaagcccgagtgggatg
(Seq ID No: 609)

Homo sapiens UDP-GlcNAc:betaGal beta-1,3-N-acetylglucosaminyltrans ferase 3 (B3GNT3):

aactctttcttcggctcgcgagctgagaggagcaggtagaggggcagaggcgggactgtcgtctgg
gggagccgcccaggaggctcctcaggccgaccccagaccctggctggccaggatg
(Seq ID No: 610)

Homo sapiens SEC11 homolog A (S. cerevisiae) (SEC11A): gcgccctttcccctgccggtgtcctgctcgccgtccccgccatg (Seq
ID No: 611)

Homo sapiens RUN and SH3 domain containing 1 (RUSC1):

ctccctccccgcgccccgtcctctcccgccctacaggccctagcagggcaggcgggaggtgagcgc
ggccatcccgctcccggagttccgggatcctggagtccgtagttcgtggtccttcgccggtgtccc
cggagcccagcggctgtggatg (Seq ID No: 612)

Homo sapiens aryl hydrocarbon receptor interacting protein-like 1 (AIPL1): cctccctttctcctgcagccatg (Seq ID No: 613)

Homo sapiens tumor necrosis factor, alpha-induced protein 8 (TNFAIP8):

cctccttttctcccgccggctctaacccgcgcttggctaaggtccgcgggaacccgtgagccaccg
agagagcagagaactcggcgccgccaaacagcccagctcgcgcttcagcgtcccggcgccgtcgcg
ccactcctccgatg (Seq ID No: 614)

Homo sapiens staphylococcal nuclease and tudor domain containing 1 (SND1):

gcgtctctttcgctccgtgtcccgctgctgctcctgtgagcgcccggcgagtccgtcccgtccacc
gtccgcagctggtagccagcctgcccctcgcctcgactccctttcaccaacaccgacacccacatt
gacacctccagtccggccagccgctccactcgttgcctttgcatctccacacatg
(Seq ID No: 615)

Homo sapiens DNA segment on chromosome 4 (unique) 234 expressed sequence (D4S234E):

cgccctcttttggtcgcccctccccaacccagcactaaggagcaccctgctctggtctccgccac
cacccagcgcctcctggacccatcccccaaacccttgaacgtcctcaggaccccaggtgagcgc
ggcgcgctgcgggcggggaccctctctgcacctccccgcacccctgggggtcgctctgtccctacg
gtccccgcctccccttctcctttctaagcgcctcgcgcccaggccgccgcccgggggtggcgcagc
ccgcagccctcccgctccgggcgccctccgccgctccgagacccctgggggcgcgtcctctcccg
ctccctgttccctcccccggctcagggcgggcgcgtggtcccaggggaggctcccgcccagcccc
gcactcctttgtgcggccgggcgggcgctgcgtcaaggtggaggcgcggccacacgcgcgcaccca
cccgcgcgcacccagcccccgggagaggcaggaagggaggcggcggcgcgaggaggagggagcggc
cgtggagcccaatcgttcgctccccttcccgggtccgcgcgcggcgccgcctcgccattgctgcg
agcaggagcaggagacgcggagctcggagcgctcagctgacctgccggagccgggcgtgggctgca
gcctcggagctcccggaacgatg (Seq ID No: 616)

Homo sapiens growth hormone inducible transmembrane protein (GHITM):

acgtcctttcgatgttgcgtcatgcagtgcgccggaggaactgtgctctttgaggccgacgctagg
ggcccggaagggaaactgcgaggcgaaggtgaccggggaccgagcatttcagatctgctcggtaga
cctggtgcaccaccaccatg (Seq ID No: 617)

Homo sapiens stress-associated endoplasmic reticulum protein 1 (SERP1):

tttccttcctctttcactccgcgctcacggcggcggccaaagcggcggcgacggcggcgcgagaac
gacccggcggccagttctcttcctcctgcgcacctgccccgctcggtcagtcagtcggcggccggc
gcccggcttgtgctcagacctcgcgcttgcggcgcccaggcccagcggccgtagctagcgtctggc
ctgagaacctcggcgctccggcggcgcgggcaccacgagccgagcctcgcagcggctccagaggag
gcaggcgagtgagcgagtccgaggggtggccggggcaggtggtggcgccgcgaagatg
(Seq ID No: 618)

Homo sapiens ADP-ribosylation factor interacting protein 1 (AR-FIP1):

cggtctcctcacttccggcttcgctgctcttggttctggttctggaggctgggttgagaggtcgcc
ggtccgactgtcctcggcggttggtcagtgtgaatttgtgacagctgcagttgctccccgcccccg
agcagccgaggagtctaccatg (Seq ID No: 619)

Homo sapiens tumor necrosis factor receptor superfamily, member 21 (TNFRSF21):

ccgccccttcggcgccaccacgtgtgtccctgcgcccggtggccaccgactcagtccctcgccgac
cagtctgggcagcggaggagggtggttggcagtggctggaagcttcgctatgggaagttgttcctt
tgctctctcgcgcccagtcctcctccctggttctcctcagccgctgtcggaggagagcacccggag
acgcgggctgcagtcgcggcggcttctccccgcctgggcggccgcgccgctgggcaggtgctgagc
gcccctagagcctcccttgccgcctccctcctctgcccggccgcagcagtgcacatggggtgttgg
aggtagatgggctcccggcccgggaggcggcggtggatgcggcgctgggcagaagcagccgccgat
tccagctgccccgcgcgcccccgggcgcccctgcgagtccccggttcagccatg
(Seq ID No: 620)

Homo sapiens sushi-repeat containing protein, X-linked 2 (SRPX2): ccccctcttctgcagcagacggactgagttcctctaatccct-
gtgttccttctcccccatctttct

```
aaaacccttctctgagagaggaataactatagcttcagggataatatagctttaaggaaacttttg
gcagatgtggacgtcgtaacatctgggcagtgttaacagaatcccggaggccgggacagaccagga
gccactcgttctaggaatgttaaagtagaaggttttttccaattgatgagaggagcagagaggaag
gagaaagaggaggagagagaaaaagggcacaaataccataaaacagatcccatatttctgcttcc
cctcactttagaagttaattgatggctgacttctgaaagtcactttcctttgccctggtacttca
ggccatatacatcttttcttgtctccataatcctccctttcaaggatg (Seq ID No: 621)
```

Homo sapiens HIV-1 Tat specific factor 1 (HTATSF1):

```
acctccctttctctgctcagctccagcgtcatttcggcctcttagttcttctgaaccctgctcctg
agctaggtaggaaacatg (Seq ID No: 622)
```

Homo sapiens trafficking protein particle complex 2 (TRAPPC2):

```
gggtctcttccgcggaaactgacattgcgtttccgttgtcggcctcccactgcaggagccatatat
tgaagaccatg (Seq ID No: 623)
```

Homo sapiens UDP-N-acetyl-alpha-D-galactosamine:polypeptide N-acet ylgalactosaminyltransferase 5 (GalNAc-T5) (GALNT5):

```
ccacctttctcttgggcttgtaggaaggtggacatgggctcccggagacaagacaagtgatatgttg
aactgttcggtggctggaatcaactgctcctggagtgacctaaggccagtgtttatcagaacttag
ccagggccagccaagcaggcacagatgctctgctatgaaatgccacgcaggcagagactgacaagc
ggtaggaactgagctttccccttggactgctgcttcctgctgtgttcaggggagggggtcactttc
tggcaactctgctgctgctgctgctgctgctgctacttcagcttcctctccactcaaggtaagcag
gctaagggagggcaggctgctagggaaagctttgtaccatg (Seq ID No: 624)
```

Homo sapiens transmembrane protein 97 (TMEM97): tggcccctcttctca-catcagcgggtccaggcccaaccgacagactatg (Seq ID No: 625)

Homo sapiens EH-domain containing 2 (EHD2):

```
cgtcctccccgctccgggccccacccggctcagacggctccggacgggaccgcgagcacaggccgc
tccgcgggcgcttcggatcctcgcgggaccccaccctctcccagcctgcccagcccgctgcagccg
ccagcgcgccccgtcggcagctctccatctgcacgtctctccgtgaaccccgtgagcggtgtgcag
ccaccatg (Seq ID No: 626)
```

Homo sapiens tubulin tyrosine ligase-like family, member 4 (TTLL4):

```
cgccctcttcttccagactctcggtctgtccgctgggggcgcgcgcggtgtgtggcaggcggcagc
ggcgctggcggccgagtgcgcttgtcacgcgtggcggtgcgtggttgctaggggcgcctgaggctg
ccgggtagcccagcaggccgagggaggaagtagcgtggagccggtgccgagccggggcgaagctgg
atccctagatagactgtcttcaagctcactgatattttcctctgcttgatccattgtgctgttga
gagcctctagtaaatttttcagactgacagacttcaaggatgcagctgctactaccggaggtgtgt
ggcaccttacctcagcaaggccatgagaccgtgtggccatgatgtgggcccctcatg
(Seq ID No: 627)
```

Homo sapiens basic leucine zipper and W2 domains 1 (BZW1):

acctctccctcctcctggcgttagttccggtcgcagaggagacaccgccgcagttgccggtacatc
ggggatttctggctctttcctcttcgccttaaattcgggtgtcttttatg
(Seq ID No: 628)

Homo sapiens centrosomal protein 57kDa (CEP57):

ttgccctttctgtgtaagctgtgagcgtaggcggccctgagggggtgtgttgcaggggtttccaag
cccagcaccagcacccttgccctttttccatcaggggttcagcctagggtccccgctggtgggcggc
tcccgagtcttggagaagagcacgagaacctagaccgcccccgaagtgcggagacccctgggcag
gctgaaagatg (Seq ID No: 629)

Homo sapiens family with sequence similarity 115, member A (FAM115A):

ctgccctttgcctcctgggcggagaagctgcttcctcctgggaacaaccgcctcccgctcctagca
ggttgctactgccccgaacccgcgctgcagggaacagcggggcaaacagtgagtggggttcagcgt
agactctggaccaggagaggcccgcggtgaccgaggcctgggccccggaaaccaatagagccatg
(Seq ID No: 630)

Homo sapiens ATG13 autophagy related 13 homolog (S. cerevisiae) (ATG13):

agccctctttcacccccccccccggccattaccgaagcggatgaaaacaaacactaacgatggcg
gcgccgggaagcgaccggctgctgggcttaaggcgggagtgaccgcttaaccagtgagggaagcac
tgaagagcgccagtcgacgtgggtgcgacaactcgcggagtcttaggagcaaaacgtctggggcct
gcgagccaggacccttctgaagccttaggtgtctatcggcgacgtgtacggtcactgcagctccgg
agcgcggaaccctcagccaggaggcgcggctggtcggtcccaggtcccggcctccgtaatgagagc
ccggaaccactctttgtgccgcagcttcgcagcatcttggactcaagtgattctcctgcctcagcc
tcctgagtagctgggactacagattcctataggcaatg (Seq ID No: 631)

Homo sapiens sorting nexin 17 (SNX17):

ccgccttcccacatcggatcgcagggctcccaaaatggcgagtgaggctgcggggactcgctgagc
agcggagggggagcgtgcagagccgctgcggccctcacagtccggagcccggccgtgccgtgccgt
agggaacatg (Seq ID No: 632)

Homo sapiens phytanoyl-CoA 2-hydroxylase interacting protein (PHY-HIP) :

cgttctttctcccttctctgcctctctctcctccacgctgctttgatttcgctcttgcctctcttc
ttgcgctgctcagctgggaacatcgtctcaccaggggcagcagcgacgcgctgcacagccagacag
gagctggctgcggggcatggaagcagcctccttggcagccgggagaggagcaagcgcacgccactg
cccgtgacccaggcgtccggctgctgtcccctgccggggagctcatccacgcagaggtctctccct
gtcctccctgcgagcttttcctctgcagagcccagtggagccagtccccacaggagacaaccctga
cgggagcatg (Seq ID No: 633)

Homo sapiens translocase of outer mitochondrial membrane 20 homolo g (yeast) (TOMM20):

cggcctttctgtgttcctggcccgcggccgtcgggtgtgagctgcgccgaccgctctgagggttcg
tggcccaccgctccttcgcggtccctgccgccaccgtccacgctcagcgttgtagagaagatg
(Seq ID No: 634)

Homo sapiens KIAA0141 (KIAA0141):

```
cggcctttctagccgctgtcccaagggttggtctcgcgctttcggctgcgagctctctgtggtgct
ggcagcgacatg (Seq ID No: 635)
```

Homo sapiens janus kinase and microtubule interacting protein 2 (JAKMIP2):

```
ctccctcctttaaacagcttctccgggtctcagcatgggcttccagggcagcgattgaggagacct
taccaaggagcaccacacagtagatgctgagacatcgtactccaggataagaaacagtaacatggc
agcacctgcttgaaagaaattaaaaaccaacagactccatttagaaaggaacaatg
(Seq ID No: 636)
```

Homo sapiens EPM2A (laforin) interacting protein 1 (EPM2AIP1): cctcctctccccttgcggcctttctaacgttggccctgctcttgtggcctcccgcagaatg (Seq ID No: 637)

Homo sapiens centrosomal protein 170kDa (CEP170):

```
cggtctttgccgttaccgctatgtgtgggcgtgtgtggaataacgttattgcccagcggagctga
```

```
gggccccggagctcgaccgcagcggcagcgacgacaacagcggcgacgacgacgacgacgaggtgg
ggggaggacggcgtgcgagagactcacgggacgcgacgcgccccgcctcccccgtccggtccctct
ctccacggtaaggggatgacgtagctttgccaaagacttagaagctaagcagaaaatg
(Seq ID No: 638)
```

Homo sapiens suppressor of Ty 7 (S. cerevisiae)-like (SUPT7L):

```
aggcctctcgaggtccagacagccgcccagcccgctctgcgacgcagcagtgaatagtgtggtacc
tccttgtctcggttcaggtccagacctccccgtcttccggctgccctgaacgtcaggcgacctcag
gaccctgtgattggcgcctgcgccggcggaccgtgaccgaggaaacccctggagggacttgggcat
tccttgggctccgtgcctgttcttcgtgctcctttcgggcaaggatctcacattatcagtctttga
ccgacacagaatgcctggcatttgataaatgtttgttgaacttgaagagacatatggacaatg
(Seq ID No: 639)
```

Homo sapiens non-SMC condensin I complex, subunit D2 (NCAPD2):

```
ttttccttttcatttcagcctgactgccggaatcagagccgcgggtgagatccccagccctgtgag
cctgtaggagtagaatg (Seq ID No: 640)
```

Homo sapiens ring finger protein 10 (RNF10):

```
ggttctttgagatgctgtttggcgactcgtcgccattcccggagcaggtcggcctcggcccagggg
cgagtatccgttgctgtgtcggagacactagtccccgacaccgagacagccagccctctcccctgc
ctcgcggcgggagagcgtgtccggccggccggccggcggggctcgcgcaacctccctcgcctcccc
ttcccccgcagcctccgccccgccaggcccggcccggactcccgagccccggcctcctcgtcctcg
gtcgccgctgccgccgggcttaacagccccgtccgccgcttctcttcctagtttgagaagccaagg
aaggaaacagggaaaaatgtcgccatgaaggccgagaaccgctgccgccgccgaccccgccggcc
ctgaacgccatgagcctgggtccccgccgcgcccgctccgctccgactgccgtcgccgccgaggcc
cccgttgatg (Seq ID No: 641)
```

Homo sapiens PAN2 poly(A) specific ribonuclease subunit homolog (S. cerevisiae) (PAN2):

```
agcccttcttgattggaagaagcgcctcggaccccggtccttggcgccgtagtggttaggttgagc
cctaggcgtggggggagaactggggaaactggaatttcccgcggagctgacagcgcttgcgctcccc
ctactcgttctaattccacgcgctccaaaatatccgccatggagaaatcttggccaggatgtccat
tctaggcccatcggtgctgtcttgctgaaggttgggtcaggcatctaaagggactgtggtaaggga
gggtgtgacacaggtgtaagctgccatcgtcatcatg (Seq ID No: 642)
```

Homo sapiens CD302 molecule (CD302): gctcctctccggccgcgcagccgctgccgcccacccgcacccgccgtcatg (Seq ID No: 643)

Homo sapiens NSA2 ribosome biogenesis homolog (S. cerevisiae) (NSA2):

```
gactctttcctgtcccggcctgcgtggtgtgggcttgtgggtctttgagacccgaaaattgagagc
gttttcgcactccagcggctgctcctggcggctctgcggccgtcaccatg
(Seq ID No: 644)
```

Homo sapiens DIS3 mitotic control homolog (S. cerevisiae) (DIS3): acgccttttgctggaagagcgctgctggggttaggattctgcgcggcgaggcaagatg (Seq ID No: 645)

Homo sapiens caspase recruitment domain family, member 8 (CARD8):

```
cctcctctgcgagcgttatttcaaaagaagttgagaaccagagaaaccgacctaaggggattctcc
catttggcccgtcctaccctaaagtcaccacctgctgcttttctggagcgcttaccagtgaccaag
aggaacagaacacagagcagcctggcagtgtccaagcaacaagcctccgctcctccttcctgcacc

ctggggctcctgaaactcacatgggtaaaaaagatacagtaaagacataaataccacatttgacaa
atg (Seq ID No: 646)
```

Homo sapiens epsin 2 (EPN2):

```
ccgcctctcgagcgctgccggtggccgcagcggcgcacccacgccggcccggaggagcagagtgtt
catttctgtgtcgggcacagtgctaagtgctgggtgctcactggtgatgaggcagatgaaggttac
caaacttgtggacaggagcctcatatcagagacgtggacctcactgtagcctggtcatggcttcca
gcttttcgaatctgaggctccaaaggaggaaatgaccattcagggatcttactccagcttgattac
ggagactgaaccttcatagggtgcgcacttaccaaggacaggaaggtttctctgtttgaagggctt
taaacttataacaaagaaaataaaaatg (Seq ID No: 647)
```

Homo sapiens pyridoxal-dependent decarboxylase domain containing 1 (PDXDC1):

```
ccgcctctcaaccatcaggttcggcagcccgcggcgccgcctggcagctcctcctcttctccgccc
cgccggccgcgggcgcggggggacgtcagcgctgccagcgtggaaggagctgcggggcgcgggagga
ggaagtagagcccgggaccgccaggccaccaccggccgcctcagccatg (Seq ID No: 648)
```

Homo sapiens nicotinamide nucleotide adenylyltransferase 2 (NMNAT2):

```
ccttcctttctccctctgcagacacaacgagacacaaaaagagaggcaacccctagaccaccgcga
aggacccatctgcaccatg (Seq ID No: 649)
```

Homo sapiens mitochondrial ribosomal protein S27 (MRPS27): tgttccttttggtacgctccaagatg (Seq ID No: 650)

Homo sapiens leucine-rich repeats and calponin homology (CH) domain containing 1 (LRCH1):

tcccctccttccagcgcctttcggtggagcactgcggcactcagcccgagctgccgttttcccctc
gcggggaacgctgtgaccccccgcaggagcggcggggcggggtggggggcccgggagaagatg
(Seq ID No: 651)

Homo sapiens PAS domain containing serine/threonine kinase (PASK):

gctcctttccgtggtgtgtagccggcttggcgtgaccctcgcctgatccagttgttagagttggaa
gcttggcagttggcctcccttcttcccatg (Seq ID No: 652)

Homo sapiens megalencephalic leukoencephalopathy with subcortical cysts 1 (MLC1):

cttcctttcctagttgggttctgacagctccgaggcagtggtttacacaaccaacacgaaacattt
ctacgatccacccgattcctcccctcattgatattcaggaagcagctctccttcccctgccttcag
ctcaagtttgctgagcttttgtttcatttgtgaatacttcttgctggaagtccctcacccagagac
cagtgctcccaacggcagagcagcggggggagataaagaactggtgacacgtggctgtacattcagc
acagctgtggtgtccccaagtgccatg (Seq ID No: 653)

Homo sapiens RRS1 ribosome biogenesis regulator homolog (S. cerevisiae) (RRS1):

ctttcttttccggattgggcatcccggcatctgcacgtggttatgctgccggagtttgggccgcca
ctgtaggaaaagtaacttcagctgcagccccaaagcgagtgagccgagccggagccatg
(Seq ID No: 654)

Homo sapiens formin binding protein 4 (FNBP4): cgctctctgctcgcgcttgggctcgcgatg (Seq ID No: 655)

Homo sapiens peptidylprolyl isomerase domain and WD repeat contain ing 1 (PPWD1): gcgccttttctgacgatgcgaacaacatg (Seq ID No: 656)

Homo sapiens sorting and assembly machinery component 50 homolog (S. cerevisiae) (SAMM50):

ccgccttctgccctcagcagcagacgctctgtcccgcccgggcagctctgcgaggcagcggctgga
gagggaaccatg (Seq ID No: 657)

Homo sapiens Yipl domain family, member 3 (YIPF3):

gcttctcctttttgtgttccggccgatcccacctctcctcgaccctggacgtctaccttccggagg
cccacatcttgcccactccgcgcgcggggctagcgcgggtttcagcgacgggagccctcaagggac
atg (Seq ID No: 658)

Homo sapiens tectonin beta-propeller repeat containing 1 (TECPR1):

caccctcttgcccggtccccgggagggccggtccgctcctcccggacgccgaggacctaccaccgc
gacttcgccccgcccggcgcgggcccaggaccctgatgtcgcttttgaacagcccctgcacctggc
agccagcgagctactgtagtaggcattgccgactgtttgcataccggatgggagtgacagtgtaat
agaaaaacaagcaagaaacctttaggtaggactcctaaggctcagaggaagttacctccagccgc
tgccatg (Seq ID No: 659)

Homo sapiens DDB1 and CUL4 associated factor 12 (DCAF12):

ccttcccttttcccggctcaagtccttcctctctctctttcctttctttccgcctatctttttttctgct
gccgctccgggtccgggccattttccgggccgggcgcactaaggtgcgcggccccgggggcccagta
tatgacccgccgtcctgctatccttcgcttcccccgccccatgtggctgcggggccgcggcggcgc
tgcccactatg (Seq ID No: 660)

Homo sapiens chromosome 3 open reading frame 17 (C3orf17): ccgcctttcgtaagtccccccgcctcgcatg (Seq ID No: 661)

Homo sapiens LETM1 domain containing 1 (LETMD1): caacctcttctctcccgcttctctcgctgtgaagatg (Seq ID No: 662)

Homo sapiens chordin-like 2 (CHRDL2):

ctccccttctgctggaccttccttcgtctctccatctctccctcctttccccgcgttctcttttccac
cttttctcttcttcccaccttagacctcccttcctgccctcctttcctgcccaccgctgcttcctgg
cccttctccgaccccgctctagcagcagacctcctggggtctgtgggttgatctgtggcccctgtg
cctccgtgtccttttcgtctcccttcctcccgactccgctcccggaccagcggcctgaccctgggg
aaaggatg (Seq ID No: 663)

Homo sapiens CCR4-NOT transcription complex, subunit 10 (CNOT10):

actcctctagccggaacctggggggcccggagccggggtaggcacagagttgtcctcggaggtccag
gacagcggccagcccggcggcgggagtcagggccacgccacctgcagggaagaacccgagtcgaag
cgggaagatg (Seq ID No: 664)

Homo sapiens THUMP domain containing 3 (THUMPD3):

cttcctcttgcagttgaggccggcgccgagccggacttcaggcggatctcgtggcggagcccatct
tgctccctctcccaggcctttacccgctccctaggattcccgggccctgtaggtgggagttgggag
acgacagtactgcttttaaagagacagtgttagggatcttggaagcacagccaacatg
(Seq ID No: 665)

Homo sapiens nipsnap homolog 3A (C. elegans) (NIPSNAP3A):

gctcctttccactcgggaaaccttcagaggagtctcagaaaggacacggctggctgcttttctcag
cgccgaagccgcgccatg (Seq ID No: 666)

Homo sapiens CAP-GLY domain containing linker protein 3 (CLIP3):

gcccctccctctccgcccccaccccctgtcggcgtctgggcctcgtcccccttctctctgtctccct
tgcctcccccatcacgtcccctgacaccgacaccccattgctcccacagtctccccagtctccact

ttggtccccagcgctgtctgcccgaggatttgcctgaaggctgcccccaactctgcacccgcccccc
cgagggccaccgaggaccatg (Seq ID No: 667)

Homo sapiens ring finger protein 167 (RNF167):

cacccttcccgaagttttttctgtcacctgtgttaggctccgtccccttccgcgttttatccccgt
accagaaaaggatacatttagtgcctcccacccagctccactaaacgggttggatatctcattctt
tgagttggtgttccttccccggcgcccccatgtagctgggaagtgggacctggggggtggttggacc
cctgggatcctaaaggaggggcagggagggcgcagaactccgcttctgctccttgctaccaggacg
cgcggcctcctcagcctctttcctcccgctgccatg (Seq ID No: 668)

Homo sapiens polymerase (RNA) II (DNA directed) polypeptide M (POLR2M):

```
cgttcttccgggaaaatggcgactcccgctcgtgccccggagtcaccgccgtccgcggatccggcg
ctagtagcggggcctgccgaggaagccgagtgcccgccgccgcgccagcctcagcccgcgcagaat
g (Seq ID No: 669)
```

Homo sapiens dihydroxyacetone kinase 2 homolog (S. cerevisiae) (DAK):

```
tcgcctctttccgccagcgcccgcaggacccggatgagagcgcacgcttcggggtctccgggaagt
cgcggcgccttcggatgtggcggatgcggccgtgagccggcggggggaggtgctgctgctgcctcca
ctgtactcagacccaggtagcacaggattgtccatcctccagcagctcagtgcaacggtgtgaact
cagcctgtttcagagcctccacaccatg (Seq ID No: 670)
```

Homo sapiens RNA polymerase II associated protein 1 (RPAP1):

```
cgatctctgcggggcaagatggcggcgcccagacaggcctggagcacggatgaataagagggaacc
cccacacggagacactgctggagagagtcgtactggggaggcagctggagcagcaagatg
(Seq ID No: 671)
```

Homo sapiens torsin A interacting protein 1 (TOR1AIP1):

```
cctcctctttggtgcctccagccaggaggcgggagcgatccacagcagctgacccagctcaggcac
tgcctctctcacagccctcaagacacaccatgggcccagaggcaggtttgctacacagcagcgacg
acgcaggcggcggccccagcgactcgcaactgcctccctgaccacagcggccaccgcccaacaccc
ccgagaagccatcgccaccaccggcaggagaacctagggtccataaagccatcttcgcgatcgact
aaagctacgtcaacaactatg (Seq ID No: 672)
```

Homo sapiens SERPINE1 mRNA binding protein 1 (SERBP1):

```
cccccctctctcggcccggccatcttgtgggaagagctgaagcaggcgctcttggctcggcgcggcc
cgctgcaatccgtggaggaacgcgccgccgagccaccatcatg (Seq ID No: 673)
```

Homo sapiens N-acetyltransferase 9 (GCN5-related, putative) (NAT9): cacccttctgcgggggacgatttcgtcggtggtaggct-gctaccatg (Seq ID No: 674)

Homo sapiens ribosomal L1 domain containing 1 (RSL1D1): gcgcctcttcacgaggtggaaacaagatg (Seq ID No: 675)

Homo sapiens SH3 domain containing, Ysc84-like 1 (S. cerevisiae) (SH3YL1): cttcctcttcctgggcagcctcgggacggggcgccgcggccgggcgggcagcatg (Seq ID No: 676)

Homo sapiens methylmalonic aciduria (cobalamin deficiency) cbld type, with homocystinuria (MMADHC):

```
acttcctttgcctgctcaccgccagcgtaggtgctaccaccgctgccgtcgccgccgccattttga
```

```
tggcaggaagagtccggttctgggacagctggagacagtggtggtgactgaaataactttaccaaa
ggaaagctattttgcgaactatcttctccagcggagatg (Seq ID No: 677)
```

Homo sapiens glioma tumor suppressor candidate region gene 2 (GLTSCR2): agttcttcctttgacaagatg (Seq ID No: 678)

Homo sapiens DDB1 and CUL4 associated factor 8 (DCAF8):

```
cagtcttctcgagcacatcgtcgcaaacggggccggaaagcgtggcagcgcaggcgcaagcgcaga
gagcggaggcggtggtggtggcggccgctggccagttccttcagtgaatctacagacctattttct
caggagctcagcctggccttacttcagtgataaaaggaggaaaggctggctacagcaaacatcatt
caagatg (Seq ID No: 679)
```

Homo sapiens UBX domain protein 1 (UBXN1):

```
ctttcttctcgtcggtgttcccggctgctatagagccgggtgagagagcgagcgcccgtcggcggg
tgtcgagggcggggttgcctcgcgctgacccttcccgccctccttctcgtcacacaccaggtccccg
cggaagccgcggtgtcggcgccatg (Seq ID No: 680)
```

Homo sapiens antizyme inhibitor 1 (AZIN1):

```
ccgccttctcacactttcaggctctgatcgcggccgcagttttttcctttttttcttctgccgtcgcc
ttctctgcctcttctcatcctttctcgctctgctgctctgcagtgtgacgagtccgaatcctcttc
ccacccagcccgcgcctttcttcttttgcctgcgctgttctatttctccttcggccgccgccgcca
ctgctgcacacagctggtgtcggtgccgcgcttttaccccaagtcgttcccgcagcctatggccc
aggccgccttgggtatttctgctcaaggtaaccacatccctctttaaaaattccgccgaaaaagag
aagacgctttacccgactctttgggccgttatctcacggcgaactttctgaccaagtatacaacta
cccagagggcctaggagaagtgctgtatagagagcagttcgacttcaacgctgagccaccttggga
acctagctgatgataggggggttccatctcccaacttgtccatggaggtcttcacttcagaaatcc
aagactcatattcatccagcttggtgtcaagtgggctgttgctgccagaattatcttgtgattatt
tgagagatgtatcagtttcttctgaagtacaatcaactgtagaagcctttgtagcagtttgttgca
tattctaaggacccagacataggcttggtggcccgtctcttgtctttcctggtttatgactttcgg
ctttgtggaatacggctgagatg (Seq ID No: 681)
```

Homo sapiens cell division cycle 40 homolog (S. cerevisiae) (CDC40): gcctcttcttcttccgccctggcagggtctccgcagaagatttgttgccgtcatg (Seq ID No: 682)

Homo sapiens stathmin-like 3 (STMN3): gcgcctctccagcctccgcaggcccaaccgccgccagcaccatg (Seq ID No: 683)

Homo sapiens nudix (nucleoside diphosphate linked moiety X)-type motif 13 (NUDT13):

```
tttcctcttttgtgctgattcctgaggactaggaaggtgccccgaaaagaattcagagacctgaca
atg (Seq ID No: 684)
```

Homo sapiens calcium homeostasis modulator 2 (CALHM2):

```
ctctcttttctggagttagattagtctgaagccgccaccagccccaggcccccgtgcagaagaaaa
gcgggagggaacggcggaggccgccgctgccctgcaccgccctcctggaggccacttggagagtcc
ggccccgaggaggccatggccacaagtgcccacagctggccccaggttgccagcgtcgctacagcc
cagaccaaggcagaataatctccggatgagctggtggcaccgctgagcctttggtctcaccagggc
ttcctgttgctggcaggcggggtggagcggagctgctgggaggctgctggataggagaggggtcac
ggctgcggaagaggaggttcttcgggacacccgtggatggacacggcaaggaaacaccaggccaac
cacagctggggataaaatagcacaaccacaccctgccgtccagcgcctcccagcctgtgccccttc
ctagtaccaccagcaaccatcaatcccgtctcctcctgcctcctctcctgcaatccaccccgccac
gactatcgccatg (Seq ID No: 685)
```

Homo sapiens NMD3 homolog (S. cerevisiae) (NMD3):

tcttctctgtggcggagacagccaggttggcagctgacgggacagccggggtctattttgttgcgg
gttttcagcaaatccagggctggtctggaggcgcgaaaacttaaggcatacagaacgatg
(Seq ID No: 686)

Homo sapiens ATPase, H+ transporting, lysosomal 50/57kDa, V1 subun it H (ATP6V1H):

gcgcctctgtcattctactgcggccgccctggcttccttctacctgtgcggccctcaacgtctcct
tggtgcgggacccgcttcactttcggctcccggagtctccctccactgctcagacctctggacctg
acaggagacgcctacttggctctgacgcggcgccccagcccggctgtgtccccggcgccccggacc
accctccctgccggctttgggtgcgttgtggggtcccgaggattcgcgagatttgttgaaagacat
tcaagattacgaagtttagatg (Seq ID No: 687)

Homo sapiens DPH5 homolog (S. cerevisiae) (DPH5):

gggccttttctctgcacggagccggcgcttttgcagttgcttctgcggaaaggtggtagttaagaa
tttgtaaaggccagagaactacctacgattctctcagcggtctctcttctcctcaagtttgaaatg
(Seq ID No: 688)

Homo sapiens polymerase (RNA) I polypeptide D, 16kDa (POLR1D):

cctcctccctccttccgtcctccgcgccttccgtcggtcggtccttgcttcctgcttcgcctccgc
gcctcgcgctatgggacagagcccccgatccgccagcaccacctgaggatccagaaaccgccccag
cgatg (Seq ID No: 689)

Homo sapiens HMP19 protein (HMP19):

ctgtcctttcagcaccacaagctcgggctgaggagggaggactcctggccgtcctcctcctcttca
aattggcttgaatcttctctgacccccacgagtgcagcacagtctgggaagaaaggcgtaaggat
g (Seq ID No: 690)

Homo sapiens adiponectin receptor 1 (ADIPOR1):

gcgccccttccggcgcggggagggcgctgaagatcggggccgctcggccgcaggccgcctccagcg
ccgcgggatgtagcgcggggggaccgcggcccccagcagagcccgcctgcccggcttgtctaccatc
agagggagatctctgcccctggggctgagagaccccaacctttccccaagctgaagctgcagggt
attgaggtaccagccagatg (Seq ID No: 691)

Homo sapiens SH3-domain GRB2-like endophilin B1 (SH3GLB1):

ttttcccttgggacccgggtccacacggcggggtcgcccgtccatctccggctcgcccgcggggcc
catcgtcgacgttagcggccgttctccgagccgactgacccatccttggcgctgccgccgcgcgct
tgttctcctccctcgccccgccttcatcctccccgttcacggaaacgacagctgcggctgcggggc
tggcgccgcctccctccacctaccacgtctgccctcgccgctctagccctgcgccccagcccggcc
gcggcacctccgcctcgccgccgctaggtcggccggctccgcccggctgccgcctaggatg
(Seq ID No: 692)

Homo sapiens anterior pharynx defective 1 homolog A (C. elegans) (APH1A):

```
gtccctcttcggcttccgtagaggaagtggcgcggaccttcatttggggtttcggttcccccct
tcccttccccggggtctgggggtgacattgcaccgcgccctcgtggggtcgcgttgccacccca
cgcggactccccagctggcgcgccctccatttgcctgtcctggtcaggcccccacccccttcc
cacctgaccagccatg (Seq ID No: 693)
```

Homo sapiens RNA binding motif protein, X-linked 2 (RBMX2): ctgcctttcccgggcgctgattcctgagtgctgagcgcgaacccgaggagatg (Seq ID No: 694)

Homo sapiens family with sequence similarity 82, member B (FAM82B):

```
atctcctttagccccgcccgcctccgtagctgcctgaagtagtgcagggtcagcccgcaagttgca
ggtcatg (Seq ID No: 695)
```

Homo sapiens UTP11-like, U3 small nucleolar ribonucleoprotein, (yeast) (UTP11L): tgatcttttccaaggctgtacagacatg
(Seq ID No: 696)

Homo sapiens chromosome 14 open reading frame 166 (C14orf166):

```
cgccctctcgccgcgtcgccggtgcctgcgcctcccgctccacctcgcttcttctctcccggccga
ggcccggggggaccagagcgagaagcggggaccatg (Seq ID No: 697)
```

Homo sapiens transmembrane emp24 protein transport domain containi ng 5 (TMED5):

```
gcttctctttcggagggagtgttcgccgccgccgcggccgccacctggagtttcttcagactccag
atttccctgtcaaccacgaggagtccagagaggaaacgcggagcggagacaacagtacctgacgcc
tctttcagcccgggatcgccccagcagggatg (Seq ID No: 698)
```

Homo sapiens coatomer protein complex, subunit zeta 1 (COPZ1): gtttcttttgcggctccacgtcggcaccagctgcggggcaagat
(Seq ID No: 699)

Homo sapiens mitochondrial ribosomal protein S16 (MRPS16):

```
ggttctttctgtgtttgttctctgccctgccaaggccgtagagctggtgcgtgcgggtagcggggc
tctccgaggagccgcacgccggcggcaccatg (Seq ID No: 700)
```

Homo sapiens charged multivesicular body protein 3 (CHMP3):

```
ctacctccttttccgcgggccccgcccaggcggctgcccgtgacctgcctgggcgcgggggaactga
aagccggaaggggcaagacgggttcagttcgtcatggggctgtttggaaagacccaggagaagccg
cccaaagaactgatatccaaagagaagaagaaaaagtgaaacgatctgtgaaagatgctgccaaga
agggccagaaggatgtctgcatagttctggccaaggagatg (Seq ID No: 701)
```

Homo sapiens RNA binding motif protein 7 (RBM7): cgaccttttggccaggttagggagggggcgacgctgagatg (Seq ID No: 702)

Homo sapiens eukaryotic translation initiation factor 3, subunit L (EIF3L): cgctctttccggcggtgctcgcaagcgaggcagccatg
(Seq ID No: 703)

Homo sapiens zinc finger protein 706 (ZNF706):

```
ccttcctttccctccggcgtcctctcccggccctctcgcgctgcactgtctctccgacgcaagact
gtcccggcccggatatg (Seq ID No: 704)
```

Homo sapiens androgen-induced 1 (AIG1): cgccctccttgccgcccagccggtccaggcctctggcgaacatg (Seq ID No: 705)

Homo sapiens interleukin-1 receptor-associated kinase 4 (IRAK4):

```
cgccccttcgcggcgcttcctagttcggctggttcttctgtcgccggcttcagcagcccgcgcccg
ggcaggaatagaagatg (Seq ID No: 706)
```

Homo sapiens transmembrane protein 66 (TMEM66):

```
cgttccttcgccgccgccaggggtagcggtgtagctgcgcagcgtcgcgcgcgctaccgcacccag
gttcggcccgtaggcgtctggcagcccggcgccatcttcatcgagcgccatg
(Seq ID No: 707)
```

Homo sapiens carboxypeptidase Q (CPQ):

```
ccgcctctcggccccgcggcctggccggcaagcagggctgcagtcacggggcggcgcggagggccc

cagcccagtcaggggtgtggccgccgccaccgtaaggctaggccgcgagcttagtcctgggagccg
cctccgtcgccgccgtcagagccgccctatcagattatcttaacaagaaaaccaactggaaaaaaa
aatg (Seq ID No: 708)
```

Homo sapiens hydroxysteroid (17-beta) dehydrogenase 12 (HSD17B12): cgctcttttcattcacgaaggtagtgaggcctagtggaaagccatg (Seq ID No: 709)

Homo sapiens protein phosphatase methylesterase 1 (PPME1): cctcccctcgatg (Seq ID No: 710)

Homo sapiens HemK methyltransferase family member 1 (HEMK1):

```
ccccctttccggcaggctactgggctccgcccacacacctcccggcctggttcctaaacgccagct
cggagcaatccccttgggctggagccaaatccctgctgtgattttaaggaagaccggcaggtccgg
gcccccaagggtcaaccccacacacatccccgcactttcctgtatgcaggcctgcgagcgtagagg
gagtggaattcacagcctccccacccatccgcaggggtctcctgggaggaacccaccagcgatagg
aacactgaagctgggctacggcgtccgcccgagccttttcttaaaggcgccgaccccggaagcggg
gcgtccgagggagcgcgcgacgggccacgcacgtccgggcgtccagttcggggcagcttctccggc
tggtgggtgggtggggcagcctttcaggcagggtggcaaccaactatatctgaggaccagagccat
tttggggcaccagagcttgtgacctctccatctccacccagctgggtccaggggccactctcagca
ctcacctcagcagctgacatcataaagcagacttgggaacctggaagcactctggagaacctttcc
ctgagacatg (Seq ID No: 711)
```

Homo sapiens N(alpha)-acetyltransferase 38, NatC auxiliary subunit (NAA38):

```
cgccctttcagttctgcttgctgtcggcaccgctgcgttacccggaaccgccgggccgaacagcat
g (Seq ID No: 712)
```

Homo sapiens cleavage and polyadenylation specific factor 3, 73kDa (CPSF3):

```
ggttcttcctttttttatttaccggtggctgtgcttccaatttaggaagaccccggcgacctgttcc
tcacccccgcttcgccctcacactttcgggatg (Seq ID No: 713)
```

Homo sapiens dynactin 4 (p62) (DCTN4): tcgcctcctccctccccaagatg (Seq ID No: 714)

Homo sapiens hydroxysteroid (17-beta) dehydrogenase 11 (HSD17B11):

```
gttcctccttgctctcgcccctactctttctggtgttagatcgagctaccctctaaaagcagttta
gagtggtaaaaaaaaaaaaaaacacaccaaacgctcgcagccacaaaagggatg
(Seq ID No: 715)
```

Homo sapiens YTH domain family, member 2 (YTHDF2):

```
tagtctttccaggtgttagtcgaaacctcgtggtgcgaccctggtcgtcccaaaccccctaggcct
taatcctggggcggtggggggcggggaggccgtgagcacggcttccgctcctccaatccgccagagg
gcgcagcggccggcctctcccttcccggggttcttcgcgccgggcccccttccgcgtgggtgagtga
atgtgagagtcagcgctcgcgccgcgcgcgccgcccgcctccgctgttcggcgctctgctttaggc
ggtggggggcgggcgcgcgtaaaagcatagagacgggcattgagctcttgggctagagcgtcgc
cgagtcggagccggagcctgagccgcgcgctgtgtctccgctgcgtccgccgaggcccccgagtgt
cagggacaaaagcctccgcctgctcccgcagccggggctcatctgccgccgccgccgcgctgagga
gagttcgccgccgtcgccgcccgtgaggatctgagagccatg (Seq ID No: 716)
```

Homo sapiens tubulin, epsilon 1 (TUBE1):

```
agctctctagcagagcgccgttgctgggggaatgcagaagcggccgcgggctagcaagctcccgga
gccggcggcgcaccaccatg (Seq ID No: 717)
```

Homo sapiens ubiquitin interaction motif containing 1 (UIMC1):

```
cctccttttcttcctcagcgggtccgcggcccgctactctccgggaggggcgcttcccgacgccaa
ggtaggcctctcccgacgccggggcggcccttcctgatgccggggtgtgtctctcgcgacgcgggg
gtgggctccggacgccggggctggccttgccgaagtcggggggtgggtccctccggacgccgaagtg
ggctcgggatgcggggctgggaccctcccgattccggggcggattccggacgccgggaccggccat
tactggtgccgggttgggcttctccagatgccggggctgggtccttcccaaggttgagacaaaagg
atg (Seq ID No: 718)
```

Homo sapiens TNF receptor-associated protein 1 (TRAP1): ccgccccttccatcgtgtacggtcccgcgtggctgcgcgcggcgctct-gggagtacgacatg (Seq ID No: 719)

Homo sapiens cereblon (CRBN): cagcctcctttgcgggtaaacagacatg (Seq ID No: 720)

Homo sapiens ribosomal L24 domain containing 1 (RSL24D1): cttcctctcaagcttggcgtttgtttggtgggggttacacgcgcgggttcaa-catg (Seq ID No: 721)

Homo sapiens leucine carboxyl methyltransferase 1 (LCMT1):

```
taccctcttctgttgctttctccctgtggctcgcgccgtcccccgccgcccgtcgaccccgcttcc
atgtccctggcggacacagctcccaggaacctccacgcccatggccactaggcagagggaatcctc
tatcacctcctgctgttccacctcgagctgcgacgcagacgacgagggcgtgcgcggcacctgcga
agatg (Seq ID No: 722)
```

Homo sapiens RAB14, member RAS oncogene family (RAB14):

```
ccccctttcttttgtggtccggcccattgcgagggtgacaggaaaccctgtgcagggagcgccgcca
tcttggaccagcccgaggaagatactgagggagcacaggagcagtcaccgctgccactgctactgc
cgctactgctgccggcgcgtctgcacctctcggcctgccagtgtacctgccggcgcctcggtcgac
cgcccccgcccctctcccgctgcgtccgcactcctgttcctggtcctgacgcccccctcccgccc
ggaaagctgcccagccaccagcaacccccagtgccaccatg (Seq ID No: 723)
```

Homo sapiens Enah/Vasp-like (EVL):

```
cttccttttcctgtttggttttaagtaggctataaaaatcaagttgctgtcttcagagggtctgtg
gtcctctgatcaacataggctggtgggagtacaggactcgcctcctcagggttccctgtgctgcca
cttttcagccatg (Seq ID No: 724)
```

Homo sapiens LIM domain and actin binding 1 (LIMA1):

```
ctctcttcccctctccctctccctctgccgggtggatgctttctccatgtggcaaggctgtaactg
ttcacagctgtctgaaacagcagtggaccaggagcagcttggagtttttaactttcattttacaaag
aacaacatgtttgaatgtttcagcaggcaagttataactggcatctacttcttgttcttctagaac
accgaaaatctctcccagcactttagaaaggggaccctgactgtgttaaagaagaagtgggagaac
ccagggctgggagcagagtctcacacagactctctacggaacagcagcactgagattaggcacaga
gcagaccatcctcctgctgaagtgacaagccacgctgcttctggagccaaagctgaccaagaagaa
caaatccaccccagatctagactcaggtcacctcctgaagccctcgttcagggtcgatatccccac
atcaaggacggtgaggatcttaaagaccactcaacagaaagtaaaaaaatg
(Seq ID No: 725)
```

Homo sapiens ubiquitin-fold modifier conjugating enzyme 1 (UFC1): gtttctcttgcgccctggtccaagatg (Seq ID No: 726)

Homo sapiens coatomer protein complex, subunit beta 1 (COPB1):

```
caccccccttccacgtcagccaaggactctggagccgccgccgccgctgctgcggttcatagccgga
gtagacggagccgcagtagacggatccgcggctgcaccaaaccactgcccctcggagcctggtagt

gggccacaagcccccagtcccagaggcgtggtgggtcgggcagagtcggaagaactggctttctag
ctggaagatgcggaaggggagcgactaggccgcttgcgtctgggcctggcagaagggaccggattt
tctggcatccttaaatcttgtgtcaaggattggttataatataaccagaaaccatg
(Seq ID No: 727)
```

Homo sapiens transmembrane protein 9 (TMEM9):

```
gggtcttttgcggctgcagcgggcttgtaggtgtccggctttgctggcccagcaagcctgataagc
atg (Seq ID No: 728)
```

Homo sapiens shisa homolog 5 (Xenopus laevis) (SHISA5):

```
ctttctttttctccaaaaggggaggaaattgaaactgagtggcccacgatgggaagaggggaagcc
caggggtacaggaggcctctgggtgaaggcagaggctaacatg (Seq ID No: 729)
```

Homo sapiens transmembrane protein 69 (TMEM69):

gtgcctttccagtggacctgggctgttgttgcggttgttttccttctctccgtgcaacgctggcaa
gtctcaaagtcgccacagaaacatgcccctgattcagtgcctctgcttagctgtaacatgttaatc
agaactacctggcatcttcctgaacaagactttcaataggggccagtatg
(Seq ID No: 730)

Homo sapiens kelch repeat and BTB (POZ) domain containing 4 (KBTBD4): agatcttcttccgggcggacgtggagccggaagcggaggttccgggctccgggatg (Seq ID No: 731)

Homo sapiens pipecolic acid oxidase (PIPOX):

cgtcctttagccgggagcctgtctttgcttgcctttgcctttgaggctctgtggctgtggggctga
gtggcatcatg (Seq ID No: 732)

Homo sapiens blocked early in transport 1 homolog (S. cerevisiae)-like (BET1L):

agctctttccccgcgactgcgccacgtctgaggcggctgtggccgcgtcggtgtccgcgtcgagga
gccggggcagggcacgatg (Seq ID No: 733)

Homo sapiens zinc finger protein 581 (ZNF581):

ttctctctttcggccggcgccgccagttcctggggcacacccagaggtccccttctcgccgccgcc
tgcaactgcgagggtagcccggggccgcttggagtcgcccggacctgagaggctgctgcactgggc
ctcagccagccctccggatg (Seq ID No: 734)

Homo sapiens armadillo repeat containing, X-linked 1 (ARMCX1):

cgtccttctaatcctagtcttcgtttggtccggttgcactcttcctatagcccagagggcgagagg
gcctgtggcctggggggaaggaggacgaggttctgcctggatcccagcagtaggacgctgtgccatt
tgggaacaaaggaatagtctgcctggaatccctgcagatcttggggccggaggccagtccaaccct
tggagcaggaagaaacgcaaagttgtcaagaaccaagtcgagctgcctcagagccggcccgcagta
gctgcagactccgcccgcgacgtgtgcgcgcttctctgggccagagcgagcctgttttgtgctcgg
gttaagagatttgtcccagctataccatg (Seq ID No: 735)

Homo sapiens spastic paraplegia 21 (auto-somal recessive, Mast syndrome) (SPG21):

cggcctcccgcacgcaccgcgcagcctgctgtgcccgtgggtcccgagtgctccgccgcccgcccc
gacccgggcccagccgcctccacggcccgcgctcgtactggagcgaagagcggcctcctgaaggag
gggaagggacgtggggggcggccacggcaggattaacctccatttcagctaatcatg
(Seq ID No: 736)

Homo sapiens.staufen, RNA binding protein, homolog 1 (Drosophila) (STAU1):

tctccctttttttccttcttccttcccctcctcgccgccaccgcccaggaccgccggccggggggacg
agctcggagcagcagccagagtttattaaccacttaacctctcagaactgaacaaagacaacattg
ttcctggaacgccctctttttaaaaaagaaagcataacccctactgtagaactaaatgcactgtgc
atg (Seq ID No: 737)

Homo sapiens adducin 2 (beta) (ADD2):

cggccttttgtcagcgcgcagggccaggagagctctcatttcctcccagcctcgtgcgggaaatgg
ctttaattctgacggcagggctgtgagggactagcgggaacccgagccttttgtcaaggaactgcg
gcgtcggtggccagtcatccccgccgccgcggagccgctgcactgctgggggatctcccagcagct
ctgacgagcgcgggctgcagcatgggcagaaaacgctgccctgcagattagctgggtggattttt
aagcgcaccccacccccaaaccataaaataacaaaaccaacccgcagtggccgaccggagatag
ctaagatgccgcgcaggagtttccacctggatgtttgaggttgtgtagatgtggccggcacccttg
agagtggagctaggggggtgcagactgagcagtgaacagaaggagccttggacagggctgggccagc
ctcccgagttccaggagcgaattgcaaacccaccgggaaaatg (Seq ID No: 738)

Homo sapiens WD repeat domain 1 (WDR1):

ccgccttccggctccagtcccccgggctcggcctcggcgaggtgtaattcgcagcgcgggccggccc
cggaggctctcggcgagcgcggcgcggtaacaagtgggcgaggatg (Seq ID No: 739)

Homo sapiens family with sequence similarity 20, member A (FAM20A):

cgacctctacttccacctctggccccaagtacagcgccagctgcggcctcgggagcgcccgcgggg
gtgcccgtgcaccggccgcgcctcctccctggcgcgggactcggccgcagctgcctcggaccccgg
cacgatcgtgcacaacttttcccgaaccgagccccggactgaaccggctggcggcagccacagcgg
gtcgagctccaagttgcaggccctcttcgcccacccgctgtacaacgtcccggaggagccgcctct
cctgggagccgaggactcgctcctggccagccaggaggcgctgcggtattaccggaggaaggtggc
ccgctggaacaggcctcagttcctgcttttgaaaggaagagggggagtctgtgacccctgaggcct
ccttgcaactctgttttccaagctttgcacatcttccgaatttcttcttcaaagtctaccctaatg
aaatatcagacaattttccaagtgtgcttcatgaacttctgggaggtgcttcacagtttctgcaaa
tgattgattgaattttcactttgaaaaaatatactttaaggcgacacaagatg
(Seq ID No: 740)

Homo sapiens kelch domain containing 4 (KLHDC4): ttttctttcctggtgtcccgtcgcggcttgggacccggcaagatg (Seq ID No: 741)

Homo sapiens calcium channel flower domain containing 1 (CACFD1):

tgctccctctcccacaaggcagcgcgccggctcggacgcggccggctaccgagcccttttgtgaggg
ctgtgagctgcgcctgacggtggcaccatg (Seq ID No: 742)

Homo sapiens zinc finger, CCHC domain containing 8 (ZCCHC8): gaatcttttccacagcccaaaatg (Seq ID No: 743)

Homo sapiens kelch-like 24 (Drosophila) (KLHL24):

gtttcctttgttgtgagctgcggcagagactggtggctggaggagacgccggcgctggagagtgcg
ctgcgccgcccgccgctgagggaccgcggggttagccactgctggctgcttccagtgttcgccgag
aggtaccggggggtgacagctccgggaccggccgaaaggcgaggaaccggtgtggaaattaaaagaa
cacacatattttgactggggctttgatcaaccaaatgctaaaaagccacataaagaagatccctaa
tagtcatttctcaacaattatatagtcaactgatgtaacaatg (Seq ID No: 744)

Homo sapiens FtsJ homolog 3 (E. coli) (FTSJ3): ctcccccttttccaccatg (Seq ID No: 745)

Homo sapiens dymeclin (DYM):

gcttccctcttctctcgccgcctcctggcctccgcaccgacgcggcccgggctggagccgagccgg

ggccgagctgcaggccggaccggagccggatctgtacccgctgagacgtggaaacatggaggcctg
agccggtgtgcgccacctgggctgcggcggcgacagcgacttctcctgacccctctgccaccctcc
catccgtccgcgggtccgtggagctggagcagatcccccagccggctgagacaggttgtcttttgg
aaatgcaggtttaaggacaaattatctgcttaagctagaagatg (Seq ID No: 746)

Homo sapiens zinc finger protein 280D (ZNF280D):

cctcctcttctcctcctcctcagggctccagtcaggccgatccgctccgctcacggaaggaaaac
agaaataacttgctggcttgtctggagtcacatgtacttaggtgacaatttacagaaagtcatctc
tgcagcttgatg (Seq ID No: 747)

Homo sapiens ankyrin repeat domain 10 (ANKRD10):

cgttcctttgtgctgcggcggcggcttctcgagtcctcccgacgcgtcctctaggccagcgagcc
ccgcgctctccggtgacggaccatg (Seq ID No: 748)

Homo sapiens SWT1 RNA endoribonuclease homolog (S. cerevisiae) (SWT1): ctctcctttggcttggggctccggagttgccactgccgccggcgctggtaagcttttcaggatg (Seq ID No: 749)

Homo sapiens leucine rich repeat containing 49 (LRRC49):

tgacctctttcgggtctctttgaatctccgctgtagcgtcacctggaaggcagatctaacagagaa
cctggactgtctcctatcatg (Seq ID No: 750)

Homo sapiens F-box and leucine-rich repeat protein 12 (FBXL12):

ccgccttctggacttggtcttagttcccagtcgcggccaaatcacgcctcagccacctcccgcaag
cctctcactgcctcagccacgctttccaggctggtttctggtccccatccgcggctggtccggccc
tgggaccgaatcacttcccagcgagaggaaggtcaaatttctcgaccggctacgggaaggtcgcgg
ccgccgccctgtcagccgcctcggcgcccccaggacccctcgggtctctttaaccggaagcggaag
tgcgtgtcggcgggatcatg (Seq ID No: 751)

Homo sapiens WD repeat domain 55 (WDR55): cagtccttctcagcatg (Seq ID No: 752)

Homo sapiens zinc finger protein 3 (ZNF3):

cgttctttgttctgtccccggtgtgtgtgggtctgtgacagggtccaacagggcctggtccgtgtccg
gtcccccaaatctgtcgtccctgcccccaggcattggcatcaacaaaagtcagaattcccgggaac
ttgaacagaggctgctaaattcccagtaattgctcctttggccttctagggactgacttcaaagaa
ggaaggaaagaatcaggcagtgcttcctcattctcttttaaaacccgcttcccgctgagtctgcac
ccaggagaccagagagcaccttgcccttccatg (Seq ID No: 753)

Homo sapiens tetratricopeptide repeat domain 27 (TTC27):

ggttcttctcctaggcggaagccagaccagagagcgtgcgtgttttttcccagggtgccccgcgctg
ctgttatggccgcctccttgaggtagtatccgcacatggaattctagggccgcaggtgtatttacg
gtaactgtcgccactagatttcagcgcctttggactctcctgttttcactttcttttgttgactcc
cgtgtggccctcgtgggagcctgttttggctgcagcggtgtctggggtgatg
(Seq ID No: 754)

Homo sapiens THUMP domain containing 1 (THUMPD1): gtttctctttcctctcagtttgcgcacaccatg (Seq ID No: 755)

Homo sapiens ankyrin repeat and KH domain containing 1 (ANKHD1):

```
tgctcttctcgttcccgagatcagcggcggcggtgaccgcgagtgggtcggcaccgtctccggctc
cgggtgcgaacaatg (Seq ID No: 756)
```

Homo sapiens syntabulin (syntaxin-interacting) (SYBU): cctcctcctggacggcggcagcggcggcgcgaggagccggcgggcagcggcgcgatg (Seq ID No: 757)

Homo sapiens coiled-coil-helix-coiled-coil-helix domain containing 3 (CHCHD3):

```
gcgccttctccttgcttctggggggtcgtggccttgctcccgctgtgcgggaaaagaatccaggccc
ttccacgcgcgtgtgggtgcggggggccccgaagtgctcgtggttccccgctaggtctccgctgggg
caggaaccggaatcatg (Seq ID No: 758)
```

Homo sapiens HAUS augmin-like complex, subunit 4 (HAUS4):

```
cctccttcgtcgcggcctctagtgcactttcggctccttccccttcccgggcctttcagcttggtc
tttccgggcctcgcttcccccagcccctgcgcccggcccgaacgagaggttccggagccccggcgc
gggcggggttctggggtgtagacgctgctggccagcccgccccagccgaggttctcggcaccgcctt
gagagcttcagctgccccaggattagaatcccaagaaaatcaaatg (Seq ID No: 759)
```

Homo sapiens solute carrier family 41, member 3 (SLC41A3): ccgcctctttcccgccgccgcctgggaggggacccgggctgccaggcgcccagctgtgcccagatg (Seq ID No: 760)

Homo sapiens phosphatidylinositol glycan anchor biosynthesis, clas s V (PIGV):

```
cttcctttccagcctcccgccctcgtctgcttccggccctgtggcctggtggggctctgcaggctc
cctcgggagtggtccttgggccgtggcccctctgggaggcctgagggagctcaatcctggtagcaa
caccctgaattcctggtggtgaaaggatg (Seq ID No: 761)
```

Homo sapiens poly (ADP-ribose) polymerase family, member 16 (PARP16):

```
agttcctttatccctgggcccaacctccccgccgacccgcggtccaggcctcggtctctctcttcg
gcggcgagccgcggcccagaccccggcagaggacacttgtcggcacgttctcaccctgtcatctc
agcccctgcctagctccaccccaggcttgggaacccggcccctgacggccattgtccgcgggcc
cagcccccgcgctgaacgcacgctcgcccttgcccctaaccagcgcgtctaccccggcaacgcgca
gtgacctgggatg (Seq ID No: 762)
```

Homo sapiens thioredoxin-like 4B (TXNL4B):

```
gtttcttttctgcgcttgtgcgttttctgttcggtttccttcccgctagcggggccacgagggttg
ctaggcaacagcccctgggtgacttggtcttagggtcctgtccggcttggggctgatgaaaggagc
tgtccgcgcccgggctcttccgagaagtggttgctgacagccacaaagtgaaagggagtgaggcgg
cgtggacgagtaaggagtgacagtgaggattcacatttgggttatttcaagatg
(Seq ID No: 763)
```

Homo sapiens slingshot homolog 3 (Drosophila) (SSH3):

```
cgtccttcctggtcctgcgggtccaggactgtccgcggggttgagggaaggggccgtgcccggtgc
cagcccaggtgctcgcggcctggctccatg (Seq ID No: 764)
```

Homo sapiens zinc finger protein 692 (ZNF692):

```
ctccctctggggcgcgggcctcagttccgggctacagcagccgacgccgagaggcaccgtttcttc
ttaaaagagaaacgctgcgcgcgcgaggtgggcccctgtcttccagcagctccgggcctgctcgct
aggcccggggaggcgcaggcgcaggcgcagtggggggtgagggcgcgtggggggcgcacagcctctggt
gcacatg (Seq ID No: 765)
```

Homo sapiens tRNA-histidine guanylyltransferase 1-like (S. cerevisiae) (THG1L): tggccctttcctttccgcgtgtagaatg (Seq ID No: 766)

Homo sapiens solute carrier family 25, member 38 (SLC25A38):

```
tctccccttctacagagttcctccggcgcttcctccaccccgggatacacagaacctcatctccta
cggtgctgaagcctgcagcagggcaggatgggcaggagagcagagccgcggagtctgcggcgcggg
tgaagagcggcgcgtaattcccgcagcaagattgttccgcgcccgcagcccctggactagcaggat
ccgaaccccggcggctgcgtgcttataggcgcagacgtcagagagcccgcggcttaaagcgcgtcg
cctggctagcgccacccccctagccttcttcaaggcctccagggctgggcccaagcgcccgtcgacg
gcaccctgggcccagaggactcgcgggcctcatctccaatg (Seq ID No: 767)
```

Homo sapiens WD repeat domain 13 (WDR13):

```
agttctttctgatagcaggcagccatcttgcctggagcctgagaaagggaggagagacagaaggaa
ccggcgacagtggtctcagggccgctccggggggcctcaagaaccggaggcagccccggaggtggt
ccccgatcccgggctatgctcttggatctgagaagggaaggcggagggcggcggggacaagatggg
tggagaatgtcaagcaaggaatgctaggcggggggaggggcgttgctatggcgactgggggaggggcg
gtgtctgttctgaatcgctgtgtgtcacccgggcgctgcccaggaagggcagggctggggtgatga
ccatggtaacacccggggggggagttcgtgacatctccggcgcggagggactcgatgtctatggcaa
tggtcgcctggtggaagggacggaactagatcccttcgctcgggacgctcacattccaggcccttg
tcctgcaggctgccgcgggcggacacgccagaggaggaggccggggaatg
(Seq ID No: 768)
```

Homo sapiens chromosome 1 open reading frame 123 (C1orf123):

```
ccgccttttacgacgcgccggaaagcaacggcaagggcggcagccagcaccgggcggagagggcta
ccatg (Seq ID No: 769)
```

Homo sapiens chromosome 20 open reading frame 11 (C20orf11):

```
ctgcctccttctactcgggcgccccggcggccgccacctctccccagcccaggagaggctgcggag
ccgcagccgcccagaccgcgcagcgcgggaggcaggttccgcacgaaataaatcagaatg
(Seq ID No: 770)
```

Homo sapiens zinc finger protein 446 (ZNF446):

```
ttcccctttggggacagatcccgaagttcgagcatccctcggataggccgggtgtcaggcctggt
ctctcaggcccgtccaggcccatcttgacgattccaagaccacccccttgagcaagaatg
(Seq ID No: 771)
```

Homo sapiens mitofusin 1 (MFN1):

ccgccctttgccactcccccctgcctcctctccgcctttaacttctcgggaagatgaggcagtttgg
catctgtggccgagttgctgttgccgggtgatagttggagcggagacttagcataatg
(Seq ID No: 772)

Homo sapiens phosphotyrosine interaction domain containing 1 (PID1):

agtcctctcgcagctgcgccaggacagccggcgcgcggccgtgcccacaagttgccggcagctgag
cgccgcgcctcctcctgctcgcagccccctacgcccacccggcggcggtggccagcgccaggacgc
acatcccgcggacaccgaccccagatgtaaagcgggaccccagccctcgccccccggcgcgatcg
acagtctcgccagcgtctcctctgccaaaacccagggctggaagatgtggcagccggccacggagc
gcctgcaggagagatttgcagacacagaagcggcacagagaaggccattgtgaagatcaaggcaga
aaccggagttatggcatcataagccaaggaatg (Seq ID No: 773)

Homo sapiens pleckstrin homology domain interacting protein (PHIP):

tttcctcctcctcctcctccgcctccgccgccgttgcttgaatggtggagccgaagctcggctcgt
gaacacacactgacagctatagggcaggcggcggcaccgtccccgcttcccctcggcggcggggtg
tcccgtcggcggccctgaagtgacccataaacatg (Seq ID No: 774)

Homo sapiens LIM and senescent cell antigen-like domains 2 (LIMS2):

tggcctttttttgggcgtctccctgctccgcggcccgggctggcgggcgggcgctcggctggcggct
gcagcagcagagggagacccgcggcaacccggcaacccagggctcggcgtcgctgccaccatg
(Seq ID No: 775)

Homo sapiens SCY1-like 2 (S. cerevisiae) (SCYL2):

aggtcttttagtcttttcccctcccttactcttcgtccccggtccctcccctccccacccctttt
ccttctagctccgacgtttgcggccgcggggggcggcggaggatatggagtaaagccagagtcagtg
gccaggcacgaaggcagagcaggaacagccaggaggcgtttattaggggggcggggggaaagagcc
ccagcaccgcccctcctggaagaaggaagaggtaagtgaccggccgccggcaccgaccgacctccc
tcaccggcggctctctcgcctgggctcccggagccggcgaggagggaatggaggactcgcgcccgg
gttaggcctcccagggccgctcaggctggtgggtgttgcctggtgacgggcctgccggcggccggc
cgggcgatcggcggtcggcgcccgcgcaaagcggggctggacgagcagcgagctccggggagcgga
tccgagagggccgagtcctcgaaagaggccttgaggcgacgggagacccgggatcgaagtcagctg
ccggagggagagccccccatgccggctcgagagctcgggtttcggtggtggagaacgtagtacctt
tcggggacattggacactactctaggaccgggtaactataactacccaatattgcagccatg
(Seq ID No: 776)

Homo sapiens ring finger protein 31 (RNF31):

caccctctctcctagtacttcctgttctcggctaaccctggcgctgggccggggggctggagagtga
ccgtggtctgagtgacctggggcggctgcgtgggccggggtgggcctcaaagccgggcaccagacg
ggaggggcggcgctcgggccgcgcgctgcccgcgccgggtcctggcgggcggcgaggctggggctg
actcctgcctcaggatg (Seq ID No: 777)

Homo sapiens mediator complex subunit 9 (MED9): cgacctctggctaacc-tacccccggagccatg (Seq ID No: 778)

Homo sapiens ATP5S-like (ATP5SL): cggccccttccggttacgaaaccttagcaa-gatg (Seq ID No: 779)

Homo sapiens GPN-loop GTPase 2 (GPN2):

tctccttttgcgcgacacggtctcagctgttccgcctgaggcgagtgacgctggccgccaacgagg
tatacgtactgggaccctcgccctcagtctcgtctccggcgcggctacctgccccgtttttccctgt
gagttgacctgctccgggccgcgggccgccaatg (Seq ID No: 780)

Homo sapiens transmembrane protein 48 (TMEM48): cggtctcctgtacgccctagactaggggccgccatctccatg (Seq ID No: 781)

Homo sapiens ankyrin repeat and zinc finger domain containing 1 (ANKZF1):

ttgtcctcttcgctgctccgtagtgacgggggattgttgtgttgcagaaatccggcaatcgacctga
ggacttgcgagccgctcagctcccgggacgtttggagctgctgctaaataatttctgctcagccat
g (Seq ID No: 782)

Homo sapiens notchless homolog 1 (Drosophila) (NLE1): ggctctttctcctccacgtggggacgcaggatg (Seq ID No: 783)

Homo sapiens cell division cycle associated 8 (CDCA8):

cgctctctctcactggcacagcgaggttttgctcagcccttgtctcgggaccgcagcctccgccga
gcgccatg (Seq ID No: 784)

Homo sapiens polymerase (RNA) III (DNA directed) polypeptide E (80kD) (POLR3E):

cgctcccccccacgtgtccgccggagtttctccaccagcaacatggccgccgcctgagaggagagc
cgggccgccgccgtctctgcagcccgcggggtaactgggccgttgccgccgtccgcgctcggcccccc
gcggagagatcgagctgaaggactgcgcggctggctctcctctagtatg (Seq ID No: 785)

Homo sapiens armadillo repeat containing 1 (ARMC1):

gagcctttgcccgccagcgccttcgctctttggctccctgagttagtccggttgcttgcgatcgcc
gcggccggggctgcgaaccgaagggctcgctccgcgccgcctgggtctctacctcatccgtaggtg
tggccctgatggtgtggcaggctctggactcctaaagctctggagcgaatttaagattttattcat
gtgcatggcatagaagatg (Seq ID No: 786)

Homo sapiens transmembrane protein 33 (TMEM33):

ccgtctttctggaaacaccgctttgatctcggcggtgcgggacaggtacctcccggctgctgcggg
tgccctggatccagtcggctgcaccaggcgagcgagacccttccctggtggaggctcagagttccg
gcagggtgcatccggcctgtgtgtggcgcgaggcagggaagccggtacccgggtcctggcccccagc
gctgacgttttctctcccctttcttctctcttcgcggttgcggcgtcgcagacgctagtgtgagcc
cccatg (Seq ID No: 787)

Homo sapiens pyridoxamine 5'-phosphate oxidase (PNPO):

ccttccttccccggggtagaagtccagggtgagaaattggttccgaactcaaaggaacccagtgcc
gggccacagccgggtcacgtggccggcggcccccccatg (Seq ID No: 788)

Homo sapiens golgi phosphoprotein 3-like (GOLPH3L):

```
attccttctctgcatcgaaggatcaggaagtttgtgctctctgcgtggctaagttttttcacctact
aggacggggggtggggtgggggagaacaggtgtccttctaaaatacagcacaagctacagcctgcgtc
cagccataacccaggagtaacatcagaaacaggtgagaatg (Seq ID No: 789)
```

Homo sapiens regulator of chromosome condensation (RCC1) and BTB (POZ) domain containing protein 1 (RCBTB1):

```
cgctcctcctcttcgctgccggtgggcaccgccgctcgctcgcacttctgcgcccattggagcttc
ggagatccctgcggtcccgcggggacggcgcggcagcagctgacctcgcagacaggatcttgctctc
ttgcccagactggaatacagtggtgtgaacacggctcactgcagcctcaacctcctggactcagag
atgtcggcttatttataggaattgcttgaagccagagtcatg (Seq ID No: 790)
```

Homo sapiens leprecan-like 1 (LEPREL1):

```
cgtccctttaagagcggctggccaggcacggcctccgcctctcagtacgcggagcgccggcggtca
cctggggctcgcggagcggccagatcgcggcggagtcggcgcgcttccccgagggaaggtgggaga
ggggacccggacgcgaggtgccccgaagccctctcgagcgtaaccgtcccgcgcctctctgaggcg
gaggatg (Seq ID No: 791)
```

Homo sapiens hedgehog acyltransferase (HHAT):

```
ctgtctcttggctcaggcttggaggcctccgagcagcaacatcgtcccaattataccccgttggag
catcttcagatcttccactcttttcacaacgcaatcaaaatcttcgtacccattttgcagtagtga
tctctgtaagttgctttacaattcataaagtttattctatttgatcttcactctaatttacaaaga
aaagcagggaagtctatttctgttttacagaggtgtacagggaggctcacaggggctaagttcaca
cagtaagccctcgaagctgccagggctgcaaagcccaccctctttccaccgcaccgaactacctcc
tttcgcctacaaaacgtaggtggggaccactggtgttggaatgacggcccacctcgagtttcaggt
gacttccactctgcaattaacttgcaggcagccccagacctgcaatgaacacacgggtggggggaga
gatatgcacgccagggtcagtgggaaccaacagccgaggggtgagcggggctaggggccccgggcc
gccggcggggcaaacgcggttcagaaacgcaggccgcgctctggcccgcccctgcagcagcacgg
cctgctcgccatcgcccggagagcgccgcgggttcccgagtccgggcgcggagggcgcgcgggcac
ggcggcaggggcgtgctcggaggacgcgcgctgcgctgctcctccaaagggcagctccggggggaaa
gagggtggcgtcccggggaagcccgcagccgcgcgatgtcgctgggactcggaagtgccgaaag
aggggtgttgggaactcgcggcgcgcgtgaacgttgccgtcgccgccgcccgggacagcccggaga
aactctcagcgtaggcatcgggaaccttcgtgccaaggagccatg (Seq ID No: 792)
```

Homo sapiens chromosome 11 open reading frame 57 (C11orf57):

```
cctccttttttctcccaaaccacttcttcccccctacccccgccacgcgaggctgcggcgcacggt
atgggtgtgtttgtgtgtatttgtgtggggagggcgtttggagggaaggttaccgggagctccgag
gccgctggggaacagggatcccggtgacaaagatggggatatttcctctgtcttccacttggaaac
ctcaaccccgcttcaggctccctagatactttctggggcccaaccgaaggccgtagccatccaaa
gcgttcccagcctttctggggagtgaaacttaccccggggttcgtcctagaggagcgtgagcggg
gaatgcccaggtcaaccgggctgtccgaattccgccccggctcagcctccggcctcagtccgggag
agagatctgcctgtcggtctgggctggggaaacgcggcagtggcctgggccacaggtgagggcag
agtaaccagtgggaaggctgcgttttcacgaaggactcgggtgaagctgcagagctgcctttgagc
cctgactccttggcttcctgggtcggaggagatcttgtaatggagtggttcttcgtctcactagca
agatgcctgatttcctcaggatcaagggattgaagaatg (Seq ID No: 793)
```

Homo sapiens high mobility group 20A (HMG20A):

```
agtccttcgccgcattggggcaaaataatcccttcattttttgtgaaggtaccgtggaaaatatttc
attttttcttctcaccggagcaattgtaaatgctatgcggtaagaggagttacctgtggaaaggtgg
ttaagagattaggtaaagaaaaggaaaggacaccaaaataaagtgctgcggaagaattttttgtcca
gctgtgagacgacgagtgcgtgaagtgaaggcgattgagaggggctgagggaattgtcctctgtgg
aagggactttcttttggccctaggccccttcctgcccctgtcgtcagcagagtctctacaaggaag
ataacggactgtaaaattctataaagcaaagctacacatcacttgacaccatacaccatcttggtt
acataatgaagagagatg (Seq ID No: 794)
```

Homo sapiens checkpoint with forkhead and ring finger domains, E3 ubiquitin protein ligase (CHFR):

```
atgtctcttgacagcggcggcggcgcagccggttccgggttcggcgcggggcggggatgtgaatcc
cgatg (Seq ID No: 795)
```

Homo sapiens nucleoporin 133kDa (NUP133):

```
ccatctcttcccttaggtgtttaagttccgcgcgcaggccaggctgcaacctgacggccagatccc
tcgctgtcctagtcgctgctccttggagtcatg (Seq ID No: 796)
```

Homo sapiens CNDP dipeptidase 2 (metallopeptidase M20 family) (CNDP2): cttccttccaagaaccttcgagatctgcggtctggggtctggttgaaagatg (Seq ID No: 797)

Homo sapiens oxoglutarate dehydrogenase-like (OGDHL):

```
gcaccccttccgcgcagcccccctgacctgcagcctccggacctcgctgcagcgcggacccggcccg
cccgcccgaatg (Seq ID No: 798)
```

Homo sapiens transmembrane protein 30A (TMEM30A):

```
ccgcctcttccgctctacagcggaggtggctgtggcggtggcgctggtggctgcggcggcggcggc
ggcagcggcgctcgagcggttcctgtcagggtcagccggcgggccccctgggtggtccacctgcaa
atcgcggagcggcgccccagggatcgatg (Seq ID No: 799)
```

Homo sapiens elongation protein 2 homolog (S. cerevisiae) (ELP2): gcgtctcttgtttgtgcggctgaccagttggcgacatg (Seq ID No: 800)

Homo sapiens WD repeat domain 12 (WDR12):

```
cgttctttttctttgtatttccgcctctcgcctctctctaaaagccgcagttagaggcgagatttag
gaaaaacctctgccgagtgagcctctggttgggaatatgtatgagaaaaaaaaactggcaaggcgt
tagtcaagcaaagctgaaggcagaggaaatttgatatctggctggagtctagaggatttaatgcaa
ataagatactctgagggcagcgtggcaaaaaaagactacaattcccggtggtcacagcgtttgaga
agcgatgctttctgagacttgtagtaactaggagctgtgtttgaactatccaggctcaggacagcc
tcttgaaaaaaattttttattaataaagcggatttgagtgggatcttttttcctaatcgattacgg
gcccacacgtatgggaagaattctaacaatgattaaagggacatgctacctttacgactatccttt
```

```
tctaatcgatgactcctaaatctaggagtaggtagtcgatgtttgtggtctgggcgtctgtagaag
ggcaacctcgtgctttctgcagaggagaccggagggcagaaggcagagtccaggcttagactgcag
ttcctcgcttacctgtgcagtctaattttgagctgcctctttgtagtcttaaaaggcaggagcttc
gtgttgtgggtctgctaacccgtacgtttccgtgggcaagtcgtgtgtactcctcgccatg
(Seq ID No: 801)
```

Homo sapiens tetratricopeptide repeat domain 17 (TTC17):

```
cgacctcttcaagatggcgggcgccggagactagcttccgcttccggtgtgagcggcccggccggg
ggggcaagatg (Seq ID No: 802)
```

Homo sapiens proline rich 11 (PRR11):

```
ttttctttatggcgtgggagaggccacagcccggactccatcgactcccccggctcttagactaaa
atcatg (Seq ID No: 803)
```

Homo sapiens TBC1 domain family, member 23 (TBC1D23):

```
ctccctctttcttcccctctggggaagctcagtgctggacttccgaagacctttacgacattgag
tctcggagttggtctcagcgccggatccacttttcggcaaagtgacgtggacgtcaacagcaatg
(Seq ID No: 804)
```

Homo sapiens leucine rich repeat neuronal 3 (LRRN3):

```
gctcctctctggggagtggagggtgttcagttattaatgaccgctgagcaggcagcaccatgtcag
tgtgacaactgatcgggtgaacgatgcaccactaaccaccatggaaacaaggaaaaataaagccag
ctcacaggatctctcttcactggattgagagcctcagcctgccgactgagaaaaagagttccagga
aaaagaaggaatcccggctgcagcctcctgccttcctttatatttttaaaatagagagataagattg
cgtgcatgtgtgcatatctatagtatatattttgtacactttgttacacagacacacaaatgcacc
tatttataccgggcaagaacacaaccatgtgattatctcaaccaaggaactgaggaatccagcacg
caaggacatcggaggtgggctagcactgaaactgcttttcaagcatcatgctgctattcctgcaaa
tactgaagaagcatgggatttaaatattttacttctaaataaatgaattactcaatctcctatgac
catctatacatactccaccttcaaaaagtacatcaatattatatcattaaggaaatagtaaccttc
tcttctccaatatgcatgacatttttggacaatgcaattgtggcactggcacttatttcagtgaag
aaaaactttgtggttctatggcattcatcatttgacaaatgcaagcatcttccttatcaatcagct
cctattgaacttactagcactgactgtggaatccttaagggcccattacatttctgaagaagaaag
ctaagatg (Seq ID No: 805)
```

Homo sapiens MIS18 binding protein 1 (MIS18BP1):

```
ggccctctctccgcgcggagccgagccggaactgcggcagtctctccctgccaggctcttcatcca
aggtttctgtggatcccttctgaagttctatctgaaaattgcgcttaagtgaattttctgttagaa
gaacttggttgctactttcttgtcaagatg (Seq ID No: 806)
```

Homo sapiens LMBR1 domain containing 1 (LMBRD1):

```
ccgcccctttaacctttagggtgcgcgggtgcagtatatctcgcgctctctcccctttccccctcc
cctttccccaccccgggcgctcaggttggtctggaccggaagcgaagatg
(Seq ID No: 807)
```

Homo sapiens ST6 (alpha-N-acetyl-neuraminyl-2,3-beta-galactosyl-1,3)-N-acetylgalactosa minide alpha-2,6-sialyltransferase 1 (ST6GALNAC1): cttcctcta-gaacccgacccaccaccatg (Seq ID No: 808)

Homo sapiens spermatogenesis associated 7 (SPATA7):

```
gctcctcttttccagtcctccactgccggggctgggcccggccgcgggaaggaccgaaggggatac
agcgtgtccctgcggcggctgcaagaggactaagcatg (Seq ID No: 809)
```

Homo sapiens docking protein 5 (DOK5):

```
cctcctccttcctcctcctcctccttcttctcctccttctcggccgggaggaggcagggctgg
atccctcagccgccgccgctcctcctcctggcaggccggccgcggagtcagctgacgccggcgctc
cagcctcgcctccccgcgccgcgctctgcgctccccgaaagtggctgcaagccggccgcccactgt
cagggttggggggacagagaaagtgatgtgcgccttctaaagcctcgcccagcgccgccgaagcag
cttcacctctccaactttctcccaccgactgcttgtcttgaccctgccctccaccctccccagagc
cacttcgggtgcgcgctcttgggtaaagggggggtcaccggctgtctgggatg
(Seq ID No: 810)
```

Homo sapiens glycosyltransferase 8 domain containing 1 (GLT8D1):

```
tctcctccatcgcctgcagtaagggcggccgcggcgagcctttgaggggaacgacttgtcggagcc
ctaaccaggggtatctctgagcctggtgggatccccggagcgtcacatcactttccgatcacttca
aagtggttaaaaactaatatttatatgacagaagaaaaagatg (Seq ID No: 811)
```

Homo sapiens cullin-associated and neddylation-dissociated 1 (CAND1):

```
tggccttttgccctagggagcgagtgcggagcgagtgggagcgagacggccctgagtggaagtgtc
tggctccccgtagaggcccttctgtacgccccgccgcccatgagctcgttctcacgcgaacagcgc
cgtcgttaggctggctctgtagcctcggcttaccccgggacaggcccacgcctcgccagggagggg
gcagcccgtcgaggcgcctccctagtcagcgtcggcgtcgcgctgcgaccctggaagcgggagccg
ccgcgagcgagaggaggagctccagtggcggcggcggcggcggcagcggcagcgggcagcagctcc
agcagcgccagcaggcgggatcgaggccgtcaacatg (Seq ID No: 812)
```

Homo sapiens BRICK1, SCAR/WAVE actin-nucleating complex subunit (BRK1): cgctcttcctcaggcggcggccatg (Seq ID No: 813)

Homo sapiens zinc finger CCCH-type containing 15 (ZC3H15):

```
cggtcttcctcctcgtcctgccgcagggccagaacccctgacggtattcagctgcgcgtaagtctg
gccggtgccatctgtctccgcaatg (Seq ID No: 814)
```

Homo sapiens polo-like kinase 1 substrate 1 (PLK1S1):

```
cggtctccttcggcaaccccggccgaacggccacccagaggctgtgctgagctggcgcagcggcag
cagcatg (Seq ID No: 815)
```

Homo sapiens dysbindin (dystro-brevin binding protein 1) domain containing 2 (DBNDD2):

```
gtttctttcctacgcagccgctcctgccgccgtggtcgctggagctttgcctctctaggccggcag
cgcctctcctccatggtcctgtctgtcagcgctgttttgggagcccgccggtgaggccgggccacg
ctcagacacttcgatcgtcgagtctgtcactgggcatg (Seq ID No: 816)
```

Homo sapiens KIAA1704 (KIAA1704):

```
gattcttttggataggggttgacgttcgtggatagactcatatctgtgaccagtgtccgccaccgc
ggatg (Seq ID No: 817)
```

Homo sapiens solute carrier family 25, member 37 (SLC25A37): cccctccctgcccacctcctgcagcctcctgcgccccgccgagct-ggcggatg (Seq ID No: 818)

Homo sapiens myoneurin (MYNN):

```
cgtcctcccaagatggcggagacagagtgaagaaactgtgttcccccctcggggttgctatcgatca
agggtaaaattccattctgatatcaaaatg (Seq ID No: 819)
```

Homo sapiens vacuolar protein sorting 33 homolog B (yeast) (VPS33B):

```
gcttctttttctggtagaaggcggggttctcctcgtacgctgcggagtctctgcggggtgtagacc
ggaatcctgctgacgggcagagtggatcagggagggagggtcgagacacggtggctgcaggtctga
gacaaggctgctccgaggtagtagctctcttgcctggaggtggccattcattcctggagtgctgct
gaggagcgagggcccatctggggtctctggaagtcggtgcccaggcctgaaggatagcccccttg
cgcttccctgggctgcggccggccttctcagaacgaagggcgtccttccaccccgcggcgcaggtg
accgctgccatg (Seq ID No: 820)
```

Homo sapiens zinc finger, C4H2 domain containing (ZC4H2):

```
aggcctctccaagcccctaccgcacaggctcatagccccaagcccggaggaggtggctacattgtg
tctattgtatcccttggctggtgtatttgtacatctctcgggacgtgaaattgacagtgaaaagta
tg (Seq ID No: 821)
```

Homo sapiens BAI1-associated protein 2-like 1 (BAIAP2L1):

```
cttcctctggcggcgtccggccgcttctcctctgctcctcgaagaaggccagggcggcgctgccgc
aagttttgacattttcgcagcggagacgcgcgcggggcactctcgggccgacggctgcggcggcggc
cgaccctccagagccccttagtcgcgccccggccctcccgctgcccggagtccggcggccacgagg
cccagccgcgtcctcccgcgcttgctcgcccggcggccgcagccatg (Seq ID No: 822)
```

Homo sapiens solute carrier family 25, member 40 (SLC25A40):

```
cgtccttctcgcgcctcgctctggccctgcaggttgtgtttccgcctctacccgcctccattccg
ttgctctctcagtctcagacccgggctctcggtccgccgcttcaggtcttggcgcagcctcagaga
gttggcgcggctctgtgttgaccaaacctagtggatgcagttagcgccggagcccggccccgcccg
tcaccagggttattcccgccttctaggtttgccaggactgccggccctgcagctgccttctgcccc
aggttttggctactgatgttacaaacaataaaatattggagcatagagttgaagaacagactcaa
accaggttttatttaattagttaaaatatg (Seq ID No: 823)
```

Homo sapiens protocadherin alpha subfamily C, 2 (PCDHAC2):

```
tttccttttccctccccctggagctgtagcggcagcagcagcaggaagccgagccgggttgagcga
ctcggaggcgagcggaggagctggaatatggggagtcagcgaggacggtggggccaggagcccttg
ggagggcctacggagggagcggcccccaggcgctttctagagcgtgagcggtgggggagcaggcgca
gggtggcacgagcggaggcggggcccgggcgtggggcacggctggggaagctgccgcctccggccc
tgcccggctgcctccgccgcggccagtggctatg (Seq ID No: 824)
```

Homo sapiens chondroitin polymerizing factor 2 (CHPF2):

```
gttccttttgggttagctttggcagtattgagttttacttcctcctcttttagtggaagacaga
ccataatcccagtgtgagtgaaattgattgtttcatttattaccgttttggctgggggttagttcc
gacaccttcacagttgaagagcaggcagaaggagttgtgaagacaggacaatcttcttggggatgc
tggtcctggaagccagcgggcctcgctctgtctttggcctcattgaccccaggttctctggttaaa
actgaaagcctactactggcctggtgcccatcaatccattgatccttgaggctgtgcccctggggc
acccacctggcagggcctaccaccatg (Seq ID No: 825)
```

Homo sapiens thioredoxin-related transmembrane protein 3 (TMX3):

```
gcttctcttccgctccgggtcggctccgtttcccttttccgggcgggcaggcggcggacccccagtgt
ctttatccctcttttgcacagtcagcttctgcagctctcccgggctagcatg
(Seq ID No: 826)
```

Homo sapiens ras homolog family member F (in filopodia) (RHOF): cgacctcttggctccgctagtgcccggcgcgccgccgccagtgctgcgggctccgggcaatg (Seq ID No: 827)

Homo sapiens amyloid beta (A4) precursor protein-binding, family B, member 1 interacting pro tein (APBB1IP):

```
ctttctctcaggaaactccactcccaactgacaggtgctatttccagccagtcctatgctgttgca
```

```
aatagtgagtccatgaatgccctctgccgtgtgcattacttattttcatcagcagatcttcgtaac
acactcctggaagtggggatgacggggtcaaaaggcgaatccatacataagttaaatagatattgct
caattctcttccacggggttcagaccattttggatttctacgagcaatgaagacagtgctattcct
ctacaccctggccggccaactgagcgtggttaaacgtggggaggggaggagggtgaggttaccaacc
tgatggttgagaaagggcctccgcccagcgcgcccttcctccaccccacccgagagacagctgaa
ctccggccgggacgcgcgtgttgccagtccagccctgcaccgcgtcccctgagggcgggctgcagg
cggccgggaagccttgcacaaccggcccaaaagaggaagcccagaaagtgctgaagtaaacacttt
gggagaccgttgcaacataaagcggcctctcagtctttggtggaaccatcactaggccccaatccc
ttagtccctcttgcgtcgaggctgcaaaatggttccattcgccaggagacgctcctgagagaaggg
cgcgcgcggcacaggggccttccttgcacctcggagcaaagcagctcggatagcgccacacgtctg
cgcgctgcgtgggaagggcagggctgacagcacttcctcccggggcagcgacctggagcccgggt
gcggcagtctgcaccgcgcgtcgctttcccggccggagtctcgccgccttcccgcgccccgcagcg
ccccgcagagcagtcgagatg (Seq ID No: 828)
```

Homo sapiens roundabout, axon guidance receptor, homolog 4 (Drosophila) (ROBO4):

```
ccttccctcttcactgtgagctcagagcagcaggacaaagtgctcgggacaaggacatagggctga
gagtagccatg (Seq ID No: 829)
```

Homo sapiens translocase of outer mitochondrial membrane 7 homolog (yeast) (TOMM7):

```
acctcctttccctttcggattcccgacgctgtggttgctgtaaggggtcctccctgcgccacacgg
ccgtcgccatg (Seq ID No: 830)
```

Homo sapiens major histocompatibility complex, class II, DR alpha (HLA-DRA):

```
ttttcttttattcttgtctgttctgcctcactcccgagctctactgactcccaacagagcgcccaa
gaagaaaatg (Seq ID No: 831)
```

Homo sapiens protein arginine methyltransferase 8 (PRMT8): cctcctctactatctcggtatcaccaaacccttgccggctcttatg (Seq ID No: 832)

Homo sapiens adducin 3 (gamma) (ADD3):

```
ctgcctcttatgaagcaatactagagaggaaaaacaaaacccattcctttaagaaagattccgcct
cctctcataagcaagcgcctaatggtaattgtagagtttactaagtcaaacacttactactcagca
ttgagagaagctgctgctaatgctgctgctgctgctgccgccgccgccgctgctgctgctgct
gttggtctgaggctgcagtaggtttctgtgcagcattgcagaatccacacctagagaacagaagac
acagacacgtacgtctactaccttgttagaaggaagctttggatcttcggtggataacaagagta
atccacagacttaaaacatg (Seq ID No: 833)
```

Homo sapiens BarH-like homeobox 1 (BARHL1):

```
agccctttggatctaatgcgcagaggaggttggcccagagctcccgggctcccccaaggctgaac
tccgtccaaggtgcccgcaggctccctgcccgccttccccatgccagcccgcagctaggggcaggg
gcagcggcggctggggttgggggtgggtggggagcttttggggaggacaggtcgcagcttggctat
g (Seq ID No: 834)
```

Homo sapiens intraflagellar transport 46 homolog (Chlamydomonas) (IFT46):

```
ttatcttttgcctagcgactgacaacaggctggttgcttggcgtggaatcctaaagtggcctggc
tttgagactggagtgagaccccagccctaggctggggttctttccattatagaggagacggattca
gaagggctacagaccaaggttgttgaaaaccagacatatgatgagcgtctagagattaacgactcc
gaagaggttgcaagtatttatactccaaccccaagacaccaaggacttcctcgttctgcccatctt
cctaacaaggctatg (Seq ID No: 835)
```

Homo sapiens carbonic anhydrase X (CA10):

```
ccccctttcgggaggagggaggcagggacttgcaggcaagagttgcacctggtctaggaacctgc
agagaaaagaactctggggtaagtagtgttctggcactggcacggaaaggggtaaagggtggggggg
catgagagggacgaaatggagagggcagggaatgaattatgcaaaaaaatctccaatatttcgcag
cggagggagagcacagcacagcactcccaggatgagtcctgcctgggtctcccgcgccgaacccgc
agcacgaagttctttttaagaagagaaactcgaaaatcctggagggtaacagaggcagccagggcg
gggcggagtgcggaggcggctgccagggactggggccgaggcggcggccaaggtggcctgaagctg
tgacacccagcctcctcctcctcctcctcatggccgcgctcagcctcacctccccgcccgggcctc
ctgcctccgcccccgggtgccgggctgcggagctgacgctgggacgcccggcggcggcgaggacgc
tcacctggccaagcctccttctcctcctccccctcccgcccccacctgtcctcctcctctctgagt
tgggaagcgtagggatccgtaggcgaggaaataacgacccctgcagttgtattgcggaaaatctcg
acagcggcgctagttgcgggcgatggaagccaggcaactgggggttctggggagttcaggaaaata
gcagaggagcaggaagggcgcgcgcgacctggagagtctgtgtgcccccaccgcgccccagtcccc
ggggcccagcccttcccctcggcgccctggacgcactgccggaacccggctgagaggctgcaggct
gcgcgcggacctggggagcagggagggtcggcggaggctgccggcggctggcggtttcgggcaata
atccctgcctctctttctctgtgtgtctgctgtgtctgctccttccccgccccccggaagcaggag
aagaactgccccggagcgcagcagccaccctccgaccatgccccggtgagggggggcggacttcgag
ggcaacttgccgcggactgcctgggcttagccagcgagctacgcgctcccgggagcccggaattgc
acggcgcagcccggcggggggctatcgtctatgtcttcttggggcgccagacgaatcggggtctcg
tttttgctggaagagcccagtgttggtggcttcaggtggctgctgccgccgccgccgccgccgccg
ctgctagtgcggtttccgccgctggtgcgaagagaagagacacgcgagcggggagacctccaaggc
agcgaggcatcggacatgtgtcagcacatctggggcgcacatccgtcgagcccgaggggagatttg
ccggaacaattcaaactgcgatattgatcttgggggtgactgtccctggccggctgtcgggtggga
gtgcgagtgtgcactcgctcggaagtgtgtgcgagtgtgtatgtgtgtgtgccgtgtcgggctccc
cccttccccccgttttcccgtcgagtgatgcacttggaatgagaatcagaggatg
(Seq ID No: 836)
```

Homo sapiens dual specificity phosphatase 22 (DUSP22):

```
cctcctccctgtaacatgccatagtgcgcctgcgaccacacggccggggcgctagcgttcgccttc
agccaccatg (Seq ID No: 837)
```

Homo sapiens olfactomedin-like 3 (OLFML3): gttccttctactctggcac-cactctccaggctgccatg (Seq ID No: 838)

Homo sapiens phosphoribosyl transferase domain containing 1 (PRTFDC1): ccgtcttcccttcccgcgttccccgggagaaacatg (Seq ID No: 839)

Homo sapiens translocase of outer mitochondrial membrane 22 homolo g (yeast) (TOMM22): cctcctttccgcttccggt-gtccctacagtcatg (Seq ID No: 840)

Homo sapiens arrestin, beta 1 (ARRB1):

```
gctcctcctgctggctgggggatttttccagcctgggcgctgacgccgcggacctccctgcgaccgtc
gcggaccatg (Seq ID No: 841)
```

Homo sapiens cytokine induced apoptosis inhibitor 1 (CIAPIN1):

```
cctcctctcgcgagaggcgcaaggcgtggagtcgacggctggagagaagccgggagcgagcccagg
cggcagtcttgattcccttttggccagcagttttttaggtctgtcagtactgcactgcaagaatg
(Seq ID No: 842)
```

Homo sapiens leucine zipper transcription factor-like 1 (LZTFL1):

```
taccctccttccccattttctgtggtccaactaccctcggcgatcccaggcttggcggggcaccgc
ctggcctctcccgttcctttaggctgccgccgctgcctgccgccatg (Seq ID No: 843)
```

Homo sapiens phospholipid scramblase 4 (PLSCR4):

```
agccctcccttccgcgcgcttactttgtttataacttgaaaaatcctctccgtctcccttccctgc
ctcctttccttttcccttttcctctgccagtacaactagacccggcgtctggcgtccccggtgcccag
cattctgcggggcaggcggattaattggaattcttcaaaatg (Seq ID No: 844)
```

Homo sapiens ectonucleoside triphosphate diphosphohydrolase 7 (ENTPD7):

```
cctccttccggctgggcaaggggccgcggggagcagctcgggactgaaccgagaggtgccgaagga
accggcgggccgcttgatcccgctgcagacgtaggagatgcctgggacaaggaggccaccttctca
gggcaaaagaaaaagaaggtgacaggcgttgagaccaccgaagggaacccatg
(Seq ID No: 845)
```

Homo sapiens fascin homolog 3, actin-bundling protein, testicular (Strongylocentrotus purpuratus) (FSCN3):

```
agttctctctgggaacatctggtgggtactacaggccctattccaggccctatggcctgtggaacc
tcaccacgggggggggagggctgggccagacggagacatcacctgtggtgtcagccccatg
(Seq ID No: 846)
```

Homo sapiens X-prolyl aminopeptidase (aminopeptidase P) 1, soluble (XPNPEP1):

```
cctccttcgcgccggcccttccgcgggtgatcagctggtctgcgctcccctgacgtgggctggggc
acgtcaccgccgaatg (Seq ID No: 847)
```

Homo sapiens REX4, RNA exonuclease 4 homolog (S. cerevisiae) (REXO4):

```
gggtctcttccggagtcttttcctggacggggtccctgcggtgggtgtgtttcggcctggcctggg
caggcgcttgtgctgccagggcgccgggcccggggaggccggggtctcgggtggccgccggcccag
gcgctggacggcagcaggatg (Seq ID No: 848)
```

Homo sapiens LYR motif containing 4 (LYRM4): ttttctttccaaaatg (Seq ID No: 849)

Homo sapiens DEAD (Asp-Glu-Ala-Asp) box polypeptide 24 (DDX24): ggttcttcactcgcgactgacggagctgcggtggcgtctccacacgcaaccatg (Seq ID No: 850)

Homo sapiens transmembrane protein 159 (TMEM159):

```
ccttcttcctcttgttcctcctcctgcctctcttcgcttcgcctgcaaacgcggtgggggctgctc
ggcggtcaggagcaggttaccctccgtctgcatgcccaccatcaaggtatgaggatggtagaagct
ctcgtcgaaccagatggatgaagaccactaacggcttttgtttcctctggtaacagcaagagacag
agcgacatgagagattggaccgcgggctgcactggagaatttactggtaggataattcatccctaa
agagattgaagtgagcttcagaatg (Seq ID No: 851)
```

Homo sapiens NDRG family member 4 (NDRG4):

```
cggcctccgcccctgcagccgcgggcacgcggaggggctcctggctgcccgcacctgcacccgcgc
gtcggcggcgccgaagccccgctccccgcctgcgcgtctgtctcgtccgcatctccgcggcctcct
gctccacgacgtgaccatg (Seq ID No: 852)
```

Homo sapiens pre-B-cell leukemia homeobox interacting protein 1 (PBXIP1):

```
ttttcttctcgggctgcaaacaaagggaagcctgcaacaagttaagctgaagaccgaagcaagagc
tggttcaggtggcagccacagcagcctcagggacctcagcaactatg (Seq ID No: 853)
```

Homo sapiens twisted gastrulation homolog 1 (Drosophila) (TWSG1):

```
ctgtctctttaaggtgcccgaggctcgcgggcgctgcgctgaggggacggcgggaggcgcggcctg
gcctcgcactcaaagccgccgcagcgcgccccgggctcggccgaccggcggggatctaggggtgg
gcgacttcgcgggaccgtggcgcatgtttcctgggagttactgatcatcttctttgaagaaacatg
(Seq ID No: 854)
```

Homo sapiens zinc finger protein 286A (ZNF286A):

```
gtcccctttgtgaggcccgggatgggaggtgcccggttcccccagggacagcttcaagcggtaggg
acagacatctgaggacccagcctcagggatgctgtccccgggcttccaggctccagcgccgtagga
ctgaggcagactccacggtgagaaagagacccgatctaacccaggcctttcatcagagcccaggag
ggaaggcaggaagtgggaccacgaggcccggggggcttctaactcgtctggccagggagatctgaa
ttgggggtgaagagcagaatctccagaacaaggaggaggtggtgatcatg (Seq ID No: 855)
```

Homo sapiens S100 calcium binding protein A14 (S100A14):

```
gctcctcctgtcttgtctcagcggctgccaacagatcatgagccatcagctcctctggggccagct
ataggacaacagaactctcaccaaaggaccagacacagtgggcaccatg (Seq ID No: 856)
```

Homo sapiens ANKHD1-EIF4EBP3 readthrough (ANKHD1-EIF4EBP3):

```
tgctcttctcgttcccgagatcagcggcggcggtgaccgcgagtgggtcggcaccgtctccggctc
cgggtgcgaacaatg (Seq ID No: 857)
```

Homo sapiens KIAA1143 (KIAA1143): ctgtctttacccagagctaccatg (Seq ID No: 858)

Homo sapiens neuroligin 4, X-linked (NLGN4X):

```
ctctcttttcttgcagaaccgtctctctcccttctctgtctcttagcacagagctcttattcagc
cactagcttggcccttcctgcttcaattgtaatgcttgttctgcccgtccacagactattggcggc
agaaacaacgaatttcctccaaactaggcggtgttggtggctcttgcattcctctggatgaggaaa
tctagttggggggttccagaagggggaaggctcctgggctttcaatacatcctcctgaatcatacct
cgtttcgggttccctagaaaaatctggacgtgtaaaaagaactcttaacggccgatgcagctcttc
caaagctaaggctgccttggagttttcataagaaattgtccctggaggtgttggatgatcacagct
tccttggagcattgcagttgctggaatccagtttcaggattaagggagggctgcctccttgcaatg
ggctgccaagaaaacggctgtgcttgttcttaacctcaggctctgtctgtgatcagtctgagagtc
tctcccaggtctactgctccctggaaagccctatctctctgcaggctcgcctctgggctttgtctc
cttggagccacatcactgggacagctgtggatgtggatgcagatttgaaccatg
(Seq ID No: 859)
```

Homo sapiens mitochondrial antiviral signaling protein (MAVS):

```
ccgcctcctcgctgcgggaagggtcctgggccccgggcggcggtcgccaggtctcagggccggggg
tacccgagtctcgtttcctctcagtccatccacccttcatggggccagagccctctctccagaatc
tgagcagcaatg (Seq ID No: 860)
```

Homo sapiens serine incorporator 1 (SERINC1): ctgtctccatcttgtctgtatccgctgctcttgtgacgttgtggagatg (Seq ID No: 861)

Homo sapiens KIAA1324 (KIAA1324):

```
cctcccctttttttccgccttctgccagcagaagcagcagccgcagcacctgagccgctactgccg
ctcactcaggacaacgctatg (Seq ID No: 862)
```

Homo sapiens synaptotagmin IV (SYT4):

```
ggacctccctctttgcctcctccctgttccaggagctggtgccctgggctctgcgctgttgttttc
agcgctccgaaagccggcgcttgagatccaggcaagtgaatccagccaggcagttttcccttcagc
acctcggacagaacacgcagtaaaaaatg (Seq ID No: 863)
```

Homo sapiens pyruvate dehyrogenase phosphatase catalytic subunit 2 (PDP2):

```
cttccttctggagctgggtcctgactagggaccgcctgggtgaggtgaggacctggtggccgcagt
tgtggcactgtgcgcaggcgctgaactgaccggacggagcgggcggctgtggcctcgccagctggt
ttaaaaatatcctttttttgctgaaggaacacatttgctggtatagtttcagaatg
(Seq ID No: 864)
```

Homo sapiens gephyrin (GPHN):

```
ctatccttttcctctcagtcctgccatctagctgccttgggtctcgcgctccgcagagcgttccgac
actctccggcctcgttctgccgcctccgcgcgctctccccgtgcggccaccgcgcccccaagctt
gcctccttcttgccggacttggggccgcgcgccctgactccttccctcccgcggacccgcgcact
cccggcgcggcctctcccccacgcaggccaccgtgcactctgtggcctcccctccttccccgctc
tcctcgcgcttctctggctccctagctgtcgcgctctcctcggcgagcgcgctcccggcccgcgcg
ctccgggctccggtttctcccggctcctgtcagtgcggtgactgcgctgggaaacatg
(Seq ID No: 865)
```

Homo sapiens deltex homolog 2 (Drosophila) (DTX2):

```
ccttctcctgagagtcggagccacagccagagccctgcccaggccgagccggagctgcagcccgag
cgcggtggtgccctcagccccgtcctcttgtcctcctcagcctcggtgccttggaatttgtgtcgc
tgagtcagcaagcctttcagatttgcccggttttttgttgtttgtggtttgtatcaagatgggaact
caaacaagtcattcctcctaaggagctggtgtcttcatccagaagggacagtttgtgccagctctc
cagagagaaaaggatctggtactgttctggagtggcctgtagcagacactgaaccaccagccagct
gcatttgttgtcctggaagtcattgccaactctgccagtcacactggggtccccagagaagtcaag
atctgccggaggcgctgggcaatgaccccgggactccaggccagagggggtctgaagctgtttggga
aagcagcgggactccttgggaagatg (Seq ID No: 866)
```

Homo sapiens melanoma antigen family E, 1 (MAGEE1):

```
ctgccttttttcaccacctctaattttcagcttcagcagttgcttggaactttggttctggcagcagc
agcaacatcattaccgctagcggcagttttgtgccgaggcacctacacacctcccgtcctctctgc
cagatcgcgggcctgtcggtgtctgctcctacacgccaacgccggtgggcaggaccatg
(Seq ID No: 867)
```

Homo sapiens G protein-coupled receptor 107 (GPR107): cgccctttcaccccggacgtgggcgggagaggaagcggctggtgatgctggaacaaacatg (Seq ID No: 868)

Homo sapiens PDZ and LIM domain 1 (PDLIM1):

```
cgctctttctccgacagctgccggggggtgccctgcaagctgttccgcgcgtcctgcccgtctgtcc
ccgcgggtcgtcgcccgccacagccgcgccatg (Seq ID No: 869)
```

Homo sapiens thymosin beta 10 (TMSB10):

```
cgctcttttgtttcttgctgcagcaacgcgagtgggagcaccaggatctcgggctcggaacgagac
tgcacggattgtttttaagaaaatg (Seq ID No: 870)
```

Homo sapiens phospholipid scramblase 1 (PLSCR1):

```
agaccctttttcagacccttttccggctgacttctgagaaggttgcgcagcagctgtgcccggcagt
ctagaggcgcagaagaggaagccatcgcctggccccggctctctggaccttgtctcgctcgggagc
ggaaacagcggcagccagagaactgttttaatcatg (Seq ID No: 871)
```

Homo sapiens eukaryotic translation elongation factor 1 beta 2 (EEF1B2):

```
gggtccttttttcctctcttcagcgtggggcgcccacaatttgcgcgctctctttctgctgctccccc
agctctcggatacagccgacaccatg (Seq ID No: 872)
```

Homo sapiens pyrophosphatase (inorganic) 1 (PPA1):

ggctctctccttgtcagtcggcgccgcgtgcgggctggtggctctgtggcagcggcggcggcagga
ctccggcactatg (Seq ID No: 873)

Homo sapiens X-ray repair complementing defective repair in Chines e hamster cells 5 (double-strand-break re-joining) (XRCC5):

ggctctttccgctatctgccgcttgtccaccggaagcgagttgcgacacggcaggttcccgcccgg
aagaagcgaccaaagcgcctgaggaccggcaacatg (Seq ID No: 874)

Homo sapiens GATA zinc finger domain containing 1 (GATAD1):

gatccctttcccagtcctgcttcccagtgcctcgggccagggaatcctggcctccgcctgcggagc
cggcggaacccgcttcccgcctccacggggcagcgccagcggcctggtcctttcaccggcagctcc
gtgccgacgctctcaccgctcttcctatcgccgggagtggcgggccgaccaggggggcggccgggct
accgtccgccattcccgtgtctctgcgcccgcggggggccgcccgagccggccaccatg
(Seq ID No: 875)

Homo sapiens enolase-phosphatase 1 (ENOPH1):

ccgccttttccagttccaggtgtgcagaagtgtcctctccccacgcgcggcgggctgcacttggtc
gctggctccgagatcgcgcgcggggccgccggaagcccaagacggtaccggggggccgcagccgcagcc
ggcgccgccctccgccctccccaacagcaggccgagtcccgtagcatccggtagggaaatg
(Seq ID No: 876)

Homo sapiens regulation of nuclear pre-mRNA domain containing 1B (RPRD1B):

agctctttccggggggcccggggaactactctccttgcctcgctctgtctccttcgaagtgctctgc
gcgaggttcagagcggccgccgcctccaaagggacggttttctagagctccgacgcctctcggtgc
ccctctgctccggcccttgccctttgacctcgctctcgcggcagggtgagaggtcgggtggccatc
ttgtggcggcggcgcgggcggctgttactgcggagacccatcccctcccccttctcgcaccccctgg
cagtctgtcagtcggtaaaaagtcccgcagcctgtcaggtgaggccccggcctcgtgccgtcgctc
ttcccgccgcactgggcggcccaggccgctccctgccgggcctcactgccgccaccatg
(Seq ID No: 877)

Homo sapiens family with sequence similarity 60, member A (FAM60A):

ctatctttctagacaaggcagttgaggaggagggagcgcttgagggggactggcctggcgtgcact
ccgcacctcggggacattattgcgcgtggaacggctgcttttggaaggcacaacttcctgaatgga
ccatgactcccaccaaagatccctgtctctgattcaccaaacagcttcaaccctgaaaccaggacg
agaagttgacaacatctgagtggacagctaattgacctaagacttcagaccagactattgcccaga
agaaaagatg (Seq ID No: 878)

Homo sapiens MID1 interacting protein 1 (MID1IP1):

gggccttttatctcggtgctgccggggggaggcgggaggaggagacaccaggggtggccctgagcgc
cggcgacacctttcctggactataaattgagcacctgggatgggtaggggggccaacgcagtcaccg
ccgtccgcagtcacagtccagccactgaccgcagcagcgcccttgcgtagcagccgcttgcagcga
gaacactgaattgccaacgagcaggagagtctcaaggcgcaagaggaggccagggctcgacccaca
gagcaccctcagccatcgcgagtttccgggcgccaaagccaggagaagccgcccatcccgcagggc
cggtctgccagcgagacgagagttggcgagggcggaggagtgccgggaatcccgccacaccggcta
tagccaggccccccagcgcgggccttggagagcgcgtgaaggcgggcatccccttgacccggccgac
catccccgtgccctgcgtccctgcgctccaacgtccgcgcggccaccatg
(Seq ID No: 879)

Homo sapiens transmembrane protein 35 (TMEM35):

ctctccctttgtcattctagctgcctgctgcctccgcagcgtcccccccagctctccctgtgctaac

tgcctgcaccttggacagagcgggtgcgcaaatcagaaggattagttgggacctgccttggcgacc
ccatg (Seq ID No: 880)

Homo sapiens Fc fragment of IgG, low affinity IIa, receptor (CD32) (FCGR2A): cttcctcttttctaagcttgtctcttaaaacccactggacgttggcacagtgctg-ggatg (Seq ID No: 881)

Homo sapiens tribbles homolog 2 (Drosophila) (TRIB2):

ctttctcttttttgtttggcttctaacgcgttgggactgagtcgccgccgtgagctccccgaagact
gcacaaactaccgcgggctcctccgccccgtctgcgattcggaagccggcctggggggtcgcgtcgg
gagccctggcgctgcagctccgcaccttagcagcccgggtactcatccagatccacgccgggggaca
cacacacagagtaactaaaagtgcggcgattctgcacatcgccgactgctttggggtaacaaaaag
acccgagttgcctgccgaccgaggacccccgggagccgggctcggagcagacgaggtatccggcgg
cgcccatttggggggcttctaactctttctccacgcagcccctcttctgtcccctcccctctcgctc
ccttttaaaatcagtggcaccgaggcgcctgcagccgcactcgccagcgactcatctctccagcgg
gttttttttttgtttgtcgtgtgcgatcctcacactcatg (Seq ID No: 882)

Homo sapiens family with sequence similarity 3, member A (FAM3A):

cgtcctctccggggggcggagcgggtcggcgggcctgacagggaacctccctgaccgagcccacgtc
tccccacggccagagaaatctccggcccggccgcatcgccagccccccaggcccggaggaacggcc
cgagcccaggagaaccacatcttcgtcccagccccggaggctcctgtgggcaagatcgtgagccaa
cgggttcctgaggcccctcctggccaggcagggtttccccgcgcgtttccgaggagccctgcctgg
ccgggcggctggacaaacaggtcgtagcaccgatcgcgcccgcgccccagcaggggtcccgcacagg
cttgcccctgacccccacccaaacctgtccttccgctttgcccccaaacagtgcacttgccggcgg
tcccaacccagcaggagaagtggacatg (Seq ID No: 883)

Homo sapiens exocyst complex component 4 (EXOC4): ggctctccccgcgtccaagatg (Seq ID No: 884)

Homo sapiens ELOVL fatty acid elongase 5 (ELOVL5):

gcgccttcctcttcccatcgcgcgggtcctagccaccggtgtctccttctacatccgcctctgcgc
cggctgccaccgcgctccctccgccgccgccgccttgctgctgctcaaagctgctgccgcccctt
gggctaaaaggttttcaaatg (Seq ID No: 885)

Homo sapiens apolipoprotein B mRNA editing enzyme, catalytic polyp eptide-like 3G (APOBEC3G):

ctttctctttcccttgcaattgccttgggtcctgccgcacagagcggcctgtctttatcagaggt
ccctctgccaggggggagggccccagagaaaaccagaaagagggtgagagactgaggaagataaagc
gtcccagggcctcctacaccagcgcctgagcaggaagcgggaggggccatgactacgaggccctgg
gaggtcactttagggagggctgtcctaaaaccagaagcttggagcagaaagtgaaaccctggtgct
ccagacaaagatcttagtcgggactagccggccaaggatg (Seq ID No: 886)

Homo sapiens gamma-aminobutyric acid (GABA) B receptor, 1 (GABBR1):

gctcctcctcctcccctccgtcggtcagtcagtccgcgaggagagtccgcggtggcggcgacggtg
gcgagagccgcggggggccgtaggaagccaaccttccctgcttctccggggccctcgcccccctcctc
cccacaaaatcagggatggaggcgcctccccggcaccctcttagcagccctccccaggaaaagtgt
cccccctgagctcctaacgctccccaacagctacccctgcccccacgccatg
(Seq ID No: 887)

Homo sapiens cofilin 2 (muscle) (CFL2):

cctccttctcctcccagtgccacagagccgaagcccgagctgccgccgcagccacagccgagggca
ctatg (Seq ID No: 888)

Homo sapiens DEAH (Asp-Glu-Ala-His) box polypeptide 35 (DHX35): tgaccttttaccccaacatg (Seq ID No: 889)

Homo sapiens resistance to inhibitors of cholinesterase 8 homolog A (C. elegans) (RIC8A): ccgccttccccggcgcgccatg (Seq ID No: 890)

Homo sapiens FK506 binding protein 10, 65 kDa (FKBP10):

agttctttgtagtgcctccctcagactctaacacactcagcctggcccccctcctcctattgcaacc
ccctcccccgctcctcccggccaggccagctcagtcttcccagcccccattccacgtggaccagcc
agggcggggggtagggaaagaggacaggaagagggggagccagttctgggaggcggggggaaggagg
ttggtggcgactccctcgctcgccctcactgccggcggtcccaactccaggcaccatg
(Seq ID No: 891)

Homo sapiens small ArfGAP 1 (SMAP1):

cctcctcccgttccagctgccgctgccgcttcctgggctgagtccgcccgcggtcccggcggcgcc
aggtgcgttcactctgcccggctccagccagcgtccgccgccgccgtagctgccccaggctccccg
ccccgctgccgagatg (Seq ID No: 892)

Homo sapiens chromosome 14 open reading frame 93 (C14orf93):

cctcctttttgcacacacacgaatacaaagagccatacgaccttcggatgccggaaggtccttctg
aatcccttccctgttccttaggttgcactagtcggggggttccatgctggggggcagaaggaatgct
ctctaccgtctgaaaccgttcatcaggaaggccttgatttgtgatgtgctaggagagcacaggatc
tgcaaatagaaggcacctgtctcccttctgcaggccgaggagaggccgccatggactgtgtgcttc
ttcatggcttgtttactcttcttttcacagaccctacagcttggggcctgggctcctctgaccatcc
tcattgagaaaggaaagtgagtccagagaagttgatgcttcctacctgttggagcggcccagcagt
gtaagcgtggttgttactgccccatccgccatg (Seq ID No: 893)

Homo sapiens brevican (BCAN):

```
cgccctcttccgaatgtcctgcggccccagcctctcctcacgctcgcgcagtctccgccgcagtct
cagctgcagctgcaggactgagccgtgcacccggaggagaccccccggaggaggcgacaaacttcgc
agtgccgcgacccaaccccagccctgggtagcctgcagcatg (Seq ID No: 894)
```

Homo sapiens H2.0-like homeobox (HLX):

```
cggcctctcttcctcagtgcgggcggagaagcgaaagcggatcgtcctcggctgccgccgccttct
ccgggactcgcgcgcccctccccgcgcgcccacccacccagtccggctggactgcggcagccgcgc
ggctcaccccggcaggatg (Seq ID No: 895)
```

Homo sapiens v-rel reticuloendotheliosis viral oncogene homolog A (avian) (RELA):

```
ccgcctctggcgaatggctcgtctgtagtgcacgccgcgggcccagctgcgaccccggccccgccc
ccgggaccccggccatg (Seq ID No: 896)
```

Homo sapiens zinc finger protein 277 (ZNF277): cctccctttctttctgccgggtaatg (Seq ID No: 897)

Homo sapiens globoside alpha-1,3-N-acetylgalactosaminyltransferase 1 (GBGT1):

```
cttcctcttttctgtctggcccgcggccccgctgcctgccctgctccaggctccacctgcgccgcc
gatcgcccgggtatcgcggggggcccaggccagctgagtccgttttccgcgccggggtggcgcccct
ccaaccgtcctaacgccgggccggcagcaaggagtgttcctgggacctcagagaccaggctcagag
cctgacatccctgcgaggggacagcctcatccgcccaggccagtgggggtctctacaagtgcccag
gctcaggtgcagcccccagcaatg (Seq ID No: 898)
```

Homo sapiens FXYD domain containing ion transport regulator 6 (FXYD6): ggtcctcctgggagtctcggaggggaccggctgtgcagacgccatg (Seq ID No: 899)

Homo sapiens nuclear RNA export factor 3 (NXF3):

```
tcctctctatgcttggggaaggaacttcctgtaagcaaggcttgaggcttgctctcgccttcgtca
gcagccctcctcaatcttctccaaactcccgtccccaggccacacagattctcctcaagagagccc
tataaggacattggtaaaatg (Seq ID No: 900)
```

Homo sapiens chromosome 14 open reading frame 133 (C14orf133):

```
attcccttccgccccttctctaagctgcacagcctgaatagaagggctggtccagcggcggcgga
ggctggcgctgtcctgagagggagggctctgtgcggaagagtcagggcgaccttgggcgctggag
tacgcttgggactggggctgcgagtgagcaccagcgattggttcggaagcggacatttggttcaga
acgagcatttaactctgccagggatccgctgggctctgacgactgcggtagatccatggcttcctg
gacgttcacccgtagagtcatcctagcttaactcttgttccctggtctcagttcacaagcctcacc
tgtatcttcctggctcggaagataattgaaaccaagtctgacttctcaatg
(Seq ID No: 901)
```

Homo sapiens X-prolyl aminopeptidase (aminopeptidase P) 3, putative (XPNPEP3): ctttctcttcccgacgcgtgagttaggccgtaatg (Seq ID No: 902)

Homo sapiens death inducer-obliterator 1 (DIDO1):

```
ggccctctggcaagatggctgctgcggaggcgttggagcgcggaaatctggaaccgggatggcgac
gtctacactgagtcggaggcgaaggagcttactccacgggaacagcctctagataatctgagttgt
tgaaaatacgaagcctgttactcgtgaacagtggctgacaacagtgttgttgtgagcctggctgtc
tgcttggacccagaggtttcgtctgccagggttttttggttgtatttaggatttcagggaaaagtgt
ccaagctttcagtgttggagcaggtatg (Seq ID No: 903)
```

Homo sapiens PERP, TP53 apoptosis effector (PERP):

```
cggcctcttcgcttttgtggcggcgcccgcgctcgcaggccactctctgctgtcgcccgtcccgcg
cgctcctccgacccgctccgctccgctccgctcggccccgcgccgcccgtcaacatg
(Seq ID No: 904)
```

Homo sapiens tubulointerstitial nephritis antigen-like 1 (TINAGL1):

```
tcctctcttgactttgagcgtccggcggtcgcagagccaggaggcggaggcgcgcgggccagcctg
ggccccagcccacaccttcaccagggcccaggagccaccatg (Seq ID No: 905)
```

Homo sapiens eukaryotic translation initiation factor 4H (EIF4H): ggttcctctcggagcggagacggcaaatg (Seq ID No: 906)

Homo sapiens non-SMC condensin I complex, subunit G (NCAPG):

```
ccccctctcgcgggaattatttgaacgttcgagcggtaaatactccctggggctgtcatagaagac
tactcggagagcgctgcctctgggttggcgggctggcaggctgtagccgagcgcgggcaggactcg
tcccggcagggttccagagccatg (Seq ID No: 907)
```

Homo sapiens MMS19 nucleotide excision repair homolog (S. cerevisiae) (MMS19): tatcccctcccacggtctctagttcgcgttatg (Seq ID No: 908)

Homo sapiens DnaJ (Hsp40) homolog, subfamily C, member 1 (DNAJC1):

```
ctgcctctacagctgtgtgtaggcctggggggcgagggtcttcggaacgtagcgctggctgcggccc
cgcccgcctacccacccgcccgtccggcagccggctcccgccgcctccgcgctctgtctggggcca
```

```
gccacctggcgggccgctccggtgcgcctgcccgcgcttttcactgacaggcgctgttccccacag
ccagcgccgcccgccacgtcccagctctcggccaacggagctgcgcggcgggtgacctttccgagc
ccagcgcgatg (Seq ID No: 909)
```

Homo sapiens stimulated by retinoic acid gene 6 homolog (mouse) (STRA6):

```
ctaccctttcatctctgcaactccttcctccctgggcctcccttctggtgtgtctgtgggtctgtc
taggtgggcttgggaaaggggaaggaaggggcgtctctttaggcagctcagactggacaagccttc
tttgaaaatggtcctttgaacacacgcctgctggtggttggtcagacagatgcgccagcgggagcc
ccggggccccaaggggacagctatctctgcaggaccagtgcgatg (Seq ID No: 910)
```

Homo sapiens 5-azacytidine induced 2 (AZI2):

```
cagccccttttccggctgagagctcatccacacttccaatcactttccggagtgcttcccctccct
ccggcccgtgctggtcccgacggcgggcctgggtctcgcgcgcgtattgctgggtaacgggccttc
tctcgcgtcggcccggcccctcctgcctcggctcgtccctccttccagaacgtcccgggctcctgc
cgagtcagaagaaatgggactccctccgcgacgtgcccggagcagctcccttcgctgtggaagcgg
cggtgtcttcgaagaaaccggaagcccgtggtgacccctggcgacccggtttgttttcggtccgtt
tccaaacactaaggaatcgaaactcggcggccttggggcggccctacgtagcctggcttctggtt
gtcatg (Seq ID No: 911)
```

Homo sapiens polymerase (RNA) I polypeptide E, 53kDa (POLR1E):

```
acgccttttccggcccgcagcgcggcctgggctcccgcgtgtttaaaagtgcgcttgtggctgctg
ctgtcttaactcctgtgcttggcggacagacaggcgagatg (Seq ID No: 912)
```

Homo sapiens mitochondrial ribosomal protein S25 (MRPS25): agtcctttctcgtcgctgctcggctcgcggcccgtggggtcggccccgccaccgttgccgccatg (Seq ID No: 913)

Homo sapiens TRM2 tRNA methyltransferase 2 homolog A (S. cerevisiae) (TRMT2A):

```
cggcctccgccgcacgcgctggcggactaagagtggctggcgaagcgagcggccggcgcgggcccc
tggcgggcgggcggtacagccccaagcctgagacccggacctgagcatcgcaggttcgagtcccgc
cccgcctggggcgaagccggggggtggcggcgacctcgcggcgttgcaccggctctgtgagcacctc
ccctctgagcacttcccttgtgacaggccacttccttgtgacaggcccaggacgaggtggccagg
cggcccccatggcgtccctggtctaggcggagaaccgcctgggcgatg (Seq ID No: 914)
```

Homo sapiens lipid phosphate phosphatase-related protein type 2 (LPPR2):

```
ccctccctccacctcggagtctgcgcggcgcggccaggcccggccgaccgcgtctcggtcttcgcg
tctgccagcctggctggcagtccgtctgtccatcccgccgcgccgggggcagtctaggcggagcggg
ggctcaggcggcggcggcctcgacgcgagtgagtgtcgtggttgggggtgctggacccagagtgcct
accctcgcctgcctgggcctcagtttccacatctgcacaatggggggtgaccatccctgccctgctg
gctgccaggagcggctgtgagtcttcaggcgtggatgcagcctgggggaagccatagggcgctttc
acaggcctggccttcaccatg (Seq ID No: 915)
```

Homo sapiens chromosome 11 open reading frame 1 (C11orf1): gaaccttttttcacctcgtctgaaatg (Seq ID No: 916)

Homo sapiens microtubule associated monoxygenase, calponin and LIM domain containing 1 (MICAL1):

```
cgccctcccacccgctcagacctggttgccagcccaacaggaagcggcccctcccggcttcggagc
cgccgccactcatctctgcccagctgctgccctccccaggaggcctccatg
(Seq ID No: 917)
```

Homo sapiens kinesin light chain 2 (KLC2):

```
gctcctttaaggcagcgaacgggccaagagaagcgtgtttcgcccccctccgacgccaccgaggtag
cggcttcacctttaaggcggcgcggggggctgctgggaaggccggcgggatggaggcggcgggaccg
gctcgcgggtgcgggtccgggtgaagcgggaggcagccagagtcggagccgggcccgagcaccagg
cgcaggcccggcgcccgcctgcccgcaccctcgtcctcacagacgccacagccatg
(Seq ID No: 918)
```

Homo sapiens DNA cross-link repair 1B (DCLRE1B):

acttccttttctgcccactctggtaacttattgctctgctgggctctttcccttagggtctctgg
ccctgttcttgccccagcatgactttttatcgggacgccgttgtggaagcctcacgcaggagccctg
cccccgtggagaagatcccactggtgactccaaccctaccaccatg (Seq ID No: 919)

Homo sapiens armadillo repeat containing, X-linked 5 (ARMCX5):

gctcctcccactgccgttgtgggtaacgcggacgtggaagaacctcgtctgcggaggaaaaggtag
atgttaaatggtaactacgcgcgaggttctgaggagccctgggaacaggaaggagaaaagaatacc
aaaagtgacaacagtttgccaatcgcagtctttaatctgataaagcggttatctcgtcttgagtcc
caggtgccgagtcaatcccatacacagccgccgccattgcctcgagtccttgtgtctgactgtct
gttcctgctgctgtatgacacagcacctcgaggcaaggaaataagaaaactgcctctgatccaagc
agagaaggtctgcctgtagatctgctgtagggcttgtcaccattggaagcaaggtcctacttcagt
ggcagatctggtggccttggagtggctgaagaccaccaccctccacagggctgggcccatgcacag
ccatccttccctaccttgagtgagcttcctctgcatgtttttctatatcactggcagagcctgtagt
tggaaaggggacagagtgactactggactttgtgtgaaaacaccaaccgggacaaaacttcagtca
aggctgagacgggtgggggtatataacttgtccttacgttaaacttggaacatg
(Seq ID No: 920)

Homo sapiens chromosome 12 open reading frame 43 (C12orf43): aatcctttgcggtggttcaagatg (Seq ID No: 921)

Homo sapiens vacuolar protein sorting 33 homolog A (S. cerevisiae) (VPS33A): ggtcctcccgtaggaaccggcggactcggttggcgttgtggggcagggggtggtggagcaagatg (Seq ID No: 922)

Homo sapiens arginine/serine-rich coiled-coil 2 (RSRC2):

gggcctcctcgcctttgtgccatccgggtctctcgcgcgagcgatttagtctgaggcgaagcttcg
gagcggccggtactgttgaaagcgacaagtggaggcgccgctctagcggccgggactctgaactat
ggcggctagtgatacagagcgagatggactagccccagaaaagacatcaccagatagagataagaa
aaaagagcagtcagaagtatctgtttctcctagagcttcaaaacatcattattcaagatcacgatc
aaggtcaagagaaagaaacgaaagtcagataatgaaggaagaaaacacaggagccggagcagaag
caaagagcgtgcttatgcgcgaagagactgaactgaagacgctgcagactcagatagcaaaataat
aagcctacttcatgataagggaagaagacatgaatccaaagataaatcctctaagaaacataagtc
tgaggaacataatgacaaagaacattcttctgataaaggaagagagcgactaaattcatctgaaaa
tggtgaggacaggcacaaacgcaaagaaagaaagtcatcaagaggcagaagtcactcaagatctag
gtctcgtgaaagacgccatcgtagtagaagcagggagcggaagaagtctcgatccaggagtaggga
gcggaagaaatcgagatccagaagcagagagaggaagaaatcgagatccagaagcagggaaagaaa
acggcggatcaggtctcgttcccgctcaagatcaagacacaggcataggactagaagcaggagtag
gacaaggagtaggagtcgagatagaaagaagagaattgaaaagccgagaagatttagcagaagttt
aagccggactccaagtccacctcccttcagaggcagaaacacagcaatg (Seq ID No: 923)

Homo sapiens integrator complex subunit 3 (INTS3):

ccgccttcccaccccccgcccttccactatggccgcttctgtgtggtgtggggagacgctggtcct
ccccgtcctcccatagcgcttattgcctcaccctcacccccctaggggccggatccaaaggcgctgc
actccccaagccttggggcatcagccaggaaggtttcctacctcctaattcagggggcaggactcct

cttttcccccacggggaaaagaggcagaaacttaggggtttccctcctttcttagggtcagacgc
tcttagggtccacttcttcaggggcggaagcctctcctacccttcccataggggcacaggcccttta
ccccactgtacttcggagccaacgcctttccctcagcactgccaccccagagtcaggacccagagg
actgtgccttcgcccccaacgcaggcgcggccttttggagaggagggaggagtggagaggacaggg
gcccttgctctcccctccccaacttgttcctcttgccccccagtccctggcaatccagagatcccg
atatctaggactgtccatccatccactccctgacctttttcccggctcctggctgcagccatg
(Seq ID No: 924)

Homo sapiens spermatogenesis associated, serine-rich 2 (SPATS2):

```
tctcctttcctcttctcagacccgggagcgtccgggacgcggagcccggagctggggcgacgaggc
gattgcgggggcctgggctagctgctggctaccaatattctactttctgtctctatgaatgtgact
accctggttacctcatataatctccctggaaaaggagacatgaatgtctgcaatgatacttcctga
caagaagttgatacaagaaaaggaaaggagattaacagctagtgagcagaatttcgaacagcagga
tttcgtattttttgcttccaactgcacacttccgttgcccacttttaaatcagagatacctacact
caaaacccagacaaggcaaaaggatacttttcttgtatattttttgagatcgaagaaacgacaatg
(Seq ID No: 925)
```

Homo sapiens fibroblast growth factor receptor 1 (FGFR1):

```
ccgcccctttcacctcctggctccctcccgggcgatccgcgcccccttgggtctcccctcccttccc
tccgtccgcgtctcctgcgcccccctccctgcgctcgtcccgccgctcttcccgccgcccaactttt
cctccaactcgcgctcgggagctggcgaggcggcggcggctcctcaggtcagtttgaaaaggagga
tcgagctcactgtggagtatccatggagatgtggagccttgtcaccaacctctaactgcagaactg
ggatg (Seq ID No: 926)
```

Homo sapiens FUN14 domain containing 2 (FUNDC2): ctccctcttccgctgccgccgtgggaatg (Seq ID No: 927)

Homo sapiens ganglioside induced differentiation associated protei n 1-like 1 (GDAP1L1): cctccttctttcctgcctctgattccgggctgtcatg (Seq ID No: 928)

Homo sapiens chromosome 19 open reading frame 43 (C19orf43): agtcctttgcgcggcacctggcgacaaaatg (Seq ID No: 929)

Homo sapiens MIS12, MIND kinetochore complex component, homolog (S. pombe) (MIS12):

```
ccctctcttctccaccagccaacgtccgggaaaaacgagtaagtacaggttccttctgccaatccc
cgccggccacagctaactttcccgcccggcccctttctgtcataattgaggtgtccacaaccagcc
aatcaggaacgcgagagtatcccgcgtttgctttcgctcgccgaggcgcgtatcagtcggaatttt
ggggagccaaccgcgccgtctgtccctggcaagccagcggcggtttaaaggaggtggcgggaagcc
tgtgtgtgcttcaaatcgtcaccctcatggtcgctccggtaagtgctgcggggcagcattttctct
gaggaggagcggggacgggcgagactggcataagcgtcttcgcgagggagcaaggcggcctgtggg
tcggcctcaccccggcctccgacctgaagatcccagcatgcagcgcgggcgcggggcccgacggaa
gccgggagccggccggaagcagttcctgcgctctggcttctgggtcctgtcctgcgcgatcgcggg
gtcttagacagctcaactcgccgagatgacctgggcacctctgcgttgaatcggcaaatactgatc
aagccgcatttattctgctctcaggaactctaagtctagcagagaagatgaggcggtagaagttca
tcaatggcttggctggaggacaagcaaattgaggacattggcaacggagtgatcaaaatgatagat
catgaggcctaaaatgaataaggaaagaagagaagtggcagaggctgagaacagaaagagagggtg
gaggggctgtaaatcttgaagattagggtataatatgagtatatgggtaagaattggaagaattgt
gtaggaggcagtagtcaaaaagtagaagcagtttggaagagtagttacaaatatcaagagccaggt
ggctaaaaggtggagctataggtcattgaagctcaagaaactgagtctctagggcattggttaagt
catctgtctagacttcaaagttgtctaggatgataattcagaagactgatctgtgccaaagtcaca
ggttttttcacgactgaaaacaacatagcaaaataagccaagatg (Seq ID No: 930)
```

Homo sapiens DEAD (Asp-Glu-Ala-Asp) box polypeptide 50 (DDX50):

```
cttcctttcacgctgtcgctgcccgtaggtggttgtggccactgtgcccggagggaggcggcggtg
gccagtaatg (Seq ID No: 931)
```

Homo sapiens chromosome 7 open reading frame 25 (C7orf25):

cggcctctgcgtgcacgcgcctgcgtgctcgcgctcgcggttctggcgctgccggaataatgctga
cagcatg (Seq ID No: 932)

Homo sapiens KxDL motif containing 1 (KXD1):

ccgccctttcctgtcgtgacttaacgcacgcaagcggctccagggtacgtccccgccacgcgcgct
cgcaggatcggtgcgtggtgacgtttcgccggcgcgggcgccatcccggaagcgcgagcaaggccg
ccagatgtgcaggcagcggaggaggagaaagagatg (Seq ID No: 933)

Homo sapiens defective in sister chromatid cohesion 1 homolog (S. cerevisiae) (DSCC1):

acttctttcttgcccgccaagcccgcagccacccgggcgcggcgggactcctagacccggcgctgc
gatg (Seq ID No: 934)

Homo sapiens zinc finger protein 426 (ZNF426):

cgttccttttgtgacgccggctgtgagcgcctgagagtcttttttgcctttcagagttaaggcctca
ctggcctgggaaaataattgctgccttttgcatccgcgttggctccgtccccaggatcttcccggt
tcagggacctggcgatttctgagtgttccggaatcccaataaccctgtttaaagaggaatggagat
tgccactgtccatttagattaatgaggtgtcctgaagtgatggtgacatcaatgaaaggagggttc
tgacacgttctcacctcgcgggatg (Seq ID No: 935)

Homo sapiens TATA box binding protein (TBP)-associated factor, RNA polymerase I, D, 41kDa (TAF1D):

caacccttttcttccgcacggttggaggaggtcggctggttatcgggagttggagggctgaggtcg
ggagggtggtgtgtacagagctctaggacaccaggccagtcgcgggtttttgggccgaggcctgggt
tacaagcagcaagtgcgcggttggggccactgcgaggccgttttagaaaactgtttaaaacaaaga
gcaattgatg (Seq ID No: 936)

Homo sapiens PHD finger protein 1 (PHF1):

ccgcctcctcctcctgccgctgccgctgctttggctgctgcgtcatacgccccagagccgccgggga
cggagggggctgggcctggggacccccccggcctccgcctgcacgcccccccacgcccggacgtgccc
tctccgcgcggggggactcgcctaggtctcctacgtctgcccctgcccggctcccggcggccccagc
tgtcaccggcccccccaggatgcaatg (Seq ID No: 937)

Homo sapiens family with sequence similarity 134, member A (FAM134A):

ccccccttccgcctgacgcgcccccggcggcggccgcgcagccctggctcctcgcgggctcgggcgg
cggctgcggcggggctatg (Seq ID No: 938)

Homo sapiens membrane bound 0-acyltransferase domain containing 7 (MBOAT7):

ccgcctcctttccggagcccgtctgttccccttcgggtccaaagcttttggctcctccttgttccg
agcccgaaggcccgccccttcacgtactcggagctcggatcccagtgtggacctggactcgaatcc
cgttgccgactcgcgctctcggcttctgctccggggcttcttccctgcccgcccggggccctgacc
gtggcttcttccccggcctgatctgcgcagcccggcgggcgcccagaaggagcaggcggcgcggggg
gcgcgctgggcggggggaggcgtggccggagctgcggcggcaagcgggctgggactgctcggccgcc
tcctgcccggcgagcagctcagaccatg (Seq ID No: 939)

Homo sapiens major facilitator superfamily domain containing 11 (MFSD11):

```
acgcccctttttttgctcagccgtcagccccgtctccgtctgaagagtgcttctgccctcatttgcc
tctccctgtgaccccggcccccctcagactccgctgcgtcgtctctcggccccgtccagccgttcct
gactgctcttcgccggagtccgcttcccaacccccctttcgccagagcccgagagctccgtcggctc
tgcgtcctggcggattgtcagtggcttcgccccgaggagagctgactgccctgggctgctgcctcc
ggcagagctgagccaaaatg (Seq ID No: 940)
```

Homo sapiens thiamine triphosphatase (THTPA):

```
ctcccttcccctctgtgggtcccgcgaggagactctcgggctttgaggtgagacctgaagttccg
ctggccggtagtgtagcaggaaagggcaggtcctcccgggtcgtgagccagtagcctcctggggtg
gcaaggtgtagagaggggggcgttgaaaggacacccgctacccggcctgctttctaggggtctctt
tggattgaggacatcagcagcagtggaagggatttactggagacctgtcactgtcagagccttaa
aatatcaccgacggggccttaatgtcaccgaggtagagagaaaagggcagtagccctagagactat
tgcgacacagtgtgcccctcataagttttttccagggagggggttctgtactgagttgacgccccagg
agctgagcaccaggctttgcatccttgggaactcagcaaacgtttgttcagccaattgcaggtagc
atg (Seq ID No: 941)
```

Homo sapiens acyl-CoA synthetase short-chain family member 3 (ACSS3):

```
tactcccttccctcaggccccaggaagttgcaagagtaccatttgtcgcacactcggggaccgcgg
gtggccggaggagatg (Seq ID No: 942)
```

Homo sapiens chromosome 6 open reading frame 211 (C6orf211):

```
gctcctccttcgcggcggtaccgcctctgtttctgcggcgattgaacagccgagctttgcggccgg
gatcgcggaaagtgatg (Seq ID No: 943)
```

Homo sapiens transmembrane protein 204 (TMEM204):

```
atttcctctctgctgagagccagggaaggcgagctctgcgcacacgggcgtccctgcagcagccac
tctgctttccaggaccggccaactgccctggaggcatccacacaggggcccaggcagcacagagga
gctgtgaacccgctccacaccggccaccctgcccggagcctggcactcacagcaggccggtgctaa
ggagtgtggcgcgggctcgactcccactgctgccggcctcccgagtgactctgttttccactgctg
caggcgagaagaggcacgcgcggcacaggccggcctccgcttcccgggaagacggcgcactcctgg
ccctgggttcttgctgctgcccacccctctgctccctgggatgggccccgaggcgagcagcttcagc
acaggcctggccctgctccaggtgcaggaaggaggataaggccgggccgagaggcggcacacctgg
accatcccatgggcctccgcccgcgccgccccgaggatgagtggtgatgtcctctagccacccta
gcagcgtcggctctccctggacgtgcggccgcggactgggacttggctttctccggataagcggcg
gcaccggcgtcagcgatg (Seq ID No: 944)
```

Homo sapiens DEAH (Asp-Glu-Ala-His) box polypeptide 40 (DHX40):

```
tcgtctttcccctcccatctcctcagatcggtggacgtgctcgcctccactcggggccaggtctat
g (Seq ID No: 945)
```

Homo sapiens importin 4 (IPO4): cctccccttttcggcccagtagcggcggctcag-ttgctgccatg (Seq ID No: 946)

Homo sapiens N-acetyltransferase 10 (GCN5-related) (NAT10):

ccttctctttcggagttgttccgtgctcccacgtgcttccccttctccactggctgggatcccccg
ggctcggggcgcagtaataattttttcaccatg (Seq ID No: 947)

Homo sapiens lin-28 homolog A (C. elegans) (LIN28A):

aaccctttgccttcggacttctccggggccagcagccgcccgaccaggggcccggggccacgggct
cagccgacgaccatg (Seq ID No: 948)

Homo sapiens CAP-GLY domain containing linker protein family, memb er 4 (CLIP4):

cggcctttcctccgcgcccccgcgtccccagccggccgctccgagaggacccggaggaggcaggtg
gctttctagaagatg (Seq ID No: 949)

Homo sapiens zinc finger, AN1-type domain 1 (ZFAND1): ccgccccttacggcgccggagagatg (Seq ID No: 950)

Homo sapiens GTPase, IMAP family member 6 (GIMAP6):

cctcccttttttctacttccgaggctgcaaagtgcaacagcagactcttctgactcaggaaggccgg
tgctcctacccacttcctgttcctccatctccagcggacactgctctttcaagggcaggtctccag
cccagctctctgaaaacattttgctgaaaatataagcaaacatcggccttgtcctccttgtgttca
tacactgtggaagcttttctctgcctcctccgtgagagtgcgtggccgggagaccagaaacgtggt
cctttctcttgcctgtgagctggtgcagagatg (Seq ID No: 951)

Homo sapiens thioredoxin domain containing 15 (TXNDC15): cttcctccggctggcagcacgactcgcgtagccgtgcgccgattgcctctcggcctgggcaatg (Seq ID No: 952)

Homo sapiens asparagine-linked glycosylation 9, alpha-1,2-mannosyl transferase homolog (S. cerevisiae) (ALG9):
aattcttttttccccaggcttgccatg (Seq ID No: 953)

Homo sapiens glutathione S-transferase, C-terminal domain containi ng (GSTCD):

acttcccttttttccggtccgccggattatgaatgacggccggcgcgagtattttccacataaggtg
gctgtcgttttttctcctggcgtctgtggaggcgagtggtctgcgggcagcagctcccagaggcagc
cttggaattccagctcggactgggcgggaaggcgcaggcggcccaggtcgccgacacgctcacgca
ccctccctgcctggccgcgcctctgcgaccaggtgacccaatgaaagaagaaaatg
(Seq ID No: 954)

Homo sapiens CXADR-like membrane protein (CLMP):

actcctttttctttccaaacagggaaaagtgttccacgaagcggtagcgcctttccgcctcgcgtt
ttcctccctgaccctggtcccggctcccgtccgggcgccagctggtggggcgagcgccgggagccc
atctgcccccaggggcacggggcgcggggccggctcccgcccggcacatggctgcagccacctcgc
gcgcaccccgaggcgccgcgcccagctcgcccgaggtccgtcggaggcgcccggccgccccggagc
caagcagcagctgagcggggaagcgcccgcgtccggggatcgggatg (Seq ID No: 955)

Homo sapiens nonhomologous end-joining factor 1 (NHEJ1):

cctcctcttgcggtggggggaaagcggcctcttactctaggcctttcggtttgcgcgagcgggcag
gaaagcgtgcgtgcggctaagagagtgggcgctctcgcggccgctgacgatg
(Seq ID No: 956)

Homo sapiens gametogenetin binding protein 2 (GGNBP2):

```
cctccttcttccactccccgcggcgcgagcggctgactgcccgtagaggaaacgacattcggagct
gcgctcccgcccaggccggccctgacgcgggcctcgtcagccagtaacagggagcagaggtgggag
ttagcgaggcgaccacgaaaacggtgaaggtcggaaccgacagcctcctccgagaagggcaggagc
tgggaggaggcggcagcggcggcggcagaaacagcagcggcggcggcggcggcagctgggaggagg
tggtgacggtggcaacggcagcgtcggggacgatg (Seq ID No: 957)
```

Homo sapiens zinc finger protein 672 (ZNF672):

```
ctttctcttttagccccgcctgcttcccggctccagctggggccggagaggctgagtggttggtac
gctgctcgctggcctcccagtcttcccagcaaccggtgacactgcccgcgccagactgaccactag
ccgacgcgggcgagagggacaggagcgtgacctccccatcccgaggggccggacgctcgggcgcct
ccccgctccccccactcggaggccgcgcgcgccgttagccccttcctcgctcccccgccccagtcc
cgcagtccgggaggcggggggtcggcagccggctgagtgggaaccgcgcggtgtctgaggaggcagt
```

```
cggcgaccggtttccacttcaagcgtgacccttttgcctgtgggatgagctccagcatggggtgag
gtacagaagagagacttgaagagcgtgccttgggactcaagcgccaaacctgtaccctagcgagtg
tcctactccgcatccgtaatggaaggaaatgcacatcttactccagaggcacaagaggaggacatc
ccatgcggctactcctgcccagcgtggtggggcagcagaagctccagagcccagacttgcaggctc
acggtgcagggtgaacctggccacagctcaccctggaacagccacaatgtctgccccttagagaag
aaccctgaaatcagaccagttttttgcggcctccccctttcctctctgttacagtgccctttccagg
ccttaagagaagtaaaacttagctgcagcgccaggaggtggaccccagagtgtgagtggcacgctt
ccctgtgaacccgtcctcaccatg (Seq ID No: 958)
```

Homo sapiens N(alpha)-acetyltransferase 60, NatF catalytic subunit (NAA60):

```
ccgcctccgtcccggctgcggcccctgccggttacataactcgttgcgggctccgcgcggtcccac
ttcccggctcccttcgcctccaggatgcgctgagccctacaacacccccagcggccgccggctccc
ccacgaggtgtgaatg (Seq ID No: 959)
```

Homo sapiens transcription elongation factor A (SII)-like 4 (TCEAL4):

```
tgccctctgtccccgcggctgggtctcgtctgctccggttcctgggctcctaattcttggtccagc
ttcttccaggtcagtgtgcggcccttccacgctgccagcggaacactggaatggcggaaggggaac
gggtctgcgcgtctgttgttcccagcgctctgcgaagcctgaaaaggaggagcaacctgtccagaa
tccccgcaggacaggaaaaggaggggaaatctcgacatg (Seq ID No: 960)
```

Homo sapiens progestin and adipoQ receptor family member VI (PAQR6):

```
tcccctttgtctccccactccccgcccaggcctggcccgcctgcctggccactcttcctccatcag
cctggctggcagcagccttggactccgcccgtggagccctgggcctgttgacccaccagcttagga
gcacccaccaagctctgggtaaggaagctcaccttctggggctcttctgggaaaatagaggtcaac
gtggaggtaccaggccaccatgctcagtctcaagctgccccaacttcttcaagtccaccaggtccc
ccgggtgttctgggaagatggcatcatgtctggctaccgccgccccaccagctcggctttggactg
tgtcctcagctccttccagatgaccaacgagacggtcaacatctggactcacttcctgcccacctg
gtgaggggaggctctgccccaggccgcggccttgagctcagaggggtacccaggcgggcagggac
cgtccaggcccacgggctgcagcggcagtcgcggggggtccgcggcggcctgagcacgcgcccgccg
caggtacttcctgtggcggctcctggcgctggcgggcggcccccggcttccgtgcggagccgtacca
ctggccgctgctggtcttcctgctgcccgcctgcctctacccctttcgcgtcgtgctgcgcgcacac
cttcagctccatg (Seq ID No: 961)
```

Homo sapiens DENN/MADD domain containing 2D (DENND2D):

```
catccttcttgctcaaccactgggtgcacaggatggaaacttctattccctctctggaagacagcg
cgtggcttggcttcacagagttgtggctggagaccgaagcagccccttctcaggcttactgtcac
cagtctgtctgtgttaggggagaggggagtccgctctgtcctgaaggcccagagatg
(Seq ID No: 962)
```

Homo sapiens family with sequence similarity 188, member A (FAM188A):

```
ccttcttctttcctgcctccaccttccaattcgtttgccgccgccgtcccgcagctgctgtttccgg
agttgccccttccccatgttccggggcaggagtccgcaaagcgaagatccgcccgccggttcctca
tcatg (Seq ID No: 963)
```

Homo sapiens neurensin 2 (NRSN2):

```
ccgcctttgctcggcggagacagcaggcagagagatgaggaaactgagacccagaaaggtggaagc
acttgtctaaggtcacgcctccaggaagcagtgtgtccacgactccagtccaagtggtcaggctcc
agagcccacagtcccaggggtccatg (Seq ID No: 964)
```

Homo sapiens tripartite motif containing 46 (TRIM46):

```
agccctcctcacacccccactgggctcctgcattaagcccgggggttcgcagccgcagccgggatcg
ggcacccaggggcgggcgggcacggtagggccatg (Seq ID No: 965)
```

Homo sapiens target of EGR1, member 1 (nuclear) (TOE1):

```
catcctctctgggaatttaccgatgcccagaacgcccttctttcccccacacgaccctctcctagt
ctaactcctgggcgtgctttaagctcagctcaggcagcgtcaccttctctggaaagcccaaaccca
gccaccccactacccgctacccgcggcccacgctgatgaagacagcagaacacggaggccccgcgt
tcccgccgcgagagcaggagagaaagattacctcccgcgagctctagcgcgcccggctttccggcg
cactccaggggggcgtggctcgggtccacccgggctgcgagccggcagcacaggccaataggcaatt
agcgcgcgccaggctgccttccccgcgccggacccgggacgtctgaacggaagttcgacccatcgg
cgacccgacggcgagaccccgccccatccccgactgcctgaaccgcgccaggagacggaccgcaag
tccagcgtacccacagacgactcaggcgggagacgagcggtgtcatg (Seq ID No: 966)
```

Homo sapiens DBF4 homolog B (S. cerevisiae) (DBF4B):

```
cgttctttaggggtggagccggcaggaaatttaaactgaagccgcggccgaaaacgccaagagat
tgatgctgtagctgccctgagataaccaggactgtggaatcgggaagagctcatggagctcgcgaa
tgtaatacggaggcctctgaggaaggagtacggaggccgagaaggagccggcatttgatg
(Seq ID No: 967)
```

Homo sapiens myc target 1 (MYCT1): atttcctttatg (Seq ID No: 968)

Homo sapiens myosin XIX (MYO19):

ggttcctttcctcactgcacgctcttgcccctcctcttttctctcctgcccgtgttcttcccgccg
cctgacctggcccgcccgcctttccagtctggccgggcggggggcctgaagcacggcggctcgggcc
gtgggaccgtgttcacaccctttccagaaattcttggctggtaaccgcgaaaccgactggagcagg
agctgggagaactggagaaaactgctctaatctcacttgactccagctaggagctgatgctgcatc
gtaataacatttgcagagcgctttcacaggcgctggagtgacttgtctgagattcctccagaactg
agccctttgttggaaccataccccagccatggtcccatgactaggtggatagtactccttgtacc
tcctgcaacccagaaccctggctgaccactttgaaggaggatg (Seq ID No: 969)

Homo sapiens KIAA0226-like (KIAA0226L):

cctcccctttctgctgttaccgggagcgcggtggccacggaacgctgcccggagccgcgcgaggga
ggacccgacgcgcggcgtttacccagcgcagcgttccaccgctcgggtttggctggataaaataaa
aaatggggatattgacctcctgtcactactgcatggactttgatggtttccaatcattactttctc
ctctgtgtcaatctgcctcttcgagaaattcatactcctgaatagctctccagaccccagctggc
catgtggtgagttcagggcccaaatcaagtagtaccagcaatcagggaactcctatctgttttgaa
tggattcacaccagccacaagcctggaaagatg (Seq ID No: 970)

Homo sapiens MUS81 endonuclease homolog (S. cerevisiae) (MUS81):

ctccctcttcccccgccccgccctgggccaggtgttcgaatcccgactccagaactggcggcgtcc
cagtcccgcgggcgtggagcgccggaggacccgccctcgggctcatg (Seq ID No: 971)

Homo sapiens zinc finger protein 430 (ZNF430):

gggcctttgtccctcgctgtggcctgagctccaggtctcgtcttcagcgctctgtgtcctctgctc
ctagaggtccaggctctgtggccctgtgacccgcaggtattgggagatctacagctaagacgccag
gaacccctggaagcctagaaatg (Seq ID No: 972)

Homo sapiens mutS homolog 5 (E. coli) (MSH5):

gctccttttgcaggctcgtggcggtcggtcagcggggcgttctcccacctgtagcgactcaggtta
ctgaaaaggcgggaaaacgctgcgatggcggcagctggggggaggaggaagataagcgcgtgaggct
ggggtcctggcgcgtggttggcagaggcagagacataagacgtgcacgactcgccccacagggccc
tcagaccccttccttccaaaggagcctccaagctcatg (Seq ID No: 973)

Homo sapiens proline rich 3 (PRR3): gccccttcctcac-taccctccaaatcccgctgcagccattgccgcagacacgatg (Seq ID No: 974)

Homo sapiens sirtuin 2 (SIRT2):

cgcccctttaccaacatggctgctgacgccacgccttctgggactcgtagtccggtcctcgcgcgct
ttcttacctaactggggcgctctgggtgttgtacgaaagcgcgtctgcggccgcaatgtctgctga
gagttgtagttctgtgccctatcacggccactcccatttctggtgccgtcacgggacagagcagtc
ggtgacaggacagagcagtcggtgacgggacacagtggttggtgacgggacagagcggtcggtgac
agcctcaagggcttcagcaccgcgcccatggcagagccagaccgactcagattcagactctgaggg
aggagccgctggtggagaagcagacatg (Seq ID No: 975)

Homo sapiens KIAA1715 (KIAA1715):

ttgtctctctgtcagtggcggctgctgcctgctctggaggcaggctgggcggtggcggccgagact
ggcggggggtggacgcccgggccgggctgcgcccgcttcttgcagctgtgaattcctttggacaatt
gatgatatttatcattgtgcccagtttctacaaataaaagatg (Seq ID No: 976)

Homo sapiens proline-rich transmembrane protein 1 (PRRT1):

```
ctgccttcatctctccatctctgcgctgctgccggctgcgccatccagcacccagactccagcacc
ggccgaggaccccactccggctgcagggaccctgtcccagcgagaccgcaggcatg
(Seq ID No: 977)
```

Homo sapiens t-complex 1 (TCP1):

```
ccgccccttccccggagcctcacttccgtcacagtcctgtttctctccctgttgtccctgcctctt
tttccttcccgccgtgccccgcggccgggccggggcagccgggaagcgggtggggtggtgtgttac
ccagtagctcctgggacatcgctcgggtacgctccacgccgtcgcagccactgctgtggtcgccgg
tcggccgaggggccgcgatactggttgcccgcggtgtaagcagaattcgacgtgtatcgctgccgt
caagatg (Seq ID No: 978)
```

Homo sapiens Yipl domain family, member 5 (YIPF5):

```
cgttctttggccctgtgacacgtagcaacggggctggttcagggtctgaaacagagtttggggggtt
gtttgggattagtgaagctactgcctttgccgccagcgcagcctcagagtttgattatttgcaatg
(Seq ID No: 979)
```

Homo sapiens glucose-fructose oxidoreductase domain containing 2 (GFOD2):

```
cctcccttttccagagcccccagttccttagaaaccaggcggcgcgttcccggtggcggcgccctgg
actcccgggcccgcgcatccccgccagccttccttaaggcggatgggtggcccccgagaccccgtc
ggacccatggtttccagtgcagcgcggagtgggcgatgccagcgtgccaggagccatgtctgacca
ggacgtttggaagatcatatccatgccagaggctcttgtgaggagatgagttggtaaagagagagg
ctgggatg (Seq ID No: 980)
```

Homo sapiens apolipoprotein L, 2 (APOL2):

```
ttccctttcgaattccagggtatatctgggaggccggaggacgtgtctggttattacacagatgca
cagctggacgtgggatccacacagctcagaacagttggatcttgctcagtctctgtcagaggaaga
tcccttggacaagaggaccctgccttggtgtgagagtgagggaagaggaagctggaacgagggtta
aggaaaaccttccagtctggacagtgactggagagctccaaggaaagcccctcggtaacccagccg
ctggcaccatg (Seq ID No: 981)
```

Homo sapiens microtubule-associated protein 4 (MAP4):

```
ccgcctccctgcgccccgcccctccggctagctcgctggctcccggctcctcccgacgtctcctac
ctcctcacggctcttcccggcgctctcctggctcccttctgccccagctccgtctcggcggcggcg
ggcagttgcagtggtgcagaatg (Seq ID No: 982)
```

Homo sapiens exonuclease NEF-sp (LOC81691): cttccttctttgccaggca-gacgcccgttgtagccgttggggaaccgttgagaatccgccatg (Seq ID No: 983)

Homo sapiens ST6 (alpha-N-acetyl-neuraminyl-2,3-beta-galactosyl-1,3)-N-acetylgalactosa minide alpha-2,6-sialyl-transferase 5 (ST6GALNAC5):

ctgtctctaatctctgcaacagccgcgcttcccgggtcccgcggctcccgcgcgcgatctgccgcg
gccggctgctgggcaaaaatcagagccgcctccgccccattacccatcatggaaaccctccaggaa
aaagtggccccggacgcgcgagcctgaggattctgcacaaaagaggtgcccaaaatg
(Seq ID No: 984)

Homo sapiens heterogeneous nuclear ribonucleoprotein A1 (HNRNPA1):

tgctcctttctgcccgtggacgccgccgaagaagcatcgttaaagtctctcttcaccctgccgtca
tg (Seq ID No: 985)

Homo sapiens zinc finger protein 93 (ZNF93):

gggtcctttgtctctcggtgcagccggagctccaggtctcctcttcactactctgtgtcctgtgct
cctacaggcccagcctctgtggccctgtgacctgcaggtattgggagatccacagctaagacacca
ggacccctggaagcctagaaatg (Seq ID No: 986)

Homo sapiens N-terminal EF-hand calcium binding protein 3 (NE-CAB3):

cggcctctagccacaccgagtccgccgcggcgtccagggtcggcagcaaccgcagccgagcccgag
cgggtggcggcgccatg (Seq ID No: 987)

Homo sapiens splicing factor 3b, subunit 5, 10kDa (SF3B5):

cattcttctgcgacggcgcggacctggagcttccgcgcggtggcttcactctcctgtaaaacgcta
gagcggcgagttgttacctgcgtcctctgacctgagagcgaaggggaaagcggcgagatg
(Seq ID No: 988)

Homo sapiens INO80 complex subunit B (INO80B): gtcccctttcctcgcag-gacctcatg (Seq ID No: 989)

Homo sapiens polyamine modulated factor 1 binding protein 1 (PMFBP1):

ctttcttcctcttggcttatattagggataggggatgtggtttgttacaaaggatgagtattttga
tagcttctcattccttgaactattctgcaggtttataacaaagctcagaaaatactaaaggttaaa
ggagaattgagagctgccaaggaaatg (Seq ID No: 990)

Homo sapiens pseudouridylate synthase 3 (PUS3):

cttcctttctcggaaacgcggcgcggccggctgccggaaaacagggcagacctgtatggttcgttt
attcctggggttgtcatatcatg (Seq ID No: 991)

Homo sapiens heterogeneous nuclear ribonucleoprotein D (AU-rich element RNA binding protein 1, 37kDa) (HN-RNPD):

tattctttttagtgcagcgggagagagcgggagtgtgcgccgcgcgagagtgggaggcgaagggg
gcaggccagggagaggcgcaggagcctttgcagccacgcgcgcgccttccctgtcttgtgtgcttc
gcgaggtagagcgggcgcgcggcagcggcggggattactttgctgctagtttcggttcgcggcagc
ggcgggtgtagtctcggcggcagcggcggagacactagcactatg (Seq ID No: 992)

Homo sapiens GABA(A) receptor-associated protein like 1 (GABARAPL1):

```
atttctccatctggctctcctctacctccaggcaggctcacccgagatccccgccccgaaccccccc
ctgcacactcggcccagcgctgttgcccccggagcggacgtttctgcagctattctgagcacacct
```

```
tgacgtcggctgagggagcgggacagggtcagcggcgaaggaggcaggccccgcgcggggatctcg
gaagccctgcggtgcatcatg (Seq ID No: 993)
```

Homo sapiens chromosome 22 open reading frame 13 (C22orf13):

```
ccttccttttccccagtgttgagcgcggtctcgcctccgcttcctcctcactccgcctgccggctgg
gaaactagggcaccagtacgatagttccggcaccggaaaagagggctgatgactgggcccggggggc
cgccgcaacgacccttggggccggcaaagagccagagagggtgctcacacttccaagcaccccaca
ccaaggacaggctggacggcaaggcggagacgcggggcttgggccctcagaccggggacagcagga
ggttgggccaagggccaggacttcccgtcacaatttcatttgttgatcccggcaccgccaggtaag
gggggccctgagtgaggctaggtatctggtacggataaagttaggtatagagtagagcggctgccc
gctcaggggttatccctaaagacagttggaggagagttgcttggggcctcggggatgcactgggcgg
gatcagggcttacacctaggactggcaaaagagcgggacccggcagaggcggggcttgccgaaggg
acgagcctctattcaggaaatgcacgagctttggggcggggctcaaagaaaggggcggggcttccg
gggcccgcgtcctggtgagctgcgcgtctgcgcgaggattgggcgagagggtggggccactcaacg
ctgaggcggcgaatggccggagcagacttaaatcaagaggctggggacctctaagatcaaagtttg
gggcggggcctaaggagggggcggggcctccagattcgagacctggaagggctggggcggcgcttg
gggcggccctgccgccgcctcccgttctcccctccgcagcggcggcggtggcggagaaggaactcg
acacgcaccgaccgccctcccgccccagccgaagcggaagctgtagcccgctctgggccgggggcca
tgggcgccccgcgccgcccgggtcatg (Seq ID No: 994)
```

Homo sapiens lon peptidase 2, peroxisomal (LONP2): ggctcttttttgacagcccccagtgcgaaaggctgccagcatg (Seq ID No: 995)

Homo sapiens RNA binding motif protein 4B (RBM4B): ggttctctctgacgtgggagccgccgtcgctgccgccacccggaggctcttgtcaggatg (Seq ID No: 996)

Homo sapiens protocadherin alpha 3 (PCDHA3):

```
aggtctttctccacaaaagaaataacagcgtgcattacgtattcagatactgctttgcttcatcct
ctctaaaatttaacaccgaggagtttaagaaatgaagataaggaactcgaattattttttaaacttt
ggatcaatgtaaaggcaatctaatatttggaaaatacttgcaatg (Seq ID No: 997)
```

Homo sapiens RAB34, member RAS oncogene family (RAB34):

```
gcctctccttgggccccttctctcccccttttcccctccctgctggttcctggcatcgccagatgct
gcgcagcagtctccgattccccatcaccaattcggctggcgtctccgagaccgcggactcccgtag
ggtccccgtggccccgagttgtagtcgggacaccccggccgcgggtgatcgtcgggtctccacgcg
cccgggtcgctgacgcggatccggcctcggcgccttctcagggcgccctgcaaggccgcaggcagg
atg (Seq ID No: 998)
```

Homo sapiens cell division cycle associated 7 (CDCA7):

```
gctcctcctgctgtgggaccgctgaccgcgcggctgctccgctctccccgctccaagcgccgatct
gggcacccgccaccagcatg (Seq ID No: 999)
```

Homo sapiens ArfGAP with GTPase domain, ankyrin repeat and PH doma in 3 (AGAP3):

```
gggtctttaggagagcactgctgcagccggcagtggagagcctgggcagggagacagggagaaaa
ctccggcagcagggtggtctctagggctgacctcggagcctggggacaggggagcctatgccgcac
tgaaggcgggacgctgtaagcgaggagcagctgggcctgggcggactcctcggccaatcagcctcg
gtcagcagcaccctcaggcgcagggcactgtttgggcattgcctagagatccgacaccccgcccag
atcagcgcagggaggcgaaagcgacagccgggcgcgggaggagaccagggcagctgtcccctccgc
gagggtggccctcgaggcaatgcgggtgggggctggtgaggaggcggaagggccgaggctgagtgg
gaggggccggggcgccagggctggagcgcgcggctcggggtggaggctgcagagccagcgagcga
gcgaggggcggggcgcccgggccggcgcgcaggaggggcggggggcggcggggagggggggctcggg
ctgcgtgtgccggagccggcggggcggcggtgcgtgcgcatgacgcggggggagggcctgggccg
```

```
cgcgctcccggtcccgttgttgttgccgctggaggctgctccgaggcagcgggatcacggcgctgg
gaagcgctcggcagcggcggccacagcgtgcgcggcggcgcctcctggcctcggcctccggcccccc
ggcccccggctccatgcgctagccccgcgccgccagcccagtagtcccggccccgccagcccgcg
ctcccgctcgccgctgccgccgccgccgccgccgcctccgccgcgccgccccgggcccgcctc
gggccccacggctccgaagccatg (Seq ID No: 1000)
```

Homo sapiens potassium channel tetramerisation domain containing 1 0 (KCTD10): ctgcctctctcagtccgggtttggagactc-ctgcgtcctccgactttcatg (Seq ID No: 1001)

Homo sapiens cyclin B1 (CCNB1):

```
cattctctgcgaccggcagccgccaatgggaagggagtgagtgccacgaacaggccaataaggagg
gagcagtgcggggtttaaatctgaggctaggctggctcttctcggcgtgctgcggcggaacggctg
ttggtttctgctgggtgtaggtccttggctggtcgggcctccggtgttctgcttctccccgctgag
ctgctgcctggtgaagaggaagccatg (Seq ID No: 1002)
```

Homo sapiens eukaryotic translation initiation factor 2A, 65kDa (EIF2A): gtttctctttccgggacaacatg (Seq ID No: 1003)

Homo sapiens protocadherin gamma subfamily B, 7 (PCDHGB7):

```
cagcctctagcctgggattccctgcgcagccaacaacagaaaagaaaaccagctcccacacagagg
ctcccggctgcgcagaccttgcccagcacaccagattgccagctccgagacccgggactcctcctg
tcctgggccgaatgctcttttagcgcggtagagtgcactttctccaactggaaaagcggggaccca
gcgagaacccgagcgaacgatg (Seq ID No: 1004)
```

Homo sapiens acyl-CoA dehydrogenase family, member 11 (ACAD11):

```
ggctctttcggcttccttcctcgctgggccggctaaacccggccgcagcagcaccgggggtgataag
tgtccagggcaggaggccagcgatgttgccttgctaaccgggtatctaagagaaacagggtctttt
tattcttaggctcgacagtctgacggccctttttctgaacgggaccctgcaggtcttccgcctgct
gttgcattaaatttggggggtggaagaggcttctgcgttgttccttacccgcaacgatgaccatggc
tttgccttctttaaaattgaggcctccaactctgacgctgactggagaattgaaacccgaacacac
attgggctctttttggcacttgactagagctaaaacctcgggattcagcgggcaagcgttgctcagc
aacggcgcgtaggctgtgtgcggttggctggagccagaccccaccccggcctcggcccatgctcta
gaggggacgttgccccaatcctgaaggacttcggcactcgagacctgtggatgccgcgttgctgtg
gcctgcggggggtgatcatg (Seq ID No: 1005)
```

Homo sapiens zinc finger, CCHC domain containing 7 (ZCCHC7):

```
ccgtccctctacgcgttttggttcccggttggtgcttcctgttcgcagctgcggcacttcaaggtt
actgactttttatg (Seq ID No: 1006)
```

Homo sapiens zinc finger, MYND-type containing 12 (ZMYND12):

```
gggcctttctggacttggactccttgggagtcgtttctcggccatttgacccgtgggacttgtggg
ttttgtgctgctttttctttctttcttcccctttttccaacttcagcaatacacccagatgttagtc
gagtcacgtcccgccgccctctgcccttgaaatgctggcaagtacgcagccccgcgatcgtcacgt
gacgccggggttcagcgtatccttgctgggcaaccgtcttagagaccagcactgctggctgcacca
tg (Seq ID No: 1007)
```

Homo sapiens forty-two-three domain containing 1 (FYTTD1):

```
cgctccctcggtgcggcgggctgcgtgcgcgagtgggaggtggcaggcctgcgactccggccttgt
ccgcgcccgctctcggcgcgacgtctccagccatg (Seq ID No: 1008)
```

Homo sapiens SH3-domain GRB2-like (endophilin) interacting protein 1 (SGIP1):

```
ctccctttctctcagcatcttcttggtagcctgcctgtaggtgaagaagcaccagcagcatccatg
```

```
gcctgtcttttggcttaacacttatctcctttggctttgacagcggacggaatagacctcagcagc
ggcgtggtgaggacttagctgggacctggaatcgtatcctcctgtgttttttcagactccttggaa
attaaggaatgcaattctgccaccatg (Seq ID No: 1009)
```

Homo sapiens GTPase activating Rap/RanGAP domain-like 3 (GARNL3): cagccctttttgcaaatg (Seq ID No: 1010)

Homo sapiens DCN1, defective in cullin neddylation 1, domain conta ining 5 (S. cerevisiae) (DCUN1D5):

```
gagcctcttgcttgctgtgactggtggagctgccgcgctgtccgcgttatctcctcccggtgagaa
cgaaccgcagtgtccaccggcgaggagccagccctgtcccggtcagagaaagacgacgaggatacc
tgggagcgggcggcggccgggctgggccgcgccggtgcgggctggcgactctgctcctccgcttgc
tgctgtctctgggaactgggtgccagcgctgaggggcttccagcggacagggacccccttccccgg
ctcccctgcccaccctgccggggagggcggaagatg (Seq ID No: 1011)
```

Homo sapiens alkB, alkylation repair homolog 7 (E. coli) (ALKBH7): tgccctctctcatgaccccgctccgggattatg (Seq ID No: 1012)

Homo sapiens nitric oxide associated 1 (NOA1): ccgcccctttggagctacttcctcatg (Seq ID No: 1013)

Homo sapiens BTB (POZ) domain containing 10 (BTBD10):

```
tcgcctcttcgcattgtgagctctcgcggtaagaggctgaggagccggcctgcaacctgccgggggc
ggctccgctacgcgcagccgcctcagtggcttcctccacagccacctccggagggatctggctgag
gaggaagtggaggtgtcactggccccggcctttgccccaatcttgtgtgggcactgaagggggact
acaggttcgagagttatgggtgctacatgtgtgctttcagagcagtagtgtgaggaagcttggagt
gggatg (Seq ID No: 1014)
```

Homo sapiens zinc finger protein 397 (ZNF397):

```
cggtctttgtggcttgcagctcggggtgggtggctcatttcctggccgctcctgggcttcgcggaa
agaagagattactcacactccttcgcaagcacagaaccagttgtactgagcttttttgctaagctgt
ttcagccaagaatg (Seq ID No: 1015)
```

Homo sapiens mitochondrial ribosomal protein L45 (MRPL45): gctcccttcccggcggcctttgcgggaacaagatg (Seq ID No:

1016)

Homo sapiens AKT1 substrate 1 (proline-rich) (AKT1S1):

```
cttccttctccatattgtatactggaattgaagccaaggaggtaccattttgctcgagggcatggc
ctaagccggtcagctaaggccatgttaatacggggctgtcccatctctctgcggggcgcgacagct
ggaagagccgaacggataagagaagaggaggtgagaggagctgtacaccacaagaggcactgaggg
actcaggataacgggatgaagccgtcagtgcccccagaaacgaagcggccccggacgaatttctga
gtcaccgtcgcgagaaagcgggctgagccgccattttgaagcctggcaaaccgaagcaagaaatgc
tgccgtgttggatctttgccagccttcgtgccgaatgggagcaggttggagggagggagagccaat
atacactatgggctgattaagcccggttggctgccatgttgttaacgagcaccgatttcctctact
tttgtcgaagaagtttattgtgggtcagggacgtcaggtcgcttgccttcgtttactgtggtcatg
attgagcatatgaggacggccattattgttggggggcaaatggaaatgctctaggcggggccatttt
tcttaggggcaagctgtcgtcaccccttgtcaactggttcggatgaagcccctgtggccgccatctt
gatctcgggcggccccgataagggaggcggagtgtgcggagaggaggcgggcaactgcgcggacg
tgacgcaaggcgccgccatgtcttttgagggcggtgacggcgccgggccggccatgctggctacgg
gcacggcgcggatg (Seq ID No: 1017)
```

Homo sapiens transmembrane protein 101 (TMEM101): ctgccctttcccaa-gatg (Seq ID No: 1018)

Homo sapiens eukaryotic translation elongation factor 1 delta (guanine nucleotide exchange protein) (EEF1D):

```
ggccctccctttcatcagtcttcccgcgtccgccgattcctcctccttggtcgccgcgtccttggc
tggcgttagagacagggtttcaacgtgttagccaggatggtctcagtctccagaccctgtgatccg
cccgcctcggcctcccaaagtgttgggattacaggtgtgagccaccgtgcctggccgaggctcctt
cttttatg (Seq ID No: 1019)
```

Homo sapiens ADP-ribosylation factor GTPase activating protein 2 (ARFGAP2):

```
cgccctccccgccgtggattggcccgcggcgggacccgtcagccgcggttgtgtctgggaaggaga
gaaaatg (Seq ID No: 1020)
```

Homo sapiens junctophilin 4 (JPH4):

```
atttctctcctccctggggggtctcagtgcatctccttctcctctctgcctgcctcctccctcaccg
aagggttagcggacacccatccttttctgcttggggaccccaccaccacccgcaacactgccgctg
tctcttcttcaccgtatccttctctacccaccctcttctctcttctcttctccctgcccctttaaa
tctgcctggcccagcctcccccgtgatgctgggatggagcaaacattgatttgtgctgggatggaa
tcggaattttgatttattttttcctctcccaaccataagaagaaaaaaataataaaaacacccctc
ttgagagccccctccccctttgcatccagctcccagctcttcttccctatctccatccaaggcaga
ttttttcccctacactattctcatcttcccccaccccttgccactacctcgcccccccacccagcct
gctcctccagctggggagagaggggactctccggactcccccacctttcctctctgggttggagca
gtctctccggaaggggaggggcttggcttgtccgggcgaggtgggagtggaggtatcctgccatg
gatgctgtgccggggaggcagcctgagccccagcccacatgagacgccgaagaaccggggcagagg
ggtcctgacagcagccagggaaacgggtgccctacgattctgcccagcccctctcaggacccca
aactgccatccacactcgacacttcggggttctagccactcaggatgagggtccggccctgcctgc
cctcgctggggcccccccgcccggccccggtctaactgccccgccccgaggcctcgcccggctcc
aaggcccccagcaggctctccagtcccaggatgcgctgagccgccggggggctgaggccgcgccaa
ctacatgcatg (Seq ID No: 1021)
```

Homo sapiens embryonal Fyn-associated substrate (EFS):

ttttctttctcctcctccaaccttggcggaggccacgactcaggcgccacagctgggggctagagg
ccgcggaccatggtgcggggcagccaccgctgaagtcagcaaaaccgagcctggcctgaggcaggc
tgcgcgggaggccaaagccatg (Seq ID No: 1022)

Homo sapiens GH3 domain containing (GHDC):

cgctccttctttctggccggatgtgtgctgagacccagagtcacccagggggtctccgtcacgtgcc
aggagtaggcagaagtgggctgtgacagatcaggaaacagagctcagtgcagcccactaaattgct
cagggccctacagctaacaagcggcagaggcaggatctgcactcaggagctgcttggagatg
(Seq ID No: 1023)

Homo sapiens acrosin binding protein (ACRBP):

ggctctctctgcggcttggcccgttagaggcggcttgtgtccacgggacgcgggcggatcttctcc
ggccatg (Seq ID No: 1024)

Homo sapiens jagunal homolog 1 (Drosophila) (JAGN1):

agttctcttcacggagccgcgcggctgcggggggcgcaaatagggtcagtgggccgcttggcggtgt
cgttgcggtaccaggtccgcgtgaggggttcggggggttctgggcaggcacaatg
(Seq ID No: 1025)

Homo sapiens ligand of numb-protein X 1, E3 ubiquitin protein liga se (LNX1):

gttcctttcctgggcatcagcttgcctgctctcagcctaagctctctcgccaaccgtggtggctcc
ttgcgttcctacatcctctcatctgagaatcagagagcataatcttcttacgggcccgtgatttat
taacgtggcttaatctgaaggttctcagtcaaattctttgtgatctactgattgtgggggcatggc

aaggtttgcttaaaggagcttggctggtttgggcccttgtagctgacagaaggtggccagggagaa .
ggcagcacactgctcggagaatg (Seq ID No: 1026)

Homo sapiens cyclin-dependent kinase 2 interacting protein (CINP): tctccttctacggatatctgtggaccttatg (Seq ID No: 1027)

Homo sapiens splA/ryanodine receptor domain and SOCS box containin g 2 (SPSB2):

gcttctttccgcccggctccttcagaggcccggcgacctccagggctgggaagtcaaccgagctcc
cttccaggtcaatccaaactggagctcaacttcagaagagaaagacgccccagcaagcctctttc
ggggagtcctctagctcctcacctccatg (Seq ID No: 1028)

Homo sapiens Berardinelli-Seip congenital lipodystrophy 2 (seipin) (BSCL2):

cctcctcctttcctccctctactctgacacagcacttagcacctgaatcttcgtttctctcccagg
gaccctccattttccatatccaggaaaatgtgatgcgccacaggtatcagcgtctggatcgccact
tcacgtttttagccacaagtgactcagtggaagatccagagtcaacagaggctcgtcaggaagatg
(Seq ID No: 1029)

Homo sapiens tubulin, alpha 1c (TUBA1C):

caccctttcactacttctcccccggactccttggtagtctgttagtgggagatccttgttgccgtc
ccttcgcctccttcaccgccgcagacccttcaagttctagtcatg (Seq ID No: 1030)

Homo sapiens 1-acylglycerol-3-phosphate O-acyltransferase 9 (AGPAT9):

tttccttcctctcttccttcgcagaggtgagtgccgggctcggcgctctgctcctggagctcccg
cgggactgcctggggacagggactgctgtggcgctcggccctccactgcggacctctcctgagtgg
gtgcgccgagtcatg (Seq ID No: 1031)

Homo sapiens 1-acylglycerol-3-phosphate O-acyltransferase 1 (lyso-phosphatidic acid acyltransferase, alpha) (AGPAT1):

gcccctttctttccttcgcttcctcttttagagaatgtccggattgctattggactttggagcgta
tggctccaaatcaactcattggctaaaacttgacggaaaatggtggttaggtggccagaatg
(Seq ID No: 1032)

Homo sapiens abhydrolase domain containing 14B (ABHD14B):

cggcctcttcccagcgttcctcctccggccccaggtcaccgccagcacgcgcctgcttcccgtctg
cgcgagtccacgcagctccccagatcaagaagctgaggccccaggttacacactaaagtaaatggc
agaggcagaaataacacctatgtcctcctgaccccaaggcatgttcttaaagttctggaaacctcc
tggaggcttccttgctgctcctctgggactgccaccctgggcagggtgttctgtggcccctcatca
tcgtggttttgaaccacaggcccttcaccagcacagcagcagcaggcatg
(Seq ID No: 1033)

Homo sapiens protein tyrosine phosphatase, non-receptor type 5 (striatum-enriched) (PTPN5):

catcctcccgccagcctgcccgcctgctcgccggcgcccggagcccgctctggccgcttgcttttt
gctgagaaagcttcctgccctggaagatggcacccttccccatccagacaccttgggaatg
(Seq ID No: 1034)

Homo sapiens carbonyl reductase 4 (CBR4):

cttcctccttttcacggcgtcttgcattactattgtgcggctgcaggaggtgtcgagcggcgttat
tttttttttgcggtttgccttttttttttctttttttttttttttggaaccgcggttgtttaaaagcct
gagggaacctggagaggggctcccactccctaccctctttcctccgagtttgtgactccgagatg
(Seq ID No: 1035)

Homo sapiens zinc finger CCCH-type containing 10 (ZC3H10):

ggctctttgtcgaagctagaggaccggcaggcggcagcagcaactacggcggcggcggcagaaccc
agcagcgatgtggaggtggagacccacaggagccccggacttcacctgagctacctcagtggtcac
caagagtggcaagataaagaaaaccctgagttgggcgggaccaggatg (Seq ID No: 1036)

Homo sapiens poly (ADP-ribose) polymerase family, member 10 (PARP10):

ccgtctttcagtttcacttttgttttcctgctcccagcagggttaggcttgctgaggggcaggcac
aggagtcctggctgagctcatggcctgaggctgcctagcggccacggggaatg
(Seq ID No: 1037)

Homo sapiens RNA pseudouridylate synthase domain containing 4 (RPUSD4): ccgcccttccttgtaagatg (Seq ID No: 1038)

Homo sapiens family with sequence similarity 73, member B (FAM73B):

```
ctgcccttccgcagcgatggcatcccgggtgagtatcggccccggccgagcccccaaggcgggcgg
gcagcgcggcagggccgggacttgagcggaggaccgagtaggcgcaggtgtccgggcccaacagga
ccaggaaggtgtcggggttggaatgagtgggtacccgggccggggacggtgcgagagggtgccttg
cttgggagcggaacgagaaggtacttgggtcagggaggtgatgcccgggcctggaacgtggcgggg
attggagcaggcgcgcaggtacccgatccgaggcggggagagcacccgggatggaaggagcaggcg
tgcgggccgtgagcggcgccagagggtacctggctctgtggaggggccctctggtatgtgtgtccc
tgtccttctggggcgtggatggtgcctgggacccagctggcaaccagttgaagacgttctccttgg
aagctcttggccctgaggactttgcctggggcattggccctgccatg (Seq ID No: 1039)
```

Homo sapiens protein phosphatase 1, regulatory subunit 15B (PPP1R15B):

```
gcgtctcttccggcgtctaggggggtgtcctgccggcgcgcgggccctgcggccattttgggcttc
gcttccaccgcaccagccggcctacccagtccttccggtatcgcgttgctcaggggcttttcaacc
ctctgtcagtcggaaaaccatcgccgaggccgtggggggactcctatccatggtgttgaagcgtcg
agccgactagggaacctccttccccgccaggatggaagtcgcatcagtcgccgcctattgcgcggg
ctgttcttccctgtgttctgccgcccgctgccgcattcgctgccctctgtggcttttctgctggct
cgaagatcggcctggagcagcgacgccaccgctgggcaaggccgagactctgtaggcttcctccga
atcccgtcgacctccagccgctgagcgccgcggccctacctgagagactgtcaagaaaaggagat
g (Seq ID No: 1040)
```

Homo sapiens family with sequence similarity 104, member A (FAM104A): ccctctcttcgcggagcggcgccgcgtagcttccatccgccagctgccatg (Seq ID No: 1041)

Homo sapiens PRP38 pre-mRNA processing factor 38 (yeast) domain containing A (PRPF38A):

```
agccctttacactacggtgtttccggcttcaagatggtcgcctaagctgtttagtgaaacttcttc
cacctttctccattcctctaggtgcttttttctgaacctggatgtgaggcattaaaggatccgacgg
aaatagaattgaaggcattctaaaatg (Seq ID No: 1042)
```

Homo sapiens synaptotagmin-like 1 (SYTL1):

```
cctcctccgtgtgggggcagctgctggctgggctgcctgttgagtcagccttcttccctcacggctc
ttctcccggtccctgaaactcggctgccaggggagctggagccacctgcgaaggtgtcctcccata
ctggacccctacaggaagctccgtgtgcccagctggggcacagccccagctgatg
(Seq ID No: 1043)
```

Homo sapiens ubiquitin associated and SH3 domain containing B (UB-ASH3B):

```
gctccttttcttttttgatccattcaaaaattactcattgcaaattcccggactgctaggcgagga
gagggaaggggggcggaggagacagggctactgcaggcgcagagctgggggcagccggggggcccgag
tggctgaggctggtcccgcagcggccgcttgccggcgttctggctcctgtggcctcaccaggaagc
gtcagagtcccgacactggggaagctcggagcgccgcctccgctgccgccgcctcctgcctggctc
tgggtccccgagccccctcccctggcccagcccgactccctcctccttcccgaaccatccggctcg
ggctccttccctggcgatggctggccgctgagccatg (Seq ID No: 1044)
```

Homo sapiens transmembrane protein 241 (TMEM241):

```
ccgtctctgggcggctgctgccgctgccgctgctgctgctgcggggggtcgggcggcggccaggggga
tttgggcaggcaccgtggatccccgagaaggggacgagttgacagatg (Seq ID No: 1045)
```

Homo sapiens ataxia, cerebellar, Cayman type (ATCAY):

```
gagcctctgccagccctgagctgggaagaagcagctacctcggaggcagggcgcgcaggcgggcgg
cgatgagagggggcgcagccgcagccccgcgctggggagcccaccgctaaccctgcaccccaccca
cccctgcacaaaagagctggcgggcgctggccacgtcgccctgggtgaccttcctcggatgcagaa
tccgcccctgcgagcatcctcttcctcctaggctctgaaggcccgggggagcgtgagcgatgcccag
ctgcacccgggcagggctcgcctttgtttgccagtaaggaggagaggctgtctcagctgcagaggg
gtcatccctgcttcaagccagtgcctcttcccagctcccatg (Seq ID No: 1046)
```

Homo sapiens ELL associated factor 1 (EAF1):

```
attcctctctcacccccacgcagaggagagaacttgcttctggacccgggtgggtgccggctcggc
tctccttgtcttccagagcggtggcccggaagcacagtcctcccagacgccagcgccagaagctcg
gatcgcggctgcaccgggagagcgccgatctgggtgcgaggcaggtgcggggccatg
(Seq ID No: 1047)
```

Homo sapiens tripartite motif containing 5 (TRIM5):

```
gttcctctaggaaaattcctttgtgcagatcaggcccgtggattggtgagtgaatcctaaccacgt
cttccctggcctgtcttcactcttctccccagaatcaccacttctgcactggtgtctgaaggtgta
ttgagtgattttgtggagggcagaagtaggaagtctttgggacaaaactgtatttaccttgggatc
tgtgaacaagaggaacctcagcagccaggacaggcaggagcagtggaatagctactatg
(Seq ID No: 1048)
```

Homo sapiens wingless-type MMTV integration site family, member 3A (WNT3A): cgccctctcgcgcggcgatg (Seq ID No: 1049)

Homo sapiens chromosome 16 open reading frame 45 (C16orf45):

```
ctccctccctgcagcccgcaacgggaatggagtaaagggagacccgtcgacctggccacggggatc
agcgatg (Seq ID No: 1050)
```

Homo sapiens zinc finger protein 502 (ZNF502):

```
cattcttccggtttcagaagttaaggctggtgtcctggccccagtccacctctgggagcgcctgcg
ccgctccgcggagagtccgtggatctcacagtgaaaaatgtttgctgacccttgacattgacaaac
tgctgacagctcagatgatccatgattggaaggatgtggtcatcaccaagatgtctttctttctcc
ggttcccagttttccagacctgaagtgttttccaatcaaagcgaagagacgatctgtggatg
(Seq ID No: 1051)
```

Homo sapiens armadillo repeat containing 6 (ARMC6):

```
ggctctcttgcgcaagcgcgctgtccgcttcttctgggcggacgctctggaggcaaaacatttccc
tgctggggggcggcgaccaccgtgagcgtcccggaaggggcggcaaagacgcctccgtcgcgcacga
ggtggcctcgttggctttaccttggttcgcggtcgtccttggttatcgtgagcgtccgcgagtctc
tgggaggccaagcctaggggcgccacagcgcctgcgcgcgtacggcggccggaaggggctagaggc
ggctccctgggtgacaaccgcgcgccccacctttccccacgtggccgcgaagaccggctcaggagc
atctatcggctgcacgccaacatcaacacaggcgaagatg (Seq ID No: 1052)
```

Homo sapiens post-GPI attachment to proteins 3 (PGAP3): gctcctcccccggcggcgagccagggagaaaggatg (Seq ID No: 1053)

Homo sapiens histone cluster 3, H2a (HIST3H2A): tgccctcttgttttttagtctcgcttttcggttgccgttgtctttttttccttgactcggaaatg (Seq ID No: 1054)

Homo sapiens ethanolaminephosphotransferase 1 (CDP-ethanolamine-specific) (EPT1):

```
ggctctcctaccttctcgggcagcccagtctttgccatccttgcccagccggtgtggtgcttgtgt
gtcacagccttgtagccgggagtcgctgccgagtgggcgctcagttttcgggtcgtcatg
(Seq ID No: 1055)
```

Homo sapiens F-box and leucine-rich repeat protein 5 (FBXL5):

```
ccgcctctgccccgcggcgagggtgtctatggagaggcggcggccgcggctgctgaggcggaggct
gaggcagtggcgatggcgccctttcctgaagaagtggacgtcttcaccgccccacactggcggatg
aagcagctggtggggctctactgcgacaagctttctaaaaccaattttttccaacaacaacgatttc
cgtgctcttctgcagtctttgtatgctactttcaaggagttcaaaatgcatgagcagattgaaaat
gaatacattattggtttgcttcaacaacgcagccagaccatttataatgtacattctgacaataaa
ctctccgagatgcttagcctctttgaaaagggactgaagaatgttaagcctactactgttgactgg
aagccttaccaataacataaaacaatcgaataacaattatttcatgtattatatgtaaaatatata
tactggattcttacagtaagaatgaatatgaacagttaaattatgcaaaacaactgaaagagagat
tggaggctttacaagagatttttcttcctcacatg (Seq ID No: 1056)
```

Homo sapiens major histocompatibility complex, class II, DP alpha 1 (HLA-DPA1):

```
ctgcctccactcggcctcagttcctcatcactgttcctgtgctcacagtcatcaattatagacccc
acaacatg (Seq ID No: 1057)
```

Homo sapiens secretory carrier membrane protein 1 (SCAMP1): tcgtctctctctctgcgcctgggtcgggtgggtgacgccgagagccagagagatg (Seq ID No: 1058)

Homo sapiens chromosome 15 open reading frame 57 (C15orf57):

```
ccgcccctcccgatttcctccgggctacaggcgacagagctgagccaagcgtttactgggcagctg
ttacggtaagtgaggagggggctggggtgcccagcgttttggatctcccactctggcccggccccgg
aataccacatagaggccttgggacctgattcatcccgtccagacagccctagagacctgagcgact
gaggcctgggatctggacgccggaatttcctgcgtggttctggacgccctgccctgggctcagatt
ccaaatg (Seq ID No: 1059)
```

Homo sapiens WD repeat and FYVE domain containing 2 (WDFY2):

```
cctcctcttgtagtggcgccggcttgcatcccaggtcgtggcggttttggtgcctgaagcagggag
cgcggagtcgttcccgagagaggcggccaggctatgctcgccggtttccggcgttccgctccggcc
agccagagtctctgtctcaacctgtgtccgtgctccagcagtctcctcagcccggccccgcggcgc
ggttggcggcggcgccccaggcgcgccccctcctccgatg (Seq ID No: 1060)
```

Homo sapiens topoisomerase (DNA) I, mitochondrial (TOP1MT): cgctctttcccggaggctggcagatg (Seq ID No: 1061)

Homo sapiens intraflagellar transport 122 homolog (Chlamydomonas) (IFT122) :

```
ctttccctttcggacatgcgcgctcggagcaaggcgccctcgcactcagcttaccgcgcatgtacg
ttgccaggggtaacgcaggtagccaaagtggcttgtggagtggcgaccgttagtgaggcggttgct
```

```
gagacagacgctgaggcgggtaggaggagcccgagccgtaagggaagccgtgatg
(Seq ID No: 1062)
```

Homo sapiens mitochondrial ribosomal protein L53 (MRPL53): agttcttccggggcggaggtcaccatg (Seq ID No: 1063)

Homo sapiens T-cell activation RhoGTPase activating protein (TA-GAP):

```
ccgccccttcgcttataatgcagagcatgtgaagggagaccggctcggtctctctctctcccagtg
gactagaaggagcagagagttatgctgtttctcccattctttacagctcaccggatgtaaaagaac
tctggctagagaccctccaaggacagaggcacagccacacgggagtgaaatccacccctggacagt
cagccgcaatactgatgaagctgagaagcagccacaatgcttcaaaaacactaaacgccaataata
tggagacactaatcgaatgtcaatcagagggtgatatcaaggaacatcccctgttggcatcatgtg
agagtgaagacagtatttgccagctcattggacattctcactattctatgccttaaaggcccttca
acggaagggatattcaggagagcagccaacgagaaagcccgtaaggagctgaaggaggagctcaac
tctggggatgcggtggatctggagaggctccccgtgcacctcctcgctgtggtctttaaggacttc
ctcagaagtatcccccggaagctactttcaagcgacctctttgaggagtggatg
(Seq ID No: 1064)
```

Homo sapiens phosphoserine aminotransferase 1 (PSAT1): ggtcctccttggctgactcaccgccctggccgccgcaccatg (Seq ID No: 1065)

Homo sapiens CD97 molecule (CD97):

```
cccccctccttcataaagtcctggcctcgggacagcctgcacagctgcctagcctgtggagacggga
cagccctgtcccactcactctttcccctgccgctcctgccggcagctccaaccatg
(Seq ID No: 1066)
```

Homo sapiens protein tyrosine phosphatase, non-receptor type 2 (PTPN2):

```
cgctctccccggatcgtgcggggcctgagcctctccgccggcgcaggctctgctcgcgccagctcg
ctcccgcagccatg (Seq ID No: 1067)
```

Homo sapiens chromosome 20 open reading frame 112 (C20orf112):

```
gcccctctccccgggcagccgcggcggcagcagcagcagcagcagctggagctgtggggctgtcac
cgccgcccgccccgctcactcgcggatcccgaccgcccatctccgcctcgcttccagcccaggatg
agacttctgtgagcagcgaggattttgatatg (Seq ID No: 1068)
```

Homo sapiens APEX nuclease (multifunctional DNA repair enzyme) 1 (APEX1):

```
cacccttctttgtgctcgggttaggaggagctaggctgccatcgggccggtgcagatacggggttg
ctcttttgctcataagaggggcttcgctggcagtctgaacggcaagcttgagtcaggacccttaat
taagatcctcaattggctggagggcagatctcgcgagtagggcaacgcggtaaaaatattgcttcg
gtgggtgacgcggtacagctgcccaagggcgttcgtaacgggaatg (Seq ID No: 1069)
```

Homo sapiens intermediate filament family orphan 1 (IFFO1):

```
tttcctcttgagccatcatgcacatctgactgcagccccagcgagcccttccttccttgtctgact
gctcttcttctcgatttcttcttgttctgccttctcggtttgcagccctgaccccgctgtgtgtc
tggcccttggtgactgtccgtgtttctgttcctgtcattgtaactgtgacttttctctctgtctgc
ccccccttcctactggttcatgcttctcccccattcccaccctctctgcccggcctcccgctcccg
ccctttctcctcatgcacccggcctcgtctctgtagtctctgcacttgtctcccattaaggtccca
tccatg (Seq ID No: 1070)
```

Homo sapiens neuralized homolog 2 (Drosophila) (NEURL2):

```
cagtcttcctcccgcccttctttggtccctacggacctggggggcggtggcggtcaatgccgggt
```

```
caaggtccgcgggcctcgcagatcgtagcccgggcgcacgcgatcagatgatcctgttgtggacgg
ctaagttgtaggcgggatggctgagaaagcggcgctaggaccccgggcagaggctcggggaaggg
agtcagggggaaatgccttacaaggtcgccttgcggtcaccatcattgcccgccgcccaaaatag
cccccggcgccagctggcctgccctatggccgagagatg (Seq ID No: 1071)
```

Homo sapiens drebrin 1 (DBN1):

```
ctccctctttccctccctcctcctccgtccgcccgtccgtccgcgcgtctgtccgttcggcccggt
ccggcccgaagcatg (Seq ID No: 1072)
```

Homo sapiens WW domain containing adaptor with coiled-coil (WAC):

```
cagcctcccttatttagtccgcgatggcttccctcgcgccccaccgtcctcttccggaaggcggct
ccctccctgcgcagcccggagcccctgagatcagcctcgagcaggcgcccgagcgagactatccct
aaacgggaacggcggtggccgactcgcgagtgaggaaaagaaggaaagggcagactggtcgcgaag
agaagatccaggcctcagaggaggagaaaggccggagccagccgaggtttgccgagggcggtgttc
cggacccgcgcggtgcggggaggaaggccgagggtgggagaggagggggcccggcggaaactgccga
ggtttcccgaaggcggcagcgtccgagttgcccggatgtagttggtggagcggcagcggcggcacc
agcggcggcggcggcggcgggaggaggaggaggagaagaaggaccaggcggcggcagcagcggcgg
cggcggggggaggaggggaggaggcggcggagcaggaggaggagaaggcggaggaggcagtcgctc
tccgcggggctgagccggacgcgtcgtcttgcccccctccccccggttcgcggtgccgccgtgtag
ttggcgccgctgccccggctgagagtgagcgtggtgtcgacggagggagatggcccgggagcgccg
gcgccagtaactgggagctgatgagagtcgccgagggcgcgccgggcccaggtgccggggctgccc
gccgcccgccgccgccgcctgcgcgcccgcccgcctttcgcggccgctctcccccctccccga
cacacactcacaggccgggcattgatg (Seq ID No: 1073)
```

Homo sapiens kelch-like 6 (Drosophila) (KLHL6): cgctccttcagtctcgatg (Seq ID No: 1074)

Homo sapiens GTPase, IMAP family member 1 (GIMAP1):

```
cagccttctgcactcacagccgaagggaaagcagcaggttggggcttcttgtggccaacttcagag
cctgtcaccaggaaaggtaagcatg (Seq ID No: 1075)
```

Homo sapiens RAB24, member RAS oncogene family (RAB24):

```
cgccctctagccccctcccgcgggagtcgcggcgctgcgggtaggagccgggttgcgggagacccc
aggttcggttgggattcccagccagaacggagcttaagccgggcaggcgagcgaatgacggagtag
cgagctgcacggcggcgtgctgcgctgttgaggacgctgtcccgcgcgctcccaggccgccccgag
gcttggggtcttcgaaggataatcggcgcccggggccgaacagcgggggcacacggggcgctgccg
aagtgcaaggccacggccagagctcgagcccgacgcgctgtctggagtcgtaggaccctgacgtgg
ctgaagcggccccgggagcatg (Seq ID No: 1076)
```

Homo sapiens adaptor-related protein complex 2, alpha 1 subunit (AP2A1):

```
agccctccccgcggccggctcggctccttggcgctgcctggggtcctttccgcccggtccccgctt
gccagcccccgctgctctgtgccctgtccggccaggcctggagccgacaccaccgccatcatg
(Seq ID No: 1077)
```

Homo sapiens copine IV (CPNE4):

```
ctccctcttttctcagtaccctcctctttactctccgagttaactgagagccgacctgacatctcc
aacattttcaccctcttcccccaccccatcaccgagaatggagtcagggtttccggagagaccga
actctgctctcagcacctttcccagccgctgttgctaaactgacctcggaggacgagaggggaagg
aggtgcgacgccccttacatcagtacataactaccacaccaaccacctccacttcaaagccggatt
ttgcatcctggggggcgggacagacctcgtcccgggctgaattctctctccactcttcgagattggc
acacccagaatg (Seq ID No: 1078)
```

Homo sapiens synaptosomal-associated protein, 25kDa (SNAP25):

```
ctgtctttccttccctccctgctcggcggctccaccacagttgcaacctgcagaggcccggagaac
acaaccctcccgagaagcccaggtccagagccaaacccgtcactgacccccagcccaggcgccca
gccactccccaccgctaccatg (Seq ID No: 1079)
```

Homo sapiens cAMP responsive element binding protein 3-like 4 (CREB3L4):

```
aggtctcttgactctttccgcctttgtttacaaccctgccatgatctccctcttgcaaaagcgagg
gctacagaacaggcattcaggagtcctgtgctccagtcacagccttttctgttcttcagctaggag
acaccaaaccctcaggaagatttactatagctaagagaaaactgcagcagaaagggcgcggctacc
tacttcttaaattccgtttgtggaccctcagactcttagtcccctactcccagatacagcggccct
accgtggctcctggcaaggtggcatccacttttgtagtaagcatg (Seq ID No: 1080)
```

Homo sapiens leucine-rich pentatricopeptide repeat containing (LRPPRC): ctgtccttctggcggagcgtgcttcccgctgcggggacgttcgagcaatg (Seq ID No: 1081)

Homo sapiens zinc finger protein 418 (ZNF418):

```
cgttctctggtagcgaccattttggttaatgttgggtgtgtttctgcggtttgtgaggtgagaggc
gctggagctatgggtccgaaccgcggtgtctgaacccagaaggtgaagagtccttcttgctgcaca
gaggcagatcttaggccccgtaacggcgcccgccgctcccggcagtgctttccccgcgtactcggg
atggcggcggccgcgctgaggctcccggctcaggcatcatctggctgcaaagaagagaacacactg
tgtttgagggaggaggaaggaggatcagagtttaaactcctgccataatg
(Seq ID No: 1082)
```

Homo sapiens tetratricopeptide repeat domain 14 (TTC14):

gtttcttccgcttcctgtaccacccggctcaagtagcggacacggaacagggaactatcagcccgt
cggcctccgggccctgcattctctagccatg (Seq ID No: 1083)

Homo sapiens BMP binding endothelial regulator (BMPER):

agcccttttcgactgtgagctgcggcagctgagcagaggcggcggcgcgggacctgcagtcgccag
ggattccctccaggtgacgatg (Seq ID No: 1084)

Homo sapiens zinc finger protein 384 (ZNF384):

ccccctttttcgtttccggcgctcccgccttctctccgcagagctcttctctgagcctgttgggggg
agggagggggggcgtggaggaactggggttcgcgggagcacgagctgcagcaccacttccgggtgag
tgcaaggggagggcagcaaggaggggggggccacccactacctcgcgcccccgccctgcgggtgtct
cgcgcgcgttccgtgcgtgtgagtgtgtgggtctgtctcgctccagaagtgcgtgcccgcgcgctg
cgccttgcgcttttttcccctccctcgcccccttcctggtcctcccaccctcctcggctccctcctttt
cccagcaaacgccgcccctcccgcgccctggctcaggctctggcgccgccgcagccgtcgccgccc
gaaagttcaggagccctggaaaggagaaggaataagacggcaggaggaagagagagagagggtaga
atg (Seq ID No: 1085)

Homo sapiens RAD51-like 3 (S. cerevisiae) (RAD51L3):

ctctcctttctcctccggcagccagcgcgcctgtgtcctctctaggaaggggtaggggaggggcgt
ctggagaggaccccccgcgaatgcccacgtgacgtgcagtcccctggggctgttccggcctgcgg
ggaacatg (Seq ID No: 1086)

Homo sapiens CD99 molecule-like 2 (CD99L2):

gctcctcctcccgctcctcctcggcctcccccttcgggcgctctcgcgctaactgtgctcctccggg
gccctccgcctgctcccagccatg (Seq ID No: 1087)

Homo sapiens glucosamine-6-phosphate deaminase 2 (GNPDA2):

gcgcctttatctgcatccgggtccgtgggattcgcgctccactggtcagctggggtcgctctcggg

tggttgggtgttgcttgttcccgctgttccagcgtcgaagaaccattgggtctgccggtttgaact
tgttctggaagctgtgcgtcaccgtaatg (Seq ID No: 1088)

Homo sapiens methionyl-tRNA synthetase 2, mitochondrial (MARS2): ccgcctcctccgcttgcggccggtctgcaccatg (Seq ID No: 1089)

Homo sapiens chromosome 12 open reading frame 57 (C12orf57):

tttcctttccgctcccaggggcgttgggaacggttgtaggacgtggctctttattcgtgagttttc
catttacctccgctgaacctagagcttcagacgccctatg (Seq ID No: 1090)

Homo sapiens tRNA-yW synthesizing protein 3 homolog (S. cerevisiae) (TYW3):

ggaccttttcggccaccgctcgcttcaatatggctgcccccagggagagacgaggctaccatgaag
gagccgagcgcagaccctgagtccgtcacccatg (Seq ID No: 1091)

Homo sapiens Spl transcription factor (SP1):

ctccctcctccttacccccccctccctgtccggtccgggttcgcttgcctcgtcagcgtccgcgtt
tttcccggcccccccaacccccccggacaggacccccttgagcttgtccctcagctgccaccatg
(Seq ID No: 1092)

Homo sapiens histidine triad nucleotide binding protein 3 (HINT3):

cgccctctagtggcagccggttttgaggccggcctccggctttgaagttcctcaccgcgtctcctt
ccctctccccaaagcctggatcaccgcccagcgtcaggcgaggggcgacgtctcgaggtaaaacgg
aggaggtgcgggacgcggagactgcgcgggcccggtagccctggagaggccgaggctctaggccgc
gaggggcgggtgcaatg (Seq ID No: 1093)

Homo sapiens M-phase specific PLK1 interacting protein (MPLKIP):

agttctctgcggagggccggttgatacagttccggtgggagaacgcggctgcgaggttttcggctt
tggctcctgatatg (Seq ID No: 1094)

Homo sapiens palmitoyl-protein thioesterase 2 (PPT2):

cacccttccccccgccaccgtgggttccagacttgggataagtaaacagcgggtggagcgaggcct
acggacccaggccaggtgggagtctgcactcttcaaggggcctgggctgctgctcacgggtattaa
agaactccgcgttgttcatggctgaggcgatgcattaggaagatcctggacctagagaacaagtcc
cccgaacgctgagttggaggcgggacttcgggtgcgcgttggcgggagcatg
(Seq ID No: 1095)

Homo sapiens BCL2-like 14 (apoptosis facilitator) (BCL2L14):

aagcctcttttcaggctgagtcctaaacctgaagaaagtttagagcctgggctctaaactacctg
agtctttccaaacgacaagccaagaagacctgttgaaagtttcctcttaagtttcgtggagagaga
ctcaggtatagaaatatccttactgccacctgacctgaagcagaagaaatcacagacagcttccag
accaggcccaacatg (Seq ID No: 1096)

Homo sapiens galactose mutarotase (aldose 1-epimerase) (GALM):

acgccccttctcctgtaaacttgggtcgcctctagcttagcgagcgctggagtttgaagagcgggc
agtggctgcacacgccaaactttccctatg (Seq ID No: 1097)

Homo sapiens carboxymethylenebutenolidase homolog (Pseudomonas) (CMBL):

cttccttcccttccccgactttgcagatttctcttcccccaggcctccctcctccacctctccgcc
ccctccgggcttggctctcccaggaggctacgactggagccactggtcccgcaggatccccgcgtc
ctcggtcgccgcgtccacgtccctctcgcgtccccgcccggcgccacgccgcctcctctgggttcg
gcctccgcgcggtgcagcgcagtctcaggccgcgggacaagcccgacttaaatctctgcaatg
(Seq ID No: 1098)

Homo sapiens chromosome 7 open reading frame 31 (C7orf31):

cgtccttctcccgcccccgcccctgcctgccagctccaccgggccgtaggtgcggacgacctcaaa
attcctcggcccgcgaaggccgccagctgcggggagggagggagggagggcgcggtcccgcagcgcccc
caggctcatgtcccaggtatgtccagacccccgaggcaccgcttgcagggcagtgacagcccgtga
ggctcggcctcgacccctggcacccttggtcccagctacgccggctcctggccttcccccaagtcc
gagagagaggtgggattctccccgacgcagttggaaaccgggaatccccttagggtcccgttcgt
gctgcactactgactccaccatctgcaaagggattcttgtccagaatccccgaaggctttaggaca
gcgcttattttgttgaatgaagagtctctaattttcggaaagaccacaggctaaaagtcaagttgt
gcctttttagccaagaagcatg (Seq ID No: 1099)

Homo sapiens secretory carrier membrane protein 5 (SCAMP5):

cggcctttcggcagccgaacggccgcggcagttcaggacaaagaggtgtgggcaggccactgggcc
agctggtaacatcatg (Seq ID No: 1100)

Homo sapiens mitogen-activated protein kinase 10 (MAPK10):
tgctcctttcggttgccatagcaaccccattccccaagccctctgtccgtctcctctggtaggttc
cacaatggtacaggcagcatcacgctgcacaatggtttccaggcagtgaaagagggtgattcagca
agccactcttcttctattttctttaacctccccttcacttttattttatggggtgtgggtggtgc
ttgctatatgcttacctttttcttttcttttttcattttacaaatttcctttttttgtcctcaccc
ctcaattcctagggcttgagtgagtttaagattgggttttcttggaaatcacctgtccatcgtta
attttaaacaatctccatatctccaaagaatctcttccatgttagtctggaatgtggttaatgaaa
aacaagtagggaggatttctggggcaaacactgccggatcaggatcgtagttctcaggcacggaat
ggctagtgtgagaaacaccaacagcaggcccatctcagatcttcactatggcaacttatgcaagaa
actgttgaattagacccgtttcctatagatgagaaaccatacaagctgtggtatttatgagcctcc
atttcttatactactgcagtgaaccaacattggatgtgaaaattgccttttgtcaggtgtgtgttc
cttacaggtaaaacaagggattcgataaacaagtggatgtgtcatatattgccaaacattacaaca
tg (Seq ID No: 1101)

Homo sapiens beta-site APP-cleaving enzyme 2 (BACE2):

cgtcctccccgccgccgccggtcccggtgcgcgcccatccctgcccgcagccccgcgcgccggccg
agtcgctgagccgcggctgccggacgggacgggaccggctaggctgggcgcgcccccgggccccg
ccgtgggcatg (Seq ID No: 1102)

Homo sapiens SWI/SNF related, matrix associated, actin dependent r egulator of chromatin, subfamily d, member 1 (SMARCD1):

acgccttttccgctagtcgccccgctctatcccatagtctcgctgccctgagcctcccgtgccggc
cggccggccggggggaacaggcgggcgctcggggggcgctcgggggggcggggggagttccggttccg
gttctttgtgcggctgcatcggcggctccgggaagatg (Seq ID No: 1103)

Homo sapiens family with sequence similarity 175, member A (FAM175A): cgtcctcttgtgtagcctgaggcggcggtagcatg (Seq ID No: 1104)

Homo sapiens adenosine deaminase domain containing 1 (testis-specific) (ADAD1):

aggcctcttttgaaagatgcggccctgaccctgtgaacctcgcgcagagcggcctgaagcgagagg
ttgaggctggggaggtgagaaaatg (Seq ID No: 1105)

Homo sapiens acyl-CoA synthetase short-chain family member 2 (ACSS2) :

gcccctctacggaggccccgcctctagttcggcctgtttttctcagtcccggcacccgccgcgaccg
caaaggcggccgcggttctaggaacttgacgtgatg (Seq ID No: 1106)

Homo sapiens multiple coagulation factor deficiency 2 (MCFD2):

cttcccttactcaccggtgtccggaaaggtgaacgctgcgctcgggctgcctcgcctgttacctcc
gccgccgggcatg (Seq ID No: 1107)

Homo sapiens SPOC domain containing 1 (SPOCD1):

gctccttttcagctagtgggtggaaccccaggagggaaaactcagggaagcccagggcccgtgttg
tgcttttggcccaggtaggtggacagacatg (Seq ID No: 1108)

Homo sapiens LY6/PLAUR domain containing 1 (LYPD1):

agttccttcagtctcagccgccaactccggaggcgcggtgctcggcccgggagcgcgagcgggagg
agcagagacccgcagccgggagcccgagcgcgggcgatgcaggctccgcgagcggcacctgcggct
cctctaagctacgaccgtcgtctccgcggcagcagcgcgggcccagcagcctcggcagccacagc
cgctgcagccggggcagcctccgctgctgtcgcctcctctgatgcgcttgccctctcccggccccg
ggactccgggagaatg (Seq ID No: 1109)

Homo sapiens cytochrome b5 domain containing 1 (CYB5D1):

cattctttcatactgcctcctcccttgtttttctgtctcagagagatagtctgtcctaaatatccc
atgtagcccaggccactgaattaaaacggagcgtattcgttctctgccccaccccgcaactcctga
aagcggcgcaactcaattacttgatccttatatgccccacgcgggactcatactacgtttcccgtg
aacacgtgcagtccaaaccccgcccctgatatttatctcagtggacggtggccggaaaaggacaat
ggtttccatgtcagcggataaacgctctcccctcggctcccggacgcgacggaggtcgtagtagta
gtgagtacgtgctgaggagcaaaggagtaaccaagagatccagtgaccgacagagcaagagccatg
(Seq ID No: 1110)

Homo sapiens synaptoporin (SYNPR):

tctcctcctttgcttcataaaaagagggacaagtggctggtgctgtggacagagaagctttatttt
tagtatgagacaacctctattttctttcaggagagggaagttggattatcaattcttttgtaaatg
(Seq ID No: 1111)

Homo sapiens heterogeneous nuclear ribonucleoprotein U-like 1 (HNRPUL1):

cccccccctttcccccttcgcctcctgacaggaaaggtttaaggggggacagagccctgggaggccgg
gccgggctcggggggccaccccggggggcccgggccatg (Seq ID No: 1112)

Homo sapiens schlafen family member 5 (SLFN5):

ggttctctgctctggacttggggaggctccgttgcctgctcccggagggagacgcgctgccgaggag
aacccagcgggagaacatttcaggataggaataggccaagtgctgagaagatg
(Seq ID No: 1113)

Homo sapiens MAS-related GPR, member F (MRGPRF):

ccatctcttccagcaggagagggctctactctgagctcctattttccaaggctccgggccgcgctc
ggcgctggcctgctgccccggcgggtccgccggccggaggcgggagtcacaggaagagccctccac
aaaaggaggcctcggcggatcaggacagctgcaggtggggtgtgcagactggtgagctgccagcagg
ggcccagacgcgccaggcctggagatg (Seq ID No: 1114)

Homo sapiens ubiquitin-like domain containing CTD phosphatase 1 (UBLCP1):

cggtctctcagcggccggtttctgcgtccgctgccgcaggttccaccgcgctccaggtattttttt
ttctgaaggaaagctgcttcctcatatgtttcaagaatg (Seq ID No: 1115)

Homo sapiens Rab interacting lysosomal protein-like 2 (RILPL2):

cctccttttccgttgtcccttcgcgccccaaaccacatcctggagcgcactctccagcgtggctgg
cagcggggacggtgcgccggggcgcaggcccaagagtcgcgtgcgcggccccttgcaccatccccc
cgggcccaccccgggccgcgctgattgggcaggtagggactctgcccagcggaaagtttgggtg

ccgggaggaagtctaacctttgggagactccaagacagcagctccgaggtcggcggggggtctgggt
ggccatg (Seq ID No: 1116)

Homo sapiens zinc finger with UFM1-specific peptidase domain (ZUFSP):

acttcttttccgtgggagtaaggaagtgcttttgaatgaggtactgagggccaaggtgttggaagt
tcctaattctttcctcggttaactgtgaaactctgcgtattgggaaggcctggcctcagtcatcag
gccaggagaggtactggacgccgcgcacgcactcgtctgccagcgaggcccaaaggggaagcctag
cggagctcagtgtggcagctgctggcctctgggccgctacttgtcaataccatg
(Seq ID No: 1117)

Homo sapiens mitogen-activated protein kinase kinase 5 (MAP2K5):

ccgccttcctcctcctcctctcgccgctaccgccgtcgccgccgccgcagccgccgccggtccgcg
cggcctcgggtggccggagctcagcctgcgcgcgccgcgccctgtgtctccgggtggggcagaaga
ctcgccccttgaacctcccgcggggactctccgtggtgtggcggccctggggctctttcttaatag
ccccggactgagtcccctccagtcgaggaccctctcctagtccactgacgagcggtggacacctgc
cgctgtatctcccccaaaccgagtccttgccctgctgcctcctcatacccacacggcggcagagac
cttcaccatagcgttcgctcaactccagaaccttccgacctccgctagttcctgcgggcctttgcc
cgcttcccggtgcaccctccccgggagacacctcagacccccgacagcctgggcaggctcggtgcc
tgcgggtgcgttcctgatcacccctcccctcttccctccccctcatcctccattcccttgttttca
ccctctgtcctctgcccgtcactccccttgtcacctcttggagcccctcctaaccagcggccagt
gggtttcccataccccaggatgtgagcctctttaacctgtaatg (Seq ID No: 1118)

Homo sapiens solute carrier family 2 (facilitated glucose transporter), member 12 (SLC2A12):

cactcttctttagcatgctattatggggaaagtgaccactcctgggagcgggggtggtcggggcgg
tttggtggcggggaagcggctgtaacttctacgtgaccatg (Seq ID No: 1119)

Homo sapiens mitochondrial ribosomal protein L30 (MRPL30):

cttcctctgctctgcttcccttcggaggaaaatttcaggctgaaggtttagcgggtgccgcctcta
aagagagcaatcactacacttatg (Seq ID No: 1120)

Homo sapiens tripartite motif containing 11 (TRIM11):

```
gctcctcttcctgccggcatccgggatccctacgtcccgcgtcccccgagcgctcggagcctacgc
gcccagcgctaccgaaacccagagtcctgcgccctggagtccccgcgccccggagcccgagcaccc
gggagtcccgagcctcgcgccccggagtgcccgagcctgcgccgccgcacccggatacccgcgtc
cccgcgagctgccgaggccgcccgccgccgccccgcggacagtaccgccttcctcccctctgtccg
cgccatg (Seq ID No: 1121)
```

Homo sapiens proline-rich transmembrane protein 2 (PRRT2):

```
ctccctccctagctgacttgctccctcccgggctgcggctgctgcaaaagccagcagcggcagcgg
gagctgtccggaggccggcgtcgagggtttgccgctgtctctgctattccatcctccccatagggg
ctctctccctctcccatctcaagatg (Seq ID No: 1122)
```

Homo sapiens zinc finger protein 626 (ZNF626):

```
cggcctttgtctctcgctgcagtcagagctccaggtctggttcttctcctaaaggcccaggctgtg
tggccccgtgtcctgcaggtattgggagatccacagctaagacaccgggacctcctggaagccaaa
aatg (Seq ID No: 1123)
```

Homo sapiens solute carrier family 25, member 43 (SLC25A43): cggtcttccgggcccgggtcggggctcgatg (Seq ID No: 1124)

Homo sapiens crystallin, zeta (quinone reductase)-like 1 (CRYZL1):

```
ggctctctgacgaaggactggaaggtggcggtggtgaaggtgcaggccgttggggcggctcagagg
caggtgactatg (Seq ID No: 1125)
```

Homo sapiens mitogen-activated protein kinase kinase kinase 7 (MAP3K7):

```
ctgcctctaccccgccacggatcgccgggtagtaggactgcgcggctccaggctgagggtcggtc
cggaggcgggtgggcgcgggtctcacccggattgtccgggtggcaccgttcccggccccaccgggc
gccgcgagggatcatg (Seq ID No: 1126)
```

Homo sapiens septin 6 (SEPT6):

```
ctttctctttgtcggaggagctcctctgtttcctgtgcagtagctcccgttgcggcggcacccgtg
gcagccctggcggacgcaggagcgatg (Seq ID No: 1127)
```

Homo sapiens myotrophin (MTPN):

```
ctgcctctcctcggccaggcggaacctctctgctgggcccggtggccgcaaaagaactttctttct
cccgcccgaacggtcgccgcggccaactgcctcgcccgcctggcagcctaaccctccttctcttct
tctcctctccggcttcgcgcggccctgcctccctctcgcccggcggcatccgcttgctgctgccac
cgcctcctcatcttctgcccggccaaccggcctgccccgctgcagtgatg
(Seq ID No: 1128)
```

Homo sapiens annexin A11 (ANXA11):

```
ccctcccttgcactgcctctggcacctggggcagccgcgcccgcggagttttccgcccggcgctga
cggctgctgcgcccgcggctccccagtgccccgagtgccccgcgggccccgcgagcgggagtggga
cccagcccctaggcagaacccaggcgccgcgcccgggacgcccgcggagagagccactcccgccca
cgtcccatttcgcccctcgcgtccggagtccccgtggccagggattattggacctgcctggtttaa
actattgtcttagttaattttgtgctgctctaacaaaatatcacagactgagtaatttataagcaa
tagtagcttatttggctcacagttctggaggctgagaagatcgtgaggctgcatctggcaagggcc
ttcttgctgcttcataacatggcagaagacatcatgcgggtgtgtgtctggggaagagacttacag
aagtggagttgctgagtcaaagatctaaccatg (Seq ID No: 1129)
```

Homo sapiens RNA binding protein, fox-1 homolog (C. elegans) 1 (RBFOX1):

```
ttttctttctttcctctcccggcgttgatgagtgcttggctcctgacagaagggatttggctccca
gctttgtagttcggaagaagttgggtctatagatttcccccctaactctccattgatgtgttgagct
tcagagggaataataactctacgtaaagcatg (Seq ID No: 1130)
```

Homo sapiens prefoldin subunit 5 (PFDN5): cttcctcttcgttaagtcggccttcccaacatg (Seq ID No: 1131)

Homo sapiens high mobility group AT-hook 1 (HMGA1):

```
cgctcttttttaagctcccctgagccggtgctgcgctcctctaattgggactccgagccggggctat
ttctggcgctggcgcggctccaagaaggcatccgcatttgctaccagcggcggccgcggcggagcc
aggccggtcctcagcgcccagcaccgccgctcccggcaacccggagcgcgcaccgcaggccggcgg
ccgagctcgcgcatcccagccatcactcttccacctgctccttagagaagggaagatg
(Seq ID No: 1132)
```

Homo sapiens zinc finger protein 323 (ZNF323):

```
cggcctttgcggttgatcggtcattgggggtgctgcagccccgccacctgttccgtagcttgccggt
gccccgaaggtgtcttctcctaaggaagattaaatcagaaaattttaaatcacagttatcccttta
cttaaagccagagtaagccttccaaattaaccccaggaatg (Seq ID No: 1133)
```

Homo sapiens tumor protein p53 inducible protein 3 (TP53I3):

```
ctttctcttctcttagcagcacccagcttgcccacccatgctcaagatgggcgggatgccagcctg
```

```
ttacataaatgtgccaaaagcctggccatgcctggaaaatggaccaatccgcccgccaagaggttg
ggtctcgttccctagagagaaggaagtttcctctccttgaagtgagagctagaatcgcactttctg
tcaagctgagagaaagactcttttccagaggctaaaaggacaagaaaatctgatttgcttgcttct
aactttgcgttttaaaggggggaaggaggaaaggaaagaggggggagggtggttctgcttagccccac
ccctccggctaccccaggtccagccgtccattccggtggaggcagaggcagtcctggggctctggg
gctcgggctttgtcaccgggacccgcaggagccagaaccactcggcgccgcctggtgcatgggagg
ggagccgggccaggaacaatatg (Seq ID No: 1134)
```

Homo sapiens ceramide synthase 5 (CERS5):

```
ccgcctccccgcgggttccgttggctgtggcggcagctgacgcttgtggcggcggtggcttcgggg
tgggcgtaagatg (Seq ID No: 1135)
```

Homo sapiens TRAF3 interacting protein 2 (TRAF3IP2):

tgttcttctacttacctgggcccggagaaggtggagggagacgagaagccgccgagagccgactac
cctccgggcccagtctgtctgtccgtggtggatctaagaaactagaatg
(Seq ID No: 1136)

Homo sapiens Smith-Magenis syndrome chromosome region, candidate 7 (SMCR7):

ggtccttcacgttccattcccaggctggtctgagctccggggccgtggtcccgctgcctcctccgg
tcgtcgtgcggaagctgcgacgcaggcagaccatg (Seq ID No: 1137)

Homo sapiens mitochondrial ribosomal protein L10 (MRPL10):

cattcttccggtggagatggctgcggccgtggcggggatgctgcgagggggtctcctgccccaggc
gggctagagtgcagtggcatg (Seq ID No: 1138)

Homo sapiens proteasome (pro-some, macropain) subunit, alpha type, 1 (PSMA1):

acttctctgtagatcgctgagcgatactttcggcagcacctccttgattctcagttttgctggagg
ccgcaaccaggcccgcgccgccaccatg (Seq ID No: 1139)

Homo sapiens sorting nexin 5 (SNX5):

cggtctttctctagacgcgtcttgctgggagagtgtccgttgcttcccgtccgtgtcgcggccctg
cggttggcggcctcctcgtggagcggagcaaggccaggcggcccctgctcgagtcccgcgtcgcca
tg (Seq ID No: 1140)

Homo sapiens zinc finger protein 276 (ZNF276):

gggcccctccgcgcgtactgcgggcccacgggtgttagtggcggggggcggcagagtccgggtgg
gttgtcgcgacggagccgggcctcttcgccgtcttgagacggggctggcgagaagggcccctcacg
gagttgccatgggcgtctaaccgcggcagccaggcccctctctacgtgagaccccggccccccctcc
cctttctgcagcccgcccgccacctgcgcgccgcgtggcctccgccggcgcctgcccgccccgcgc
ctccgtctcccacggagcaggccgggctctcgccatg (Seq ID No: 1141)

Homo sapiens zinc finger protein 561 (ZNF561):

ccatcttttccggcgctggctcctctccgtcagtgcggtttcgcctttatggtggtggagtctgcc
caggctgtggaccgcaaataaccctgtacaaagaggaatggagattgcctctatccacctagattc
ataagctggcctgaggtgatcttggcatcaaggaagggatgcacatcatcacaccatcagcttcag
agaatg (Seq ID No: 1142)

Homo sapiens mucin 7, secreted (MUC7):

ctttctcttcttttgcttctagttaccatcctcaaaggattggctaaaagcaagcaactggattga
acaccctaagaagaaagattcacactgcaccaggagacatcagaaagaatg
(Seq ID No: 1143)

Homo sapiens threonyl-tRNA synthetase (TARS):

```
gcgcctttcgattgcatcagctggtccagccgaggccaagtcccgggcgctagcccacctcccacc
cgcctcttggctcctctcctctaggccgtcgctttcgggttctctcatcgcttcgtcgttcgccaa
tg (Seq ID No: 1144)
```

Homo sapiens ATPase, Na+/K+ transporting, alpha 3 polypeptide (ATP1A3):

```
cagcctctgtgcggtgggaccaacggacggacggacggacgcgcgcacctaccgaggcgcgggcgc
tgcagaggctcccagcccaagcctgagcctgagcccgccccgaggtccccgccccgcccgcctggc
tctctcgccgcggagccgccaagatg (Seq ID No: 1145)
```

Homo sapiens chromosome 11 open reading frame 46 (C11orf46):

```
cgtcctctcagtggtagcgcgggggactggctgggaagcggtcggtcgagtgtggcctgtgtggact
cgcatcttgcccgaagccgggcggaggagagctcaagctaagggtgatcagcccatgacctaaacc
tccagacaaaataaaacggaaaatttgctagaatcaagaatg (Seq ID No: 1146)
```

Homo sapiens chromosome 17 open reading frame 45 (C17orf45):

```
tgacctttttcattcccgttgttatggaggtaggctctctaggaatctgggagtagtagctggggggg
caagagcaaataaagagctcgagcttctgtggtctctggggagatg (Seq ID No: 1147)
```

Homo sapiens AHA1, activator of heat shock 90kDa protein ATPase ho molog 2 (yeast) (AHSA2):

```
gggccttctggcagtttctgggagctgcgaacgcgccgccccgggggctcggcggccggaaacgctg
gcttcggagccttaggcgccgcggcctttccttgtttttccgcccagtccacgccgccatggccaag
tggggccagggggaaccccccactggatcgtggaggagcgggaggacgggaccaacgtgaacaactgg
cgctggcgcggctggcggcggcctccttccgggatctggggagggccgggccgcgggagccggggc
tgccctggggtctgtgcggggccgcggggccaggggggtcaggggggccgcccccccctcagctgctgg
acgcagggctcggccttcgcctctcggctcgggagagtccttgagtacggagaccggctaggaggg
ttgcagctgcctcttttttgaaagttgggttgggccccaagagtgacttccgacagacctttccact
cccaccgtctgtggcctgagggccttcccttctcctcccgcccaccccctctggatgtttcggggag
ttagaagggagctggattgagagactgtgttaggggcgggggtatggaacgtagtggaaagggcag
aaatttggatctcagttcgcgcccacccccgcaggcgcctcccgcgagccgggccctctgtgagtga
gacaagctccccttcctttacgcgcctcacctggcgcgtggggagaggtcggcagccctccgccgc
agaacctccggaagggatgtcctctgccctgcgcctctggccggggctgtggtccctccaggccgt
cgaggggatgctgaggccggtccccagaggagcatgacttggctggtccggaggagctctgagggc
atgggcaatcttggctcgctgcaacctcagcttccagagttcaagcgagtctcctgcttcagcctc
atgagtagctgggactacagatgcgtgccactacgtccgtctgatgtttgtattttttagtagagac
agggtttcaccatgttggtcaggctgctctcgaactccagatctcgtgatccgcccgcctgggcct
actaaagtgctgggattacaggcgtgagctagatctgactttctagtgtcctagccttggcccgat
ggacatgtcatttctctcagctcgtttctgtcccctaaagtgagaatattgcctgggaagattaca
ttagacgatgtatatgcgaagacacttgatagctggtattgtcatgattctgattagttcactact
gctactttccctgtggcctaggctttgcctatttccagtgggcgagctagctagatcctcctccct
taaataagccagtgtttttaagacagaatactacttgcatagtggacaataatatcttaaagaact
gagcaggatgaaaagaatttgatagaaagcaggtttgaggagcacattggaggttggcaggtttcg
aggctgcttgagaggacttgggccgatctgggctgggcttggacgtgaccctggcacccaggcagg
tggatcccagctggggcttccattcacgactttctggtccctggcaggacagagcgggatgccacc
agcttgtccaaagggaagttccaggagctcctggtgggcatcgttgtggagaatgacgctggccgc
ggcgagatcaacgagttgaagcaggtggaagggggaggcttcgtgcagcagccgcaaaggaaagctg
attttcttctatgagtggaacatcaaactgggctggaaaggcatcgttaaagaatctggagtgaag
cacaagggattgattgaaatacccaatctttctgaggaaaatgaagtagatgacactgagaattta
caacgggaatg (Seq ID No: 1148)
```

Homo sapiens GrpE-like 2, mitochondrial (E. coli) (GRPEL2): ctgcctctcagcccaaattggaaacatg (Seq ID No: 1149)

Homo sapiens xyloside xylosyltransferase 1 (XXYLT1):

```
ccgccccctttcatggccgccgcctggcgccggggctaagtggccgccggcgtccgggtacccgagg
gctctcccgcgttgctggcaccgctggcgccgcggtctcgtagcgcatg
(Seq ID No: 1150)
```

Homo sapiens chromosome 7 open reading frame 60 (C7orf60):

```
cctcctctggctgctgcctccgcagctccctcctcctaccccacctcctccatctggggagcgtct
gcggggccctgaggggcggcggcggcggcggcggctgcgatatg (Seq ID No: 1151)
```

Homo sapiens tetratricopeptide repeat domain 39B (TTC39B):

```
ccctcctttgcgctgggctgagcccagagccgagagcaggggtcggctctgagttccctgcttggt
ttttgggtggcagcagccagaggaggaatatg (Seq ID No: 1152)
```

Homo sapiens motile sperm domain containing 2 (MOSPD2):

```
cacccttctctgtctacctctgggcgggactgccgggtgatgagatactcggtcggcgacggtaga
acgggcgacggcgacaaccgcaatcacatccacgacggtgatcatg (Seq ID No: 1153)
```

Homo sapiens major facilitator superfamily domain containing 6-lik e (MFSD6L):

```
ggcccctttcggtccaacggcaggacctggggggctgtggccggggggcggccgttgacctggtgacc
gcggcgccgccccagaccggggggcgcagtcccactcgctccgagcccccggtcccccaagcctccct
cccgggtacctggggccgcgcccgccctgcgcccagctccgccctccgtcggcccaggcctgacag
agcccggcagccatg (Seq ID No: 1154)
```

Homo sapiens consortin, connexin sorting protein (CNST):

```
cttcctctctagccgccagtgctctatgctccgcggtcgcgggccgccagcctccagccggccagc .
cgcgaggggtgcgcagagggaggcggggcggaaaggcgagaggtgtctcctccaccggagccaggg
gagacccgagcaagctccgtgacagcacgtcggccgccatgtcgccgagtggggctggaaacagac
ccggcgcccagcggtagccctccttgcgcctccgattcccagacatggaaggtctttaatgtaact
ttaaatggttcaccaaaggatgctctaatg (Seq ID No: 1155)
```

Homo sapiens zinc finger protein 92 (ZNF92):

```
gggcctttgtctctcgctgcagccggcgctccacgtctagtcttcactgctctgcgtcctgtgctg
ataaaggctcgccgctgtgaccctgttacctgcaagaacttggaggttcacagctaagacgccagg
accccctggaagcctagaaatg (Seq ID No: 1156)
```

Homo sapiens DnaJ (Hsp40) homolog, subfamily C, member 18 (DNAJC18): ccccttctctttcagcctcgggcacggggggaggctcggcggacctgctgattgggaaccgatatg (Seq ID No: 1157)

Homo sapiens polymerase (RNA) I polypeptide D, 16kDa (POLR1D):

cctcctccctccttccgtcctccgcgccttccgtcggtcggtccttgcttcctgcttcgcctccgc
gcctcgcgctatgggacagagcccccgatccgccagcaccacctgaggatccagaaaccgccccag
cgatg (Seq ID No: 1158)

Homo sapiens ring finger protein 182 (RNF182):

acctccctcccctcccaggcgccgccgcagccggagcggctcccgggccctgggccgccgccggcc
aggaagaaatacttgtgttggctgcatttccagggatgctaccagagctcaaggctgtcacctggt
cttgcccagaagagccgttcttagaggcaggacttgatgaaggctttcctgctgatggaataggtt
tgctagagctggccttggaattagaacccttcatgtggcctttataaatatgcgtttgagacagag

ttatatgcagaagttgaaaatgcctggaagatttctggtttctttcactacttatcctgcttttt
gcatcgctgccagatttggatgatatgatattcagaggggcaccttaatcaaagccattcttcaac
aagacccacctggcataagattgcacacataattcaagatg (Seq ID No: 1159)

Homo sapiens transmembrane protein 18 (TMEM18):

cctcctctgtggattctggccaggccgggttcggcggttgctgtgagagcgggcttcccaacacca
tg (Seq ID No: 1160)

Homo sapiens Hermansky-Pudlak syndrome 4 (HPS4):

aggcctctctgccgcgcgcgcaggtacggggcagaagtcgcaggtacccagctgctgcccacattt
ctggtccagagtcccgaaccccgagcactgggatgcctggctactccgagccaaggcactgatgtt
tgaactggaaacttcaaaacgtttaataagagtcttcaggatgggtttgaactagacaagctagaa
atttctttagaacaccagctctagcatgcatctcccacttttggctttcctggagaggagcttgaa
gaggtggttctgcagacagccacagtgatacttaggaaaccagaggaatggatttgacttttctgc
taggattctctgttatagtttctccctgagttgtaagaggcatggaaatatacatgaaactgaaga
acctgcaaggaagggaagtggaactttccatgctgagtgaaaactaaccaagtggcagttgtgact
gaaaacactgaaacctaccacgtccagattcactggattgggggatagaggaacggtcacagctag
ggagaaagaagtgataccggaaaagaaaacctaaatgaagagaatgaggatgactgcacagtagat
g (Seq ID No: 1161)

Homo sapiens PTK7 protein tyrosine kinase 7 (PTK7): agctcctttcctgagcccgccgcgatg (Seq ID No: 1162)

Homo sapiens kelch repeat and BTB (POZ) domain containing 6 (KBTBD6):

agttctcctgggcgcctagcattgtcgcccacgctgcagtagcggcttctgcggctccaagccagc
gggtcctgtgaaggcgagcagacgcggagaaaggacgcgggagtgagagagggtgagtcagccact
gtctaaacgataacgggaggcggctctgcggggtagggttgaattcagtaaatgggctcgtgctgc
tgtctcttcggagacgctgctatcttagcgtcagcgagggaaggttgaggaggagccagagccggg
tcctgcagcgtttctcgccatcagcgcccgtcgccatctccaccatg (Seq ID No: 1163)

Homo sapiens sperm antigen with calponin homology and coiled-coil domains 1 (SPECC1):

ctttctttgactggagcggacccgccggacgcaaccgcctcgccagccggagccagcgcgagctcg
gcacggtggacacccggtccgaggccggcaagccggctggtgcccgagtcggccaagcatg
(Seq ID No: 1164)

Homo sapiens ST6 (alpha-N-acetyl-neuraminyl-2,3-beta-galactosyl-1,3)-N-acetylgalactosa minide alpha-2,6-sialyl-transferase 3 (ST6GALNAC3):

ggtccccttatttggatctgcgggaatgtgggctggagaggtcctgccgtggtaccagcctccagc
ctgcccccaggactgcccctgacccaggcgcgcccgctgctcggtggcaggagggccggcggagcg
ccatg (Seq ID No: 1165)

Homo sapiens transportin 1 (TNPO1):

gattctctttgttccgcagccatttcaggccccggacaggaggcagtgccgcttcggccgaaggcc
cgagcgcccgaggcgtctgggatg (Seq ID No: 1166)

Homo sapiens heat shock 70kDa protein 8 (HSPA8): cttccttcgttattggagccaggcctacaccccagcaaccatg (Seq ID No: 1167)

Homo sapiens hyaluronoglucosaminidase 1 (HYAL1):

ggctccttcctccaggagtctctggtgcagctggggtggaatctggccaggccctgcttaggcccc
catcctggggtcaggaaatttggaggataaggcccttcagccccaaggacatcctggctgccatac

ctgctcctgacttctcagggctggcagtcatcgactgggaggcatggcgcccacgctgggccttca
actgggacaccaaggacatttaccggcagcgctcacgggcactggtacaggcacagcaccctgatt
ggccagctcctcaggtggaggcagtagcccaggaccagttccagggagctgcacgggcctggatg
(Seq ID No: 1168)

Homo sapiens STE20-related kinase adaptor alpha (STRADA):

agtcctcccggtcgccccactgcgcatggcacgttgcgtactcccctcccagcaaccggtctggcg
gcggcgcggcagtaaaactgaggaggcggagccaagacggtcggggctgcttgctaactccaggaa
caggtttaagttttttgaaactgaagtaggcctacacagtaggaactcatg
(Seq ID No: 1169)

Homo sapiens transmembrane protein 161B (TMEM161B):

ccctctctttcgctgtttgagagtctctcggctcaaggaccgggaggtaagaggtttgggactgcc
ccggcaactccagggtgtctggtccacgacctatcctaggcgccatg (Seq ID No: 1170)

Homo sapiens Usher syndrome 1C (autosomal recessive, severe) (USH1C):

ggctctttccagctcctggcagccgggcacccgaaggaacgggtcgtgcaacgacgcagctggacc
tggcccagccatg (Seq ID No: 1171)

Homo sapiens interleukin 12 receptor, beta 1 (IL12RB1):

cagtcttttctccttgctcagcttcaatgtgttccggagtggggacggggtggctgaacctcgcag
gtggcagagaggctcccctggggctgtggggctctacgtggatccgatg
(Seq ID No: 1172)

Homo sapiens Meis homeobox 2 (MEIS2):

```
atccccttcctctcttttctgttcgccctcttctccctgctcttttttccctttccaccccctcctc
tgttctccctcacctcctgcgcccctccccttcccgggttctgacagtacgatgagctgcccca
ttacggcgggatg (Seq ID No: 1173)
```

Homo sapiens G elongation factor, mitochondrial 2 (GFM2):

```
ttttcttttcgtttagatacattgccttttgcctaggctggcgtcgagacttgaggccgttgcaga
ctttggcgcggctcgcgcctcctgcttcaagagcccagcggtgagagctggcctgcggcacgcggc
ctaatgccagacagtaacagtttggaggatcaagatg (Seq ID No: 1174)
```

Homo sapiens lamin A/C (LMNA):

```
gagcctttgccccggcgtcggtgactcagtgttcgcgggagcgccgcacctacaccagccaaccca
gatcccgaggtccgacagcgcccggcccagatccccacgcctgccaggagcaagccgagagccagc
cggccggcgcactccgactccgagcagtctctgtccttcgacccgagccccgcgccctttccggga
cccctgccccgcgggcagcgctgccaacctgccggccatg (Seq ID No: 1175)
```

Homo sapiens calcium/calmodulin-dependent protein kinase II delta (CAMK2D):

```
cgctctttctctcgccgcgccgtcttgaagccgcgcgggctcgtgagcagcgcgaggccgccaagg
tgcctcgcttcgccggagccgctgccgcccgccggagggaagccggcctcgggcgcgcacgctcgt
cggagccccggcgcgcccccgcgcctgagcctgctgacagcggccgctgggctcaggctgtccgctc
tgggctccgcggcctcggccccgctgcactccacctccgcccccctcggactccctcccctctgctt
ctactcctcctgctccagtgcggatcgtttcgcaactgcttgccactcgtcccgtgcctggctgtt
tttccatttcccggccccctcttcttgagtactttaccccctgcatttggggacagggactggaaa
agggggcgggtggagcgtccagtggagaagaaggaagcgaggcccgcaggaggaggaggatcggcgg
actgtggggaggagacccccacgccaccctttctggtcatctcccctcccgccccgcccctgcgcac
actccctcgcgggcgagctactttcggaccaggaaagtaagagcggccctgggtgacagcgccgcg
gggccagtcccggggttagccgcgcgtctgctcgcttctggtccgtcgcgctcccagccagggcac
agcccggaccgaggatg (Seq ID No: 1176)
```

Homo sapiens calcium/calmodulin-dependent protein kinase II gamma (CAMK2G):

```
ccgtctcctcctcttgctccctcggccgggcggcggtgactgtgcaccgacgtcggcgcgggctgc
accgccgcgtccgcccgcccgccagcatg (Seq ID No: 1177)
```

Homo sapiens interleukin 15 (IL15):

```
ttttcttttcgccaggggttgggactccgggtggcaggcgcccggggggaatcccagctgactcgct
cactgccttcgaagtccggcgcccccccgggagggaactgggtggccgcaccctcccggctgcggtg
gctgtcgccccccaccctgcagccaggactcgatggagaatccattccaatatatggccatgtggc
tctttggagcaatgttccatcatgttccatgctgctgacgtcacatggagcacagaaatcaatgtt
agcagatagccagcccatacaagatcgttttcaactagtggccccactgtgtccggaattgatggg
ttcttggtctcactgacttcaagaatgaagccgcggaccctcgcggtgagtgttacagctcttaag
gtggcgcatctggagtttgttccttctgatgttcggatgtgttcggagtttcttccttctggtggg
ttcgtggtctcgctggctcaggagtgaagctacagaccttcgcggaggcattgtggatggatggct
gctggaaacccttgccatagccagctcttcttcaatacttaaggatttaccgtggctttgagtaa
tgagaatttcgaaaccacatttgagaagtatttccatccagtgctacttgtgtttacttctaaaca
gtcattttctaactgaagctggcattcatgtcttcattttgggatgcagctaatatacccagttgg
cccaaagcacctaacctatagttatataatctgactctcagttcagtttttactctactaatgcctt
catg (Seq ID No: 1178)
```

Homo sapiens protein O-fucosyltransferase 1 (POFUT1): gtccctccttccctccccgactgtgcgccgcggctggctcgggttcccgggccgacatg (Seq ID No: 1179)

Homo sapiens calpain 3, (p94) (CAPN3):

```
cactctctttctctctccctctggcatgcatgctgctggtaggagacccccaagtcaacattgctt
cagaaatcctttagcactcatttctcaggagaacttatggcttcagaatcacagctcggtttttaa
gatggacataacctgtacgaccttctgatgggcttttcaactttgaactggatgtggacacttttct
ctcagatgacagaattactccaacttcccctttgcagttgcttcctttccttgaaggtagctgtat
cttattttctttaaaaagctttttcttccaaagccacttgccatg (Seq ID No: 1180)
```

Homo sapiens PTK2B protein tyrosine kinase 2 beta (PTK2B):

```
agcccttttactcagccacagcctccggagccgttgcacacctacctgcccggccgacttacctgt
acttgccgccgtcccggctcacctggcggtgcccgaggagtagtcgctggagtccgcgcctccctg
ggactgcaatgtgccgatcttagctgctgcctgagaggatg (Seq ID No: 1181)
```

Homo sapiens ST6 beta-galactosamide alpha-2,6-sialyltranferase 1 (ST6GAL1):

```
cttccttccttctccagtcccttccactgtgcgtcttctgtcccccgttcttccccagcggacccc
tctttcgagactccctagtggggtccccagctcccgggcgatcctgcccttgccgagcgcgttttc
tggagtcacctggggggaggggagtcctgggcagggccgggctggggaagacgcctggggcactgcc
cggcgttaacaaagggagccgataccgaccggcgtgggcgcggagcgggcggccgccaccgagcgt
gctgagcaaccgcagcctccgcggccgagagtgcagcgagcaaggggagagccagttgcgcagagc
cctgcaaccagcagtccagggagaagtggtgaatgtcatggagcccagctgaaatggactggcccc
cttgagcctgtcccaagccctggtgccaggtgtccatccccgtgctgagatgagttttgatcatcc
tgagaaaaatgggcccttggcctgcagacccaataaaccttccctcccatggataatagtgctaatt
cctgaggacctgaagggcctgccgcccctggggggattagccagaagcagatgatcatgacgcagtc
ctgaggtttaatggggcacccacagccaacttccaacaagatgtgggcacaaaaactaccattcgc
ctgatg (Seq ID No: 1182)
```

Homo sapiens ubiquitin-conjugating enzyme E2Q family member 2 (UBE2Q2): ctccccttccgcgcccggctccccttccgcgcccctcccgccggagatgaggg-gaagatg (Seq ID No: 1183)

Homo sapiens membrane magnesium transporter 1 (MMGT1): gcttcttttgctgggctgctgctccttcggcatcatg (Seq ID No: 1184)

Homo sapiens PAP associated domain containing 4 (PAPD4):

```
cggtcttccgggtgtctttgacagggttttctacgccgcttttttcggcgacttttttgctcttccgc
ttttttgccaccgcccccaaccttctatatccttgcagcccctaccttttcttgtgttgctcctccc
ctggcagccgtgagggggggttagatctcagccggagccggagctgggcctagctgtcccacgggcc
accactacctcctttggttcgggagaaagctacgaccaagtacgcccagctcgggccttagaactt
ctgaacgggcagtgcgggtaggccctgcttagcccttcccggaggacacctgaccaaaagaggaag
atagtcttgggacccttgcatggtgtttcaaagggtggtgaagaactaaggtagaagaatacatgt
tcacttccagtgaacaagagcatg (Seq ID No: 1185)
```

Homo sapiens chromosome 3 open reading frame 23 (C3orf23):

```
ctcccttctggtgtactgggtgggaggtggaactagtcggacaaagccctcgcgtcggacccttgc
cagaactcaattaatggatgcctcgaagttgacgtacatatatattcagaaatg
(Seq ID No: 1186)
```

Homo sapiens mucosa associated lymphoid tissue lymphoma translocat ion gene 1 (MALT1):

```
cgcccctttgcgcggctggcgcggccagccggccaggctcccctcggcaaacctgtctaattggggg
cggggagcggagcttcctcctctgagggccgtgccgcgctgccagatttgttcttcgcccctgcc
tccgcggctcggaggcgagcggaaggtgccccggggccgaggcccgtgacggggcgggcgggagcc
ccggcagtccggggtcgccggcgaggccatg (Seq ID No: 1187)
```

Homo sapiens UDP glycosyltransferase 3 family, polypeptide A2 (UGT3A2):

```
ctacctctacccacagccagtgcctttggcgcactgaggtgcacagggtcccttagccgggcgcag
ggcgcgcagcccaggctgagatccgcggcttccgtagaagtgagcatg (Seq ID No: 1188)
```

Homo sapiens sodium channel, voltage-gated, type IV, beta subunit (SCN4B):

```
cctcctctcgctctctgcccgctaactttcccgagccccgaccggcggcgcagagctccgggggtag
ctttgtggccgaacgccgacctcgggcggagagcgcggctgtgcccagtatcccatccccgcgacc
cccgcgcgctccggagagaacaggactatg (Seq ID No: 1189)
```

Homo sapiens JAZF zinc finger 1 (JAZF1):

```
tcccctctgcctcccggtggctcctcgctctccttccatctctctcgccccctctccctccgtccc
gtcctcgccgctcccctcaccccgcctctctccccctcccccagcccctcctctcctcaccccacc
cggcctccctccctccctcgcccgcccggcgctcgcagagccgacaccaggggggctctcgatgta
gcaccatg (Seq ID No: 1190)
```

Homo sapiens chromosome 15 open reading frame 55 (C15orf55):

```
ttcccttccttggatccctgtgcacctactggagccaggttactctgggtcctggacctgactgcc
tcattctggaggcttccagacagccacagttagtgcccaaacctgagaggatg
(Seq ID No: 1191)
```

Homo sapiens ras homolog family member C (RHOC): cgccctctcttcctgcagcctgggaacttcagccggctggagccccaccatg (Seq ID No: 1192)

Homo sapiens CTP synthase II (CTPS2):

```
cattctctttccttttccttctctcctgagcgctcctgcagttcctggggcgtagtaggggatcca
caagcgtttgtgaccagtgaagttctttacaagggtgagatctgcacgggaggacccgagcgaggg
tctcggcttgccaggaagccggggttccccgggaagcgtggagttcacccgcgcactcgaagtgcc
```

```
tttgcaaaattatatctgggtgttggcacccagccactattctgccaatg
(Seq ID No: 1193)
```

Homo sapiens PRP4 pre-mRNA processing factor 4 homolog B (yeast) (PRPF4B):

```
agctctttcttcttcctccacttcccctaccctccaccgtccgggagccgccgccaccgccgcc
gaggagtcaggaagttcaagatg (Seq ID No: 1194)
```

Homo sapiens molybdenum cofactor synthesis 2 (MOCS2):

```
gcgcctttgcggccgtgattcggtcccgctgtcctaggcgggatggtgccgctgtgccaggtaagg
gtggcgggtgtgcgtgcgggcctgggtgcggagccctcctcgacgtgtctctcccgccctttccct
ccacatacccagccttggtcagtcggacctccccactagcccccaacctggccggcgtcttgggtt
cggggcgcccccgcccccgcccccgggcccttcctgtctccgggctttactgcgactgccccagc
agaagtcgggtcctctccgagaactcttgtcagctcacggcagcaaggacggactcgttctgaagg
cgcctccaccttttatgaccacctctttcccagattattcgttttgatgaagctaaaattttaatc
taaaagaaatgcacctcatggagaattcttgtgaagaactgtgcttcatctgtggatttctacac
ccttgatcatttgcaaacctgtaattatttcgtaaagagttgtttgcacggagtgacaggttgaag
tattgtattttgcaaaaagtgctgaaataacaggagttcgttcagagaccatttctgtgcctcaag
aaataaaagcgttgcagctgtggaaggagatagaaactcgacatcctggattggctgatgttagaa
atcagataatatttgctgttcgtcaagaatatg (Seq ID No: 1195)
```

Homo sapiens cat eye syndrome chromosome region, candidate 1 (CECR1): tttccttttttccggaggggagatg (Seq ID No: 1196)

Homo sapiens solute carrier family 13 (sodium-dependent citrate transporter), member 5 (SLC13A5): ctgcccctcactcgtctcgcccgccagtctccctcccgcgcgatg (Seq ID No: 1197)

Homo sapiens armadillo repeat containing, X-linked 3 (ARMCX3):

```
agtccttcttgtcctggtcgttgttcccgtctgagtaccagctccccactgccctgagggcgggcc
ggcctgcggcggagggaaaaaggaagaggagaaggaaattgtcccgaatccctgcagtgggtccaa
gcctctcccgggtggccagtctttctgtaggttgcggcacaacgccaggcaaaagaagaggaagga
atttaatcctaatcggtggaggtcgatttgagggtctgctgtagcaggtggctccgcttgaagcga
gggaggaagtttcctccgatcagtagagattggaaagattgttgggagtggcacaccactagggaa
aagaagaaggggcgaactgcttgtcttgaggaggtcaaccccccagaatcagctcttgtggccttga
agtggctgaagacgatcacctccacaggcttgagcccagtcccacagccttcctcccccagcctg
agtgactactctattccttggtccctgctattgtcggggacgattgcatg
(Seq ID No: 1198)
```

Homo sapiens armadillo repeat containing, X-linked 2 (ARMCX2):

```
cgtcctcctctgggtaccaactctattgcgcagctcgctgccgtgcgtttaacccaggcgaggagg
aggaggagaaaattccccccagattcgggcaggcccgcaccccacattccgtcctgttttgagagga
ggagggaagagaaataaacgtggcagcgcatagaaggccagcagggagactgctttccagacacct
ccggcccacacagccgttcaccccccgtcttttcagtcctggaaaaggaattcggtctgtccttag
gatgaagctctaactgaactgaagtaaggagaaacagccttgaatctttggagggtctgtcttcct
tttgggctctgtgcaactgcagctacagtggaaaaaagcaaactgctcttgatcccaggccctgcc
taagcctcagcagaacttgtaagcctaaactgaagagcctcacccggacgagcaggcatcccttaa
ccttaagcaatccagttccacgccctggatcagtgaataaccccagctgcaccatg
(Seq ID No: 1199)
```

Homo sapiens UBA domain containing 2 (UBAC2):

```
cgccctctggggctccgagcccggcgggaccatgttcaccagcaccggctccagtgggctctgtga
gtaccggcctccgccatcctggctgcccccctacacgccaccctaggcacctctttgaggaggctgg
ggcagcggggaccctcgggtttgccggaggtggtggggccgaccctccagacccgcgtccgaaccc
```

```
tgctagttcccggtcttgggggtcagcggaaaccgcccccatttcggcctggagggggcgaatgggg
acaaagccccgccgcccgccccgaccccacctggtatccccaggtgctctgcccaggagtctcttg
gggccgctgcaagtgggcaggtgccctggtgttctcgtgggccggccccaggccctttgcggagcg
tgtgccgcgctgaaggaaggggccgtcccccttaccatgccccattcttttaggcttgggggaccg
aactaactcccccgcccccacttgcaaagttcagcctccgctttagaagctgacctctcagtttc
acttggatg (Seq ID No: 1200)
```

Homo sapiens cancer susceptibility candidate 4 (CASC4):

```
cctcctccctcggccggccctggggccgtgtccgccgggcaactccagccgaggcctgggcttctg
cctgcaggtgtctgcggcgaggcccctagggtacagcccgatttggccccatg
(Seq ID No: 1201)
```

Homo sapiens protein phosphatase, Mg2+/Mn2+ dependent, 1G (PPM1G):

```
cgctccctcacagctcccgtcccgttaccgcctcctggccggcctcgcgcctttcaccggcacctt
gcgtcggtcgcgccgcggggcctgctcctgccgcgcgcacccccggggcttcggctccggcacggg
tcgcgcccagctttcctgcacctgaggccgccggccagccgccgccatg
(Seq ID No: 1202)
```

Homo sapiens StAR-related lipid transfer (START) domain containing 13 (STARD13):

```
ctttctttttaaaaatcgctgggtctgttgagctgtcctgggctgggtgccttgctctttgactga
gactggagacagacggcaacagccacaggcagactgaggtggcaataggaaatctgccgagatg
(Seq ID No: 1203)
```

Homo sapiens tubulin, beta class I (TUBB):

```
gattctcccgcctcccagccccggcgcacgcgcgccccgcccagcctgctttccctccgcgccctc
ccctctcctttctccctctcagaaccttcctgccgtcgcgtttgcacctcgctgctccagcctctg
gggcgcattccaaccttccagcctgcgacctgcggagaaaaaaaattacttattttcttgccccat
acataccttgaggcgagcaaaaaaattaaattttaaccatg (Seq ID No: 1204)
```

Homo sapiens cytochrome P450, family 4, subfamily X, polypeptide 1 (CYP4X1):

```
tttccttcttcccgcgagtcagaagcttcgcgagggcccagagaggcggtgggggtgggcgaccct a
cgccagctccgggcgggagaaagcccaccctctcccgcgccccaggaaaccgccggcgttcggcgc
tgcgcagagccatg (Seq ID No: 1205)
```

Homo sapiens doublecortin (DCX):

```
ttttctttctctcagcatctccacccaaccagcagaaaaccggtgagtggggcttttaagtgattt
tcaagaagaatgtaacagatgtcaaacgggaaaagcacaaggcaaagcctgctctctctgtctctc
tgtctcctcttctccttttttgccttattctatccgattttttccctaagcttctacctgggattt
tcctttggaaaagtctctgaggttccaccaaaatatg (Seq ID No: 1206)
```

Homo sapiens protein phosphatase 2, regulatory subunit B', gamma (PPP2R5C):

```
ttgtctttttttttttaaactaaaatggaggctggtttcttgccttaaggagcccattgcctttcc
cgctgaagtctagatg (Seq ID No: 1207)
```

Homo sapiens solute carrier family 9, subfamily B (cation proton antiporter 2), member 2 (SLC9B2):

```
ccacctttccggggggaagccacgcgcaccaggcatcgcacgcggctctgcacccgcgccgccggac
ctgaaacccggcggagggcacacggggctgccgctgcgggccccggaccaacccatgcttactccg
gagcctgtaccggcgccgacgggtcggacctccctgcgcggtgtcgcccagcgggttcgtgcgaaa
ggcggggccgactacacgcggtgccgcgccctgagaccgtttatctgcagtcaacgcagcctcccg
gctcagcctgggaagatgcgcgaatcgggaaccccagagcgcggtggctagaccgggctccgccgc
```

```
ctcccccacagcccctttcctaatcgttcagacggagcctggtcgacttcgccggagactgccaga
tctcgttcctcttccctgtgtcatcttcttaattataaataatg (Seq ID No: 1208)
```

Homo sapiens hypoxia inducible factor 1, alpha subunit (basic helix-loop-helix transcription factor) (HIF1A):

```
caccctcttcgtcgcttcggccagtgtgtcgggctgggccctgacaagccacctgaggagaggctc
ggagccgggcccggaccccggcgattgccgcccgcttctctctagtctcacgagggggtttcccgcc
tcgcacccccacctctggacttgcctttccttctcttctccgcgtgtggagggagccagcgcttag
gccggagcgagcctggggggccgcccgccgtgaagacatcgcggggaccgattcaccatg
(Seq ID No: 1209)
```

Homo sapiens interleukin 21 receptor (IL21R):

```
cctcctcttcctcccccactctgcacatgcggctgggtggcagccagcggcctcagacagacccact
ggcgtctctctgctgagtgaccgtaagctcggcgtctggccctctgcctgcctctccctgagtgtg
gctgacagccacgcagctgtgtctgtctgtctgcggcccgtgcatccctgctgcggccgcctggta
ccttccttgccgtctctttcctctgtctgctgctctgtgggacacctgcctggaggcccagctgcc
cgtcatcagagtgacaggtcttatgacagcctgattggtgactcgggctgggtgtggattctcacc
ccaggcctctgcctgctttctcagaccctcatctgtcaccccacgctgaacccagctgccacccc
cagaagcccatcagactgcccccagcacacggaatggatttctgagaaagaagccgaaacagaaga
tgaggcaatgaggctgcgagaggtagagtgattttccctcggtgactcaactgggacgtagcaggt
cgggcagtcaagccaggtgaccccatg (Seq ID No: 1210)
```

Homo sapiens DDB1 and CUL4 associated factor 4 (DCAF4):

```
tggtctttccgggtccttgcacgcttcgctccaactcctgcagagctgagccggagggggaatccgg
aagggacacgctgaacaggtctgactcccgggcagcacagcccgctcacgattccggccacggtga
tgacgagtctccgtcaacctcgtctggcacagctgggacctcctctgtgccagagctacctgggtt
ttactttgaccctgaaaagaaacgctacttccgcttgctccctggacataacaactgcaaccccct
gacgaaagagagcatccggcagaaggagatg (Seq ID No: 1211)
```

Homo sapiens oxidation resistance 1 (OXR1):

```
ccgcctcttgtgaggcgcgcggagccgcctcccctgggtcaggtctgatgggccggtgggcgcgct
agtggtggccgccaccgccgaaaccgtcgacctcctgggccccagttccgcgtccagccccgcggc
agcatg (Seq ID No: 1212)
```

Homo sapiens cut-like homeobox 1 (CUX1): ccccctctctat-cagccgctcactccgtctcaatatgtctcaagatg (Seq ID No: 1213)

Homo sapiens atlastin GTPase 1 (ATL1):

ctcccttttcctccccactccttcccaccagcgccacagcaacatcctcagagtctgagcgaactg
cgcccagcgcgggcacggagcctccaccgccagcaacctgcggccccggagaaggcagcgagcgc
agtgacagcgcctcaccgccaccagctcctggaccaccatg (Seq ID No: 1214)

Homo sapiens chemokine-like factor superfamily 5 (CKLFSF5):

ctgccttctctcccggggccctgtgggcaagcctcctgcttcactttcaggtttctcgaagtgcct
tcttgctcctgtctgtttccccatcctgccagatttctgtttctcttgctgggcttttggcagtag
ggggctgtgttggtgggccctacgaagatg (Seq ID No: 1215)

Homo sapiens transmembrane emp24 protein transport domain containi ng 7 (TMED7):

aggccttttccgcttctcttttacctccccaggtccgcccgtctgcgcccctcacaggaagccgga
gggtcgctctgatcccgaatctcccacaggcgtgaacctgctctgctgtgtatctttcggggtgtgg
cctgcgctgaggcctgccgcgcgcggtgagtccgcgcagacctgaccctgcgtctcgcagctcggt
tgaggccgccgccgccttctcgggatg (Seq ID No: 1216)

Homo sapiens ubiquitin-conjugating enzyme E2D 3 (UBE2D3):

cttcctttaccttcctcccatggtctccttccggttctcgatgcttctctgagcctaagggtttcc
gccactcgttcaccctcccccagctcatgatcctcctccctcccccgccctcctggtccaatctc
cgatctgtttagtaagaaggtgctgttccgagaagaaggaaaagggcttgacacgtattcactcgg
ccccggacgtgggaagcaagccgtctggcttcggcctcacatcggtcttgtgctcgggacggcggc
gttggcggactgatccgcggcggtgaagagaggccgggaagttaaacttgtagccaccacctccgc
tcttcccgtcaccctcgcccccacttcgggccgaaagcacggtacagaggctgttggtggctttgc
cacgccacccacccaccccggatcgcggctgtcttaagggacctggattcatcaggggctcttcg
gggcctgtgcgagtgctgatctgctccgttttttgcaaaaggcgcctgtgtctggcagagctggtgt
gagacgagacaatcctgccccgccgccgggataatcaagagttttggccggacctttgagcataca
ccgagagagtgaggagccagacgacaagcacacactatg (Seq ID No: 1217)

Homo sapiens zinc finger protein 595 (ZNF595):

tttcctctggctcctgcgagggcttggtttagggcttcagctctctgcgttctcggctccgggagg
cctcggtgattcagccacagcctctgcctcccgttgctctgtgacctgagggtattggacaatttg
tagctaagactcccggataccctgaagtcgggaaatg (Seq ID No: 1218)

Homo sapiens acyl-CoA synthetase medium-chain family member 2B (ACSM2B):

tgctctcttccaaggctgtaggagttctggagctgctggctggagaggagggtggacgaagctctc
tccagaaagacatcctgagaggacttggcagcctgcagatggcctattgtgggaccttgtgatcat
gcctgaacatg (Seq ID No: 1219)

Homo sapiens SRSF protein kinase 2 (SRPK2):

tttcccttttatagcaccattgaatcccagtcctaacagaagtactgcgaatcttgtggcctcattc
tgaacaaaagggattagagaagaaaaatctcttgatataaggcttgaaagcaagggcaggcaatct
tggttgtgaatattttctgattttttccagaaatcaagcagaagattgagctgctgatg
(Seq ID No: 1220)

Homo sapiens synaptophysin-like 1 (SYPL1):

```
tgcccttcctcgccaccgggctgctctggtctcgtcggtcccctcctccgccccgtcgtcctgact
ctctctccctcctttcctcagaggatg (Seq ID No: 1221)
```

Homo sapiens thioredoxin reductase 1 (TXNRD1):

```
aacccttttcacctcagttttcttcactccggcatttgcagcagagcgaaaggtggtcgagtcctga
aggagggcctgatgtcttcatcattctcaaattcttgtaagctctgcgtcgggtgaaaccagacaa
agccgcgagcccagggatgggagcacgcgggggacggcctgccggcggggacgacagcattgcgcc
tgggtgcagcagtgtgcgtctcggggaagggaagatattttaaggcgtgtctgagcagacggggag
gcttttccaaacccaggcagcttcgtggcgtgtgcggtttcgacccggtcacacaaagcttcagca
tgtcatgtggcttatcaggagggcagacttcaaaagctactaaaaatg (Seq ID No: 1222)
```

Homo sapiens minichromosome maintenance complex component 7 (MCM7):

```
gtagccgaggatgaccccgagttggtggactcaatttgtgagaatgccaggcgctacgcgaagctc
tttgctgatgccgtacaagagctgctgcctcagtacaaggagagggaagtggtaaataaagatgtc
ctggacgtttacattgagcatcggctaatgatggagcagcggagtcgggaccctgggatggtccga
agcccccagaaccagtaccctgctgaactcatgcgcagattgtgagtggtctctgtcgggaaagat
gtagggattggttctccaggatcttgtttgtgactgttttctccccttagtgagctgtattttcaa
ggccctagcagcaacaagcctcgtgtgatccgggaagtgcgggctgactctgtggggaagttggta
actgtgcgtggaatcgtcactcgtgtctctgaagtcaaacccaagatg (Seq ID No: 1223)
```

Homo sapiens pre-B-cell colony enhancing factor 1 (PBEF1):

```
tttccccctctccccctcctccgccgaccgagcagtgacttaagcaacggagcgcggtgaagctca
tttttctccttcctcgcagccgcgccagggagctcgcggcgcgcggcccctgtcctccggcccgag
atg (Seq ID No: 1224)
```

Homo sapiens cyclin B1 interacting protein 1, E3 ubiquitin protein ligase (CCNB1IP1):

```
ctttctttccctctccgttttggtgggctggttgaagatgaaatccactgaggagggaagtccagc
accctgtgtgccagtccagaactggcccatctgtagacccctgaaaatcatatgggcttggattt
ggatattctcaacagaaagggttaaaggctgatggtacctaaagcctggtacttgaatttgatca
agataagctgccttaagttctcttcattacacaaatgatcctagataattgatagatcctgtggtt
caactggatttctagatagaagctggattcatgtgatgccagaggagtaaaatttcaagagactga
aaccagatctgagtttcgctgttccagtctggacctctttggtgctgtaaatcctggatatactgt
agatgagtactgcgttttcttttatggcctctcttcagcttctggagacctcactatcctattat
g (Seq ID No: 1225)
```

Homo sapiens STEAP family member 3, metalloreductase (STEAP3):

```
ccgccttcgccgcggaccttcagctgccgcggtcgctccgagcggcgggccgcagagatgacattt
attcattttatgcatcctgggttctactggtcgtcccacctcagttcctgtagcaaagagacttga
gtctgagccactaattatcacccgtgaggtttcctccccgagcaggaagcagcaggccagagctgc
gctctctcagtgcactctccaaccaagcatcagtcaccactcccggtccagcccctgtggccaaga
gctggcgtgcaggctgcgggaggcagctggctgtgcaagaccctggcagggccctcgcctcctgag
aaaccgagagtcagaaccaaagccaggctgtcctggttggagactgagccagaaagggtggctcac
ctcacggtgaggctgtcgagtgacctgagagcctcagaccctcacgtcagccggatg
(Seq ID No: 1226)
```

Homo sapiens nicotinamide nucleotide transhydrogenase (NNT):

```
tgttcttccggggttggaggcgcagcgccgcggggcccaagcccgggtctgccagcgcgacgtcctc
tcgcggccctcagggcacagcccaaggctgtcagcctcccggcccagtgatttgccttcaaggaaa
ctggggagtcagaaaattgggaactcatatcaacatg (Seq ID No: 1227)
```

Homo sapiens SHC (Src homology 2 domain containing) transforming protein 1 (SHC1):

```
gtccctctccctccccaggacttctgtgactcctgggccacagaggtccaaccaggctaagggcct
ggggatacccctgcctggcccccttgcccaaactggcaggggggccaggctgggcagcagcccct
ctttcacctcaactatg (Seq ID No: 1228)
```

Homo sapiens bromodomain containing 8 (BRD8): cggcccttcca-gaccgtctctcctcagggttggagacttcggggccaagatg (Seq ID No: 1229)

Homo sapiens ring finger protein 13 (RNF13):

```
tcgcctctttagtaggtcgggtgagtgtagtgtgcagggaagagacgcgtcagcgccagggccagg
cccgcccggggggcagcccggcagccgaatcttgggctactctgtcccaacagccggagcagatcag
accgaccggccctgcccgctcggtcccgcgccctccagaccctacggtctccgtttctaggggcac
atggttagcggcaggcgcccacagccaatccactttgccagcctgcccccttcctctgccaagagca
gcttcttcagccgcgctccagttccgcagacgcctgccccaccctgctcttcccttcagggaaga
```

```
cggatcacgcggccaagaacgagactcgcaaactgggcatttctccgagccgggctagagcaagta
gcgagactccgcgtgagagtgggaaagagccttaacaggcaaccatgttgcccagtgggttttctg
tgcctttgggtgcggaccaatgaggcgcgtggggcgggacttccgcttcgcctaggtgttgtcgtc
cctgctagtactccgggctgtgggggtcggtgcggatattcagtcatgaaatcagggtagggactt
ctcccgcagcgacgcggctggcaagactgtttgtgttgcgggggccggacttcaagagagaaagag
agagtgggcagacatcgaagccaaacagcagtatcccggaagcactcatgcaactttggtggcggc
cactcagttttctctgccagtgtctggtgattttacaacgagatg (Seq ID No: 1230)
```

Homo sapiens aldolase A, fructose-bisphosphate (ALDOA):

```
ccgcctcctgcgccgcccccttccgaggctaaatcggctgcgttcctctcggaacgcgccgcagaag
gggtcctggtgacgagtcccgcgttctctccttgaatccactcgccagcccgccgccctctgccgc
cgcaccctgcacacccgccccctctcctgtgccaggaacttgctactaccagcaccatg
(Seq ID No: 1231)
```

Homo sapiens LY6/PLAUR domain containing 6 (LYPD6):

```
cgctccttccctgagctcccgggctccggcagcgcgctggcggggcgccgcattgcacactctggg
ggcgccgcagtgttcgtgggatggggcagcgggctgcagctggcggccggaatccgcgcgcagccc
gggtgcaagttctctcctgttgccctgagtgcccactccaggccctctgtatgagtgacacttca
gtctgccatg (Seq ID No: 1232)
```

Homo sapiens butyrophilin, subfamily 3, member A1 (BTN3A1):

```
cagtctctgctttcttttccttttcttccagaaggagatttaaccatagtagaaagaatggagaac
tattaactgcctttcttctgtgggctgtgattttcagaggggaatgctaagaggtgattttcaatg
ttgggactcaaaggtgaagacactgaaggacagaattttggcagaggaaagatcttcttcggtca
ccatacttgagttagctctaggaagtggaggtttccatttggaattctatagcttcttccaggtc
atagtgtctgccccccaccttccagtatctcctgatatgcagcatgaatg
(Seq ID No: 1233)
```

Homo sapiens lipoic acid synthetase (LIAS): ctgtcctttcccgggagttagcgatccctcaacccctgcactgcgctagtcctaaagaggaaatg (Seq ID No: 1234)

Homo sapiens C-type lectin domain family 7, member A (CLEC7A):

```
gattctcttttgtccacagacagtcatctcaggagcagaaagaaaagagctcccaaatgctatatc
tattcaggggctctcaagaacaatg (Seq ID No: 1235)
```

Homo sapiens CD247 molecule (CD247):

```
actccttttctcctaaccgtcccggccaccgctgcctcagcctctgcctcccagcctctttctgag
ggaaaggacaagatg (Seq ID No: 1236)
```

Homo sapiens myeloid zinc finger 1 (MZF1):

```
aagcctttctccattttgcggtctaggaagtagcagaggcccccttcctgtagggagttgccatgga
gacgcggtggggcaccgacggagttctaatgacggccgtgattggtgcaggatcctgctaatctca
ggaaggcccgtagagaagtgaggaaaacgtggtggggggcatgcgcgatctggtaggcggtgctgc
cgtctgttgtacctgagaggcttgcgcatgccgacgcacggattcgaggcggggagcatgggaaga
agcggccaggagtatgacctgatcattgcgaccaccgctaggggaagggaggagagggtgtagaaa
cggggacgagggtggggaagggcaaggaggcgctcgagctggtgcgcggagcatcctgggagacg
tagtccagcgggagggggaagtcgaagactgcgcgtgctcaggagcgcggagcggcccgctgagcg
cagaggggcagacactggcctcagatacctgacctggtaccctctatg (Seq ID No: 1237)
```

Homo sapiens E2F transcription factor 6 (E2F6):

```
cctcctcttttttccgtctgcgtcgggagctcccgggcacgtgaggccgtgccgcgtttactggcgg
gcgggacggcctagccgggcggcgcctcggaggaagccgcggacccccttaggtgctgggcccttgg

aaatcggcgcgtgggggggcggtgctcgagctgagcgcgagagggcgggagagctcgtggggtgcga
ggggagcaggacgcccggccgggcagcatg (Seq ID No: 1238)
```

Homo sapiens purinergic receptor P2Y, G-protein coupled, 10 (P2RY10):

```
cttcctctttcaacaacaaatgtgtcagttatcagcaggatccatgccgccagagtaaagctttct
accctttactccctgcaaagaaacaagagtgcttatcccagctaagctccagggtaatgttatcat
gacagcttcaacttttagaccacaggcaaatgctttgttaaaactctatgctggtcattcccttca
ggatttggcactcaccaacataccttctttcaagtgaaaaggcatctcttttaatggtcctgacc
tttggaataggaagcatgtaccctggacagagcacttcaaactagaggaaccataaatccatg
(Seq ID No: 1239)
```

Homo sapiens chromosome 9 open reading frame 85 (C9orf85): catccttttgcctgctcccggcgaggggtggctttgatttcggcgatg (Seq ID No: 1240)

Homo sapiens ERGIC and golgi 3 (ERGIC3): cgtcccctttccggccggtccccatg (Seq ID No: 1241)

Homo sapiens ankyrin repeat domain 46 (ANKRD46):

```
ccctcccctccgcccgtcaccgcctccttgaagctgccgctgtcgctgctgctcgttcgagtcgca
gatccttgccagcacattacagaatattttttgttgaaccttcttgagaattcagagaaactgctga
gtgaccactgaacgaaaagatctaatcttaaggcttacgcctcactttgatgcccaggctggagtg
ctgtggctcaatcacagctcatcgcaacctcgacctcccgggctcaagtgatcctctcacctcagc
gtcccgaacaggcgtgttccatccaccacatcagaacaatg (Seq ID No: 1242)
```

Homo sapiens Ras and Rab interactor-like (RINL):

```
tcctctctccacttcctgctactgcaggcctctcctccgagaacagaggccaggtcatgactcact
ggcttcctgcaacctgacgatggcccagccagaagacaaggcacctgaagtccccacagagggggt
gaggtgaacaaagcagacaggacccctctaggggtcctcagcaccctagagccacttactcgcctg
cagaggacatgggggggtgtggcatgtgccagagctggatacccaggatgcggaggcccttgtgggg
ctgtggccactagggagtttcttggtcacaggacgtgaccccagccaggccctggtgttgaggtca
ggacctttaccaggagaagtcaatacctaccagatccagaagattcccagaggtgtgtccctggaa
tcctccaacctctgcatg (Seq ID No: 1243)
```

Homo sapiens embigin (EMB):

```
ccgccttttcttcagcgtcctacccgcggcactggctgcgagcgccgggccacctgcgagtgtgcg
cagggactctggacacccgcggcggcgagctgagggagcagtctccacgaggacccaggcggaccc
tctggcgccatg (Seq ID No: 1244)
```

Homo sapiens MMS22-like, DNA repair protein (MMS22L):

```
ccgcctttccggagcgcgggcgcgcggtggcgggaatttcgcctgtttgcggtttagaccccaaag
attcctgttggtggtctgggtcacaggaggcaggtttcgggagctggaaatgtgagcgggtacgac
aggcaccgcgggtaaccgacgccccgggtccttgctgcagccgggtacgcgggataccggcacccc
gccttctccgcccgagtgctgccaggcgtgggcctggaatctcttcacaccttctctttggagccc
ttaatgatacgacgaaccccaagtgtttcagaacatgaagtaaacaatg
(Seq ID No: 1245)
```

Homo sapiens chromosome 19 open reading frame 54 (C19orf54):

```
actcctttcctttttccagtggttatcgcggcgcccaccggcctctgatctctgagtcttctccaa
cccacagacgttttttgttgctctggttccaggaccttctccacaactaggccattttccctgcca
ggtgtcctttttgacctcttgacctctgactcaaagggcctgctcccctcatgtcttcggcctgg
agaagagccagctcctgaaggaggcctttgataaggccggcccggtccccaagggcagagaagatg
tgaagaggcttctgaaactacacaaggaccggttccgaggtgacctgcggtggatcctcttctgtg
cagacctgccgtccctcatccaagaaggccctcaatg (Seq ID No: 1246)
```

Homo sapiens zinc finger protein 621 (ZNF621):

```
cgcccttccggctcggcctttagttagtgaccagctcctcggcgttctgcagagcgtgggtttcag
cgagttctacgtgccaggtccgcccggtgccggcttcctcgctgcccctggcggctcgtcagcccc
cactaccctgaacttggtcccaatggcggcccgcccctccttcacccggaccgtgggcatctggg
cctcgccgaagccgtcaaggtggctgctcgggcttctagagccgtgtccagccctttgccaccga
ggcctgatcctctttctgccctaaagaacttgccctgacagcctctggctcccgctcttgaggat
cttgcttgtccaaacccagaagacagtgcatgaagccaggggacatccgccatg
(Seq ID No: 1247)
```

Homo sapiens family with sequence similarity 73, member A (FAM73A): ccgccttctccatg (Seq ID No: 1248)

Homo sapiens RNA binding motif protein 43 (RBM43):

```
ccgccctttcttcgtagcctccaagggagctggaacaaaaaacgaaaccaaaacctgcctgctcg
ctcctctccccatcgcctgcgttccgctggttgtgggctttctgcggccgctgagggcgcgtctcc
cctccgccatg (Seq ID No: 1249)
```

Homo sapiens spermatogenesis and centriole associated 1 (SPATC1):

```
caccctccttcagcccaggcaaggcctggggccctgggcagcctccaggtgcagtgccctcccgtg
ggccgcacccttgccactgccccagggcatg (Seq ID No: 1250)
```

Homo sapiens carbonic anhydrase XIII (CA13): ctttctcttccttccaccccgagggaccatg (Seq ID No: 1251)

Homo sapiens transglutaminase 2 (C polypeptide, protein-glutamine-gamma-glutamyltransferase) (TGM2):

```
cgctctccgcctcggcagtgccagccgccagtggtcgcacttggagggtctcgccgccagtggaag
gagccaccgcccccgcccgaccatg (Seq ID No: 1252)
```

Homo sapiens NOP2/Sun domain family, member 4 (NSUN4):

```
atttcctttcccttttttcgctcgtgtcccgccgggtggcgctcaccacctccccggaacacgcga
gtctcctgtcgcggttccggtcggaattaccccgtggagcacgccgatatg
(Seq ID No: 1253)
```

Homo sapiens mitochondrial ribosome recycling factor (MRRF): gagtctttccttagtaacctgggcgatagctgtggatgtttccaaggattgtcttcagtcatg (Seq ID No: 1254)

Homo sapiens PHD finger protein 17 (PHF17):

```
cttcctccataacaagccaaacgccagaccgagagtgcctccgtgcgcgagtgcccggtgtgtgcg
cgccggcgagagcaggggcccgcccggctccccgcccgccgcggcccgaactcatgcagctccgag
cgagcgagcggcgcccagcccagcgcctcggccgaacccctccgcagcaggctgcctgctgtttcc
cggggagatcatg (Seq ID No: 1255)
```

Homo sapiens prolylcarboxypeptidase (angiotensinase C) (PRCP): cctccttttcgccctcccacccgcactgcagtctccagcctgagccatg (Seq ID No: 1256)

Homo sapiens proteolipid protein 1 (PLP1):

```
aagcccttttcattgcaggagaagaggacaaagatactcagagagaaaaagtaaaagaccgaagaa
ggaggctggagagaccaggatccttccagctgaacaaagtcagccacaaagcagactagccagccg
gctacaattggagtcagagtcccaaagacatg (Seq ID No: 1257)
```

Homo sapiens coiled-coil domain containing 80 (CCDC80):

cagccttctcactcctcactgagtccactctgaacgtgctaaaatgggaaggaggcggtgttttgc
tgatctgttaaattcttagtgaagtttccttgatttccagtggctgctgttgtttgagtttggtttt
ggagcaaaactgaggtagtcctaacatttctgggactgaatccaggcaagagaaagaagaaaaaga
agaagaaaaagaggaggaaaaaggtagggagaaataaagggaggagagaagcacagtgaaagaaaa
aaaaagtcccttttcgacatcacattcctgtgttttccctcagcctggaaaacatattaatcccag
tgcttttacgcccggaaacaaagagactaagccagactatggggggaaagggagataagaaggatcc
tggaactttaaagagggaaagagtgagattcagaaatcgccaggactggactttaagggacgtcct
gtgtcagcacaagggactggcacacacagacacgagaccgaggagaaactgcagacaaatggag
atacaaagacttagaaggacagctcctttcacctcatcctacttgtccagaaggtaaaaagacaca
gccagaaagaaaaggcatcggctcagctctcagatcaggacaggctgtggatctgtggcggtactc
tgaaagctggagctgcagcacacccttttgtattgctcaccctcggtaaagagagagagggctgg
gaggaaaagtagttcatctaggaaactgtcctgggaaccaaacttctgatttctttgcaaccctc
tgcattccatctctatgagccaccattggattacacaatg (Seq ID No: 1258)

Homo sapiens chromosome 20 open reading frame 44 (C20orf44):

cgacctctttgcgcctgcgccccccttgccagtctttcgccggcaaaaggaggacgtagaaaaggg
gacaccggaaactcactcttcacccggaaatggttattgaggaacatg (Seq ID No: 1259)

Homo sapiens tryptophanyl tRNA synthetase 2, mitochondrial (WARS2): cgcccttctcaagatg (Seq ID No: 1260)

Homo sapiens myotubularin related protein 2 (MTMR2):

ctttccctgtgctgcccctgccgcgcgatggagaagagctcgagctgcgagagtcttggctcccag
ccggcggcggctcggccgcccagcgtggactccttgtccagttaatgtgttaagagccattgacat
ttgaagatcatcagaagtgaagataaaacatctcaaaaattataattgcctccacttctcattcag
agaattcagtgcatacaaaatcagcttctgttgtatcatcagattccatttcaacttctgccgaca
acttttctcctgatttgaggagggagtctcgctctatcccctaggctggagtgcattggcgccatc
tcggctcatttgcaacctctgtctcccgggttcaagcgattctcctgcctcagcttcccgaggagc
tgggattacaggtcctgagggagtctaacaagttagcagaaatg (Seq ID No: 1261)

Homo sapiens reticulon 3 (RTN3):

cgccctctagctgcgctcggctgagtcagtcagtctgtcggagtctgtcctcggagcaggcggagt
aaagggacttgagcgagccagttgccggattattctatttcccctccctctctcccgccccgtatc
tcttttcacccttctcccaccctcgctcgcgtagccatg (Seq ID No: 1262)

Homo sapiens G protein-coupled receptor 56 (GPR56):

gtccctccctctccgcactagctgtctgccctgccctgccgtaggagatgggctgggagcctccca
cgctctccagctcactcggcaggcagcggggaccagggctggcaggttaagcctctgggggtggat
cctgaaaggtggtccagccgcctggccctgcgtgggaccctccacctggcagcagacagggtctcg
ctctgtcacacaggctggagtgcagtggtgtgatcttggctcatcgtaacctccacctcccgggtt
caagtgattctcatgcctcagcctcccgagtagctgggattacaggtggtgacttccaagagtgac
tccgtcggaggaaaatg (Seq ID No: 1263)

Homo sapiens immunoglobulin superfamily containing leucine-rich re peat (ISLR):

gctcctccctgccgcctcctctcagtggatggttccaggcaccctgtctggggcagggagggcaca
ggcctgcacatcgaaggtggggtgggaccaggctgccctcgccccagcatccaagtcctcccttg
ggcgcccgtggccctgcagactctcagggctaaggtcctctgttgcttttttggttccaccttagaa
gaggctccgcttgactaagagtagcttgaaggaggcaccatg (Seq ID No: 1264)

Homo sapiens glycoprotein M6A (GPM6A):

```
atttcttttccccattttaaatgcaaagcaagacttgtgaatcatagtgtctctgctcctgggatt
cagaccaaatttccccccaaaattctcaggctatttgtttgaatacctgcttacagtggtacacaa

tgggcagctttgagaagaaaaattgataatcttcacggaagagtaatttgaatgaaattacacttg
acagcctgtctccaagcaaacaagaggaacgagggagcctgagctaagctctgaggacttgcccaa
gccactgctgttggagcttcccaggaaaaaaaaaaaaaaaaaaaaaaaaaaaaaaaaaaaaaaaaac
accagtttttccaacatctaattgagcttttgattaattccgtgtaccagattctactgaagaaag
gtagccatg (Seq ID No: 1265)
```

Homo sapiens splicing factor 1 (SF1):

```
ctccctctttgtgcgtctcgcgccgccgccgcccgccgcgtgagaggacgggctccgcgcgctccg
gcagcgcattcgggtcccctcccccgggaggcttgcgaaggagaagccgccgcagaggaaaagca
ggtgccggtgcctgtccccggggggcgccatg (Seq ID No: 1266)
```

Homo sapiens cell cycle associated protein 1 (CAPRIN1):

```
ccgcccctcgcgacccagagggctgctggctggctaagtccctcccgctcccggctctcgcctcac
taggagcggctctcggtgcagcgggacagggcgaagcggcctgcgcccacggagcgcgcgacactg
cccggaagggaccgccacccttgcccctcagctgcccactcgtgatttccagcggcctccgcgcg
cgcacgatg (Seq ID No: 1267)
```

Homo sapiens hypothetical protein FLJ90297 (LOC388152):

```
ctgccctcttgcgtgccccggccacccccgggcggcttgtagccggtgcgcggggtggctggggct
acgtgcagagctgtcgcggagccggaacagcagcggtgaagcccctcggctcggccgagaccgccg
tgcccattgctcgcctcggttgccgccgctttagccgcagccgctgctgccgccgccggggggagag
gcagcctattgtctttctccgcggcgaaggtgaggagctgtctcggctcggcccgcggggggagccc
cgggagccgcacggagatggaggaggacatctggacagtgagcaggaggcgcctcggcccatg
(Seq ID No: 1268)
```

Homo sapiens kelch-like ECH-associated protein 1 (KEAP1):

```
cgccctctccccgcctccttttcgggcgtcccgaggccgctccccaaccgacaaccaagaccccgc
aggccacgcagccctggagccgaggccccccgacggcggaggcgcccgcgggtcccctacagccaa
ggtccctgagtgccagaggtggtggtgttgcttatcttctggaaccccatg
(Seq ID No: 1269)
```

Homo sapiens F-box protein 38 (FBXO38):

```
ctccctctcaaccacaataacaggcggagggtcggcgtaggtactttgaactcaagtaaacaaaag
ggaagattttctcgttgatactggagactgcacaacaatg (Seq ID No: 1270)
```

Homo sapiens musculoskeletal, embryonic nuclear protein 1 (MUST1):

```
agatcttttccagcagctgctgcctgccagagaggcgccttcagagacccagcgcttacacaatac
ccaccatg (Seq ID No: 1271)
```

Homo sapiens QKI, KH domain containing, RNA binding (QKI): cctcctctccggcggcggcggcggcggcggcggcgggcggagtgagct-gcggagcctggaatatg (Seq ID No: 1272)

Homo sapiens protein phosphatase 1, catalytic subunit, beta isofor m (PPP1CB):

```
gggcctctcttgtttatttatttattttccgtgggtgcctccgagtgtgcgcgcgctctcgctacc
cggcgggggagggggtggggggagggcccgggaaaaggggggagttggagccggggtcgaaacgccgc
gtgacttgtaggtgagagaacgccgagccgtcgccgcagcctccgccgccgagaagcccttgttcc
cgctgctgggaaggagagtctgtgccgacaagatg (Seq ID No: 1273)
```

Homo sapiens methyltransferase like 21B (METTL21B):

```
cagcctctaccccgctccggatccgggatctgagcgccggccgcggtgcccaggcactcccttggc
gggccggatg (Seq ID No: 1274)
```

Homo sapiens adaptor-related protein complex 3, mu 1 subunit (AP3M1):

```
cggccttctcggcttctccagcttcggtaggagaggatccggcgccgaatcactgactggcacagg
tgttgggatagtgtctcacttggtcacccaggctggagtgcagtggcgcaatcttagctctctaca
gcgtcgatcttcctcctgggctcaagcaattctcctgcttcatcctcctgagtacctaggactaca
gaaaatg (Seq ID No: 1275)
```

Homo sapiens muscleblind-like splicing regulator 1 (MBNL1):

```
cagtcttttcactgcagctgaatgagttgtggcgcccacaatgctcccatgacaaggagctgacaa
gttccattttccgtcgcgggcatcttggaatcatgactcccacaatgccttgggcacttggtcgac
agtggggccgcctctgaaaaaaaaatgtgagaggttggtactaagaagtgcctttcctgacgtctc
tgctgcttggaaccgcttctagagcagtctctgcttttgccttgcttgctgccagctagactgtga
cgacagcacatccaccctccacctctagcccagacacccccatttctacttataatcaagagaaaa
gctctaagtatctggcattgccctaggctgctttagtgttaaaagaaaagtttgctgaaaaagtaa
gatatcttctgccaggaaatcaaggaggaaaaaaaaatcattttctcgattttgctctaaactgc
tgcatctgtctatgccaaactaatcaataccgattgcaccaccaaactccattgcaaattcagctg
tgaggagattccctttcagacaactttgctgaaagcagcttggaaattcggtgtcgaagggtctgc
cacgttttcatgcttgcattttgggctccaaattggcactgggaaggggttactgagagcacaagg
ctgataccaggccctacttttaaacgttcatctacttacaatcctagtatttctctaaaaaccaaa
acctctttgaattaacagtttcatgctgtgaatttctagtgggagatcttttccttgatattgacg
acacaattttccatgtacttttaaagcagggagtggggaaaagtattttgaggggacattttcatc
atcagttcagcttttttttttttggttgttgctcttttttgggggggttgggtttgttggtttcact
gaaacatttaactacctgtaaaatctaaacatg (Seq ID No: 1276)
```

Homo sapiens lipid phosphate phosphatase-related protein type 1 (LPPR1):

```
cagccttttgctctttcctttcattaaacaaacaggagatcctgaaacctggaccctgtgcaagct
gcagcgccaggaggaggcagcggaggaagcagagcgcgggatgggcgcccagcggcatctgtgatc
ccgcgcacctccgccccacgggcgcgcgcacaaacacggacacacacatacacacactcgcgcaca
cactcgcacaaacacacactcgtacacgcccgcgccgctcgctcgccggcttgctctcccacgcaa
gcggaatgcagcagcgcctggagagcgtgtctcggaccgccgcctgaatgtacctcgctcccggga
gccggacggcccagtagggcgcactggaggacgctccgctgcgggagcctggacagttttttgacgg
tgcagtcttgctatatggtgtgagaaatg (Seq ID No: 1277)
```

Homo sapiens muscleblind-like splicing regulator 2 (MBNL2):

```
ctgtctttgcttcatcatctgaaggtaaaattttccagatacggcagacggctttcagagtacaat
aaacagggaatgagaactatttacatggaagtttctttctcatgatgcggtggagaagcctcggcc
acttggttctgccagatgttcctggggttactgtaaatgggaaggacaggcagagctaaacaaggt
ttatcatttaaaagtgcctgtgtgaagtcacttttgctggaaaactgcagcttgggagctttcttt
gtattcacatcccactcttctgtcaagtacactttaccctgaccttatgagtggatgaagatacct
cagttgtctgactttgccaattgcttaatttcagaatttaaaaaggggaaagaaaaacatcctgct
aaaatatgaacatctgagtgtcttattttccaacatcgtcaatagctgtgagcgtcagcattaaat
attctcccaaggagtgccatgatattgaagtcactttattaataacagctgtatctgcaaaacagt
caagagactcggacgttgaaagccagagatgacactgagcatgctttattgcggcctaccatctt
taagtgggacatattgattgatgagtgattgcctgtccatacactctctcatcatcctgttccttg
gattggacttcactaagcaatttatcactccttcagacttacatgtgggagttttcacaacagt
agttttggaatcattagaacttggattgatttcatcatttaacagaaacaaacagcccaaattact
ttatcaccatg (Seq ID No: 1278)
```

Homo sapiens chromosome 3 open reading frame 25 (C3orf25):

```
gcgcctttcgcacgacttggagttacggtttatctgataccgcggtacccctacgcaagcaagccc
acatcgacacacattcacacacgcccttcagcaccccctcccagcaccacgaccatg
(Seq ID No: 1279)
```

Homo sapiens testis expressed 19 (TEX19):

```
cctcctcctttccctgggtgcccacatgaacagagacaccaggatgctctcctgagaccacagcaa
ctgcagaagctgaagacatttccagaagttcaagcttccaccctctgcaggtccccactgagctgg
gacccaggtcatccaccccaccccaaatccctggataggaaacccctttctcctcctgctccttgt
ccccttcatccctgccgcccagcatcctactggcctcagcacctgtggccagaccgtccaagatcc
tctgaaggcccagctcttgctgtccaccccggcagtaggcaggcagcctggccatg
(Seq ID No: 1280)
```

Homo sapiens protein kinase C, beta (PRKCB):

```
gcctccctcccccgcagctggggccagcggtgccaagcgcagctggacgagcggcagcagctgggc
gagtgacagccccggctccgcgcgccgcggccgccagagccggcgcaggggaagcgcccgcggccc
cgggtgcagcagcggccgccgcctcccgcgcctccccggcccgcagcccgcggtcccgcggccccg
gggccggcacctctcgggctccggctccccgcgcgcaagatg (Seq ID No: 1281)
```

Homo sapiens protein kinase N1 (PKN1): ccctccctccgcgcggggacccctggcgggcggcaggaggacatg (Seq ID No: 1282)

Homo sapiens hemochromatosis type 2 (juvenile) (HFE2):

```
ccttctctggttccctgacctcagtgagacagcagccggcctggggacctgggggagacacggagg
accccctggctggagctgacccacagagtagggaatcatggctggagaattggatagcagagtaat
gtttgacctctggaaacatcacttacagggcttccggtcaaaattcactaggtaggagggtcatca
gctgggaagaaccggcgcctgggaaacctggctggataggtatg (Seq ID No: 1283)
```

Homo sapiens ribosomal protein L9 (RPL9): cgttctttctttgctgcgtctactgcgagaatg (Seq ID No: 1284)

Homo sapiens ribosomal protein L3 (RPL3): cggcctctaccggcgggatttgatggcgtgatg (Seq ID No: 1285)

Homo sapiens ribosomal protein L4 (RPL4): acttccttttcctgtggcagcagccgggctgagaggagcgtggctgtctcctctctccgccatg (Seq ID No: 1286)

Homo sapiens ribosomal protein L5 (RPL5):

```
tggccctttttcccaccccctagcgccgctgggcctgcaggtctctgtcgagcagcggacgccggtc
tctgttccgcaggatg (Seq ID No: 1287)
```

Homo sapiens ribosomal protein L6 (RPL6): aattctctttcccatcttgcaa-gatg (Seq ID No: 1288)

Homo sapiens ribosomal protein L7 (RPL7): cttcctctttttccggctggaac-catg (Seq ID No: 1289)

Homo sapiens ribosomal protein L7a (RPL7A): ctttcctttctctctcctcccgccgcccaagatg (Seq ID No: 1290)

Homo sapiens ribosomal protein L11 (RPL11): ctttctcttcctgctctccat-catg (Seq ID No: 1291)

Homo sapiens ribosomal protein L12 (RPL12):

```
cggcctctcggctttcggctcggaggaggccaaggtgcaacttccttcggtcgtcccgaatccggg
ttcatccgacaccagccgcctccaccatg (Seq ID No: 1292)
```

Homo sapiens ribosomal protein L13 (RPL13): gcttcctttccgctcggctgttttcctgcgcaggagccgcagggccgtaggcagccatg (Seq ID No: 1293)

Homo sapiens ribosomal protein L23 (RPL23): acttcctttttctttttccggcgttcaagatg (Seq ID No: 1294)

Homo sapiens ribosomal protein L18 (RPL18): cgttctctctttccggacctggccgagcaggaggcgccatcatg (Seq ID No: 1295)

Homo sapiens ribosomal protein L18a (RPL18A): acttccttttgcgggtggcggcgaacgcggagagcacgccatg (Seq ID No: 1296)

Homo sapiens ribosomal protein L19 (RPL19): agctctttcctttcgctgctgcggccgcagccatg (Seq ID No: 1297)

Homo sapiens ribosomal protein L21 (RPL21): gcctctttcctttcggccggaaccgccatcttccagtaattcgccaaaatg (Seq ID No: 1298)Homo sapiens ribosomal protein L22 (RPL22): acctccctttctaactccgctgccgccatg (Seq ID No: 1299)

Homo sapiens ribosomal protein L23a (RPL23A): agacccttttcacaagatg (Seq ID No: 1300)

Homo sapiens ribosomal protein L17 (RPL17):

```
cgctcttcctctctttccctaagcagcctgagggttgactggattggtgaggcccgtgtggctacttc
tgtggaagcagtgctgtagttactggaagataaaagggaaagcaagcccttggtgggggaaagtat
ggctgcgatgatggcatttcttaggacacctttggattaataatgaaaacaactactctctgagca
gctgttcgaatcatctgatatttatactgaatgagttactgtaagtacgtattgacagaattacac
tgtactttcctctaggtgatctgtgaaaatg (Seq ID No: 1301)
```

Homo sapiens ribosomal protein L24 (RPL24): ttctctctttcttttcgccatcttttgtctttccgtggagctgtcgccatg (Seq ID No: 1302)

Homo sapiens ribosomal protein L26 (RPL26): agttctcttccctttttgcggccatcaccgaagcgggagcggccaaaatg (Seq ID No: 1303)

Homo sapiens ribosomal protein L27 (RPL27): ctttcctttttgctggtagggccgggtggttgctgccgaaatg (Seq ID No: 1304)

Homo sapiens ribosomal protein L30 (RPL30):

```
aagtctttcctttctcgttccccggccatcttagcggctgctgttggttgggggccgtcccgctcc
taaggcaggaagatg (Seq ID No: 1305)
```

Homo sapiens ribosomal protein L27a (RPL27A): ccttcctttttcgtctgggctgccaacatg (Seq ID No: 1306)

Homo sapiens ribosomal protein L28 (RPL28): cttcctctttccgtctcaggtcgccgctgcgaagggagccgccgccatg (Seq ID No: 1307)

Homo sapiens ribosomal protein L29 (RPL29): cagcccctttctcttccggttctaggcgcttcgggagccgcggcttatggtgcagacatg (Seq ID No: 1308)

Homo sapiens ribosomal protein L31 (RPL31): cgctcttcctttccaacttggacgctgcagaatg (Seq ID No: 1309)

Homo sapiens ribosomal protein L32 (RPL32): ccgtcccttctctcttcctcggcgctgcctacggaggtggcagccatctccttctcggcatcatg (Seq ID No: 1310)

Homo sapiens ribosomal protein L35a (RPL35A):

```
cgtccttctcttaccgccatcttggctcctgtggaggcctgctgggaacgggacttctaaaaggaa
ctatg (Seq ID No: 1311)
```

Homo sapiens ribosomal protein L37 (RPL37): ccttctcttccggtctttctggtctcggccgcagaagcgagatg (Seq ID No: 1312)

Homo sapiens ribosomal protein L37a (RPL37A): gcgtctcttcctttctgggctcggacctaggtcgcggcgacatg (Seq ID No: 1313)

Homo sapiens ribosomal protein L38 (RPL38):

```
cgttcttttcgtccttttccccggttgctgcttgctgtgagtgtctctagggtgatacgtgggtg
agaaaggtcctggtccgcgccagagcccagcgcgcctcgtcgccatg (Seq ID No: 1314)
```

Homo sapiens ribosomal protein L39 (RPL39): ccctcctcttcctttctccgccatcgtggtgtgttcttgactccgctgctcgccatg (Seq ID No: 1315)

Homo sapiens ribosomal protein, large, P0 (RPLP0):

```
aggcccttctctcgccaggcgtcctcgtggaagtgacatcgtctttaaaccctgcgtggcaatccc
tgacgcaccgccgtgatg (Seq ID No: 1316)
```

Homo sapiens ribosomal protein, large, P1 (RPLP1):

```
cggtccttccgaggaagctaaggctgcgttggggtgaggccctcacttcatccggcgactagcacc
gcgtccggcagcgccagccctacactcgcccgcgccatg (Seq ID No: 1317)
```

Homo sapiens ribosomal protein, large, P2 (RPLP2):

```
ccttccttttcctccctgtcgccaccgaggtcgcacgcgtgagacttctccgccgcctccgccgca
gacgccgccgcgatg (Seq ID No: 1318)
```

Homo sapiens ribosomal protein S3 (RPS3): acttcctttcctttcagcggagcgcggcggcaagatg (Seq ID No: 1319)

Homo sapiens ribosomal protein S3A (RPS3A): ccgcccttttggctctctgaccagcaccatg (Seq ID No: 1320)

Homo sapiens ribosomal protein S4, X-linked (RPS4X): ggtcctctttccttgcctaacgcagccatg (Seq ID No: 1321)

Homo sapiens ribosomal protein S4, Y-linked 1 (RPS4Y1): gattctcttccgtcgcagagtttcgccatg (Seq ID No: 1322)

Homo sapiens ribosomal protein S5 (RPS5):

```
ttttcttcccagttaaaagtgttggcccgcggcgcgcggcctcttcctgtctgtaccagggcggcg
```

```
              cgtggtctacgccgagtgacagagacgctcaggctgtgttctcaggatg
              (Seq ID No: 1323)
```

Homo sapiens ribosomal protein S6 (RPS6): ggccctcttttccgtggcgcctcggaggcgttcagctgcttcaagatg (Seq ID No: 1324)

Homo sapiens ribosomal protein S7 (RPS7): gggtctcttcctaagccggcgctcggcaagttctcccaggagaaagccatg (Seq ID No: 1325)

Homo sapiens ribosomal protein S8 (RPS8): gtttctctttccagccagcgccgagcgatg (Seq ID No: 1326)

Homo sapiens ribosomal protein S9 (RPS9): gcgcctctttctcagtgaccgggtggtttgcttaggcgcagacggggaagcggagccaacatg (Seq ID No: 1327)

Homo sapiens ribosomal protein S10 (RPS10): gctccttcctttccagccccggtaccggaccctgcagccgcagagatg (Seq ID No: 1328)

Homo sapiens ribosomal protein S11 (RPS11): ctgcccctttctttttttcaggcggccgggaagatg (Seq ID No: 1329)

Homo sapiens ribosomal protein S12 (RPS12):

```
 aggcctctttccctgccgccgccgagtcgcgcggaggcggaggcttgggtgcgttcaagattcaac
 ttcacccgtaacccaccgccatg (Seq ID No: 1330)
```

Homo sapiens ribosomal protein S13 (RPS13): cgctctcctttcgttgcctgatcgccgccatcatg (Seq ID No: 1331)

Homo sapiens ribosomal protein S15 (RPS15): cgatctcttctgaggatccggcaagatg (Seq ID No: 1332)

Homo sapiens ribosomal protein S15a (RPS15A):

```
 cgtcctctttccgccatctttccgcgccggtgagtagcactctctgagagctccaatttcatccgt
 ctgccatcggcgccatcctgcaatctaagccacaatg (Seq ID No: 1333)
```

Homo sapiens ribosomal protein S16 (RPS16): ctttccttttccggttgcggcgccgcgcggtgaggttgtctagtccacgctcggagccatg (Seq ID No: 1334)

Homo sapiens ribosomal protein S19 (RPS19): cgttccctttcccctggctggcagcgcggaggccgcacgatg (Seq ID No: 1335)

Homo sapiens ribosomal protein S20 (RPS20):

```
 ccacccctttcttttttgaggaagacgcggtcgtaagggctgaggatttttggtccgcacgctcctg
 ctcctgactcaccgctgttcgctctcgccgaggaacaagtcggtcaggaagcccgcgcgcaacagc
 catg (Seq ID No: 1336)
```

Homo sapiens ribosomal protein S21 (RPS21): gcttcctttctctctcgcgcgcggtgtggtggcagcaggcgcagcccagcctcgaaatg (Seq ID No: 1337)

Homo sapiens ribosomal protein S23 (RPS23): gcttctctctttcgctcaggcccgtggcgccgacaggatg (Seq ID No: 1338)

Homo sapiens ribosomal protein S25 (RPS25):

gcttccttttttgtccgacatcttgacgaggctgcggtgtctgctgctattctccgagcttcgcaat
g (Seq ID No: 1339)

Homo sapiens ribosomal protein S26 (RPS26): ccgtctcctctctccggtccgtgcctccaagatg (Seq ID No: 1340)

Homo sapiens ribosomal protein S27 (RPS27): cgctcctttccggcggtgacgacctacgcacacgagaacatg (Seq ID No: 1341)

Homo sapiens ribosomal protein S28 (RPS28): actcctctccgccagaccgccgccgcgccgccatcatg (Seq ID No: 1342)

Homo sapiens ribosomal protein S29 (RPS29): gcttcttccttttacctcgttgcactgctgagagcaagatg (Seq ID No: 1343)

Homo sapiens ribosomal protein L15 (RPL15): agctctttcctttccgtctggcggcagccatcaggtaagccaagatg (Seq ID No: 1344)

Homo sapiens ribosomal protein S2 (RPS2): cgttcttcttttccgacaaaacaccaaatg (Seq ID No: 1345)

Homo sapiens ribosomal protein L14 (RPL14): gggtcttcttccttctcgcctaacgccgccaacatg (Seq ID No: 1346)

Homo sapiens ribosomal protein S14 (RPS14): ctctctttccggtgtggagtctggagacgacgtgcagaaatg (Seq ID No: 1347)

Homo sapiens ribosomal protein L10 (RPL10): gcgcctctttcccttcggtgtgccactgaagatcctggtgtcgccatg (Seq ID No: 1348)

Homo sapiens ribosomal protein L10a (RPL10A): tagtctcttttccggttagcgcggcgtgagaagccatg (Seq ID No: 1349)

Homo sapiens ribosomal protein L35 (RPL35): tcctctttccctcggagcgggcggcggcgttggcggcttgtgcagcaatg (Seq ID No: 1350)

Homo sapiens ribosomal protein L13a (RPL13A): cctcctccttttccaagcggctgccgaagatg (Seq ID No: 1351)

Homo sapiens ribosomal protein L36 (RPL36): cagcccttccgccacggccgtctctggagagcagcagccatg (Seq ID No: 1352)

Homo sapiens ribosomal protein L36a (RPL36A): gtttctttctttccgcgccgatagcgctcacgcaagcatg (Seq ID No: 1353)

Homo sapiens ribosomal protein L41 (RPL41): tcgcc tttctctcggccttagcgccatttttttggaaacctctgcgccatg (Seq ID No: 1354)

Homo sapiens ribosomal protein S18 (RPS18): cgctctctcttccacaggaggcctacacgccgccgcttgtgctgcagccatg (Seq ID No: 1355)

Homo sapiens ribosomal protein S24 (RPS24): ggttctcttttcctccttggctgtctgaagatagatcgccatcatg (Seq ID No: 1356)

Homo sapiens ribosomal protein L8 (RPL8): tttcctctttcggccgcgctggtgaacaggtaggtcatccttgcggccttgcggcatg (Seq ID No: 1357)

Homo sapiens ribosomal protein L34 (RPL34): cttcctcttccggggacgttgtctgcaggtatg (Seq ID No: 1358)

Homo sapiens ribosomal protein S17 (RPS17): gtttcctcttttaccaaggacccgccaacatg (Seq ID No: 1359)

Homo sapiens ribosomal protein SA (RPSA):

ctgtcttttccgtgctacctgcagaggggtccatacggcgttgttctggattcccgtcgtaactta
aagggaaattttcacaatg (Seq ID No: 1360)

Homo sapiens eukaryotic translation initiation factor 3, subunit C (EIF3C):

```
cttctctctcggcgtttccgctgtcagggccctgcggtgtgactcgcgggctcagctggtccggcc
gtagcacctccgcgccgtcgccatg (Seq ID No: 1361)
```

Homo sapiens poly(A) binding protein, cytoplasmic 1 (PABPC1): cgctctcctcctctcacggaaaggtcgcggcctgtggccctgcgggcagccgtgccgagatg (Seq ID No: 1362)

Homo sapiens tubulin, beta 1 class VI (TUBB1):

```
cactcccttccaaaagcatgacaggcagaaagcagagaagggccaggactggctgagggcggggag
ctgggcctctggggtggacacacccttggtcacattgtgagggtagcttggttggccagtcccacc
actgcagtgaccacagttgtgttgggctcacaccagtgaaccgaagctctggattctgagagtctg
aggattccgtgaagatctcagacttgggctcagagcaaggatg (Seq ID No: 1363)
```

PpLuc(GC) - A64N64

```
GGGAGAAAGCTTGAGGATGGAGGACGCCAAGAACATCAAGAAGGGCCCGGCGCCCTTCTA
CCCGCTGGAGGACGGGACCGCCGGCGAGCAGCTCCACAAGGCCATGAAGCGGTACGCCCT
GGTGCCGGGCACGATCGCCTTCACCGACGCCCACATCGAGGTCGACATCACCTACGCGGA
GTACTTCGAGATGAGCGTGCGCCTGGCCGAGGCCATGAAGCGGTACGGCCTGAACACCAA
CCACCGGATCGTGGTGTGCTCGGAGAACAGCCTGCAGTTCTTCATGCCGGTGCTGGGCGC
CCTCTTCATCGGCGTGGCCGTCGCCCCGGCGAACGACATCTACAACGAGCGGGAGCTGCT
GAACAGCATGGGGATCAGCCAGCCGACCGTGGTGTTCGTGAGCAAGAAGGGCCTGCAGAA
GATCCTGAACGTGCAGAAGAAGCTGCCCATCATCCAGAAGATCATCATCATGGACAGCAA
GACCGACTACCAGGGCTTCCAGTCGATGTACACGTTCGTGACCAGCCACCTCCCGCCGGG
CTTCAACGAGTACGACTTCGTCCCGGAGAGCTTCGACCGGGACAAGACCATCGCCCTGAT
CATGAACAGCAGCGGCAGCACCGGCCTGCCGAAGGGGGTGGCCCTGCCGCACCGGACCGC
CTGCGTGCGCTTCTCGCACGCCCGGGACCCCATCTTCGGCAACCAGATCATCCCGGACAC
CGCCATCCTGAGCGTGGTGCCGTTCCACCACGGCTTCGGCATGTTCACGACCCTGGGCTA
CCTCATCTGCGGCTTCCGGGTGGTCCTGATGTACCGGTTCGAGGAGGAGCTGTTCCTGCG
GAGCCTGCAGGACTACAAGATCCAGAGCGCGCTGCTCGTGCCGACCCTGTTCAGCTTCTT
CGCCAAGAGCACCCTGATCGACAAGTACGACCTGTCGAACCTGCACGAGATCGCCAGCGG
GGGCGCCCCGCTGAGCAAGGAGGTGGGCGAGGCCGTGGCCAAGCGGTTCCACCTCCCGGG
CATCCGCCAGGGCTACGGCCTGACCGAGACCACGAGCGCGATCCTGATCACCCCCGAGGG
GGACGACAAGCCGGGCGCCGTGGGCAAGGTGGTCCCGTTCTTCGAGGCCAAGGTGGTGGA
CCTGGACACCGGCAAGACCCTGGGCGTGAACCAGCGGGGCGAGCTGTGCGTGCGGGGGCC
GATGATCATGAGCGGCTACGTGAACAACCCGGAGGCCACCAACGCCCTCATCGACAAGGA

CGGCTGGCTGCACAGCGGCGACATCGCCTACTGGGACGAGGACGAGCACTTCTTCATCGT
CGACCGGCTGAAGTCGCTGATCAAGTACAAGGGCTACCAGGTGGCGCCGGCCGAGCTGGA
GAGCATCCTGCTCCAGCACCCCAACATCTTCGACGCCGGCGTGGCCGGGCTGCCGGACGA
CGACGCCGGCGAGCTGCCGGCCGCGGTGGTGGTGCTGGAGCACGGCAAGACCATGACGGA
GAAGGAGATCGTCGACTACGTGGCCAGCCAGGTGACCACCGCCAAGAAGCTGCGGGGCGG
CGTGGTGTTCGTGGACGAGGTCCCGAAGGGCCTGACCGGGAAGCTCGACGCCCGGAAGAT
CCGCGAGATCCTGATCAAGGCCAAGAAGGGCGGCAAGATCGCCGTGTAAGACTAGTAGAT
CTAAAAAAAAAAAAAAAAAAAAAAAAAAAAAAAAAAAAAAAAAAAAAAAAAAAAAAAAAA
AAAAAATGCATCCCCCCCCCCCCCCCCCCCCCCCCCCCCCCCCAAAGGCTCTTTTCAGAGC
CACCAGAATT (SEQ ID No: 1364)
```

PpLuc(GC) - albumin7 - A64N64

```
GGGAGAAAGCTTGAGGATGGAGGACGCCAAGAACATCAAGAAGGGCCCGGCGCCCTTCTA
CCCGCTGGAGGACGGGACCGCCGGCGAGCAGCTCCACAAGGCCATGAAGCGGTACGCCCT
GGTGCCGGGCACGATCGCCTTCACCGACGCCCACATCGAGGTCGACATCACCTACGCGGA
GTACTTCGAGATGAGCGTGCGCCTGGCCGAGGCCATGAAGCGGTACGGCCTGAACACCAA
CCACCGGATCGTGGTGTGCTCGGAGAACAGCCTGCAGTTCTTCATGCCGGTGCTGGGCGC
CCTCTTCATCGGCGTGGCCGTCGCCCCGGCGAACGACATCTACAACGAGCGGGAGCTGCT
GAACAGCATGGGGATCAGCCAGCCGACCGTGGTGTTCGTGAGCAAGAAGGGCCTGCAGAA
GATCCTGAACGTGCAGAAGAAGCTGCCCATCATCCAGAAGATCATCATCATGGACAGCAA
GACCGACTACCAGGGCTTCCAGTCGATGTACACGTTCGTGACCAGCCACCTCCCGCCGGG
CTTCAACGAGTACGACTTCGTCCCGGAGAGCTTCGACCGGGACAAGACCATCGCCCTGAT
CATGAACAGCAGCGGCAGCACCGGCCTGCCGAAGGGGGTGGCCCTGCCGCACCGGACCGC
CTGCGTGCGCTTCTCGCACGCCCGGGACCCCATCTTCGGCAACCAGATCATCCCGGACAC
CGCCATCCTGAGCGTGGTGCCGTTCCACCACGGCTTCGGCATGTTCACGACCCTGGGCTA
CCTCATCTGCGGCTTCCGGGTGGTCCTGATGTACCGGTTCGAGGAGGAGCTGTTCCTGCG
GAGCCTGCAGGACTACAAGATCCAGAGCGCGCTGCTCGTGCCGACCCTGTTCAGCTTCTT
CGCCAAGAGCACCCTGATCGACAAGTACGACCTGTCGAACCTGCACGAGATCGCCAGCGG
GGGCGCCCCGCTGAGCAAGGAGGTGGGCGAGGCCGTGGCCAAGCGGTTCCACCTCCCGGG
CATCCGCCAGGGCTACGGCCTGACCGAGACCACGAGCGCGATCCTGATCACCCCCGAGGG
GGACGACAAGCCGGGCGCCGTGGGCAAGGTGGTCCCGTTCTTCGAGGCCAAGGTGGTGGA
CCTGGACACCGGCAAGACCCTGGGCGTGAACCAGCGGGGCGAGCTGTGCGTGCGGGGGCC
GATGATCATGAGCGGCTACGTGAACAACCCGGAGGCCACCAACGCCCTCATCGACAAGGA
CGGCTGGCTGCACAGCGGCGACATCGCCTACTGGGACGAGGACGAGCACTTCTTCATCGT
CGACCGGCTGAAGTCGCTGATCAAGTACAAGGGCTACCAGGTGGCGCCGGCCGAGCTGGA
GAGCATCCTGCTCCAGCACCCCAACATCTTCGACGCCGGCGTGGCCGGGCTGCCGGACGA
CGACGCCGGCGAGCTGCCGGCCGCGGTGGTGGTGCTGGAGCACGGCAAGACCATGACGGA
GAAGGAGATCGTCGACTACGTGGCCAGCCAGGTGACCACCGCCAAGAAGCTGCGGGGCGG
CGTGGTGTTCGTGGACGAGGTCCCGAAGGGCCTGACCGGGAAGCTCGACGCCCGGAAGAT
CCGCGAGATCCTGATCAAGGCCAAGAAGGGCGGCAAGATCGCCGTGTAAGACTAGTGCAT
CACATTTAAAAGCATCTCAGCCTACCATGAGAATAAGAGAAAGAAAATGAAGATCAATAG
CTTATTCATCTCTTTTTCTTTTTCGTTGGTGTAAAGCCAACACCCTGTCTAAAAAACATA
AATTTCTTTAATCATTTTGCCTCTTTTCTCTGTGCTTCAATTAATAAAAAATGGAAAGAA
CCTAGATCTAAAAAAAAAAAAAAAAAAAAAAAAAAAAAAAAAAAAAAAAAAAAAAAAAAAA
AAAAAAAAAAAAATGCATCCCCCCCCCCCCCCCCCCCCCCCCCCCCCCCCAAAGGCTCTTT
TCAGAGCCACCAGAATT (SEQ ID No: 1365)
```

RPL32RPL32 - PpLuc(GC) - A64N64

```
GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
```

```
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTAGATCTAAAAAAAAAAAAAAAAAAAA
AAAAAAAAAAAAAAAAAAAAAAAAAAAAAAAAAAAAAAAAAAAAATGCATCCCCCCCCCC
CCCCCCCCCCCCCCCCCCCCAAAGGCTCTTTTCAGAGCCACCAGAATT (SEQ ID No: 1366)
```

RPL32 - PpLuc(GC) - albumin7 - A64N64

```
GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
```

```
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAATGCATCCCC
CCCCCCCCCCCCCCCCCCCCCCCCCCAAAGGCTCTTTTCAGAGCCACCAGAATT (SEQ ID
No: 1367)
```

5'UTR of human ribosomal protein Large 32 (RPL32) lacking the 5' terminal oligopyrimidine tract GGCGCTGCCTACGGAGGTGGCAGCCATCTCCTTCTCGGCATC (SEQ ID No. 1368) Human albumin 3'UTR

```
CATCACATTT AAAAGCATCT CAGCCTACCA TGAGAATAAG AGAAAGAAAA TGAAGATCAA
AAGCTTATTC ATCTGTTTTT CTTTTTCGTT GGTGTAAAGC CAACACCCTG TCTAAAAAAC
ATAAATTTCT TTAATCATTT TGCCTCTTTT CTCTGTGCTT CAATTAATAA AAAATGGAAA
GAATCT (SEQ ID No: 1369)
```

3'UTR of Homo sapiens hemoglobin, alpha 1 (HBA1)

```
gctggagcctcggtggccatgcttcttgccccttgggcctccccccagcccctcctcccttcctg
cacccgtaccccgtggtctttgaataaagtctgagtgggcggc (SEQ ID No: 1370)
```

3'UTR of Homo sapiens hemoglobin, alpha 2 (HBA2)

```
gctggagcctcggtagccgttcctcctgcccgctgggcctcccaacgggccctcctcccctccttg
caccggcccttcctggtctttgaataaagtctgagtgggcag (SEQ ID No: 1371)
```

3'UTR of Homo sapiens hemoglobin, beta (HBB)

```
Gctcgctttcttgctgtccaatttctattaaaggttcctttgttccctaagtccaactactaaact
gggggatattatgaagggccttgagcatctggattctgcctaataaaaaacatttattttcattgc
(SEQ ID No: 1372)
```

3'UTR of Homo sapiens tyrosine hydroxylase (TH)

```
gtgcacggcgtccctgagggccccttcccaacctcccctggtcctgcactgtcccggagctcaggcc
ctggtgaggggctgggtcccgggtgccccccatgccctccctgctgccaggctcccactgcccctg
cacctgcttctcagcgcaacagctgtgtgtgcccgtggtgaggttgtgctgcctgtggtgaggtcc
tgtcctggctcccagggtcctggggggctgctgcactgccctccgcccttccctgacactgtctgct
gccccaatcaccgtcacaataaaagaaactgtggtctcta (SEQ ID No: 1373)
```

3'UTR of Homo sapiens arachidonate 15-lipoxygenase (ALOX15)

gcgtcgccacccttttggttatttcagcccccatcacccaagccacaagctgacccccttcgtggtta
tagccctgccctcccaagtcccaccctcttcccatgtcccaccctccctagaggggcacctttttca
tggtctctgcacccagtgaacacatttttactctagaggcatcacctgggaccttactcctctttcc

ttccttcctcctttcctatcttccttcctctctctcttcctctttcttcattcagatctatatggc
aaatagccacaattatataaatcatttcaagactagaataggggggatataatacatattactccac
accttttatgaatcaaatatgatttttttgttgttgttaagacagagtctcactttgacacccagg
ctggagtgcagtggtgccatcaccacggctcactgcagcctcagcgtcctgggctcaaatgatcct
cccacctcagcctcctgagtagctgggactacaggctcatgccatcatgcccagctaatattttt
tattttcgtggagacgggggcctcactatgttgcctaggctggaataggattttgaacccaaattg
agtttaacaataataaaaagttgttttacgctaaagatggaaaagaactaggactgaactatttta
ataaaatattggc (SEQ ID No: 1374)

3'UTR of Homo sapiens collagen, type I, alpha 1 (COL1A1)

actccctccatcccaacctggctccctcccacccaaccaactttccccccaacccggaaacagaca
agcaacccaaactgaaccccctcaaaagccaaaaaatgggagacaatttcacatggactttggaaa
atatttttttcctttgcattcatctctcaaacttagtttttatctttgaccaaccgaacatgacca
aaaaccaaaagtgcattcaaccttaccaaaaaaaaaaaaaaaaaaagaataaataaataactttt
aaaaaaggaagcttggtccacttgcttgaagacccatgcggggggtaagtccctttctgcccgttgg
gcttatgaaaccccaatgctgccctttctgctcctttctccacacccccttggggcctcccctcc
actccttcccaaatctgtctccccagaagacacaggaaacaatgtattgtctgcccagcaatcaaa
ggcaatgctcaaacacccaagtggccccacccctcagcccgctcctgcccgcccagcaccccagg
ccctggggggacctggggttctcagactgccaaagaagccttgccatctggcgctcccatggctctt
gcaacatctcccccttcgttttgaggggggtcatgccggggggagccaccagcccctcactgggttcg
gaggagagtcaggaagggccacgacaaagcagaaacatcggatttggggaacgcgtgtcaatccct
tgtgccgcagggctgggcgggagagactgttctgttccttgtgtaactgtgttgctgaaagactac
ctcgttcttgtcttgatgtgtcaccggggcaactgcctggggggcggggatgggggcagggtggaag
cggctccccattttataccaaaggtgctacatctatgtgatgggtgggggtggggagggaatcactg
gtgctatagaaattgagatgccccccaggccagcaaatgttccttttttgttcaaagtctattttt
attccttgatattttctttttttttttttttttttttgtggatggggacttgtgaatttttctaaag
gtgctatttaacatgggaggagagcgtgtgcggctccagcccagcccgctgctcactttccaccct
ctctccacctgcctctggcttctcaggcctctgctctccgacctctctcctctgaaaccctcctcc
acagctgcagcccatcctcccggctccctcctagtctgtcctgcgtcctctgtccccgggtttcag
agacaacttcccaaagcacaaagcagttttttcccctaggggtgggaggaagcaaaagactctgta
cctattttgtatgtgtataataatttgagatgttttaattattttgattgctggaataaagcatg
tggaaatgacccaaacataa (SEQ ID No: 1375)

albumin7 3'UTR

CATCACATTTAAAAGCATCTCAGCCTACCATGAGAATAAGAGAAAGAAAATGAAGATCAATAGCTT
ATTCATCTCTTTTTCTTTTTCGTTGGTGTAAAGCCAACACCCTGTCTAAAAAACATAAATTTCTTT
AATCATTTTGCCTCTTTTCTCTGTGCTTCAATTAATAAAAAATGGAAAGAACCT (SEQ ID No:
1376)

Human albumin 3'UTR + poly(A) sequence

```
CATCACATTT AAAAGCATCT CAGCCTACCA TGAGAATAAG AGAAAGAAAA TGAAGATCAA
AAGCTTATTC ATCTGTTTTT CTTTTTCGTT GGTGTAAAGC CAACACCCTG TCTAAAAAAC
ATAAATTTCT TTAATCATTT TGCCTCTTTT CTCTGTGCTT CAATTAATAA AAAATGGAAA
GAATCTAGAT CTAAAAAAAA AAAAAAAAAA AAAAAAAAAA AAAAAAAAAA AAAAAAAAAA
AAAAAAAAAA AAAAAA (SEQ ID No: 1377)
```

Human albumin 3'UTR fragment 1

```
AAAAGCATCT CAGCCTACCA TGAGAATAAG AGAAAGAAAA TGAAGATCAA AAGCTTATTC
ATCTGTTTTT CTTTTTCGTT GGTGTAAAGC CAACACCCTG TCTAAAAAAC ATAAATTTCT
TTAATCATTT TGCCTCTTTT CTCTGTGCTT CAATT (SEQ ID No: 1378)
```

Human albumin 3'UTR fragment 2

```
CATCACATTT AAAAGCATCT CAGCCTACCA TGAGAATAAG AGAAAGAAAA TGAAGATCAA
AAGCTTATTC ATCTGTTTTT CTTTTTCGTT GGTGTAAAGC CAACACCCTG (SEQ ID No:
1379)
```

Human albumin 3'UTR fragment 3

```
AAAAGCATCT CAGCCTACCA TGAGAATAAG AGAAAGAAAA TGAAGATCAA AAGCTTATTC
ATCTGTTTTT CTTTTTCGTT GGTGTAAAGC CAACACCCTG TCTAAAAAAC (SEQ ID No:
1380)
```

Human albumin 3'UTR fragment 4

```
CAGCCTACCA TGAGAATAAG AGAAAGAAAA TGAAGATCAA AAGCTTATTC ATCTGTTTTT
CTTTTTCGTT GGTGTAAAGC CAACACCCTG TCTAAAAAAC ATAAATTTCT (SEQ ID No:
1381)
```

Human albumin 3'UTR fragment 5

```
TGAGAATAAG AGAAAGAAAA TGAAGATCAA AAGCTTATTC ATCTGTTTTT CTTTTTCGTT
GGTGTAAAGC CAACACCCTG TCTAAAAAAC ATAAATTTCT TTAATCATTT (SEQ ID No:
1382)
```

Human albumin 3'UTR fragment 6

```
AGAAAGAAAA TGAAGATCAA AAGCTTATTC ATCTGTTTTT CTTTTTCGTT GGTGTAAAGC
CAACACCCTG TCTAAAAAAC ATAAATTTCT TTAATCATTT TGCCTCTTTT (SEQ ID No:
1383)
```

Human albumin 3'UTR fragment 7

```
TGAAGATCAA AAGCTTATTC ATCTGTTTTT CTTTTTCGTT GGTGTAAAGC CAACACCCTG
TCTAAAAAAC ATAAATTTCT TTAATCATTT TGCCTCTTTT CTCTGTGCTT (SEQ ID No:
1384)
```

Human albumin 3'UTR fragment 8

```
AAGCTTATTC ATCTGTTTTT CTTTTTCGTT GGTGTAAAGC CAACACCCTG TCTAAAAAAC
ATAAATTTCT TTAATCATTT TGCCTCTTTT CTCTGTGCTT CAATTAATAA (SEQ ID No:
1385)
```

Human albumin 3'UTR fragment 9

```
ATCTGTTTTT CTTTTTCGTT GGTGTAAAGC CAACACCCTG TCTAAAAAAC ATAAATTTCT
TTAATCATTT TGCCTCTTTT CTCTGTGCTT CAATTAATAA AAAATGGAAA (SEQ ID No:
1386)
```

Human albumin 3'UTR fragment 10

```
CAGCCTACCA TGAGAATAAG AGAAAGAAAA TGAAGATCAA AAGCTTATTC ATCTGTTTTT
CTTTTTCGTT GGTGTAAAGC CAACACCCTG TCTAAAAAAC ATAAATTTCT TTAATCATTT
TGCCTCTTTT CTCTGTGCTT CAATTAATAA A (SEQ ID No: 1387)
```

Human albumin 3'UTR fragment 11

```
TGAAGATCAA AAGCTTATTC ATCTGTTTTT CTTTTTCGTT GGTGTAAAGC CAACACCCTG
TCTAAAAAAC ATAAATTTCT TTAATCATTT TGCCTCTTTT CTCTGTGCTT CAATTAATAA
A (SEQ ID No: 1388)
```

Human albumin 3'UTR fragment 12

```
CTTTTTCGTT GGTGTAAAGC CAACACCCTG TCTAAAAAAC ATAAATTTCT TTAATCATTT
TGCCTCTTTT CTCTGTGCTT CAATTAATAA A (SEQ ID No: 1389)
```

Human albumin 3'UTR fragment 13 AAGCTTATTC ATCTGTTTTT CTTTTTCGTT GGTGTAAAGC CAACACCCTG TCTAAAAAAC (SEQ ID No: 1390)

Albumin7 3'UTR - poly(A) sequence - poly(C) sequence - HL

```
CATCACATTTAAAAGCATCTCAGCCTACCATGAGAATAAGAGAAAGAAAATGAAGATCAATAGCTT
ATTCATCTCTTTTTCTTTTTCGTTGGTGTAAAGCCAACACCCTGTCTAAAAAACATAAATTTCTTT
AATCATTTTGCCTCTTTTCTCTGTGCTTCAATTAATAAAAAATGGAAAGAACCTAGATCTAAAAAA
AAAAAAAAAAAAAAAAAAAAAAAAAAAAAAAAAAAAAAAAAAAAAAAAAAAAAAAATGCATCCC
CCCCCCCCCCCCCCCCCCCCCCCCCCCAAAGGCTCTTTTCAGAGCCACCAGAATT (SEQ ID
No: 1391)
```

Albumin7 3'UTR - poly(A) sequence - poly(C) sequence

```
CATCACATTTAAAAGCATCTCAGCCTACCATGAGAATAAGAGAAAGAAAATGAAGATCAATAGCTT
ATTCATCTCTTTTTCTTTTTCGTTGGTGTAAAGCCAACACCCTGTCTAAAAAACATAAATTTCTTT
AATCATTTTGCCTCTTTTCTCTGTGCTTCAATTAATAAAAAATGGAAAGAACCTAGATCTAAAAAA
AAAAAAAAAAAAAAAAAAAAAAAAAAAAAAAAAAAAAAAAAAAAAAAAAAAAAAAATGCATCCC
CCCCCCCCCCCCCCCCCCCCCCCCCCC (SEQ ID No: 1392)
```

Center, α-complex-binding portion of the 3'UTR of an α-globin gene GCCCGATGGGCCTCCCAACGGGCCCTCCTCCCCTCCTTGCACCG (SEQ ID NO: 1393)

Histone stem-loop CAAAGGCTCTTTTCAGAGCCACCA (SEQ ID NO: 1394)

ATP synthase lipid-binding protein, mitochondrial (atp5g2)

```
     tagttt ctcctctcga acgccaggtg gagcaaccgg ccggataccg ccacagccct
     ggcaggcggc gctgtgatg (SEQ ID NO: 1395)
```

RPL35 - PpLuc(GC) - albumin7 - A64N64

```
GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
```

```
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAATGCATCCCCCCCCCCCC
CCCCCCCCCCCCCCCCCCAAAGGCTCTTTTCAGAGCCACCAGAATT (SEQ ID NO: 1396)
```

RPL21 - PpLuc(GC) - albumin7 - A64N64

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA

CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAATGCATCCCCCCCCCCC
CCCCCCCCCCCCCCCCCCAAAGGCTCTTTTCAGAGCCACCAGAATT (SEQ ID NO: 1397)

ATP5A1 - PpLuc(GC) - albumin7 - A64N64

GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC
AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG
CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTGCATCACATTTAAAAGCATCTCAGCCTACCATGAGAATAAGAGAAAGAAAATG
AAGATCAATAGCTTATTCATCTCTTTTTCTTTTTCGTTGGTGTAAAGCCAACACCCTGTC
TAAAAAACATAAATTTCTTTAATCATTTTGCCTCTTTTTCTCTGTGCTTCAATTAATAAAA
AATGGAAAGAACCTAGATCTAAAAAAAAAAAAAAAAAAAAAAAAAAAAAAAAAAAAAAAA
AAAAAAAAAAAAAAAAAAAAAATGCATCCCCCCCCCCCCCCCCCCCCCCCCCCCCCCCCC
AAAGGCTCTTTTCAGAGCCACCAGAATT (SEQ ID NO: 1398)

HSD17B4 - PpLuc(GC) - albumin7 - A64N64

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTGCATCA
CATTTAAAAGCATCTCAGCCTACCATGAGAATAAGAGAAAGAAAATGAAGATCAATAGCT
TATTCATCTCTTTTTCTTTTTCGTTGGTGTAAAGCCAACACCCTGTCTAAAAAACATAAA
TTTCTTTAATCATTTTGCCTCTTTTCTCTGTGCTTCAATTAATAAAAAATGGAAAGAACC
TAGATCTAAAAAAAAAAAAAAAAAAAAAAAAAAAAAAAAAAAAAAAAAAAAAAAAAAAAAA
AAAAAAAAAAATGCATCCCCCCCCCCCCCCCCCCCCCCCCCCCCCCCCCAAAGGCTCTTTTC
AGAGCCACCAGAATT (SEQ ID NO: 1399)

AIG1 - PpLuc(GC) - albumin7 - A64N64

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA

```
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTGCATCACATTTAAAAGCATCTCAGCCTACCATGAGAA
TAAGAGAAAGAAAATGAAGATCAATAGCTTATTCATCTCTTTTTCTTTTTCGTTGGTGTA
AAGCCAACACCCTGTCTAAAAAACATAAATTTCTTTAATCATTTTGCCTCTTTTCTCTGT
GCTTCAATTAATAAAAAATGGAAAGAACCTAGATCTAAAAAAAAAAAAAAAAAAAAAAAA
AAAAAAAAAAAAAAAAAAAAAAAAAAAAAAAAAAAAAAAAATGCATCCCCCCCCCCCCCC
CCCCCCCCCCCCCCCAAAGGCTCTTTTCAGAGCCACCAGAATT (SEQ ID NO: 1400)
```

COX6C - PpLuc(GC) - albumin7 - A64N64

```
GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
```

TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTGCATCACATTTA
AAAGCATCTCAGCCTACCATGAGAATAAGAGAAAGAAAATGAAGATCAATAGCTTATTCA
TCTCTTTTTCTTTTTCGTTGGTGTAAAGCCAACACCCTGTCTAAAAAACATAAATTTCTT
TAATCATTTTGCCTCTTTTCTCTGTGCTTCAATTAATAAAAAATGGAAAGAACCTAGATC
TAAAAAAAAAAAAAAAAAAAAAAAAAAAAAAAAAAAAAAAAAAAAAAAAAAAAAAAAAAAA
AAAAATGCATCCCCCCCCCCCCCCCCCCCCCCCCCCCCCCAAAGGCTCTTTTCAGAGCC
ACCAGAATT (SEQ ID NO: 1401)

ASAH1 - PpLuc(GC) - albumin7 - A64N64

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAATGCATCCCC
CCCCCCCCCCCCCCCCCCCCAAAGGCTCTTTTCAGAGCCACCAGAATT (SEQ ID
NO: 1402)

mRPL21 - PpLuc(GC) - albumin7 - A64N64

GGGGCCGCCGCAGCCATCTTCCAGTAACTCGCCAAAAAGCTTGAGGATGGAGGACGCCAA
GAACATCAAGAAGGGCCCGGCGCCCTTCTACCCGCTGGAGGACGGGACCGCCGGCGAGCA

```
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTTCTC
TGTGCTTCAATTAATAAAAAATGGAAAGAACCTAGATCTAAAAAAAAAAAAAAAAAAAAAA
AAAAAAAAAAAAAAAAAAAAAAAAAAAAAAAAAAAAAAAAAATGCATCCCCCCCCCCCC
CCCCCCCCCCCCCCCCCCAAAGGCTCTTTTCAGAGCCACCAGAATT (SEQ ID NO: 1403)
```

mRPL35A - PpLuc(GC) - albumin7 - A64N64

```
GGGCCATCTTGGCGCCTGTGGAGGCCTGCTGGGAACAGGACTTCTAACAGCAAGTAAGCT
TGAGGATGGAGGACGCCAAGAACATCAAGAAGGGCCCGGCGCCCTTCTACCCGCTGGAGG
ACGGGACCGCCGGCGAGCAGCTCCACAAGGCCATGAAGCGGTACGCCCTGGTGCCGGGCA
CGATCGCCTTCACCGACGCCCACATCGAGGTCGACATCACCTACGCGGAGTACTTCGAGA
TGAGCGTGCGCCTGGCCGAGGCCATGAAGCGGTACGGCCTGAACACCAACCACCGGATCG
TGGTGTGCTCGGAGAACAGCCTGCAGTTCTTCATGCCGGTGCTGGGCGCCCTCTTCATCG
GCGTGGCCGTCGCCCCGGCGAACGACATCTACAACGAGCGGGAGCTGCTGAACAGCATGG
GGATCAGCCAGCCGACCGTGGTGTTCGTGAGCAAGAAGGGCCTGCAGAAGATCCTGAACG
TGCAGAAGAAGCTGCCCATCATCCAGAAGATCATCATCATGGACAGCAAGACCGACTACC
AGGGCTTCCAGTCGATGTACACGTTCGTGACCAGCCACCTCCCGCCGGGCTTCAACGAGT
ACGACTTCGTCCCGGAGAGCTTCGACCGGGACAAGACCATCGCCCTGATCATGAACAGCA
GCGGCAGCACCGGCCTGCCGAAGGGGGTGGCCCTGCCGCACCGGACCGCCTGCGTGCGCT
TCTCGCACGCCCGGGACCCCATCTTCGGCAACCAGATCATCCCGGACACCGCCATCCTGA
GCGTGGTGCCGTTCCACCACGGCTTCGGCATGTTCACGACCCTGGGCTACCTCATCTGCG
GCTTCCGGGTGGTCCTGATGTACCGGTTCGAGGAGGAGCTGTTCCTGCGGAGCCTGCAGG
ACTACAAGATCCAGAGCGCGCTGCTCGTGCCGACCCTGTTCAGCTTCTTCGCCAAGAGCA
CCCTGATCGACAAGTACGACCTGTCGAACCTGCACGAGATCGCCAGCGGGGGCGCCCCGC
```

TGAGCAAGGAGGTGGGCGAGGCCGTGGCCAAGCGGTTCCACCTCCCGGGCATCCGCCAGG
GCTACGGCCTGACCGAGACCACGAGCGCGATCCTGATCACCCCCGAGGGGGACGACAAGC
CGGGCGCCGTGGGCAAGGTGGTCCCGTTCTTCGAGGCCAAGGTGGTGGACCTGGACACCG
GCAAGACCCTGGGCGTGAACCAGCGGGGCGAGCTGTGCGTGCGGGGGCCGATGATCATGA
GCGGCTACGTGAACAACCCGGAGGCCACCAACGCCCTCATCGACAAGGACGGCTGGCTGC
ACAGCGGCGACATCGCCTACTGGGACGAGGACGAGCACTTCTTCATCGTCGACCGGCTGA
AGTCGCTGATCAAGTACAAGGGCTACCAGGTGGCGCCGGCCGAGCTGGAGAGCATCCTGC
TCCAGCACCCCAACATCTTCGACGCCGGCGTGGCCGGGCTGCCGGACGACGACGCCGGCG
AGCTGCCGGCCGCGGTGGTGGTGCTGGAGCACGGCAAGACCATGACGGAGAAGGAGATCG
TCGACTACGTGGCCAGCCAGGTGACCACCGCCAAGAAGCTGCGGGGCGGCGTGGTGTTCG
TGGACGAGGTCCCGAAGGGCCTGACCGGGAAGCTCGACGCCCGGAAGATCCGCGAGATCC
TGATCAAGGCCAAGAAGGGCGGCAAGATCGCCGTGTAAGACTAGTGCATCACATTTAAAA
GCATCTCAGCCTACCATGAGAATAAGAGAAAGAAAATGAAGATCAATAGCTTATTCATCT
CTTTTTCTTTTTCGTTGGTGTAAAGCCAACACCCTGTCTAAAAAACATAAATTTCTTTAA
TCATTTTGCCTCTTTTCTCTGTGCTTCAATTAATAAAAAATGGAAAGAACCTAGATCTAA
AAAAAAAAAAAAAAAAAAAAAAAAAAAAAAAAAAAAAAAAAAAAAAAAAAAAAAAAAAAA
AATGCATCCCCCCCCCCCCCCCCCCCCCCCCCCCCCCAAAGGCTCTTTTCAGAGCCACC
AGAATT (SEQ ID NO: 1404)

RPL35 - PpLuc(GC) - A64N64

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTAGATCTAAAAAAAAAAAAAAAAAAAAAAAAAAAA
AAAAAAAAAAAAAAAAAAAAAAAAAAAAAAAAAAAATGCATCCCCCCCCCCCCCCCCCCC
CCCCCCCCCCCCAAAGGCTCTTTTCAGAGCCACCAGAATT (SEQ ID NO: 1405)

RPL21 - PpLuc(GC) - A64N64

```
GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTAGATCTAAAAAAAAAAAAAAAAAAAAAAAAAAAA
AAAAAAAAAAAAAAAAAAAAAAAAAAAAAAAAAAAATGCATCCCCCCCCCCCCCCCCCCC
CCCCCCCCCCCCAAAGGCTCTTTTCAGAGCCACCAGAATT (SEQ ID NO: 1406)
```

ATP5A1 - PpLuc(GC) - A64N64

```
GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
```

CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC
AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG
CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTAGATCTAAAAAAAAAAAAAAAAAAAAAAAAAAAAAAAAAAAAAAAAAAAAAAAA
AAAAAAAAAAAAAAAAATGCATCCCCCCCCCCCCCCCCCCCCCCCCCCCCCCCAAAGGCT
CTTTTCAGAGCCACCAGAATT (SEQ ID NO: 1407)

HSD17B4 - PpLuc(GC) - A64N64

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTAGATCT
AAAAAAAAAAAAAAAAAAAAAAAAAAAAAAAAAAAAAAAAAAAAAAAAAAAAAAAAAAAAA
AAAATGCATCCCCCCCCCCCCCCCCCCCCCCCCCCCCCCCAAAGGCTCTTTTCAGAGCCA
CCAGAATT (SEQ ID NO: 1408)

AIG1 - PpLuc(GC) - A64N64

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTAGATCTAAAAAAAAAAAAAAAAAAAAAAAAAAAAAAA
AAAAAAAAAAAAAAAAAAAAAAAAAAAAAAAAATGCATCCCCCCCCCCCCCCCCCCCCCC
CCCCCCCCCAAAGGCTCTTTTCAGAGCCACCAGAATT (SEQ ID NO: 1409)

COX6C - PpLuc(GC) - A64N64

GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC

AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTAGATCTAAAAAA
AAAAAAAAAAAAAAAAAAAAAAAAAAAAAAAAAAAAAAAAAAAAAAAAAAAAAAAAAAATG
CATCCCCCCCCCCCCCCCCCCCCCCCCCCCCCCCCAAAGGCTCTTTTCAGAGCCACCAGAA
TT (SEQ ID NO: 1410)

ASAH1 - PpLuc(GC) - A64N64

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTAGATCTAAAAAAAAAAAAAAAAAAAA
AAAAAAAAAAAAAAAAAAAAAAAAAAAAAAAAAAAAAAAAAAATGCATCCCCCCCCCCC
CCCCCCCCCCCCCCCCCCCCAAAGGCTCTTTTCAGAGCCACCAGAATT (SEQ ID NO: 1411)

5'UTR of human ribosomal protein Large 35 (RPL35) lacking the 5' terminal oligopyrimidine tract
GGAGCGGGCGGCGGCGTTGGCGGCTTGTGCAGCA (SEQ ID NO: 1412)5'UTR of human ribosomal protein Large 21 (RPL21) lacking the 5' terminal oligopyrimidine tract GGCCGGAACCGCCATCTTCCAGTAATTCGCCAAA (SEQ ID NO: 1413)

5'UTR of human ATP synthase, H+ transporting, mitochondrial F1 complex, alpha subunit 1, cardiac muscle (ATP5A1) lacking the 5' terminal oligopyrimidine tract

```
GCGGCTCGGCCATTTTGTCCCAGTCAGTCCGGAGGCTGCGGCTGCAGAAGTACCGCCTGCGGAGTA
ACTGCAAAG (SEQ ID NO: 1414)
```

5'UTR of human hydroxysteroid (17-beta) dehydrogenase 4 (HSD17B4) lacking the 5' terminal oligopyrimidine tract GTCCCGCAGTCGGCGTCCAGCGGCTCTGCTTGTTCGTGTGTGTGTCGTTGCAGGCCTTATTC (SEQ ID NO: 1415)

5'UTR of human androgen-induced 1 (AIG1) lacking the 5' terminal oligopyrimidine tract GCCGCCCAGCCGGTCCAGGCCTCTGGCGAAC (SEQ ID NO: 1416)

5'UTR of human cytochrome c oxidase subunit VIc (COX6C) lacking the 5' terminal oligopyrimidine tract AGTCAGGAAGGACGTTGGTGTTGAGGTTAGCATACGTATCAAGGACAGTAACTACC (SEQ ID NO: 1417)

5'UTR of human N-acylsphingosine amidohydrolase (acid ceramidase) 1 (ASAH1) lacking the 5' terminal oligopyrimidine tract GCCTCTGCTGGAGTCCGGGGAGTGGCGTTGGCTGCTAGAGCG (SEQ ID NO: 1418)

5'UTR of mouse ribosomal protein Large 21 (mRPL21) lacking the 5' terminal oligopyrimidine tract GGCCGCCGCAGCCATCTTCCAGTAACTCGCCAAA (SEQ ID NO: 1419)

5'UTR of mouse ribosomal protein large 35A (mRPL35A) lacking the 5' terminal oligopyrimidine tract GCCATCTTGGCGCCTGTGGAGGCCTGCTGGGAACAGGACTTCTAACAGCAAGT (SEQ ID NO: 1420)

Mouse ribosomal protein Large 21 (mRPL21)

```
TCCTCCTTTCGGCCGCCGCAGCCATCTTCCAGTAACTCGCCAAAATGCCATCTTCCAGTAACTCGC
CAAAATG (SEQ ID NO: 1421)
```

mouse ribosomal protein large 35A (mRPL35A)

```
CTTCCTCTTTCCGCCATCTTGGCGCCTGTGGAGGCCTGCTGGGAACAGGACTTCTAACAGCAAGTA
TG (SEQ ID NO: 1422)
```

**Claims**

1. An artificial nucleic acid molecule comprising:

   a. at least one 5'-untranslated region element (5'UTR element) which comprises or consists of

   a nucleic acid sequence, which consists of a continuous stretch of nucleotides corresponding to a continuous stretch of nucleotides in the full-length 5'UTR of a TOP gene, which represents at least 80% of the full-length 5'UTR of a TOP gene,
   or
   a nucleic acid sequence, which consists of a continuous stretch of nucleotides corresponding to a continuous stretch of nucleotides in a variant of the 5'UTR of a TOP gene, which represents at least 80% of the variant of the 5'UTR of a TOP gene, wherein the variant of the 5'UTR of a TOP gene is at least 80% identical to the naturally occuring 5'UTR, from which the variant is derived,

   wherein the at least one 5'UTR element does not comprise a functional 5'TOP motif;

b. at least one open reading frame (ORF); and

c. at least one 3'UTR element, which comprises or consists of

a nucleic acid sequence which consists of a continuous stretch of nucleotides corresponding to a continuous stretch of nucleotides in the full-length 3'UTR of an albumin gene, which represents at least 80% of the full-length 3'UTR of an albumin gene,

or

a nucleic acid sequence which consists of a continuous stretch of nucleotides corresponding to a continuous stretch of nucleotides in a variant of a 3'UTR of an albumin gene, which represents at least 80% of the variant of the 3'UTR of an albumin gene, wherein the variant of the 3'UTR of an albumin gene is at least 80% identical to the naturally occuring 3'UTR the variant is derived from.

2. The artificial nucleic acid molecule according to claim 1, wherein the at least one 3'UTR element comprises or consist of a nucleic acid sequence which is derived from the 3'UTR of a vertebrate albumin gene or from a variant thereof, preferably from the 3'UTR of a mammalian albumin gene or from a variant thereof, more preferably from the 3'UTR of a human albumin gene or from a variant thereof, even more preferably from the 3'UTR of the human albumin gene according to GenBank Accession number NM_000477.5 or from a variant thereof.

3. The artificial nucleic acid molecule according to claims 1 or 2, wherein the at least one 3'UTR element exhibits a length of at least about 50 nucleotides, preferably of at least about 75 nucleotides, more preferably of at least about 100 nucleotides, even more preferably of at least about 125 nucleotides, most preferably of at least about 150 nucleotides.

4. The artificial nucleic acid molecule according to claims 1-3, wherein the 5'UTR element and the open reading frame are heterologous.

5. The artificial nucleic acid molecule according to claim 1 to 4, wherein the 5'UTR element is suitable for increasing protein production from the artificial nucleic acid molecule.

6. The artificial nucleic acid molecule according to any one of claims 1-5, wherein the nucleic acid sequence which is derived from a 5'UTR of a TOP gene, preferably the 5'UTR element, starts at its 5'-end with a nucleotide located at position 1, 2, 3, 4, 5, 6, 7, 8, 9 or 10 downstream of the TOP-motif of the 5'UTR of the TOP gene.

7. The artificial nucleic acid molecule according to any one of claims 1-6, wherein the nucleic acid sequence which is derived from a 5'UTR of a TOP gene, preferably the 5'UTR element terminates at its 3'-end with a nucleotide located at position 1, 2, 3, 4, 5, 6, 7, 8, 9 or 10 upstream of the start codon of the open reading frame of the gene or mRNA it is derived from.

8. The artificial nucleic acid molecule according to any one of claims 1-7, wherein the nucleic acid sequence which is derived from the 5'UTR of a TOP gene is derived from the 5'UTR of a eukaryotic TOP gene or from a variant thereof, preferably from the 5'UTR of a plant or animal TOP gene or from a variant thereof, more preferably from the 5'UTR of a chordate TOP gene or from a variant thereof, even more preferably from the 5'UTR of a vertebrate TOP gene or from a variant thereof, most preferably from the 5'UTR of a mammalian TOP gene, such as a human TOP gene, or from a variant thereof.

9. The artificial nucleic acid molecule according to any of claims 1 to 8, wherein the at least one 3'UTR element and the at least one 5'UTR element act at least additively, preferably synergistically to increase protein production from said artificial nucleic acid molecule.

10. The artificial nucleic acid molecule according to any one of claims 1-9, further comprising

d. a poly(A) sequence and/or a polyadenylation signal.

11. The artificial nucleic acid molecule according to claim 10, wherein the polyadenylation signal is located within the 3'UTR element.

12. The artificial nucleic acid molecule according to claim 10 or 11, wherein the polyadenylation signal comprises the consensus sequence NN(U/T)ANA, with N = A or U, preferably AA(U/T)AAA or A(U/T)(U/T)AAA.

13. The artificial nucleic acid molecule according to any one of claims 10-12, wherein the polyadenylation signal, preferably the consensus sequence NNUANA, is located less than about 50 nucleotides upstream of the 3'-end of the 3'UTR.

14. The artificial nucleic acid molecule according to any one of claims 10-13, wherein the poly(A) sequence has a length of about 20 to about 300 adenine nucleotides, preferably of about 40 to about 200 adenine nucleotides, more preferably of about 50 to about 100 adenine nucleotides.

15. The artificial nucleic acid molecule according to any one of claims 1 to 14, which further comprises a histone stem-loop sequence.

16. The artificial nucleic acid molecule according to any one of claims 1-15, wherein the 5'UTR element is derived from a 5'UTR of a TOP gene encoding a ribosomal Large protein (RPL) or from a variant of a 5'UTR of a TOP gene encoding a ribosomal Large protein (RPL).

17. The artificial nucleic acid molecule according to any one of claims 1-16, wherein the 5'UTR element comprises or consists of a nucleic acid sequence having an identity of at least about 80%, more preferably of at least about 90%, even more preferably of at least about 95%, even more preferably of at least about 99% to a nucleic acid sequence extending from nucleotide position 5 to the nucleotide position immediately 5' to the start codon of a nucleic acid sequence according to any of SEQ ID NOs. 1-1363, 1395, 1421 or 1422 or to a corresponding RNA sequence, or wherein the at least one 5'UTR element comprises or consists of a fragment of a nucleic acid sequence which has an identity of at least about 80%, more preferably of at least about 90%, even more preferably of at least about 95%, even more preferably of at least about 99% to a nucleic acid sequence extending from nucleotide position 5 to the nucleotide position immediately 5' to the start codon of a nucleic acid sequence according to SEQ ID NOs. 1-1363, 1395, 1421 or 1422 or to a corresponding RNA sequence.

18. The artificial nucleic acid molecule according to any one of claims 1-17, wherein the 5'UTR element comprises or consists of a nucleic acid sequence having an identity of at least about 80%, more preferably of at least about 90%, even more preferably of at least about 95%, even more preferably of at least about 99% to the 5'UTR of a nucleic acid sequence according to any of SEQ ID NOs. 67, 259, 1284-1318, 1344, 1346, 1348-1354, 1357, 1358, 1421 or 1422 or to a corresponding RNA sequence, or wherein the at least one 5'UTR element comprises or consists of a fragment of a nucleic acid sequence which has an identity of at least about 80%, more preferably of at least about 90%, even more preferably of at least about 95%, even more preferably of at least about 99% to the 5'UTR of a nucleic acid sequence according to SEQ ID No. SEQ ID NOs: 67, 259, 1284-1318, 1344, 1346, 1348-1354, 1357, and 1358, 1421 or 1422 or to a corresponding RNA sequence.

19. The artificial nucleic acid molecule according to any one of claims 1-18, wherein the 5'UTR element comprises or consists of a nucleic acid sequence which is derived from the 5'UTR a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene (HSD17B4), an androgen-induced 1 gene (AIG1), cytochrome c oxidase subunit VIc gene (COX6C), or a N-acylsphingosine amidohydrolase (acid ceramidase) 1 gene (ASAH1) or from a variant thereof, preferably from a vertebrate ribosomal protein Large 32 gene (RPL32), a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit 1, cardiac muscle (ATP5A1) gene, a vertebrate hydroxysteroid (17-beta) dehydrogenase 4 gene (HSD17B4), a vertebrate androgen-induced 1 gene (AIG1), a vertebrate cytochrome c oxidase subunit VIc gene (COX6C), or a vertebrate N-acylsphingosine amidohydrolase (acid ceramidase) 1 gene (ASAH1) or from a variant thereof, more preferably from a mammalian ribosomal protein Large 32 gene (RPL32), a ribosomal protein Large 35 gene (RPL35), a ribosomal protein Large 21 gene (RPL21), a mammalian ATP synthase, H+ transporting, mitochondrial F1 complex, alpha subunit 1, cardiac muscle (ATP5A1) gene, a mammalian hydroxysteroid (17-beta) dehydrogenase 4 gene (HSD17B4), a mammalian androgen-induced 1 gene (AIG1), a mammalian cytochrome c oxidase subunit VIc gene (COX6C), or a mammalian N-acylsphingosine amidohydrolase (acid ceramidase) 1 gene (ASAH1) or from a variant thereof, most preferably from a human ribosomal protein Large 32 gene (RPL32), a human ribosomal protein Large 35 gene (RPL35), a human ribosomal protein Large 21 gene (RPL21), a human ATP synthase, H+ transporting, mitochondrial F1 complex, alpha subunit 1, cardiac muscle (ATP5A1) gene, a human hydroxysteroid (17-beta) dehydrogenase 4 gene (HSD17B4), a human androgen-induced 1 gene (AIG1 a human cytochrome c oxidase subunit VIc gene (COX6C), or a human N-acylsphingosine amidohydrolase (acid ceramidase) 1 gene (ASAH1) or from a variant thereof.

20. The artificial nucleic acid molecule according to any one of claims 1-19, wherein the 5'UTR element comprises or consists of a nucleic acid sequence which has an identity of at least about 80%, more preferably of at least about 90%, even more preferably of at least about 95%, even more preferably of at least about 99% to the nucleic acid sequence according to SEQ ID NO. 1368 or SEQ ID NOs 1412-1420, or to a corresponding RNA sequence, or wherein the at least one 5'UTR element comprises or consists of a fragment of a nucleic acid sequence which has an identity of at least about 80%, more preferably of at least about 90%, even more preferably of at least about 95%, even more preferably of at least about 99% to the nucleic acid sequence according to SEQ ID No. 1368 or SEQ ID NOs 1412-1420, or to a corresponding RNA sequence.

21. The artificial nucleic acid molecule according to any one of claims 18 or 20, wherein the fragment consists of a continuous stretch of nucleotides corresponding to a continuous stretch of nucleotides in the full-length sequence, which represents at least 80%, preferably at least 90%, of the full-length sequence the fragment is derived from.

22. The artificial nucleic acid molecule according to any one of claims 1-21, wherein the at least one 5'UTR element exhibits a length of at least about 20 nucleotides, preferably of at least about 30 nucleotides, more preferably of at least about 40 nucleotides.

23. The artificial nucleic acid molecule according to any one of claims 1-22, wherein the at least one 3'UTR element comprises or consists of a nucleic acid sequence which has an identity of at least about 80%, more preferably of at least about 90%, even more preferably of at least about 95%, even more preferably of at least about 99% to a nucleic acid sequence selected from SEQ ID NOs. 1369-1393 or to a corresponding RNA sequence, or wherein the at least one 3'UTR element comprises or consists of a fragment of a nucleic acid sequence which has an identity of at least about 80%, more preferably of at least about 90%, even more preferably of at least about 95%, even more preferably of at least about 99% to a nucleic acid sequence selected from SEQ ID NOs. 1369-1393 or to a corresponding RNA sequence.

24. The artificial nucleic acid molecule according to claim 23, wherein the fragment consists of a continuous stretch of nucleotides corresponding to a continuous stretch of nucleotides in the full-length sequence, which represents at least 80%, preferably at least 90%, of the full-length sequence the fragment is derived from.

25. The artificial nucleic acid molecule according to claim 23 or 24, wherein the fragment exhibits a length of at least about 50 nucleotides, preferably of at least about 75 nucleotides, more preferably of at least about 100 nucleotides, even more preferably of at least about 125 nucleotides, most preferably of at least about 150 nucleotides.

26. The artificial nucleic acid molecule according to any one of claims 1-25, wherein the at least one 3'UTR element exhibits a length of at least about 50 nucleotides, preferably of at least about 75 nucleotides, more preferably of at least about 100 nucleotides, even more preferably of at least about 125 nucleotides, most preferably of at least about 150 nucleotides.

27. The artificial nucleic acid molecule according to any one of claims 1-26, wherein the open reading frame does not code for a reporter protein, such as a GFP protein, a luciferase protein, a globin protein, human growth hormon, or human albumin, preferably not for albumin.

28. The artificial nucleic acid molecule according to any one of claims 1-27, further comprising a 5'-cap structure, a poly(C) sequence and/or an IRES-motif.

29. The artificial nucleic acid molecule according to any one of claims 1-28, further comprising a promoter containing-sequence.

30. The artificial nucleic acid molecule according to any one of claims 1-29, wherein the artificial nucleic acid molecule, preferably the open reading frame, is at least partially G/C modified, preferably wherein the G/C content of the open reading frame is increased compared to the wild type open reading frame.

31. The artificial nucleic acid molecule according to any one of claims 1-30, wherein the open reading frame comprises a codon-optimized region, preferably, wherein the open reading frame is codon-optimized.

32. The artificial nucleic acid molecule according to any one of claims 1-31, which is an RNA, preferably an mRNA molecule.

**33.** A vector comprising:

a. at least one 5'-untranslated region element (5'UTR element) which comprises or consists of

a nucleic acid sequence which consists of a continuous stretch of nucleotides corresponding to a continuous stretch of nucleotides in the full-length 5'UTR of a TOP gene, which represents at least 80% of the full-length 5'UTR of a TOP gene, or
a nucleic acid sequence which consists of a continuous stretch of nucleotides corresponding to a continuous stretch of nucleotides in a variant of the 5'UTR of a TOP gene, which represents at least 80% of the variant of the 5'UTR of a TOP gene, wherein the variant of the 5'UTR of a TOP gene is at least 80% identical to the naturally occuring 5'UTR the variant is derived from,
wherein the at least one 5'UTR element does not comprise a functional 5'TOP motif;

b. at least one open reading frame (ORF) and/or at least one cloning site; and
c. at least one 3'UTR element, which comprises or consists of

a nucleic acid sequence which consists of a continuous stretch of nucleotides corresponding to a continuous stretch of nucleotides in the full-length 3'UTR of an albumin gene, which represents at least 80% of the full-length 3'UTR of an albumin gene, or
a nucleic acid sequence which consists of a continuous stretch of nucleotides corresponding to a continuous stretch of nucleotides in a variant of a 3'UTR of an albumin gene, which represents at least 80% of the variant of the 3'UTR of an albumin gene, wherein the variant of the 3'UTR of an albumin gene is at least 80% identical to the naturally occuring 3'UTR the variant is derived from.

**34.** The vector according to claim 33, wherein the 5'UTR element and the open reading frame are heterologous.

**35.** The vector according to claim 33 or 34, which is an RNA or DNA vector.

**36.** The vector according to any one of claims 33-35, which is a plasmid vector or a viral vector, preferably a plasmid vector.

**37.** The vector according to any one of claims 33-36, which comprises or codes for an artificial nucleic acid molecule according to any one of claims 1-32.

**38.** The vector according to any one of claims 33-37, which is a circular molecule.

**39.** The vector according to claim 38, wherein the ORF, the poly(A) sequence or the 3'UTR element of the coding strand is followed in 5'→3' direction by a restriction site for linearization of the circular vector molecule.

**40.** A cell comprising the artificial nucleic acid molecule according to any one of claims 1-32 or the vector according to any one of claims 33-39, preferably a mammalian cell, more preferably an isolated cell of a mammalian subject, preferably of a human subject.

**41.** A pharmaceutical composition comprising the artificial nucleic acid molecule according to any one of claims 1-32, the vector according to any one of claims 33-39, or the cell according to claim 40, preferably further comprising one or more pharmaceutically acceptable diluents and/or excipients and/or one or more adjuvants.

**42.** The artificial nucleic acid molecule according to any one of claims 1-32, the vector according to any one of claims 33-39, the cell according to claim 40, or the pharmaceutical composition according to claim 41 for use as a medicament.

**43.** The artificial nucleic acid molecule according to any one of claims 1-32, the vector according to any one of claims 33-39, the cell according to claim 40, or the pharmaceutical composition according to claim 41 for use as a vaccine or for use in gene therapy.

**Patentansprüche**

**1.** Künstliches Nukleinsäuremolekül, umfassend:

a. wenigstens ein Element einer 5'-untranslatierten Region (5'-UTR-Element), umfassend oder bestehend aus

eine(r) Nukleinsäuresequenz, welche aus einem fortlaufenden Abschnitt von Nukleotiden besteht, welcher einem fortlaufenden Abschnitt von Nukleotiden in der 5'-UTR voller Länge eines TOP-Gens entspricht, welcher wenigstens 80% der 5'-UTR voller Länge eines TOP-Gens darstellt, oder
eine(r) Nukleinsäuresequenz, welche aus einem fortlaufenden Abschnitt von Nukleotiden besteht, welcher einem fortlaufenden Abschnitt von Nukleotiden in einer Variante der 5'-UTR eines TOP-Gens entspricht, welcher wenigstens 80% der Variante der 5'-UTR eines TOP-Gens darstellt, wobei die Variante der 5'-UTR eines TOP-Gens zu wenigstens 80% identisch mit der natürlich vorkommenden 5'-UTR, von der die Variante abgeleitet ist, ist,

wobei das wenigstens eine 5'-UTR-Element kein funktionelles 5'-TOP-Motiv umfasst;
b. wenigstens einen offenen Leserahmen ("open reading frame", ORF); und
c. wenigstens ein 3'-UTR-Element, umfassend oder bestehend aus

eine(r) Nukleinsäuresequenz, welche aus einem fortlaufenden Abschnitt von Nukleotiden besteht, der einem fortlaufenden Abschnitt von Nukleotiden in der 3'-UTR voller Länge eines Albumin-Gens entspricht, welcher wenigstens 80% der 3'-UTR voller Länge eines Albumin-Gens darstellt, oder
eine(r) Nukleinsäuresequenz, welche aus einem fortlaufenden Abschnitt von Nukleotiden besteht, der einem fortlaufenden Abschnitt von Nukleotiden in einer Variante der 3'-UTR eines Albumin-Gens entspricht, welcher wenigstens 80% der Variante der 3'-UTR eines Albumin-Gens darstellt, wobei die Variante der 3'-UTR eines Albumin-Gens zu wenigstens 80% identisch mit der natürlich vorkommenden 3'-UTR, von der die Variante abgeleitet ist, ist.

2. Künstliches Nukleinsäuremolekül gemäß Anspruch 1, wobei das wenigstens eine 3'-UTR-Element eine Nukleinsäuresequenz umfasst oder aus einer Nukleinsäuresequenz besteht, die von der 3'-UTR eines Vertebraten-Albumin-Gens oder von einer Variante davon, vorzugsweise von der 3'-UTR eines Säugetier-Albumin-Gens oder von einer Variante davon, bevorzugter von der 3'-UTR eines humanen Albumin-Gens oder von einer Variante davon, noch bevorzugter von der 3'-UTR des humanen Albumin-Gens gemäß der GenBank-Zugangsnummer NM_000477.5 oder von einer Variante davon, abgeleitet ist.

3. Künstliches Nukleinsäuremolekül gemäß Anspruch 1 oder 2, wobei das wenigstens eine 3'-UTR-Element eine Länge von wenigstens etwa 50 Nukleotiden, vorzugsweise von wenigstens etwa 75 Nukleotiden, bevorzugter von wenigstens etwa 100 Nukleotiden, noch bevorzugter von wenigstens etwa 125 Nukleotiden, am meisten bevorzugt von wenigstens etwa 150 Nukleotiden, aufweist.

4. Künstliches Nukleinsäuremolekül gemäß den Ansprüchen 1 bis 3, wobei das 5'-UTR-Element und der offene Leserahmen heterolog sind.

5. Künstliches Nukleinsäuremolekül gemäß Anspruch 1 bis 4, wobei das 5'-UTR-Element geeignet ist, die Proteinproduktion von dem künstlichen Nukleinsäuremolekül zu erhöhen.

6. Künstliches Nukleinsäuremolekül gemäß irgendeinem der Ansprüche 1 bis 5, wobei die Nukleinsäuresequenz, die von einer 5'-UTR eines TOP-Gens abgeleitet ist, vorzugsweise das 5'-UTR-Element, an ihrem 5'-Ende mit einem Nukleotid startet, das sich an Position 1, 2, 3, 4, 5, 6, 7, 8, 9 oder 10 stromabwärts des TOP-Motivs der 5'-UTR des TOP-Gens befindet.

7. Künstliches Nukleinsäuremolekül gemäß irgendeinem der Ansprüche 1 bis 6, wobei die Nukleinsäuresequenz, die von einer 5'-UTR eines TOP-Gens abgeleitet ist, vorzugsweise das 5'-UTR-Element, an ihrem 3'-Ende mit einem Nukleotid endet, das sich an Position 1, 2, 3, 4, 5, 6, 7, 8, 9 oder 10 stromaufwärts des Start-Codons des offenen Leserahmens des Gens oder der mRNA, von dem/der es abgeleitet ist, befindet.

8. Künstliches Nukleinsäuremolekül gemäß irgendeinem der Ansprüche 1 bis 7, wobei die Nukleinsäuresequenz, welche von der 5'-UTR eines TOP-Gens abgeleitet ist, von der 5'-UTR eines eukaryotischen TOP-Gens oder von einer Variante davon, vorzugsweise von der 5'-UTR eines pflanzlichen oder tierischen TOP-Gens oder von einer Variante davon, bevorzugter von der 5'-UTR eines Chordaten-TOP-Gens oder von einer Variante davon, noch bevorzugter von der 5'-UTR eines Vertebraten-TOP-Gens oder von einer Variante davon, am meisten bevorzugt von der 5'-UTR eines Säugetier-TOP-Gens, wie beispielsweise einem humanen TOP-Gen, oder von einer Variante davon, abgeleitet ist.

9.  Künstliches Nukleinsäuremolekül gemäß irgendeinem der Ansprüche 1 bis 8, wobei das wenigstens eine 3'-UTR-Element und das wenigstens eine 5'-UTR-Element zumindest additiv, vorzugsweise synergistisch, wirken, um die Proteinproduktion von dem künstlichen Nukleinsäuremolekül zu erhöhen.

10. Künstliches Nukleinsäuremolekül gemäß irgendeinem der Ansprüche 1 bis 9, ferner umfassend

    d. eine Poly(A)-Sequenz und/oder ein Polyadenylierungssignal.

11. Künstliches Nukleinsäuremolekül gemäß Anspruch 10, wobei das Polyadenylierungssignal innerhalb des 3'-UTR-Elements lokalisiert ist.

12. Künstliches Nukleinsäuremolekül gemäß Anspruch 10 oder 11, wobei das Polyadenylierungssignal die Konsensus-Sequenz NN(U/T)ANA, wobei N=A oder U ist, vorzugsweise AA(U/T)AAA oder A(U/T)(U/T)AAA, umfasst.

13. Künstliches Nukleinsäuremolekül gemäß irgendeinem der Ansprüche 10 bis 12, wobei das Polyadenylierungssignal, vorzugsweise die Konsensus-Sequenz NNUANA, weniger als etwa 50 Nukleotide stromaufwärts des 3'-Endes der 3'-UTR lokalisiert ist.

14. Künstliches Nukleinsäuremolekül gemäß irgendeinem der Ansprüche 10 bis 13, wobei die Poly(A)-Sequenz eine Länge von etwa 20 bis etwa 300 Adenin-Nukleotiden, vorzugsweise von etwa 40 bis etwa 200 Adenin-Nukleotiden, bevorzugter von etwa 50 bis etwa 100 Adenin-Nukleotiden, aufweist.

15. Künstliches Nukleinsäuremolekül gemäß irgendeinem der Ansprüche 1 bis 14, welches ferner eine Histon-Stammschleifen-Sequenz ("histone stem-loop sequence") umfasst.

16. Künstliches Nukleinsäuremolekül gemäß irgendeinem der Ansprüche 1 bis 15, wobei das 5'-UTR-Element von einer 5'-UTR eines TOP-Gens, welches ein ribosomales Large-Protein (RPL) kodiert, oder von einer Variante einer 5'-UTR eines TOP-Gens, welches ein ribosomales Large-Protein (RPL) kodiert, abgeleitet ist.

17. Künstliches Nukleinsäuremolekül gemäß irgendeinem der Ansprüche 1 bis 16, wobei das 5'-UTR-Element eine Nukleinsäuresequenz umfasst oder aus einer Nukleinsäuresequenz besteht, die eine Identität von wenigstens etwa 80%, bevorzugter von wenigstens etwa 90%, noch bevorzugter von wenigstens etwa 95%, noch bevorzugter von wenigstens etwa 99%, zu einer Nukleinsäuresequenz, die sich von der Nukleotid-Position 5 bis zu der Nukleotid-Position unmittelbar 5' zu dem Start-Codon einer Nukleinsäuresequenz gemäß irgendeiner der SEQ ID NOs. 1-1363, 1395, 1421 oder 1422 erstreckt, oder zu einer entsprechenden RNA-Sequenz aufweist, oder wobei das wenigstens eine 5'-UTR-Element ein Fragment einer Nukleinsäuresequenz umfasst oder aus einem Fragment einer Nukleinsäuresequenz besteht, das eine Identität von wenigstens etwa 80%, bevorzugter von wenigstens etwa 90%, noch bevorzugter von wenigstens etwa 95%, noch bevorzugter von wenigstens etwa 99%, zu einer Nukleinsäuresequenz, die sich von der Nukleotid-Position 5 bis zu der Nukleotid-Position unmittelbar 5' zu dem Start-Codon einer Nukleinsäuresequenz gemäß den SEQ ID NOs. 1-1363, 1395, 1421 oder 1422 erstreckt, oder zu einer entsprechenden RNA-Sequenz aufweist.

18. Künstliches Nukleinsäuremolekül gemäß irgendeinem der Ansprüche 1 bis 17, wobei das 5'-UTR-Element eine Nukleinsäuresequenz umfasst oder aus einer Nukleinsäuresequenz besteht, die eine Identität von wenigstens etwa 80%, bevorzugter von wenigstens etwa 90%, noch bevorzugter von wenigstens etwa 95%, noch bevorzugter von wenigstens etwa 99%, zu der 5'-UTR einer Nukleinsäuresequenz gemäß irgendeiner der SEQ ID NOs. 67, 259, 1284-1318, 1344, 1346, 1348-1354, 1357, 1358, 1421 oder 1422 oder zu einer entsprechenden RNA-Sequenz aufweist, oder wobei das wenigstens eine 5'-UTR-Element ein Fragment einer Nukleinsäuresequenz umfasst oder aus einem Fragment einer Nukleinsäuresequenz besteht, das eine Identität von wenigstens etwa 80%, bevorzugter von wenigstens etwa 90%, noch bevorzugter von wenigstens etwa 95%, noch bevorzugter von wenigstens etwa 99%, zu der 5'-UTR einer Nukleinsäuresequenz gemäß den SEQ ID NOs. 67, 259, 1284-1318, 1344, 1346, 1348-1354, 1357 und 1358, 1421 oder 1422 oder zu einer entsprechenden RNA-Sequenz aufweist.

19. Künstliches Nukleinsäuremolekül gemäß irgendeinem der Ansprüche 1 bis 18, wobei das 5'-UTR-Element eine Nukleinsäuresequenz umfasst oder aus einer Nukleinsäuresequenz besteht, die abgeleitet ist von der 5'-UTR eines Gens für das ribosomale Protein Large 32 (RPL32), eines Gens für das ribosomale Protein Large 35 (RPL35), eines Gens für das ribosomale Protein Large 21 (RPL21), eines ATP5A1-Gens (ATP-Synthase, H+-transportierend, mitochondrialer F1-Komplex, alpha-Untereinheit 1, Herzmuskel), eines Hydroxysteroid-(17-beta)-dehydrogenase-4-Gens (HSD17B4), eines Androgen-induzierten Gens 1 (AIG1), eines Cytochrom-C-Oxidase-Untereinheit-Vlc-Gens (COX6C) oder eines N-Acylsphingosin-Amidohydrolase (saure Ceramidase)-1-Gens (ASAH1) oder von einer Variante davon, vorzugsweise eines Vertebraten-Gens für das ribosomale Protein Large 32 (RPL32), eines Vertebraten-Gens für das ribosomale Protein Large 35 (RPL35), eines Vertebraten-Gens für das ribosomale Protein Large 21 (RPL21), eines Vertebraten-ATP5A1-Gens (ATP-Synthase, H+-transportierend, mitochondrialer F1-Komplex, alpha-Untereinheit 1, Herzmuskel), eines Vertebraten-Hydroxysteroid-(17-beta)-dehydrogenase-4-Gens (HSD17B4), eines Vertebraten-Androgen-induzierten Gens 1 (AIG1), eines Vertebraten-Cytochrom-C-Oxidase-Untereinheit-Vlc-Gens (COX6C) oder eines Vertebraten-N-Acylsphingosin-Amidohydrolase (saure Ceramidase)-1-Gens (ASAH1) oder von einer Variante davon, bevorzugter eines Säugetier-Gens für das ribosomale Protein Large 32 (RPL32), eines Säugetier-Gens für das ribosomale Protein Large 35 (RPL35), eines Säugetier-Gens für das ribosomale Protein Large 21 (RPL21), eines Säugetier-ATP5A1-Gens (ATP-Synthase, H+-transportierend, mitochondrialer F1-Komplex, alpha-Untereinheit 1, Herzmuskel), eines Säugetier-Hydroxysteroid-(17-beta)-dehydrogenase-4-Gens (HSD17B4), eines Säugetier-Androgen-induzierten Gens 1 (AIG1), eines Säugetier-Cytochrom-C-Oxidase-Untereinheit-Vlc-Gens (COX6C) oder eines Säugetier-N-Acylsphingosin-Amidohydrolase (saure Ceramidase)-1-Gens (ASAH1) oder von einer Variante davon, am meisten bevorzugt eines humanen Gens für das ribosomale Protein Large 32 (RPL32), eines humanen Gens für das ribosomale Protein Large 35 (RPL35), eines humanen Gens für das ribosomale Protein Large 21 (RPL21), eines humanen ATP5A1-Gens (ATP-Synthase, H+-transportierend, mitochondrialer F1-Komplex, alpha-Untereinheit 1, Herzmuskel), eines humanen Hydroxysteroid-(17-beta)-dehydrogenase-4-Gens (HSD17B4), eines humanen Androgen-induzierten Gens 1 (AIG1), eines humanen Cytochrom-C-Oxidase-Untereinheit-Vlc-Gens (COX6C) oder eines humanen N-Acylsphingosin-Amidohydrolase (saure Ceramidase)-1-Gens (ASAH1) oder von einer Variante davon.

20. Künstliches Nukleinsäuremolekül gemäß irgendeinem der Ansprüche 1 bis 19, wobei das 5'-UTR-Element eine Nukleinsäuresequenz umfasst oder aus einer Nukleinsäuresequenz besteht, die eine Identität von wenigstens etwa 80%, bevorzugter von wenigstens etwa 90%, noch bevorzugter von wenigstens etwa 95%, noch bevorzugter von wenigstens etwa 99%, zu der Nukleinsäuresequenz gemäß SEQ ID NO. 1368 oder SEQ ID NOs. 1412-1420 oder zu einer entsprechenden RNA-Sequenz aufweist, oder wobei das wenigstens eine 5'-UTR-Element ein Fragment einer Nukleinsäuresequenz umfasst oder aus einem Fragment einer Nukleinsäuresequenz besteht, das eine Identität von wenigstens etwa 80%, bevorzugter von wenigstens etwa 90%, noch bevorzugter von wenigstens etwa 95%, noch bevorzugter von wenigstens etwa 99%, zu der Nukleinsäuresequenz gemäß SEQ ID NO. 1386 oder SEQ ID NOs. 1412-1420 oder zu einer entsprechenden RNA-Sequenz aufweist.

21. Künstliches Nukleinsäuremolekül gemäß irgendeinem der Ansprüche 18 oder 20, wobei das Fragment aus einem fortlaufenden Abschnitt von Nukleotiden besteht, der einem fortlaufenden Abschnitt von Nukleotiden in der Sequenz voller Länge entspricht, der wenigstens 80%, vorzugsweise wenigstens 90%, der Sequenz voller Länge, von der das Fragment abgeleitet ist, darstellt.

22. Künstliches Nukleinsäuremolekül gemäß irgendeinem der Ansprüche 1 bis 21, wobei das wenigstens eine 5'-UTR-Element eine Länge von wenigstens etwa 20 Nukleotiden, vorzugsweise von wenigstens etwa 30 Nukleotiden, bevorzugter von wenigstens etwa 40 Nukleotiden, aufweist.

23. Künstliches Nukleinsäuremolekül gemäß irgendeinem der Ansprüche 1 bis 22, wobei das wenigstens eine 3'-UTR-Element eine Nukleinsäuresequenz umfasst oder aus einer Nukleinsäuresequenz besteht, die eine Identität von wenigstens etwa 80%, bevorzugter von wenigstens etwa 90%, noch bevorzugter von wenigstens etwa 95%, noch bevorzugter von wenigstens etwa 99%, zu einer Nukleinsäuresequenz ausgewählt aus den SEQ ID NOs. 1369-1393 oder zu einer entsprechenden RNA-Sequenz aufweist, oder wobei das wenigstens eine 3'-UTR-Element ein Fragment einer Nukleinsäuresequenz umfasst oder aus einem Fragment einer Nukleinsäuresequenz besteht, das eine Identität von wenigstens etwa 80%, bevorzugter von wenigstens etwa 90%, noch bevorzugter von wenigstens etwa 95%, noch bevorzugter von wenigstens etwa 99%, zu einer Nukleinsäuresequenz ausgewählt aus den SEQ ID NOs. 1369-1393 oder zu einer entsprechenden RNA-Sequenz aufweist.

24. Künstliches Nukleinsäuremolekül gemäß Anspruch 23, wobei das Fragment aus einem fortlaufenden Abschnitt von Nukleotiden besteht, der einem fortlaufenden Abschnitt von Nukleotiden in der Sequenz voller Länge entspricht, der wenigstens 80%, vorzugsweise wenigstens 90%, der Sequenz voller Länge, von der das Fragment abgeleitet ist, darstellt.

25. Künstliches Nukleinsäuremolekül gemäß Anspruch 23 oder 24, wobei das Fragment eine Länge von wenigstens etwa 50 Nukleotiden, vorzugsweise von wenigstens etwa 75 Nukleotiden, bevorzugter von wenigstens etwa 100 Nukleotiden, noch bevorzugter von wenigstens etwa 125 Nukleotiden, am meisten bevorzugt von wenigstens etwa 150 Nukleotiden, aufweist.

26. Künstliches Nukleinsäuremolekül gemäß irgendeinem der Ansprüche 1 bis 25, wobei das wenigstens eine 3'-UTR-Element eine Länge von wenigstens etwa 50 Nukleotiden, vorzugsweise von wenigstens etwa 75 Nukleotiden, bevorzugter von wenigstens etwa 100 Nukleotiden, noch bevorzugter von wenigstens etwa 125 Nukleotiden, am meisten bevorzugt von wenigstens etwa 150 Nukleotiden, aufweist.

27. Künstliches Nukleinsäuremolekül gemäß irgendeinem der Ansprüche 1 bis 26, wobei der offene Leserahmen nicht für ein Reporterprotein, wie beispielsweise ein GFP-Protein, ein Luciferase-Protein, ein Globin-Protein, einen humanen Wachstumsfaktor oder humanes Albumin, vorzugsweise nicht für Albumin, kodiert.

28. Künstliches Nukleinsäuremolekül gemäß irgendeinem der Ansprüche 1 bis 27, welches ferner eine 5'-Cap-Struktur, eine Poly(C)-Sequenz und/oder ein IRES-Motiv umfasst.

29. Künstliches Nukleinsäuremolekül gemäß irgendeinem der Ansprüche 1 bis 28, welches ferner eine einen Promotor enthaltende Sequenz umfasst.

30. Künstliches Nukleinsäuremolekül gemäß irgendeinem der Ansprüche 1 bis 29, wobei das künstliche Nukleinsäuremolekül, vorzugsweise der offene Leserahmen, zumindest teilweise G/C-modifiziert ist, wobei bevorzugt der G/C-Gehalt des offenen Leserahmens im Vergleich zu dem offenen Leserahmen der Wildtyp-Sequenz erhöht ist.

31. Künstliches Nukleinsäuremolekül gemäß irgendeinem der Ansprüche 1 bis 30, wobei der offene Leserahmen eine Codon-optimierte Region umfasst, wobei vorzugsweise der offene Leserahmen Codon-optimiert ist.

32. Künstliches Nukleinsäuremolekül gemäß irgendeinem der Ansprüche 1 bis 31, welches ein RNA-Molekül, vorzugsweise ein mRNA-Molekül, ist.

33. Vektor, umfassend:

a. wenigstens ein Element einer 5'-untranslatierten Region (5'-UTR-Element), umfassend oder bestehend aus

eine(r) Nukleinsäuresequenz, welche aus einem fortlaufenden Abschnitt von Nukleotiden besteht, der einem fortlaufenden Abschnitt von Nukleotiden in der 5'-UTR voller Länge eines TOP-Gens entspricht, welcher wenigstens 80% der 5'-UTR voller Länge eines TOP-Gens darstellt,
oder
eine(r) Nukleinsäuresequenz, welche aus einem fortlaufenden Abschnitt von Nukleotiden besteht, der einem fortlaufenden Abschnitt von Nukleotiden in einer Variante der 5'-UTR voller Länge eines TOP-Gens entspricht, welcher wenigstens 80% der Variante der 5'-UTR voller Länge eines TOP-Gens darstellt, wobei die Variante der 5'-UTR eines TOP-Gens zu wenigstens 80% identisch mit der natürlich vorkommenden 5'-UTR, von der die Variante abgeleitet ist, ist,
wobei das wenigstens eine 5'-UTR-Element kein funktionelles 5'-TOP-Motiv umfasst;

b. wenigstens einen offenen Leserahmen (ORF) und/oder wenigstens eine Klonierungsstelle; und
c. wenigstens ein 3'-UTR-Element, umfassend oder bestehend aus

eine(r) Nukleinsäuresequenz, welche aus einem fortlaufenden Abschnitt von Nukleotiden besteht, der einem fortlaufenden Abschnitt von Nukleotiden in der 3'-UTR voller Länge eines Albumin-Gens entspricht, welcher wenigstens 80% der 3'-UTR voller Länge eines Albumin-Gens darstellt, oder
eine(r) Nukleinsäuresequenz, welche aus einem fortlaufenden Abschnitt von Nukleotiden besteht, der einem fortlaufenden Abschnitt von Nukleotiden in einer Variante der 3'-UTR eines Albumin-Gens entspricht, welcher wenigstens 80% der Variante der 3'-UTR eines Albumin-Gens darstellt, wobei die Variante der 3'-UTR eines Albumin-Gens zu wenigstens 80% identisch mit der natürlich vorkommenden 3'-UTR, von der die Variante abgeleitet ist, ist.

**34.** Vektor gemäß Anspruch 33, wobei das 5'-UTR-Element und der offene Leserahmen heterolog sind.

**35.** Vektor gemäß Anspruch 33 oder 34, bei dem es sich um einen RNA- oder DNA-Vektor handelt.

**36.** Vektor gemäß irgendeinem der Ansprüche 33 bis 35, bei dem es sich um einen Plasmid-Vektor oder einen viralen Vektor, vorzugsweise um einen Plasmid-Vektor, handelt.

**37.** Vektor gemäß irgendeinem der Ansprüche 33 bis 36, welcher ein künstliches Nukleinsäuremolekül gemäß irgendeinem der Ansprüche 1 bis 32 umfasst oder für dieses kodiert.

**38.** Vektor gemäß irgendeinem der Ansprüche 33 bis 37, bei dem es sich um ein zirkuläres Molekül handelt.

**39.** Vektor gemäß Anspruch 38, wobei sich an den ORF, die Poly(A)-Sequenz oder das 3'-UTR-Element des kodierenden Stranges in 5'→3'-Richtung eine Restriktionsstelle zur Linearisierung des zirkulären Vektormoleküls anschließt.

**40.** Zelle, umfassend das künstliche Nukleinsäuremolekül gemäß irgendeinem der Ansprüche 1 bis 32 oder den Vektor gemäß irgendeinem der Ansprüche 33 bis 39, vorzugsweise eine Säugetierzelle, bevorzugter eine isolierte Zelle von einem Säugetier, bevorzugt einem Menschen.

**41.** Pharmazeutische Zusammensetzung umfassend das künstliche Nukleinsäuremolekül gemäß irgendeinem der Ansprüche 1 bis 32, den Vektor gemäß irgendeinem der Ansprüche 33 bis 39 oder die Zelle gemäß Anspruch 40, welche vorzugsweise außerdem einen oder mehrere pharmazeutisch akzeptable Verdünnungsmittel und/oder pharmazeutische Trägerstoffe und/oder ein oder mehrere Adjuvanzien umfasst.

**42.** Das künstliche Nukleinsäuremolekül gemäß irgendeinem der Ansprüche 1 bis 32, der Vektor gemäß irgendeinem der Ansprüche 33 bis 39, die Zelle gemäß Anspruch 40 oder die pharmazeutische Zusammensetzung gemäß Anspruch 41 zur Verwendung als Medikament.

**43.** Das künstliche Nukleinsäuremolekül gemäß irgendeinem der Ansprüche 1 bis 32, der Vektor gemäß irgendeinem der Ansprüche 33 bis 39, die Zelle gemäß Anspruch 40 oder die pharmazeutische Zusammensetzung gemäß Anspruch 41 zur Verwendung als Vakzine oder zur Verwendung in der Gentherapie.

**Revendications**

**1.** Molécule d'acide nucléique artificielle comprenant:

a. au moins un élément de région non traduite en 5' (élément de 5'UTR) qui comprend ou consiste en
une séquence d'acide nucléique, qui consiste en une suite continue de nucléotides correspondant à une suite continue de nucléotides dans la 5'UTR de longueur intégrale d'un gène TOP, qui représente au moins 80% de la 5'UTR de longueur intégrale d'un gène TOP,
ou
une séquence d'acide nucléique, qui consiste en une suite continue de nucléotides correspondant à une suite continue de nucléotides dans un variant de la 5'UTR d'un gène TOP, qui représente au moins 80% du variant de la 5'UTR d'un gène TOP, où le variant de la 5'UTR d'un gène TOP est identique à au moins 80 % à la 5'UTR naturelle, de laquelle le variant est dérivé,
où le au moins un élément de 5'UTR ne comprend pas un motif de 5'TOP fonctionnel;
b. au moins un cadre de lecture ouvert (ORF); et
c. au moins one élément de 3'UTR, qui comprend ou consiste en
une séquence d'acide nucléique qui consiste en une suite continue de nucléotides correspondant à une suite continue de nucléotides dans la 3'UTR de longueur intégrale d'un gène d'albumine, qui représente au moins 80% de la 3'UTR de longueur intégrale d'un gène d'albumine,
ou
une séquence d'acide nucléique qui consiste en une suite continue de nucléotides correspondant à une suite continue de nucléotides dans un variant d'une 3'UTR d'un gène d'albumine, qui représente au moins 80% du variant de la 3'UTR d'un gène d'albumine, où le variant de la 3'UTR d'un gène d'albumine est identique à au moins 80 % à la 3'UTR naturelle de laquelle est dérivé le variant.

**2.** Molécule d'acide nucléique artificielle selon la revendication 1, où le au moins un élément de 3'UTR comprend ou consiste en une séquence d'acide nucléique qui est dérivée de la 3'UTR d'un gène d'albumine de vertébré ou d'un variant de celle-ci, de préférence de la 3'UTR d'un gène d'albumine de mammifère ou d'un variant de celle-ci, de préférence encore de la 3'UTR d'un gène d'albumine humaine ou d'un variant de celle-ci, de préférence encore de la 3'UTR du gène d'albumine humaine selon GenBank numéro d'ordre NM_000477.5 ou d'un variant de celle-ci.

**3.** Molécule d'acide nucléique artificielle selon les revendications 1 ou 2, où le au moins un élément de 3'UTR présente une longueur d'au moins environ 50 nucléotides, de préférence d'au moins environ 75 nucléotides, de préférence encore d'au moins environ 100 nucléotides, de préférence encore d'au moins environ 125 nucléotides, de manière particulièrement préférable d'au moins environ 150 nucléotides.

**4.** Molécule d'acide nucléique artificielle selon les revendications 1-3, où l'élément de 5'UTR et le cadre de lecture ouvert sont hétérologues.

**5.** Molécule d'acide nucléique artificielle selon la revendication 1 à 4, où l'élément de 5'UTR est approprié pour augmenter la production de protéine à partir de la molécule d'acide nucléique artificielle.

**6.** Molécule d'acide nucléique artificielle selon l'une quelconque des revendications 1-5, où la séquence d'acide nucléique qui est dérivée d'une 5'UTR d'un gène TOP, de préférence l'élément de 5'UTR, commence à son extrémité 5' avec un nucléotide situé à la position 1, 2, 3, 4, 5, 6, 7, 8, 9 ou 10 en aval du motif TOP de la 5'UTR du gène TOP.

**7.** Molécule d'acide nucléique artificielle selon l'une quelconque des revendications 1-6, où la séquence d'acide nucléique qui est dérivée d'une 5'UTR d'un gène TOP, de préférence l'élément de 5'UTR, se termine à son extrémité 3' avec un nucléotide situé à la position 1, 2, 3, 4, 5, 6, 7, 8, 9 ou 10 en amont du codon d'initiation du cadre de lecture ouvert du gène ou de l'ARNm duquel elle est dérivée.

**8.** Molécule d'acide nucléique artificielle selon l'une quelconque des revendications 1-7, où la séquence d'acide nucléique qui est dérivée de la 5'UTR d'un gène TOP est dérivée de la 5'UTR d'un gène TOP eucaryote ou d'un variant de celle-ci, de préférence de la 5'UTR d'un gène TOP de plante ou d'animal ou d'un variant de celle-ci, de préférence encore de la 5'UTR d'un gène TOP de chordé ou d'un variant de celle-ci, de préférence encore de la 5'UTR d'un gène TOP de vertébré ou d'un variant de celle-ci, de manière particulièrement préférable de la 5'UTR d'un gène TOP de mammifère, comme un gène TOP humain, ou d'un variant de celle-ci.

**9.** Molécule d'acide nucléique artificielle selon l'une quelconque des revendications 1 à 8, où le au moins un élément de 3'UTR et le au moins un élément de 5'UTR agissent au moins additivement, de préférence de manière synergique pour augmenter la production de protéine à partir de ladite molécule d'acide nucléique artificielle.

**10.** Molécule d'acide nucléique artificielle selon l'une quelconque des revendications 1-9, comprenant en outre

   d. une séquence poly(A) et/ou un signal de polyadénylation.

**11.** Molécule d'acide nucléique artificielle selon la revendication 10, où le signal de polyadénylation est situé dans l'élément de 3'UTR.

**12.** Molécule d'acide nucléique artificielle selon la revendication 10 ou 11, où le signal de polyadénylation comprend la séquence consensus NN(U/T)ANA, avec N = A ou U, de préférence AA(U/T)AAA ou A(U/T)(U/T)AAA.

**13.** Molécule d'acide nucléique artificielle selon l'une quelconque des revendications 10-12, où le signal de polyadénylation, de préférence la séquence consensus NNUANA, est situé à moins d'environ 50 nucléotides en amont de l'extrémité 3' de la 3'UTR.

**14.** Molécule d'acide nucléique artificielle selon l'une quelconque des revendications 10-13, où la séquence poly(A) a une longueur d'environ 20 à environ 300 nucléotides à adénine, de préférence d'environ 40 à environ 200 nucléotides à adénine, de préférence encore d'environ 50 à environ 100 nucléotides à adénine.

**15.** Molécule d'acide nucléique artificielle selon l'une quelconque des revendications 1 à 14, qui comprend en outre une séquence de tige-boucle d'histone.

**16.** Molécule d'acide nucléique artificielle selon l'une quelconque des revendications 1-15, où l'élément de 5'UTR est dérivé d'une 5'UTR d'un gène TOP codant une protéine Large ribosomique (RPL) ou d'un variant d'une 5'UTR d'un gène TOP codant une protéine Large ribosomique (RPL).

**17.** Molécule d'acide nucléique artificielle selon l'une quelconque des revendications 1-16, où l'élément de 5'UTR comprend ou consiste en une séquence d'acide nucléique ayant une identité d'au moins environ 80%, de préférence encore d'au moins environ 90%, de préférence encore d'au moins environ 95%, de préférence encore d'au moins environ 99% avec une séquence d'acide nucléique s'étendant de la position de nucléotide 5 à la position de nucléotide immédiatement en 5' du codon d'initiation d'une séquence d'acide nucléique selon l'une quelconque de SEQ ID NO. 1-1363, 1395, 1421 ou 1422 ou avec une séquence d'ARN correspondante, ou bien où le au moins un élément de 5'UTR comprend ou consiste en un fragment d'une séquence d'acide nucléique qui a une identité d'au moins environ 80%, de préférence encore d'au moins environ 90%, de préférence encore d'au moins environ 95%, de préférence encore d'au moins environ 99% avec une séquence d'acide nucléique s'étendant de la position de nucléotide 5 à la position de nucléotide immédiatement en 5' du codon d'initiation d'une séquence d'acide nucléique selon SEQ ID NO. 1-1363, 1395, 1421 ou 1422 ou avec une séquence d'ARN correspondante.

**18.** Molécule d'acide nucléique artificielle selon l'une quelconque des revendications 1-17, où l'élément de 5'UTR comprend ou consiste en une séquence d'acide nucléique ayant une identité d'au moins environ 80%, de préférence encore d'au moins environ 90%, de préférence encore d'au moins environ 95%, de préférence encore d'au moins environ 99% avec la 5'UTR d'une séquence d'acide nucléique selon l'une quelconque de SEQ ID NO. 67, 259, 1284-1318, 1344, 1346, 1348-1354, 1357, 1358, 1421 ou 1422 ou avec une séquence d'ARN correspondante, ou bien où le au moins un élément de 5'UTR comprend ou consiste en un fragment d'une séquence d'acide nucléique qui a une identité d'au moins environ 80%, de préférence encore d'au moins environ 90%, de préférence encore d'au moins environ 95%, de préférence encore d'au moins environ 99% avec la 5'UTR d'une séquence d'acide nucléique selon SEQ ID No. SEQ ID NO: 67, 259, 1284-1318, 1344, 1346, 1348-1354, 1357, et 1358, 1421 ou 1422 ou avec une séquence d'ARN correspondante.

**19.** Molécule d'acide nucléique artificielle selon l'une quelconque des revendications 1-18, où l'élément de 5'UTR comprend ou consiste en une séquence d'acide nucléique qui est dérivée de la 5'UTR d'un gène de protéine Large ribosomique 32 (RPL32), d'un gène de protéine Large ribosomique 35 (RPL35), d'un gène de protéine Large ribosomique 21 (RPL21), d'un gène d'ATP synthase, transportant H+, complexe mitochondrial F1, sous-unité alpha 1, de muscle cardiaque (ATP5A1), d'un gène d'hydroxystéroïde (17-bêta) déshydrogénase 4 (HSD17B4), d'un gène induit par androgène 1 (AIG1), d'un gène de cytochrome c oxydase sous-unité VIc (COX6C), ou d'un gène de N-acylsphingosine amidohydrolase (céramidase acide) 1 (ASAH1) ou d'un variant de celle-ci, de préférence d'un gène de protéine Large ribosomique 32 (RPL32) de vertébré, d'un gène de protéine Large ribosomique 35 (RPL35) de vertébré, d'un gène de protéine Large ribosomique 21 (RPL21) de vertébré, d'un gène d'ATP synthase, transportant H+, complexe mitochondrial F1, sous-unité alpha 1, de muscle cardiaque (ATP5A1) de vertébré, d'un gène d'hydroxystéroïde (17-bêta) déshydrogénase 4 (HSD17B4) de vertébré, d'un gène induit par androgène 1 (AIG1) de vertébré, d'un gène de cytochrome c oxydase sous-unité VIc (COX6C) de vertébré, ou d'un gène de N-acylsphingosine amidohydrolase (céramidase acide) 1 (ASAH1) de vertébré ou d'un variant de celle-ci, de préférence encore d'un gène de protéine Large ribosomique 32 (RPL32) de mammifère, d'un gène de protéine Large ribosomique 35 (RPL35) de mammifère, d'un gène de protéine Large ribosomique 21 (RPL21) de mammifère, d'un gène d'ATP synthase, transportant H+, complexe mitochondrial F1, sous-unité alpha 1, de muscle cardiaque (ATP5A1) de mammifère, d'un gène d'hydroxystéroïde (17-bêta) déshydrogénase 4 (HSD17B4) de mammifère, d'un gène induit par androgène 1 (AIG1) de mammifère, d'un gène de cytochrome c oxydase sous-unité VIc (COX6C) de mammifère, ou d'un gène de N-acylsphingosine amidohydrolase (céramidase acide) 1 (ASAH1) de mammifère ou d'un variant de celle-ci, de manière particulièrement préférable d'un gène de protéine Large ribosomique 32 (RPL32) humain, d'un gène de protéine Large ribosomique 35 (RPL35) humain, d'un gène de protéine Large ribosomique 21 (RPL21) humain, d'un gène d'ATP synthase, transportant H+, complexe mitochondrial F1, sous-unité alpha 1, de muscle cardiaque (ATP5A1) humain, d'un gène d'hydroxystéroïde (17-bêta) déshydrogénase 4 (HSD17B4) humain, d'un gène induit par androgène 1 (AIG1) humain, d'un gène de cytochrome c oxydase sous-unité VIc (COX6C) humain, ou d'un gène de N-acylsphingosine amidohydrolase (céramidase acide) 1 (ASAH1) humain ou d'un variant de celle-ci.

**20.** Molécule d'acide nucléique artificielle selon l'une quelconque des revendications 1-19, où l'élément de 5'UTR comprend ou consiste en une séquence d'acide nucléique qui a une identité d'au moins environ 80%, de préférence encore d'au moins environ 90%, de préférence encore d'au moins environ 95%, de préférence encore d'au moins environ 99% avec la séquence d'acide nucléique selon SEQ ID NO. 1368 ou SEQ ID NO 1412-1420, ou avec une séquence d'ARN correspondante, ou bien où le au moins un élément de 5'UTR comprend ou consiste en un fragment d'une séquence d'acide nucléique qui a une identité d'au moins environ 80%, de préférence encore d'au moins environ 90%, de préférence encore d'au moins environ 95%, de préférence encore d'au moins environ 99% avec la séquence d'acide nucléique selon SEQ ID No. 1368 ou SEQ ID NO 1412-1420, ou avec une séquence d'ARN correspondante.

21. Molécule d'acide nucléique artificielle selon l'une quelconque des revendications 18 ou 20, où le fragment consiste en une suite continue de nucléotides correspondant à une suite continue de nucléotides dans la séquence de longueur intégrale, qui représente au moins 80%, de préférence au moins 90%, de la séquence de longueur intégrale de laquelle est dérivé le fragment.

22. Molécule d'acide nucléique artificielle selon l'une quelconque des revendications 1-21, où le au moins un élément de 5'UTR présente une longueur d'au moins environ 20 nucléotides, de préférence d'au moins environ 30 nucléotides, de préférence encore d'au moins environ 40 nucléotides.

23. Molécule d'acide nucléique artificielle selon l'une quelconque des revendications 1-22, où le au moins un élément de 3'UTR comprend ou consiste en une séquence d'acide nucléique qui a une identité d'au moins environ 80%, de préférence encore d'au moins environ 90%, de préférence encore d'au moins environ 95%, de préférence encore d'au moins environ 99% avec une séquence d'acide nucléique choisie parmi SEQ ID NO. 1369-1393 ou avec une séquence d'ARN correspondante, ou bien où le au moins un élément de 3'UTR comprend ou consiste en un fragment d'une séquence d'acide nucléique qui a une identité d'au moins environ 80%, de préférence encore d'au moins environ 90%, de préférence encore d'au moins environ 95%, de préférence encore d'au moins environ 99% avec une séquence d'acide nucléique choisie parmi SEQ ID NO. 1369-1393 ou avec une séquence d'ARN correspondante.

24. Molécule d'acide nucléique artificielle selon la revendication 23, où le fragment consiste en une suite continue de nucléotides correspondant à une suite continue de nucléotides dans la séquence de longueur intégrale, qui représente au moins 80%, de préférence au moins 90%, de la séquence de longueur intégrale de laquelle est dérivé le fragment.

25. Molécule d'acide nucléique artificielle selon la revendication 23 ou 24, où le fragment présente une longueur d'au moins environ 50 nucléotides, de préférence d'au moins environ 75 nucléotides, de préférence encore d'au moins environ 100 nucléotides, de préférence encore d'au moins environ 125 nucléotides, de manière particulièrement préférable d'au moins environ 150 nucléotides.

26. Molécule d'acide nucléique artificielle selon l'une quelconque des revendications 1-25, où le au moins un élément de 3'UTR présente une longueur d'au moins environ 50 nucléotides, de préférence d'au moins environ 75 nucléotides, de préférence encore d'au moins environ 100 nucléotides, de préférence encore d'au moins environ 125 nucléotides, de manière particulièrement préférable d'au moins environ 150 nucléotides.

27. Molécule d'acide nucléique artificielle selon l'une quelconque des revendications 1-26, où le cadre de lecture ouvert ne code pas une protéine rapporteur, comme une protéine GFP, une protéine de type luciférase, une protéine de type globine, l'hormone de croissance humaine, ou l'albumine humaine, de préférence pas l'albumine.

28. Molécule d'acide nucléique artificielle selon l'une quelconque des revendications 1-27, comprenant en outre une structure de coiffe en 5', une séquence poly(C) et/ou un motif IRES.

29. Molécule d'acide nucléique artificielle selon l'une quelconque des revendications 1-28, comprenant en outre une séquence contenant un promoteur.

30. Molécule d'acide nucléique artificielle selon l'une quelconque des revendications 1-29, où la molécule d'acide nucléique artificielle, de préférence le cadre de lecture ouvert, est au moins partiellement G/C modifié, de préférence où la teneur en G/C du cadre de lecture ouvert est augmentée par comparaison avec le cadre de lecture ouvert de type sauvage.

31. Molécule d'acide nucléique artificielle selon l'une quelconque des revendications 1-30, où le cadre de lecture ouvert comprend une région optimisée quant aux codons, de préférence, où le cadre de lecture ouvert est optimisé quant aux codons.

**32.** Molécule d'acide nucléique artificielle selon l'une quelconque des revendications 1-31, qui est un ARN, de préférence une molécule d'ARNm.

**33.** Vecteur comprenant:

a. au moins un élément de région non traduite en 5' (élément de 5'UTR) qui comprend ou consiste en
une séquence d'acide nucléique qui consiste en une suite continue de nucléotides correspondant à une suite continue de nucléotides dans la 5'UTR de longueur intégrale d'un gène TOP, qui représente au moins 80% de la 5'UTR de longueur intégrale d'un gène TOP, ou
une séquence d'acide nucléique qui consiste en une suite continue de nucléotides correspondant à une suite continue de nucléotides dans un variant de la 5'UTR d'un gène TOP, qui représente au moins 80% du variant de la 5'UTR d'un gène TOP, où le variant de la 5'UTR d'un gène TOP est identique à au moins 80 % à la 5'UTR naturelle de laquelle est dérivé le variant,
où le au moins un élément de 5'UTR ne comprend pas un motif de 5'TOP fonctionnel;
b. au moins un cadre de lecture ouvert (ORF) et/ou au moins un site de clonage; et
c. au moins un élément de 3'UTR, qui comprend ou consiste en
une séquence d'acide nucléique qui consiste en une suite continue de nucléotides correspondant à une suite continue de nucléotides dans la 3'UTR de longueur intégrale d'un gène d'albumine, qui représente au moins 80% de la 3'UTR de longueur intégrale d'un gène d'albumine, ou
une séquence d'acide nucléique qui consiste en une suite continue de nucléotides correspondant à une suite continue de nucléotides dans un variant d'une 3'UTR d'un gène d'albumine, qui représente au moins 80% du variant de la 3'UTR d'un gène d'albumine, où le variant de la 3'UTR d'un gène d'albumine est identique à au moins 80 % à la 3'UTR naturelle de laquelle est dérivé le variant.

**34.** Vecteur selon la revendication 33, où l'élément de 5'UTR et le cadre de lecture ouvert sont hétérologues.

**35.** Vecteur selon la revendication 33 ou 34, qui est un vecteur à ARN ou ADN.

**36.** Vecteur selon l'une quelconque des revendications 33-35, qui est un vecteur plasmidique ou un vecteur viral, de préférence un vecteur plasmidique.

**37.** Vecteur selon l'une quelconque des revendications 33-36, qui comprend ou code une molécule d'acide nucléique artificielle selon l'une quelconque des revendications 1-32.

**38.** Vecteur selon l'une quelconque des revendications 33-37, qui est une molécule circulaire.

**39.** Vecteur selon la revendication 38, où l'ORF, la séquence poly(A) ou l'élément de 3'UTR du brin codant est suivi dans la direction 5'->3' par un site de restriction pour la linéarisation de la molécule de vecteur circulaire.

**40.** Cellule comprenant la molécule d'acide nucléique artificielle selon l'une quelconque des revendications 1-32 ou le vecteur selon l'une quelconque des revendications 33-39, de préférence une cellule de mammifère, de préférence encore une cellule isolée d'un sujet mammifère, de préférence d'un sujet humain.

**41.** Composition pharmaceutique comprenant la molécule d'acide nucléique artificielle selon l'une quelconque des revendications 1-32, le vecteur selon l'une quelconque des revendications 33-39, ou la cellule selon la revendication 40, de préférence comprenant en outre un ou plusieurs diluants et/ou excipients pharmaceutiquement acceptables et/ou un ou plusieurs adjuvants.

**42.** Molécule d'acide nucléique artificielle selon l'une quelconque des revendications 1-32, vecteur selon l'une quelconque des revendications 33-39, cellule selon la revendication 40, ou composition pharmaceutique selon la revendication 41 destiné à être utilisé comme médicament.

**43.** Molécule d'acide nucléique artificielle selon l'une quelconque des revendications 1-32, vecteur selon l'une quelconque des revendications 33-39, cellule selon la revendication 40, ou composition pharmaceutique selon la revendication 41 destiné à être utilisé comme vaccin ou destiné à être utilisé en thérapie génique.

## Nucleotide sequence of PpLuc(GC) – A64N64

GGGAGAAAGCTTGAGGATGGAGGACGCCAAGAACATCAAGAAGGGCCCGGCGCCCTTCTA
*CCCGCTGGAGGACGGGACCGCCGGCGAGCAGCTCCACAAGGCCATGAAGCGGTACGCCCT*
*GGTGCCGGGCACGATCGCCTTCACCGACGCCCACATCGAGGTCGACATCACCTACGCGGA*
*GTACTTCGAGATGAGCGTGCGCCTGGCCGAGGCCATGAAGCGGTACGGCCTGAACACCAA*
*CCACCGGATCGTGGTGTGCTCGGAGAACAGCCTGCAGTTCTTCATGCCGGTGCTGGGCGC*
*CCTCTTCATCGGCGTGGCCGTCGCCCCGGCGAACGACATCTACAACGAGCGGGAGCTGCT*
*GAACAGCATGGGGATCAGCCAGCCGACCGTGGTGTTCGTGAGCAAGAAGGGCCTGCAGAA*
*GATCCTGAACGTGCAGAAGAAGCTGCCCATCATCCAGAAGATCATCATCATGGACAGCAA*
*GACCGACTACCAGGGCTTCCAGTCGATGTACACGTTCGTGACCAGCCACCTCCCGCCGGG*
*CTTCAACGAGTACGACTTCGTCCCGGAGAGCTTCGACCGGGACAAGACCATCGCCCTGAT*
*CATGAACAGCAGCGGCAGCACCGGCCTGCCGAAGGGGGTGGCCCTGCCGCACCGGACCGC*
*CTGCGTGCGCTTCTCGCACGCCCGGGACCCCATCTTCGGCAACCAGATCATCCCGGACAC*
*CGCCATCCTGAGCGTGGTGCCGTTCCACCACGGCTTCGGCATGTTCACGACCCTGGGCTA*
*CCTCATCTGCGGCTTCCGGGTGGTCCTGATGTACCGGTTCGAGGAGGAGCTGTTCCTGCG*
*GAGCCTGCAGGACTACAAGATCCAGAGCGCGCTGCTCGTGCCGACCCTGTTCAGCTTCTT*
*CGCCAAGAGCACCCTGATCGACAAGTACGACCTGTCGAACCTGCACGAGATCGCCAGCGG*
*GGGCGCCCCGCTGAGCAAGGAGGTGGGCGAGGCCGTGGCCAAGCGGTTCCACCTCCCGGG*
*CATCCGCCAGGGCTACGGCCTGACCGAGACCACGAGCGCGATCCTGATCACCCCCGAGGG*
*GGACGACAAGCCGGGCGCCGTGGGCAAGGTGGTCCCGTTCTTCGAGGCCAAGGTGGTGGA*
*CCTGGACACCGGCAAGACCCTGGGCGTGAACCAGCGGGGCGAGCTGTGCGTGCGGGGGCC*
*GATGATCATGAGCGGCTACGTGAACAACCCGGAGGCCACCAACGCCCTCATCGACAAGGA*
*CGGCTGGCTGCACAGCGGCGACATCGCCTACTGGGACGAGGACGAGCACTTCTTCATCGT*
*CGACCGGCTGAAGTCGCTGATCAAGTACAAGGGCTACCAGGTGGCGCCGGCCGAGCTGGA*
*GAGCATCCTGCTCCAGCACCCCAACATCTTCGACGCCGGCGTGGCCGGGCTGCCGGACGA*
*CGACGCCGGCGAGCTGCCGGCCGCGGTGGTGGTGCTGGAGCACGGCAAGACCATGACGGA*
*GAAGGAGATCGTCGACTACGTGGCCAGCCAGGTGACCACCGCCAAGAAGCTGCGGGGCGG*
*CGTGGTGTTCGTGGACGAGGTCCCGAAGGGCCTGACCGGGAAGCTCGACGCCCGGAAGAT*
*CCGCGAGATCCTGATCAAGGCCAAGAAGGGCGGCAAGATCGCCGTGTAAGACTAGTAGAT*
CTAAAAAAAAAAAAAAAAAAAAAAAAAAAAAAAAAAAAAAAAAAAAAAAAAAAAAAAAAAA
AAAAAATGCATCCCCCCCCCCCCCCCCCCCCCCCCCCCCCCCCCAAAGGCTCTTTTCAGAGC
CACCAGAATT

## Fig. 1

## Nucleotide sequence of PpLuc(GC) – albumin7 – A64N64

GGGAGAAAGCTTGAGG*ATGGAGGACGCCAAGAACATCAAGAAGGGCCCGGCGCCCTTCTA*
*CCCGCTGGAGGACGGGACCGCCGGCGAGCAGCTCCACAAGGCCATGAAGCGGTACGCCCT*
*GGTGCCGGGCACGATCGCCTTCACCGACGCCCACATCGAGGTCGACATCACCTACGCGGA*
*GTACTTCGAGATGAGCGTGCGCCTGGCCGAGGCCATGAAGCGGTACGGCCTGAACACCAA*
*CCACCGGATCGTGGTGTGCTCGGAGAACAGCCTGCAGTTCTTCATGCCGGTGCTGGGCGC*
*CCTCTTCATCGGCGTGGCCGTCGCCCCGGCGAACGACATCTACAACGAGCGGGAGCTGCT*
*GAACAGCATGGGGATCAGCCAGCCGACCGTGGTGTTCGTGAGCAAGAAGGGCCTGCAGAA*
*GATCCTGAACGTGCAGAAGAAGCTGCCCATCATCCAGAAGATCATCATCATGGACAGCAA*
*GACCGACTACCAGGGCTTCCAGTCGATGTACACGTTCGTGACCAGCCACCTCCCGCCGGG*
*CTTCAACGAGTACGACTTCGTCCCGGAGAGCTTCGACCGGGACAAGACCATCGCCCTGAT*
*CATGAACAGCAGCGGCAGCACCGGCCTGCCGAAGGGGGTGGCCCTGCCGCACCGGACCGC*
*CTGCGTGCGCTTCTCGCACGCCCGGGACCCCATCTTCGGCAACCAGATCATCCCGGACAC*
*CGCCATCCTGAGCGTGGTGCCGTTCCACCACGGCTTCGGCATGTTCACGACCCTGGGCTA*
*CCTCATCTGCGGCTTCCGGGTGGTCCTGATGTACCGGTTCGAGGAGGAGCTGTTCCTGCG*
*GAGCCTGCAGGACTACAAGATCCAGAGCGCGCTGCTCGTGCCGACCCTGTTCAGCTTCTT*
*CGCCAAGAGCACCCTGATCGACAAGTACGACCTGTCGAACCTGCACGAGATCGCCAGCGG*
*GGGCGCCCCGCTGAGCAAGGAGGTGGGCGAGGCCGTGGCCAAGCGGTTCCACCTCCCGGG*
*CATCCGCCAGGGCTACGGCCTGACCGAGACCACGAGCGCGATCCTGATCACCCCCGAGGG*
*GGACGACAAGCCGGGCGCCGTGGGCAAGGTGGTCCCGTTCTTCGAGGCCAAGGTGGTGGA*
*CCTGGACACCGGCAAGACCCTGGGCGTGAACCAGCGGGGCGAGCTGTGCGTGCGGGGGCC*
*GATGATCATGAGCGGCTACGTGAACAACCCGGAGGCCACCAACGCCCTCATCGACAAGGA*
*CGGCTGGCTGCACAGCGGCGACATCGCCTACTGGGACGAGGACGAGCACTTCTTCATCGT*
*CGACCGGCTGAAGTCGCTGATCAAGTACAAGGGCTACCAGGTGGCGCCGGCCGAGCTGGA*
*GAGCATCCTGCTCCAGCACCCCAACATCTTCGACGCCGGCGTGGCCGGGCTGCCGGACGA*
*CGACGCCGGCGAGCTGCCGGCCGCGGTGGTGGTGCTGGAGCACGGCAAGACCATGACGGA*
*GAAGGAGATCGTCGACTACGTGGCCAGCCAGGTGACCACCGCCAAGAAGCTGCGGGGCGG*
*CGTGGTGTTCGTGGACGAGGTCCCGAAGGGCCTGACCGGGAAGCTCGACGCCCGGAAGAT*
*CCGCGAGATCCTGATCAAGGCCAAGAAGGGCGGCAAGATCGCCGTGTAAGACTAGTG*<u>CAT</u>
<u>CACATTTAAAAGCATCTCAGCCTACCATGAGAATAAGAGAAAGAAAATGAAGATCAATAG</u>
<u>CTTATTCATCTCTTTTTCTTTTTCGTTGGTGTAAAGCCAACACCCTGTCTAAAAAACATA</u>
<u>AATTTCTTTAATCATTTTGCCTCTTTTCTCTGTGCTTCAATTAATAAAAAATGGAAAGAA</u>
<u>CCTAGATCTAAAAAAAAAAAAAAAAAAAAAAAAAAAAAAAAAAAAAAAAAAAAAAAAAAAA</u>
<u>AAAAAAAAAAAAATGCATCCCCCCCCCCCCCCCCCCCCCCCCCCCCCCCCAAAGGCTCTTT</u>
<u>TCAGAGCCACCAGAATT</u>

Fig. 2

## Nucleotide sequence of RPL32 – PpLuc(GC) – A64N64

GGGGCGCTGCCTACGGAGGTGGCAGCCATCTCCTTCTCGGCATCAAGCTTGAGGA*TGGAG*
*GACGCCAAGAACATCAAGAAGGGCCCGGCGCCCTTCTACCCGCTGGAGGACGGGACCGCC*
*GGCGAGCAGCTCCACAAGGCCATGAAGCGGTACGCCCTGGTGCCGGGCACGATCGCCTTC*
*ACCGACGCCCACATCGAGGTCGACATCACCTACGCGGAGTACTTCGAGATGAGCGTGCGC*
*CTGGCCGAGGCCATGAAGCGGTACGGCCTGAACACCAACCACCGGATCGTGGTGTGCTCG*
*GAGAACAGCCTGCAGTTCTTCATGCCGGTGCTGGGCGCCCTCTTCATCGGCGTGGCCGTC*
*GCCCCGGCGAACGACATCTACAACGAGCGGGAGCTGCTGAACAGCATGGGGATCAGCCAG*
*CCGACCGTGGTGTTCGTGAGCAAGAAGGGCCTGCAGAAGATCCTGAACGTGCAGAAGAAG*
*CTGCCCATCATCCAGAAGATCATCATCATGGACAGCAAGACCGACTACCAGGGCTTCCAG*
*TCGATGTACACGTTCGTGACCAGCCACCTCCCGCCGGGCTTCAACGAGTACGACTTCGTC*
*CCGGAGAGCTTCGACCGGGACAAGACCATCGCCCTGATCATGAACAGCAGCGGCAGCACC*
*GGCCTGCCGAAGGGGGTGGCCCTGCCGCACCGGACCGCCTGCGTGCGCTTCTCGCACGCC*
*CGGGACCCCATCTTCGGCAACCAGATCATCCCGGACACCGCCATCCTGAGCGTGGTGCCG*
*TTCCACCACGGCTTCGGCATGTTCACGACCCTGGGCTACCTCATCTGCGGCTTCCGGGTG*
*GTCCTGATGTACCGGTTCGAGGAGGAGCTGTTCCTGCGGAGCCTGCAGGACTACAAGATC*
*CAGAGCGCGCTGCTCGTGCCGACCCTGTTCAGCTTCTTCGCCAAGAGCACCCTGATCGAC*
*AAGTACGACCTGTCGAACCTGCACGAGATCGCCAGCGGGGGCGCCCCGCTGAGCAAGGAG*
*GTGGGCGAGGCCGTGGCCAAGCGGTTCCACCTCCCGGGCATCCGCCAGGGCTACGGCCTG*
*ACCGAGACCACGAGCGCGATCCTGATCACCCCCGAGGGGGACGACAAGCCGGGCGCCGTG*
*GGCAAGGTGGTCCCGTTCTTCGAGGCCAAGGTGGTGGACCTGGACACCGGCAAGACCCTG*
*GGCGTGAACCAGCGGGGCGAGCTGTGCGTGCGGGGGCCGATGATCATGAGCGGCTACGTG*
*AACAACCCGGAGGCCACCAACGCCCTCATCGACAAGGACGGCTGGCTGCACAGCGGCGAC*
*ATCGCCTACTGGGACGAGGACGAGCACTTCTTCATCGTCGACCGGCTGAAGTCGCTGATC*
*AAGTACAAGGGCTACCAGGTGGCGCCGGCCGAGCTGGAGAGCATCCTGCTCCAGCACCCC*
*AACATCTTCGACGCCGGCGTGGCCGGGCTGCCGGACGACGACGCCGGCGAGCTGCCGGCC*
*GCGGTGGTGGTGCTGGAGCACGGCAAGACCATGACGGAGAAGGAGATCGTCGACTACGTG*
*GCCAGCCAGGTGACCACCGCCAAGAAGCTGCGGGGCGGCGTGGTGTTCGTGGACGAGGTC*
*CCGAAGGGCCTGACCGGGAAGCTCGACGCCCGGAAGATCCGCGAGATCCTGATCAAGGCC*
*AAGAAGGGCGGCAAGATCGCCGTGTAAGACTAGTAGATCTAAAAAAAAAAAAAAAAAAAAA*
AAAAAAAAAAAAAAAAAAAAAAAAAAAAAAAAAAAAAAAAAAAAAATGCATCCCCCCCCCC
CCCCCCCCCCCCCCCCCCCAAAGGCTCTTTTCAGAGCCACCAGAATT

## Fig. 3

## Nucleotide sequence of RPL32 – PpLuc(GC) – albumin7 – A64N64

GGGGCGCTGCCTACGGAGGTGGCAGCCATCTCCTTCTCGGCATCAAGCTTGAGGA*TGGAG*
*GACGCCAAGAACATCAAGAAGGGCCCGGCGCCCTTCTACCCGCTGGAGGACGGGACCGCC*
*GGCGAGCAGCTCCACAAGGCCATGAAGCGGTACGCCCTGGTGCCGGGCACGATCGCCTTC*
*ACCGACGCCCACATCGAGGTCGACATCACCTACGCGGAGTACTTCGAGATGAGCGTGCGC*
*CTGGCCGAGGCCATGAAGCGGTACGGCCTGAACACCAACCACCGGATCGTGGTGTGCTCG*
*GAGAACAGCCTGCAGTTCTTCATGCCGGTGCTGGGCGCCCTCTTCATCGGCGTGGCCGTC*
*GCCCCGGCGAACGACATCTACAACGAGCGGGAGCTGCTGAACAGCATGGGGATCAGCCAG*
*CCGACCGTGGTGTTCGTGAGCAAGAAGGGCCTGCAGAAGATCCTGAACGTGCAGAAGAAG*
*CTGCCCATCATCCAGAAGATCATCATCATGGACAGCAAGACCGACTACCAGGGCTTCCAG*
*TCGATGTACACGTTCGTGACCAGCCACCTCCCGCCGGGCTTCAACGAGTACGACTTCGTC*
*CCGGAGAGCTTCGACCGGGACAAGACCATCGCCCTGATCATGAACAGCAGCGGCAGCACC*
*GGCCTGCCGAAGGGGGTGGCCCTGCCGCACCGGACCGCCTGCGTGCGCTTCTCGCACGCC*
*CGGGACCCCATCTTCGGCAACCAGATCATCCCGGACACCGCCATCCTGAGCGTGGTGCCG*
*TTCCACCACGGCTTCGGCATGTTCACGACCCTGGGCTACCTCATCTGCGGCTTCCGGGTG*
*GTCCTGATGTACCGGTTCGAGGAGGAGCTGTTCCTGCGGAGCCTGCAGGACTACAAGATC*
*CAGAGCGCGCTGCTCGTGCCGACCCTGTTCAGCTTCTTCGCCAAGAGCACCCTGATCGAC*
*AAGTACGACCTGTCGAACCTGCACGAGATCGCCAGCGGGGGCGCCCCGCTGAGCAAGGAG*
*GTGGGCGAGGCCGTGGCCAAGCGGTTCCACCTCCCGGGCATCCGCCAGGGCTACGGCCTG*
*ACCGAGACCACGAGCGCGATCCTGATCACCCCCGAGGGGGACGACAAGCCGGGCGCCGTG*
*GGCAAGGTGGTCCCGTTCTTCGAGGCCAAGGTGGTGGACCTGGACACCGGCAAGACCCTG*
*GGCGTGAACCAGCGGGGCGAGCTGTGCGTGCGGGGGCCGATGATCATGAGCGGCTACGTG*
*AACAACCCGGAGGCCACCAACGCCCTCATCGACAAGGACGGCTGGCTGCACAGCGGCGAC*
*ATCGCCTACTGGGACGAGGACGAGCACTTCTTCATCGTCGACCGGCTGAAGTCGCTGATC*
*AAGTACAAGGGCTACCAGGTGGCGCCGGCCGAGCTGGAGAGCATCCTGCTCCAGCACCCC*
*AACATCTTCGACGCCGGCGTGGCCGGGCTGCCGGACGACGACGCCGGCGAGCTGCCGGCC*
*GCGGTGGTGGTGCTGGAGCACGGCAAGACCATGACGGAGAAGGAGATCGTCGACTACGTG*
*GCCAGCCAGGTGACCACCGCCAAGAAGCTGCGGGGCGGCGTGGTGTTCGTGGACGAGGTC*
*CCGAAGGGCCTGACCGGGAAGCTCGACGCCCGGAAGATCCGCGAGATCCTGATCAAGGCC*
*AAGAAGGGCGGCAAGATCGCCGTGTAAGACTAGTG*CATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAATGCATCCCC
CCCCCCCCCCCCCCCCCCCCCCCCCCAAAGGCTCTTTTCAGAGCCACCAGAATT

Fig. 4

**PpLuc from mRNA with combined UTRs (HDF)**

Fig. 5

Nucleotide sequence of RPL35 – PpLuc(GC) – albumin7 – A64N64

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAAAATGCATCCCCCCCCCCC
CCCCCCCCCCCCCCCCCCAAAGGCTCTTTTCAGAGCCACCAGAATT

Fig. 6

## Nucleotide sequence of RPL21 – PpLuc(GC) – albumin7 – A64N64

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAAA
AAAAAAAAAAAAAAAAAAAAAAAAAAAAAAAAAAAAAAAAAAAATGCATCCCCCCCCCCCC
CCCCCCCCCCCCCCCCCAAAGGCTCTTTTCAGAGCCACCAGAATT

**Fig. 7**

## Nucleotide sequence of atp5a1 – PpLuc(GC) – albumin7 – A64N64

GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC
AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG
CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTGCATCACATTTAAAAGCATCTCAGCCTACCATGAGAATAAGAGAAAGAAAATG
AAGATCAATAGCTTATTCATCTCTTTTTCTTTTTCGTTGGTGTAAAGCCAACACCCTGTC
TAAAAAACATAAATTTCTTTAATCATTTTGCCTCTTTTCTCTGTGCTTCAATTAATAAAA
AATGGAAAGAACCTAGATCTAAAAAAAAAAAAAAAAAAAAAAAAAAAAAAAAAAAAAAAAA
AAAAAAAAAAAAAAAAAAAAAAAATGCATCCCCCCCCCCCCCCCCCCCCCCCCCCCCCCCC
AAAGGCTCTTTTCAGAGCCACCAGAATT

Fig. 8

Nucleotide sequence of HSD17B4 – PpLuc(GC) – albumin7 – A64N64

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTGCATCA
CATTTAAAAGCATCTCAGCCTACCATGAGAATAAGAGAAAGAAAATGAAGATCAATAGCT
TATTCATCTCTTTTTCTTTTTCGTTGGTGTAAAGCCAACACCCTGTCTAAAAAACATAAA
TTTCTTTAATCATTTTGCCTCTTTTCTCTGTGCTTCAATTAATAAAAAATGGAAAGAACC
TAGATCTAAAAAAAAAAAAAAAAAAAAAAAAAAAAAAAAAAAAAAAAAAAAAAAAAAAAAA
AAAAAAAAAAATGCATCCCCCCCCCCCCCCCCCCCCCCCCCCCCCCCCCAAAGGCTCTTTTC
AGAGCCACCAGAATT

Fig. 9

## Nucleotide sequence of AlG1 – PpLuc(GC) – albumin7 – A64N64

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTGCATCACATTTAAAAGCATCTCAGCCTACCATGAGAA
TAAGAGAAAGAAAATGAAGATCAATAGCTTATTCATCTCTTTTTCTTTTTCGTTGGTGTA
AAGCCAACACCCTGTCTAAAAAACATAAATTTCTTTAATCATTTTGCCTCTTTTCTCTGT
GCTTCAATTAATAAAAAATGGAAAGAACCTAGATCTAAAAAAAAAAAAAAAAAAAAAAAA
AAAAAAAAAAAAAAAAAAAAAAAAAAAAAAAAAAAAAAAAATGCATCCCCCCCCCCCCCC
CCCCCCCCCCCCCCCAAAGGCTCTTTTCAGAGCCACCAGAATT

## Fig. 10

## Nucleotide sequence of COX6C – PpLuc(GC) – albumin7 – A64N64

GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGT<u>GCATCACATTTA</u>
<u>AAAGCATCTCAGCCTACCATGAGAATAAGAGAAAGAAAATGAAGATCAATAGCTTATTCA</u>
<u>TCTCTTTTTCTTTTTCGTTGGTGTAAAGCCAACACCCTGTCTAAAAAACATAAATTTCTT</u>
TAATCATTTTGCCTCTTTTCTCTGTGCTTCAATTAATAAAAAATGGAAAGAACCTAGATC
TAAAAAAAAAAAAAAAAAAAAAAAAAAAAAAAAAAAAAAAAAAAAAAAAAAAAAAAAAAAA
AAAAATGCATCCCCCCCCCCCCCCCCCCCCCCCCCCCCCCCCAAAGGCTCTTTTCAGAGCC
ACCAGAATT

Fig. 11

Nucleotide sequence of ASAH1 – PpLuc(GC) – albumin7 – A64N64

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAAATGCATCCCC
CCCCCCCCCCCCCCCCCCCCCCCCCAAAGGCTCTTTTCAGAGCCACCAGAATT

Fig. 12

## Nucleotide sequence of mRPL21 – PpLuc(GC) – albumin7 – A64N64

GGGGCCGCCGCAGCCATCTTCCAGTAACTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAAAAATGCATCCCCCCCCCCC
CCCCCCCCCCCCCCCCCCAAAGGCTCTTTTCAGAGCCACCAGAATT

## Fig. 13

## Nucleotide sequence of mRPL35A – PpLuc(GC) – albumin7 – A64N64

GGG*CCATCTTGGCGCCTGTGGAGGCCTGCTGGGAACAGGACTTCTAACAGCAAGTAAGCT*
*TGAGGATGGAGGACGCCAAGAACATCAAGAAGGGCCCGGCGCCCTTCTACCCGCTGGAGG*
*ACGGGACCGCCGGCGAGCAGCTCCACAAGGCCATGAAGCGGTACGCCCTGGTGCCGGGCA*
*CGATCGCCTTCACCGACGCCCACATCGAGGTCGACATCACCTACGCGGAGTACTTCGAGA*
*TGAGCGTGCGCCTGGCCGAGGCCATGAAGCGGTACGGCCTGAACACCAACCACCGGATCG*
*TGGTGTGCTCGGAGAACAGCCTGCAGTTCTTCATGCCGGTGCTGGGCGCCCTCTTCATCG*
*GCGTGGCCGTCGCCCCGGCGAACGACATCTACAACGAGCGGGAGCTGCTGAACAGCATGG*
*GGATCAGCCAGCCGACCGTGGTGTTCGTGAGCAAGAAGGGCCTGCAGAAGATCCTGAACG*
*TGCAGAAGAAGCTGCCCATCATCCAGAAGATCATCATCATGGACAGCAAGACCGACTACC*
*AGGGCTTCCAGTCGATGTACACGTTCGTGACCAGCCACCTCCCGCCGGGCTTCAACGAGT*
*ACGACTTCGTCCCGGAGAGCTTCGACCGGGACAAGACCATCGCCCTGATCATGAACAGCA*
*GCGGCAGCACCGGCCTGCCGAAGGGGGTGGCCCTGCCGCACCGGACCGCCTGCGTGCGCT*
*TCTCGCACGCCCGGGACCCCATCTTCGGCAACCAGATCATCCCGGACACCGCCATCCTGA*
*GCGTGGTGCCGTTCCACCACGGCTTCGGCATGTTCACGACCCTGGGCTACCTCATCTGCG*
*GCTTCCGGGTGGTCCTGATGTACCGGTTCGAGGAGGAGCTGTTCCTGCGGAGCCTGCAGG*
*ACTACAAGATCCAGAGCGCGCTGCTCGTGCCGACCCTGTTCAGCTTCTTCGCCAAGAGCA*
*CCCTGATCGACAAGTACGACCTGTCGAACCTGCACGAGATCGCCAGCGGGGGCGCCCCGC*
*TGAGCAAGGAGGTGGGCGAGGCCGTGGCCAAGCGGTTCCACCTCCCGGGCATCCGCCAGG*
*GCTACGGCCTGACCGAGACCACGAGCGCGATCCTGATCACCCCCGAGGGGGACGACAAGC*
*CGGGCGCCGTGGGCAAGGTGGTCCCGTTCTTCGAGGCCAAGGTGGTGGACCTGGACACCG*
*GCAAGACCCTGGGCGTGAACCAGCGGGGCGAGCTGTGCGTGCGGGGGCCGATGATCATGA*
*GCGGCTACGTGAACAACCCGGAGGCCACCAACGCCCTCATCGACAAGGACGGCTGGCTGC*
*ACAGCGGCGACATCGCCTACTGGGACGAGGACGAGCACTTCTTCATCGTCGACCGGCTGA*
*AGTCGCTGATCAAGTACAAGGGCTACCAGGTGGCGCCGGCCGAGCTGGAGAGCATCCTGC*
*TCCAGCACCCCAACATCTTCGACGCCGGCGTGGCCGGGCTGCCGGACGACGACGCCGGCG*
*AGCTGCCGGCCGCGGTGGTGGTGCTGGAGCACGGCAAGACCATGACGGAGAAGGAGATCG*
*TCGACTACGTGGCCAGCCAGGTGACCACCGCCAAGAAGCTGCGGGGCGGCGTGGTGTTCG*
*TGGACGAGGTCCCGAAGGGCCTGACCGGGAAGCTCGACGCCCGGAAGATCCGCGAGATCC*
*TGATCAAGGCCAAGAAGGGCGGCAAGATCGCCGTGTAAGACTAGTG*CATCACATTTAAAA
GCATCTCAGCCTACCATGAGAATAAGAGAAAGAAAATGAAGATCAATAGCTTATTCATCT
CTTTTTCTTTTTCGTTGGTGTAAAGCCAACACCCTGTCTAAAAAACATAAATTTCTTTAA
TCATTTTGCCTCTTTTCTCTGTGCTTCAATTAATAAAAAATGGAAAGAACCTAGATCTAA
AAAAAAAAAAAAAAAAAAAAAAAAAAAAAAAAAAAAAAAAAAAAAAAAAAAAAAAAAAAAA
AATGCATCCCCCCCCCCCCCCCCCCCCCCCCCCCCCCCCCAAAGGCTCTTTTCAGAGCCACC
AGAATT

Fig. 14

## Nucleotide sequence of RPL35 – PpLuc(GC) – A64N64

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTAGATCTAAAAAAAAAAAAAAAAAAAAAAAAAAAA
AAAAAAAAAAAAAAAAAAAAAAAAAAAAAAAAAAAAATGCATCCCCCCCCCCCCCCCCCC
CCCCCCCCCCCCAAAGGCTCTTTTCAGAGCCACCAGAATT

Fig. 15

## Nucleotide sequence of RPL21 – PpLuc(GC) – A64N64

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTAGATCTAAAAAAAAAAAAAAAAAAAAAAAAAAAA
AAAAAAAAAAAAAAAAAAAAAAAAAAAAAAAAAAATGCATCCCCCCCCCCCCCCCCCCCC
CCCCCCCCCCCAAAGGCTCTTTTCAGAGCCACCAGAATT

Fig. 16

## Nucleotide sequence of atp5a1 – PpLuc(GC) – A64N64

GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
*GCGCCCTTCTACCCGCTGGAGGACGGGACCGCCGGCGAGCAGCTCCACAAGGCCATGAAG*
*CGGTACGCCCTGGTGCCGGGCACGATCGCCTTCACCGACGCCCACATCGAGGTCGACATC*
*ACCTACGCGGAGTACTTCGAGATGAGCGTGCGCCTGGCCGAGGCCATGAAGCGGTACGGC*
*CTGAACACCAACCACCGGATCGTGGTGTGCTCGGAGAACAGCCTGCAGTTCTTCATGCCG*
*GTGCTGGGCGCCCTCTTCATCGGCGTGGCCGTCGCCCCGGCGAACGACATCTACAACGAG*
*CGGGAGCTGCTGAACAGCATGGGGATCAGCCAGCCGACCGTGGTGTTCGTGAGCAAGAAG*
*GGCCTGCAGAAGATCCTGAACGTGCAGAAGAAGCTGCCCATCATCCAGAAGATCATCATC*
*ATGGACAGCAAGACCGACTACCAGGGCTTCCAGTCGATGTACACGTTCGTGACCAGCCAC*
*CTCCCGCCGGGCTTCAACGAGTACGACTTCGTCCCGGAGAGCTTCGACCGGGACAAGACC*
*ATCGCCCTGATCATGAACAGCAGCGGCAGCACCGGCCTGCCGAAGGGGGTGGCCCTGCCG*
*CACCGGACCGCCTGCGTGCGCTTCTCGCACGCCCGGGACCCCATCTTCGGCAACCAGATC*
*ATCCCGGACACCGCCATCCTGAGCGTGGTGCCGTTCCACCACGGCTTCGGCATGTTCACG*
*ACCCTGGGCTACCTCATCTGCGGCTTCCGGGTGGTCCTGATGTACCGGTTCGAGGAGGAG*
*CTGTTCCTGCGGAGCCTGCAGGACTACAAGATCCAGAGCGCGCTGCTCGTGCCGACCCTG*
*TTCAGCTTCTTCGCCAAGAGCACCCTGATCGACAAGTACGACCTGTCGAACCTGCACGAG*
*ATCGCCAGCGGGGGCGCCCCGCTGAGCAAGGAGGTGGGCGAGGCCGTGGCCAAGCGGTTC*
*CACCTCCCGGGCATCCGCCAGGGCTACGGCCTGACCGAGACCACGAGCGCGATCCTGATC*
*ACCCCCGAGGGGGACGACAAGCCGGGCGCCGTGGGCAAGGTGGTCCCGTTCTTCGAGGCC*
*AAGGTGGTGGACCTGGACACCGGCAAGACCCTGGGCGTGAACCAGCGGGGCGAGCTGTGC*
*GTGCGGGGGCCGATGATCATGAGCGGCTACGTGAACAACCCGGAGGCCACCAACGCCCTC*
*ATCGACAAGGACGGCTGGCTGCACAGCGGCGACATCGCCTACTGGGACGAGGACGAGCAC*
*TTCTTCATCGTCGACCGGCTGAAGTCGCTGATCAAGTACAAGGGCTACCAGGTGGCGCCG*
*GCCGAGCTGGAGAGCATCCTGCTCCAGCACCCCAACATCTTCGACGCCGGCGTGGCCGGG*
*CTGCCGGACGACGACGCCGGCGAGCTGCCGGCCGCGGTGGTGGTGCTGGAGCACGGCAAG*
*ACCATGACGGAGAAGGAGATCGTCGACTACGTGGCCAGCCAGGTGACCACCGCCAAGAAG*
*CTGCGGGGCGGCGTGGTGTTCGTGGACGAGGTCCCGAAGGGCCTGACCGGGAAGCTCGAC*
*GCCCGGAAGATCCGCGAGATCCTGATCAAGGCCAAGAAGGGCGGCAAGATCGCCGTGTAA*
GACTAGTAGATCTAAAAAAAAAAAAAAAAAAAAAAAAAAAAAAAAAAAAAAAAAAAAAAAA
AAAAAAAAAAAAAAAAATGCATCCCCCCCCCCCCCCCCCCCCCCCCCCCCCCCCCAAAGGCT
CTTTTCAGAGCCACCAGAATT

Fig. 17

## Nucleotide sequence of HSD17B4 – PpLuc(GC) – A64N64

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTAGATCT
AAAAAAAAAAAAAAAAAAAAAAAAAAAAAAAAAAAAAAAAAAAAAAAAAAAAAAAAAAAAA
AAAATGCATCCCCCCCCCCCCCCCCCCCCCCCCCCCCCCCAAAGGCTCTTTTCAGAGCCA
CCAGAATT

## Fig. 18

## Nucleotide sequence of AIG1 – PpLuc(GC) – A64N64

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTAGATCTAAAAAAAAAAAAAAAAAAAAAAAAAAAAAAA
AAAAAAAAAAAAAAAAAAAAAAAAAAAAAAAATGCATCCCCCCCCCCCCCCCCCCCCCCC
CCCCCCCCAAAGGCTCTTTTCAGAGCCACCAGAATT

## Fig. 19

## Nucleotide sequence of COX6C – PpLuc(GC) – A64N64

GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTAGATCTAAAAAA
AAAAAAAAAAAAAAAAAAAAAAAAAAAAAAAAAAAAAAAAAAAAAAAAAAAAAAAAAATG
CATCCCCCCCCCCCCCCCCCCCCCCCCCCCCCCCCCAAAGGCTCTTTTCAGAGCCACCAGAA
TT

Fig. 20

## Nucleotide sequence of ASAH1 – PpLuc(GC) – A64N64

GGG<u>CCTCTGCTGGAGTCCGGGGAGTGGCGTTGGCTGCTAGAGCGAAGCTTGAGGA</u>*TGGAG*
*GACGCCAAGAACATCAAGAAGGGCCCGGCGCCCTTCTACCCGCTGGAGGACGGGACCGCC*
*GGCGAGCAGCTCCACAAGGCCATGAAGCGGTACGCCCTGGTGCCGGGCACGATCGCCTTC*
*ACCGACGCCCACATCGAGGTCGACATCACCTACGCGGAGTACTTCGAGATGAGCGTGCGC*
*CTGGCCGAGGCCATGAAGCGGTACGGCCTGAACACCAACCACCGGATCGTGGTGTGCTCG*
*GAGAACAGCCTGCAGTTCTTCATGCCGGTGCTGGGCGCCCTCTTCATCGGCGTGGCCGTC*
*GCCCCGGCGAACGACATCTACAACGAGCGGGAGCTGCTGAACAGCATGGGGATCAGCCAG*
*CCGACCGTGGTGTTCGTGAGCAAGAAGGGCCTGCAGAAGATCCTGAACGTGCAGAAGAAG*
*CTGCCCATCATCCAGAAGATCATCATCATGGACAGCAAGACCGACTACCAGGGCTTCCAG*
*TCGATGTACACGTTCGTGACCAGCCACCTCCCGCCGGGCTTCAACGAGTACGACTTCGTC*
*CCGGAGAGCTTCGACCGGGACAAGACCATCGCCCTGATCATGAACAGCAGCGGCAGCACC*
*GGCCTGCCGAAGGGGGTGGCCCTGCCGCACCGGACCGCCTGCGTGCGCTTCTCGCACGCC*
*CGGGACCCCATCTTCGGCAACCAGATCATCCCGGACACCGCCATCCTGAGCGTGGTGCCG*
*TTCCACCACGGCTTCGGCATGTTCACGACCCTGGGCTACCTCATCTGCGGCTTCCGGGTG*
*GTCCTGATGTACCGGTTCGAGGAGGAGCTGTTCCTGCGGAGCCTGCAGGACTACAAGATC*
*CAGAGCGCGCTGCTCGTGCCGACCCTGTTCAGCTTCTTCGCCAAGAGCACCCTGATCGAC*
*AAGTACGACCTGTCGAACCTGCACGAGATCGCCAGCGGGGGCGCCCCGCTGAGCAAGGAG*
*GTGGGCGAGGCCGTGGCCAAGCGGTTCCACCTCCCGGGCATCCGCCAGGGCTACGGCCTG*
*ACCGAGACCACGAGCGCGATCCTGATCACCCCCGAGGGGGACGACAAGCCGGGCGCCGTG*
*GGCAAGGTGGTCCCGTTCTTCGAGGCCAAGGTGGTGGACCTGGACACCGGCAAGACCCTG*
*GGCGTGAACCAGCGGGGCGAGCTGTGCGTGCGGGGGCCGATGATCATGAGCGGCTACGTG*
*AACAACCCGGAGGCCACCAACGCCCTCATCGACAAGGACGGCTGGCTGCACAGCGGCGAC*
*ATCGCCTACTGGGACGAGGACGAGCACTTCTTCATCGTCGACCGGCTGAAGTCGCTGATC*
*AAGTACAAGGGCTACCAGGTGGCGCCGGCCGAGCTGGAGAGCATCCTGCTCCAGCACCCC*
*AACATCTTCGACGCCGGCGTGGCCGGGCTGCCGGACGACGACGCCGGCGAGCTGCCGGCC*
*GCGGTGGTGGTGCTGGAGCACGGCAAGACCATGACGGAGAAGGAGATCGTCGACTACGTG*
*GCCAGCCAGGTGACCACCGCCAAGAAGCTGCGGGGCGGCGTGGTGTTCGTGGACGAGGTC*
*CCGAAGGGCCTGACCGGGAAGCTCGACGCCCGGAAGATCCGCGAGATCCTGATCAAGGCC*
*AAGAAGGGCGGCAAGATCGCCGTGTAA*GACTAGTAGATCTAAAAAAAAAAAAAAAAAAAA
AAAAAAAAAAAAAAAAAAAAAAAAAAAAAAAAAAAAAAAAAAAAATGCATCCCCCCCCCC
CCCCCCCCCCCCCCCCCCAAAGGCTCTTTTCAGAGCCACCAGAATT

Fig. 21

# PpLuc from mRNA with TOP 5'-UTR (HDF)

5'-UTR

**PpLuc [RLU]**

mean, SEM

Fig. 22

**PpLuc from mRNA with combined UTRs (HDF)**

Fig. 23

**PpLuc from mRNA with combined UTRs (HDF)**

Fig. 24

**PpLuc from mRNA with combined UTRs (HDF)**

Fig. 25

**PpLuc from mRNA with combined UTRs (HDF)**

Fig. 26

## PpLuc from mRNA with combined UTRs (HDF)

Fig. 27

**PpLuc from mRNA with combined UTRs (HDF)**

Fig. 28

Fig. 29

Fig. 30

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02098443 A

### Non-patent literature cited in the description

- **FRIEDEL et al.** Conserved principles of mammalian transcriptional regulation revealed by RNA half-life. *Nucleic Acid Research,* 2009, 1-12
- **JOHNSON et al.** Newly synthesized RNA: Simultaneous measurement in intact cells of transcription rates and RNA stability of insulin-like growth factor I, actin, and albumin in growth hormone-stimulated hepatocytes. *Proc. Natl. Acad. Sci.,* 1991, vol. 88, 5287-5291
- **RODGERS et al.** Regulated α-globin mRNA decay is a cytoplasmic event proceeding through 3'-to-5' exosome-dependent decapping. *RNA,* 2002, vol. 8, 1526-1537
- **WANG et al.** An mRNA stability complex functions with poly(A)-binding protein to stabilize mRNA in vitro. *Molecular and Cellular biology,* July 1999, vol. 19 (7), 4552-4560
- **IADEVAIA et al.** All translation elongation factors and the e, f, and h subunits of translation initiation factor 3 are encoded by 5'-terminal oligopyrimidine (TOP) mRNAs. *RNA,* 2008, vol. 14, 1730-1736
- **AVNI et al.** Vertebrate mRNAs with a 5'-terminal pyrimidine tract are Candidates for translational repression in quiescent cells: characterization of the translational cis-regulatory element. *Molecular and Cellular Biology,* 1994, 3822-3833
- **MEYUHAS et al.** Translational Control of Ribosomal Protein mRNAs in Eukaryotes, Translational Control. Cold Spring Harbor Monograph Archive. Cold Spring Harbor Laboratory Press, 1996, 363-388
- **YAMASHITA et al.** Comprehensive detection of human terminal oligo-pyrimidine (TOP) genes and analysis of their characteristics. *Nucleic Acids Res.,* June 2008, vol. 36 (11), 3707-15
- **LEVY et al.** *Proc Natl Acad Sci USA,* 15 April 1991, vol. 88 (8), 3319-23
- **ØROM et al.** MicroRNA-10a binds the 5'UTR of ribosomal protein mRNAs and enhances their translation. *Mol Cell,* 23 May 2008, vol. 30 (4), 460-71
- **DAMGAARD et al.** *Genes & Development,* 2011, vol. 25 (19), 2057-2068
- **AKASHI.** *Curr. Opin. Genet. Dev.,* 2001, vol. 11 (6), 660-666